(19) Europäisches Patentamt
European Patent Office
Office européen des brevets

(11) EP 1 319 007 B1

# (12) EUROPEAN PATENT SPECIFICATION

(45) Date of publication and mention of the grant of the patent:
**01.03.2006 Bulletin 2006/09**

(21) Application number: **01948503.6**

(22) Date of filing: **20.06.2001**

(51) Int Cl.:
*C07D 491/08* (2006.01) *C07D 495/08* (2006.01)
*C07D 471/08* (2006.01) *A61K 31/40* (2006.01)
*A61K 31/439* (2006.01) *A61P 35/00* (2006.01)

(86) International application number:
**PCT/US2001/019655**

(87) International publication number:
**WO 2002/024702 (28.03.2002 Gazette 2002/12)**

## (54) FUSED HETEROCYCLIC SUCCINIMIDE COMPOUNDS AND ANALOGS THEREOF, MODULATORS OF NUCLEAR HORMONE RECEPTOR FUNCTION

KONDENSIERTE HETEROZYKLISCHE SUCCINIMID DERIVATE UND DEREN ANALOGA, MODULATOREN DER KERN HORMON REZEPTOR FUNKTIONEN

COMPOSES DE SUCCINIMIDE HETEROCYCLIQUES FUSIONNES ET LEURS ANALOGUES, MODULATEURS DE LA FONCTION DE RECEPTEUR HORMONAL NUCLEAIRE

(84) Designated Contracting States:
**AT BE CH CY DE DK ES FI FR GB GR IE IT LI LU MC NL PT SE TR**
Designated Extension States:
**AL LT LV MK RO SI**

(30) Priority: **19.09.2000 US 233519 P**
**18.04.2001 US 284730 P**
**18.04.2001 US 284438 P**

(43) Date of publication of application:
**18.06.2003 Bulletin 2003/25**

(73) Proprietor: **Bristol-Myers Squibb Company**
**Princeton, NJ 08543-4000 (US)**

(72) Inventors:
- **SALVATI, Mark, E.**
  **Lawrenceville, NJ 08648 (US)**
- **BALOG, James, Aaron**
  **Scotch Plains, NJ 07076 (US)**
- **PICKERING, Dacia, A.**
  **Lawrenceville, NJ 08648-3844 (US)**
- **GIESE, Sören**
  **New Hope, PA 18938 (US)**
- **FURA, Aberra**
  **Lawrencville, NJ 08648 (US)**
- **LI, Wenying**
  **Middletown, CT 06457 (US)**
- **PATEL, Ramesh, N.**
  **Bridgewater, NJ 08807 (US)**
- **HANSON, Ronald, L.**
  **Morris Plains, NJ 07950 (US)**

(74) Representative: **Vossius & Partner**
**Siebertstrasse 4**
**81675 München (DE)**

(56) References cited:
**EP-A- 0 678 507**
**WO-A-01/19831**
**WO-A-02/00617**
**WO-A-96/19458**
**WO-A-97/49709**
**WO-A-98/49555**
**WO-A-99/27365**

- **XU,B.: "Pharmacology of some natural products of China" TRENDS IN PHARMACOLOGICAL SCIENCE, vol. 2, October 1981 (1981-10), pages 271-2272, XP002188686 AMSTERDAM**
- **WALTER,W.G.: "Antitumor Imide Derivatives of 7-Oxabicyclo[2.2.1]heptane-2,3-di- methyl-2,3-dicarboxylic Acid " J.PHARM.SCI., vol. 78, no. 1, January 1989 (1989-01), pages 66-67, XP002188687**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **EVANS R M: "THE STERIOD AND THYRIOD HORMONE RECEPTOR SUPERFAMILY" SCIENCE, AMERICAN ASSOCIATION FOR THE ADVANCEMENT OF SCIENCE, US, vol. 240, no. 4854, 13 May 1988 (1988-05-13), pages 889-895, XP000574469 ISSN: 0036-8075 cited in the application**
- **DENISON,M.S. ET AL.: "Xenobiotic-inducible Transcription of Cytochrome P450 Genes" J.BIOL.CHEM., vol. 270, no. 31, 4 August 1995 (1995-08-04), pages 18175-18178, XP002188273 ROCKVILLE PIKE**
- **WALLER,C.W. ET AL.: "Three-Dimensional Quantitative Structure-Activity Relationships for androgen Receptro Ligands" TOXICOL.APPL.PHARMACOL., vol. 137, no. 2, April 1996 (1996-04), pages 219-227, XP002188688 NEW YORK**

## Description

This application claims priority from U.S. Application Serial No. 60/233,519, filed September 19,2000, from U.S. Application Serial No. 60/284,730, filed April 18, 2001, and from U.S. Application Serial No. 60/284,438, filed April 18, 2001, which provisional applications are incorporated herein by reference in their entirety.

The present invention relates to fused cyclic compounds, to methods of preparing such compounds to pharmaceutical compositions containing such compounds, and to the use of such compounds for the preparation of a medicament for modulating the function of a nuclear hormone receptor or for treating contains conditions or disorders such as cancer.

Nuclear hormone receptors (NHR's) constitute a large super-family of ligand-dependent and sequence-specific transcription factors. Members of this family influence transcription either directly, through specific binding to the promoter target genes (Evans, in *Science* **240:** 889-895 (1988)), or indirectly, via protein-protein interactions with other transcription factors (Jonat et al., *Cell* **62:** 1189-1204 (1990), Schuele et al., *Cell* **62:** 1217-1226 (1990), and Yang-Yen et al., *Cell* **62:** 1205-1215 (1990)). The nuclear hormone receptor super-family (also known in the art as the "steroid/thyroid hormone receptor super-family") includes receptors for a variety of hydrophobic ligands, including cortisol, aldosterone, estrogen, progesterone, testosterone, vitamine $D_3$, thyroid hormone and retinoic acid (Evans, 1988, supra). In addition to these conventional nuclear hormone receptors, the super-family contains a number of proteins that have no known ligands, termed orphan nuclear hormone receptors (Mangelsdorf et al., *Cell* **83**: 835-839 (1995), O'Malley et al., *Mol. Endocrinol.* **10:** 1293 (1996), Enmark et al., *Mol. Endocrinol.* **10,** 1293-1307 (1996) and Giguere, *Endocrin. Rev.* **20**, 689-725 (1999)). The conventional nuclear hormone receptors are generally transactivators in the presence of ligand, and can either be active repressors or transcriptionally inert in the absence ofligand. Some of the orphan receptors behave as if they are transcriptionally inert in the absence of ligand. Others, however, behave as either constitutive activators or repressors. These orphan nuclear hormone receptors are either under the control of ubiquitous ligands that have not been identified, or do not need to bind ligand to exert these activities.

In common with other transcription factors, the nuclear hormone receptors have a modular structure, being comprised of three distinct domains: an N-terminal domain of variable size containing a transcriptional activation function AF-1, a highly conserved DNA binding domain and a moderately conserved ligand-binding domain. The ligand-binding domain is not only responsible for binding the specific ligand but also contains a transcriptional activation function called AF-2 and a dimerisation domain (Wurtz et al., *Nature Struc. Biol.* **3**, 87-94 (1996), Parker et al., *Nature Struc. Biol.* **3**, 113-115 (1996) and Kumar et al., *Steroids* **64**, 310-319 (1999)). Although the overall protein sequence of these receptors can vary significantly, all share both a common structural arrangement indicative of divergence from an ancestral archetype, and substantial homology (especially, sequence identity) at the ligand-binding domain.

The steroid binding nuclear hormone receptors (SB-NHR's) comprise a sub-family of nuclear hormone receptors. These receptors are related in that they share a stronger sequence homology to one another, particularly in the ligand binding domain (LBD), than to the other members of the NHR super-faanily (Evans, 1988, supra) and they all utilize steroid based ligands. Some examples of this sub-family ofNHR's are the androgen receptor (AR), the estrogen receptor (ER), the progesterone receptor (PR), the glucocorticoid receptor (GR), the mineralocorticoid receptor (MR), the aldosterone receptor (ALDR) and the steroid and xenobiotic receptor (SXR) (Evans *et al.*, WO 99/35246). Based on the strong sequence homology in the LBD, several orphan receptors may also be members of the SB-NHR sub-family.

Consistent with the high sequence homology found in the LBD for each of the SB-NHR's, the natural ligands for each are derived from a common steroid core. Examples of some of the steroid based ligands utilized by members of the SB-NHR's include cortisol, aldosterone, estrogen, progesterone, testosterone and dihydrotestosterone. Specificity of a particular steroid based ligand for one SB-NHR versus another is obtained by differential substitution about the steroid core. High affinity binding to a particular SB-NHR, coupled with high level specificity for that particular SB-NHR, can be achieved with only minor structural changes about the steroid core (e.g., Waller et al., *Toxicol. Appl. Pharmacol.* **137**, 219-227 (1996) and Mekenyan et al., *Environ. Sci. Technol.* **31,** 3702-3711 (1997), binding affinity for progesterone towards the androgen receptor as compared to testosterone).

Numerous synthetically derived steroidal and non-steroidal agonists and antagonists have been described for the members of the SB-NHR family. Many of these agonist and antagonist ligands are used clinically in man to treat a variety of medical conditions. RU486 is an example of a synthetic agonist of the PR, which is utilized as a birth control agent (Vegeto et al., *Cell* **69:** 703-713 (1992)); and Flutamide is an example of an antagonist of the AR, which is utilized for the treatment of prostate cancer (Neri et al., *Endo.* **91**, 427-437 (1972)). Tamoxifen is an example of a tissues specific modulator of the ER furiction, that is used in the treatment of breast cancer (Smigel, *J. Natl. Cancer Inst.* **90**, 647-648 (1998)). Tamoxifen can function as an antagonist of the ER in breast tissue while acting as an agonist of the ER in bone (Grese et al., *Proc. Natl. Acad. Sci. USA* **94,** 14105-14110 (1997)). Because of the tissue selective effects seen for Tamoxifen, this agent and agents like it are referred to as "partial-agonists" or "partial-antagonists". In addition to synthetically derived non-endogenous ligands, non-endogenous ligands for NHR's can be obtained from food sources (Regal et al., *Proc. Soc. Exp. Biol. Med.* **223,** 372-378 (2000) and Henzpstock et al., *J. Med. Food* **2,** 267-269 (1999)).

The flavanoid phytoestrogens are an example of an unnatural ligand for SB-NHR's that are readily obtained from a food source such as soy (Quella et al., *J. Clin. Oncol.* **18,** 1068-1074 (2000) and Banz et al., *J. Med. Food* **2,** 271-273 (1999)). The ability to modulate the transcriptional activity of individual NHR by the addition of a small molecule ligand, makes them ideal targets for the development of pharmaceutical agents for a variety of disease states.

As mentioned above, non-natural ligands can be synthetically engineered to serve as modulators of the function of NHR's. In the case of SB-NHR's, engineering of an unnatural ligand can include the identification of a core structure which mimics the natural steroid core system. This can be achieved by random screening against several SB-NHR's or through directed approaches using the available crystal structures of a variety of NHR ligand binding domains (Bourguet et al., *Nature* **375,** 377-382 (1995), Brzozowski, et al., *Nature* **389**, 753-758 (1997), Shiau et al., *Cell* **95**, 927-937 (1998) and Tanenbaum et al., *Proc. Natl. Acad Sci. USA* **95**, 5998-6003 (1998)). Differential substitution about such a steroid mimic core can provide agents with selectivity for one receptor versus another. In addition, such modifications can be employed to obtain agents with agonist or antagonist activity for a particular SB-NHR. Differential substitution about the steroid mimic core can result in the formation of a series of high affinity agonists and antagonists with specificity for, for example, ER versus PR versus AR versus GR versus MR. Such an approach of differential substitution has been reported, for example, for quinoline based modulators of steroid NHR's in *J. Med. Chem.,* **41**, 623 (1999); WO 9749709; US 5696133; US 5696130; US 5696127; US 5693647; US 5693646; US 5688810; US 5688808 and WO 9619458, all incorporated herein by reference.

The compounds of the present invention comprise a core which serves as a steroid mimic, and are useful as modulators of the function of steroid binding nuclear hormone receptors, as well as other NHR's as described following.

The present invention provides fused cyclic compounds of the following formula I, salts and hydrates thereof, which compounds are especially useful as modulators of nuclear hormone receptor function:

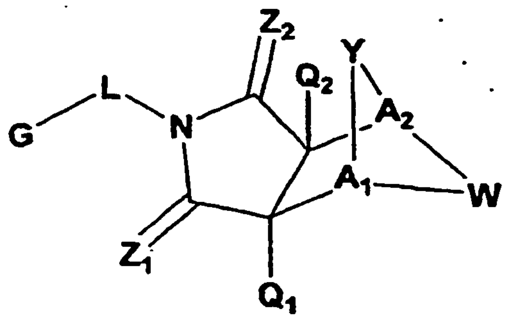

(I)

As used in formula I, and throughout the specification, the symbols have the following meanings unless otherwise indicated, and are, for each occurrence, independently selected:

G is an aryl (especially, phenyl or naphthyl) or heterocyclo (especially those heterocyclo groups G of the compounds of the Examples herein) group, where said group is mono- or polycyclic, and which is optionally substituted at one or more positions, preferably with substituents as exemplified in any of the compounds of the Examples herein;

L is a bond, $(CR^7R^7)_n$, (where n is 1 and $R^7$ and $R^{7'}$ are each independently H, alkyl or substituted alkyl), or $-CH_2$-NH-;

$A_1$ and $A_2$ are each independently $CR^7$ where $R^7$ (i) is hydrogen, alkyl or substituted alkyl, arylalkyl or substituted arylalkyl, alkenyl or substituted alkenyl (for example, alkenyl substituted with aryl (especially, phenyl or naphthyl) or substituted aryl, or alkenyl substituted with heterocyclo or substituted heterocyclo), aryl or substituted aryl, heterocyclo or substituted heterocyclo, heterocycloalkyl or substituted heterocycloalkyl, where, for each substituents are one or mope groups selected from $V^1$ (especially $A_1$ and $A_2$ groups of the formula $CR^7$ where $R^7$ for each of $A_1$ and/or $A_2$ is independently selected from $C_{1-4}$ alkyl which alkyl is substituted by one or more groups $V^1$), or (ii) forms, together with $R^7$ of a group W (especially where W is $CR^7R^{7'}$-$CR^7R^{7'}$), a heterocyclic ring;

$V^1$ is OH, CN, halo, -O-aryl, -O-substituted aryl, -O-heterocyclo, -O-substituted heterocyclo, -O-CO-alkyl, -O-CO-substituted alkyl, -O-(alkylsilyl), -O-arylalkyl, -O-substituted arylalkyl, -O-CO-arylalkyl, -O-CO-substituted arylalkyl, -O-CO-aryl, -O-CO-substituted aryl, -O-CO-heterocyclo, -O-CO-substituted heterocyclo, -S-(optionally substituted aryl)-NH-CO-(optionally substituted alkyl), -SO-(optionally substituted aryl)-NH-CO-(optionally substituted alkyl), $-SO_2$-(optionally substituted aryl)-NH-CO-(optionally substituted alkyl), $-NH-SO_2$-aryl, $-NH-SO_2$-substituted aryl,

-NH-CO-O-(optionally substituted arylalkyl), -NH-CO-O-alkyl, -NH-CO-O-substituted alkyl, -NH-CO-alkyl, -NH-CO-substituted alkyl, -NH-CO-aryl, -NH-CO-substituted aryl, -NH-CO-(optionally substituted arylalkyl), -NH-CO-(optionally substituted alkyl)-O-(optionally substituted aryl), -N(optionally substituted alkyl)(optionally substituted aryl), -N(optionally substituted alkyl)(optionally substituted arylalkyl), -COH, -COOH, -COO-alkyl, -CO-O-substituted alkyl, -CO-O-optionally substituted arylalkyl, -CO-aryl, -CO-substituted aryl, -O-CO-NH-aryl, -O-CO-NH-substituted aryl, -CO-NH-aryl, -CO-NH-substituted aryl, -CO-NH-arylalkyl, -CO-NH-substituted arylalkyl, or -O-(optionally substituted aryl)-NH-CO-(optionally substituted alkyl);

Y is -O-, -SO-, -N($V^2$)-, -$CH_2$-N($V^2$)-, -CO-N(alkyl)-, -$CH_2$-S-, or -$CH_2$-$SO_2$-;

$V^2$ is hydrogen, alkyl, arylalkyl, -CO-alkyl, -CO-O-aryl, or -CO-O-arylalkyl;

$Z_1$ and $Z_2$ are O;

$Q_1$ and $Q_2$ are H;

W is $CR^7R^{7'}$-$CR^7R^{7'}$, $CR^7R^{7'}$-C=O, $NR^9$ -$CR^7R^{7'}$, N=$CR^8$, N=N, $NR^9$-$NR^{9'}$, cycloalkyl or substituted cycloalkyl, cycloalkenyl or substituted cycloalkenyl, heterocyclo or substituted heterocyclo, or aryl or substituted aryl, wherein, when W is not $NR^9$-$CR^7R^{7'}$, N=$CR^8$, N=N, $NR^9$-$NR^{9'}$, or heterocyclo or substituted heterocyclo, then Y must be -O-, -$CH_2$S-, -SO-, -$CH_2$-$SO_2$-, -N($V^2$)- or -($H_2$-N ($V^2$)-;
$R^1$ and $R^{1'}$ are each mdependently H, alkyl or substituted alkyl, cycloalkyl or substituted cycloalkyl, cycloalkenyl or substituted cycloalkenyl, heterocyclo or substituted heterocyclo, cycloalkylalkyl or substituted cycloallcyalkyl, cycloalkenylalkyl or substituted cycloalkenylalkyl, heterocycloalkyl or substituted heterocycloalkyl, aryl or substituted aryl, arylallcyl or substituted arylalkyl;
$R^2$ is alkyl or substituted alkyl, cycloalkyl or substituted cycloalkyl, cycloalkenyl or substituted cycloalkenyl, heterocyclo or substituted heterocyclo, cycloalkylalkyl or substituted cycloalkylallcyl, cycloalkenylalkyl or substituted cycloalkenylalkyl, heterocycloalkyl or substituted heterocycloalkyl, aryl or - substituted aryl, arylalkyl or substituted arylalkyl;
$R^4$ is H, alkyl or substituted alkyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, beterocycloalkyl or substituted heterocycloalkyl, aryl or substituted aryl, arylalkyl or substituted arylalkyl, $R^1$C=O, $R^1$NHC=O, $SO_2OR^1$, or $SO_2NR^1R^{1'}$;
$R^5$ is alkyl or substituted alkyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, $R^1$C=O, $R^1$NHC=O, $SO_2R^1$, $SO_2OR^1$, or $SO_2NR^1R^{1'}$;
$R^7$ and $R^{7'}$ are each independently H, alkyl or substituted alkyl, alkenyl or substituted alkenyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, halo, CN, $OR^1$, nitro, hydroxylamine, hydroxylamide, amino, $NHR^4$, $NR^2R^5$, $NOR^1$, thiol, alkylthio or substituted alkylthio, $R^1$C=O, $R^1$OC=O, $R^1$NHC=O, $SO_2R^1$, $SOR^1$, $PO_3R^1R^{1'}$, $R^1R^{1'}$NC=O, C=$OSR^1$, $SO_2R^1$, $SO_2OR^1$, or $SO_2NR^1R^{1'}$, or, wherein $A_1$ or $A_2$ contains a group $R^7$ and W contains a group $R^7$, said $R^7$ groups of $A_1$ or $A_2$ and W together form a heterocyclic ring;
$R^8$ is H, alkyl or substituted alkyl, alkenyl or substituted alkenyl, cycloalkyl or substituted cycloalkyl, cycloalkenyl or substituted cycloalkenyl, heterocyclo or substituted hetetocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, nitro, halo, CN, $OR^1$, amino, $NHR^4$, $NR^2R^5$, $NOR^1$, alkylthio or substituted alkylthio, C=$OSR^1$, $R^1$OC=O, $R^1$C=O, $R^1$NHC=O, $R^1R^{1'}$NC=O, $SO_2OR^1$, S=$OR^1$, $SO_2R^1$, $PO_3R^1R^{1'}$, or $SO_2NR^1R^{1'}$; and
$R^9$ and $R^{9'}$ are each independently H, alkyl or substituted alkyl, alkenyl or substituted alkenyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, CN, OH, $OR^1$, $R^1$C=O, $R^1$OC=O, $R^1$NHC=O, $SO_2R^1$, $SO_2OR^1$, or $SO_2NR^1R^{1'}$;

with the provisos that:

(1) when Y is -O-, $Q_1$ and $Q_2$ are hydrogen, $Z_1$ and $Z_2$ are O, W is -$CH_2$-$CH_2$-, and one of $A_1$ and $A_2$ is CH and the

other is $CR^7$, then G-L is not unsubstituted Phenyl;

(2) when Y is -O-, $Q_1$ and $Q_2$ are hydrogen, $Z_1$ and $Z_2$ are O, W is $-CH_2-CH_2-$, and one of $A_1$ and $A_2$ is CH and the other is $C-CH_3$, then G-L is not phenyl substituted with chloro and/or methyl;

(3) when Y is -O-, $Q_1$ and $Q_2$ are hydrogen, $Z_1$ and $Z_2$ are O, W is $-CH_2-CH_2-$ and one of $A_1$ and $A_2$ is CH and the other is C-alkyl, then G-L is not N-substituted piperazine-alkyl- or N-substituted imidazolidine-alkyl-;

(4) when Y is -O-; $Q_1$ and $Q_2$ are hydrogen, $Z_1$ and $Z_2$ are O, W is $-CH_2-CH_2-$, and $A_1$ and $A_2$ are $C-CH_3$, then G-L is not thiazole or substituted thiazole;

(5) when Y is - $CH_2$-S-, -SO-, $CH_2-SO_2-$, $-N(V^2)-$ or - $CH_2-N(V^2)$-W is $CR^7R^{7'}$- $CR^7R^{7'}$, and $Z_1$ and $Z_2$ are O, then G-L is not unsubstituted phenyl;

(6) when Y is $NR^7$, W' is unsubstituted or substituted phenyl, and $Q_1$ and $Q_2$ are hydrogen, then these compounds of formula I are not included;

(7) when Y is -O-, $Q_1$ and $Q_2$ are hydrogen, $Z_1$ and $Z_2$ are O, W is ethylene oxide, and $A_1$ and $A_2$ are CH, then G-L is not methylphenyl or chlorophenyl;

(8) the compound of formula I is not 6,10-epithio-4H-thieno-[3',4':5,6]cyclooct[1,2-*f*]isoindole-7,9(5H,8H)dione, 8-(3,5-dichlorophenyl)-6,6a,9a,10,11,12,-hexahydro-1,3,6,10-tetramethyl-2,2,13-trioxide, (6R,6aR,9aS,10S);

(9) when Y is -O-, $Q_1$ and $Q_2$ are hydrogen, $Z_1$ and $Z_2$ are O, W is cyclopentyl, cyclohexyl, 3-phenyl-2-isoxazoline or $CR^7R^{7'}-CR^7R^{7'}$ where $R^7$ and $R^{7'}$ are each independently defined as H and 4-butyrolactone and $R^7$ and $R^{7'}$ are not all simultaneously H, and $A_1$ and $A_2$ are CH, then G-L is not an unsubstituted naphthyl ring or a monosubstituted phenyl ring, where said substituent is methoxy, Br, Cl, $NO_2$, methyl, ethyl, $CH_2$-phenyl, S-phenyl, or O-phenyl;

(10) when Y is -O- and W is $-CH_2-CH_2-$, then at least one of $A_1$ or $A_2$ is not CH;

(11) wherein the compound of formula (I) is not

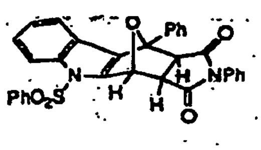
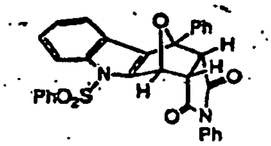

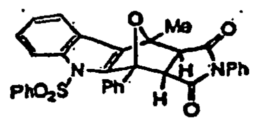
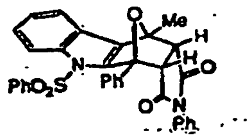

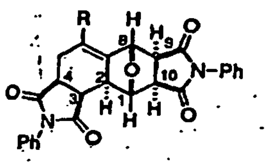

R = TBDMSO
R = Me

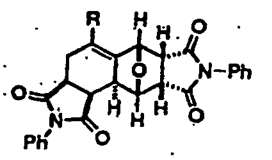

R = TBDMSO
R = Me

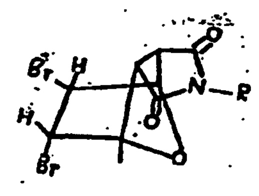

R =$C_6H_4-CH_3$ (2')
R =$C_6H_4-OCH_3$ (2')
R =1-Naphthyl

Preferably, compounds of formula I are monomeric, and are not comprised within other oligomers or polymers.

The compounds of formula I and salts thereof comprise a core which can serve as a steroid mimic (and do not require the presence of a steroid-type (e.g., cyclopentanoperhydrophenanthrene analog) structure).

The present invention further relates to the compounds defined in appended claim 3; the pharmaceutical compositions defined in appended claims 4 and 5; the uses defined in appended claims 7 and 9; and the methods of preparation defined in appended claims 10 and 11.

The following are definitions of terms used in the present specification. The initial definition provided for a group or term herein applies to that group or term throughout the present specification individually or as part of another group, unless otherwise indicated.

The terms "alkyl" and "alk" refer to a straight or branched chain alkane (hydrocarbon) radical containing from

1 to 12 carbon atoms, preferably 1 to 6 carbon atoms. Exemplary such groups include, but are not limited to, methyl, ethyl, propyl, isopropyl, n-butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, and the like. "Substituted alkyl" refers to an alkyl group substituted with 1 to 4 substituents, at any available point of attachment. Substituents are one or more of the following groups: halo (e.g., a single halo substituent or multiple halo substitutents forming, in the latter case, groups such as a perfluoroalkyl group or an alkyl group bearing (Cl, or $CF_3$), alkoxy, alkylthio, hydroxy, carboxy (i.e., -COOH), alkoxycarbanyl, alkylcarbonyloxy, amino (i.e., $-NH_2$), carbamoyl or substituted carbamoyl, carbamate or substituted carbamate, urea or substituted urea, amidinyl or substituted amidinyl, thiol (i.e., -SH), aryl, heterocycle, cycloalkyl, heterocycloalkyl, -S-aryl, -S-heterocycle, -S=O-aryl, -S=O-heterocycle, arylalkyl-O-, $-S(O)_2$-aryl, $-S(O)_2$-heterocycle, $-NHS(O)_2$-aryl, $-NHS(O)_2$-heterocycle, $-NHS(O)_2NH$-aryl, $-NHS(O)_2NH$-heterocycle, $-P(O)_2$-aryl, $-P(O)_2$-heterocycle, $-NHP(O)_2$-aryl, $-NHP(O)_2$-heterocycle, $-NHP(O)_2NH$-aryl, $-NHP(O)_2NH$-heterocycle, -O-aryl, -O-heterocycle, -NH-aryl, -NH-heterocycle, -NHC=O-aryl, -NHC=O-alkyl, -NHC=O-heterocycle, -OC=O-aryl, -OC=O-heterocycle, -NHC=ONH-aryl, -NHC=ONH-heterocycle, -OC=OO-aryl, -OC=OO-heterocycle, -OC=ONH-aryl, -OC=ONH-heterocycle, -NHC=OO-aryl, -NHC=OO-heterocycle, -NHC=OO-alkyl, -C=ONH-aryl, -C=ONH-heterocycle, -C=OO-aryl, -C=OO-heterocycle, -N(alkyl)$S(O)_2$-aryl, -N(alkyl)$S(O)_2$-heterocycle, -N(alkyl)$S(O)_2NH$-aryl, -N(alkyl)$S(O)_2NH$-heterocycle, -N(alkyl)$P(O)_2$-aryl, -N(alkyl)$P(O)_2$-heterocycle, -N(alkyl)$P(O)_2NH$-aryl, -N(alkyl)$P(O)_2NH$-heterocycle, -N(alkyl)-aryl, -N(alkyl)-heterocycle, -N(alkyl)C=O-aryl, -N(alkyl)C=O-heterocycle, -N(alkyl)C=ONH-aryl, -N(alkyl)C=ONH-heterocycle, -OC=ON(alkyl)-aryl, -OC=ON(alkyl)-heterocycle, -N(alkyl)C=OO-aryl, -N(alkyl)C=OO-heterocycle, -C=ON(alkyl)-aryl, -C=ON(alkyl)-heterocycle, $-NHS(O)_2N$(alkyl)-aryl, $-NHS(O)_2N$(alkyl)-heterocycle, $-NHP(O)_2N$(alkyl)-aryl, $NHP(O)_2N$(alkyl)-heterocycle, -NHC=ON(alkyl)-aryl, -NHC=ON(alkyl)-heterocycle, -N(alkyl)$S(O)_2N$(alkyl)-aryl, -N(alkyl)$S(O)_2$(alkyl)-heterocycle, -N(alkyl)$P(O)_2N$(alkyl)-aryl, -N(alkyl)$P(O)_2N$(alkyl)-heterocycle, -N(alkyl)C=ON(alkyl)-aryl, and -N(alkyl)C=ON(alkyl)-heterocycle. In the aforementioned substitutents, in each instance, groups such as "alkyl", "aryl" and "heterocycle" can themselves be optionally substituted; for example, "alkyl" in the group "NCH=OO-alkyl" recited above can be optionally substituted so that both "NHC=OO-alkyl" and "NHC=OO-substituted alkyl" are exemplary substitutents. Alkyl substituents also include groups such as "T" and "T-$R^{12}$" (wherein T is defined as a nitrogen, oxygen or sulfur-containing group and $R^{12}$ has the same definition as $R^7$ defined earlier), especially for substituted alkyl groups within $A_1$ or $A_2$.

The term "alkenyl" refers to a straight or branched chain hydrocarbon radical containing from 2 to 12 carbon atoms and at least one carbon-carbon double bond. Exemplary such groups include ethenyl or allyl. "Substituted alkenyl" refers to an alkenyl group substituted with 1 to 4 substituents, at any available point of attachment. The substituents are selected from alkyl or substituted alkyl, as well as those groups recited above as alkyl substituents.

The term "alkynyl" refers to a straight or branched chain hydrocarbon radical containing from 2 to 12 carbon atoms and at least one carbon to carbon triple bond. Exemplary such groups include ethynyl. "Substituted alkynyl" refers to an alkynyl group substituted with one or more substituents, preferably 1 to 4 substituents, at any available point of attachment. Exemplary substituents include, but are not limited to, alkyl or substituted alkyl, as well as those groups recited above as exemplary alkyl substituents.

The term "cycloalkyl" refers to a fully saturated cyclic hydrocarbon group containing from 1 to 4 rings and 3 to 8 carbons per ring. Exemplary such groups include cyclopropyl, cyclobutyl, cyclopentyl, cyclohexyl, etc. "Substituted cycloalkyl" refers to a cycloalkyl group substituted with 1 to 4 substituents, at any available point of attachment. The substituents are selected from nitro, cyano, alkyl or substituted alkyl, as well as those groups recited above as alkyl substituents, and hydrogen, alkyl or substituted alkyl, alkenyl or substituted alkenyl, alkynyl or substituted alkynyl, halo, cycloalkyl or substituted cycloalkyl, cycloalkenyl or substituted cycloalkenyl, aryl or substituted aryl, heterocyclo or substituted heterocyclo, arylalkyl or substituted arylalkyl, heterocycloalkyl or substituted heterocycloalkyl, CN, $R^1OC$=O, $R^1C$=O, $R^1C$=S, $R^1HNC$=O, $R^1R^2NC$=O, $HOCR^3R^{3'}$, nitro, $R^1OCH_2$, $R^1O$, $NH_2$, $NR^4R^5$, $SR^1$, S=$OR^1$, $SO_2R^1$, $SO_2OR^1$, $SO_2NR^1R^{1'}$, $(R^1O)(R^{1'}O)P$=O, oxo, $(R^1)(R^{1'})P$=O, $(R^{1'})(NHR^1)P$=O. The substituents also include spiro-attached or fused cyclic substituents, especially cycloalkenyl or substituted cycloalkenyl.

The term "cycloalkenyl" refers to a partially unsaturated cyclic hydrocarbon group containing 1 to 4 rings and 3 to 8 carbons per ring. Exemplary such groups include cyclobutenyl, cyclopentenyl, cyclohexenyl, etc. "Substituted cycloalkenyl" refers to a cycloalkenyl group substituted with 1 to 4 substituents, at any available point of attachment. The substituents are selected from nitro, cyano, alkyl or substituted alkyl, as well as those groups recited above as alkyl substituents, and hydrogen, alkyl or substituted alkyl, alkenyl or substituted alkenyl, alkynyl or substituted alkynyl, halo, cycloalkyl or substituted cycloalkyl, cycloalkenyl or substituted cycloalkenyl, aryl or substituted aryl, heterocyclo or substituted heterocyclo, arylalkyl or substituted arylalkyl, heterocycloalkyl or substituted heterocycloalkyl, CN, $R^1OC$=O, $R^1C$=O, $R^1C$=S, $R^1HNC$=O, $R^1R^2NC$=O, $HOCR^3R^{3'}$, nitro, $R^1OCH_2$, $R^1O$, $NH_2$, $NR^4R^5$, $SR^1$, S=$OR^1$, $SO_2R^1$, $SO_2OR^1$, $SO_2NR^1R^{1'}$, $(R^1O)(R^{1'}O)P$=O, oxo, $(R^1)(R^{1'})P$=O, $(R^{1'})(NHR^1)P$=O, and spiro-attached or fused, cycloalkyl or substituted cycloalkyl.

The terms "alkoxy" or "alkylthio" refer to an alkyl group as described above bonded through an oxygen linkage (-O-) or a sulfur linkage (-S-), respectively. The terms "substituted alkoxy" or "substituted alkylthio" refer to a substituted alkyl group as described above bonded through an oxygen or sulfur linkage, respectively.

The term "alkoxycarbonyl" refers to an alkoxy group bonded through a carbonyl group.

The term "alkylcarbonyl" refers to an alkyl group bonded through a carbonyl group. The term "alkylcarbonyloxy" refers to an alkylcarbonyl group bonded through an oxygen linkage.

The terms "arylalkyl", "substituted arylalkyl", "cycloalkylalkyl", "substituted cycloalkylalkyl", "cycloalkenylalkyl", "substituted cycloalkenylalkyl", "heterocycloalkyl" and "substituted heterocycloalkyl" refer to aryl, cycloalkyl, cycloalkenyl and heterocyclo groups bonded through an alkyl group, substituted on the aryl, cycloalkyl, cycloalkenyl or heterocyclo and/or the alkyl group where indicated as "substituted".

The term "aryl" refers to cyclic, aromatic hydrocarbon groups which have 1 to 5 aromatic rings, especially monocyclic or bicyclic groups such as phenyl, biphenyl or , naphthyl. Where containing two or more aromatic rings (bicyclic, etc.), the aromatic rings of the aryl group may be joined at a single point (e.g., biphenyl), or fused (e.g., naphthyl, phenanthrenyl and the like). "Substituted aryl" refers to an aryl group substituted by 1,2,3,4 or 5 substituents, at any point of attachment. The substituents are selected from nitro, cycloalkyl or substituted cycloalkyl, cycloalkenyl or substituted cycloalkenyl, cyano, alkyl-$S(O)_m$- (m=0, 1 or 2), alkyl or substituted alkyl, as well as those groups recited above as alkyl substituents and hydrogen, alkyl or substituted alkyl, alkenyl or substituted alkenyl, alkynyl or substituted alkynyl, halo, cycloalkyl or substituted cycloalkyl, cycloalkenyl or substituted cycloalkenyl, aryl or substituted aryl, heterocyclo or substituted heterocyclo, arylalkyl or substituted arylalkyl, heterocycloalkyl or substituted heterocycloalkyl, CN, $R^1OC=O$, $R^1C=O$, $R^1C=S$, $R^1HNC=O$, $R^1R^2NC=O$, $HOCR^3R^{3'}$, nitro, $R^1OCH_2$, $R^1O$, $NH_2$, $NR^4R^5$, $SR^1$, $S=OR^1$, $SO_2R^1$, $SO_2OR^1$, $SO_2NR^1R^{1'}$, $(R^1O)(R^{1'}O)P=O$, oxo, $(R^1)(R^{1'})P=O$, $(R^{1'})(NHR^1)P=O$, and fused heterocyclo or cycloalkenyl or substituted heterocyclo or cycloalkenyl, groups (e.g., thereby forming a fluoroenyl, tetrahydronapthalenyl, or dihydroindenyl group).

"Carbamoyl" refers to the group -CONH- which is bonded on one end to the remainder of the molecule and on the other to hydrogen or an organic moiety (such as alkyl, substituted alkyl, aryl, substituted aryl, heterocycle, alkylcarbonyl, hydroxyl and substituted nitrogen). "Carbamate" refers to the group -O-CO-NH- which is bonded on one end to the remainder of the molecule and on the other to hydrogen or an organic moiety (such as those listed above). "Urea" refers to the group -NH-CO-NH- which is bonded on one end to the remainder of the molecule and on the other to hydrogen or an organic moiety (such as those listed above). "Amidinyl" refers to the group $-C(=NH)(NH_2)$. "Substituted carbamoyl", "substituted carbamate", "substituted urea" and "substituted amidinyl" refer to carbamoyl, carbamate, urea or amidinyl groups as described above in which one or more of the hydrogen groups are replaced by an organic moiety listed above.

The terms "heterocycle", heterocyclic" and "heterocyclo" refer to fully saturated, or partially or fully unsaturated, including aromatic (i.e., "heteroaryl") cyclic groups (3 to 7 membered monocyclic, 7 to 11 membered bicyclic, or 10 to 16 membered tricyclic ring systems) which have at least one heteroatom in at least one carbon atom-containing ring. Each ring of the heterocyclic group containing a heteroatom may have 1, 2, 3, or 4 heteroatoms selected from nitrogen atoms, oxygen atoms and/or sulfur atoms, where the nitrogen and sulfur heteroatoms may optionally be oxidized and the nitrogen heteroatoms may optionally be quaternized. (The term "heteroarylium" refers to a heteroaryl group bearing a quaternary nitrogen atom and thus a positive charge.) The heterocyclic group may be attached to the remainder of the molecule at any heteroatom or carbon atom of the ring or ring system. Exemplary monocyclic heterocyclic groups include ethylene oxide, azetidinyl, pyrrolidinyl, pyrrolyl, pyrazolyl, oxetanyl, pyrazolinyl, imidazolyl, imidazolinyl, imidazolidinyl, oxazolyl, oxazolidinyl, isoxazolinyl, isoxazolyl, thiazolyl, thiadiazolyl, thiazolidinyl, isothiazolyl, isothiazolidinyl, furyl, tetrahydrofuryl, thienyl, oxadiazolyl, piperidinyl, piperazinyl, 2-oxopiperazinyl, 2-oxopiperidinyl, 2-oxopyrrolodinyl, 2-oxoazepinyl, azepinyl, hexahydrodiazepinyl, 4-piperidonyl, pyridyl, pyrazinyl, pyrimidinyl, pyridazinyl, triazinyl, triazolyl, tetrazolyl, tetrahydropyranyl, morpholinyl, thiamorpholinyl, thiamorpholinyl sulfoxide, thiamorpholinyl sulfone, 1,3-dioxolane and tetrahydro-1,1-dioxothienyl, and the like. Exemplary bicyclic heterocyclic groups include indolyl, isoindolyl, benzothiazolyl, benzodioxolyl, benzoxazolyl, benzoxadiazolyl, benzothienyl, quinuclidinyl, quinolinyl, tetrahydroisoquinolinyl, isoquinolinyl, benzimidazolyl, benzopyranyl, indolizinyl, benzofuryl, benzofurazanyl, chromonyl, coumarinyl, benzopyranyl, cinnolinyl, quinoxalinyl, indazolyl, pyrrolopyridyl, furopyridinyl (such as furo[2,3-c]pyridinyl, furo[3, 2-b]pyridinyl] or furo[2,3-b]pyridinyl), dihydrobenzodioxinyl, dihydrodioxidobenzothiophenyl, dihydroisoindolyl, dihydroindolyl, dihydroquinolinyl, dihydroquinazolinyl (such as 3,4-dihydro-4-oxo-quinazolinyl), triazinylazepinyl, tetrahydroquinolinyl and the like. Exemplary tricyclic heterocyclic groups include carbazolyl, benzidolyl, phenanthrolinyl, dibenzofuranyl, acridinyl, phenanthridinyl, xanthenyl and the like.

"Substituted heterocycle", "substituted heterocyclic", and "substituted heterocyclo" (such as "substituted heteroaryl") refer to heterocycle, heterocyclic or heterocyclo groups substituted with 1 to 4 substituents, at any available point of attachment. The substituents are selected from cycloalkyl or substituted cycloalkyl, cycloalkenyl or substituted cycloalkenyl, nitro, oxo (i.e., = O); cyano, alkyl-$S(O)_m$-(m = 0, 1 or 2), alkyl or substituted alkyl, as well as those groups recited above as alkyl substituents, and hydrogen, alkyl or substituted alkyl, alkenyl or substituted alkenyl, alkynyl or substituted alkynyl, halo, cycloalkyl or substituted cycloalkyl, cycloalkenyl or substituted cycloalkenyl, aryl or substituted aryl, heterocyclo or substituted heterocyclo, arylalkyl or substituted arylalkyl, heterocycloalkyl or substituted heterocycloalkyl, CN, $R^1OC=O$, $R^1C=O$, $R^1C=S$, $R^1HNC=O$, $R^1R^2NC=O$, $HOCR^3R^{3'}$, nitro, $R^1OCH_2$, $R^1O$, $NH_2$, $NR^4R^5$, $SR^1$,

S=$OR^1$, $SO_2R^1$, $SO_2OR^1$, $SO_2NR^1R^{1'}$, $(R^1O)(R^{1'}O)P=O$, oxo, $(R^1)(R^{1'})P=O$, $(R^{1'})(NHR^1)P=O$.

The term "quaternary nitrogen" refers to a tetravalent positively charged nitrogen atom including, for example, the positively charged nitrogen in a tetraalkylammonium group (e.g., tetramethylammonium, N-methylpyridinium), the positively charged nitrogen in protonated ammonium species (e.g., trimethyl-hydroammonium, N-hydropyridinium), the positively charged nitrogen in amine N-oxides (e.g., N-methyl-morpholine-N-oxide, pyridine-N-oxide), and the positively charged nitrogen in an N-amino-ammonium group (e.g., N-aminopyridinium).

The terms "halogen" or "halo" refer to chlorine, bromine, fluorine or iodine.

The terms "hydroxylamine" and "hydroxylamide" refer to the groups OH-NH-and OH-NH-CO-, respectively.

When a functional group is termed "protected", this means that the group is in modified form to mitigate, especially preclude, undesired side reactions at the protected site. Suitable protecting groups for the methods and compounds described herein include, without limitation, those described in standard textbooks, such as Greene, T. W. et al., *Protective Groups in Organic Synthesis*, Wiley, N.Y. (1991).

When a term such as "$(CRR)_n$" is used, it denotes an optionally substituted alkyl chain existing between the two fragments to which it is bonded, the length of which chain is defined by the range described for the term n. An example of this is n=0-3, implying from zero to three (CRR) units existing between the two fragments, which are attached to the primary and terminal (CRR) units. In the situation where the term n is set to zero (n = 0) then a bond exists between the two fragments attached to (CRR).

Unless otherwise indicated, any heteroatom with unsatisfied valences is assumed to have hydrogen atoms sufficient to satisfy the valences.

Divalent groups, such as those in the definition ofW (e.g., $NR^9$-$CR^7R^{7'}$), may be bonded in either direction to the remainder of the molecule (e.g,

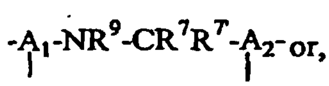 or, 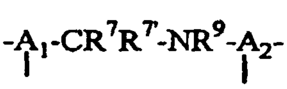

for the aforementioned group within the definition of W).

Carboxylate anion refers to a negatively charged group $-COO^-$.

The compounds of formula I form salts which are also within the scope of this invention. Reference to a compound of the formula I herein is understood to include reference to salts thereof, unless otherwise indicated. The term "salt(s)", as employed herein, denotes acidic and/or basic salts formed with inorganic and/or organic acids and bases. In addition, when a compound of formula I contains both a basic moiety, such as but not limited to a pyridine or imidazole, and an acidic moiety such as but not limited to a carboxylic acid, zwitterions ("inner salts") may be formed and are included within the term "salt(s)" as used herein. Pharmaceutically acceptable (i.e., non-toxic, physiologically acceptable) salts are preferred, although other salts are also useful, e.g., in isolation or purification steps which may be employed during preparation. Salts of the compounds of the formula I may be formed, for example, by reacting a compound I with an amount of acid or base, such as an equivalent amount, in a medium such as one in which the salt precipitates or in an aqueous medium followed by lyophilization.

The compounds of formula I which contain a basic moiety, such as but not limited to an amine or a pyridine or imidazole ring, may form salts with a variety of organic and inorganic acids. Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, hydroxyethanesulfonates (e.g., 2-hydroxyethanesulfonates), lactates, maleates, methanesulfonates, naphthalenesulfonates (e.g., 2-naphthalenesulfonates), nicotinates, nitrates, oxalates, pectinates, persulfates, phenylpropionates (e.g., 3-phenylpropionates), phosphates, picrates, pivalates, propionates, salicylates, succinates, sulfates (such as those formed with sulfuric acid), sulfonates (such as those mentioned herein), tartrates, thiocyanates, toluenesulfonates such as tosylates, undecanoates, and the like.

The compounds of formula I which contain an acidic moiety, such but not limited to a carboxylic acid, may form salts with a variety of organic and inorganic bases. Exemplary basic salts include ammonium salts, alkali metal salts such as sodium, lithium and potassium salts, alkaline earth metal salts such as calcium and magnesium salts, salts with organic bases (for example, organic amines) such as benzathines, dicyclohexylamines, hydrabamines (formed with N, N-bis(dehydroabietyl)ethylenediamine), N-methyl-D-glucamines, N-methyl-D-glycamides, t-butyl amines, and salts with amino acids such as arginine, lysine and the like. Basic nitrogen-containing groups may be quatemized with agents such as lower alkyl halides (e.g. methyl, ethyl, propyl, and butyl chlorides, bromides and iodides), dialkyl sulfates (e.g. dimethyl, diethyl, dibutyl, and diamyl sulfates), long chain halides (e.g. decyl, lauryl, myristyl and stearyl chlorides, bromides and iodides), aralkyl halides (e.g. benzyl and phenethyl bromides), and others.

Prodrugs and solvates of the compounds of the invention are also contemplated herein. The term "prodrug" as employed herein denotes a compound which, upon administration to a subject, undergoes chemical conversion by metabolic or chemical processes to yield a compound of the formula I, or a salt and/or solvate thereof. Solvates of the compounds of formula I include, for example, hydrates.

Compounds of the formula I, and salts thereof, may exist in their tautomeric form (for example, as an amide or imino ether). All such tautomeric forms are contemplated herein as part of the present invention.

All stereoisomers of the present compounds (for example, those which may exist due to asymmetric carbons on various substituents), including enantiomeric forms and diastereomeric forms, are contemplated within the scope of this invention. Individual stereoisomers of the compounds of the invention may, for example, be substantially free of other isomers (e.g., as a pure or substantially pure optical isomer having a specified activity), or may be admixed, for example, as racemates or with all other, or other selected, stereoisomers. The chiral centers of the present invention may have the S or R configuration as defined by the IUPAC 1974 Recommendations. The racemic forms can be resolved by physical methods, such as, for example, fractional crystallization, separation or crystallization of diastereomeric derivatives or separation by chiral column chromatography. The individual optical isomers can be obtained from the racemates by any suitable method, including without limitation, conventional methods, such as, for example, salt formation with an optically active acid followed by crystallization.

All configurational isomers of the compounds of the present invention are contemplated, either in admixture or in pure or substantially pure form. The definition of compounds of the present invention embraces both cis (*Z*) and trans (*E*) alkene isomers, as well as cis and trans isomers of cyclic hydrocarbon or heterocyclo rings. In certain cases, for example, the exo or endo conformation can be preferred for the fused ring system bonded to G-L in formula I. For example, for androgen receptor antagonists (or selective androgen receptor modulators), where Y is O or $NR^7$, the exo configuration can be preferred, while for most other definitions of Y, the endo configuration can be preferred. As can be appreciated, the preferred configuration can be a function of the particular compound and its preferred activity. Separation of configurational isomers can be achieved by any suitable method, such as column chromatography.

Throughout the specifications, groups and substituents thereof may be chosen to provide stable moieties and compounds.

Embodiments indicated herein as exemplary or preferred are intended to be illustrative and not limiting.

## Methods of Preparation

The compounds of the present invention may be prepared by methods such as those illustrated in the following Schemes I to XI. Solvents, temperatures, pressures, and other reaction conditions may readily be selected by one of ordinary skill in the art. Starting materials are commercially available or readily prepared by one of ordinary skill in the art. Combinatorial techniques may be employed in the preparation of compounds, for example, where the intermediates possess groups suitable for these techniques. See the following which describe other methods which may be employed in the preparation of compounds of the present invention: Li *et al*., *Eur. J. Org. Chem.* **9**, 1841-1850 (1998); Li, Y-Q, *Synlett.* **5**, 461-464 (1996); Thiemann *et al., Bull. Chem. Soc. Jpn.* **67**, 1886-1893 (1994); Tsuge *et al*., *Heterocycles* **14**, 423-428 (1980); Ward *et al*., *Can J. Chem.* **75**, 681-693 (1997); Ward *et al*., *Can J. Chem.* **69**, 1487-1497 (1991); Ward *et al*., *Tetrahedron Lett.* **31**, 845-848 (1990); Fleming *et al*., *J. Org. Chem.* **44**, 2280-2282 (1979); Jankowski *et al., J. Organomet. Chem.* **595**, 109-113 (2000); Keglevich *et al., J. Organomet. Chem.* **579**, 182-189 (1999); Keglevich *et al., J. Organomet. Chem.* **570**, 49-539 (1998); Jankowski *et al., Hetroat. Chem.* **7**, 369-374 (1996); Jankowski *et al*., *J. Am. Chem. Soc.* **113**, 7011-7017 (1991); Quin *et al., Tetrahedron Lett.* **31**, 6473-6476 (1990); Quin *et al., J. Org. Chem.* **59**, 120-129 (1994); Quin *et al., J. Org. Chem.* **58,** 6212-6216 (1993); Quin *et al., Phosphorous, Sulfur Silicon Relat. Elem.* **63**, 349-362 (1991); Quin *et al., Hetroat. Chem.* **2,** 359-367 (1991); Hussong *et al., Phosphorus Sulfur.* **25**, 201-212 (1985); Quin *et al., J. Org. Chem.* **51**, 3341-3347 (1986); Myers *et al., J. Am. Chem. Soc.* **114,** 5684-5692 (1992); Myers *et al., J. Am. Chem. Soc.* **113,** 6682-6683 (1991); Shen *et al*., US Patent No. 5817679; Cordone *et al., J. Am. Chem. Soc.* **111,** 5969-5970 (1989); Jung *et al., J. Chem. Soc. Commun.* 630-632 (1984); Lay *et al., J. Am. Chem. Soc.* **104,** 7658-7659 (1982); Gonzalez *et al., J. Am. Chem. Soc.* 117, 3405-3421 (1995); Kreher *et al., Chem Ber.* **125,** 183-189 (1992); Simig *et al., Synlett.* **7,** 425-426 (1990); Sha *et al., J. Org. Chem.* **55**, 2446-2450 (1990); Drew *et al., J. Chem. Soc., Perkin Trans. 1* **7**, 1277-1284 (1985); Kreher *et al., Anorg. Chem., Org Chem.* **31B,** 599-604 (1976); Avalos *et al., Tetrahedron Lett.* **39,** 9301-9304 (1998); Gousse *et al., Macromolecules* **31**, 314-321 (1998); Mikhailyuchenko *et al., Khim. Geterotsikl. Soedin.* **6,** 751-758 (1993); Lubowitz *et al.,* US Patent No. 4476184; Padwa *et al., J. Org. Chem.* **61,** 3706-3714 (1996); Schlessinger *et al., J. Org. Chem.* **59,** 3246-3247 (1994); Buchmeiser *et al*., WO Publication No. 9827423; Tanabe *et al.,* Japanese Patent Document JP 07144477; Mochizucki *et al.,* Japanese Patent Document JP 63170383; Hosoda *et al*., Japanese Patent Document JP 62053963; Onaka *et al.,* Japanese Patent Document JP 62053964; Kato *et al*., Japanese Patent Document JP 53086035; Kato *et al*., Japanese Patent Document JP 51088631; Tottori *et al.,* Japanese Patent Document JP 49124225; Augustin *et al*., German Patent Document DD101271; Title *et al*., French Patent Document FR 2031538; Gousse *et al., Polym. Int.* **48,** 723-731 (1999); Padwa *et*

*al., J. Org. Chem.* **62**, 4088-4096 (1997); Theurillat-Moritz *et al., Tetrahedron: Asymmetry* **7**, 3163-3168 (1996); Mathews *et al., J. Carbohydr. Chem.* **14**, 287-97 (1995); Srivastava *et al., Natl. Acad. Sci. Lett. (India)* **15,** 41-44 (1992); Mayorga *et al., Rev. Cubana Quim.* **4,** 1-6 (1988); Kondoli *et al., J. Chem. Res., Synop.* **3,** 76 (1987); Primelles *et al., Cent. Azucar* 7-14 (1985); Solov'eva *et al., Khim. Geterotsikl. Soedin.* **5,** 613-15 (1984); Liu *et al., Yaoxue Xuebao* **18,** 752-759 (1983); Joshi *et al., Indian J. Chem. Sect. B.* **22B,** 131-135 (1983); Amos *et al.*, WO Publication No. 9829495; Odagiri *et al.*, US Patent No. 4670536; Gallucci *et al.*, European Patent Document EP 355435; Redmore, D., US Patent No. 3821232; Nakano *et al., Heterocycles* **35**, 37-40 (1993); Tomisawa *et al., Chem. Pharm. Bull.* **36**,1692-1697 (1988); Krow *et al., J. Heterocycl. Chem.* **22**, 131-135 (1985); Krow *et al., J. Org. Chem.* **47**, 1989-1993 (1982); Liu *et al., Yaoxue Xuebao* **18**, 752-759 (1983); Nishikawa *et al., Yaoxue Xuebao* JP 01061457; and/or Rice *et al., J. Med Chem.* **11**, 183-185 (1968).

All documents cited in the present specification, such as those cited in this "Methods of Preparation" as well as other sections herein, are incorporated herein by reference in their entirety. Reference to any document herein is not to be construed as an admission that such document is prior art.

**Scheme I**

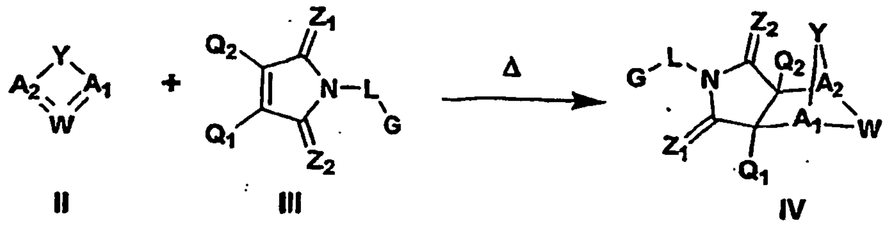


II III IV

As illustrated in Scheme **I,** a diene of formula **II** can be reacted with a dienophile of formula **III,** under conditions readily selected by one skilled in the art (such as by the addition of heat ("Δ")), to obtain a compound of formula **IV,** which is a compound of formula **I.** An intermediate diene of formula **II** can be obtained from commercial sources or readily made by one skilled in the art, for example, in accordance with the following literature documents and the references found therein: Hofman *et al., J. Agric. Food Chem.* **45,** 898-906 (1997); Baciocchi *et al., J. Chem. Soc., Perkin Trans. 2* **8,** 821-824 (1975); Wu *et al., J. Heterocycles* **38,** 1507-1518 (1994); Yin *et al., Tetrahedron Lett.* **38,** 5953-5954 (1997); Mic'ovic' *et al., Tetrahedron* **20,** 2279-2287 (1964); Gorbunova *et al., J. Org. Chem..* **35,** 1557-1566 (1999); Rassu *et al., Chem. Soc. Rev.* **29,** 109-118 (2000); Kaberdin *et al., Russ: Chem. Rev..* **68,** 765-779 (1999); Barluenga *et al., Aldrichimica Acta* **32,** 4-15 (1999); Bogdanowicz-Szwed *et al., Pol. Wiad Chem.* **52,** 821-842 (1998); Casiraghi *et al., Adv. Asymmetric Synth.* **3,** 113-189 (1998); and/or Baeckvall *et al., Chem. Rev.* **98,** 2291-2312 (1998). An intermediate dieneophile of formula **III** can be obtained from commercial sources or readily made by one skilled in the art, for example, in accordance with the following literature references and the references found therein: Deshpande *et al., Heterocycles* **51,** 2159-2162 (1999); Seijas *et al., J. Chem. Res., Synop.* **7,** 420-421 (1999); Langer *et al., Eur. J. Org. Chem.* **7,** 1467-1470 (1998); Kita *et al.,* Japanese Patent Document JP 09194458; Lopez-Alvarado et *al., J. Org. Chem.* **61,** 5865-5870 (1996); Condon *et al.,* US Patent No. 5523277; Sasakihara *et al.,* Japanese Patent Document JP 04290868; Igarashi *et al.*, Japanese Patent Document JP 04149173; Aoyama *et al.*, Japanese Patent Document JP 04134063; Aoyama *et al.,* Japanese Patent Document JP 04134062; Pastor *et al., J. Org. Chem.* **53,** 5776-5779 (1988); and/or Takahashi *et al., Chem. Lett.* **6,** 1229-1232 (1987).

**Scheme II**

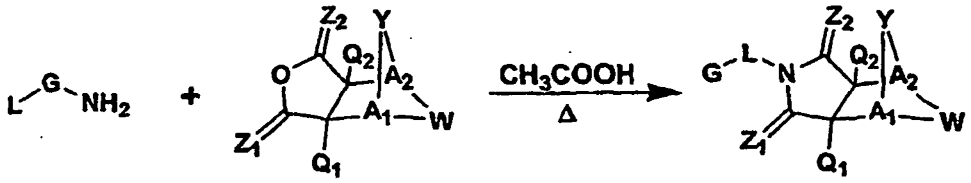


V VI IV

As illustrated in Scheme **II**, compounds of formula **I** can be obtained by reaction of a primary amine of formula **V** with a substituted anhydride-like intermediate of formula **VI**, for example, in a solvent such as acetic acid with or without heating, to yield a compound of formula **IV,** which is a compound of formula L Primary amines of formula **V** can be obtained from commercial sources or readily synthesized by one skilled in the art. Anhydride-like agents of formula **VI** can be obtained from commercial sources or readily synthesized by one skilled in the art. The documents listed following describe exemplary approaches for the synthesis of intermediates of formula **VI** as well as synthetic approaches which can be applied to the synthesis of compounds of formula **IV** (all incorporated herein by reference in their entirety): Kohler, E. P.; Tishler, M.; Potter, H.; Thompson, H. T., *J. Am. Chem. Soc.* 1939,1057-1061; Yur'ev, Y. K.; Zefirov, N. S., *J. Gen. Chem. U.S.S.R. (Engl. Transl.)* 1961, 31, 772-5; Norman G. Gaylord, US Patent No. 3,995,099; Schueler, P. E.; Rhodes, Y. E., *J. Org. Chem.* 1974, 39, 2063-9; Ishitobi, H.; Tanida, H; Tsuji, T., *Bull. Chem. Soc. Japan* 1971, 44, 2993-3000; Stájer, G.; Virág, M.; Szabó, A. E.; Bernáth, G.; Sohár, P.; Sillanpää, R., *Acta. Chem. Scand.* 1996, 50, 922-30; Hart, H.; Ghosh, T., *Tetrahedron Lett.* 1988,29,881-884; Kato, M.; Yamamoto, S.; Yoshihara, T.; Furuichi, K; Miwa, T., *Chem. Lett.* 1987, 1823-1826; Kottwitz, J.; Vorbrüggen, H., *Synthesis* 1995, 636-637; Creary, X., *J. Org. Chem.* 1975, 40, 3326-3331; Alder, K.; Ache, H.-J.; Flock, F. *H., Chem. Ber.* 1960, 93,1888-1895; Toder, B. H.; Branca, S. J.; Dieter, R. K.; Smith, A. B., III *Synth. Commun.* 1975, 5, 435-439; Sprague, P. W.; Heikes, J. E.; Gougoutas, J. Z.; Malley, M. F.; Hanris, D. N.; and/or Greenberg, R *J., Med Chem.* 1985,28,1580-1590.

The aforementioned approach(es) can be applied in a combinatorial fashion, for example, by utilizing a multi-well reaction block such as is described in Waldemar Ruediger, Wen-Jeng Li, John W., Allen Jr., and Harold N. Weller III, US Patent No. 5,961,925, Apparatus for Synthesis of Multiple Organic Compounds With Pinch Valve Block (incorporated herein by reference in its entirety). By utilizing the above-mentioned multi-well reaction block, one can, for example, perform multiples of 96 reactions at a time. Solvent can then be removed from the reaction tubes without removal from the reaction block and the crude products can be precipitated using a base such as sodium bicarbonate. The precipitates can be collected by filtration of the reaction block and then the desired products can be transferred directly to 96 well plates for screening. In this fashion, a large array of compounds of formula I can be synthesized, and tests conducted as desired by an automated approach.

**Scheme III**

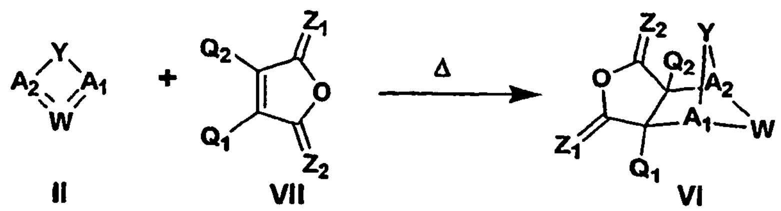

Scheme **III** describes a method for preparing an intermediate compound of formula **VI** which can be used to synthesize a compound of formula **I**, as described in Scheme **II.** As described in Scheme **III,** a diene of formula **II** can be reacted with a dieneophile of formula **VII** to yield the intermediate of formula **VI**. The methods applied to obtain such a transformation are analogous to those described in Scheme **I**.

**Scheme IV**

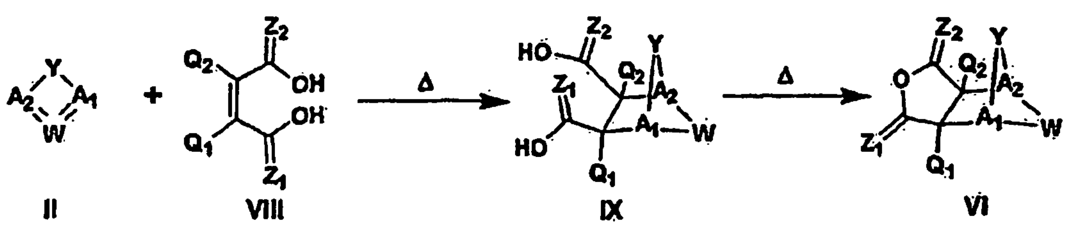

Scheme **IV** describes a method for preparing an intermediate compound of formula **VI** which can be used to synthesize a compound of formula **I,** as described in Scheme **II.** As shown in Scheme **IV,** a diene of formula **II** can be reacted with a dieneophile of formula **VIII** to yield the intermediate of formula **IX**. The intermediate of formula **IX** can be

dehydrated to an anhydride-like intermediate of formula **VI.** Dehydration of the bis-acid intermediate of formula **IX** can be achieved by a variety of methods, such as those known to one skilled in the art and described in the following documents and the references embodied therein: Sprague *et al., J. Med. Chem.* **28,** 1580-1590 (1985); and/or Retemi *et al., J. Org. Chem.* **61**, 6296-6301 (1996).

Schemes **I** to **IV** describe general methods for the synthesis of compounds of formula **I**, and intermediates thereof, in which substitution about the ring system is incorporated directly, for example, at the level of the intermediate diene, dienophile, anhydride-like intermediate and amine groups. In addition to these approaches, additional substitution can be incorporated onto an already-prepared compound of formula **I** by a variety of approaches to prepare other compounds of the formula **I.** Exemplary methods for further substitution are described in Schemes **V** to **XI.**

## Scheme V

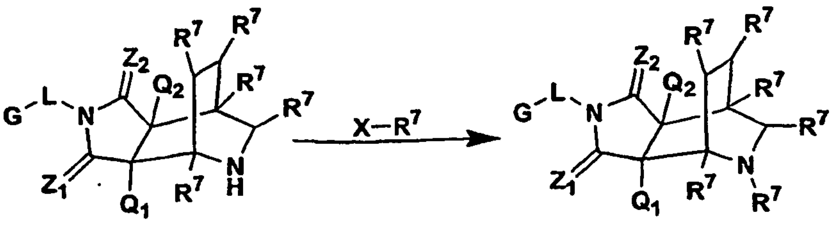


X XI

Scheme **V** describes one such approach to incorporating additional substitution into a structure of formula **I.** As illustrated in Scheme **V**, a compound of formula **X**, which is a compound of formula **I** where $A_1$ and $A_2$ are $CR^7$, W is NH— $CHR^7$ and Y is $CHR^7$—$CHR^7$, can be functionalized at the free amine of the group W by reaction with any of a variety of electrophilic agents such as acid halides or alkyl halides in the presence of base, for example, by methods known by one skilled in the art. In Scheme **V,** X is a leaving group, and a compound of formula **XI** is a compound of formula **I** where $A_1$ and $A_2$ are $CR^7$, W is $NR^7$—$CHR^7$ and Y is $CHR^7$— $CHR^7$.

## Scheme VI

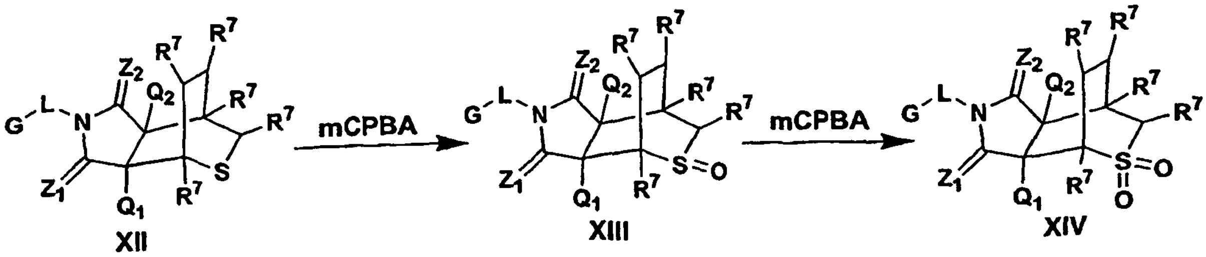


XII XIII XIV

Scheme **VI** describes an additional approach for further incorporating substitution onto a compound of formula **I**. As illustrated in Scheme **VI**, a compound of formula **XII,** which is a compound of formula **I** where $A_1$ and $A_2$ are $CR^7$, W is S-$CHR^7$ and Y is $CHR^7$—$CHR^7$, can be partially oxidized with an oxidizing agent such as mCPBA or other agents such as those known to one skilled in the art, to give the sulfoxide analog of formula **XIII,** which is a compound of formula **I** where $A_1$ and $A_2$ are $CR^7$, W is SO—$CHR^7$ and Y is $CHR^7$—$CHR^7$. Further treatment of a compound of formula **XIII** with an oxidizing agent such as mCPBA or other agents such as those known to one skilled in the art, can yield the sulphone analog of formula **XIV,** which is a compound of formula **I** where $A_1$ and $A_2$ are $CR^7$, W is $SO_2$—$CHR^7$ and Y is $CHR^7$—$CHR^7$. Alternatively, a compound of formula **XII** can be converted directly to a compound of formula **XIV** by prolonged treatment with an oxidizing agent, such as mCPBA, or with other agents such as those known to one skilled in the art.

**Scheme VII**

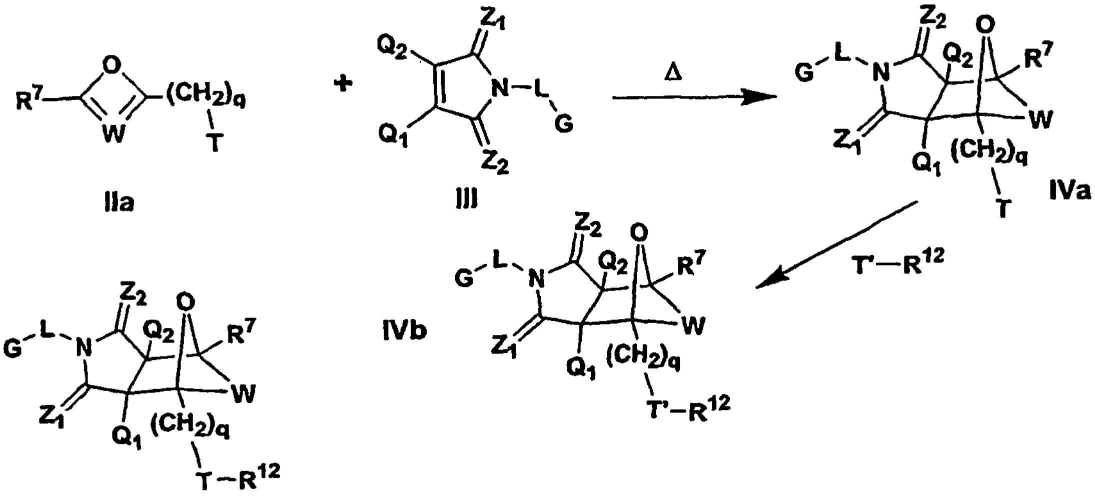


IIa III IVa IVb IVc

Scheme **VII** describes another approach to incorporating additional substitution onto a compound of formula **I.** As illustrated in Scheme **VII,** a diene of formula **IIa** can be reacted with a dienophile of formula **III,** as described in Scheme **I,** to yield a compound of formula **IVa,** which is a compound of formula **I** where Y is O, $A_2$ is $CR^7$ and $A_1$ is C—$(CH_2)_q$—T. The compound of formula **IVa** can be reacted with a reagent of formula $R^{12}$—T' to obtain a compound of formula **IVb** or **IVc** which are compounds of formula **I** where Y is O, $A_2$ is $CR^7$ and $A_1$ is C—$(CH_2)_q$—T'—$R^{12}$ or C—$(CH_2)_q$—T—$R^{12}$, respectively. The reagent $R^{12}$—T' can be obtained from commercial sources or can readily be prepared by one skilled in the art.

In the above Scheme, $R^{12}$ has the same definition as $R^7$ defined earlier, q is zero or an integer from 0-8, and T is defined either as (1) a nucleophilic center such as, but not limited, to a nitrogen, oxygen or sulfur-containing group, capable of undergoing a nucleophilic substitution reaction with the leaving group T' or (2) a leaving group capable undergoing a nucleophilic substitution reaction with a nucleophilic group T' (such as, but not limited, to a nitrogen, oxygen or sulfur-containing nucleophilic group). T' has the same definition as T. In the present case, for example, a nucleophilic substitution reaction occurs when the attacking reagent (the nucleophile) brings an electron pair to the substrate, using this pair to form the new bond, and the leaving group (the nucleofuge) comes away with the electron pair, leaving as an anionic intermediate. For a detailed discussion of the mechanism of aliphatic nucleophilic substitutions and a review of specific aliphatic nucleophilic substitution reactions see *Advanced Organic Chemistry, Reactions, Mechanisms, and Structure, 4$^{th}$ Addition.* Jerry March (Ed.), John Wiley & Sons, New York (1992) 293-500 and the references therein. Compounds of the formulae **IVa, IVb,** or **IVc** may, of course, be employed in the methods described herein (especially, in the treatment of nuclear hormone receptor-associated conditions) without undergoing further reaction of T or T'.

## Scheme VIII

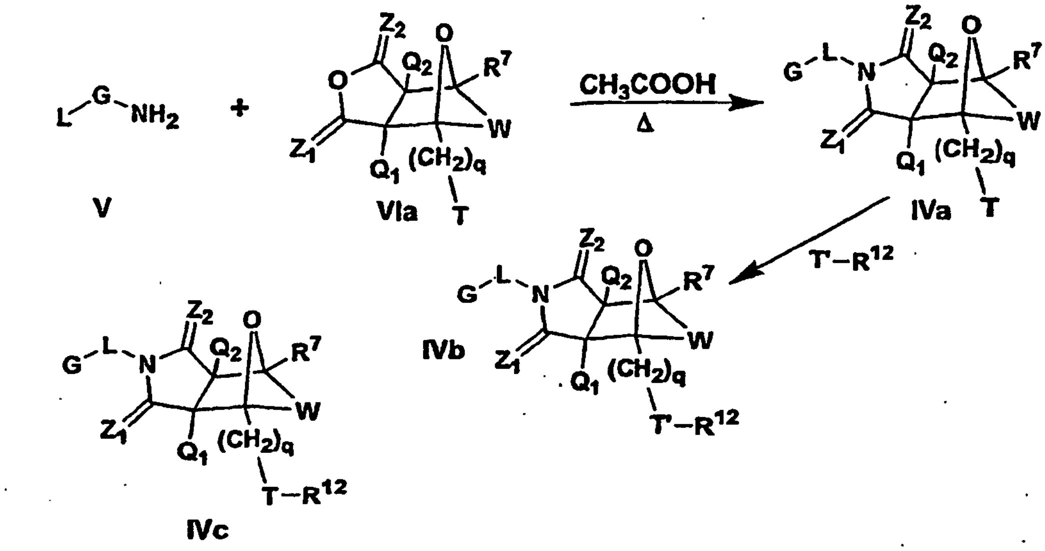


V  VIa  IVa  IVb  IVc

An alternate approach to compounds of formula **IVa, IVb** and **IVc** is illustrated in Scheme **VIII.** For this approach, techniques such as those described in Schemes **II, III** and **IV** can be applied to the preparation of an intermediate of formula **VIa,** where T and q are as defined in Scheme **VII.** The intermediate of formula **VIa** can be reacted with a substituted amine of formula **V,** as described in Scheme **II,** to yield the compound of formula **IVa,** which is a compound of formula **I** where Y is O, $A_2$ is $CR^7$ and $A_1$ is $C—(CH_2)_q—T$. The compound of formula **IVa** can be treated in the manner described in Scheme **VII** to obtain compounds of formula **IVb** or **IVc** which are compounds of formula **I** where Y is O, $A_2$ is $CR^7$ and $A_1$ is $C— (CH_2)_q—T'—R^{12}$ or $C—(CH_2)_q—T—R^{12}$, respectively.

## Scheme IX

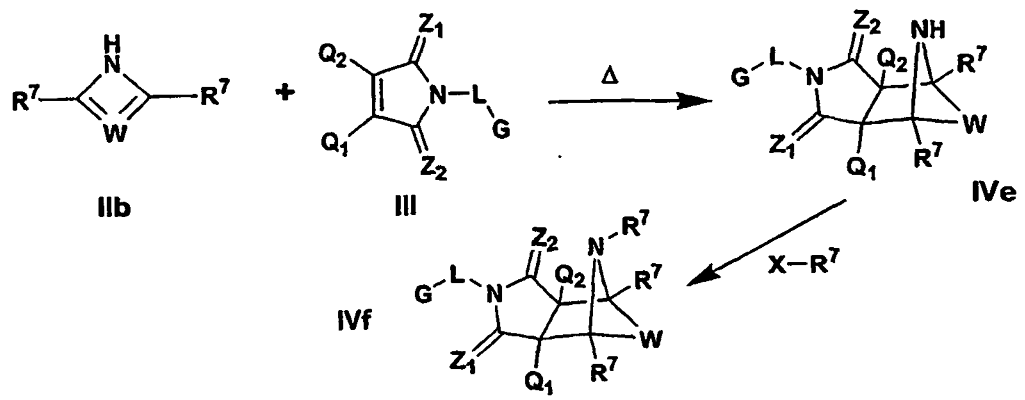


IIb  III  IVe  IVf

Scheme **IX** describes another approach to incorporating further substitution onto a compound of formula **I.** As illustrated in Scheme **IX** (where **X** is a leaving group), a diene of formula **IIb** can be reacted with a dienophile of formula **III,** as described in Scheme **I**, to yield a compound of formula **IVe**, which is a compound of formula **I** where Y is NH, and $A_1$ and $A_2$ are $CR^7$. The compound of formula **IVe** can be functionalized at the free amine by reacting with a variety of electrophilic agents such as acid halides or alkyl halides in the presence of base, for example by methods known by one skilled in the art and described in Scheme **V**, to yield a compound of formula **IVf**, which is a compound of formula **I** where Y is $NR^7$ and $A_1$ and $A_2$ are $CR^7$.

## Scheme X

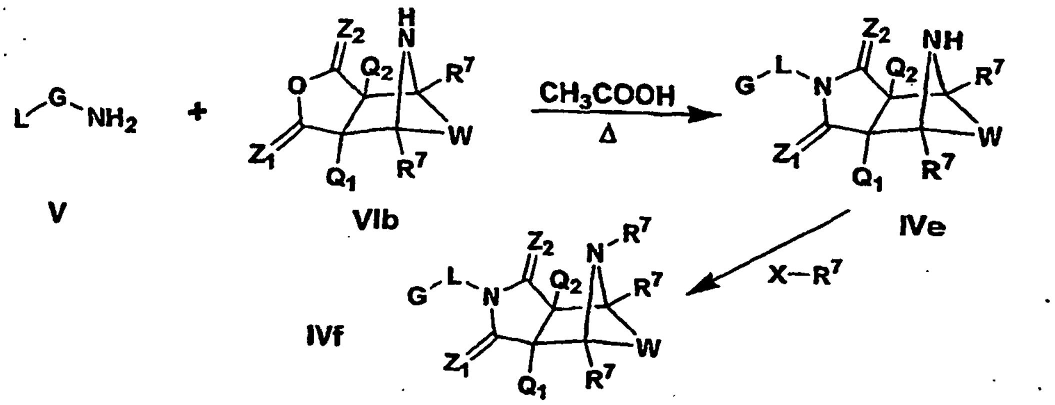


V VIb IVe IVf

An alternate approach to compounds of formula **IVe** and **IVf** is illustrated in Scheme **X**. For this approach, techniques as described in Schemes **II, III** and **IV** can be applied to the preparation of an intermediate of formula **VIb.** The intermediate of formula **VIb** can be reacted with a substituted amine of formula **V,** as described in Scheme **II,** to yield a compound of formula **IVe**, which is a compound of formula **I** where Y is NH, and $A_1$ and $A_2$ are $CR^7$. The latter intermediate can be treated in the manner described in Scheme V to obtain a compound of formula **IVf,** which is a compound of formula **I** where Y is $NR^7$, and $A_1$ and $A_2$ are $CR^7$.

## Scheme XI

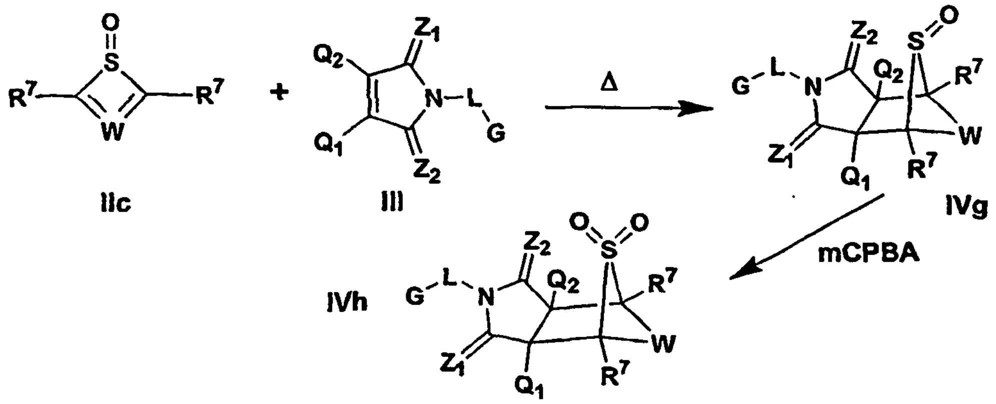


IIc III IVg IVh

Scheme **XI** describes another approach to incorporating additional substitution onto a compound of formula **I.** As illustrated in Scheme **XI,** a diene of formula **IIc** can be reacted with a dienophile of formula **III,** as described in Scheme **I,** to yield a compound of formula **IVg,** which is a compound of formula **I** where Y is SO and $A_1$ and $A_2$ are $CR^7$. A compound of formula **IVg** can be treated with an oxidizing agent such as mCPBA, as described in Scheme **VI,** to yield a compound of formula **IVh,** which is a compound of formula I where Y is $SO_2$ and $A_1$ and $A_2$ are $CR^7$.

## Scheme XII

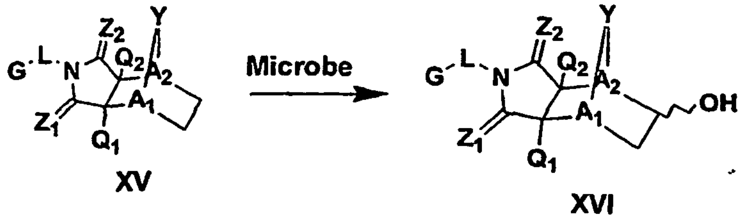


Scheme **XII** describes another approach to incorporating additional substitution onto a compound of formula **I.** As illustrated in Scheme **XII,** a compound of formula **XV,** which can be prepared in accordance with the above Schemes, can be incubated in the presence of a suitable enzyme or microorganism resulting in the formation of a hydroxylated analog of formula **XVI.** Such a process can be employed to yield regiospecific as well as enantiospecific incorporation of a hydroxyl group into a molecule of formula **XV** by a specific microorganism or by a series of different microorganisms. Such microorganisms can, for example, be bacterial, yeast or fungal in nature and can be obtained from distributors such as ATCC or identified for use in this method such as by methods known to one skilled in the art. Compound **XVI** is a compound of formula I where Y is as described above and $A_1$ and $A_2$ are preferably $CR^7$.

## Scheme XIII

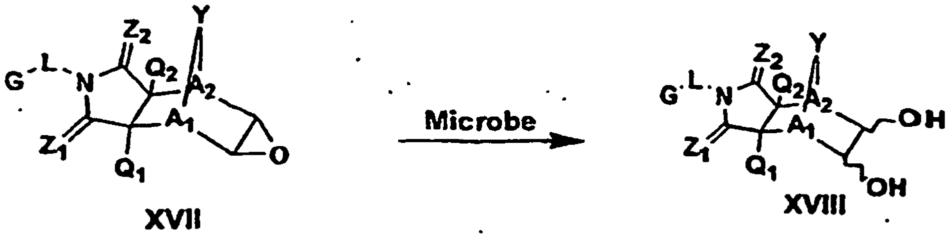


Scheme **XIII** describes another approach to incorporating additional substitution onto a compound of formula **I**. As illustrated in Scheme **XIII,** a compound of formula **XVII,** which can be prepared in accordance with the above Schemes, can be incubated in the presence of a suitable enzyme or microorganism resulting in the formation of a diol analog of formula **XVIII.** Such a process can be employed to yield regiospecific as well as enantiospecific transformation of a compound of formula **XVII** to a 1,2-diol of formula **XVIII** by a specific microorganism or by a series of different microorganisms. Such microorganisms can, for example, be bacterial, yeast or fungal in nature and can be obtained from distributors such as ATCC or identified for use in this method such as by methods known to one skilled in the art. Compound **XVIII** is a compound of formula I where Y is as described above and $A_1$ and $A_2$ are preferably $CR^7$.

The present invention also provides the methods of Schemes XII and XIII.

Thus, in one embodiment, the present invention provides a method for preparation of a compound of the following formula XVI, or salt thereof:

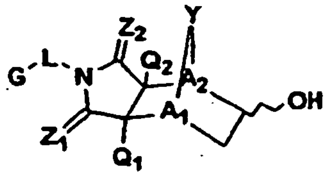

XVI

where the symbols are as defined in claim 3,
comprising the steps of contacting a compound of the following formula XV, or salt thereof:

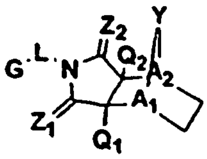

XV

where the symbols are as defined above;
with an enzyme or microorganism capable of catalyzing the hydroxylation of said compound XV to form said compound XVI, and effecting said hydroxylation.

In another preferred embodiment, the present invention provides a method for preparation of a compound of the following formula XVIII, or salt thereof:

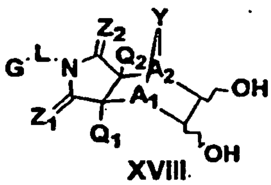

XVIII

where the symbols are as defined in claim 3,
comprising the steps of contacting a compound of the following formula XVII, or salt thereof:

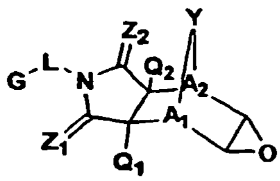

XVII

where the symbols are as defined above;
with an enzyme or microorganism capable of catalyzing the opening of the epoxide ring of compound XVII to form the diol of said compound XVIII, and effecting said ring opening and diol formation.

All stereoconfigurations of the unspecified chiral centers of the compounds of the formulae XV, XVI, XVII and XVIII are contemplated in the methods of the present invention, either alone (that is, substantially free of other stereoisomers) or in admixture with other stereoisomeric forms. Conversion of one isomer selectively (e.g., hydroxylation of the exo isomer preferentially to hydroxylation of the endo isomer) when contacting an isomeric mixture is a preferred embodiment of the invention. Conversion to one isomer selectively (e.g., hydroxylation on the exo face "exo isomer" preferentially to the endo face "endo isomer" or regioselective opening of an epoxide to form only one of two possible regioisomers of a trans diol) is a preferred embodiment of the invention. Hydroxylation of an achiral intermediate to form

a single optical isomer of the hydroxylated product is also a preferred embodiment of the invention. Resolution of a racemic mixture of an intermediate by selective hydroxylation, or epoxide ring opening and diol formation, to generate one of the two possible optical isomers is also a preferred embodiment of the invention. The term "resolution" as used herein denotes partial, as well as, preferably, complete resolution.

The terms "enzymatic process" or "enzymatic method", as used herein, denote a process or method of the present invention employing an enzyme or microorganism. The term "hydroxylation", as used herein, denotes the addition of a hydroxyl group to a methylene group as described above. Hydroxylation can be achieved, for example, by contact with molecular oxygen according to the methods of the present invention. Diol formation can be achieved, for example, by contact with water according to the methods of the present invention. Use of "an enzyme or microorganism" in the present methods includes use of two or more, as well as a single, enzyme or microorganism.

The enzyme or microorganism employed in the present invention can be any enzyme or microorganism capable of catalyzing the enzymatic conversions described herein. The enyzmatic or microbial materials, regardless of origin or purity, can be employed in the free state or immobilized on a support such as by physical adsorption or entrapment. Microorganisms or enzymes suitable for use in the present invention can be selected by screening for the desired activity, for example, by contacting a candidate microorganism or enzyme with a starting compound XV or XVII or salt thereof, and noting conversion to the corresponding compound XVI or XVIII or salt thereof. The enzyme may, for example, be in the form of animal or plant enzymes or mixtures thereof, cells of microorganisms, crushed cells, extracts of cells, or of synthetic origin.

Exemplary microorganisms include those within the genera: *Streptomyces* or *Amycolatopsis*. Particularly preferred microorganisms are those within the species *Streptomyces griseus*, especially *Streptomyces griseus* ATCC 10137, and *Amycolatopsis orientalis* such as ATCC 14930, ATCC 21425, ATCC 35165, ATCC 39444, ATCC 43333, ATCC 43490, ATCC 53550, ATCC 53630, and especially ATCC 43491. The term "ATCC" as used herein refers to the accession number of the American Type Culture Collection, 10801 University Blvd., Manassas Virginia 20110-2209, the depository for the organism referred to. It should be understood that mutants of these organisms are also contemplated by the present invention, for use in the methods described herein, such as those modified by the use of chemical, physical (for example, X-rays) or biological means (for example, by molecular biology techniques).

Preferred enzymes include those derived from microorganisms, particularly those microorganisms described above. Enzymes may be isolated, for example, by extraction and purification methods such as by methods known to those of ordinary skill in the art. An enzyme may, for example, be used in its free state or in immobilized form. One embodiment of the invention is that where an enzyme is adsorbed onto a suitable carrier, e.g., diatomaceous earth (porous Celite Hyflo Supercel), microporous polypropylene (Enka Accurel® polypropylene powder), or a nonionic polymeric adsorbent such as Amberlite® XAD-2 (polystyrene) or XAD-7 (polyacrylate) from Rohm and Haas Co. When employed to immobilize an enzyme, a carrier may control the enzyme particle size and prevent aggregation of the enzyme particles when used in an organic solvent. Immobilization can be accomplished, for example, by precipitating an aqueous solution of the enzyme with cold acetone in the presence of the Celite Hyflo Supercel followed by vacuum drying, or in the case of a nonionic polymeric adsorbent, incubating enzyme solutions with adsorbent on a shaker, removing excess solution and drying enzyme-adsorbent resins under vacuum. While it is desirable to use the least amount of enzyme possible, the amount of enzyme required will vary depending upon the specific activity of the enzyme used.

Hydroxylation as described above can occur *in vivo.* For example, liver enzyme can selectively, relative to the endo isomer, hydroxylate the exo isomer of a compound of the present invention. In conducting the methods of the present invention outside the body, liver microsomal hydroxylase can be employed as the enzyme for catalysis.

These processes may also be carried out using microbial cells containing an enzyme having the ability to catalyze the conversions. When using a microorganism to perform the conversion, these procedures are conveniently carried out by adding the cells and the starting material to the desired reaction medium.

Where microorganisms are employed, the cells may be used in the form of intact wet cells or dried cells such as lyophilized, spray-dried or heat-dried cells, or in the form of treated cell material such as ruptured cells or cell extracts. Cell extracts immobilized on Celite® or Accurel® polypropylene as described earlier may also be employed. The use of genetically engineered organisms is also contemplated The host cell may be any cell, e.g. *Escherichia coli,* modified to contain a gene or genes for expressing one or more enzymes capable of catalysis as described herein.

Where one or more microorganisms are employed, the enzymatic methods of the present invention may be carried out subsequent to the fermentation of the microorganism (two-stage fermentation and conversion), or concurrently therewith, that is, in the latter case, by *in situ* fermentation and conversion (single-stage fermentation and conversion).

Growth of the microorganisms can be achieved by one of ordinary skill in the art by the use of an appropriate medium. Appropriate media for growing microorganisms include those which provide nutrients necessary for the growth of the microbial cells. A typical medium for growth includes necessary carbon sources, nitrogen sources, and elements (e.g. in trace amounts). Inducers may also be added. The term "inducer", as used herein, includes any compound enhancing formation of the desired enzymatic activity within the microbial cell.

Carbon sources can include sugars such as maltose, lactose, glucose, fructose, glycerol, sorbitol, sucrose, starch, mannitol, propylene glycol, and the like; organic acids such as sodium acetate, sodium citrate, and the like; and alcohols such as ethanol, propanol and the like.

Nitrogen sources can include N-Z amine A, corn steep liquor, soy bean meal, beef extracts, yeast extracts, molasses, baker's yeast, tryptone, nutrisoy, peptone, yeastamin, amino acids such as sodium glutamate and the like, sodium nitrate, ammonium sulfate and the like.

Trace elements can include magnesium, manganese, calcium, cobalt, nickel, iron, sodium and potassium salts. Phosphates may also be added in trace or, preferably, greater than trace amounts.

The medium employed can include more than one carbon or nitrogen source or other nutrient

Preferred media for growth include aqueous media.

The agitation and aeration of the reaction mixture affect the amount of oxygen available during the conversion process when conducted, for example, in shake-flask cultures or fermentor tanks during growth of microorganisms.

Incubation of the reaction medium is preferably at a temperature between about 4 and about 60°C. The reaction time can be appropriately varied depending upon the amount of enzyme used and its specific activity. Reaction times may be reduced by increasing the reaction temperature and/or increasing the amount of enzyme added to the reaction solution.

It is also preferred to employ an aqueous liquid as the reaction medium, although an organic liquid, or a miscible or immiscible (biphasic) organic/aqueous liquid mixture, may also be employed. The amount of enzyme or microorganism employed relative to the starting material is selected to allow catalysis of the enzymatic conversions of the present invention.

Solvents for the organic phase of a biphasic solvent system may be any organic solvent immiscible in water, such as toluene, cyclohexane, xylene, trichlorotrifluoroethane and the like. The aqueous phase is conveniently of water, preferably deionized water, or a suitable aqueous buffer solution, especially a phosphate buffer solution. The biphasic solvent system preferably comprises between about 10 to 90 percent by volume of organic phase and between about 90 to 10 percent by volume of aqueous phase, and most preferably contains at or about 20 percent by volume of organic phase and at or about 80 percent by volume of the aqueous phase.

An exemplary embodiment of such processes starts with preparation of an aqueous solution of the enzyme(s) or microbes to be used. For example, the preferred enzyme(s) or microbes can be added to a suitable amount of an aqueous solvent, such as phosphate buffer or the like. This mixture is preferably adjusted to and maintained at a desired pH.

The compounds XVI and XVIII produced by the processes of the present invention can be isolated and purified, for example, by methods such as extraction, distillation, crystallization, and column chromatography.

## Preferred Compounds

A preferred subgenus of the compounds of the present invention includes compounds of the formula I or salts thereof wherein one or more, preferably all, of the following substituents are as defined below:

G is an aryl or heterocyclo (e.g., heteroaryl) group, where said group is mono- or polycyclic, and which is optionally substituted at one or more positions, preferably with hydrogen, alkyl or substituted alkyl, alkenyl or substituted alkenyl, alkynyl or substituted alkynyl, halo, cycloalkyl or substituted cycloalkyl, cycloalkenyl or substituted cycloalkenyl, aryl or substituted aryl, heterocyclo or substituted heterocyclo, arylalkyl or substituted arylalkyl, heterocycloalkyl or substituted heterocycloalkyl, CN, $R^1OC{=}O$, $R^1C{=}O$, $R^1HNC{=}O$, $R^1R^2NC{=}O$, $HOCR^3R^{3'}$, nitro, $R^1OCH_2$, $R^1O$, $NH_2$, $NR^4R^5$, $S{=}OR^1$, $SO_2R^1$, $SO_2NR^1R^{1'}$, $(R^1)(R^{1'})P{=}O$, or $(R^{1'})(NHR^1)P{=}O$;

$R^1$ and $R^{1'}$ are each independently H, alkyl or substituted alkyl, cycloalkyl or substituted cycloalkyl, cycloalkenyl or substituted cycloalkenyl, heterocyclo or substituted heterocyclo, cycloalkylalkyl or substituted cycloalkyalkyl, cycloalkenylalkyl or substituted cycloalkenylalkyl, heterocycloalkyl or substituted heterocycloalkyl, aryl or substituted aryl, arylalkyl or substituted arylalkyl;

$R^2$ is alkyl or substituted alkyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl;

$R^4$ is H, alkyl or substituted alkyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloallcyl, aryl or substituted aryl, arylalkyl or substituted arylalkyl, $R^1C{=}O$, $R^1NHC{=}O$, or $SO_2NR^1R^{1'}$;

$R^5$ is alkyl or substituted alkyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, $R^1C=O$, $R^1NHC=O$, $SO_2R^1$, or $SO_2NR^1R^{1'}$;

$R^7$ and $R^{7'}$ are each independently H, alkyl or substituted alkyl, alkenyl or substituted alkenyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, halo, CN, $OR^1$, nitro, hydroxylamine, hydroxylamide, amino, $NHR^4$, $NR^2R^5$, $NOR^1$, thiol, alkylthio or substituted alkylthio, $R^1C=O$, $R^1OC=O$, $R^1NHC=O$, $SOR^1$, $PO_3R^1R^{1'}$, $R^1R^{1'}NC=O$, $C=OSR^1$, $SO_2R^1$, or $SO_2NR^1R^{1'}$;

$R^8$ is H, alkyl or substituted alkyl, alkenyl or substituted alkenyl, cycloalkyl or substituted cycloalkyl, cycloalkenyl or substituted cycloalkenyl, heterocyclo or substituted heterocyclo, cycloalkylalkyl or substituted cycloalkyalkyl, cycloalkenylalkyl or substituted cycloalkenylalkyl, heterocycloalkyl or substituted heterocycloalkyl, aryl or substituted aryl, arylalkyl or substituted arylalkyl, nitro, halo, CN, $OR^1$, amino, $NHR^4$, $NR^2R^5$, $NOR^1$, alkylthio or substituted alkylthio, $C=OSR^1$, $R^1OC=O$, $R^1C=O$, $R^1NHC=O$, $R^1R^{1'}NC=O$, $S=OR^1$, $SO_2R^1$, $PO_3R^1R^{1'}$, or $SO_2NR^1R^{1'}$;

$R^9$ and $R^{9'}$ are each independently H, alkyl or substituted alkyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, CN, OH, $OR^1$, $R^1C=O$, $R^1OC=O$, $R^1NHC=O$, or $SO_2NR^1R^{1'}$;

with the provisos (1) to (11) of said formula I where appropriate to this subgenus, and most preferably where (i) when Y is -O- and W is $CR^7R^{7'}$ —$CR^7R^{7'}$, $A_1$ and $A_2$ are not simultaneously CH; and (ii) when L is a bond, G is not an unsubstituted phenyl group.

Another, more preferred subgenus of the compounds of the invention includes compounds of the formula I or salts thereof wherein one or more, preferably all, of the following substituents are as defined below:

G is an aryl or heterocyclo (e.g., heteroaryl) group, where said group is mono- or polycyclic, and which is optionally substituted at one or more positions, preferably with hydrogen, alkyl or substituted alkyl, alkenyl or substituted alkenyl, alkynyl or substituted alkynyl, halo, cycloaucyl or substituted cycloalkyl, cycloalkenyl or substituted cycloalkenyl, aryl or substituted aryl, heterocyclo or substituted heterocyclo, arylalkyl or substituted arylalkyl, heterocycloalkyl or substituted heterocycloalkyl, CN, $R^1C=O$, $R^1HNC=O$, $R^1R^2NC=O$, $HOCR^3R^{3'}$, nitro, $R^1OCH_2$, $R^1O$, $NH_2$, $NR^4R^5$, $SO_2R^1$, or $SO_2NR^1R^{1'}$;

$Z_1$ is O;

$Z_2$ is O;

$A_1$ is $CR^7$;

$A_2$ is $CR^7$;

L is a bond;

$R^1$ and $R^{1'}$ are each independently H, alkyl or substituted alkyl, cycloalkyl or substituted cycloalkyl, cycloalkenyl or substituted cycloalkenyl, heterocyclo or substituted heterocyclo, cycloalkylalkyl or substituted cycloalkyalkyl, cycloalkenylalkyl or substituted cycloalkenylalkyl, heterocycloalkyl or substituted heterocycloalkyl, aryl or substituted aryl, arylalkyl or substituted arylalkyl;

$R^2$ is alkyl or substituted alkyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl;

$R^4$ is H, alkyl or substituted alkyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, $R^1C=O$, $R^1NHC=O$, or $SO_2NR^1R^{1'}$;

$R^5$ is alkyl or substituted alkyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, $R^1C=O$, $R^1NHC=O$, $SO_2R^1$, or $SO_2NR^1R^{1'}$;

$R^7$ and $R^{7'}$ are each independently H, alkyl or substituted alkyl, alkenyl or substituted alkenyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloallcenylalkyl, heterocycloallcyl or substituted heterocycloalkyl, aryl or substituted aryl, arylalkyl or substituted arylalkyl, halo, CN, $OR^1$, nitro, amino, $NHR^4$, $NR^2R^5$, alkylthio or substituted alkylthio, $R^1C=O$, $R^1NHC=O$, $SO_2R^1$, $R^1R^{1'}NC=O$, or $SO_2NR^1R^{1'}$;

$R^8$ is H, alkyl or substituted alkyl, alkenyl or substituted alkenyl, cycloalkyl or substituted cycloalkyl, cycloalkenyl or substituted cycloalkenyl, heterocyclo or substituted heterocyclo, cycloalkylalkyl or substituted cycloalkyalkyl, cycloalkenylalkyl or substituted cycloalkenylalkyl, heterocycloalkyl or substituted heterocycloalkyl, aryl or substituted aryl, arylalkyl or substituted arylalkyl, nitro, halo, CN, $OR^1$, amino, $NHR^4$, $NR^2R^5$, alkylthio or substituted alkylthio, $R^1C=O$, $R^1NHC=O$, $R^1R^{1'}NC=O$, $SO_2R^1$, or $SO_2NR^1R^{1'}$; and

$R^9$ and $R^{9'}$ are each independently H, alkyl or substituted alkyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, CN, OH, $OR^1$, $R^1C=O$, $R^1NHC=O$, or $SO_2NR^1R^{1'}$;

with the provisos (1) to (11) of said formula I where appropriate to this subgenus, and most preferably where (i) when Y is -O- and W is $CR^7R^{7'}$—$CR^7R^{7'}$, $A_1$ and $A_2$ are not simultaneously CH; and (ii) when L is a bond, G is not an unsubstituted phenyl group.

Preferred G-L groups are optionally substituted naphthyl and optionally substituted fused bicyclic heterocyclic groups such as optionally substituted benzo-fused heterocyclic groups (e.g., bonded to the remainder of the molecule through the benzene portion), especially such groups wherein the heterocyclic ring bonded to benzene has 5 members exemplified by benzoxazole, benzothiazole, benzothiadiazole, benzoxadiazole or benzothiophene, for example:

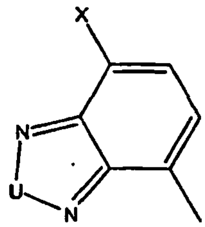

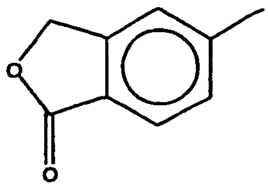

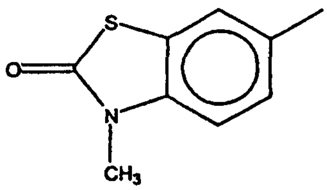

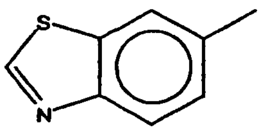

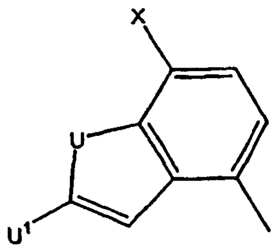

(e.g., 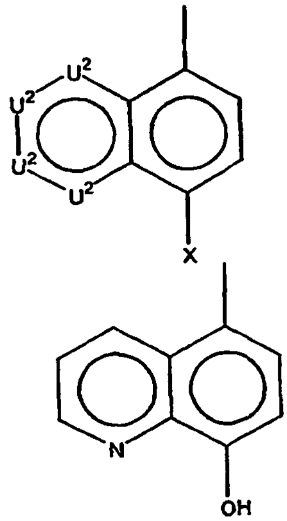 )

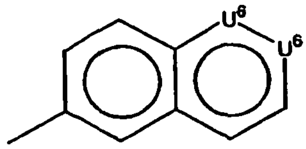

or

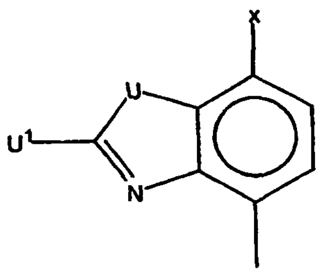

where
X = halo (esp F), OH, CN, $NO_2$ or

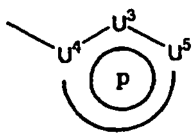

(e.g.,

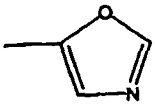 , 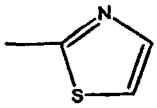 or 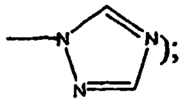);

U is O or S (where S can optionally be oxygenated, e.g., to SO);
$U^1$ is $CH_3$ or $CF_3$;
each $U^2$ is independently N, CH or CF;
$U^3$ is N, O or S;
$U^4$ and $U^5$, together with the atoms to which they are bonded, form an optionally substituted 5-membered heterocyclic ring which can be partially unsaturated or aromatic and which contains 1 to 3 ring heteroatoms;
each $U^6$ is independently CH or N; and

Ⓟ denotes optional double bond(s) within the ring formed by $U^3$, $U^4$ and $U^5$.

An especially preferred subgenus includes compounds of the formula I having the following structure, or salts thereof:

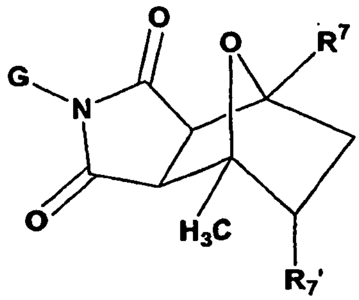

where G is an optionally substituted naphthyl or benzo-fused bicyclic heterocyclic group, $R^7$ is $CH_3$ or $C_{1-4}$alkyl substituted by V' and $R^{7'}$ is H or hydroxyL

Compounds where $R^{7'}$ is hydroxyl can provide enhanced water solubility and metabolic stability, relative to the corresponding compounds where $R^{7'}$ is H, in addition to having good permeability and high systemic blood levels. These hydroxyl-bearing compounds can be obtained *in vivo* by metabolism of the corresponding compound where $R^{7'}$ is H, as well as by synthetic preparative methods such as those described herein.

**Use and Utility**

The compounds of the present invention modulate the function of nuclear hormone receptors (NHR), and include compounds which are, for example, agonists, partial agonists, antagonists or partial antagonists of the androgen receptor (AR), the estrogen receptor (ER), the progesterone receptor (PR), the glucocorticoid receptor (GR), the mineralocorticoid receptor (MR), the steroid and xenobiotic receptor (SXR), other steroid binding NHR's, the Orphan receptors or other NHR's. Selective modulation of one such NHR relative to others within the NHR family is preferred. "Modulation" includes, for example, activation (e.g., agonist activity such as selective androgen receptor agonist activity) or inhibition (e.g., antagonist activity).

The present compounds are thus useful in the treatment of NHR-associated conditions. A "NHR-associated condition", as used herein, denotes a condition or disorder which can be treated by modulating the function of a NHR in a subj ect, wherein treatment comprises prevention (e.g., prophylactic treatment), partial alleviation or cure of the condition or disorder. Modulation may occur locally, for example, within certain tissues of the subject, or more extensively throughout a subject being treated for such a condition or disorder.

The compounds of the present invention are useful for the treatment of a variety of conditions and disorders including, but not limited to, those described following:

Compounds of formula I can be applied as agonists, partial agonists, antagonists, or partial antagonists of the estrogen receptor, preferably selectively to that receptor, in an array of medical conditions which involve modulation of the estrogen receptor pathway. Applications of said compounds include but are not limited to: osteoporosis, hot flushes, vaginal dryness, prostate cancer, breast cancer, endometrial cancer, cancers expressing the estrogen receptor such

as the aforementioned cancers and others, contraception, pregnancy termination, menopause, amennoreahea, and dysmennoreahea.

Compounds of formula I can be applied as agonists, partial agonists, antagonists or partial antagonists of the progesterone receptor, preferably selectively to that receptor, in an array of medical conditions which involve modulation of the progesterone receptor pathway. Applications of said compounds include but are not limited to: breast cancer, other cancers containing the progesterone receptor, endometriosis, cachexia, contraception, menopause, cyclesynchrony, meningioma, dysmennoreahea, fibroids, pregnancy termination, labor induction and osteoporosis.

Compounds of formula I can be applied as agonists, partial agonists; antagonists or partial antagonists of the glucocorticoid receptor, preferably selectively to that receptor, in an array of medical conditions which involve modulation of the glucocorticoid receptor pathway. Applications of said compounds include but are not limited to: inflammatory diseases, autoimmune diseases, prostate cancer, breast cancer, Alzheimer's disease, psychotic disorders, drug dependence, non-insulin dependent Diabetes Mellitus, and as dopamine receptor blocking agents or otherwise as agents for the treatment of dopamine receptor mediated disorders.

Compounds of formula I can be applied as agonists, partial agonists, antagonists or partial antagonists of the mineralocorticoid receptor, preferably selectively to that receptor, in an array of medical conditions which involve modulation of the mineralocorticoid receptor pathway. Applications of said compounds include but are not limited to: drug withdrawal syndrome and inflammatory diseases.

Compounds of formula I can be applied as agonists, partial agonists, antagonists or partial antagonists of the aldosterone receptor, preferably selectively to that receptor, in an array of medical conditions which involve modulation of the aldosterone receptor pathway. One application of said compounds includes but is not limited to: congestive heart failure.

Compounds of formula I can be applied as agonists, partial agonists, antagonists or partial antagonists of the androgen receptor, preferably selectively to that receptor, in an array of medical conditions which involve modulation of the androgen receptor pathway. Applications of said compounds include but are not limited to: hirsutism, acne, seborrhea, Alzheimer's disease, androgenic alopecia, hypogonadism, hyperpilosity, benign prostate hypertrophia, adenomas and neoplasies of the prostate (such as advanced metastatic prostate cancer), treatment of benign or malignant tumor cells containing the androgen receptor such as is the case for breast, brain, skin, ovarian, bladder, lymphatic, liver and kidney cancers, pancreatic cancers modulation of VCAM expression and applications therein for the treatment of heart disease, inflammation and immune modulations, modulation of VEGF expression and the applications therein for use as antiangiogenic agents, osteoporosis, suppressing spermatogenesis, libido, cachexia, endometriosis, polycystic ovary syndrome, anorexia, androgen supplement for age related decreased testosterone levels in men, male menopause, male hormone replacement, male and female sexual dysfunction, and inhibition of muscular atrophy in ambulatory patients. For example, pan AR modulation is contemplated, with prostate selective AR modulation ("SARM") being particularly preferred, such as for the treatment of early stage prostate cancers..

Compounds of formula I can be applied as (preferably, selective) antagonists of the mutated androgen receptor, for example, found in many tumor lines. Examples of such mutants are those found in representative prostate tumor cell lines such as LNCap (T877A mutation, Biophys. Acta 187, 1052 (1990)), PCa2b, (L701H & T877A mutations, J. Urol. 162, 2192 (1999)) and CWR22 (H874Y mutation, Mol. Endo. 11, 450 (1997)). Applications of said compounds include but are not limited to: adenomas and neoplasies of the prostate, breast cancer and endometrial cancer.

Compounds of formula I can be applied as agonists, partial agonists, antagonists or partial antagonists of the steroid and xenobiotic receptor, preferably selectively to that receptor, in an array of medical conditions which involve modulation of the steroid and xenobiotic receptor pathway. Applications of said compounds include but are not limited to: treatment of disregulation of cholesterol homeostasis, attenuation of metabolism of pharmaceutical agents by co-administration of an agent (compound of the present invention) which modulates the P450 regulator effects of SXR.

Along with the aforementioned NHR, there also exist a number of NHR for which the activating or deactivating ligands may not be characterized. These proteins are classified as NHR due to strong sequence homology to other NHR, and are known as the Orphan receptors. Because the Orphan receptors demonstrate strong sequence homology to other NHR, compounds of formula I include those which serve as modulators of the function of the Orphan NHR. Orphan receptors which are modulated by NHR modulators such as compounds within the scope of formula I are exemplified, but not limited to, those listed in Table 1. Exemplary therapeutic applications of modulators of said Orphan receptors are also listed in Table 1, but are not limited to the examples therein.

**Table 1**. Exemplary Orphan nuclear hormone receptors, form (M = monomeric, D = heterodimeric, H = homodimeric), tissue expression and target therapeutic applications. (CNS=central nervous system)

| Receptor | Form | Tissue Expression | Target Therapeutic Application |
|---|---|---|---|
| NURR1 | M/D | Dopaminergic Neurons | Parkinson's Disease |
| RZRβ | M | Brain (Pituitary), Muscle | Sleep Disorders |

Table continued

| Receptor | Form | Tissue Expression | Target Therapeutic Application |
|---|---|---|---|
| RORα | M | Cerebellum, Purkinje Cells | Arthritis, Cerebellar Ataxia |
| NOR-1 | M | Brain, Muscle, Heart, Adrenal, Thymus | CNS Disorders, Cancer |
| NGFI-Bβ | M/D | Brain | CNS Disorders |
| COUP-Tfα | H | Brain | CNS Disorders |
| COUP-TFβ | H | Brain | CNS Disorders |
| COUP-TFγχ | H | Brain | CNS Disorders |
| Nur77 | H | Brain, Thymus, Adrenals | CNS Disorders |
| Rev-ErbAα | H | Muscle, Brain (Ubiquitous) | Obesity |
| HNF4α | H | Liver, Kidney, Intestine | Diabetes |
| SF-1 | M | Gonads, Pituitary | Metabolic Disorders |
| LXRα,β | D | Kidney (Ubiquitous) | Metabolic Disorders |
| GCNF | M/H | Testes, Ovary | Infertility |
| ERRα,β | M | Placenta, Bone | Infertility, Osteoporosis |
| FXR | D | Liver, Kidney | Metabolic Disorders |
| CARα | H | Liver, Kidney | Metabolic Disorders |
| PXR | H | Liver, Intestine | Metabolic Disorders |

Thus methods for the treatment of NHR-associated conditions, comprising the step of administering to a subject in need thereof at least one compound of formula I in an amount effective therefor are described herein. Other therapeutic agents such as those described below may be employed with the inventive compounds in the methods described herein (for example, separately, or formulated together as a fixed dose). In the methods described herein such other therapeutic agent(s) can be administered prior to, simultaneously with or following the administration of the compound(s) of the present invention.

The present invention also provides pharmaceutical compositions comprising at least one of the compounds of the formula I capable of treating a NHR-associated condition in an amount effective therefor, and a pharmaceutically acceptable carrier (vehicle or diluent). The compositions of the present invention can contain other therapeutic agents as described below, and can be formulated, for example, by employing conventional solid or liquid vehicles or diluents, as well as pharmaceutical additives of a type appropriate to the mode of desired administration (for example, excipients, binders, preservatives, stabilizers, flavors, etc.) according to techniques such as those well known in the art of pharmaceutical formulation.

It should be noted that the compounds of the present invention are, without limitation as to their mechanism of action, useful in treating any of the conditions or disorders listed or described herein such as inflammatory diseases or cancers, or other proliferate diseases, and in compositions for treating such conditions or disorders. Such conditions and disorders include, without limitation, any of those described previously, as well as those described following such as: maintenance of muscle strength and function (e.g., in the elderly); reversal or prevention of frailty or age-related functional decline ("ARFD") in the elderly (e.g., sarcopenia); treatment of catabolic side effects of glucocorticoids; prevention and/or treatment of reduced bone mass, density or growth (e.g., osteoporosis and osteopenia); treatment of chronic fatigue syndrome (CFS); chronic malagia; treatment of acute fatigue syndrome and muscle loss following elective surgery (e.g., post-surgical rehabilitation); acceleration of wound healing; accelerating bone fracture repair (such as accelerating the recovery of hip fracture patients); accelerating healing of complicated fractures, e.g. distraction osteogenesis; in joint replacement; prevention of post-surgical adhesion formation; acceleration of tooth repair or growth; maintenance of sensory function (e.g., hearing, sight, olefaction and taste); treatment of periodontal disease; treatment of wasting secondary to fractures and wasting in connection with chronic obstructive pulmonary disease (COPD), chronic liver disease, AIDS, weightlessness, cancer cachexia, burn and trauma recovery, chronic catabolic state (e.g., coma), eating disorders (e.g., anorexia) and chemotherapy; treatment of cardiomyopathy; treatment of thrombocytopenia; treatment of growth retardation in connection with Crohn's disease; treatment of short bowel syndrome; treatment of irritable bowel syndrome; treatment of inflammatory bowel disease; treatment of Crohn's disease and ulcerative colits; treatment of complications associated with transplantation; treatment of physiological short stature including growth hormone deficient children and short stature associated with chronic illness; treatment of obesity and growth retardation associated with obesity; treatment of anorexia (e.g., associated with cachexia or aging); treatment of hypercortisolism and Cushing's syndrome; Paget's disease; treatment of osteoarthritis; induction of pulsatile growth hormone release; treatment of osteochondrodysplasias; treatment of depression, nervousness, irritability and stress; treatment of reduced mental energy and low self-esteem (e.g., motivation/assertiveness); improvement of cognitive function (e.g., the treatment of dementia, including Alzheimer's disease and short term memory loss); treatment of catabolism in connection with pulmonary dysfunction and ventilator dependency; treatment of cardiac dysfunction (e.g., associated with valvular disease, myocardial infarction, cardiac hypertrophy or congestive heart failure); lowering blood pressure; protection against ventricular dysfunction or prevention of reperfusion events; treatment of adults in chronic dialysis; reversal or slowing of the catabolic state of aging; attenuation or reversal of protein catabolic responses following trauma (e.g., reversal of the catabolic state associated with surgery, congestive heart failure, cardiac myopathy, burns, cancer, COPD etc.); reducing cachexia and protein loss due to chronic illness such as cancer or AIDS; treatment of hyperinsulinemia including nesidioblastosis; treatment of immunosuppressed patients; treatment of wasting in connection with multiple sclerosis or other neurodegenerative disorders; promotion of myelin repair; maintenance of skin thickness; treatment of metabolic homeostasis and renal homeostasis (e.g., in the frail elderly); stimulation of osteoblasts, bone remodeling and cartilage growth; regulation of food intake; treatment of insulin resistance, including NIDDM, in mammals (e.g., humans); treatment of insulin resistance in the heart; improvement of sleep quality and correction of the relative hyposomatotropism of senescence due to high increase in REM sleep and a decrease in REM latency; treatment of hypothermia; treatment of congestive heart failure; treatment of lipodystrophy (e.g., in patients taking HIV or AIDS therapies such as protease inhibitors); treatment of muscular atrophy (e.g., due to physical inactivity, bed rest or reduced weight-bearing conditions); treatment of musculoskeletal impairment (e.g., in the elderly); improvement of the overall pulmonary function; treatment of sleep disorders; and the treatment of the catabolic state of prolonged critical illness; treatment of hirsutism, acne, seborrhea, androgenic alopecia, anemia, hyperpilosity, benign prostate hypertrophy, adenomas and neoplasies of the prostate (e.g., advanced metastatic prostate cancer) and malignant tumor cells containing the androgen receptor, such as is the case for breast, brain, skin, ovarian, bladder, lymphatic, liver and kidney cancers; cancers of the skin, pancreas, endometrium, lung and colon; osteosarcoma; hypercalcemia of malignancy; metastatic bone disease; treatment of spermatogenesis, endometriosis and polycystic ovary syndrome; conteracting preeclampsia, eclampsia of pregnancy and preterm labor; treatment of premenstrual syndrome; treatment of vaginal dryness; age related decreased testosterone levels in men, male menopause, hypogonadism, male hormone replacement, male and female sexual dysfunction (e.g., erectile dysfunction, decreased sex drive, sexual well-being, decreased libido), male and female contraception, hair loss, Reaven's Syndrome and the enhancement of bone and muscle performance/strength; and the conditions, diseases, and maladies collectively referenced to as "Syndrome X" or Metabolic Syndrome as detailed in Johannsson *J. Clin. Endocrinol. Metab.*, 82, 727-34 (1997).

The present compounds have therapeutic utility in the modulation of immune cell activation/proliferation, e.g., as competitive inhibitors of intercellular ligand/receptor binding reactions involving CAMs (Cellular Adhesion Molecules) and Leukointegrins. For example, the present compounds modulate LFA-ICAM 1, and are particularly useful as LFA-ICAM 1 antagonists, and in the treatment of all conditions associated with LFA-ICAM 1 such as immunological disorders. Preferred utilities for the present compounds include, but are not limited to: inflammatory conditions such as those resulting from a response of the non-specific immune system in a mammal (e.g., adult respiratory distress syndrome, shock, oxygen toxicity, multiple organ injury syndrome secondary to septicemia, multiple organ injury syndrome secondary to trauma, reperfusion injury of tissue due to cardiopulmonary bypass, myocardial infarction or use with thrombolysis agents, acute glomerulonephritis, vasculitis, reactive arthritis, dermatosis with acute inflammatory components, stroke, thermal injury, hemodialysis, leukapheresis, ulcerative colitis, necrotizing enterocolitis and granulocyte transfusion associated syndrome) and conditions resulting from a response of the specific immune system in a mammal (e.g., psoriasis, organ/tissue transplant rejection, graft vs. host reactions and autoimmune diseases including Raynaud's syndrome, autoimmune thyroiditis, dermatitis, multiple sclerosis, rheumatoid arthritis, insulin-dependent diabetes mellitus, uveitis, inflammatory bowel disease including Crohn's disease and ulcerative colitis, and systemic lupus erythematosus). The present compounds can be used in treating asthma or as an adjunct to minimize toxicity with cytokine therapy in the treatment of cancers. The present compounds can be employed in the treatment of all diseases currently treatable through steroid therapy. The present compounds may be employed for the treatment of these and other disorders alone or with other immunosuppressive or antiinflammatory agents. A compound of the formula I can be administered prior to the onset of inflammation (so as to suppress an anticipated inflammation) or after the initiation of inflammation. When provided prophylactically, the immunosuppressive compound(s) are preferably provided in advance of any inflammatory response or symptom (for example, prior to, at, or shortly after the time of an organ or tissue transplant but in advance of any symptoms or organ rejection). The prophylactic administration of a compound of the formula I prevents or attenuates any subsequent inflammatory response (such as, for example, rejection of a transplanted organ or tissue, etc.) Administration of a compound of the formula I attenuates any actual inflammation (such as, for example, the rejection of a transplanted organ or tissue).

The compounds of the formula I can be administered for any of the uses described herein by any suitable means, for example, orally, such as in the form of tablets, capsules, granules or powders; sublingually; buccally; parenterally, such as by subcutaneous, intravenous, intramuscular, or intrasternal injection or infusion techniques (e.g., as sterile injectable aqueous or non-aqueous solutions or suspensions); nasally, including administration to the nasal membranes,

such as by inhalation spray; topically, such as in the form of a cream or ointment; or rectally such as in the form of suppositories; in dosage unit formulations containing non-toxic, pharmaceutically acceptable vehicles or diluents. The present compounds can, for example, be administered in a form suitable for immediate release or extended release. Immediate release or extended release can be achieved by the use of suitable pharmaceutical compositions comprising the present compounds, or, particularly in the case of extended release, by the use of devices such as subcutaneous implants or osmotic pumps. The present compounds can also be administered liposomally.

Exemplary compositions for oral administration include suspensions which can contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which can contain, for example, microcrystalline cellulose, dicalcium phosphate, starch, magnesium stearate and/or lactose and/or other excipients, binders, extenders, disintegrants, diluents and lubricants such as those known in the art. The compounds of formula I can also be delivered through the oral cavity by sublingual and/or buccal administration. Molded tablets, compressed tablets or freeze-dried tablets are exemplary forms which may be used. Exemplary compositions include those formulating the present compound(s) with fast dissolving diluents such as mannitol, lactose, sucrose and/or cyclodextrins. Also included in such formulations may be high molecular weight excipients such as celluloses (avicel) or polyethylene glycols (PEG). Such formulations can also include an excipient to aid mucosal adhesion such as hydroxy propyl cellulose (HPC), hydroxy propyl methyl cellulose (HPMC), sodium carboxy methyl cellulose (SCMC), maleic anhydride copolymer (e.g., Gantrez), and agents to control release such as polyacrylic copolymer (e.g. Carbopol 934). Lubricants, glidants, flavors, coloring agents and stabilizers may also be added for ease of fabrication and use.

Exemplary compositions for nasal aerosol or inhalation administration include solutions in saline which can contain, for example, benzyl alcohol or other suitable preservatives, absorption promoters to enhance bioavailability, and/or other solubilizing or dispersing agents such as those known in the art.

Exemplary compositions for parenteral administration include injectable solutions or suspensions which can contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid, or Cremaphor.

Exemplary compositions for rectal administration include suppositories which can contain, for example, a suitable non-irritating excipient, such as cocoa butter, synthetic glyceride esters or polyethylene glycols, which are solid at ordinary temperatures, but liquify and/or dissolve in the rectal cavity to release the drug.

Exemplary compositions for topical administration include a topical carrier such as Plastibase (mineral oil gelled with polyethylene).

The effective amount of a compound of the present invention can be determined by one of ordinary skill in the art, and includes exemplary dosage amounts for a adult human of from about 1 to 100 (for example, 15) mg/kg of body weight of active compound per day, which can be administered in a single dose or in the form of individual divided doses, such as from 1 to 4 times per day. It will be understood that the specific dose level and frequency of dosage for any particular subject can be varied and will depend upon a variety of factors including the activity of the specific compound employed, the metabolic stability and length of action of that compound, the species, age, body weight, general health, sex and diet of the subject, the mode and time of administration, rate of excretion, drug combination, and severity of the particular condition. Preferred subjects for treatment include animals, most preferably mammalian species such as humans, and domestic animals such as dogs, cats and the like, subject to NHR-associated conditions.

As mentioned above, the compounds of the present invention can be employed alone or in combination with each other and/or other suitable therapeutic agents useful in the treatment of NHR-associated conditions, e.g., an antibiotic or other pharmaceutically active material.

For example, the compounds of the present invention can be combined with growth promoting agents, such as, but not limited to, TRH, diethylstilbesterol, theophylline, enkephalins, E series prostaglandins, compounds disclosed in U.S. Patent No. 3,239,345, e.g., zeranol, and compounds disclosed in U.S. Patent No. 4,036,979, e.g., sulbenox or peptides disclosed in U.S. Patent No. 4,411,890.

The compounds of the invention can also be used in combination with growth hormone secretagogues such as GHRP-6, GHRP-1 (as described in U.S. Patent No. 4,411,890 and publications WO 89/07110 and WO 89/07111), GHRP-2 (as described in WO 93/04081), NN703 (Novo Nordisk), LY444711 (Lilly), MK-677 (Merck), CP424391 (Pfizer) and B-HT920, or with growth hormone releasing factor and its analogs or growth hormone and its analogs or somatomedins including IGF-1 and IGF-2, or with alpha-adrenergic agonists, such as clonidine or serotinin 5-$HT_D$ agonists, such as sumatriptan, or agents which inhibit somatostatin or its release, such as physostigmine and pyridostigmine. A still further use of the disclosed compounds of the invention is in combination with parathyroid hormone, PTH(1-34) or bisphosphonates, such as MK-217 (alendronate).

A still further use of the compounds of the invention is in combination with estrogen, testosterone, a selective estrogen receptor modulator, such as tamoxifen or raloxifene, or other androgen receptor modulators, such as those disclosed in Edwards, J. P., et al., *Bio. Med. Chem. Let.* 9,1003-1008 (1999) and Hamann, L. G., et al., *J. Med. Chem.*,

42, 210-212 (1999).

A further use of the compounds of this invention is in combination with progesterone receptor agonists ("PRA"), such as levonorgestrel, medroxyprogesterone acetate (MPA).

The compounds of the present invention can be employed alone or in combination with each other and/or other modulators of nuclear hormone receptors or other suitable therapeutic agents useful in the treatment of the aforementioned disorders including: anti-diabetic agents; anti-osteoporosis agents; anti-obesity agents; anti-inflammatory agents; anti-anxiety agents; anti-depressants; anti-hypertensive agents; anti-platelet agents; anti-thrombotic and thrombolytic agents; cardiac glycosides; cholesterol/lipid lowering agents; mineralocorticoid receptor antagonists; phosphodiesterase inhibitors; protein tyrosine kinase inhibitors; thyroid mimetics (including thyroid receptor agonists); anabolic agents; HIV or AIDS therapies; therapies useful in the treatment of Alzheimer's disease and other cognitive disorders; therapies useful in the treatment of sleeping disorders; anti-proliferative agents; and anti-tumor agents.

Examples of suitable anti-diabetic agents for use in combination with the compounds of the present invention include biguanides (e.g., metformin), glucosidase inhibitors (e.g,. acarbose), insulins (including insulin secretagogues or insulin sensitizers), meglitinides (e.g., repaglinide), sulfonylureas (e.g., glimepiride, glyburide and glipizide), biguanide/glyburide combinations (e.g., Glucovance®), thiazolidinediones (e.g., troglitazone, rosiglitazone and pioglitazone), PPAR-alpha agonists, PPAR-gamma agonists, PPAR alpha/gamma dual agonists, SGLT2 inhibitors, glycogen phosphorylase inhibitors, inhibitors of fatty acid binding protein (aP2) such as those disclosed in U.S. Patent No.6,548,529 filed March 6, 2000, glucagon-like peptide-1 (GLP-1), and dipeptidyl peptidase IV (DP4) inhibitors.

Examples of suitable anti-osteoporosis agents for use in combination with the compounds of the present invention include alendronate, risedronate, PTH, PTH fragment, raloxifene, calcitonin, steroidal or non-steroidal progesterone receptor agonists, RANK ligand antagonists, calcium sensing receptor antagonists, TRAP inhibitors, selective estrogen receptor modulators (SERM), estrogen and AP-1 inhibitors.

Examples of suitable anti-obesity agents for use in combination with the compounds of the present invention include aP2 inhibitors, such as those disclosed in U.S. Patent No. 6,548,529 filed March 6, 2000, PPAR gamma antagonists, PPAR delta agonists, beta 3 adrenergic agonists, such as AJ9677 (Takeda/Dainippon), L750355 (Merck), or CP331648 (Pfizer) or other known beta 3 agonists as disclosed in U.S. Patent Nos. 5,541,204, 5,770,615, 5,491,134, 5,776,983 and 5,488,064, a lipase inhibitor, such as orlistat or ATL-962 (Alizyme), a serotonin (and dopamine) reuptake inhibitor, such as sibutramine, topiramate (Johnson & Johnson) or axokine (Regeneron), a thyroid receptor beta drug, such as a thyroid receptor ligand as disclosed in WO 97/21993 (U. Cal SF), WO 99/00353 (KaroBio) and GB98/284425 (KaroBio), and/or an anorectic agent, such as dexamphetamine, phentermine, phenylpropanolamine or mazindol.

Examples of suitable anti-inflammatory agents for use in combination with the compounds of the present invention include prednisone, dexamethasone, Enbrel®, cyclooxygenase inhibitors (i.e., COX-1 and/or COX-2 inhibitors such as NSAIDs, aspirin, indomethacin, ibuprofen, piroxicam, Naproxen®, Celebrex®, Vioxx®), CTLA4-Ig agonists/antagonists, CD40 ligand antagonists, IMPDH inhibitors, such as mycophenolate (CellCept®), integrin antagonists, alpha-4 beta-7 integrin antagonists, cell adhesion inhibitors, interferon gamma antagonists, ICAM-1, tumor necrosis factor (TNF) antagonists (e.g., infliximab, OR1384), prostaglandin synthesis inhibitors, budesonide, clofazimine, CNI-1493, CD4 antagonists (e.g., priliximab), p38 mitogen-activated protein kinase inhibitors, protein tyrosine kinase (PTK) inhibitors, IKK inhibitors, and therapies for the treatment of irritable bowel syndrome (e.g., Zelmac® and Maxi-K® openers such as those disclosed in U.S. Patent No. 6,184,231 B1).

Example of suitable anti-anxiety agents for use in combination with the compounds of the present invention include diazepam, lorazepam, buspirone, oxazepam, and hydroxyzine pamoate.

Examples of suitable anti-depressants for use in combination with the compounds of the present invention include citalopram, fluoxetine, nefazodone, sertraline, and paroxetine.

Examples of suitable anti-hypertensive agents for use in combination with the compounds of the present invention include beta adrenergic blockers, calcium channel blockers (L-type and T-type; e.g. diltiazem, verapamil, nifedipine, amlodipine and mybefradil), diuretics (e.g., chlorothiazide, hydrochlorothiazide, flumethiazide, hydroflumethiazide, bendroflumethiazide, methylchlorothiazide, trichloromethiazide, polythiazide, benzthiazide, ethacrynic acid tricrynafen, chlorthalidone, furosemide, musolimine, bumetanide, triamferene, amiloride, spironolactone), renin inhibitors, ACE inhibitors (e.g., captopril, zofenopril, fosinopril, enalapril, ceranopril, cilazopril, delapril, pentopril, quinapril, ramipril, lisinopril), AT-1 receptor antagonists (e.g., losartan, irbesartan, valsartan), ET receptor antagonists (e.g., sitaxsentan, atrsentan and compounds disclosed in U.S. Patent Nos. 5,612,359 and 6,043,265), Dual ET/AII antagonist (e.g., compounds disclosed in WO 00/01389), neutral endopeptidase (NEP) inhibitors, vasopepsidase inhibitors (dual NEP-ACE inhibitors) (e.g., omapatrilat and gemopatrilat), and nitrates.

Examples of suitable anti-platelet agents for use in combination with the compounds of the present invention include GPIIb/IIIa blockers (e.g., abciximab, eptifibatide, tirofiban), P2Y12 antagonists (e.g., clopidogrel, ticlopidine, CS-747), thromboxane receptor antagonists (e.g., ifetroban), aspirin, and PDE-III inhibitors (e.g., dipyridamole) with or without aspirin.

Examples of suitable cardiac glycosides for use in combination with the compounds of the present invention

include digitalis and ouabain.

Examples of suitable cholesterol/lipid lowering agents for use in combination with the compounds of the present invention include HMG-CoA reductase inhibitors (e.g., pravastatin, lovastatin, atorvastatin, simvastatin, NK-104 (a.k.a. itavastatin, or nisvastatin or nisbastatin) and ZD-4522 (a.k.a. rosuvastatin, or atavastatin or visastatin)), squalene synthetase inhibitors, fibrates, bile acid sequestrants, ACAT inhibitors, MTP inhibitors, lipooxygenase inhibitors, cholesterol absorption inhibitors, and cholesterol ester transfer protein inhibitors (e.g., CP-529414).

Examples of suitable mineralocorticoid receptor antagonists for use in combination with the compounds of the present invention include spironolactone and eplerinone.

Examples of suitable phospodiesterase inhibitors for use in combination with the compounds of the present invention include PDE-III inhibitors such as cilostazol, and PDE-V inhibitors such as sildenafil.

Examples of suitable thyroid mimetics for use in combination with the compounds of the present invention include thyrotropin, polythyroid, KB-130015, and dronedarone.

Examples of suitable anabolic agents for use in combination with the compounds of the present invention include testosterone, TRH diethylstilbesterol, estrogens, β-agonists, theophylline, anabolic steroids, dehydroepiandrosterone, enkephalins, E-series prostaglandins, retinoic acid and compounds as disclosed in U.S. Pat. No. 3,239,345, e.g., Zeranol®; U.S. Patent No. 4,036,979, e.g., Sulbenox® or peptides as disclosed in U.S. Pat. No. 4,411,890.

Examples of suitable HIV or AIDS therapies for use in combination with the compounds of the present invention include indinavir sulfate, saquinavir, saquinavir mesylate, ritonavir, lamivudine, zidovudine, lamivudine/zidovudine combinations, zalcitabine, didanosine, stavudine, and megestrol acetate.

Examples of suitable therapies for treatment of Alzheimer's disease and cognitive disorders for use in combination with the compounds of the present invention include donepezil, tacrine, revastigmine, 5HT6, gamma secretase inhibitors, beta secretase inhibitors, SK channel blockers, Maxi-K blockers, and KCNQs blockers.

Examples of suitable therapies for treatment of sleeping disorders for use in combination with the compounds of the present invention include melatonin analogs, melatonin receptor antagonists, ML1B agonists, and GABA/NMDA receptor antagonists.

Examples of suitable anti-proliferative agents for use in combination with the compounds of the present invention include cyclosporin A, paclitaxel, FK 506, and adriamycin.

Examples of suitable anti-tumor agents for use in combination with the compounds of the present invention include paclitaxel, adriamycin, epothilones, cisplatin and carboplatin.

Compounds of the present invention can further be used in combination with nutritional supplements such as those described in U.S. 5,179,080, especially in combination with whey protein or casein, amino acids (such as leucine, branched amino acids and hydroxymethylbutyrate), triglycerides, vitamins (e.g., A, $B_6$, $B_{12}$, folate, C, D and E), minerals (e.g., selenium, magnesium, zinc, chromium, calcium and potassium), carnitine, lipoic acid, creatine, and coenzyme Q-10.

In addition, compounds of the present invention can be used in combination with therapeutic agents used in the treatment of sexual dysfunction, including but not limited to PDE5 inhibitors, such as sildenafil or IC-351; with an antiresorptive agent, hormone replacement therapies, vitamin D analogues, calcitonins, elemental calcium and calcium supplements, cathepsin K inhibitors, MMP inhibitors, vitronectin receptor antagonists, Src $SH_2$ antagonists, vacular -$H^+$-ATPase inhibitors, progesterone receptor agonists, ipriflavone, fluoride, RANK antagonists, PTH and its analogues and fragments, Tibolone, HMG-CoA reductase inhibitors, SERM's, p38 inhibitors, prostanoids, 17-beta hydroxysteroid dehydrogenase inhibitors and Src kinase inhibitors.

Compounds of the present invention can be used in combination with male contraceptives, such as nonoxynol 9 or therapeutic agents for the treatment of hair loss, such as minoxidil and finasteride or chemotherapeutic agents, such as with LHRH agonists.

For their preferred anticancer or antiangiogenic use, the compounds of the present invention can be administered either alone or in combination with other anti-cancer and cytotoxic agents and treatments useful in the treatment of cancer or other proliferative diseases, for example, where the second drug has the same or different mechanism of action than the present compounds of formula I. Examples of classes of anti-cancer and cytotoxic agents useful in combination with the present compounds include but are not limited to: alkylating agents such as nitrogen mustards, alkyl sulfonates, nitrosoureas, ethylenimines, and triazenes; antimetabolites such as folate antagonists, purine analogues, and pyrimidine analogues; antibiotics such as anthracyclines, bleomycins, mitomycin, dactinomycin, and plicarnycin; enzymes such as L-asparaginase; famesyl-protein transferase inhibitors; 5α reductase inhibitors; inhibitors of 17β-hydroxy steroid dehydrogenase type 3; hormonal agents such as glucocorticoids, estrogens/ antiestrogens, androgens/ antiandrogens, progestins, and luteinizing hormone-releasing hormone antagonists, octreotide acetate; microtubule-disruptor agents, such as ecteinascidins or their analogs and derivatives; microtubule-stabilizing agents such as taxanes, for example, paclitaxel (Taxol®), docetaxel (Taxotere®), and their analogs, and epothilones, such as epothilones A-F and their analogs; plant-derived products, such as vinca alkaloids, epipodophyllotoxins, taxanes; and topo isomerase inhibitors; prenyl-protein transferase inhibitors; and miscellaneous agents such as hydroxyurea, procarbazine, mitotane, hexamethylmelamine, platinum coordination complexes such as cisplatin and carboplatin; and other agents used as

anti-cancer and cytotoxic agents such as biological response modifiers, growth factors; immune modulators and monoclonal antibodies. The compounds of the invention may also be used in conjunction with radiation therapy.

Representative examples of these classes of anti-cancer and cytotoxic agents include but are not limited to mechlorethamine hydrochloride, cyclophosphamide, chlorambucil, melphalan, ifosfamide, busulfan, carmustin, lomustine, semustine, streptozocin, thiotepa, dacarbazine, methotrexate, thioguanine, mercaptopurine, fludarabine, pentastatin, cladribin, cytarabine, fluorouracil, doxorubicin hydrochloride, daunorubicin, idarubicin, bleomycin sulfate, mitomycin C, actinomycin D, safracins, saframycins, quinocarcins, discodermolides, vincristine, vinblastine, vinorelbine tartrate, etoposide, etoposide phosphate, teniposide, paclitaxel, tamoxifen, estramustine, estramustine phosphate sodium, flutamide, buserelin, leuprolide, pteridines, diyneses, levamisole, aflacon, interferon, interleukins, aldesleukin, filgrastim, sargramostim, rituximab, BCG, tretinoin, irinotecan hydrochloride, betamethasone, gemcitabine hydrochloride, altretamine, and topoteca and any analogs or derivatives thereof.

Preferred members of these classes include, but are not limited to, paclitaxel, cisplatin, carboplatin, doxorubicin, carminomycin, daunorubicin, aminopterin, methotrexate, methopterin, mitomycin C, ecteinascidin 743, or porfiromycin, 5-fluorouracil, 6-mercaptopurine, gemcitabine, cytosine arabinoside, podophyllotoxin or podophyllotoxin derivatives such as etoposide, etoposide phosphate or teniposide, melphalan, vinblastine, vincristine, leurosidine, vindesine and leurosine.

Examples of anticancer and other cytotoxic agents include the following: epothilone derivatives as found in German Patent No. 4138042.8; WO 97/19086, WO 98/22461, WO 98/25929, WO 98/38192, WO 99/01124, WO 99/02224, WO 99/02514, WO 99/03848, WO 99/07692, WO 99/27890, WO 99/28324, WO 99/43653, WO 99/54330, WO 99/54318, WO 99/54319, WO 99/65913, WO 99/67252, WO 99/67253 and WO 00/00485; cyclin dependent kinase inhibitors as found in WO 99/24416 (see also U.S. Patent No. 6,040,321); and prenyl-protein transferase inhibitors as found in WO 97/30992 and WO 98/54966; and agents such as those described generically and specifically in U.S. Patent No. 6,011,029 (the compounds of which U.S. Patent can be employed together with any NHR modulators (including, but not limited to, those of present invention) such as AR modulators, ER modulators, with LHRH modulators, or with surgical castration, especially in the treatment of cancer).

The pharmaceutical compositions of the present invention can also be formulated or co-administered with other therapeutic agents that are selected for their particular usefulness in administering therapies associated with the aforementioned conditions. For example, the compounds of the invention may be formulated with agents to prevent nausea, hypersensitivity and gastric irritation, such as antiemetics, and H, and $H_2$ antihistaminics.

As it pertains to the treatment of cancer, the compounds of this invention are most preferably used alone or in combination with anti-cancer treatments such as radiation therapy and/or with cytostatic and/or cytotoxic agents, such as, but not limited to, DNA interactive agents, such as cisplatin or doxorubicin; inhibitors of farnesyl protein transferase, such as those described in U.S. Patent No. 6,011,029; topoisomerase II inhibitors, such as etoposide; topoisomerase I inhibitors, such as CPT-11 or topotecan; tubulin stabilizing agents, such as paclitaxel, docetaxel, other taxanes, or epothilones; hormonal agents, such as tamoxifen; thymidilate synthase inhibitors, such as 5-fluorouracil; antimetabolites, such as methoxtrexate; antiangiogenic agents, such as angiostatin, ZD6474, ZD6126 and comberstatin A2; kinase inhibitors, such as her2 specific antibodies, Iressa and CDK inhibitors; histone deacetylase inhibitors, such as CI-994 and MS-27-275. Such compounds may also be combined with agents which suppress the production of circulating testosterone such as LHRH agonists or antagonists or with surgical castration.

For example, known therapies for advanced metastatic prostate cancer include "complete androgen ablation therapy" wherein tumor growth is inhibited by controlling the supply of androgen to the prostate tissues via chemical castration (castration serves to inhibit the production of circulating testosterone (T) and dihydrotestosterone (DHT)) followed by the administration of androgen receptor (AR) antagonists (which inhibit the function of T/DHT derived from the conversion of circulating androgen precursors to T/DHT by the prostate tissue). The compounds of the present invention can be employed as AR antagonists in complete ablation therapy, alone or in combination with other AR antagonists such as Flutamide, Casodex, Nilutamide, or Cyproterone acetate.

The compounds of the present invention may be employed adjuvant to surgery.

Another application of the present compounds is in combination with antibody therapy such as but not limited to antibody therapy against PSCA. An additional application is in concert with vaccine / immune modulating agents for the treatment of cancer.

Compounds of the present invention can be employed in accordance with the methods described in U.S. Provisional Patent Application Serial No. 60/284,438, entitled "Selective Androgen Receptor Modulators and Methods for Their Identification, Design and Use" filed April 18, 2001 by Mark E. Salvati *et al*. (Attorney Docket No. LD0250(PSP)), which Provisional Patent Application is incorporated herein by reference in its entirety (including, but not limited to, reference to all specific compounds within formula I of the present invention), and U.S. Patent Application Serial No. __________________(unassigned), entitled "Selective Androgen Receptor Modulators and Methods for Their Identification, Design and Use" filed June 20, 2001 by Mark E. Salvati *et al*. (Attorney Docket No. LD0250(NP)), which Patent Application is incorporated herein by reference in its entirety (including, but not limited to, reference to all specific compounds within formula I of the present invention).

For racemates of compounds of the present invention, one enantiomer can, for example be a full AR antagonist while the other can be an AR antagonist in tumor tissue while having no activity or agonist activity in nontumor tissue containing the androgen receptor.

The above other therapeutic agents, when employed in combination with the compounds of the present invention, can be used, for example, in those amounts indicated in the Physicians' Desk Reference (PDR) or as otherwise determined by one of ordinary skill in the art.

The following assays can be employed in ascertaining the activity of a compound as a NHR modulator. Preferred are those compounds with an activity greater than 20μm for binding or transactivation in any of these assays. Various compounds of the present invention were determined to have AR modulator activity utilizing the transactivation assay, and standard AR binding assays as described following.

**Transactivation Assays:**

**AR Specific Assay:**

Compounds of the present invention were tested in transactivation assays of a transfected reporter construct and using the endogenous androgen receptor of the host cells. The transactivation assay provides a method for identifying functional agonists and partial agonists that mimic, or antagonists that inhibit, the effect of native hormones, in this case, dihydrotestosterone (DHT). This assay can be used to predict *in vivo* activity as there is a good correlation in both series of data. See, e.g. T. Berger et al., *J. Steroid Biochem. Molec. Biol.* 773 (1992), the disclosure of which is herein incorporated by reference.

For the transactivation assay a reporter plasmid is introduced by transfection (a procedure to induce cells to take foreign genes) into the respective cells. This reporter plasmid, comprising the cDNA for a reporter protein, such as secreted alkaline phosphatase (SEAP), is controlled by prostate specific antigen (PSA) upstream sequences containing androgen response elements (AREs). This reporter plasmid functions as a reporter for the transcription-modulating activity of the AR. Thus, the reporter acts as a surrogate for the products (mRNA then protein) normally expressed by a gene under control of the AR and its native hormone. In order to detect antagonists, the transactivation assay is carried out in the presence of constant concentration of the natural AR hormone (DHT) known to induce a defined reporter signal. Increasing concentrations of a suspected antagonist will decrease the reporter signal (e.g., SEAP production). On the other hand, exposing the transfected cells to increasing concentrations of a suspected agonist will increase the production of the reporter signal.

For this assay, LNCaP and MDA 453 cells were obtained from the American Type Culture Collection (Rockville, MD), and maintained in RPMI 1640 or DMEM medium supplemented with 10% fetal bovine serum (FBS; Gibco) respectively. The respective cells were transiently transfected by electroporation according to the optimized procedure described by Heiser, 130 Methods Mol. Biol. 117 (2000), with the pSEAP2/PSA540/Enhancer reporter plasmid. The reporter plasmid, was constructed as follows: commercial human placental genomic DNA was used to generate by Polymerase Cycle Reaction (PCR) a fragment containing the BglII site (position 5284) and the Hind III site at position 5831 of the human prostate specific antigen promoter (Accession # U37672), Schuur et al., *J. Biol. Chem.* **271** (12): 7043-51 (1996). This fragment was subcloned into the pSEAP2/basic (Clontech) previously digested with BglII and HindIII to generate the pSEAP2/PSA540 construct. Then a fragment bearing the fragment of human PSA upstream sequence between positions -5322 and -3873 was amplified by PCR from human placental genomic DNA. A XhoI and a BglII sites were introduced with the primers. The resulting fragment was subcloned into pSEAP2/PSA540 digested with XhoI and BglII respectively, to generate the pSEAP2/PSA540/Enhancer construct. LNCaP and MDA 453 cells were collected in media containing 10% charcoal stripped FBS. Each cell suspension was distributed into two Gene Pulser Cuvetts (Bio-Rad) which then received 8 μg of the reporter construct, and electroporated using a Bio-Rad Gene Pulser at 210 volts and 960 μFaraday. Following the transfections the cells were washed and incubated with media containing charcoal stripped fetal bovine serum in the absence (blank) or presence (control) of 1 nM dihydrotestosterone (DHT; Sigma Chemical) and in the presence or absence of the standard anti-androgen bicalutamide or compounds of the present invention in concentrations ranging from $10^{-10}$ to $10^{-5}$ M (sample). Duplicates were used for each sample. The compound dilutions were performed on a Biomek 2000 laboratory workstation. After 48 hours, a fraction of the supernatant was assayed for SEAP activity using the Phospha-Light Chemiluminescent Reporter Gene Assay System (Tropix, Inc). Viability of the remaining cells was determined using the CellTiter 96 Aqueous NonRadioactive Cell Proliferation Assay (MTS Assay, Promega). Briefly, a mix of a tetrazolium compound (3-(4,5-dimethylthiazol-2-yl)-5-(3-carboxymethoxyphenyl)-2-(4-sulfophenyl)-2H-tetrazolium, inner salt; MTS) and an electron coupling reagent (phenazine methosulfate; PMS) are added to the cells. MTS (Owen's reagent) is bioreduced by cells into a formazan that is soluble in tissue culture medium, and therefore its absorbance at 490mn can be measured directly from 96 well assay plates without additional processing. The quantity of formazan product as measured by the amount of 490nm absorbance is directly proportional to the number of living cells in culture. For each replicate the SEAP reading was normalized by the Abs490 value derived from the

MTS assay. For the antagonist mode, the % Inhibition was calculated as:

$$\% \text{ Inhibition} = 100 \times ( 1 - [\text{average control} - \text{average blank} / \text{average sample} - \text{average blank} ])$$

Data was plotted and the concentration of compound that activates to levels 50% of the normalized SEAP for the control was quantified ($EC_{50}$).

For the aganist mode % Control was referred as the effect of the tested compound compared to the maximal effect observed with the natural hormone, in this case DHT, and was calculated as

$$\% \text{ Control} = 100 \times \text{average sample} - \text{average blank} / \text{average control} - \text{average blank}$$

Data was plotted and the concentration of compound that activates to levels 50% of the normalized SEAP for the control was quantified ($EC_{50}$).

**GR Specificity Assay:**

The reporter plasmid utilized was comprised of the cDNA for the reporter SEAP protein, as described for the AR specific transactivation assay. Expression of the reporter SEAP protein was controlled by the mouse mammary tumor virus long terminal repeat (MMTV LTR) sequences that contain three hormone response elements (HREs) that can be regulated by both GR and PR see, e.g. G. Chalepakis et al., Cell **53**(3), 371 (1988). This plasmid was transfected into A549 cells, which expresses endogenous GR, to obtain a GR specific transactivation assay. A549 cells were obtained from the American Type Culture Collection (Rockville, MD), and maintained in RPMI 1640 supplemented with 10% fetal bovine serum (FBS; Gibeo). Determination of the GR specific antagonist activity of the compounds of the present invention was identical to that described for the AR specific transactivation assay, except that the DHT was replaced with 5 nM dexamethasone (Sigma Chemicals), a specific agonist for GR. Determination of the GR specific agonist activity of the compounds of the present invention was performed as described for the AR transactivation assay, wherein one measures the activation of the GR specific reporter system by the addition of a test compound, in the absence of a known GR specific agonists ligand.

**PR Specific Assay:**

The reporter plasmid utilized was comprised of the cDNA for the reporter SEAP protein, as described for the AR specific transactivation assay. Expression of the reporter SEAP protein was controlled by the mouse mammary tumor virus long terminal repeat (MMTV LTR) sequences that contain three hormone response elements (HREs) that can be regulated by both GR and PR. This plasmid was transfected into T47D, which expresses endogenous PR, to obtain a PR specific transactivation assay. T47D cells were obtained from the American Type Culture Collection (Rockville, MD), and maintained in DMEM medium supplemented with 10% fetal bovine serum (FBS; Gibco). Determination of the PR specific antagonist activity of the compounds of the present invention was identical to that described for the AR specific transactivation assay, except that the DHT was replaced with 1 nM Promegastone (NEN), a specific agonist for PR. Determination of the PR specific agonist activity of the compounds of the present invention was performed as described for the AR transactivation assay, wherein one measures the activation of the PR specific reporter system by the addition of a test compound, in the absence of a known PR specific agonists ligand.

**AR Binding Assay:**

For the whole cell binding assay, human LNCaP cells (T877A mutant AR) or MDA 453 (wild type AR) in 96-well microtiter plates containing RPMI 1640 or DMEM supplemented with 10% charcoal stripped CA-FBS (Cocaleco Biologicals) respectively, were incubated at 37°C to remove any endogenous ligand that might be complexed with the receptor in the cells. After 48 hours, either a saturation analysis to determine the $K_d$ for tritiated dihydrotestosterone, [$^3$H]-DHT, or a competitive binding assay to evaluate the ability of test compounds to compete with [$^3$H]-DHT was performed. For the saturation analysis, media (RPMI 1640 or DMEM - 0.2% CA-FBS) containing [$^3$H]-DHT (in concentrations ranging from 0.1 nM to 16 nM) in the absence (total binding) or presence (non-specific binding) of a 500-fold molar excess of unlabeled DHT were added to the cells. After 4 hours at 37°C, an aliquot of the total binding media at each concentration

of [$^3$H]-DHT was removed to estimate the amount of free [$^3$H]-DHT. The remaining media was removed, cells were washed three times with PBS and harvested onto UniFilter GF/B plates (Packard), Microscint (Packard) was added and plates counted in a Top-Counter (Packard) to evaluate the amount of bound [$^3$H]-DHT.

For the saturation analysis, the difference between the total binding and the non-specific binding, was defined as specific binding. The specific binding was evaluated by Scatchard analysis to determine the $K_d$ for [$^3$H]-DHT. See e.g. D. Rodbard, Mathematics and statistics of ligand assays: an illustrated guide: In: J. Langon and J. J. Clapp, eds., Ligand Assay, Masson Publishing U.S.A., Inc., New York, pp. 45-99 (1981), the disclosure of which is herein incorporated by reference.

For the competition studies, media containing 1 nM ($^3$H)-DHT and compounds of the invention ("test compounds") in concentrations ranging from $10^{-10}$ to $10^{-5}$ M were added to the cells. Two replicates were used for each sample. After 4 hours at 37°C, cells were washed, harvested and counted as described above. The data were plotted as the amount of [$^3$H]-DHT (% of control in the absence of test compound) remaining over the range of the dose response curve for a given compound. The concentration of test compound that inhibited 50% of the amount of [$^3$H]-DHT bound in the absence of competing ligand was quantified ($IC_{50}$) after log-logit transformation. The $K_1$ values were determined by application of the Cheng-Prusoff equation to the $IC_{50}$ values, where:

$$K_I = \frac{IC_{50}}{(1 + (^3\text{H-DHT}) / K_d \text{ for } ^3\text{H-DHT})}.$$

After correcting for non-specific binding, $IC_{50}$ values were determined. The $IC_{50}$ is defined as the concentration of competing ligand needed to reduce specific binding by 50%. The $K_d$s for [$^3$H]-DHT for MDA 453 and LNCaP were 0.7 and 0.2 nM respectively.

**Human Prostate Cell Proliferation Assay:**

Compounds of the present invention were tested ("test compounds") on the proliferation of human prostate cancer cell lines. For that, MDA PCa2b cells, a cell line derived from the metastasis of a patient that failed castration, Navone et al., *Clin. Cancer Res.* **3**, 2493-500 (1997), were incubated with or without the test compounds for 72 hours and the amount of [$^3$H]-thymidine incorporated into DNA was quantified as a way to assess number of cells and therefore proliferation. The MDA PCa2b cell line was maintained in BRFF-HPC1 media (Biological Research Faculty & Facility Inc., MD) supplemented with 10% FBS. For the assay, cells were plated in Biocoated 96-well microplates and incubated at 37°C in 10% FBS (charcoal-stripped)/BRFF-BMZERO (without androgens). After 24 hours, the cells were treated in the absence (blank) or presence of 1 nM DHT (control) or with test compounds (sample) of the present invention in concentrations ranging from $10^{-10}$ to $10^{-5}$ M. Duplicates were used for each sample. The compound dilutions were performed on a Biomek 2000 laboratory work station. Seventy two hours later 0.44 μCi of [$^3$H]-Thymidine (Amersham) were added per well and incubated for another 24 h followed by tripsinization, harvesting of the cells onto GF/B filters. Micro-scint PS was added to the filters before counting them on a Beckman TopCount.

The % Inhibition was calculated as:

$$\%\ \text{Inhibition} = 100 \times (1 - [\text{average}_{\text{control}} - \text{average}_{\text{blank}} / \text{average}_{\text{sample}} - \text{average}_{\text{blank}}])$$

Data were plotted and the concentration of compound that inhibited 50% of the [$^3$H]-Thymidine incorporation was quantified ($IC_{50}$).

**C2C12 Mouse Myoblast Transactivation Assay:**

Two functional transactivation assays were developed to assess the efficacy of androgen agonists in a muscle cell background using a luciferase reporter. The first assay (ARTA Stable 1) uses a cell line, Stable 1 (clone #72), which stably expresses the full length rat androgen receptor but requires the transient transfection of an enhancer/reporter. This cell line was derived from C2C12 mouse myoblast cells. The second assay (ARTA Stable 2) uses a cell line, Stable 2 (clone #133), derived from Stable 1 which stably expresses both rAR and the enhancer/lucifeiase reporter.

The enhancer/reporter construct used in this system is pGL3/2XDR-1/luciferase. 2XDR-1 was reported to be an AR specific response element in CV-1 cells, Brown et. al., *The Journal of Biological Chemistry* **272**, 8227-8235

(1997). It was developed by random mutagenesis of an AR/GR consensus enhancer sequence.

**ARTA Stable 1:**

1. Stable 1 cells are plated in 96 well format at 6,000 cells/well in high glucose DMEM without phenol red (Gibco BRL, Cat. No.: 21063-029) containing 10% charcoal and dextran treated FBS (HyClone Cat. No.: SH30068.02), 50 mM HEPES Buffer (Gibco BRL, Cat. No.: 15630-080), 1x MEM Na Pyruvate (Gibco BRL, Cat. No.: 11360-070), 0.5x Antibiotic-Antimycotic, and 800 μg/ml Geneticin (Gibco BRL, Cat. No.: 10131-035).
2. 48 hours later, cells are transfected with pGL3/2XDR-1/luciferase using LipofectAMINE Plus™ Reagent (Gibco BRL, Cat. No.: 10964-013). Specifically, 5 ng/well pGL3/2XDR-1/luciferase DNA and 50 ng/well Salmon Sperm DNA (as carrier) are diluted with 5 μl/well Opti-MEMem media (Gibco BRL, Cat No.: 31985-070). To this, 0.5 μl/well Plus reagent are added. This mixture is incubated for 15 minutes at room temperature. In a separate vessel, 0.385 μl/well LipofectAMINE reagent are diluted with 5 μl/well Opti-MEM. The DNA mixture is then combined with the LipofectAMINE mixture and incubated for an additional 15 minutes at room temperature. During this time, the media from the cells are removed and replaced with 60 μl/well of Opti-MEM. To this are added 10 μl/well of the DNA/LipofectAMINE transfection mixture. The cells are incubated for 4 hours.
3. The transfection mixture is removed from the cells and replaced with 90 μl of media as in #1 above.
4. 10 μl/well of appropriate drug dilution are placed in each well.
5. 24 hours later, the Steady-Glo™ Luciferase Assay System is used to detect activity according to the manufacturer's instructions (Promega, Cat. No.: E2520).

**ARTA stable 2:**

1. Stable 2 cells are plated in 96 well format at 6,000 cells/well in high glucose DMEM without phenol red (Gibco BRL, Cat. No.: 21053-029) containing 10% charcoal and dextran treated FBS (HyClone Cat. No.: SH30068.02), 50 mM HEPES Buffer (Gibco BRL, Cat. No.: 15630-080), 1x MEM Na Pyruvate (Gibco BRL, Cat. No.: 11360-070), 0.5x Antibiotic-Antimycotic, 800 μg/ml Geneticin (Gibco BRL, Cat. No.: 10131-035) and 800 μg/ml Hygromycin β (Gibco BRL, Cat. No.: 10687-010).
2. 48 hours later, the media on the cells are removed and replaced with 90 μl fresh. 10 μl/well of appropriate drug dilution are placed in each well.
3. 24 hours later, the Steady-GloTM Luciferase Assay System is used to detect activity according to the manufacturer's instructions (Promega, Cat. No.: E2520).

See U.S. Patent Application Serial No. __________________ (unassigned), entitled "Cell Lines and Cell-BasedAssays for Identification of Androgen Receptor Modulators" filed June 20, 2001 by Jacek Ostrowski *et al.* (Attorney Docket No. D0177), which Patent Application is incorporated herein by reference in its entirety.

**Proliferation Assays**

**Murine Breast Cell Proliferation Assay:**

The ability of compounds of the present invention ("test compounds") to modulate the function of the AR was determined by testing said compounds in a proliferation assay using the androgen responsive murine breast cell line derived from the Shionogi tumor, Hiraoka *et al., Cancer Res.* **47**, 6560-6564 (1987). Stable AR dependent clones of the parental Shionogi line were established by passing tumor fragments under the general procedures originally described in Tetuo *et al., Cancer Research* **25**, 1168-1175 (1965). From the above procedure, one stable line, SC114, was isolated, characterized and utilized for the testing of example compounds. SC114 cells were incubated with or without the test compounds for 72 hours and the amount of [$^3$H]-thymidine incorporated into DNA was quantified as a surrogate endpoint to assess the number of cells and therefore the proliferation rate as described in Suzuki *et al., J. Steroid Biochem. Mol. Biol.* **37**, 559-567 (1990). The SC114 cell line was maintained in MEM containing $10^{-8}$ M testosterone and 2% DCC-treated FCS. For the assay, cells were plated in 96-well microplates in the maintenance media and incubated at 37°C. On the following day, the medium was changed to serum free medium [Ham's F-12:MEM (1:1, v/v) containing 0.1% BSA] with (antagonist mode) or without (agonist mode) $10^{-8}$ M testosterone and the test compounds of the present invention in concentrations ranging from $10^{-10}$ to $10^{-5}$ M. Duplicates were used for each sample. The compound dilutions were

performed on a Biomek 2000 laboratory work station. Seventy two hours later 0.44μCi of [$^3$H]-Thymidine (Amersham) were added per well and incubated for another 2 hr followed by tripsinization, and harvesting of the cells onto GF/B filters. Micro-scint PS was added to the filters before counting them on a Beckman TopCount. For the antagonist mode, the % Inhibition was calculated as:

$$\% \text{ Inhibition} = 100 \times (1 - [\text{average}_{\text{sample}} - \text{average}_{\text{blank}} / \text{average}_{\text{control}} - \text{average}_{\text{blank}}])$$

Data were plotted and the concentration of compound that inhibited 50% of the [$^3$H]-Thymidine incorporation was quantified ($IC_{50}$).

For the agonist mode % Control was referred as the effect of the tested compound compared to the maximal effect observed with the natural hormone, in this case DHT, and was calculated as:

$$\% \text{ Control} = 100 \times (\text{average}_{\text{sample}} - \text{average}_{\text{blank}}) / (\text{average}_{\text{control}} - \text{average}_{\text{blank}})$$

Data were plotted and the concentration of compound that inhibited 50% of the [$^3$H]-Thymidine incorporation was quantified ($EC_{50}$).

***In Vitro* Assay to Measure GR Induced AP-1 Transrepression:**

The AP-1 assay is a cell based luciferase reporter assay. A549 cells, which contain endogenous glucocorticoid receptor, were stably transfected with an AP-1 DNA binding site attached to the luciferase gene. Cells are then grown in RPMI + 10% fetal calf serum (charcoal-treated) + Penicillin/Streptomycin with 0.5mg/ml geneticin. Cells are plated the day before the assay at approximately 40000 cells/well. On assay day, the media are removed by aspiration and 20 μl assay buffer (RPMI without phenol red + 10% FCS (charcoal-treated) + Pen/Strep) are added to each well. At this point either 20 μl assay buffer (control experiments), the compounds of the present invention ("test compounds") (dissolved in DMSO and added at varying concentrations) or dexamethasone (100 nM in DMSO, positive control) are added to each well. The plates are then pre-incubated for 15 minutes at 37°C, followed by stimulation of the cells with 10 ng/ml PMA. The plates are then incubated for 7 hrs at 37°C after which 40 μl luciferase substrate reagent are added to each well. Activity is measured by analysis in a luminometer as compared to control experiments treated with buffer or dexamethasone. Activity is designated as % inhibition of the reporter system as compared to the buffer control with 10 ng/ml PMA alone. The control, dexamethasone, at a concentration of ≤10 μM typically suppresses activity by 65%. Test compounds which demonstrate an inhibition of PMA induction of 50% or greater at a concentration of test compound of ≤10 μM are deemed active.

**Wet Prostate Weight Assay AR Antagonist Assay:**

The activity of compounds of the present invention as AR antagonists was investigated in an immature male rat model, a standard, recognized test of antiandrogen activity of a given compound, as described in L. G. Hershberger et al., *Proc. Soc. Expt. Biol. Med.* **83,** 175 (1953); P. C. Walsh and R. F. Gittes, "Inhibition of extratesticular stimuli to prostate growth in the castrated rat by antiandrogens", *Endocrinology* **86,** 624 (1970); and B. J. Furr et al., "ICI 176,334: A novel non-steroid, peripherally selective antiandrogen", *J. Endocrinol.* **113,** R7-9 (1987), the disclosures of which are herein incorporated by reference.

The basis of this assay is the fact that male sexual accessory organs, such as the prostate and seminal vesicles, play an important role in reproductive function. These glands are stimulated to grow and are maintained in size and secretory function by the continued presence of serum testosterone (T), which is the major serum androgen (>95%) produced by the Leydig cells in the testis under the control of the pituitary luteinizing hormone (LH) and follicle stimulating hormone (FSH). Testosterone is converted to the more active form, dihydrotestosterone, (DHT), within the prostate by 5α-reductase. Adrenal androgens also contribute about 20% of total DHT in the rat prostate, compared to 40% of that in 65-year-old men. F. Labrie et al. Clin. Invest. Med. **16**, 475-492 (1993). However, this is not a major pathway, since in both animals and humans, castration leads to almost complete involution of the prostate and seminal vesicles without concomitant adrenalectomy. Therefore, under normal conditions, the adrenals do not support significant growth of prostate tissues. M. C. Luke and D. S. Coffey, "*The Physiology of Reproduction*" ed. By E. Knobil and J. D. Neill, 1, 1435-1487 (1994). Since the male sex organs are the tissues most responsive to modulation of the androgen activity, this model

is used to determine the androgen dependent growth of the sex accessory organs in immature castrated rats.

Male immature rats (19-20 days old Sprague-Dawley, Harlan Sprague-Dawely) were castrated under metofane anestesia. Five days after surgery these castrated rats (60-70g, 23-25 day-old) were dosed for 3 days. Animals were dosed sub-cutaneously (s.c.) 1mg/kg with Testosterone Proprionate (TP) in arachis oil vehicle and anti-androgen test compounds (compounds of the present invention) were dosed orally by gavage (p.o.) dissolved in suspensions of 80% PEG 400 and 20% Tween 80 (PEGTW). Animals were dosed (v/w) at 0.5 ml of vehicle /100g body weight. Experimental groups were as follows:

1. Control vehicle
2. Testosterone Propionate (TP) (3 mg/rat/day, subcutaneous)
3. TP plus Casodex (administered p.o. in PEGTW, QD), a recognized antiandrogen, as a reference compound.
4. To demonstrate antagonist activity, a compound of the present invention ("test compound") was administered (p.o. in PEGTW, QD) with TP (s.c. as administered in group 2) in a range of doses.
5. To demonstrate agonist activity a compound of the present invention ("test compound") was administered alone (p.o.. in PEGTW, QD) in a range of doses.

At the end of the 3-day treatment, the animals were sacrificed, and the ventral prostate was weighed. To compare data from different experiments, the sexual organs weights were first standardized as mg per 100 g of body weight, and the increase in organ weight induced by TP was considered as the maximum increase (100%). ANOVA followed by one-tailed Student or Fischer's exact test were used for statistical analysis.

The gain and loss of sexual organ weight reflect the changes of the cell number (DNA content) and cell mass (protein content), depending upon the serum androgen concentration. See Y. Okuda et al., *J. Urol.* **145,** 188-191 (1991), the disclosure of which is herein incorporated by reference. Therefore, measurement of organ wet weight is sufficient to indicate the bioactivity of androgens and androgen antagonist. In immature castrated rats, replacement of exogenous androgens increases seminal vesicles (SV) and the ventral prostate (VP) in a dose dependent manner.

The maximum increase in organ weight was 4 to 5-fold when dosing 3 mg/rat/day of testosterone (T) or 1 mg/rat/day of testosterone propionate (TP) for 3 days. The $EC_{50}$ of T and TP were about 1 mg and 0.03 mg, respectively. The increase in the weight of the VP and SV also correlated with the increase in the serum T and DHT concentration. Although administration ofT showed 5-times higher serum concentrations ofT and DHT at 2 hours after subcutaneous injection than that ofTP, thereafter, these high levels declined very rapidly. In contrast, the serum concentrations of T and DHT in TP-treated animals were fairly consistent during the 24 hours, and therefore, TP showed about 10-30-fold higher potency than free T.

In this immature castrated rat model, a known AR antagonist (Casodex) was also administered simultaneously with 0.1 mg of TP ($ED_{80}$), inhibiting the testosterone-mediated increase in the weights of the VP and SV in a dose dependent manner. The antagonist effects were similar when dosing orally or subcutaneously. Compounds of the invention also exhibited AR antagonist activity by suppressing the testosterone-mediated increase in the weights of VP and SV.

**Levator Ani & Wet Prostate Weight Assay AR Agonist Assay:**

The activity of compounds of the present invention as AR agonists was investigated in an immature male rat model, a recognized test of anabolic effects in muscle and sustaining effects in sex organs for a given compound, as described in L. G. Hershberger et al., *Proc. Soc. Expt. Biol. Med.* **83**, 175 (1953); B. L. Beyler et al, "Methods for evaluating anabolic and catabolic agents in laboratory animals", *J. Amer. Med. Women's Ass.* **23,** 708 (1968); H. Fukuda et al., "Investigations of the levator ani muscle as an anabolic steroid assay", *Nago Dai. Yak. Ken. Nem.* **14,** 84 (1966) the disclosures of which are herein incorporated by reference.

The basis of this assay lies in the well-defined action of androgenic agents on the maintenance and growth of muscle tissues and sexual accessory organs in animals and man. Androgenic steroids, such as testosterone (T), have been well characterized for their ability to maintain muscle mass. Treatment of animals or humans after castrations with an exogenous source of T results in a reversal of muscular atrophy. The effects ofT on muscular atrophy in the rat levator ani muscle have been well characterized. M. Masuoka et al., "Constant cell population in normal, testosterone deprived and testosterone stimulated levator ani muscles", *Am. J. Anat.* **119,** 263 (1966); Z. Gori et al., "Testosterone hypertrophy of levator ani muscle of castrated rats. I. Quantitative data", *Boll. -Soc. Ital. Biol. Sper.* **42,** 1596 (1966); Z. Gori et al., "Testosterone hypertrophy of levator ani muscle of castrated rats. II. Electron-microscopic observations", *Boll. -Soc. Ital. Biol.* Sper. **42**, 1600 (1966); A. Boris et al., *Steroids* **15**, 61 (1970). As described above, the effects of androgens on maintenance of male sexual accessory organs, such as the prostate and seminal vesicles, are well described. Castration results in rapid involution and atrophy of the prostate and seminal vesicles. This effect can be reversed by exogenous addition of androgens. Since both the levator ani muscle and the male sex organs are the tissues most responsive to

the effects of androgenic agents, this model is used to determine the androgen dependent reversal of atrophy in the levator ani muscle and the sex accessory organs in immature castrated rats. Sexually mature rats (200-250 g, 6-8 weeks-old, Sprague-Dawley, Harlan) were acquired castrated from the vendor (Taconic). The rats were divided into groups and treated daily for 7 to 14 days with one of the following:

1. Control vehicle
2. Testosterone Propionate (TP) (3 mg/rat/day, subcutaneous)
3. TP plus Casodex (administered p.o. in PEGTW, QD), a recognized antiandrogen, as a reference compound.
4. To demonstrate antagonist activity, a compound of the present invention ("test compound") was administered (p.o. in PEGTW, QD) with TP (s.c. as administered in group 2) in a range of doses.
5. To demonstrate agonist activity a compound of the present invention ("test compound") was administered alone (p.o. in PEGTW, QD) in a range of doses.

At the end of the 7-14-day treatment, the animals were sacrificed by carbon dioxide, and the levator ani, seminal vesicle and ventral prostate weighed. To compare data from different experiments, the levator ani muscle and sexual organ weights were first standardized as mg per 100 g of body weight, and the increase in organ weight induced by TP was considered as the maximum increase (100%). Super-ANOVA (one factor) was used for statistical analysis.

The gain and loss of sexual organ weight reflect the changes of the cell number (DNA content) and cell mass (protein content), depending upon the serum androgen concentration. See Y. Okuda et al., *J. Urol.* **145,** 188-191 (1991), the disclosure of which is herein incorporated by reference. Therefore, measurement of organ wet weight is sufficient to indicate the bioactivity of androgens and androgen antagonist. In immature castrated rats, replacement of exogenous androgens increases levator ani, seminal vesicles (SV) and prostate in a dose dependent manner.

The maximum increase in organ weight was 4 to 5-fold when dosing 3 mg/rat/day of testosterone (T) or 1 mg/rat/day of testosterone propionate (TP) for 3 days. The $EC_{50}$ ofT and TP were about 1 mg and 0.03 mg, respectively. The increase in the weight of the VP and SV also correlated with the increase in the serum T and DHT concentration. Although administration ofT showed 5-times higher serum concentrations of T and DHT at 2 hours after subcutaneous injection than that of TP, thereafter, these high levels declined very rapidly. In contrast, the serum concentrations ofT and DHT in TP-treated animals were fairly consistent during the 24 hours, and therefore, TP showed about 10-30-fold higher potency than free T.

**<u>MDA PCa2b Human Prostate Zenograft Assay:</u>**

In Vivo Antitumor Testing: MDA-PCa-2b human prostate tumors were maintained in Balb/c nu/nu nude mice. Tumors were propagated as subcutaneous transplants in adult male nude mice (4-6 weeks old) using tumor fragments obtained from donor mice. Tumor passage occurred every 5-6 weeks.

For antitumor efficacy trial, the required number of animals needed to detect a meaningful response was pooled at the start of the experiment and each was given a subcutaneous implant of a tumor fragment (~50 mg) with a 13-gauge trocar. Tumors were allowed to grow to approx. 100-200 mg (tumors outside the range were excluded) and animals were evenly distributed to various treatment and control groups. Treatment of each animal was based on individual body weight. Treated animals were checked daily for treatment related toxicity/mortality. Each group of animals was weighed before the initiation of treatment (Wt1) and then again following the last treatment dose (Wt2). The difference in body weight (Wt2-Wt1) provides a measure of treatment-related toxicity.

Tumor response was determined by measurement of tumors with a caliper twice a week, until the tumors reached a predetermined "target" size of 0.5 g. Tumor weights (mg) were estimated from the formula: Tumor weight = (length x $width^2$) ÷ 2

Tumor response end-point was expressed in terms of tumor growth inhibition (%T/C), defined as the ratio of median tumor weights of the treated tumors (T) to that of the control group (C).

To estimate tumor cell kill, the tumor volume doubling time was first calculated with the formula:

**TVDT = Median time (days) for control tumors to reach target size –**

**Median time (days) for control tumors to reach half the target size ;**

**And, Log cell kill = (T-C) ÷ (3.32 x TVDT)**

Statistical evaluations of data were performed using Gehan's generalized Wilcoxon test.

**Dunning Prostate Tumor:**

Dunning R3327H prostate tumor is a spontaneously derived, well differentiated androgen responsive adenocarcinoma of the prostate (Smolev, J.K. Heston, WD, Scott, W.W., and Coffey, D.S. *Cancer Treat Rep.* **61,** 273-287 (1977)). The growth of the R3327H subline has been selected for its highly androgen-dependent and reproducible growth in intact male rats. Therefore, this model and other sublines of this tumor have been widely used to evaluate *in vivo* antitumor activities of antiandrogens such as flutamide and bacilutamide/Casodex (Maucher, A., and von Angerer, *J. Cancer Res. Clin. Oncol.* **119,** 669-674 (1993), Furr, B.J.A., *Euro. URL.* **18** (suppl. 3), 2-9 (1990), Shain, S.A., and Huot, R.I. *J. Steroid Biochem.* **31**, 711-718 (1988)).

At the beginning of the study, the Dunning tumor pieces (about 4 x 4 mm) are transplanted subcutaneously to the flank of mature male Copenhagen rats (6-7 weeks old, Harlan-Sprague Dawley, Indianapolis, MD). About 6 weeks after the implantation, the animals with tumors of measurable size (about 80 - 120 $mm^2$) are randomized into treatment groups (8-10 rats/group) and the treatments are initiated. One group of the rats is castrated to serve as the negative control of tumor growth. Animals are treated daily with compounds of the current invention, standard antiandrogens such as bacilutamide or vehicle (control) for an average of 10 to 14 weeks. Test compounds are dissolved in a vehicle of (2.5 mL/kg of body weight) 10% polyethylene glycol and 0.05% Tween-80 in 1% carboxymethyl cellulose, PEG/CMC (Sigma, St Louis, MO). Typical therapeutic experiments would include three groups of three escalating doses for each standard or test compound (in a range of 300-3 mg/kg).

Tumors in the vehicle (control) group reach a size of 1500 to 2500 $mm^3$, whereas the castrated animal group typically shows tumor stasis over the 14 weeks of observation. Animals treated orally with 20 mg/kg of bicalutamide or flutamide would be expected to show a 40% reduction in tumor volumes compared to control after 14 weeks of treatment The size of tumors is measured weekly by vernier caliper (Froboz, Switzerland), taking perpendicular measurements of length and width. Tumor volumes are measured in $mm^3$ using the formula: Length x Width x Height = Volume. Statistical differences between treatment groups and control are evaluated using multiple ANOVA analysis followed by one tail non-parametric Student t test.

**Mature Rat Prostate Weight Assay:**

The activity of compounds of the present invention was investigated in a mature male rat model, which is a variation of the Levator ani & wet prostate weight assay described above. The above *in vivo* assays are recognized assays for determining the anabolic effects in muscle and sustaining effects in sex organs for a given compound, as described in L. G. Hershberger *et al., 83 Proc. Soc. Expt. Biol. Med.*, 175 (1953); B. L. Beyler *et al.*, "Methods for evaluating anabolic and catabolic agents in laboratory animals", *23 J. Amer. Med. Women's Ass.* 708 (1968); H. Fukuda *et al.,* "Investigations of the levator ani muscle as an anabolic steroid assay", *14 Nago Dai. Yak. Ken. Nem.* 84 (1966) the disclosures of which are herein incorporated by reference. The basis of this assay lies in the well-defined action of androgenic agents on the maintenance and growth of muscle tissues and sexual accessory organs in animals and man.

The male sexual accessory organs, such as the prostate and seminal vesicles, play an important role in reproductive function. These glands are stimulated to grow and are maintained in size and secretory function by the continued presence of serum testosterone (T), which is the major serum androgen (>95%) produced by the Leydig cells in the testis under the control of the pituitary hrteinizing hormone (LH) and follicle stimulating hormone (FSH). Testosterone is converted to the more active form, dihydrotestosterone, (DHT), within the prostate by 5$\alpha$-reductase. Adrenal androgens also contribute about 20% of total DHT in the rat prostate, compared to 40% of that in 65-year-old men. F. Labrie *et al., 16 Clin. Invest. Med.,* 475-492 (1993). However, this is not a major pathway, since in both animals and humans, castration leads to almost complete involution of the prostate and seminal vesicles without concomitant adrenalectomy. Therefore, under normal conditions, the adrenals do not support significant growth of prostate tissues, M. C. Luke and D. S. Coffey, "The Physiology of Reproduction" ed. By E. Knobil and J. D. Neill, 1,1435-1487 (1994). Since the male sex organs and the levator ani are the tissues most responsive to modulation of the androgen activity, this model is used to determine the activity of compounds that modulate the androgen receptor pathway in mature rats.

Along with its mitogenic activity on tissues such as prostate, seminal vesicle and muscle, testosterone also serves as a negative regulator for its own biosynthesis. Testosterone production in the Leydig cells of the testis is controlled by the level of circulating LH released from the pituitary gland. LH levels are themselves controlled by the level of LHRH produced in the hypothalmic region. Testosterone levels in the blood serve to inhibit the secretion of LHRH and subsequently reduce levels of LH and ultimately the levels of circulating testosterone. By measuring blood levels of LH as they are effected by compounds of the present invention ("test compounds"), it is possible to determine the level of agonist or antagonist activity of said compounds at the hypothalamic axis of this endocrine cycle.

Matched sets of Harlan Sprague-Dawely rats (40-42 days old, 180-220 g), were dosed orally by gavage (p.o.)

with the test compounds dissolved in suspensions of 80% PEG 400 and 20% Tween 20 (PEGTW) for 14 days. Two control groups, one intact and one castrated were dosed orally only with the PEGTW vehicle. Animals were dosed (v/w) at 0.5 ml of vehicle /100g body weight. Experimental groups were as follows:

1. Intact vehicle (p.o., PEGTW, QD)
2. Control vehicle (p.o., PEGTW, QD)
3. Bicalutamide (Casodex, a recognized antiandrogen, as a reference compound) or a compound of the present invention, p.o. in PEGTW QD (in a range of doses). At the end of the 14-day treatment, the animals were sacrificed, and the ventral prostate, the seminal vesicles, and the levator ani were removed surgically and weighed. To compare data from different experiments, the organ weights were first standardized as mg per 100 g of body weight, and expressed as a percentage of the value of the respective organ in the intact group.

Rat luteinizing hormone (rLH) is quantitatively determined with the Biotrak [$^{125}$I] kit (Amersham Pharmacia Biotek), following the manufacturer directions. The assay is based on the competition by the LH present in the serum of the binding of [$^{125}$I] rLH to an Amerlex-M bead/antibody suspension. The radioactivity that remains after incubation with the serum and subsequent washes is extrapolated into a standard curve to obtain a reading in ng/ml.

The gain and loss of sexual organ and levator ani weight reflect the changes of the cell number (DNA content) and cell mass (protein content), depending upon the serum androgen concentration, see Y. Okuda *et al., J. Urol.* **145**, 188-191 (1991), the disclosure of which in herein incorporated by reference. Therefore, measurement of organ wet weight is sufficient to indicate the bioactivity of androgens and androgen antagonist. In the mature rats assay, active agonist agents will have no effect or will increase the weight of one or more of the androgen responsive organs (levator aui, prostate, seminal vessicle) and will have no effect or a suppressive effect on LH secretion. Compounds with antagonist activity will decrease the weight of one or more of the androgen responsive organs (levator ani, prostate, seminal vesicle) and will have no effect or a reduced suppressive effect on LH secretion.

## CWR22 Human Prostate Zenograft Assay:

*In Vivo* Antitumor Testing: CWR22 human prostate tumors were maintained in Balb/c nu/nu nude mice. Tumors were propagated as subcutaneous transplants in adult male nude mice (4-6 weeks old) using tumor fragments obtained from donor mice. Tumor passage occurred every 5-6 weeks.

For antitumor efficacy trial, the required number of animals needed to detect a meaningful response was pooled at the start of the experiment and each was given a subcutaneous implant of a tumor fragment (~50 mg) with a 13-gauge trocar. Tumors were allowed to grow to approx. 100-200 mg (tumors outside the range were excluded) and animals were evenly distributed to various treatment and control groups. Treatment of each animal was based on individual body weight Treated animals were checked daily for treatment related toxicity/mortality. Each group of animals was weighed before the initiation of treatment (Wt1) and then again following the last treatment dose (Wt2). The difference in body weight (Wt2-Wt1) provides a measure of treatment-related toxicity.

Tumor response was determined by measurement of tumors with a caliper twice a week, until the tumors reached a predetermined "target" size of 0.5 g. Tumor weights (mg) were estimated from the formula: Tumor weight = (length x width$^2$) ÷ 2.

Tumor response end-point was expressed in terms of tumor growth inhibition (%T/C), defined as the ratio of median tumor weights of the treated tumors (T) to that of the control group (C).

To estimate tumor cell kill, the tumor volume doubling time was first calculated with the formula:

TVDT = Median time (days) for control tumors to reach target size - Median time (days) for control tumors to reach half the target size

And, Log cell kill = (T-C) ÷ (3.32 x TVDT)

Statistical evaluations of data were performed using Gehan's generalized Wilcoxon test.

The following Examples illustrate embodiments of the present invention, and are not intended to limit the scope of the claims.

## Abbreviations

The following abbreviations are used herein:

DBU = 1,8-diazabicyclo[5.4.0]undec-7-ene
4-DMAP = 4-dimethylaminopyridine

ee = enantiomeric excess
DMF = dimethylformamide
EtOAc = ethyl acetate
LDA = lithium diisopropylamide
Hunig's Base = N,N-diisopropylethylamine
Me = methyl
RT = retention time
TFA = trifluoroacetic acid
THF = tetrahydrofuran
TLC = thin layer chromatography
TMS = trimethylsilyl
pTSA = para-toluenesulfonic acid
Δ = heat
*t*-Bu = *tert*-butyl
$PhCH_3$ = toluene
Pd/C = palladium on activated charcoal
TsCl = tosyl chloride
TBSOTf = *tert*-butyldimethylsilyl trifluoromethane sulfonate
TBS = *tert*-butyldimethylsilane
MeI = methyl iodide
$(BOC)_2O$ = di-*tert*-butyl dicarbonate
TEA = triethylamine
*n*-BuLi = *n*-butyllithium
rt = room temperature
LC = liquid chromatography
Ts = tosyl
Ph = phenyl
EtOH = ethanol
DCE = dichloroethane
DMSO = dimethylsulfoxide
Ra-Ni = Raney Nickel
MS = molecular sieves
MS(ES) = Electro-Spray Mass Spectrometry
mCPBA = m-chloroperoxybenzoic acid
sat = saturated
AcOH = acetic acid
MeOH = methanol
$Et_2O$ = diethyl ether
Ac = acetyl
DEAD = diethyl azodicarboxylate
h = hours
Et = ethyl
WSDCC = water soluble dicarbonyl diimide, 1-(3-dimethylaminopropyl)-3-ethylcarbodiimide hydrochloride
TBAF = tetrabutylammonium fluoride
DBAD = di-terbutylazodicarboxylate
DCC = Dicyclohexylcarbodiimide
Wilkinson's catalyst = $RhCl(PPh_3)_3$
ADDP = 1,1-[azodicarbonyl]dipiperidine
DMA = dimethylacetamide
DME = 1,2-dimethoxyethane
BOP = benzotriazol-1-yloxytris(dimethylamino)-phosphonium hexafluorophosphate

**Example 1**

**(3aα,4α,7α,7aα)-2-(4-Bromo-3-methylphenyl)tetrahydro-4,7-ethanothiopyrano[3,4-c]pyrrole-1,3,8(2H,4H)-trione (1C)**

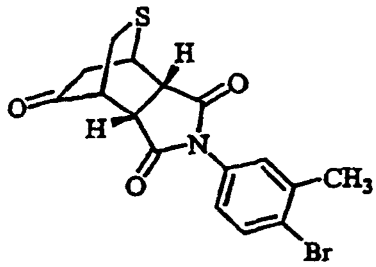

**A. 4-(tert-Butyldimethylsiloxy)-2H-thiopyran (1A)**

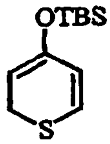

2,3-Dihydro-4H-thiopyran-4-one (1.50 g, 13.14 mol, synthesized as described in Richards *et al., J. Org. Chem.* **46,** 4836-4842 (1981)) was dissolved in $CH_2Cl_2$ (130 mL) and triethylamine (5.47 mL, 39.41 mmol) was added. *tert*-Butyldimethylsilyl trifluoromethanesulfonate (3.62 mL, 15.77 mmol) was then added. After 10 minutes, the volatiles were removed by rotary evaporator at 25°C. The resulting yellow oil was passed through a short column of $SiO_2$ eluting with 3% TEA in hexanes to yield 1.82 g of compound **1A** as an orange oil.

**B. 1-[4-bromo-3-methylphenyl]-1H-pyrrole-2,5-dione (1B)**

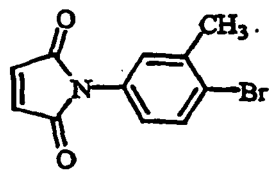

4-Bromo-3-methylaniline (1.55 g, 8.33 mmol) and maleic anhydride (0.898 g, 9.16 mmol) were dissolved in acetic acid (10 mL) and heated at 115°C for 12 h. The reaction mixture was then cooled to 25°C and the acetic acid was removed in vacuo. The resulting residue was suspended in 5% $K_2CO_3$ (100 mL), stirred for 25 minutes, followed by filtering and rinsing with water. The material was then dried in vacuo to give compound **1B** as a light brown solid (1.65 g). HPLC: 100% at 2.96 min (retention time) (YMC S5 ODS column 4.6 x 50 mm eluting with 10-90% aqueous methanol over 4 minutes containing 0.1 % TFA, 4 mL/min, monitoring at 220 nm).

**C. (3aα,4α,7α,7aα)-2-(4-Bromo-3-methylphenyl)tetrahydro-4,7-ethanothiopyrano[3,4-c]pyrrole-1,3,8(2H,4H)-trione (1C)**

Compound **1A** (0.313 g, 1.41 mmol) and compound **1B** (0.250 g, 0.94 mmol) were dissolved in toluene and heated to reflux for 5 h. The toluene was then removed by passing a stream of argon through the reaction flask. The residue was then purified by flash chromatography on $SiO_2$ eluting with 20% hexane in chloroform. This gave 0.168 g of the enol ether intermediate as a yellow solid. The enol ether intermediate was dissolved in dichloroethane (2.0 mL) and TFA (0.25 mL) was added. After 0.5 h, the reaction mixture was quenched with saturated aqueous $NaHCO_3$ and extracted with $CH_2Cl_2$ (2 x 30 mL). The organics were dried over anhydrous sodium sulfate and evaporated to give 0.079 g of compound 1C as a white solid: HPLC: 99% at 3.010 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 396.9 $[M+ NH_4]^+$.

## Example 2

(3aα,4α,7α,7aα)-2-(4-Bromo-3-methylphenyl)tetrahydro-4,7-ethanothiopyrano[3,4-c]pyrrole-1,3,8(2H,4H)-trione 5,5-dioxide (2)

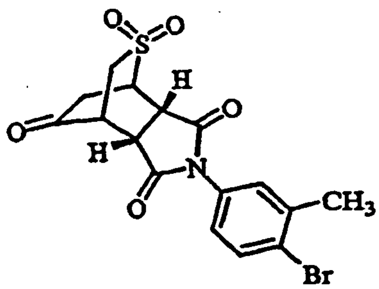

Compound **1C** (0.040 g, 0.105 mmol) was dissolved in $CH_2Cl_2$ (4.0 mL) and cooled to 0°C. *m*-CPBA (60% purity, 0.061 g, 0.210 mmol) was added and the reaction mixture was then warmed to 25°C. After 1 h, a 1:1 mixture of saturated $NaHCO_3$ and saturated sodium sulfite (20 mL) was added with vigorous stirring. After 15 minutes, the mixture was extracted with $CH_2Cl_2$ (2 x 30 mL) and the organics were dried over anhydrous sodium sulfate to yield 0.031 g of compound 2 as a white solid. No purification was necessary. HPLC: 78% at 2.290 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 429.8 $[M+ NH_4]^+$.

## Example 3

(3aα,4β,7β,7aα)-2-(3-Chlorophenyl)hexahydro-4-methyl-4-7-epoxy-1H-isoindole-1,3(2H)-dione (3)

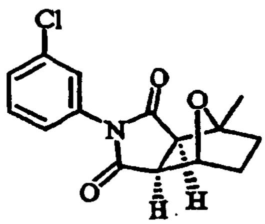

3-Chloroaniline (0.100 g, 0.787 mmol) and 3,6-endoxo-3-methylhexahydrophthalic anhydride (0.172 g, 0.945 mmol) were dissolved in AcOH (2.0 mL) and heated to 110°C for 11 h. The reaction mixture was then cooled to 25°C and poured into cold saturated aq $K_2CO_3$ and stirred vigorously for 10 min. The solution was then filtered and rinsed

with water. The resulting filtrate was dried in vacuo to give 0.118 g of compound 3 as a white solid. No further purification was needed. HPLC: 99% at 2.510 min (retention time) (YMC S5 ODS column 4.6 x 50 mm eluting with 10-90% aqueous methanol over 4 minutes containing 0.1 % TFA, 4 mL/min, monitoring at 220 nm), MS (ES): m/z 292.32 $[M+H]^+$.

The 2:1 mixture of compounds 4i and 4ii (0.361 g, from Example **4**) was dissolved in ethyl acetate (25 mL) and Pd/C (10% Pd, 0.2 g) was added. Hydrogen was introduced via a balloon and the reaction mixture was stirred at 25°C for 4 h; followed by filtration through celite and rinsed with ethyl acetate. Concentration in vacuo gave a yellow oil that was determined to be a 2:1 mixture of compound **5i** and compound **5ii** (0.348 g), which was not separated. HPLC: 100% at 2.900 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 401.0 $[M+ NH_4]^+$.

## Example 6

**(3aα,4α,7α,7aα)- and (3aα,4β,7β,7aα)-3a,4,7,7a-Tetrahydro-5-(hydroxymethyl)-2-[3-(trifluoromethyl)phenyl]-4,7-epoxy-1H-isoindole-1,3(2H)-dione (6i and 6ii, respectively)**

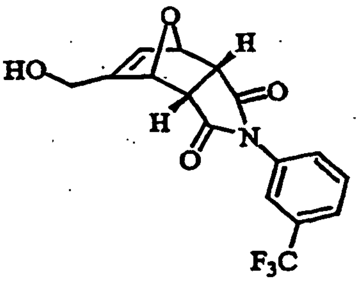

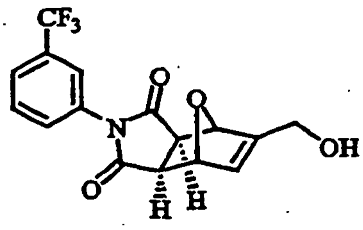

1-[3-(Trifluoromethyl)phenyl]-1H-pyrrole-2,5-dione (0.500 g, 2.39 mmol) and 3-furanmethanol (0.412 mL, 4.78 mmol) were dissolved in methylene chloride (3.0 mL) and stirred at 25°C for 20 h. The volatiles were then removed in vacuo and the resulting material purified by flash chromatography on $SiO_2$ eluting with chloroform/acetone to give 0.379 g of compound **6i** and 0.220 g of compound **6ii,** both as white solids. Compound **6i**: HPLC: 100% at 2.197 min (retention time) (YMC S5 ODS column 4.6 x 50 mm eluting with 10-90% aqueous methanol over 4 minutes containing 0.1% TFA, 4 mL/min, monitoring at 220 mn), MS (ES): m/z 338.0 $[M-H]^-$. Compound **6ii**: HPLC: 100% at 2.477 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 338.0 $[M-H]^-$

## Example 7

**(3aα,4α,7α,7aα)-3a,4,7,7a-Tetrahydro-5-(hydroxymethyl)-4-methyl-2-[3-(trifluoromethyl)phenyl]-4,7-epoxy-1H-isoindole-1,3(2H)-dione (7)**

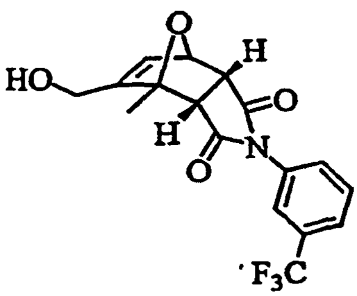

2-Methyl-3-furanmethanol (0.537 g, 4.78 mmol) and 1-[3-(trifluoromethyl)-phenyl]-1H-pyrrole-2,5-dione (0.500 g, 2.39 mmol) were dissolved in dichloroethane (2.0 mL) and stirred at 25°C for 20 h. The reaction mixture was then concentrated in vacuo and purified by flash chromatography in $SiO_2$ eluting with ethyl acetate/methylene chloride to give

0.317 g of compound **7** as a white solid. No other possible isomer was isolated after chromatography. HPLC: 100% at 2.197 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 351.9 $[M-H]^-$.

**Example 8**

**(3aα,4β,7β,7aα)-2-[3,5-Bis(trifluoromethyl)phenyl]hexahydro-4,7-epoxy-1H-isoindole-1,3(2H)-dione (8)**

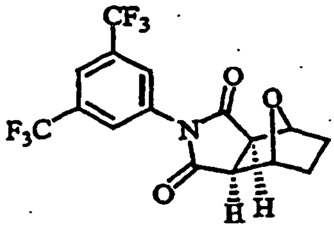

3,5-Bis(trifluoromethyl)-aniline (0.017 g, 0.0075 mmol) was dissolved in acetic acid (0.300 mL) and transferred to a 1.5 mL conical vial with a septa cap. Stock solutions of an additional 95 amines were prepared as descnbed above. To each of the above vials was added 0.4 mL (0.12 mmol) of a stock solution of exo-7-oxabicyclo[2.2.1]heptane-2,3-dicarboxylic anhydride in acetic acid. The vials were then sealed and heated at 110°C for 11 h. Upon cooling to 25°C, the caps were removed from the vials and the acetic acid was removed in vacuo. To each vial was added 1 mL of 2:1 acetone/methylene chloride and the vials were heated at 40°C for 1 h. Once all products were in solution, they were transferred via robot to filter tubes with coarse frits pre-wetted with 0.2 mL of water. Nitrogen was blown through each tube until the volatile organics were removed. 1.5 mL of 10% aq $K_2CO_3$ were then added to each tube followed by vigorous shaking at 25°C for 15 min. The tubes were then drained, resealed and 1.0 mL of water was added to each tube followed by shaking. The tubes were drained again and washed with water a second time. The resulting residues in each tube were then dried in vacuo for 48 h. After drying, 1.0 mL of 20% TFA in methylene chloride was added to each tube and the racks were shaken for 30 min. The tubes were then drained into a 96-well plate with pre-tared custom micro-tubes present. Each tube was assayed for product purity (analytical LC) and identity (LC-MS). The tubes were then concentrated in vacuo and weighed for yields. The tube containing the reaction mixture of 3,5-bistrifluoromethylaniline and exo-7-oxabicyclo[2.2.1]heptane-2,3-dicarboxylic anhydride, yielded 0.022 g of compound 8 as a white solid. HPLC: 94% at 4.03 min (retention time) (YMC S5 ODS column 4.6 x 50 mm eluting with 10-90% aqueous methanol over 4 minutes containing 0.1 % TFA, 4 mL/min, monitoring at 220 nm), MS (ES): m/z 434.2 $[M+Na+MeOH]^+$. Of the remaining 95 additional reaction runs, a total of 80 final compounds were obtained in >70% purity and >5 mg yield. Several samples needed further purification which was performed by short $SiO_2$ column eluting with methylene chloride/acetone. See Table 2 below.

**Example 9**

**(3aα,4α,7α,7aα)-2-(4-Bromophenyl)octahydro-1,3-dioxo-4,7-etheno-5H-pyrrolo[3,4-c]pyridine-5-carboxylic acid phenyl ester (9)**

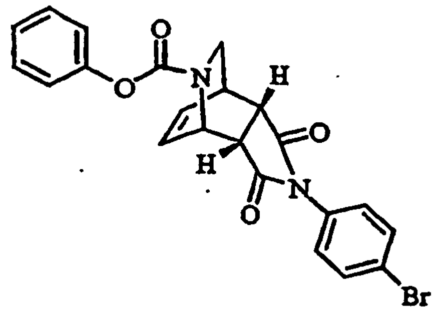

1-[4-Bromophenyl]-1H-pyrrole-2,5-dione (0.250 g, 0.992 mmol, synthesized as described in Example **1B**) and 1(2H)-pyridinecarboxylic acid phenylmethyl ester (0.299 g, 1.49 mmol, synthesized as described in Richard *et al., J. Org. Chem.* **46**, 4836-4842 (1981)) were dissolved in toluene and heated to 85°C for 1 h. Upon cooling to 25°C, the toluene was removed in vacuo. The resulting residue was dissolved in a minimum amount of chloroform and the product was precipitated by addition of hexanes. After 1 h at 25°C, the product was filtered and rinsed with cold 20% hexanes in chloroform giving compound 9 as a white solid (0.243 g) as a single isomer. HPLC: 100% at 3.393 min (retention time) (YMC S5 ODS column 4.6 x 50 mm eluting with 10-90% aqueous methanol over 4 minutes containing 0.1 % TFA, 4 mL/min, monitoring at 220 nm), MS (ES): m/z 454.98 $[M+H]^+$.

**Example 10**

**(3aα,4α,7α,7aα)-2-(4-Bromophenyl)octahydro-1,3-dioxo-4,7-etheno-5H-pyrrolo[3,4-c]pyridine-5-carboxylic acid phenylmethyl ester (10)**

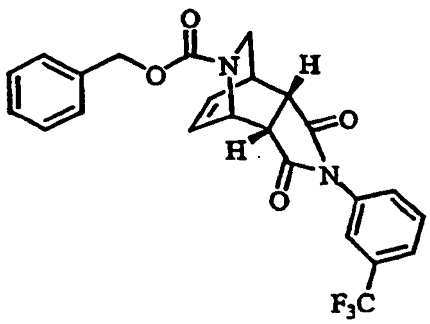

1-[3-(Trifluoromethyl)phenyl]-1H-pyrrole-2,5-dione (3.78 g, 15.7 mmol) and 1(2H)-pyridinecarboxylic acid phenylmethyl ester (4.0 g, 18.8 mmol, synthesized as described in Richard *et al., J. Org. Chem.* **46**,4836-4842 (1981)) were dissolved in toluene and heated at 80°C for 3 h. After cooling to 25°C, the toluene was removed in vacuo and the resulting residue was purified by flash chromatography on $SiO_2$ eluting with methanol/methylene chloride to give 3.2 g of compound **10** as a yellow oil. HPLC: 95% at 3.510 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 4572 $[M+H]^+$.

**Example 11**

**(3aα,4α,7α,7aα)-Hexahydro-2-[3-(trifluoromethyl)phenyl]-4,7-ethano-1H-pyrrolo[3,4-c]pyridine-1,3(2H)-dione trifluoroacetate (11)**

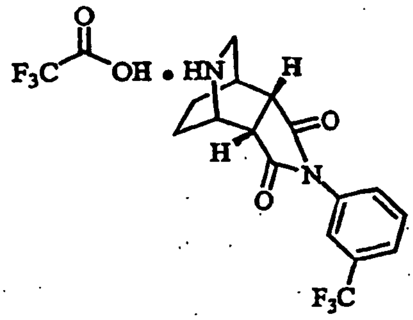

Compound **10** (3.2 g) was dissolved in 100 ml of MeOH and 10% Pd/C DeGussa catalyst (2 g) was added. Hydrogen was then introduced via a balloon. After 1 h, the reaction mixture was filtered through celite and rinsed with MeOH. The volatiles were removed in vacuo and the resulting crude material was purified by reverse phase preparative HPLC to yield 2.5 g of compound **11** as the TFA salt (white solid). HPLC: 99% at 1.843 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 325.12 $[M+H]^+$.

## Example 12

**(3aα,4α,7α,7aα)-5-Acetylhexahydro-2-[3-(trifluoromethyl)phenyl]-4,7-ethano-1H-pyrrolo[3,4-c]pyridine-1,3(2H)-dione (12)**

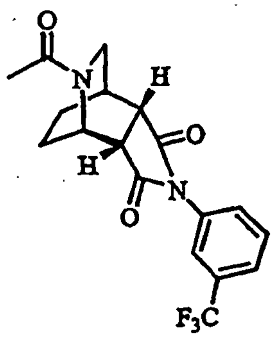

Compound **11** (0.100 g, 0.23 mmol) was suspended in THF (5.0 mL) and TEA (0.097 mL, 0.46 mmol) was added resulting in a homogeneous solution. Acetyl chloride (0.033 mL, 0.46 mmol) was then added. After 2 h, the reaction mixture was quenched with saturated aqueous $NaHCO_3$ and extracted with methylene chloride (3 x 15 mL). The crude material was purified by preparative-TLC eluting with chloroform/acetone to give 0.099 g of compound 12 as a colorless oil. HPLC: 99% at 2.66 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 367.0 $[M+H]^+$.

## Example 13

**(3aα,4α,7α,7aα)-5-Benzoylhexahydro-2-[3-(trifluoromethyl)phenyl]-4,7-ethano-1H-pyrrolo[3,4-c]pyridine-1,3(2H)-dione (13)**

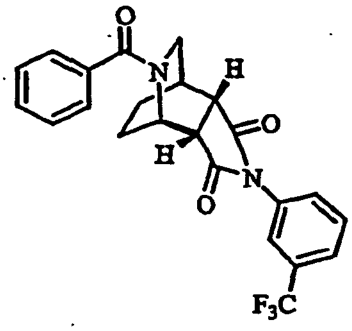

Compound **11** (0.100 g, 0.23 mmol) was suspended in THF (5.0 mL) and TEA (0.097 mL, 0.46 mmol) was added resulting in a homogeneous solution. Benzoyl chloride (0.05433 mL, 0.46 mmol) was then added. After 2 h, the reaction mixture was quenched with saturated aqueous $NaHCO_3$ and extracted with methylene chloride (3 x 15 mL). The crude material was purified by reverse phase preparative-HPLC to give 0.020 g of compound 13 as a white foam. HPLC: 99% at 3.183 min (retention time) (YMC S5 ODS column 4.6 x 50 mm eluting with 10-90% aqueous methanol over 4 minutes containing 0.1 % TFA, 4 mL/min, monitoring at 220 nm), MS (ES): m/z 429.1 $[M+H]^+$.

## Example 14

**(3aα,4α,7α,7aα)-Hexahydro-5-methyl-2-[3-(trifluoromethyl)phenyl]-4,7-ethano-1H-pyrrolo[3,4-c]pyridine-1,3 (2H)-dione(14)**

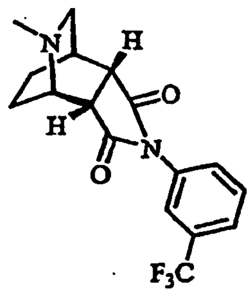

Compound **11** (0.100 g, 0.23 mmol) was suspended in THF (5.0 mL) and TEA (0.097 mL, 0.46 mmol) was added resulting in a homogeneous solution. Dimethyl sulfate (0.043 mL, 0.46 mmol) was added and the reaction mixture stirred at 25°C. After 14 h, the reaction mixture was concentrated in vacuo and the crude material was purified by preparative-TLC eluting with 10% MeOH in methylene chloride to give 0.030 g of compound **14** as a white solid. HPLC: 100% at 1.797 min (retention time) (YMC S5 ODS column 4.6 x 50 mm eluting with 10-90% aqueous medianol over 4 minutes containing 0.1% TFA, 4 mL/min, monitoring at 220 nm), MS (ES): m/z 339.21 $[M+H]^+$.

## Example 15

**(3aα,4α,7α,7aα)-Hexahydro-5-(phenylmethyl)-2-[3-(trifluoromethyl)phenyl]-4,7-ethano-1H-pyrrolo[3,4-c]pyridine-1,3(2H)-dione trifluoroacetate (15)**

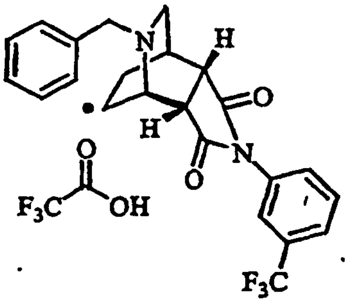

Compound **11** (0.100 g, 0.23 mmol) was dissolved in DMF (5.0 mL) and $K_2CO_3$ (0.063 g, 0.46 mmol) was added. Benzyl bromide (0.041 mL, 0.35 mmol) was then added. The reaction mixture was stirred at 25°C for 1 h, and then filtered and concentrated. The crude material was purified by reverse phase preparative-HPLC to give 0.055 g of compound **15** as a white solid. HPLC: 100% at 2.31 min (retention time) (YMC S5 ODS column 4.6 x 50 mm eluting with 10-90% aqueous methanol over 4 minutes containing 0. 1 % TFA, 4 mL/min, monitoring at 220 nm), MS (ES): m/z 415.36 $[M+H]^+$.

## Example 16

**(3aα,4α,7α,7aα)-Hexahydro-5-propyl-2-[3-(trifluoromethyl)phenyl]-4,7-ethano-1H-pyrrolo[3,4-c]pyridine-1,3 (2H)-dione trifluoroacetate (16)**

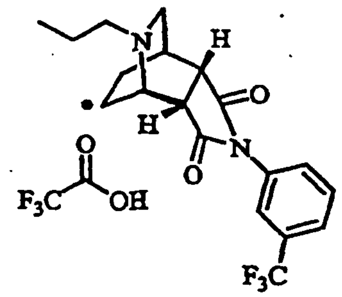

Compound **11** (0.100 g, 0.23 mmol) was dissolved in DMF (5.0 mL) and $K_2CO_3$ (0.079 g, 0.57 mmol) was added, followed by 1-bromopropane (0.031 mL, 0.34 mmol). The reaction mixture was stirred at 25°C for 6 h, and then filtered and concentrated. The crude material was purified by reverse phase preparative-HPLC to give 0.070 g of compound 16 as a white solid. HPLC: 100% at 1.907 min (retention time) (YMC S5 ODS column 4.6 x 50 mm eluting with 10-90% aqueous methanol over 4 minutes containing 0.1 % TFA, 4 mL/min, monitoring at 220 nm), MS (ES): m/z 340.22 $[M+H]^+$.

## Example 17

**(3aα,4α,4aβ,5aβ,6α,6aα)-2-[4-Cyano-3-(trifluoromethyl)phenyl]decahydro-1,3-dioxo-4,6-(iminomethano)cycloprop[f]isoindole-7-carboxylic acid phenylmethyl ester (17)**

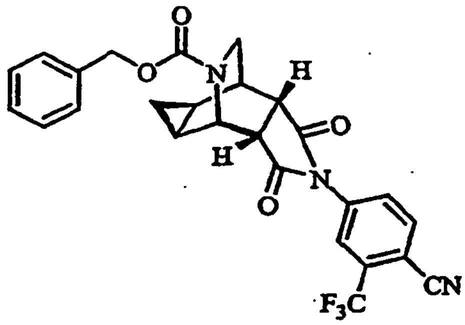

1-Methyl-3-nitro-1-nitrosoguanidine (2.5 g, 17 mmol) was added portion-wise to a solution of 40% KOH/$H_2O$ (15 mL) and $Et_2O$ (25 mL) at 0°C. The ether layer turned yellow once addition was complete. After 30 min at 0°C, the ether layer was poured into a solution of (3aα,4α,7α,7aα)-2-[4-cyano-3-(trifluoromethyl)phenyl]-octahydro-1,3-dioxo-4,7-etheno-5H-pyrrolo[3,4-c]pyridine-5-carboxylic acid phenylmethyl ester (0.50 g, 1.09 mmol, prepared as described in Example **10**) and $Pd(OAc)_2$ (0.010 g) in THF (10 mL) at 0°C. The reaction mixture was then warmed slowly to 25°C and stirred for 24 h and then filtered through celite, rinsing with THF. The crude material was then purified by flash chromatography on $SiO_2$ eluting with MeOH/$CH_2Cl_2$ to give 0.34 g of compound **17** as a white solid and a single isomer. HPLC: 100% at 3.61 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 496.25 $[M+H]^+$.

**Example 18**

**(3aα,4α,4aβ,5aβ,6α,6aα)-4-[Decahydro-1,3-dioxo-4,6-(iminomethano)cycloprop[f]isoindol-2-yl]-2-(trifluoromethyl)benzonitrile (18)**

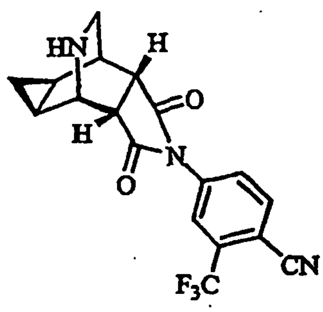

Compound **17** (0.200 g, 0.404 mmol) was dissolved in MeOH (20 mL) and 5% Pd/C (0.200 g) was added. Hydrogen was then introduced via balloon. After 3 h, the reaction mixture was filtered through celite, rinsed with MeOH and the volatiles were removed in vacuo to yield compound **18** (0.130 g) as a white solid. HPLC: 100% at 1.80 min (retention time) (YMC S5 ODS column 4.6 x 50 mm eluting with 10-90% aqueous methanol over 4 minutes containing 0.1 % TFA, 4 mL/min, monitoring at 220 nm), MS (ES): m/z 362.09 $[M+H]^+$.

**Example 19**

**(3aα,4α,4aβ,5aβ,6α,6aα)-4-[Decahydro-7-methyl-1,3-dioxo-4,6-(iminomethano)cycloprop[f]isoindol-2-yl]-2-(trifluoromethyl)benzonitrile (19)**

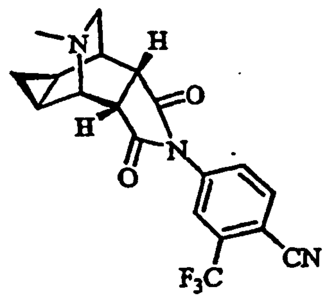

Compound **17** (0.100 g, 0.277 mmol) was dissolved in $CH_3CN$ (2.0 mL). TEA (0.19 mL, 1.39 mmol) and MeI (0.052 mL, 0.83 mmol) were then added and the reaction mixture was stirred at 25°C for 14 h. The reaction mixture was concentrated and the crude material was dissolved in $CH_2Cl_2$/water and extracted with $CH_2Cl_2$ (3 x 15 mL). The combined organics were dried over anhydrous $Na_2SO_4$. The crude material was purified by flash chromatography eluting with 3% MeOH/$CH_2Cl_2$ to give 0.030 g of compound **19** as a light yellow solid. HPLC: 100% at 1.720 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 376.11 $[M+H]^+$.

**Example 20**

**(3aα,4β,7β,7aα)-4-(Octahydro-4,7-dimethyl-1,3-dioxo-4,7-epoxy-2H-isoindol-2-yl)-2-(trifluoromethyl)benzonitrile (20B)**

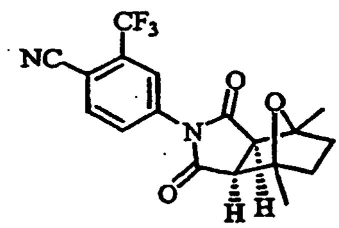

**A. (3aα,4β,7β,7aα)-Hexahydro-4,7-epoxyisobenzofuran-1,3-dione (20A)**

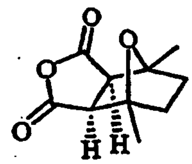

Freshly distilled dimethyl furan (1.60 mL, 15.3 mmol) was dissolved in $CH_2Cl_2$ (2.0 mL) and maleic anhydride (1.0 g, 10.2 mmol) was added. The reaction mixture was stirred at 25°C for 16 h and was then concentrated in vacuo to give a yellow solid. This solid was dissolved in ethyl acetate (30 mL) and Pd/C (10% Pd, 0.200 g) was added. Hydrogen was then introduced by a balloon and the reaction mixture stirred for 24 h. The Pd was removed by filtration through celite rinsing with EtOAc followed by concentration in vacuo to give the compound **20A** (1.69 g) as a white solid. 2-Dimensional NOE experiments confirmed the structural assignment to be that of compound **20A**.

**B. (3aα,4β,7β,7aα)-4-(Octahydro-4,7-dimethyl-1,3-dioxo-4,7-epoxy-2H-isoindol-2-yl)-2-(trifluoromethyl)benzonitrile (20B)**

A solution of compound **20A** (603 mg, 3.21 mmol, 1 eq), 5-amino-2-cyanobenzotrifluoride (640 mg, 3.44 mmol,

1.07 eq) and TsOH (10 mg, cat amount) in toluene (5 mL) was heated in a sealed tube for 2 days. The reaction mixture was cooled to room temperature and then concentrated under reduced pressure. Purification by flash chromatography on silica gel eluting with 50% EtOAc/hexanes gave 400 mg (1.10 mmol, 34 %) of compound **20B** as a white solid. HPLC: 99% at 3.04 min (retention time) (YMC S5 ODS column 4.6 x 50 mm, 10-90% aqueous methanol over 4 minutes containing 0.2% phosphoric acid, 4 mL/min, monitoring at 220 nm), MS (ESI): m/z 382.2 $[M+NH_4]^+$.

**Example 21**

**(3aα,4β,7β,7aα)-N-[4-[[2-[2-[4-Cyano-3-(trifluoromethyl)phenyl]octahydro-7-methyl-1,3-dioxo-4,7-epoxy-4H-isoindol-4-yl]ethyl]thio]phenyl]acetamide (21E)**

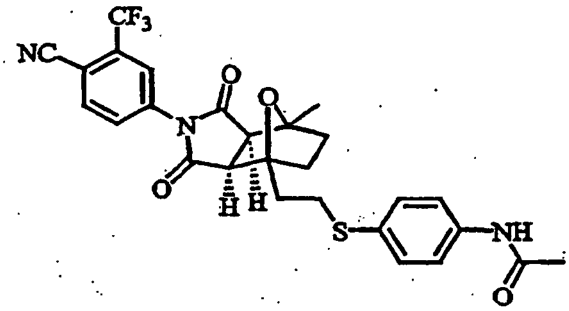

**A. 5-Methyl-2-furanethanol (21A)**

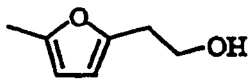

A solution ofn-BuLi (83 mL, 133.0 mmol, 1.2 eq, 1.6 M in hexanes) was added to a stirred solution of 2-methylfuran (10 mL, 110.8 mmol, 1 eq) in THF (85 mL) at 0°C under inert atmosphere. The reaction mixture was stirred for 4 h at room temperature, then cooled to 0°C. Ethylene oxide (8.3 mL, 166.3 mmol, 1.5 eq) was added dropwise and the reaction mixture was allowed to warm to room temperature overnight. After quenching with saturated aqueous $NH_4Cl$, the resulting layers were separated and the aqueous layer was extracted with $Et_2O$ (2x). The combined organic layers were dried over $Na_2SO_4$ and concentrated under reduced pressure. Distillation at atmospheric pressure (170-185°C) gave 10.13 g (80.3 mmol, 72%) of compound 21A as a light yellow oil.

**B. 2-(2-Bromoethyl)-5-methylfuran (21B)**

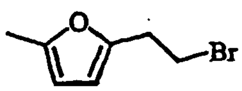

$Ph_3Br_2$ (3.68 g, 8.72 mmol, 1.1 eq) was added to a solution of compound 21A (1 g, 7.93 mmol, 1 eq) in DMF (8 mL) and the reaction mixture was stirred at room temperature for 1 h. The reaction mixture was added to $H_2O$ and extracted with EtOAc (3x). The combined organic layers were washed with $H_2O$ (2x), dried over $Na_2SO_4$ and concentrated under reduced pressure. Purification by flash chromatography on silica gel eluting with 10% EtOAc/hexanes gave 0.507 g (2.68 mmol, 34%) of compound **21B**.

**C. N-[4-[[2-(5-Methyl-2-furanyl)ethyl]thio]phenyl]acetamide (21C)**

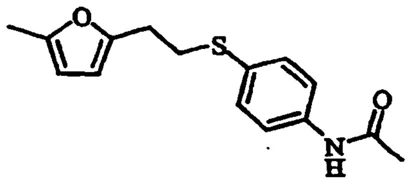

To a solution of 4-acetamidothiophenol (442 mg, 2.64 mmol, 1 eq) in THF (1 mL) at 0°C under inert atmosphere was added a solution of n-BuLi (2 mL, 3.17 mmol, 1.2 eq, 1.6 M in hexanes) in THF (1 mL). The reaction solution was stirred at room temperature for 10 min and a solution of compound **21B** (0.5 g, 2.64 mmol, 1 eq) in THF (3 mL) was added. After all the starting material was consumed as determined by TLC, the reaction was quenched with $H_2O$ and the mixture was extracted with EtOAc (2x), dried over $Na_2SO_4$ and concentrated under reduced pressure. Purincation by flash chromatography on silica gel eluting with 50% EtOAc/hexanes gave 0.644 g (2.34 mmol, 88%) of compound **21C.** MS (ESI): m/z 276.09 $[M+H]^+$.

**D. (3aα,4β,7β,7aα)-N-[4-[[2-[2-[4-Cyano-3-(trifluoromethyl)phenyl]-1,2,3,3a,7,7a-hexahydro-7-methyl-1,3-dioxo-4,7-epoxy-4H-isoindol-4-yl]ethyl]thio]phenyl]acetamide (21D)**

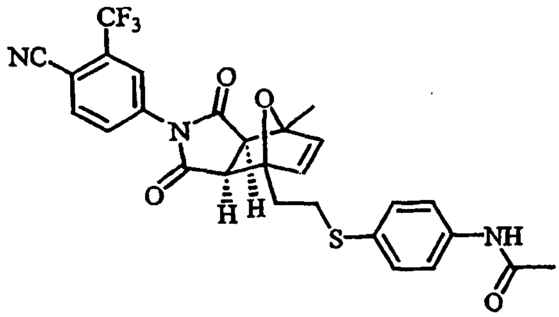

A solution of compound **21C** (195 mg, 0.708 mmol, 1 eq) and 4-(2,5-dihydro-2,5-dioxo-1H-pyrrol-1-yl)-2-trifluoromethylbenzonitrile (377 mg, 1.416 mmol, 2 eq, prepared as described for Example 1B) in $CH_2Cl_2$ (1.5 mL) was stirred at room temperature for two days. The reaction mixture was concentrated under reduced pressure to yield compound **21D** as determined by NMR analysis. Compound **21D** was used directly in the next step without purification.

**E. (3aα,4β,7β,7aα)-N-[4-[[2-[2-[4-Cyano-3-(trifluoromethyl)phenyl]-octahydro-7-methyl-1,3-dioxo-4,7-epoxy-4H-isoindol-4-yl]ethyl]thio]-phenyl]acetamide (21E)**

A solution of crude compound **21D** (0.708 mmol) and 10% Pd/C (200 mg) in MeOH (20 mL) was stirred under a hydrogen atmosphere over night. Purification by preparative chromatography [HPLC at 34.4 min (retention time) (YMC S5 ODS column 20 x 250 mm, 0-100% aqueous methanol over 30 minutes containing 0.1% TFA, 10 mL/min, monitoring at 220 nm)] followed by flash chromatography on silica gel eluting with 1% $MeOH/CH_2Cl_2$ gave 29 mg (0.053 mmol, 7.5%) of compound **21E** as a yellow powder. HPLC: 99% at 3.44 min (retention time) (YMC S5 ODS column 4.6 x 50 mm, 10-90% aqueous methanol over 4 minutes containing 0.2% phosphoric acid, 4 mL/min, monitoring at 220 nm), MS (ESI): m/z 544.01 $(M+H)^+$.

**Example 22**

**(3aα,4β,7β,7aα)-N-[4-[[2-[2-[4-Cyano-3-(trifluoromethyl)phenyl]octahydro-7-methyl-1,3-dioxo-4,7-epoxy-4H-isoindol-4-yl]ethyl]sulfinyl]phenyl]acetamide (22)**

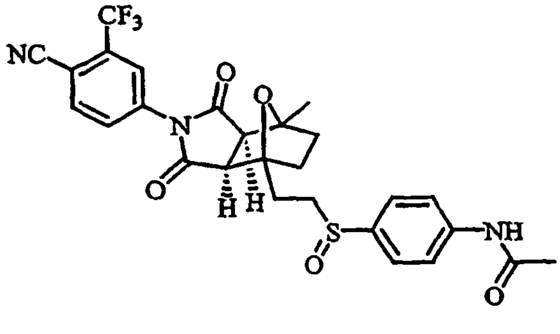

*m*CPBA (12 mg, 0.05 mmol) was added portion-wise to a solution of crude compound **21E** (65 mg, 4.12 mmol, 1 eq) in $CH_2Cl_2$ (6 mL) until the starting material was consumed. Purification by preparative chromatography [HPLC at 30.5 min (retention time) (YMC S5 ODS column 30 x 250 mm, 0-100% aqueous methanol over 30 minutes containing 0.1 % TFA, 25 mL/min, monitoring at 220 nm)] gave 27.5 mg (0.049 mmol, 41 %) of compound 22 as a tan solid (~1:1 mixture of diastereomers). HPLC: 96% at 2.88 min (retention time) (YMC S5 ODS column 4.6 x 50 mm eluting with 10-90% aqueous methanol over 4 minutes containing 0.1 % TFA, 4 mL/min, monitoring at 220 nm), MS (ESI): m/z 559.97 $[M+H]^+$.

**Example 23**

**(3aα,4β,7β,7aα)-N-[4-[[2-[2-[4-Cyano-3-(trifluoromethyl)phenyl]octahydro-7-methyl-1,3-dioxo-4,7-epoxy-4H-isoindol-4-yl]ethyl]sulfonyl]phenyl]acetamide (23)**

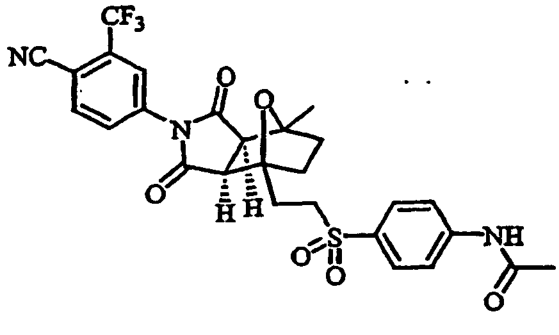

*m*CPBA (26 mg, 0.105 mmol, 3 eq) was added to a solution of compound **21E** (19 mg, 0.035 mmol, 1 eq) in $CH_2Cl_2$ (6 mL) and the reaction mixture was stirred at rt until starting material and the intermediate sulfoxide (compound **22**) were consumed as was apparent by TLC. Purification by preparative chromatography [HPLC at 53.3 min (retention time) (YMC S5 ODS column 30 x 250 mm, 0-70% aqueous methanol over 45 minutes containing 0.1% TFA, 25 mL/min, monitoring at 220 nm)] gave 27.5 mg (0.049 mmol, 40%) of compound **23** as a white solid. HPLC: 99% at 2.94 min (retention time) (YMC S5 ODS column 4.6 x 50 mm, 10-90% aqueous methanol over 4 minutes containing 0.2% phosphoric acid, 4 mL/min, monitoring at 220 nm), MS (ESI): m/z 575.95 $[M+H]^+$.

**Example 24**

**(3aα,4β,7β,7aα)- and (3aα,4α,7α,7aα)-N-[2-[2-[4-Cyano-3-(trifluoromethyl)phenyl]octahydro-7-methyl-1,3-dioxo-4,7-epoxy-4H-isoindol-4-yl]ethyl]benzenesulfonamide (24Ci and 24Cii, respectively)**

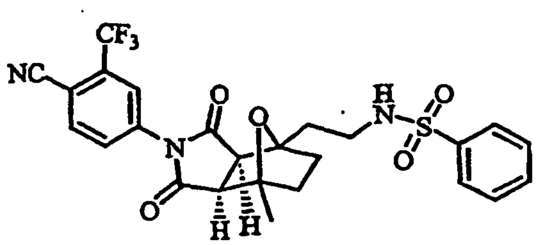

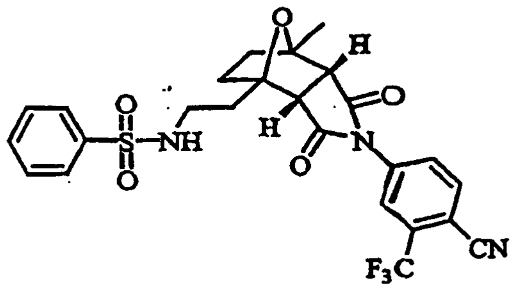

**A. 5-Methyl-2-furanethanol 4-methylbenzenesulfonate (24A)**

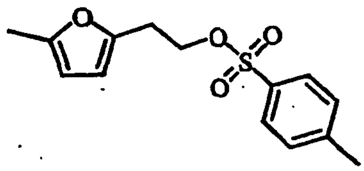

4-Methylbenzenesulfonyl chloride (907 mg, 4.76 mmol) was added to a solution of compound **21A** (500 mg, 3.96 mmol) in 6 ml of dry pyridine. The reaction mixture was stirred at room temperature for 4 h, then quenched with ice. The reaction mixture was extracted with $CH_2Cl_2$ and the combined organic layers were washed with saturated aqueous sodium bicarbonate and water, dried and concentrated under reduced pressure to give 900 mg (81%) of compound **24A** as a yellow oil.

**B. N-[2-(5-Methyl-2-furanyl)ethyl]benzenesulfonamide (24B)**

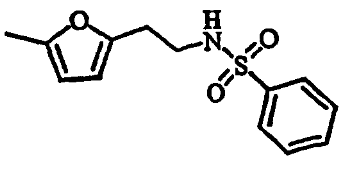

Benzenesulfonamide (157 mg, 1 mmol) was added to a 10% aqueous solution of sodium hydroxide (0.4 ml, 1 mmol). A solution of compound **24A** (280 mg, 1 mmol) in acetone (1 mL) was then added. The reaction mixture was heated at 90°C for 8 h, then cooled to room temperature. Ice was added and the mixture was extracted with $CH_2Cl_2$. The combined organic layers were washed with water, dried and concentrated under reduced pressure. Purification by flash chromatography on silica gel, eluting with $CH_2Cl_2$ gave 60 mg (23%) of compound **24B** as yellow oil.

**C. (3aα,4β,7β,7aα)- and (3aα,4α,7α,7aα)-N-[2-[2-[4-Cyano-3-(trifluoromethyl)phenyl]octahydro-7-methyl-1,3-dioxo-4,7-epoxy-4H-isoindol-4-yl]ethyl]benzenesulfonamide (24Ci and 24Cii, respectively)**

4-(2,5-Dihydro-2,5-dioxo-1H-pyrrol-1-yl)-2-trifluoromethylbenzonitrile (129 mg, 0.45 mmol, prepared as described in Example 1B) was added to a solution of compound **24B** (60 mg, 0.23 mmol) in $CH_2Cl_2$ (2 mL). The reaction mixture was stirred at room temperature for 2 days, concentrated under reduced pressure and purified by flash chromatography on silica gel, eluting with 70% EtOAc/hexanes, to give 20 mg (16%) of the unsaturated Diels-Alder product. The unsaturated product (20 mg) was immediately dissolved in 2 ml of ethanol and 10 mg of 10% Pd/C were added. The solution was stirred at room temperature overnight under a hydrogen atmosphere. The mixture was filtered and the filtrate was concentrated under reduced pressure. Purification by preparative reverse phase HPLC gave 7 mg of compound **24Ci** and 2 mg of compound **24Cii.** Compound **24Ci:** HPLC: 96% at 3.17 min (retention time) (YMC ODSA S5 C18 4.6 x 50 mm, 10%-90% aqueous methanol over 4 min gradient with 0.1% TFA, detected at 220 nm), MS (ES): m/z: 533.99 $[M+H]^+$. Compound **24Cii:** HPLC: 99% at 38.95 min (retention time) (YMC ODS S5 20 x 250 mm, 10%-90% aqueous methanol over 40 min gradient with 0.1 % TFA, detected at 220 nm), MS (ES): m/z 533.99 $[M+H]^+$.

## Example 25

**(3aα,4β,7β,7aα)-4-[Octahydro-4-(2-hydroxyethyl)-7-methyl-1,3-dioxo-4,7-epoxy-2H-isoindol-2-yl]-2-(trifluoromethyl)benzonitrile (25B)**

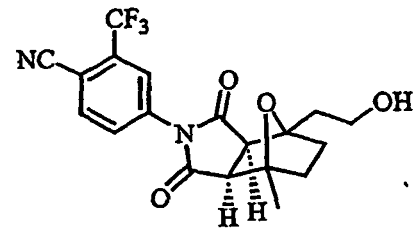

**A. (3aα,4β,7β,7aα)- and (3aα,4α,7α,7aα)-4-[1,3,3a,4,7,7a-Hexahydro-4-(2-hydroxyethyl)-7-methyl-1,3-dioxo-4,7-epoxy-2H-isoindol-2-yl]-2-(trifluoromethyl)benzonitrile (25Ai and 25Aii, respectively)**

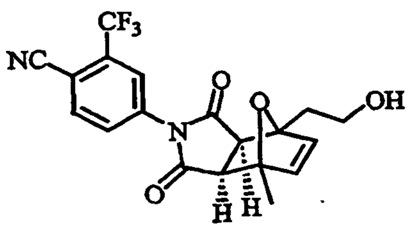

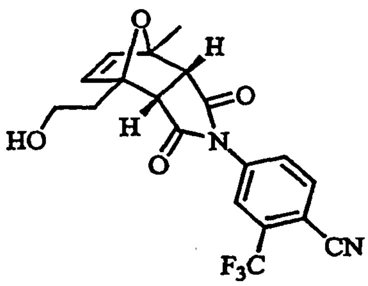

A solution of compound **21A** (252 mg, 2 mmol, 1 eq) and 4-(2,5-dihydro-2,5-dioxo-1H-pyaol-1-yl)-2-trifluoromethylbenzonitrile (798 mg, 3 mmol, 1.5 eq) in $CH_2Cl_2$ (10 mL) was stirred at room temperature for 2 days. The reaction mixture was concentrated under reduced pressure. Purification by flash chromatography on silica gel eluting with 65% EtOAc/hexanes gave 217 mg of pure compound **25Ai,** 73 mg of pure compound **25Aii** and 310 mg of a mixture of both compound **25Ai** and **25Aii**. All three fractions were isolated as white solids with a total isolated yield of 600 mg (1.53 mmol, 76.5%). Compound **25Ai**: HPLC 90% at 2.56 min (retention time) (YMC S5 ODS column 4.6 x 50 mm, 10-90% aqueous methanol over 4 minutes containing 0.2% phosphoric acid, 4 mL/min, monitoring at 220 nm). Compound **25Aii:** HPLC 90% at 2.56 min (retention time) (YMC S5 ODS column 4.6 x 50 mm, 10-90% aqueous methanol over 4 minutes containing 0.2% phosphoric acid, 4 mL/min, monitoring at 220 nm).

**B. (3aα,4β,7β,7aα)-4-[Octahydro-4-(2-hydroxyethyl)-7-methyl-1,3-dioxo-4,7-epoxy-2H-isoindol-2-yl]-2-(trifluaromethyl)benzonitrile (25B)**

A solution of compound **25Ai** (0.2 g, 0.51 mmol, 1 eq) and 10% Pd/C (43 mg, cat.) in EtOH (12 mL) was stirred under a hydrogen atmosphere at room temperature for 2 h. The reaction mixture was filtered through celite and concentrated under reduced pressure to give 0.2 g (0.51 mmol, 100%) of compound **25B** as a white solid. HPLC: 95% at 2.59 min (retention time) (YMC S5 ODS column 4.6 x 50 mm eluting with 10-90% aqueous methanol over 4 minutes containing 0.1 % TFA, 4 mL/min, monitoring at 220 nm), MS (ESI): m/z 394.97 $[M+H]^+$.

**Example 26**

**(3aα,4α,7α,7aα)- and (3aα,4β,7aα)-N-[4-[2-[2-[4-Cyano-3-(trifluoromethyl)phenyl]octahydro-7-methyl-1,3-dioxo-4,7-epoxy-4H-isoindol-4-yl]ethozy]phenyl]acetamide (26Ci and 26Cii, respectively)**

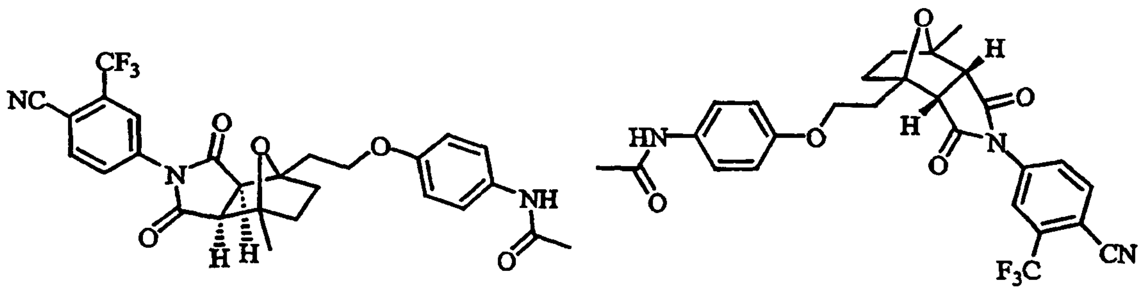

**A. 2-[4-[2-(5-Methyl-2-furanyl)ethoxy]phenyl]acetamide (26A)**

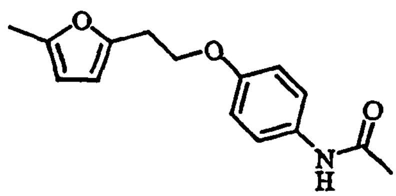

Triphenylphosphine (681 mg, 2.6 mmol, 1.3 eq) was added to a solution of compound **21A** (252 mg, 2 mmol, I eq) and 4-acetamidophenol (302 mg, 2 mmol, 1 eq) in $CH_2Cl_2$ (4 mL). THF (5 mL) was added to make the reaction mixture homogeneous and the mixture was then cooled to 0°C. DEAD (0.41 mL, 2.6 mmol, 1.3 eq) was added dropwise and the reaction mixture was stirred at room temperature overnight, then concentrated under reduced pressure. Purification by flash chromatography on silica gel eluting with 60% EtOAc/hexanes followed by preparative reverse phase HPLC gave 270 mg (52%, 1.04 mmol) of compound 26A as a light brown solid. MS (ESI): m/z 260.09 $[M+H]^+$.

**B. (3aα,4α,7α,7aα)- and (3aα,4β,7β,7aα)-N-[4-[2-[2-[4-Cyano-3-(trifluoromethyl)phenyl]-1,2,3,3a,7,7a-hexahydro-7-methyl-1,3-dioxo-4,7-epoxy-4H-isoindol-4-yl]ethoxy]phenyl]acetamide (26Bi and 26Bii, respectively)**

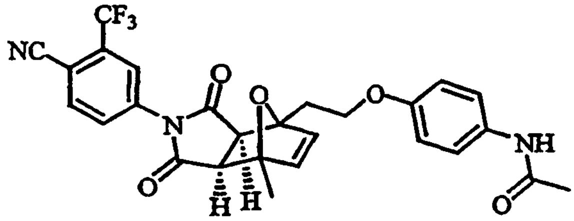

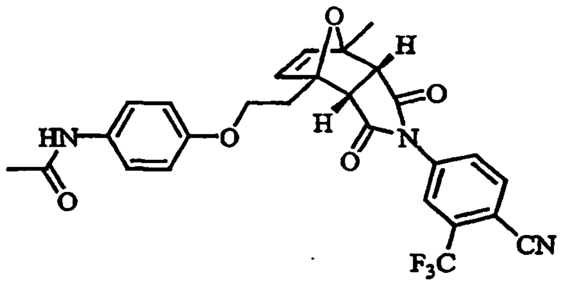

A solution of compound **26A** (40 mg, 0.154 mmol, 1 eq) and 4-(2,5-dihydro-2,5-dioxo-1H-pyrrol-1-yl)-2-trifluoromethylbenzonitrile (88 mg, 0.31 mmol, 2 eq) in $CH_2Cl_2$ (2 mL) was stirred at room temperature for 2 days. The reaction mixture was concentrated under reduced pressure. Purification by flash chromatography on silica gel eluting with 75% EtOAc/hexanes gave 55 mg (0.105 mmol, 68 %) of a 5 to 1 mixture of compounds **26Bi** and **26Bii** as a white solid, which was used directly in the next step. HPLC 90% at 3.28 min (retention time) (YMC S5 ODS column 4.6 x 50 mm, 10-90% aqueous methanol over 4 minutes containing 0.2% phosphoric acid, 4 mL/min, monitoring at 220 nm).

**C. (3aα,4α,7α,7aα)- and (3aα,4β,7β,7aα)-N-[4-[2-[2-[4-Cyano-3-(trifluoromethyl)phenyl]octahydro-7-methyl-1,3-diozo-4,7-epoxy-4H-isoindol-4-yl]ethoxy]phenyl]acetamide (26Ci and 26Cii, respectively)**

A solution of a mixture of compounds **26Bi** and **26Bii** (55 mg, 0.105 mmol, 1 eq) and 10% Pd/C (12 mg, cat.) in EtOH (3 mL) was stirred under a hydrogen atmosphere at room temperature overnight. The reaction mixture was filtered through celite and concentrated under reduced pressure to give 50 mg of crude product. Purification by flash chromatography on silica gel eluting with 70% EtOAc/hexanes gave 18 mg (0.034 mmol, 32 %) of compound 26Ci [HPLC: 96% at 3.33 min (retention time) (YMC S5 ODS column 4.6 x 50 mm, 10-90% aqueous methanol over 4 minutes containing 0.2% phosphoric acid, 4 mL/min, monitoring at 220 nm). MS (ES): m/z 528.01 $[M+H]^+$]; and 2.3 mg (0.004 mmol, 4%, 85:15-endo:exo) of an 85:15 mixture of compound (by $^1H$ NMR) **26Cii** and compound 26Ci respectively [HPLC: 90% at 3.35 min (retention time) (YMC S5 ODS column 4.6 x 50 mm, 10-90% aqueous methanol over 4 minutes containing 0.2% phosphoric acid, 4 mL/min, monitoring at 220 nm), MS (ESI): m/z 528.12 $[M+H]^+$].

**Example 27**

**(3aα,4α,7α,7aα)-Hexahydro-2-(2-naphthalenyl)-4,7-epoxy-1H-isoindole-1,3(2H)-dione (27D)**

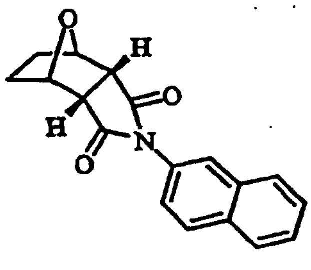

**A. (endo, endo)-7-Oxabicyclo[2.2.1]hept 5-ene-2,3-dicarboxylic acid (27A)**

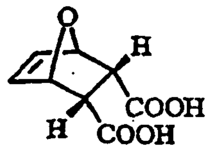

Compounds **27A, 27B** and **27C** were synthesized in accordance with the approaches described in Sprague *et al., J. Med. Chem.* **28,** 1580-1590 (1985). A mixture of furan (100 mL, 1.38 mol, 1 eq) and maleic acid (159.6 g, 1.38 mol, 1 eq) in $H_2O$ (340 mL) was stirred at room temperature for 5 days. The mixture was placed in a separatory funnel and the aqueous layer was separated from the layer containing the unreacted furan. The aqueous layer was treated with charcoal, filtered through celite and placed in the refrigerator. The desired product crystallized from solution upon seeding, was filtered, washed with cold water and dried over $P_2O_5$ to give 70 g (0.38 mol, 28%) of compound **27A** as a white solid.

**B. (endo, endo)-7-Oxabicyclo[2.2.1]heptane-2,3-dicarboxylic acid (27B)**

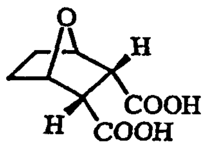

To a solution of compound **27A** (69 g, 0.375 mol, 1 eq) in EtOH (700 mL) was added 10% Pd/C (4.5 g, cat.) and the mixture was shaken under a hydrogen atmosphere at 55 psi until gas uptake ceased. The mixture was filtered through celite and concentrated in vacuo to give 66 g (0.355 mol, 95%) of compound **27B** as a white solid.

**C. (3aα,4α,7α,7aα)-Hexahydro-4,7-epoxyisobenzofuran-1,3-dione (27C)**

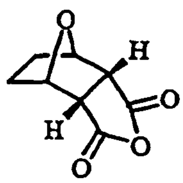

A solution of compound **27B** (66 g, 355 mol) in acetyl chloride (300 mL) was refluxed for 1 h. The reaction solution was concentrated in vacuo and the resulting residue was recrystallized from benzene to give 49.2 g (0.292 mol, 82%) of compound **27C** as a white solid (>99% endo by $^1$H NMR).

**D. (3aα,4α,7α,7aα)-Hexahydro-2-(2-naphthalenyl)-4,7-epoxy-1H-isoindole-1,3(2H)-dione (27D)**

Compound **27C** (45 mg, 0.30 mmol, 1 eq) was combined with 2-naphthalenamine (47 mg, 0.33 mmol, 1.1 eq) in acetic acid (1 mL) and heated at 115°C overnight. After the reaction mixture was cooled to rt, a drop of water was added, and the resulting precipitate was filtered. The material was washed with methanol and dried to provide 65.7 mg (74.5%) of compound **27D** as a white crystalline solid. HPLC: 99% at 2.68 min (retention time) (YMC S5 ODS column 4.6 x 50 mm, 10-90% aqueous methanol over 4 minutes containing 0.2% phosphoric acid, 4 mL/min, monitoring at 220 nm), MS (ESI): m/z 294.0 $[M+H]^+$.

**Example 28**

**(1aα,2β,2aα,5aα,6β,6aα)-Hexahydro-4-(2-naphthalenyl)-2,6-epoxy-3H-oxireno[f]isoindole-3,5(4H)-dione (28B)**

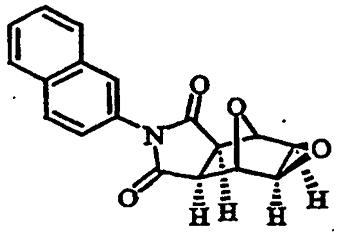

**A. (1aα,2β,2aα,5aα,6β,6aα)-Tetrahydro-2,6-epoxyoxireno[f]isobenzofuran-3,5(2aH,5aH)-dione (28A)**

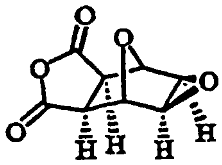

As described in Yur'ev *et al., J. Gen. Chem. U.S.S.R. (Engl. Transl.)* **31**, 772-775 (1961), a solution of exo-7-oxabicyclo[2.2.1]heptane-2,3-dicarboxylic anhydride (5 g, 30.09 mmol), formic acid (10 mL) and hydrogen peroxide (6 mL) was stirred at room temperature. After 30 min, the reaction mixture was placed in an ice bath (it became exothermic along with gas evolution) and was allowed to warm to room temperature slowly. After stirring overnight, the resulting precipitate was collected by filtration and washed with glacial acetic acid and dried to yield 3.02 g of a white powder. The crude solid was boiled in acetyl chloride (100 mL) for 10 hours and the mixture was concentrated to ~20 mL under reduced pressure. The resulting precipitate was filtered, washed with dioxanes and dried to give 2.37 g of compound **28A** as a white powder.

**B. (1aα,2β,2aα,5aα,6β,6aα)-Hexahydro-4-(2-naphthalenyl)-2,6-epoxy-3H-oxireno[f]isoindole-3,5(4H)-dione (28B)**

Compound **28A** (100 mg, 0.520 mmol, 1.2 eq) was combined with 2-naphthalenamine (0.434 mmol, 1 eq) in acetic acid (2 mL) and heated at 115°C overnight. After the reaction mixture was allowed to cool to rt, water was added, and the resulting precipitate was filtered. The material was washed sequentially with aqueous $K_2CO_3$ and water and then dried in a vacuum oven to provide 113.7 mg (85.3%) of compound 28B as an oil-white crystalline solid. HPLC: 99% at 1.76 min (retention time) (YMC S5 ODS column 4.6 x 50 mm, 10-90% aqueous methanol over 4 minutes containing 0.2% phosphoric acid, 4 mL/min, monitoring at 220 nm), MS (ESI): m/z 308.0 $[M+H]^+$.

**Example 29**

**(3aα,4α,7α,7aα)-2-[4-Bromo-3-(trifluoromethyl)phenyl]-3a,4,7,7a-tetrahydro-4,7-dimethyl-4,7-epithio-1H-isoindole-1,3(2H)-dione 8-oxide (29)**

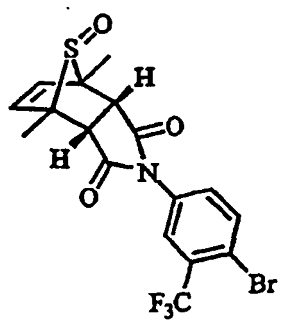

2,5-Dimethylthiophene (0.048 mL, 0.42 mmol) and 4-(2,5-dihydro-2,5-dioxo-1H-pyrrol-1-yl)-2-trifluoromethylbenzonitrile (0.290 g, 0.625 mmol) were dissolved in $CH_2Cl_2$ (8.0 mL) and cooled to -20°C. $BF_3$•$Et_2O$ (0.412 mL, 3.36 mmol) was added slowly followed by addition of *m*CPBA (~50%, 0.290 g, 0.84 mmol). After 2 h at -20°C, the reaction mixture was poured into saturated aq $NaHCO_3$ and extracted with $CH_2Cl_2$ (3 x 20 mL) and the organics dried over anhydrous $Na_2SO_4$. The crude product was purified by flash chromatography on $SiO_2$ eluting with 5%-10%-20% EtOAc in $CH_2Cl_2$ to give 0.119 g of compound **29** as a white solid. HPLC: 91% at 3.303 min (retention time) (YMC S5 ODS column 4.6 x 50 mm, 10-90% aqueous methanol over 4 minutes containing 0.2% phosphoric acid, 4 mL/min, monitoring at 220 nm). MS (ESI): m/z 480.2 $[M+H]^+$.

**Example 30**

**(3aα,4α,7α,7aα)-2-[4-Bromo-3-(trifluoromethyl)phenyl]-3a,4,7,7a-tetrahydro-4,7-epithio-1H-isoindole-1,3(2H)-dione 8-oxide (30)**

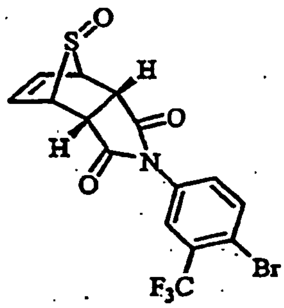

Thiophene (0.375 mL, 4.69 mmol) and 4-(2,5-dihydro-2,5-dioxo-1H-pyrrol-1-yl)-2-trifluoromethylbenzonitrile (0.100 g, 0.313 mmol) were dissolved in $CH_2Cl_2$ (50 mL) and *m*CPBA (~50%, 1.62 g, 4.69 mmol) was added and then stirred at 25°C for 3 h. Triphenylphosphine (2.0 g) was then added. After 15 min, the volatiles were removed in vacuo and the resulting residue was dissolved in $CH_2Cl_2$ (200 mL) and washed with saturated aq $NaHCO_3$ (3 x 50 mL) and dried over $Na_2SO_4$. The crude material was then purified by flash chromatography on $SiO_2$ eluting with 1% - 3% - 5% methanol in $CH_2Cl_2$ to give compound 30 as a white powder (0.059 g). NMR spectroscopy and LC analysis showed a single diastereomer. HPLC: 100% at 3.437 min (retention time) (YMC S5 ODS column 4.6 x 50 mm, 10-90% aqueous methanol over 4 minutes containing 0.2% phosphoric acid, 4 mL/min, monitoring at 220 nm), MS (ESI): m/z 443.2 $[M+H]^+$.

**Example 31**

**(3aα,4α,7α,7aα)-Hexahydro-2-[3-(trifluoromethyl)phenyl]-4,7-imino-1H-isoindole-1,3(2H)-dione (31D)**

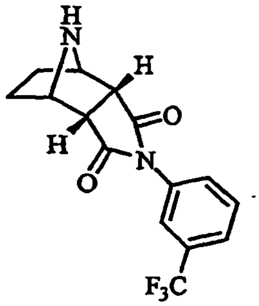

A. 7-Azabicyclo[2.2.1]hepta-2,5-diene-2,3,7-tricarboxylic acid 2,3-dimethyl 7-(1,1-dimethylethyl) ester (31A)

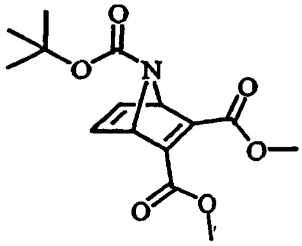

The freshly distilled acetylenedicarboxylic acid dimethyl ester (6.7 mL, 54.0 mmol) and N-(tert-butyloxycarbonyl)-1H-pyrrole (9.0 mL, 54.0 mmol) were combined and heated at 120°C for 3 h. Purification by flash chromatography on $SiO_2$ eluting with EtOAc/$CH_2Cl_2$ gave 8.3 g of compound 31A as a yellow solid.

**B. (exo,endo)-7-Azabicyclo[2.2.1]hept-2,5-diene-2,3,7-tricarboxylic acid 7-(1,1-dimethylethyl) ester (31B)**

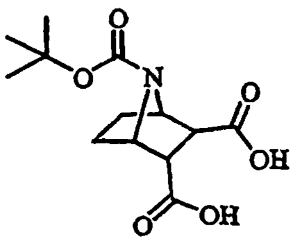

Compound **31A** (1.0 g, 3.5 mmol) was dissolved in MeOH (2.0 mL) and aq KOH (1 g in 5 mL $H_2O$) was added. The reaction mixture was heated to 50°C for 1 h. The reaction mixture was then cooled to 25°C and Pd/C (0.5 g, 10% Pd) was added and the mixture was placed in a Parr apparatus for 14 h at 25°C. The reaction mixture was then filtered through celite rinsing with water. The aqueous solution was acidified to pH = 2 by addition of 1 N HCl and then extracted with EtOAc (2 x 100 mL). Concentration of the organics gave the compound **31B** as a pale yellow solid.

**C. (3aα,4α,7α,7aα)-Hexahydro-1,3-dioxo-4,7-iminoisobeuzofuran-8-carboxylic acid 1,1-dimethylethyl ester (31C)**

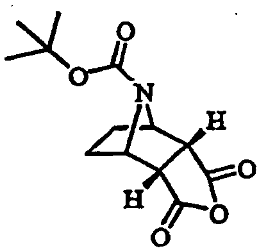

The crude compound **31B** was heated to 120°C in vacuo in a sublimation chamber, resulting in sublimation of compound **31C** as a white solid (0.051 g), which was collected directly and used in the next step without further purification.

**D. (3aα,4α,7α,7aα)-Hexahydro-2-[3-(trifluoromethyl)phenyl]-4,7-imino-1H-isoindole-1,3(2H)-dione (31D)**

Compound **31C** (0.050 g, 0.187 mmol) and the 1-amino-3-(trifluoromethyl)benzene (0.030 g, 0.187 mmol) were dissolved in AcOH (2.5 mL) and heated to 115°C for 4.5 h. The reaction mixture was quenched by addition of saturated aqueous $NaHCO_3$ and then extracted with methylene chloride (3 x 15 mL). The crude material was purified by preparative reverse phase HPLC to give 0.030 g of compound **31D** as a white solid. HPLC: 99% at 2.33 min (retention time) (YMC S5 ODS column 4.6 x 50 mm, 10-90% aqueous methanol over 4 minutes containing 0.2% phosphoric acid, 4 mL/min, monitoring at 220 nm), MS (ESI): m/z 311.15 $[M+H]^+$.

**Example 32**

**(3aα,4β,7β,7aα)- and (3aα,4α,7α,7aα)-3a,4,7,7a-Tetrahydro-4,7-dimethyl-2-[3-(trifluoromethyl)phenyl]-4,7-epoxy-1H-isoindole-1,3(2H)-dione (32i and 32ii, respectively)**

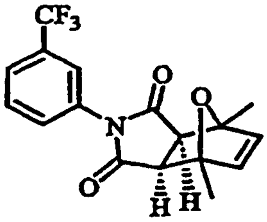

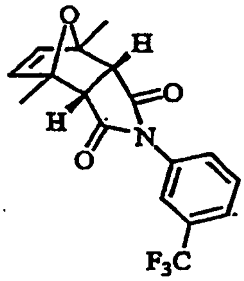

Freshly distilled 2,5-dimethylfuran (0.32 mL, 2.6 mmol) was dissolved in $CH_2Cl_2$ (2.0 mL) and 1-[3-(trifluoromethyl)phenyl]-1H-pyrrole-2,5-dione (0.5 g, 2.5 mmol) was added. The reaction mixture was stirred at 25°C for 16 h and was then concentrated under reduced pressure. Purification by flash chromatography on silica gel eluting with 0.5% $MeOH/CH_2Cl_2$ gave 50 mg of compound **32i** and 250 mg of compound **32ii,** as white solids. Compound **32i**: HPLC: 92% at 3.047 min (retention time) (YMC S5 ODS column 4.6 x 50 min, 10-90% aqueous methanol over 4 minutes containing 0.2% phosphoric acid, 4 mL/min, monitoring at 220 nm), MS (ES): m/z: 338.15 $[M+H]^+$; Compound **32ii:** HPLC: 98% at 3.08 min (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 338.30 $[M+H]^+$.

**Example 33**

**(3aα,4α,7α,7aα)-Hexahydro-4,7-dimethyl-2-[3-(trifluoromethyl)phenyl]-4,7-epoxy-1H-isoindole-1,3(2H)-dione (33)**

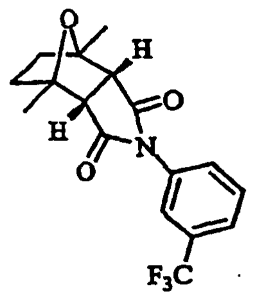

Compound **32ii** (0.080 g, 0.237 mmol) was dissolved in EtOAc (2 mL) and EtOH (1 ml) and Pd/C (10% Pd, 0.050 g) was added. Hydrogen was then introduced by a balloon and the reaction mixture was stirred for 24 h. The mixture was filtered through celite, rinsed with EtOAc and concentrated in vacuo to give compound 33 (0.075g) as a white solid. No further purification was needed. HPLC: 90% at 3.233 min (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 340.40 $[M+H]^+$.

**Example 34**

**(3aα,4α,7α,7aα)-Tetrahydro-5-methyl-2-(4-nitro-1-naphthalenyl)-4,7-etheno-1H-pyrrolo[3,4-c]pyridine-1,3,6(2H,5H)-trione (34B)**

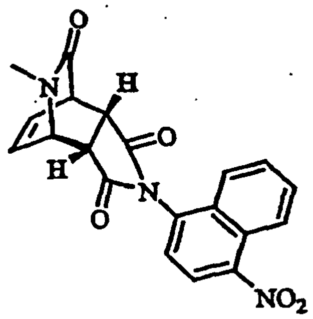

**A. 4,5,7,7a-Tetrahydro-5-methyl-4,7-ethenofuro[3,4-c]pyridine-1,3,6(3aH)-trione (34A)**

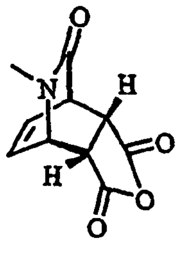

Compound **34A** was synthesized by a modification of the methods described in Tomisawa *et al., Heterocycles* **6,** 1765-1766 (1977) and *Tetrahedron Lett.* ***29,*** 2465-2468 (1969). Maleic anhydride and 1-methyl-2-pyridone were suspended in 30 ml of anhydrous toluene. The reaction vessel was fitted with a Dean Stark trap and refluxed for 48 hours. The dark colored solution was allowed to cool to rt and then the volatiles were removed in vacuo. The resulting brown paste was dissolved in 10 ml of boiling toluene and the hot solution was filtered under a nitrogen flow to remove particulates. On standing at 25°C the desired product precipitated from solution. The solid was isolated by filtration and washed with cold toluene to give compound **34A**, which was used without further purification.

**B. (3aα,4α,7α,7aα)-Tetrahydro-5-methyl-2-(4-nitro-1-naphthalenyl)-4,7-etheno-1H-pyrrolo[3,4-c]pyridine-1,3,6 (2H,5H)-trione (34B)**

1-Amino-4-nitronaphthalene (0.094 g, 0.5 mmol) and compound **34A** (0.130 g, 0.63 mmol) were dissolved in AcOH (2.0 mL) and heated to 110°C for I 1 h. The reaction mixture was then cooled to 25°C and poured into cold saturated aqueous $K_2CO_3$ and stirred vigorously for 10 min. The solution was filtered and rinsed with water. The resulting filtrate was dried in vacuo and purified by silica gel chromatography using a solvent system of 4:6 EtOAc/Hexane, to give 0.172 g of compound 34B as a white solid. HPLC: 92% at 2.472 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 378.29 $[M+H]^+$.

## Example 35

**(3aα,4β,7β,7aα)-4-[4-[2-(4-Fluorophenoxy)ethyl]octahydro-7-methyl-1,3-dioxo-4,7-epoxy-2H-isoindol-2-yl]-2-(trifluoromethyl)benzonitrile (35)**

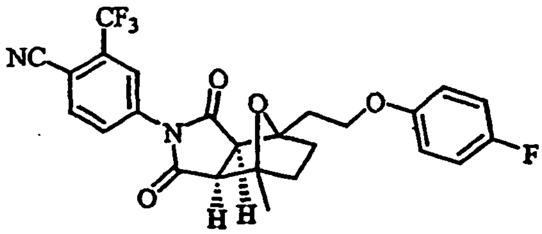

DEAD (0.06 mL, 0.380 mmol, 1.5 eq) was added to a solution of triphenylphosphine (100 mg, 0.380 mmol, 1.5 eq) in THF (1.3 mL) at room temperature under an inert atmosphere. After stirring for 10 min, 4-fluorophenol (43 mg, 0.380 mmol, 1.5 eq) was added in one portion. The reaction mixture was stirred for 5 min, compound **25B** (100 mg, 0.254 mmol, 1 eq) was added and stirring was continued for 3.5 h. Purification by flash chromatography on silica gel eluting with 50% EtOAc/Hexanes followed by preparative chromatography [HPLC: 11.93 min (retention time) (YMC S5 ODS column 20 x 100 mm, 0-100% aqueous methanol over 10 minutes containing 0.1% TFA, 20 mL/min, monitoring at 220 nm)] gave 72 mg (58%) of compound 35 as a solid. HPLC: 99% at 3.74 min (retention time) (YMC S5 ODS column 4.6 x 50 mm, 10-90% aqueous methanol over 4 minutes containing 0.2% phosphoric acid, 4 mL/min, monitoring at 220 nm), MS (ESI): m/z 487.1 $[M-H]^-$.

## Example 36

**(3aα,4β,7β,7aα)-4-[4-(2-Bromoethyl)octahydro-7-methyl-1,3-dioxo-4,7-epoxy-2H-isoindol-2-yl]-2-(trifluoromethyl)benzonitrile (36)**

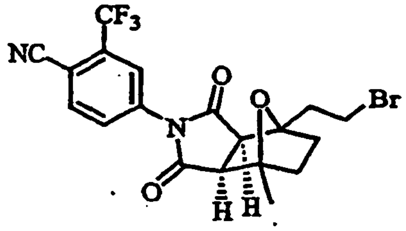

A solution of compound **25B** (495 mg, 1.26 mmol, 1 eq) and pyridine (0.1 ml, 1.26 mmol, 1 eq) in $CH_2Cl_2$(2 ml) was added to a solution of $Ph_3PBr_2$ (636 mg,1.51 mmol, 1.2 eq) in $CH_2Cl_2$ (2ml) at 0°C. The reaction mixture was stirred at room temperature for 3 hr, then the solvent was removed under reduced pressure. The resulting residue was washed 2x with 10 ml portions of EtOAc-hexane (6:4) and the combined washings were purified by flash chromatography

on silica gel eluting with 60% EtOAc/hexane to give 390 mg (0.85 mmol, 67.7%) of compound **36** as a white solid. HPLC: 99% at 3.51 min (retention time) (YMC S5 ODS column 4.6 x 50 mm, 10-90% aqueous methanol over 4 minutes containing 0.2% phosphoric acid, 4 mL/min, monitoring at 220 nm). MS (ESI): m/z 456.7 [M-H]$^-$.

## Example 37

**(3aα,4β,7β,7aα)-Hexahydro-4,7-dimethyl-2-(3-methyl-4-nitrophenyl)-4,7-epoxy-1H-isoindole-1,3(2H)-dione (37)**

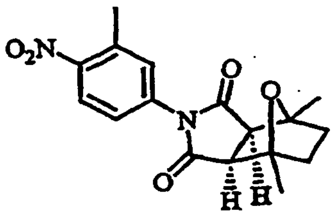

A combination of 4-nitro-3-methylaniline (0.050 g, 0.33 mmol), compound **20A** (0.083 g, 0.43 mmol), TEA (0.2 mL), $MgSO_4$ (0.075 g) and toluene (0.8 mL) was combined in a sealed tube and the mixture was heated to 120°C for 14 h. After cooling to 25°C, the reaction mixture was filtered, rinsed with $CH_2Cl_2$ and concentrated. The crude product was purified by preparative-TLC on $SiO_2$ eluting with $CH_2Cl_2$ to give 0.075 g of compound 37 as a pale yellow solid. HPLC: 100% at 2.733 min (retention time) (YMC S5 ODS column, 4.6 x 50 mm; 10-90% MeOH/$H_2O$ gradient,+ 0.1% TFA; 4 mL/min, 220 nm detection), MS (ES): m/z 348.2 [M+$NH_4$]$^+$.

## Examples 38 to 121

Additional compounds of the present invention were prepared by procedures analogous to those described above. The compounds of Examples 38 to 121 have the following structure (L is a bond):

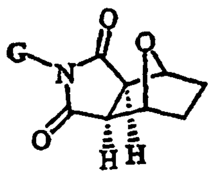

where G, the compound name, retention time, molecular mass, and the procedure employed, are set forth in **Table 2.** The chromatography techniques used to determine the compound retention times of **Table 2** are as follows: LCMS = YMC S5 ODS column, 4.6 x 50 mm eluting with 10-90% MeOH/$H_2O$ over 4 minutes containing 0.1% TFA; 4 mL/min, monitoring at 220 nm. The molecular mass of the compounds listed in **Table 2**, where provided, was determined by MS (ES) by the formula m/z.

**Table 2**

| Ex. No. | G | Compound Name | Retention Time (Min.)/ Molecular Mass | Pro. of Ex. |
|---|---|---|---|---|
| 38 | 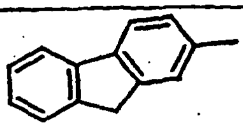 | (3aα,4β,7β,7aα)-2-(2-Fluorenyl)hexahydro-4,7-epoxy-1H-isoindole-1,3(2H)-dione | 3.72 LCMS/ 332.20 $[M+H]^+$ | 8 |
| 39 | 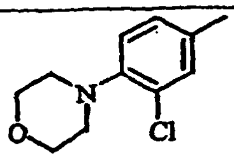 | (3aα,4β,7β,7aα)-2-[3-Chloro-4-(4-morpholinyl)phenyl]hexahydro-4,7-epoxy-1H-isoindole-1,3(2H)-dione | 3.20 LCMS/ 363.20 $[M+H]^+$ | 8 |
| 40 | 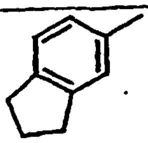 | (3aα,4β,7β,7aα)-2-(2,3-Dihydro-1H-inden-5-yl)hexahydro-4,7-epoxy-1H-isoindole-1,3(2H)-dione | 3.26 LCMS/ 284.22 $[M+H]^+$ | 8 |

| Ex. No. | G | Compound Name | Retention Time (Min.)/ Molecular Mass | Pro. of Ex. |
|---|---|---|---|---|
| 41 | 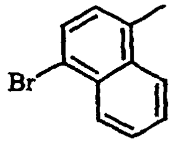 | (3aα,4β,7β,7aα)-2-(4-Bromo-1-naphthalenyl)hexahydro-4,7-epoxy-1H-isoindole-1,3(2H)-dione | 3.73 LCMS/ 404.11 $[M+CH_3OH+H]^+$ | 8 |
| 42 | 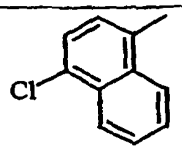 | (3aα,4β,7β,7aα)-2-(4-Chloro-1-naphthalenyl)hexahydro-4,7-epoxy-1H-isoindole-1,3(2H)-dione | 3.63 LCMS/ 328.14 $[M+H]^+$ | 8 |
| 43 | 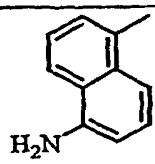 | (3aα,4β,7β,7aα)-2-(5-Amino-1-naphthalenyl)hexahydro-4,7-epoxy-1H-isoindole-1,3(2H)-dione | 1.64 LCMS/ | 8 |
| 44 | 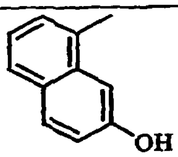 | (3aα,4β,7β,7aα)-Hexahydro-2-(7-hydroxy-1-naphthalenyl)-4,7-epoxy-1H-isoindole-1,3(2H)-dione | 2.54 LCMS/ 308.23 $[M-H]^-$ | 8 |
| 45 | 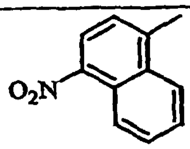 | (3aα,4β,7β,7aα)-Hexahydro-2-(4-nitro-1-naphthalenyl)-4,7-epoxy-1H-isoindole-1,3(2H)-dione | 3.117 LCMS/ 404.11 $[M+CH_3OH+H]^+$ | 8 |
| 46 | 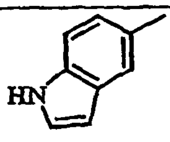 | (3aα,4β,7β,7aα)-Hexahydro-2-(1H-indol-5-yl)-4,7-epoxy-1H-isoindole-1,3(2H)-dione | 2.39 LCMS/ 283.23 $[M+H]^+$ | 8 |
| 47 | 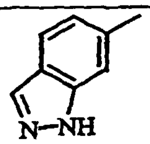 | (3aα,4β,7β,7aα)-Hexahydro-2-(1H-indazol-6-yl)-4,7-epoxy-1H-isoindole-1,3(2H)-dione | 2.35 LCMS/ 282.23 $[M-H]^-$ | 8 |
| 48 | 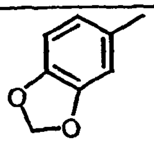 | (3aα,4β,7β,7aα)-2-(1,3-Benzodioxol-5-yl)hexahydro-4,7-epoxy-1H-isoindole-1,3(2H)-dione | 2.47 LCMS/ 288.20 $[M+H]^+$ | 8 |

| Ex. No. | G | Compound Name | Retention Time (Min.)/ Molecular Mass | Pro. of Ex. |
|---|---|---|---|---|
| 49 | 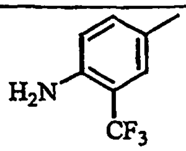 | (3aα,4β,7β,7aα)-2-[4-Amino-3-(trifluoromethyl)phenyl]hexahydro-4,7-epoxy-1H-isoindole-1,3(2H)-dione | 2.71 LCMS/ 327.20 $[M+H]^+$ | 8 |
| 50 | 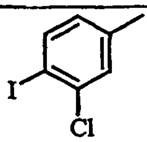 | (3aα,4β,7β,7aα)-2-(3-Chloro-4-iodophenyl)hexahydro-4,7-epoxy-1H-isoindole-1,3(2H)-dione | 3.70 LCMS/ 435.2 $[M+CH_3OH]^{+\cdot}$ | 8 |
| 51 | 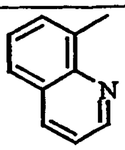 | (3aα,4β,7β,7aα)-Hexahydro-2-(8-quinolinyl)-4,7-epoxy-1H-isoindole-1,3(2H)-dione | 2.28 LCMS/ 295.22 $[M+H]^+$ | 8 |
| 52 | 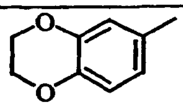 | (3aα,4β,7β,7aα)-2-(2,3-Dihydro-1,4-benzodioxin-6-yl)hexahydro-4,7-epoxy-1H-isoindole-1,3(2H)-dione | 2.55 LCMS/ 302.23 $[M+H]^+$ | 8 |
| 53 | 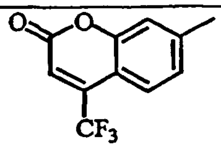 | (3aα,4β,7β,7aα)-Hexahydro-2-[2-oxo-4-(trifluoromethyl)-2H-1-benzopyran-7-yl]-4,7-epoxy-1H-isoindole-1,3(2H)-dione | 3.38 LCMS/ 412.17 $[M+CH_3OH+H]^+$ | 8 |
| 54 | 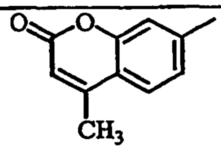 | (3aα,4β,7β,7aα)-Hexahydro-2-(4-methyl-2-oxo-2H-1-benzopyran-7-yl)-4,7-epoxy-1H-isoindole-1,3(2H)-dione | 2.74 LCMS/ 326.20 $[M+H]^+$ | 8 |
| 55 | 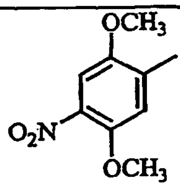 | (3aα,4β,7β,7aα)-2-(2,5-Dimethoxy-4-nitrophenyl)hexahydro-4,7-epoxy-1H-isoindole-1,3(2H)-dione | 2.70 LCMS/ 349.23 $[M+H]^+$ | 8 |
| 56 | 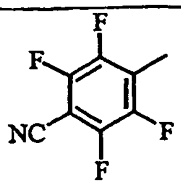 | (3aα,4β,7β,7aα)-2,3,5,6-Tetrafluoro-4-(octahydro-1,3-dioxo-4,7-epoxy-2H-isoindol-2-yl)benzonitrile | 2.97 LCMS | 8 |

| Ex. No. | G | Compound Name | Retention Time (Min.)/ Molecular Mass | Pro. of Ex. |
|---|---|---|---|---|
| 57 | 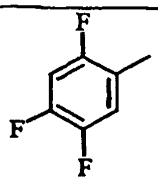 | (3aα,4β,7β,7aα)-Hexahydro-2-(2,4,5-trifluorophenyl)-4,7-epoxy-1H-isoindole-1,3(2H)-dione | 2.90 LCMS | 8 |
| 58 | 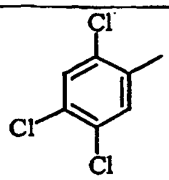 | (3aα,4β,7β,7aα)-Hexahydro-2-(2,4,5-trichlorophenyl)-4,7-epoxy-1H-isoindole-1,3(2H)-dione | 3.64 LCMS/ 346.39 $[M]^{+\cdot}$ | 8 |
| 59 | 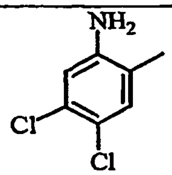 | (3aα,4β,7β,7aα)-2-(2-Amino-4,5-dichlorophenyl)hexahydro-4,7-epoxy-1H-isoindole-1,3(2H)-dione | 3.23 LCMS | 8 |
| 60 | 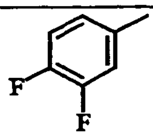 | (3aα,4β,7β,7aα)-2-(3,4-Difluorophenyl)hexahydro-4,7-epoxy-1H-isoindole-1,3(2H)-dione | 2.91 LCMS/ 280.23 $[M+H]^+$ | 8 |
| 61 | 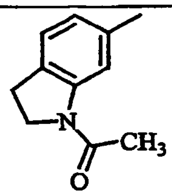 | (3aα,4β,7β,7aα)-1-Acetyl-2,3-dihydro-6-(octahydro-1,3-dioxo-4,7-epoxy-2H-isoindol-2-yl)-1H-indole | 2.43 LCMS/ 359.26 $[M+CH_3OH+H]^+$ | 8 |
| 62 | 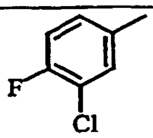 | (3aα,4β,7β,7aα)-2-(3-Chloro-4-fluorophenyl)hexahydro-4,7-epoxy-1H-isoindole-1,3(2H)-dione | 3.21 LCMS/ 328.14 $[M+CH_3OH+H]^+$ | 8 |
| 63 | 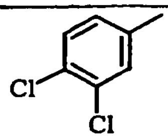 | (3aα,4β,7β,7aα)-2-(3,4-Dichlorophenyl)hexahydro-4,7-epoxy-1H-isoindole-1,3(2H)-dione | 3.54 LCMS/ 311.79 $[M-H]^-$ | 8 |
| 64 | 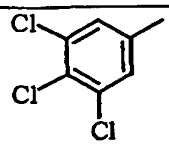 | (3aα,4β,7β,7aα)-Hexahydro-2-(3,4,5-trichlorophenyl)-4,7-epoxy-1H-isoindole-1,3(2H)-dione | 4.05 LCMS/ 378.10 $[M+CH_3OH+H]^+$ | 8 |

| Ex. No. | G | Compound Name | Retention Time (Min.)/ Molecular Mass | Pro. of Ex. |
|---|---|---|---|---|
| 65 | 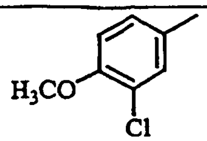 | (3aα,4β,7β,7aα)-2-(3-Chloro-4-methoxyphenyl)hexahydro-4,7-epoxy-1H-isoindole-1,3(2H)-dione | 2.99 LCMS/ 308.11 $[M+H]^+$ | 8 |
| 66 | 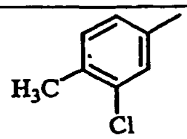 | (3aα,4β,7β,7aα)-2-(3-Chloro-4-methylphenyl)hexahydro-4,7-epoxy-1H-isoindole-1,3(2H)-dione | 3.39 LCMS/ 292.20 $[M+H]^+$ | 8 |
| 67 | 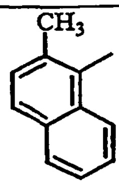 | (3aα,4β,7β,7aα)-Hexahydro-2-(2-methyl-1-naphthalenyl)-4,7-epoxy-1H-isoindole-1,3(2H)-dione | 3.28 LCMS/ 308.23 $[M+H]^+$ | 8 |
| 68 | 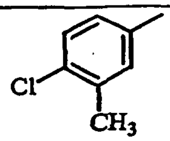 | (3aα,4β,7β,7aα)-2-(4-Chloro-3-methylphenyl)hexahydro-4,7-epoxy-1H-isoindole-1,3(2H)-dione | 3.40 LCMS/ 292.20 $[M+H]^+$ | 8 |
| 69 | 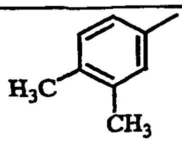 | (3aα,4β,7β,7aα)-2-(3,4-Dimethylphenyl)hexahydro-4,7-epoxy-1H-isoindole-1,3(2H)-dione | 3.11 LCMS/ 272.23 $[M+H]^+$ | 8 |
| 70 | 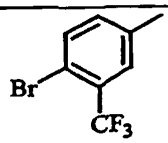 | (3aα,4β,7β,7aα)-2-[4-Bromo-3-(trifluoromethyl)phenyl]hexahydro-4,7-epoxy-1H-isoindole-1,3(2H)-dione | 3.76 LCMS/ 421.98 $[M+CH_3OH+H]^+$ | 8 |
| 71 | 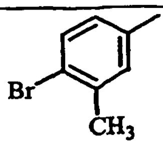 | (3aα,4β,7β,7aα)-2-(4-Bromo-3-methylphenyl)hexahydro-4,7-epoxy-1H-isoindole-1,3(2H)-dione | 3.50 LCMS/ 336.05 $[M+H]^+$ | 8 |
| 72 | 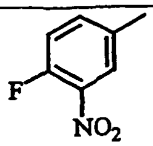 | (3aα,4β,7β,7aα)-2-(4-Fluoro-3-nitrophenyl)hexahydro-4,7-epoxy-1H-isoindole-1,3(2H)-dione | 2.80 LCMS/ 305.25 $[M-H]^-$ | 8 |

| Ex. No. | G | Compound Name | Retention Time (Min.)/ Molecular Mass | Pro. of Ex. |
|---|---|---|---|---|
| 73 | 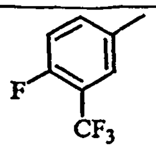 | (3aα,4β,7β,7aα)-2-[4-Fluoro-3-(trifluoromethyl)phenyl]hexahydro-4,7-epoxy-1H-isoindole-1,3(2H)-dione | 3.45 LCMS/ 362.26 $[M+CH_3OH+H]^+$ | 8 |
| 74 | 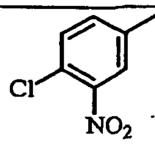 | (3aα,4β,7β,7aα)-2-(4-Chloro-3-nitrophenyl)hexahydro-4,7-epoxy-1H-isoindole-1,3(2H)-dione | 3.19 LCMS/ 322.86 $[M]^{+\cdot}$ | 8 |
| 75 | 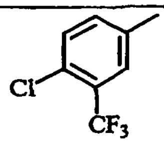 | (3aα,4β,7β,7aα)-2-[4-Chloro-3-(trifluoromethyl)phenyl]hexahydro-4,7-epoxy-1H-isoindole-1,3(2H)-dione | 3.68 LCMS/ 345.83 $[M]^{+\cdot}$ | 8 |
| 76 | 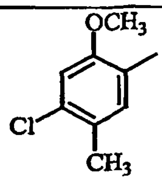 | (3aα,4β,7β,7aα)-2-(4-Chloro-2-methoxy-5-methylphenyl)hexahydro-4,7-epoxy-1H-isoindole-1,3(2H)-dione | 3.31 LCMS/ 322.20 $[M+H]^+$ | 8 |
| 77 | 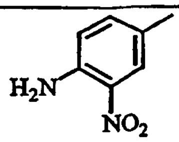 | (3aα,4β,7β,7aα)-2-(4-Amino-3-nitrophenyl)hexahydro-4,7-epoxy-1H-isoindole-1,3(2H)-dione | 2.34 LCMS/ 302.27 $[M-H]^-$ | 8 |
| 78 | 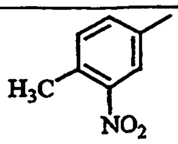 | (3aα,4β,7β,7aα)-Hexahydro-2-(4-methyl-3-nitrophenyl)-4,7-epoxy-1H-isoindole-1,3(2H)-dione | 3.02 LCMS/ 335.20 $[M+CH_3OH+H]^+$ | 8 |
| 79 | 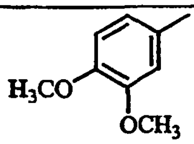 | (3aα,4β,7β,7aα)-2-(3,4-Dimethoxyphenyl)hexahydro-4,7-epoxy-1H-isoindole-1,3(2H)-dione | 2.35 LCMS/ 304.25 $[M+H]^+$ | 8 |
| 80 | 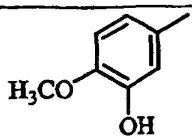 | (3aα,4β,7β,7aα)-Hexahydro-2-(3-hydroxy-4-methoxyphenyl)-4,7-epoxy-1H-isoindole-1,3(2H)-dione | 0.98 LCMS/ 321.19 $[M+CH_3OH]^{+\cdot}$ | 8 |

| Ex. No. | G | Compound Name | Retention Time (Min.)/ Molecular Mass | Pro. of Ex. |
|---|---|---|---|---|
| 81 | 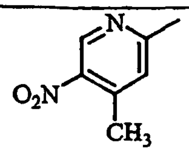 | (3aα,4β,7β,7aα)-Hexahydro-2-(4-methyl-5-nitro-2-pyridinyl)-4,7-epoxy-1H-isoindole-1,3(2H)-dione | 0.54 LCMS/ 304.20 $[M+H]^+$ | 8 |
| 82 | 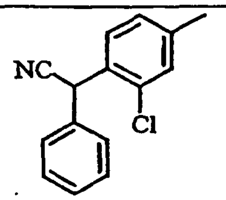 | (3aα,4β,7β,7aα)-2-Chloro-4-(octahydro-1,3-dioxo-4,7-epoxy-2H-isoindol-2-yl)-α-phenylbenzeneacetonitrile | 3.67 LCMS/ 423.8 $[M+CH_3OH]^{+\cdot}$ | 8 |
| 83 | 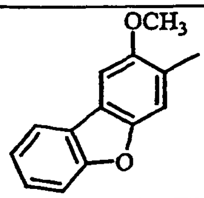 | (3aα,4β,7β,7aα)-Hexahydro-2-(2-methoxy-3-dibenzofuranyl)-4,7-epoxy-1H-isoindole-1,3(2H)-dione | 3.66 LCMS/ 364.25 $[M+H]^+$ | 8 |
| 84 | 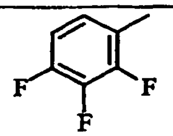 | (3aα,4β,7β,7aα)-Hexahydro-2-(2,3,4-trifluorophenyl)-4,7-epoxy-1H-isoindole-1,3(2H)-dione | 3.06 LCMS/ 298.14 $[M+H]^+$ | 8 |
| 85 | 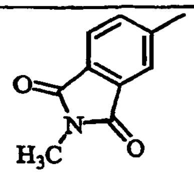 | (3aα,4β,7β,7aα)-2-(2,3-Dihydro-2-methyl-1,3-dioxo-1H-isoindol-5-yl)hexahydro-4,7-epoxy-1H-isoindole-1,3(2H)-dione | 2.70 LCMS/ 359.22 $[M+CH_3OH+H]^+$ | 8 |
| 86 | 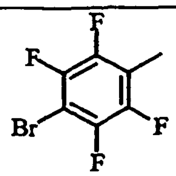 | (3aα,4β,7β,7aα)-2-(4-Bromo-2,3,5,6-tetrafluorophenyl)hexahydro-4,7-epoxy-1H-isoindole-1,3(2H)-dione | 3.72 LCMS/ 426.07 $[M+CH_3OH+H]^+$ | 8 |
| 87 | 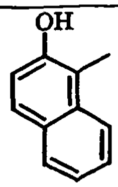 | (3aα,4β,7β,7aα)-Hexahydro-2-(2-hydroxy-1-naphthalenyl)-4,7-epoxy-1H-isoindole-1,3(2H)-dione | 2.52 LCMS/ 308.26 $[M-H]^-$ | 8 |
| 88 | 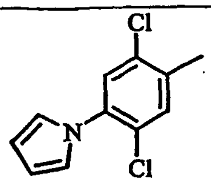 | (3aα,4β,7β,7aα)-2-[2,5-Dichloro-4-(1H-pyrrol-1-yl)phenyl]hexahydro-4,7-epoxy-1H-isoindole-1,3(2H)-dione | 3.70 LCMS/ 376.64 $[M-H]^-$ | 8 |

| Ex. No. | G | Compound Name | Retention Time (Min.)/ Molecular Mass | Pro. of Ex. |
|---|---|---|---|---|
| 89 | 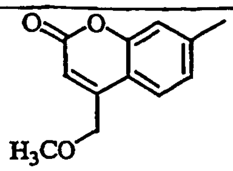 | (3aα,4β,7β,7aα)-Hexahydro-2-[4-(methoxymethyl)-2-oxo-2H-1-benzopyran-7-yl]-4,7-epoxy-1H-isoindole-1,3(2H)-dione | 2.79 LCMS/ 356.26 $[M+H]^+$ | 8 |
| 90 | 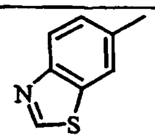 | (3aα,4β,7β,7aα)-2-(6-Benzothiazolyl)hexahydro-4,7-epoxy-1H-isoindole-1,3(2H)-dione | 2.46 LCMS/ 301.19 $[M+H]^+$ | 8 |
| 91 | 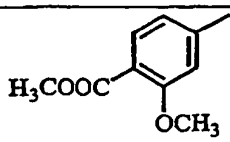 | (3aα,4β,7β,7aα)-2-Methoxy-4-(octahydro-1,3-dioxo-4,7-epoxy-2H-isoindol-2-yl)benzoic acid methyl ester | 2.75 LCMS/ 332.25 $[M+H]^+$ | 8 |
| 92 | 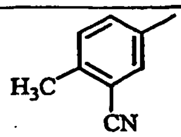 | (3aα,4β,7β,7aα)-2-Methyl-5-(octahydro-1,3-dioxo-4,7-epoxy-2H-isoindol-2-yl)benzonitrile | 2.80 LCMS/ 315.26 $[M+CH_3OH+H]^+$ | 8 |
| 93 | 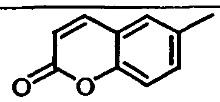 | (3aα,4β,7β,7aα)-Hexahydro-2-(2-oxo-2H-1-benzopyran-6-yl)-4,7-epoxy-1H-isoindole-1,3(2H)-dione | 2.45 LCMS/ 312.20 $[M+H]^+$ | 8 |
| 94 | 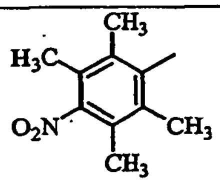 | (3aα,4β,7β,7aα)-Hexahydro-2-(2,3,5,6-tetramethyl-4-nitrophenyl)-4,7-epoxy-1H-isoindole-1,3(2H)-dione | 3.59 LCMS/ 377.25 $[M+CH_3OH+H]^+$ | 8 |
| 95 | 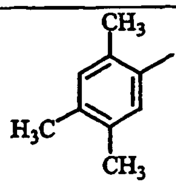 | (3aα,4β,7β,7aα)-Hexahydro-2-(2,4,5-trimethylphenyl)-4,7-epoxy-1H-isoindole-1,3(2H)-dione | 3.33 LCMS/ 286,30 $[M+H]^+$ | 8 |
| 96 | 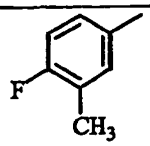 | (3aα,4β,7β,7aα)-2-(4-Fluoro-3-methylphenyl)hexahydro-4,7-epoxy-1H-isoindole-1,3(2H)-dione | 3.00 LCMS/ 276.23 $[M+H]^+$ | 8 |

| Ex. No. | G | Compound Name | Retention Time (Min.)/ Molecular Mass | Pro. of Ex. |
|---|---|---|---|---|
| 97 | 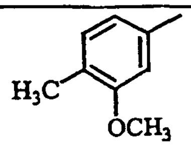 | (3aα,4β,7β,7aα)-Hexahydro-2-(3-methoxy-4-methylphenyl)-4,7-epoxy-1H-isoindole-1,3(2H)-dione | 3.05 LCMS/ 288.23 $[M+H]^+$ | 8 |
| 98 | 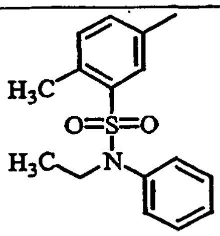 | (3aα,4β,7β,7aα)-N-Ethyl-2-methyl-5-(octahydro-1,3-dioxo-4,7-epoxy-2H-isoindol-2-yl)-N-phenylbenzenesulfonamide | 3.56 LCMS/ 441.26 $[M+H]^+$ | 8 |
| 99 | 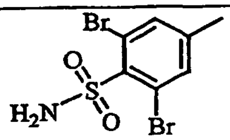 | (3aα,4β,7β,7aα)-2,6-Dibromo-4-(octahydro-1,3-dioxo-4,7-epoxy-2H-isoindol-2-yl)benzenesulfonamide | 2.25 LCMS | 8 |
| 100 | 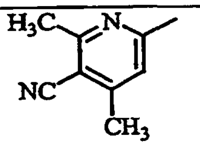 | (3aα,4β,7β,7aα)-2,4-Dimethyl-6-(octahydro-1,3-dioxo-4,7-epoxy-2H-isoindol-2-yl)-3-pyridinecarbonitrile | 2.75 LCMS/ 298.23 $[M+H]^+$ | 8 |
| 101 | 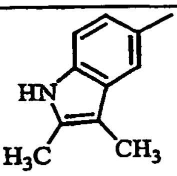 | (3aα,4β,7β,7aα)-2-(2,3-Dimethyl-1H-indol-5-yl)hexahydro-4,7-epoxy-1H-isoindole-1,3(2H)-dione | 3.00 LCMS/ 311.26 $[M+H]^+$ | 8 |
| 102 | 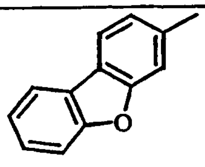 | (3aα,4β,7β,7aα)-2-(3-Dibenzofuranyl)hexahydro-4,7-epoxy-1H-isoindole-1,3(2H)-dione | 3.72 LCMS/ 366.23 $[M+CH_3OH+H]^+$ | 8 |
| 103 | 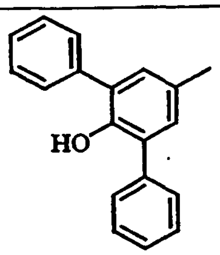 | (3aα,4β,7β,7aα)-Hexahydro-2-(2'-hydroxy[1,1':3',1"-terphenyl]-5'-yl)-4,7-epoxy-1H-isoindole-1,3(2H)-dione | 3.70 LCMS/ 412.23 $[M+H]^+$ | 8 |
| 104 | 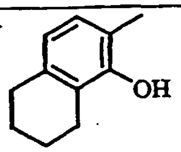 | (3aα,4β,7β,7aα)-Hexahydro-2-(5,6,7,8-tetrahydro-3-hydroxy-2-naphthalenyl)-4,7-epoxy-1H-isoindole-1,3(2H)-dione | 3.24 LCMS/ 312.32 $[M+H]^+$ | 8 |

| Ex. No. | G | Compound Name | Retention Time (Min.)/ Molecular Mass | Pro. of Ex. |
|---|---|---|---|---|
| 105 | 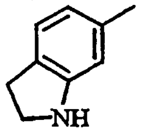 | (3aα,4β,7β,7aα)-2-(2,3-Dihydro-1H-indol-6-yl)hexahydro-4,7-epoxy-1H-isoindole-1,3(2H)-dione | 2.42 LCMS/ 285.29 $[M+H]^+$ | 8 |
| 106 | 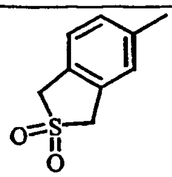 | (3aα,4β,7β,7aα)-2-(1,3-Dihydro-2,2-dioxidobenzo[c]thiophen-5-yl)hexahydro-4,7-epoxy-1H-isoindole-1,3(2H)-dione | 1.99 LCMS/ 366.26 $[M+CH_3OH+H]^+$ | 8 |
| 107 | 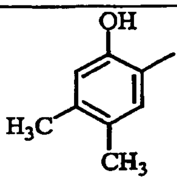 | (3aα,4β,7β,7aα)-Hexahydro-2-(2-hydroxy-4,5-dimethylphenyl)-4,7-epoxy-1H-isoindole-1,3(2H)-dione | 2.78 LCMS/ 286.32 $[M-H]^-$ | 8 |
| 108 | 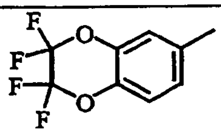 | (3aα,4β,7β,7aα)-2-(2,3-Dihydro-2,2,3,3-tetrafluoro-1,4-benzodioxin-6-yl)hexahydro-4,7-epoxy-1H-isoindole-1,3(2H)-dione | 3.82 LCMS/ 406.19 $[M+CH_3OH+H]^+$ | 8 |
| 109 | 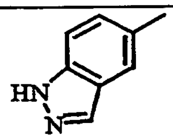 | (3aα,4β,7β,7aα)-Hexahydro-2-(1H-indazol-5-yl)-4,7-epoxy-1H-isoindole-1,3(2H)-dione | 2.13 LCMS/ 284.23 $[M+H]^+$ | 8 |
| 110 | 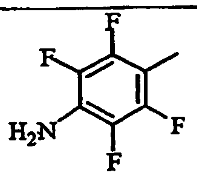 | (3aα,4β,7β,7aα)-2-(4-Amino-2,3,5,6-tetrafluorophenyl)-hexahydro-4,7-epoxy-1H-isoindole-1,3(2H)-dione | 2.60 LCMS/ 363.22 $[M+CH_3OH+H]^+$ | 8 |
| 111 | 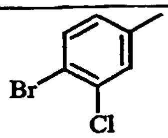 | (3aα,4β,7β,7aα)-2-(4-Bromo-3-chlorophenyl)hexahydro-4,7-epoxy-1H-isoindole-1,3(2H)-dione | 3.64 LCMS/ 389.64 $[M+CH_3OH+H]^+$ | 8 |
| 112 | 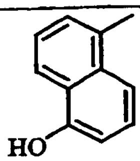 | (3aα,4β,7β,7aα)-Hexahydro-2-(5-hydroxy-1-naphthalenyl)-4,7-epoxy-1H-isoindole-1,3(2H)-dione | 2.48 LCMS/ 308.27 $[M-H]^-$ | 8 |

| Ex. No. | G | Compound Name | Retention Time (Min.)/ Molecular Mass | Pro. of Ex. |
|---|---|---|---|---|
| 113 | 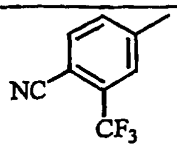 | (3aα,4β,7β,7aα)-4-(Octahydro-1,3-dioxo-4,7-epoxy-2H-isoindol-2-yl)-2-(trifluoromethyl)benzonitrile | 3.28 LCMS/ 337.16 $[M+H]^+$ | 8 |
| 114 | 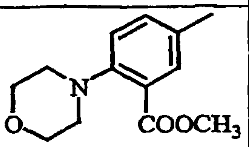 | (3aα,4β,7β,7aα)-2-(4-Morpholinyl)-5-(octahydro-1,3-dioxo-4,7-epoxy-2H-isoindol-2-yl)benzoic acid methyl ester | 2.72 LCMS/ 387.17 $[M+H]^+$ | 8 |
| 115 | 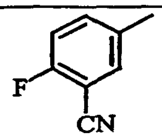 | (3aα,4β,7β,7aα)-2-Fluoro-5-(octahydro-1,3-dioxo-4,7-epoxy-2H-isoindol-2-yl)benzonitrile | 2.69 LCMS/ 319.26 $[M+CH_3OH+H]^+$ | 8 |
| 116 | 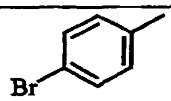 | (3aα,4β,7β,7aα)-2-(4-Bromophenyl)hexahydro-4,7-epoxy-1H-isoindole-1,3(2H)-dione | 5.80 LCMS/ 393.0 $[M+H]^+$ | 8 |
| 117 | 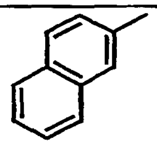 | (3aα,4β,7β,7aα)-Hexahydro-2-(2-naphthalenyl)-4,7-epoxy-1H-isoindole-1,3(2H)-dione | 6.92 LCMS/ 333.7 $[M+H]^+$ | 8 |
| 118 | 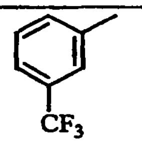 | (3aα,4β,7β,7aα)-Hexahydro-2-[3-(trifluoromethyl)phenyl]-4,7-epoxy-1H-isoindole-1,3(2H)-dione | 3.27 LCMS/ 312.2 $[M+H]^+$ | 8 |
| 119 | 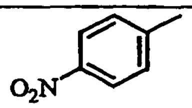 | (3aα,4β,7β,7aα)-Hexahydro-2-(4-nitrophenyl)-4,7-epoxy-1H-isoindole-1,3(2H)-dione | 2.88 LCMS/ 343.2 $[M+H]^+$ | 8 |
| 120 | 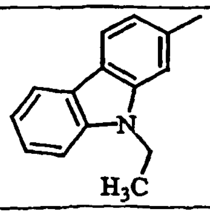 | (3aα,4β,7β,7aα)-2-(9-Ethyl-9H-carbazol-3-yl)hexahydro-4,7-epoxy-1H-isoindole-1,3(2H)-dione | 3.73 LCMS/ 360.1 $[M+H]^+$ | 8 |

| Ex. No. | G | Compound Name | Retention Time (Min.)/ Molecular Mass | Pro. of Ex. |
|---|---|---|---|---|
| 121 | 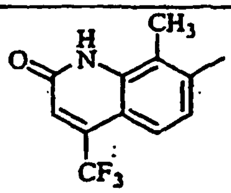 | (3aα,4β,7β,7aα)-2-[1,2-Dihydro-8-methyl-2-oxo-4-(trifluoromethyl)-7-quinolinyl]hexahydro-4,7-epoxy-1H-isoindole-1,3(2H)-dione | 3.11 LCMS/ 393.0 $[M+H]^+$ | 8 |

Examples 116, 118 and 119 are Reference Examples.

**Examples 122 to 164**

Further compounds of the present invention were prepared by procedures analogous to those described above. **Table 3** provides the compound name and structure, retention time, as well as the Example number of the procedure on which the preparation of **Table 3** was based, for the compounds of Examples 122 to 164. The chromatography techniques used to determine the compound retention times of **Table 3** are as follows:
LCMS = YMC S5 ODS column, 4.6 x 50 mm eluting with 10-90% MeOH/$H_2O$ over 4 minutes containing 0.1% TFA; 4 mL/min, monitoring at 220 nm.
LC = YMC S5 ODS column 4.6 x 50 mm eluting with 10-90% MeOH/$H_2O$ over 4 minutes containing 0.2% phosphoric acid, 4 mL/min, monitoring at 220 nm

## Table 3

| Ex. No. | Compound Structure | Compound Name | Retention Time Min./ Molecular Mass | Pro. of Ex. |
|---|---|---|---|---|
| 122 | 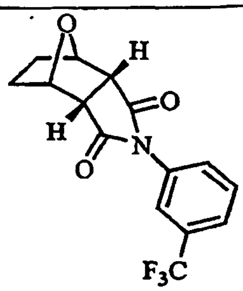 | (3aα,4α,7α,7aα)-Hexahydro-2-[3-(trifluoromethyl)phenyl]-4,7-epoxy-1H-isoindole-1,3(2H)-dione | 2.66 LCMS | 27 |
| 123 | 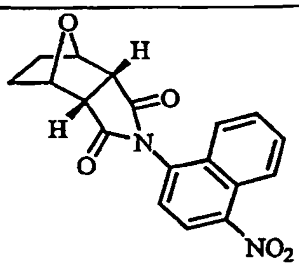 | (3aα,4α,7α,7aα)-Hexahydro-2-(4-nitro-1-naphthalenyl)-4,7-epoxy-1H-isoindole-1,3(2H)-dione | 2.76 LCMS | 27 |
| 124 | 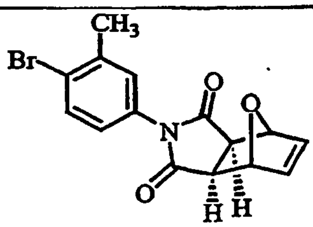 | (3aα,4β,7β,7aα)-2-(4-Bromo-3-methylphenyl)-3a,4,7,7a-tetrahydro-4,7-epoxy-1H-isoindole-1,3(2H)-dione | 6.36 LCMS | 8 |
| 125 | 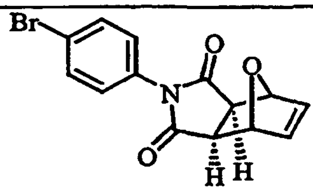 | (3aα,4β,7β,7aα)-2-(4-Bromophenyl)-3a,4,7,7a-tetrahydro-4,7-epoxy-1H-isoindole-1,3(2H)-dione | 5.72 LCMS | 8 |
| 126 | 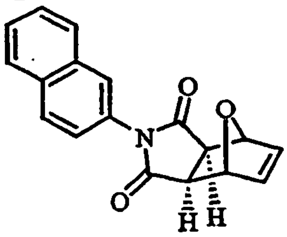 | (3aα,4β,7β,7aα)-3a,4,7,7a-Tetrahydro-2-(2-naphthalenyl)-4,7-epoxy-1H-isoindole-1,3(2H)-dione | 5.92 LCMS | 8 |

| Ex. No. | Compound Structure | Compound Name | Retention Time Min./ Molecular Mass | Pro. of Ex. |
|---|---|---|---|---|
| 127 | 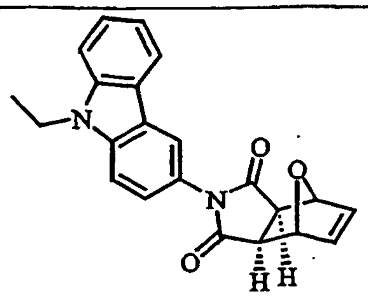 | (3aα,4β,7β,7aα)-2-(9-Ethyl-9H-carbazol-3-yl)-3a,4,7,7a-tetrahydro-4,7-epoxy-1H-isoindole-1,3(2H)-dione | 3.73 LCMS | 8 |
| 128 | 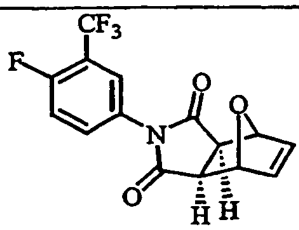 | (3aα,4β,7β,7aα)-2-[4-Fluoro-3-(trifluoromethyl)phenyl]-3a,4,7,7a-tetrahydro-4,7-epoxy-1H-isoindole-1,3(2H)-dione | 3.40 LCMS | 8 |
| 129 | 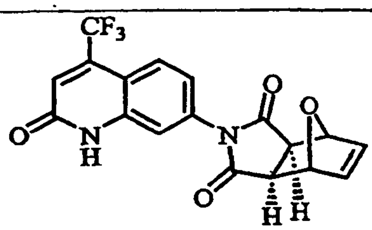 | (3aα,4β,7β,7aα)-2-[1,2-Dihydro-8-methyl-2-oxo-4-(trifluoromethyl)-7-quinolinyl]-3a,4,7,7a-tetrahydro-4,7-epoxy-1H-isoindole-1,3(2H)-dione | 3.14 LCMS | 8 |
| 130 | 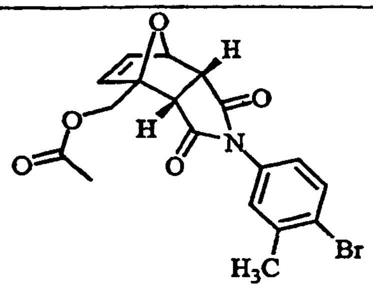 | (3aα,4α,7α,7aα)-4-[(Acetyloxy)methyl]-2-(4-bromo-3-methylphenyl)-3a,4,7,7a-tetrahydro-4,7-epoxy-1H-isoindole-1,3(2H)-dione | 2.95 LC | 4 |
| 131 | 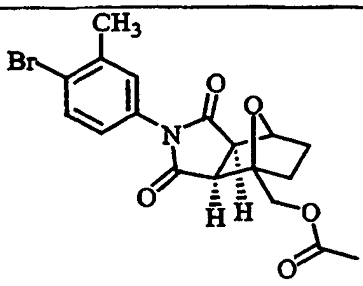 | (3aα,4β,7β,7aα)-4-[(Acetyloxy)methyl]-2-(4-bromo-3-methylphenyl)-3a,4,7,7a-tetrahydro-4,7-epoxy-1H-isoindole-1,3(2H)-dione. | 2.97 LCMS | 5 |

| Ex. No. | Compound Structure | Compound Name | Retention Time Min./ Molecular Mass | Pro. of Ex. |
|---|---|---|---|---|
| 132 | 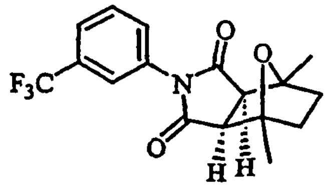 | (3aα,4β,7β,7aα)-Hexahydro-4,7-dimethyl-2-[3-(trifluoromethyl)phenyl]-4,7-epoxy-1H-isoindole-1,3(2H)-dione | 3.08 LC | 20 |
| 133 | 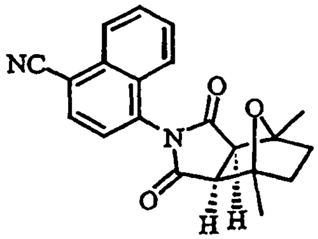 | (3aα,4β,7β,7aα)-4-(Octahydro-4,7-dimethyl-1,3-dioxo-4,7-epoxy-2H-isoindol-2-yl)-1-naphthalenecarbonitrile | 3.00 LC | 20 |
| 134 | 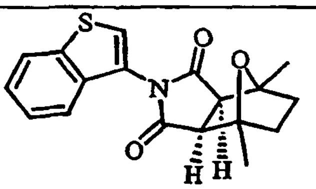 | (3aα,4β,7β,7aα)-(Benzo[b]thiophen-3-yl)hexahydro-4,7-dimethyl-4,7-epoxy-1H-isoindole-1,3(2H)-dione. | 3.61 LC | 20 |
| 135 | 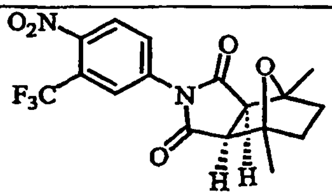 | (3aα,4β,7β,7aα)-Hexahydro-4,7-dimethyl-2-[4-nitro-3-(trifluoromethyl)phenyl]-4,7-epoxy-1H-isoindole-1,3(2H)-dione | 3.21 LC | 20 |
| 136 | 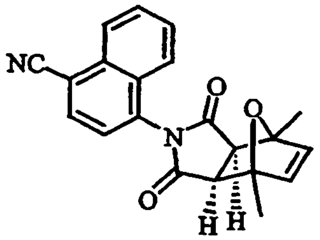 | (3aα,4β,7β,7aα)-4-(1,3,3a,4,7,7a-Hexahydro-4,7-dimethyl-1,3-dioxo-4,7-epoxy-2H-isoindol-2-yl)-1-naphthalenecarbonitrile | 2.94 LC | 32 |
| 137 | 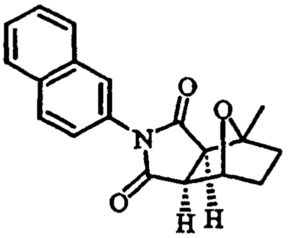 | (3aα,4α,7α,7aα)-Hexahydro-4-methyl-2-(2-naphthalenyl)-4,7-epoxy-1H-isoindole-1,3(2H)-dione | 2.88 LC | 3 |

| Ex. No. | Compound Structure | Compound Name | Retention Time Min./ Molecular Mass | Pro. of Ex. |
|---|---|---|---|---|
| 138 | 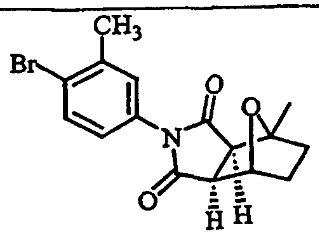 | (3aα,4β,7β,7aα)-2-(4-Bromo-3-methylphenyl)hexahydro-4-methyl-4,7-epoxy-1H-isoindole-1,3(2H)-dione | 3.11 LC | 3 |
| 139 | 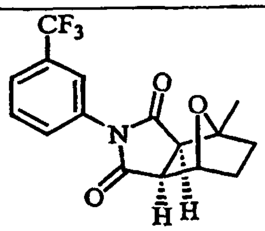 | (3aα,4β,7β,7aα)-Hexahydro-4-methyl-2-[3-(trifluoromethyl)phenyl]-4,7-epoxy-1H-isoindole-1,3(2H)-dione | 2.90 LC | 3 |
| 140 | 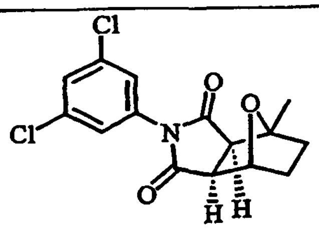 | (3aα,4β,7β,7aα)-2-(3,5-Dichlorophenyl)hexahydro-4-methyl-4,7-epoxy-1H-isoindole-1,3(2H)-dione | 3.31 LC | 3 |
| 141 | 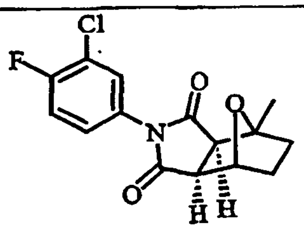 | (3aα,4β,7β,7aα)-2-(3-Chloro-4-fluorophenyl)-hexahydro-4-methyl-4,7-epoxy-1H-isoindole-1,3(2H)-dione | 2.72 LC | 3 |
| 142 | 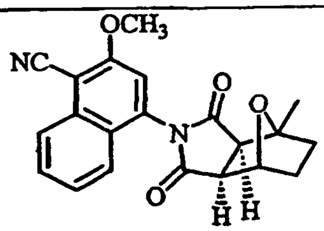 | (3aα,4β,7β,7aα)-2-Methoxy-4-(octahydro-1,3-dioxo-4-methyl-4,7-epoxy-2H-isoindol-2-yl)-1-naphthalenecarbonitrile | 2.72 LC | 3 |
| 143 | 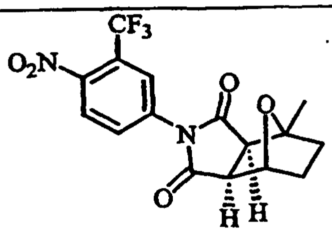 | (3aα,4β,7β,7aα)-Hexahydro-4-methyl-2-[4-nitro-3-(trifluoromethyl)phenyl]-4,7-epoxy-1H-isoindole-1,3(2H)-dione | 3.10 LC | 3 |

| Ex. No. | Compound Structure | Compound Name | Retention Time Min./ Molecular Mass | Pro. of Ex. |
|---|---|---|---|---|
| 144 | 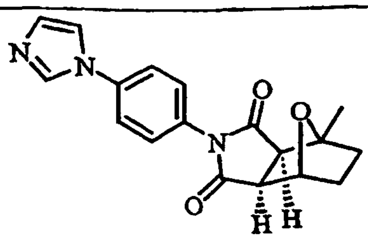 | (3aα,4β,7β,7aα)-Hexahydro-2-[4-(1H-imidazol-1-yl)phenyl]-4-methyl-4,7-epoxy-1H-isoindole-1,3(2H)-dione | 1.16 LC | 3 |
| 145 | 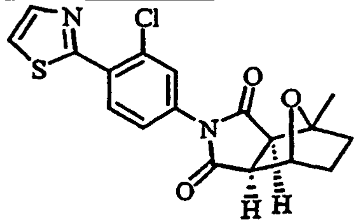 | (3aα,4β,7β,7aα)-2-[3-Chloro-4-(2-thiazolyl)phenyl]hexahydro-4-methyl-4,7-epoxy-1H-isoindole-1,3(2H)-dione | 2.81 LC | 3 |
| 146 | 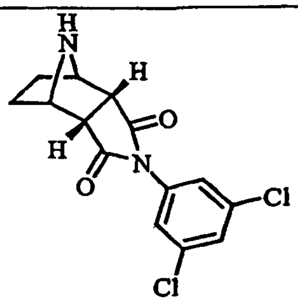 | (3aα,4α,7α,7aα)-2-(3,5-Dichlorophenyl)hexahydro-4,7-imino-1H-isoindole-1,3(2H)-dione | 2.72 LC | 31 |
| 147 | 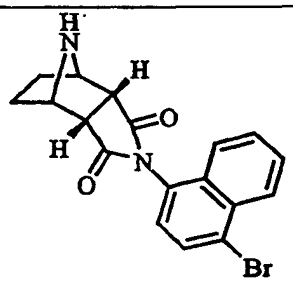 | (3aα,4α,7α,7aα)-2-(4-Bromo-1-naphthalenyl)hexahydro-4,7-imino-1H-isoindole-1,3(2H)-dione | 2.95 LC | 31 |
| 148 | 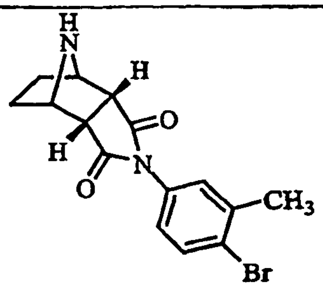 | (3aα,4α,7α,7aα)-2-(4-Bromo-3-methylphenyl)hexahydro-4,7-imino-1H-isoindole-1,3(2H)-dione | 2.65 LC | 31 |

| Ex. No. | Compound Structure | Compound Name | Retention Time Min./ Molecular Mass | Pro. of Ex. |
|---|---|---|---|---|
| 149 | 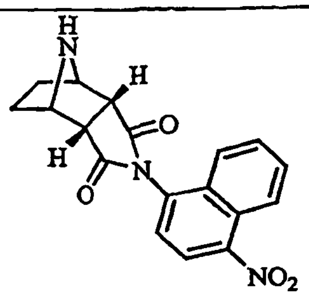 | (3aα,4α,7α,7aα)-Hexahydro-2-(4-nitro-1-naphthalenyl)-4,7-imino-1H-isoindole-1,3(2H)-dione | 2.49 LC | 31 |
| 150 | 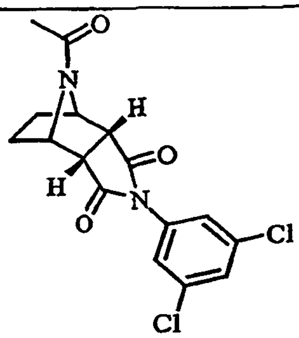 | (3aα,4α,7α,7aα)-8-Acetyl-2-(3,5-dichlorophenyl)hexahydro-4,7-imino-1H-isoindole-1,3(2H)-dione | 3.53 LC | 31 |
| 151 | 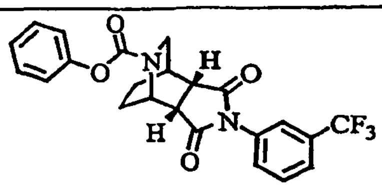 | (3aα,4α,7α,7aα)-Octahydro-1,3-dioxo-2-[3-(trifluoromethyl)phenyl]-4,7-ethano-5H-pyrrolo[3,4-c]pyridine-5-carboxylic acid phenyl ester | 3.397 LC | 9 |
| 152 | 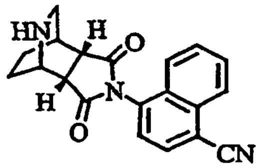 | (3aα,4α,7α,7aα)-4-(Octahydro-1,3-dioxo-4,7-ethano-2H-pyrrolo[3,4-c]pyridin-2-yl)-1-naphthalenecarbonitrile | 1.74 LC | 11 |
| 153 | 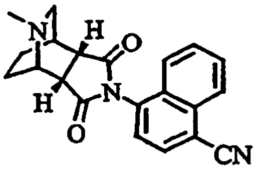 | (3aα,4α,7α,7aα)-4-(Octahydro-5-methyl-1,3-dioxo-4,7-ethano-2H-pyrrolo[3,4-c]pyridin-2-yl)-1-naphthalenecarbonitrile | 1.71 LC | 14 |

| Ex. No. | Compound Structure | Compound Name | Retention Time Min./ Molecular Mass | Pro. of Ex. |
|---|---|---|---|---|
| 154 | 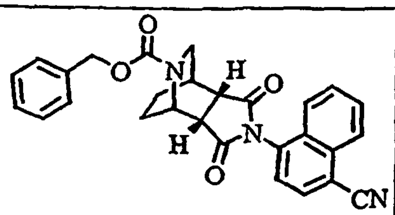 | (3aα,4α,7α,7aα)-2-(4-Cyano-1-naphthalenyl)octahydro-1,3-dioxo-4,7-etheno-5H-pyrrolo[3,4-c]pyridine-5-carboxylic acid phenylmethyl ester | 3.40 LC | 10 |
| 155 | 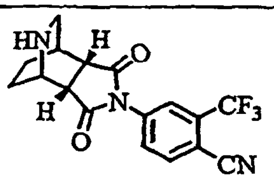 | (3aα,4α,7α,7aα)-4-(Octahydro-1,3-dioxo-4,7-ethano-2H-pyrrolo[3,4-c]pyridin-2-yl)-2-(trifluoromethyl)benzonitrile | 1.74 LC | 11 |
| 156 | 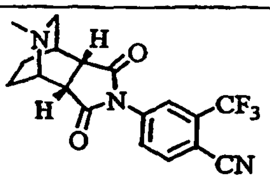 | (3aα,4α,7α,7aα)-4-(Octahydro-5-methyl-1,3-dioxo-4,7-ethano-2H-pyrrolo[3,4-c]pyridin-2-yl)-2-(trifluoromethyl)benzonitrile | 1.65 LC | 14 |
| 157 | 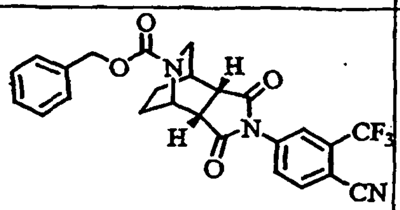 | (3aα,4α,7α,7aα)-2-[4-Cyano-3-(trifluoromethyl)phenyl]octahydro-1,3-dioxo-4,7-etheno-5H-pyrrolo[3,4-c]pyridine-5-carboxylic acid phenylmethyl ester | 3.53 LC | 10 |
| 158 | 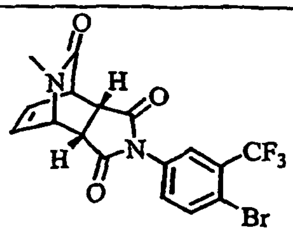 | (3aα,4α,7α,7aα)-2-[4-Bromo-3-(trifluoromethyl)phenyl]tetrahydro-5-methyl-4,7-etheno-1H-pyrrolo[3,4-c]pyridine-1,3,6(2H,5H)-trione | 2.95 LCMS | 34 |

| Ex. No. | Compound Structure | Compound Name | Retention Time Min./ Molecular Mass | Pro. of Ex. |
|---|---|---|---|---|
| 159 | 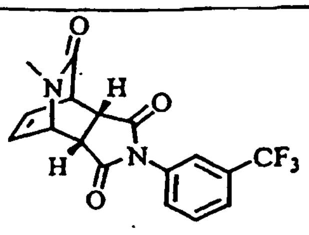 | (3aα,4α,7α,7aα)-Tetrahydro-5-methyl-2-[3-(trifluoromethyl)phenyl]-4,7-etheno-1H-pyrrolo[3,4-c]pyridine-1,3,6(2H,5H)-trione | 2.53 LCMS | 34 |
| 160 | 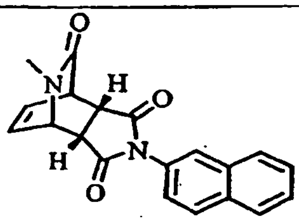 | (3aα,4α,7α,7aα)-Tetrahydro-5-methyl-2-(2-naphthalenyl)-4,7-etheno-1H-pyrrolo[3,4-c]pyridine-1,3,6(2H,5H)-trione | 2.58 LCMS | 34 |
| 161 | 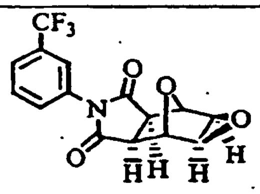 | (1aα,2β,2aα,5aα,6β,6aα)-Hexahydro-4-[3-(trifluoromethyl)phenyl]-2,6-epoxy-3H-oxireno[f]isoindole-3,5(4H)-dione | 1.80 LCMS | 28 |
| 162 | 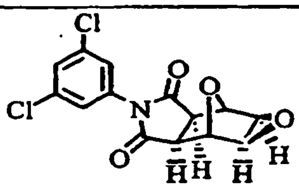 | (1aα,2β,2aα,5aα,6β,6aα)-4-(3,5-Dichlorophenyl)-hexahydro-2,6-epoxy-3H-oxireno[f]isoindole-3,5(4H)-dione | 1.45 LCMS | 28 |
| 163 | 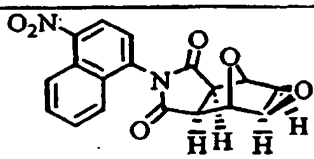 | (1aα,2β,2aα,5aα,6β,6aα)-Hexahydro-4-(4-nitro-1-naphthalenyl)-2,6-epoxy-3H-oxireno[f]isoindole-3,5(4H)-dione | 1.52 LCMS | 28 |
| 164 | 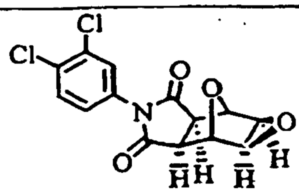 | (1aα,2β,2aα,5aα,6β,6aα)-4-(3,4-Dichlorophenyl)-hexahydro-2,6-epoxy-3H-oxireno[f]isoindole-3,5(4H)-dione | 3.21 LCMS | 28 |

Example 125 is a Reference Example.

Examples 165 to 203

Additional compounds of the present invention were prepared and are described further below in **Table 4. Table 4** sets forth the compound name and structure, as well as the Example number of the procedure on which the preparation of **Table 4** was based, for the compounds of Examples 165 to 203.

**Table 4**

| Ex. No. | Compound Structure | Compound Name | Pro. of Ex. |
|---|---|---|---|
| 165 | 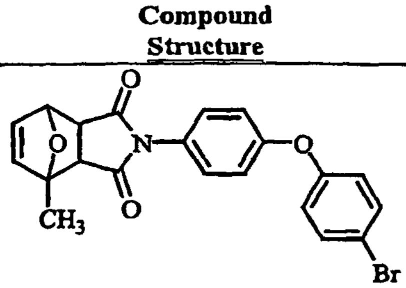 | 2-[4-(4-Bromo-phenoxy)phenyl]-3a,4,7,7a-tetrahydro-4,7-dimethyl-4,7-epoxy-1H-isoindole-1,3(2H)-dione | 32 |
| 166 | 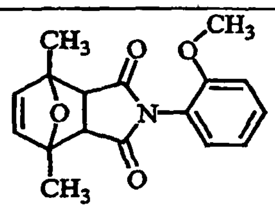 | 3a,4,7,7a-Tetrahydro-2-(2-methoxyphenyl)-4,7-dimethyl-4,7-epoxy-1H-isoindole-1,3(2H)-dione | 32 |
| 167 | 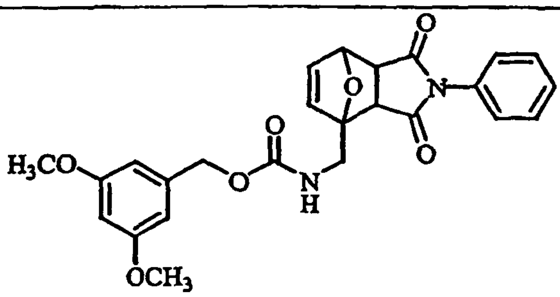 | [(1,2,3,3a,7,7a-Hexahydro-2-phenyl-4,7-epoxy-4H-isoindol-4-yl)methyl]carbamic acid (3,5-dimethoxyphenyl)methyl ester | 21-26 |

| Ex. No. | Compound Structure | Compound Name | Pro. of Ex. |
|---|---|---|---|
| 168 | 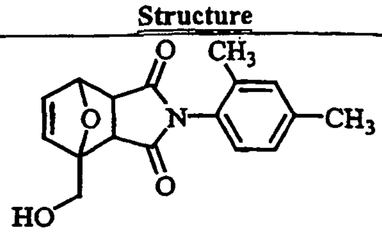 | 2-(2,4-Dimethylphenyl)-3a,4,7,7a-tetrahydro-4-(hydroxymethyl)-4,7-epoxy-1H-isoindole-1,3(2H)-dione | 21-26 |
| 169 | 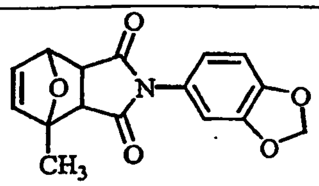 | 2-(1,3-Benzodioxol-5-yl)-3a,4,7,7a-tetrahydro-4-methyl-4,7-epoxy-1H-isoindole-1,3(2H)-dione | 32 |
| 170 | 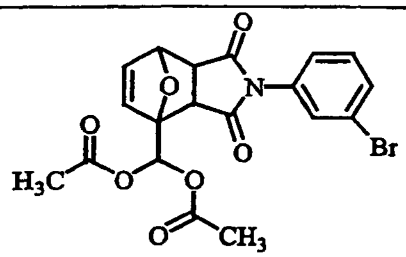 | 4-[Bis(acetyloxy)methyl]-2-(3-bromophenyl)-3a,4,7,7a-tetrahydro-4,7-epoxy-1H-isoindole-1,3(2H)-dione | 21-26 |
| 171 | 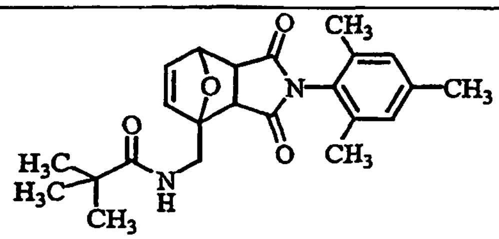 | N-[[1,2,3,3a,7,7a-Hexahydro-2-(2,4,6-trimethylphenyl)-4,7-epoxy-4H-isoindol-4-yl]methyl]-2,2-dimethylpropanamide | 21-26 |
| 172 | 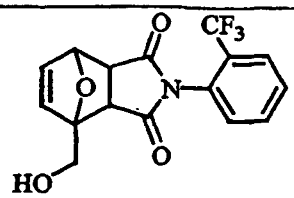 | 3a,4,7,7a-Tetrahydro-4-(hydroxymethyl)-2-[2-(trifluoromethyl)phenyl]-4,7-epoxy-1H-isoindole-1,3(2H)-dione | 21-26 |

| Ex. No. | Compound Structure | Compound Name | Pro. of Ex. |
|---|---|---|---|
| 173 | 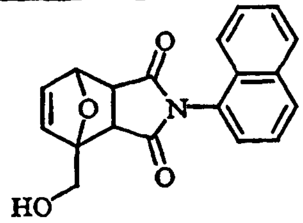 | 3a,4,7,7a-Tetrahydro-4-(hydroxymethyl)-2-(1-naphthalenyl)-4,7-epoxy-1H-isoindole-1,3(2H)-dione | 21-26 |
| 174 | 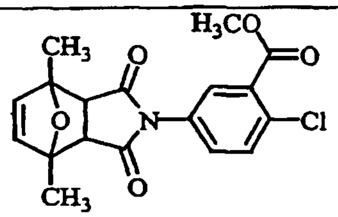 | 2-Chloro-5-(1,3,3a,4,7,7a-hexahydro-4,7-dimethyl-4,7-epoxy-2H-isoindol-2-yl)benzoic acid methyl ester | 32 |
| 175 | 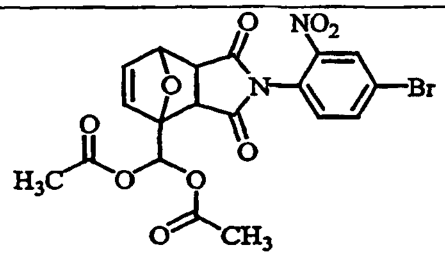 | 4-[Bis(acetyloxy)methyl]-2-(4-bromo-2-nitrophenyl)-3a,4,7,7a-tetrahydro-4,7-epoxy-1H-isoindole-1,3(2H)-dione | 21-26 |
| 176 | 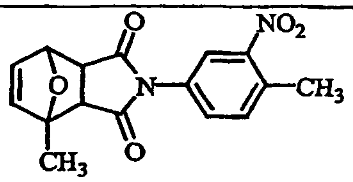 | 3a,4,7,7a-Tetrahydro-4-methyl-2-(4-methyl-3-nitrophenyl)-4,7-epoxy-1H-isoindole-1,3(2H)-dione | 32 |
| 177 | 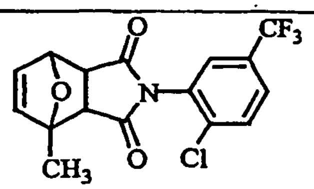 | 2-[2-Chloro-5-(trifluoromethyl)phenyl]-3a,4,7,7a-tetrahydro-4-methyl-4,7-epoxy-1H-isoindole-1,3(2H)-dione | 32 |

| Ex. No. | Compound Structure | Compound Name | Pro. of Ex. |
|---|---|---|---|
| 178 | 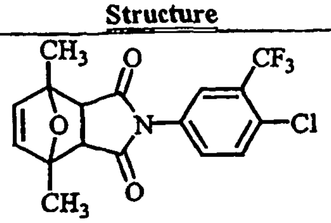 | 2-[4-Chloro-3-(trifluoromethyl)phenyl]-3a,4,7,7a-tetrahydro-4,7-dimethyl-4,7-epoxy-1H-isoindole-1,3(2H)-dione | 32 |
| 179 | 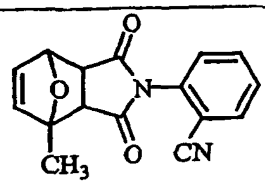 | 2-(1,3,3a,4,7,7a-Hexahydro-4-methyl-4,7-epoxy-2H-isoindol-2-yl)benzonitrile | 32 |
| 180 | 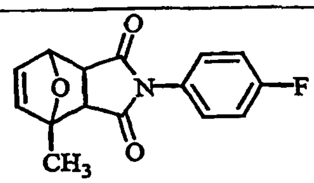 | 2-(4-Fluorophenyl)-3a,4,7,7a-tetrahydro-4-methyl-4,7-epoxy-1H-isoindole-1,3(2H)-dione | 32 |
| 181 | 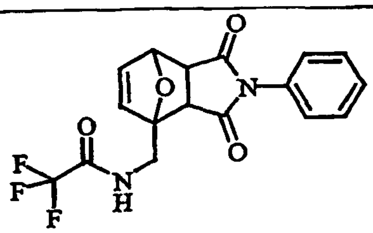 | 2,2,2-Trifluoro-N-[(1,2,3,3a,7,7a-hexahydro-2-phenyl-4,7-epoxy-4H-isoindol-4-yl)methyl]acetamide | 21-26 |
| 182 | 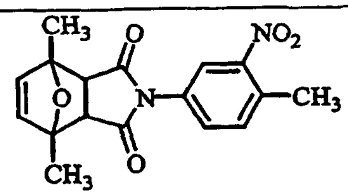 | 3a,4,7,7a-Tetrahydro-4,7-dimethyl-2-(4-methyl-3-nitrophenyl)-4,7-epoxy-1H-isoindole-1,3(2H)-dione | 32 |
| 183 | 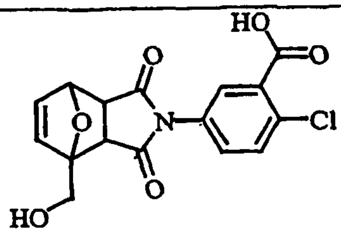 | 2-Chloro-5-[1,3,3a,4,7,7a-hexahydro-4-(hydroxymethyl)-4,7-epoxy-2H-isoindol-2- | 21-26 |

| Ex. No. | Compound Structure | Compound Name | Pro. of Ex. |
|---|---|---|---|
| | | yl]benzoic acid | |
| 184 | 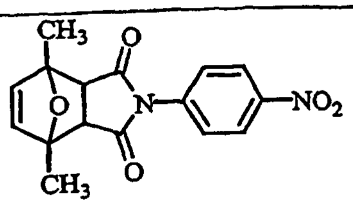 | 3a,4,7,7a-Tetrahydro-4,7-dimethyl-2-(4-nitrophenyl)-4,7-epoxy-1H-isoindole-1,3(2H)-dione | 32 |
| 185 | 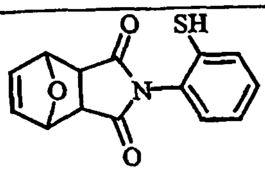 | 3a,4,7,7a-Tetrahydro-2-(2-mercaptophenyl)-4,7-epoxy-1H-isoindole-1,3(2H)-dione | 32 |
| 186 | 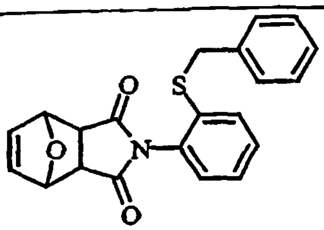 | 3a,4,7,7a-Tetrahydro-2-[2-[(phenylmethyl)thio]phenyl]-4,7-epoxy-1H-isoindole-1,3(2H)-dione | 32 |
| 187 | 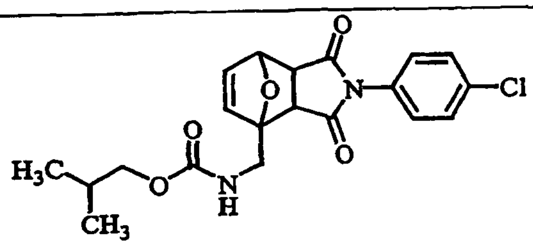 | [[2-(4-Chlorophenyl)-1,2,3,3a,7,7a-hexahydro-4,7-epoxy-4H-isoindol-4-yl]methyl]carbamic acid 2-methylpropyl ester | 21-26 |
| 188 | 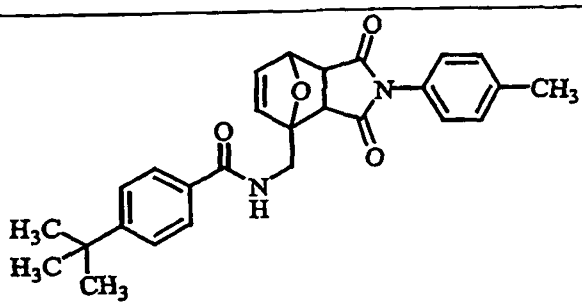 | 4-(1,1-Dimethylethyl)-N-[[1,2,3,3a,7,7a-hexahydro-2-(4-methylphenyl)-4,7-epoxy-4H-isoindol-4-yl]methyl]benzamide | 21-26 |

| Ex. No. | Compound Structure | Compound Name | Pro. of Ex. |
|---|---|---|---|
| 189 | 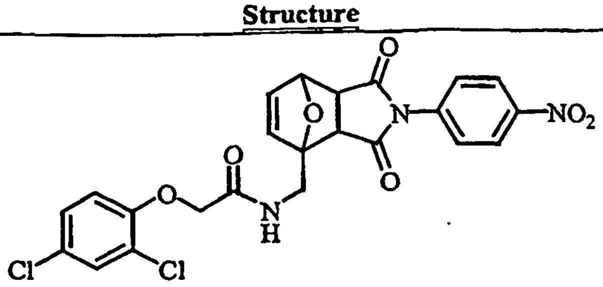 | 2,4-Dichloro-N-[[1,2,3,3a,7,7a-hexahydro-2-(4-nitrophenyl)-4,7-epoxy-4H-isoindol-4-yl]methyl]benzamide | 21-26 |
| 190 | 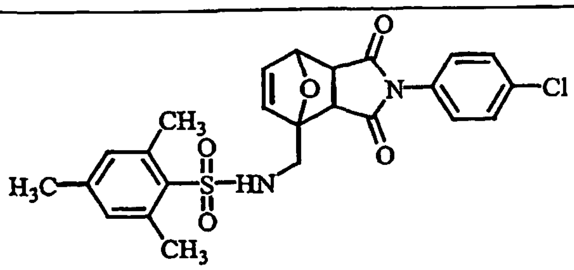 | N-[[2-(4-Chlorophenyl)-1,2,3,3a,7,7a-hexahydro-4,7-epoxy-4H-isoindol-4-yl]methyl]-2,4,6-trimethylbenzenesulfonamide | 21-26 |
| 191 | 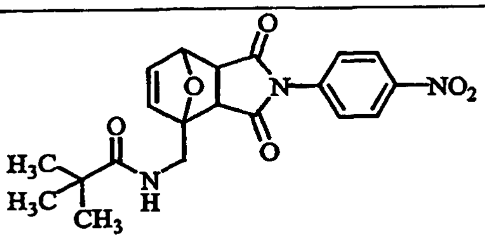 | N-[[1,2,3,3a,7,7a-Hexahydro-2-(4-nitrophenyl)-4,7-epoxy-4H-isoindol-4-yl]methyl]-2,2-dimethylpropanamide | 21-26 |
| 192 | 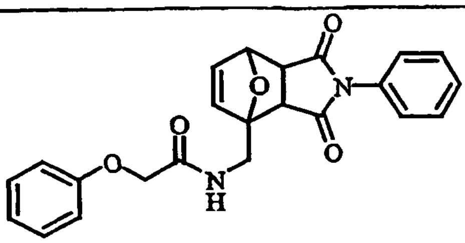 | N-[(1,2,3,3a,7,7a-Hexahydro-2-phenyl-4,7-epoxy-4H-isoindol-4-yl)methyl]-2-phenoxyacetamide | 21-26 |
| 193 | 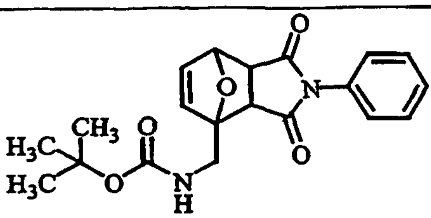 | [(1,2,3,3a,7,7a-Hexahydro-2-phenyl-4,7-epoxy-4H-isoindol-4-yl)methyl]carbamic acid 1,1-dimethylethyl ester | 21-26 |

| Ex. No. | Compound Structure | Compound Name | Pro. of Ex. |
|---|---|---|---|
| 194 | 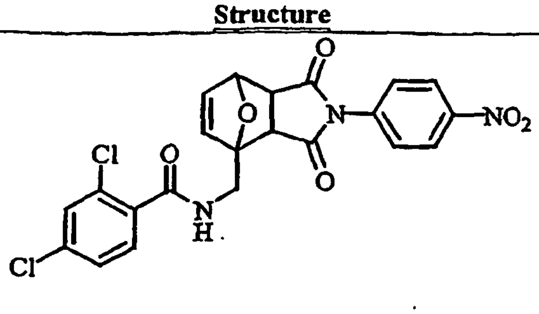 | 2-(2,4-Dichlorophenoxy)-N-[[1,2,3,3a,7,7a-hexahydro-2-(4-nitrophenyl)-4,7-epoxy-4H-isoindol-4-yl]methyl]acetamide | 21-26 |
| 195 | 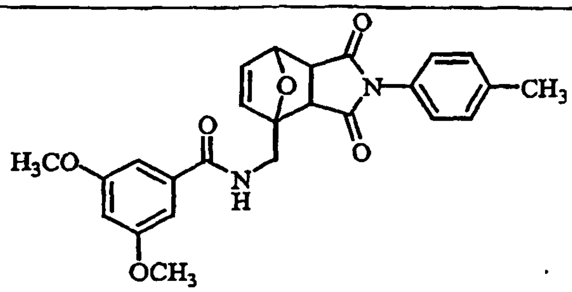 | N-[[1,2,3,3a,7,7a-Hexahydro-2-(4-methylphenyl)-4,7-epoxy-4H-isoindol-4-yl]methyl]-3,5-dimethoxybenzamide | 21-26 |
| 196 | 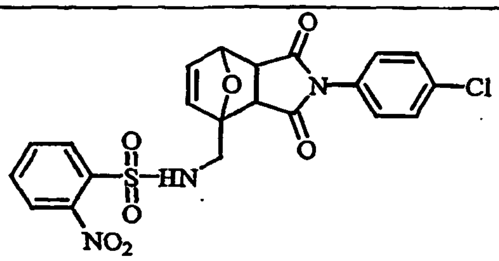 | N-[[2-(4-Chlorophenyl)-1,2,3,3a,7,7a-hexahydro-4,7-epoxy-4H-isoindol-4-yl]methyl]-2-nitrobenzenesulfonamide | 21-26 |
| 197 | 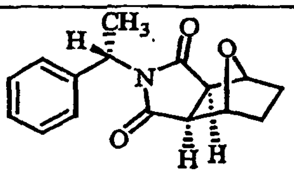 | (3aα,4β,7β,7aα)-Hexahydro-2-[(1S)-1-phenylethyl]-4,7-epoxy-1H-isoindole-1,3(2H)-dione. | 8 |
| 198 | 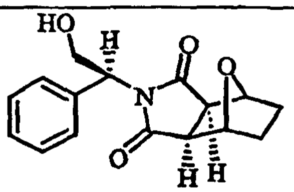 | (3aα,4β,7β,7aα)-Hexahydro-2-[(1S)-2-hydroxy-1-phenylethyl]-4,7-epoxy-1H-isoindole-1,3(2H)-dione. | 8 |

| Ex. No. | Compound Structure | Compound Name | Pro. of Ex. |
|---|---|---|---|
| 199 | 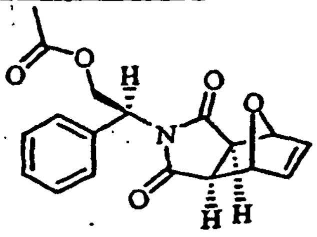 | (3aα,4β,7β,7aα)-2-[(1S)-2-(Acetyloxy)-1-phenylethyl]-3a,4,7,7a-tetrahydro-4,7-epoxy-1H-isoindole-1,3(2H)-dione. | 8 |
| 200 | 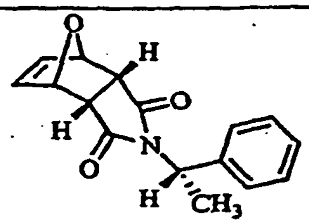 | (3aα,4α,7α,7aα)-3a,4,7,7a-Tetrahydro-2-[(1S)-1-phenylethyl]-4,7-epoxy-1H-isoindole-1,3(2H)-dione. | 8 |
| 201 | 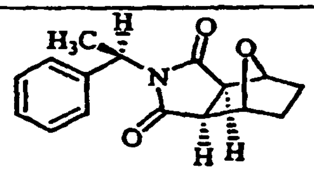 | (3aα,4β,7β,7aα)-Hexahydro-2-[(1R)-1-phenylethyl]-4,7-epoxy-1H-isoindole-1,3(2H)-dione. | 8 |
| 202 | 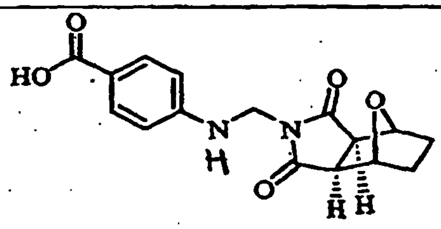 | (3aα,4β,7β,7aα)-4-[[(Octahydro-1,3-dioxo-4,7-epoxy-2H-isoindol-2-yl)methyl]amino]benzoic acid. | 8 |
| 203 | 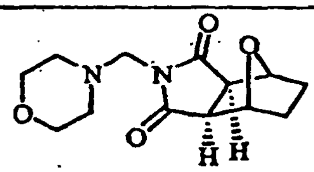 | (3aα,4β,7β,7aα)-Hexahydro-2-(4-morpholinylmethyl)-4,7-epoxy-1H-isoindole-1,3(2H)-dione. | 8 |

**Example 204**

**(3aα,4β,7β,7aα)-4-[Octahydro-4-(2-hydroxyethyl)-7-methyl-1,3-dioxo-4,7-epoxy-2H-isoindol-2-yl]-2-(trifluoromethyl)benzonitrile (204D/25B)**

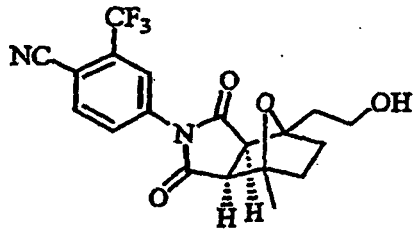

**A. 2-[2-[[(1,1-Dimethylethyl)dimethylsilyl]oxy]ethyl]-5-methylfuran (204A)**

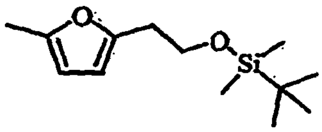

To a solution of compound **21A** (2.00 g, 15.9 mmol) in DMF (50 mL) was added imidazole (1.62 g, 23.9 mmol), followed by *tert*-butyldimethylsilyl chloride (2.63 g, 17.5 mmol). After 2 h at 25°C, the reaction mixture was poured into diethyl ether (300 mL) and washed with water (1 x 100 mL), 1N HCl (1 x 100 mL), water (1 x 100 mL), brine (1 x 50 mL) and dried over anhydrous $MgSO_4$. Crude compound **204A** was analyzed by LCMS and NMR and determined to be pure enough to be carried on directly to the next step. HPLC: 100% at 4.347 min (retention time) (YMC S5 ODS column 4.6 x 50 mm eluting with 10-90% aqueous methanol over 4 minutes containing 0.1% TFA, 4 mL/min, monitoring at 220 nm).

**B. (3aα,4β,7β,7aα)-4-[2-[[(1,1-Dimethylethyl)dimethylsilyl]-oxy]ethyl]hexahydro-7-methyl-4,7-epoxy-1H-isobenzofuran-1,3(2H)-dione (204B)**

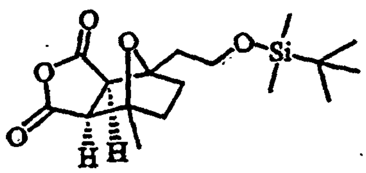

Compound **204A** (4.0 g, 18.9 mmol) and maleic anhydride (1.42 g, 14.51 mmol) were dissolved in dichloroethane (10 mL) and stirred at 25°C for 60 hours. The volatiles were then removed in vacuo and the resulting orange oil was dissolved in absolute ethanol (50 mL) and Pd/C (10 % Pd, 1.00 g) was added. Hydrogen was then introduced via a balloon. After 3h, the reaction mixture was filtered through celite rinsing with EtOAc and concentrated in vacuo. The crude anhydride was purified by rapid flash chromatography in $SiO_2$ eluting with acetone/chloroform (0 - 2 - 4% acetone) to give 1.30 g of compound **204B** as a clear oil, in addition to 3.00 g of the starting compound **204A.** Characterization by proton NMR spectroscopy showed only the exo isomer. $^1H$ NMR, 400 MHz, $CDCl_3$, 3.83 (2 H, t, *J*= 6.0 Hz), 3.22 (1 H, d, *J*= 8.2 Hz), 3.06 (1 H, d, *J*= 8.2 Hz), 1.70-2.25 (6 H, m), 1.55 (3 H, s), 0.82 (9 H, s), 0.00 (6 H, s).

**C. (3aα,4β,7β,7aα)-4-[4-[2-[[(1,1-Dimethylethyl)dimethylsilyl]-oxy]ethyl]octahydro-7-methyl-1,3-dioxo-4,7-epoxy-2H-isoindol-2-yl]-2-(trifluoromethyl)benzonitrile (204C)**

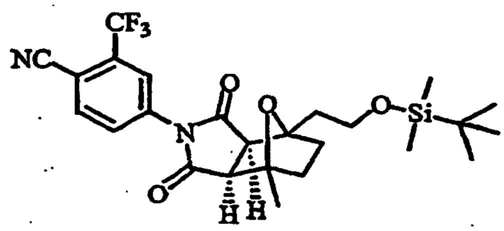

Compound **204B** (0.250 g, 0.8 mmol) and 4-amino-2-trifluoromethylbenzonitrile (0.124 g, 0.668 mmol) were suspended in dry toluene (2.0 mL) in a sealed tube. $MgSO_4$ (0.200 g) and triethylamine (0.5 mL) were then added and the tube was sealed and placed in a oil bath at 125°C. After 40 h, the reaction mixture was cooled to 25°C, filtered and concentrated in vacuo. The crude material was purified by flash chromatography on $SiO_2$ eluting with $CH_2Cl_2$ to give 0.111 g of compound **204C** as a yellow solid. HPLC: 92% at 4.203 min (retention time) (YMC S5 ODS column 4.6 x 50 mm eluting with 10-90% aqueous methanol over 4 minutes containing 0.1 % TFA, 4 mL/min, monitoring at 220 nm). MS (ESI): m/z 531.1 $[M+Na]^+$.

**D. (3aα,4β,7β,7aα)-4-[Octahydro-4-(2-hydroxyethyl)-7-methyl-1,3-dioxo-4,7-epoxy-2H-isoindol-2-yl]-2-(trifluoromethyl)benzonitrile (204D)**

Compound **204C** (0.031 g, 0.061 mmol) was dissolved in THF (0.5 mL) and transferred to a polypropylene container followed by cooling to 0°C. HF•pyridine (~47% HF, 0.1 mL) was then added. After 15 min, the reaction was complete as determined by LC and the mixture was poured into cold sat aqueous $NaHCO_3$. The mixture was extracted with $CH_2Cl_2$ (3 x 10 mL). The combined organic layers were washed with 1 N HCl (1 x 20 mL) and dried over anhydrous $Na_2SO_4$. Compound **204D** was isolated as a yellow oil and compared to the material prepared in Example 25. No purification was necessary.

**Example 205**

**(3aα,4β,7β,7aα)- and (3aα,4α,7α,7aα)-4-[Octahydro-4-methyl-1,3-dioxo-7-(phenylmethyl)-4,7-epoxy-2H-isoindol-2-yl]-2-(trifluoromethyl)benzonitrile (205Ci and 205Cii, respectively)**

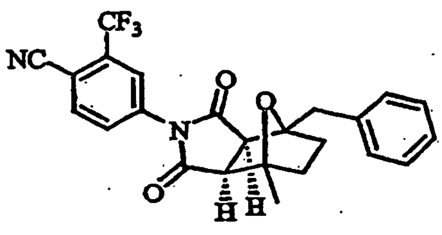

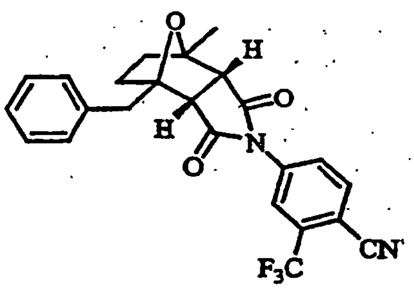

**A. 2-Methyl-5-(phenylmethyl)-furan (205A)**

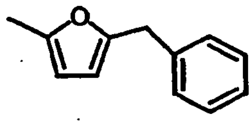

n-BuLi (1.8 ml, 4.51 mmol, 1.1 eq, 2.5 M in hexane) was added to a solution of 2-methyl-furan (0.37 ml, 4.10 mmol, 1 eq) in anhydrous THF (3 mL) at -25°C. The resulting solution was stirred at room temperature for 3 h and then cooled to -15°C. Benzyl bromide (0.59 ml, 4.92 mmol, 1.2 eq), which was passed through a plug of aluminum oxide, was added and the solution was warmed to rt and stirred overnight. Saturated $NH_4Cl$ solution (5 mL) was added and the mixture was stirred for 1 h. The reaction mixture was then extracted by ether (2 x) and the combined organic extracts were dried and concentrated under reduced pressure. Purification by flash chromatography on silica gel eluting with hexanes gave 323 mg (46%, 1.88 mmol) of compound 205A as colorless oil. HPLC: 95% at 3.72 min (retention time) (YMC S5 ODS column 4.6 x 50 mm, 10-90% aqueous methanol over 4 minutes containing 0.2% phosphoric acid, 4 mL/min, monitoring at 220 nm) and about 400 mg mixture of product and benzyl bromide (~2:1 by HPLC).

**B. (3aα,4β,7β,7aα)- and (3aα,4α,7α,7aα)-4-[Octahydro-4-methyl-1,3-dioxo-7-(phenylmethyl)-4,7-epoxy-2H-isoindol-2-yl]-2-(trifluoromethyl) benzonitrile (205Bi and 205Bii, respectively)**

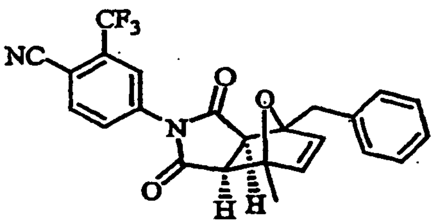

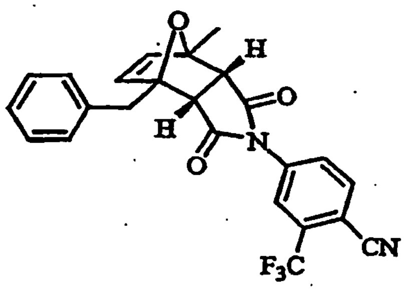

A solution of compound **205A** (124 mg, 0.72 mmol, 1 eq) and 4-(2,5-dihydro-2,5-dioxo-1H-pyrrol-1-yl)-2-trifluoromethylbenzontrile (290 mg, 1.09 mmol, 1.5 eq) in $CH_2Cl_2$ (2 mL) was stirred at room temperature. After 4 days, the reaction mixture was concentrated under reduced pressure. Purification by flash chromatography on silica gel eluting with $CH_2Cl_2$ gave 62 mg (0.14 mmol, 20 %) of a mixture of compounds **205Bi** and **205Bii** as a white solid, which was used directly in the next step. HPLC: 93% at 3.69 min (retention time) (YMC S5 ODS column 4.6 x 50 mm, 10-90% aqueous methanol over 4 minutes containing 0.2% phosphoric acid, 4 mL/min, monitoring at 220 nm).

**C. (3aα,4β,7β,7aα)- and (3aα,4α,7α,7aα)-4-[Octahydro-4-methyl-1,3-dioxo-7-(phenylmethyl)-4,7-epoxy-2H-isoindol-2-yl]-Z-(trifluoromethyl)-benzonitrile (205Ci and 205Cii, respectively)**

A solution of a mixture of compounds **205Bi** and **205Bii** (62 mg, 0.14 mmol, 1 eq) and 10% Pd/C (12 mg, cat.) in EtOH (3.5 mL) was stirred under a hydrogen atmosphere at room temperature for 2 h. The reaction mixture was filtered through celite and concentrated under reduced pressure. Purification by flash chromatography on silica gel eluting with 35% EtOAc/hexanes gave 22 mg (0.05 mmol, 35 %) of compound **205Ci** and 12 mg (0.027 mmol, 19%) of compound **205Cii.** Compound 205Ci: HPLC: 98% at 3.75 min (retention time) (YMC S5 ODS column 4.6 x 50 mm, 10-90% aqueous methanol over 4 minutes containing 0.2% phosphoric acid, 4 mL/min, monitoring at 220 nm), MS (ESI): m/z 458.2 $[M+NH_4]^+$. Compound **205Cii:** HPLC: 97% at 3.78 min (YMC S5 ODS column 4.6 x 50 mm, 10-90% aqueous methanol over 4 minutes containing 0.2% phosphoric acid, 4 mL/min, monitoring at 220 nm), MS (ESI): m/z 473.45 $[M+CH_3OH]^+$.

<u>**Example 206**</u>

<u>**(3aα,4β,7β,7aα)-2-[4-Cyano-3-(trifluoromethyl)phenyl]octahydro-7-methyl-1,3-dioxo-4,7-epoxy-4H-isoindole-4-propanenitrile (206)**</u>

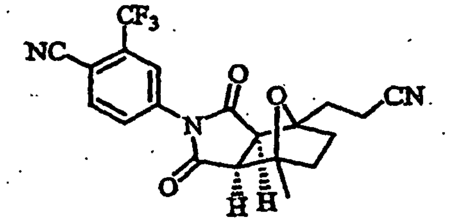

A solution of compound **36** (34 mg, 0.074 mmol) and NaCN (24 mg, 0.49 mmol) in DMSO (1 mL) was heated at 100°C for 0.5 h. After cooling, the reaction mixture was poured into $H_2O$ and the aqueous layer was extracted with EtOAc (2x). The combined organic layers were washed with $H_2O$ (2x), dried over $Na_2SO_4$ and concentrated under reduced pressure. Purification by flash chromatography on $SiO_2$ eluting with 50% EtOAc/hexanes followed by preparative HPLC, 30.41 min (retention time) (YMC S5 ODS 30 x 250 mm, 10-90% aqueous methanol over 30 minutes containing 0.1% TFA, 25 mL/min, monitoring at 220 nm) gave 6.6 mg (22%) of compound **206** as a white solid. HPLC: 99% at 2.89 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 402.1 $[M-H]^-$

**Example 207**

**(3aα,4β,7β,7aα)-4-[Octahydro-4-methyl-7-[2-(4-morpholinyl)ethyl]-1,3-dioxo-4,7-epoxy-2H-isoindol-2-yl]-2-(trifluoromethyl)benzonitrile, trifluoroacetate (1:1) (207)**

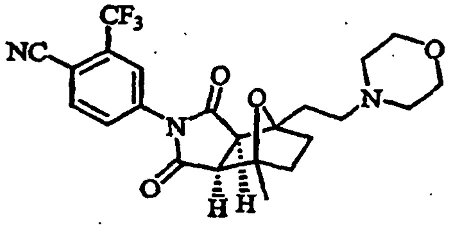

A solution of compound **36** (15.6 mg, 0.034 mmol) and morpholine (6 μL, 0.068 mmol) in toluene (1 mL) was heated at 100°C overnight. After cooling, the reaction mixture was concentrated under reduced pressure. Purification by flash chromatography on $SiO_2$ eluting with 10% $MeOH/CH_2Cl_2$ followed by preparative HPLC, 23.96 min (retention time) (YMC S5 ODS 30 x 250 mm, 10-90% aqueous methanol over 30 minutes containing 0.1% TFA, 25 mL/min, monitoring at 220 nm) gave 8.7 mg (55%) of compound **207** (TFA salt) as a white solid. HPLC: 99% at 2.02 min (retention time) (YMC S5 ODS column 4.6 x 50 mm eluting with 10-90% aqueous methanol over 4 minutes containing 0.2% phosphoric acid, 4 mUmin, monitoring at 220 nm), MS (ES): m/z 464.3 $[M+H]^+$.

**Example 208**

**(3aα,4β,7β,7aα)-2-(5-Fluoro-1-naphthalenyl)hexahydro-4,7-dimethyl-4,7-epoxy-1H-isoindole-1,3(2H)-dione (208C)**

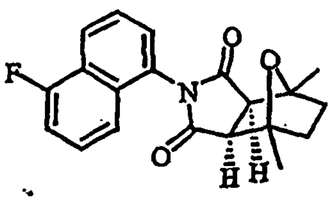

**A. 1-Fluoro-5-nitronaphthalene (208A)**

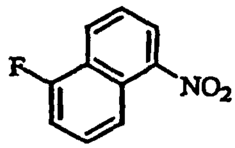

To a solution of 6N HCl (12 mL) was added 1.47 g (7.83 mmol) of finely powdered 5-nitro-1-naphthyiamine, as described in *J. Chem. Soc.* 1187 (1949). The mixture was cooled to 0°C and a cold solution of $NaNO_2$ (547 mg, 7.93 mmol) in 2 mL $H_2O$ was added slowly so that the temperature was kept near 0°C. After the addition was complete, the reaction mixture was stirred for 30 min and filtered. The filtrate was cooled to 0°C and treated with cold 4.5 M $NaBF_4$ solution (5 ml) to give complete precipitation of the diazonium borofluoride. The mixture was kept at 0°C for 30 min before it was filtered and the precipitates were washed with cold 4.5 M $NaBF_4$ solution (5 mL), ice-cold ethanol (10 mL) and $Et_2O$ (20 mL). The obtained solids were air dried to yield 1.74 g (77%) of the corresponding diazonium salt.

To 1.70 g (5.92 mmol) of the above diazonium borofluoride were added 5 g of sand and the components were thoroughly mixed. The reaction mixture was heated cautiously under reduced pressure until decomposition set in. Toward the end of the reaction the flask was further heated for 30 min to 130°C to assure complete conversion. After cooling the reaction mixture was dissolved in acetone and the contents were preabsorbed on silica gel. Purification was achieved by flash chromatography (silica gel, EtOAc in hexanes, 0 to 10%) to give 449 mg (50%) of compound **208A** as a white solid.

**B. 1-Amino-5-fluoronaphthalene (208B)**

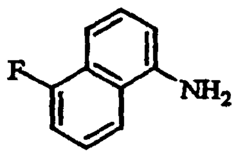

A solution of compound **208A** (62 mg, 0.32 mmol) in 1 mL EtOH containing 0.1 mL 12N HCl was heated to reflux. Iron powder (62 mg, 1.11 mmol) was added in small portions and heating was continued for 2 h. The mixture was cooled, neutralized with 1N NaOH solution and the aqueous layer was extracted with $CH_2Cl_2$. The combined organic phases were dried over $MgSO_4$ and concentrated to leave a residue which was purified by flash chromatography (silica gel, EtOAc in hexanes, 40 to 80%) yielding 42 mg (80%) of compound **208B** as a yellow solid.

**C. (3aα,4β,7β,7aα)-2-(5-Fluoro-1-naphthalenyl)hexahydro-4,7-dimethyl-4,7-epoxy-1H-isoindole-1,3(2H)-dione (208C)**

Compound **208B** (42 mg, 0.26 mmol), compound **20A** (54 mg, 0.27 mmol), $MgSO_4$ (69 mg, 0.58 mmol) and triethylamine (191 μL, 1.37 mmol) were taken up in 2 mL of toluene and placed in a sealed tube. The sealed tube was heated to 135°C for 14 h. The cooled reaction mixture was filtered through a short pad of Celite elutirig with $CH_2Cl_2$ and the solvent was removed under reduced pressure. The residue was purified by preparative reverse phase HPLC (YMC S5 ODS 20 x 100 mm eluting with 30-100% aqueous methanol over 10 min containing 0.1% TFA, 20 mL/min) to give 15 mg (17%) of compound **208C** as a light yellow solid. HPLC: 16% at 2.96 min & 77% at 3.06 min (retention time, atropisomers) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 340.2 $[M+H]^+$.

**Example 209**

**(3aα,4β,7β,7aα)-2-(5-Fluoro-4-nitro-1-naphthalenyl)hexahydro-4,7-dimethyl-4,7-epoxy-1H-isoindole-1,3(2H)-dione (209C)**

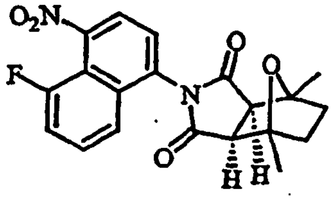

**A. N-(5-Fluoro-1-naphthalenyl)acetamide (209A)**

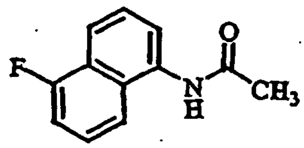

A solution of 141 mg (0.74 mmol) of compound **208A** in 2 mL of AcOH was heated to reflux and treated with small portions of iron powder (118 mg, 2.11 mmol). The mixture was kept at reflux for 15 min before 73 μL (0.78 mmol) of $Ac_2O$ were added. After an additional 15 min at reflux, the mixture was cooled and filtered eluting with $CH_2Cl_2$. The filtrate was then concentrated and the residue was purified by flash chromatography (silica gel, EtOAc in hexane, 20 to 50%) to give compound **209A** (145 mg, 97%) as a white solid.

**B. 1-Amino-5-fluoro-4-nitronaphthalone (209B)**

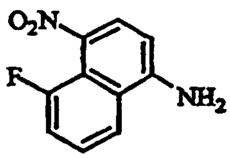

Compound **209A** (133 mg, 0.66 mmol) was dissolved in 1 mL AcOH and the resulting solution were cooled to 10°C. At this temperature, 80 μL (2.00 mmol) of red fuming $HNO_3$ was added and stirring was continued for 15 min before the reaction was quenched by the addition of crushed ice. The aqueous layer was extracted with $CH_2Cl_2$ and the combined organic phases were dried over $MgSO_4$ and concentrated. The resulting residue was dissolved in 3 mL EtOH, heated to reflux and treated with 0.5 mL of 40% aqueous NaOH solution. Stirring was continued for 15 min before the reaction mixture was cooled and diluted with $H_2O$. The aqueous layer was extracted with $CH_2Cl_2$ and the combined organic phases were dried over $MgSO_4$ and concentrated. The resulting residue was purified by flash chromatography (silica gel, EtOAc in hexane, 40 to 70%) to afford 36 mg (27%) of compound **209B** as a yellow solid.

**C. 3aα,4β,7β,7aα)-2-(5-Fluoro-4-nitro-1-naphthalenyl)hexahydro-4,7-dimethyl-4,7-epoxy-1H-isoindole-1,3(2H)-dione (209C)**

Compound **209B** (36 mg, 0.18 mmol) was reacted in a sealed tube with compound **20A** (38 mg, 0.19 mmol), $MgSO_4$ (46 mg, 0.39 mmol) and $Et_3N$ (128 μL, 0.92 mmol) in 250 μL toluene according to the above procedure described in example **208C** to give, after purification by preparative reverse phase HPLC (YMC S5 ODS 20 x 100 mm eluting with

30-100% aqueous methanol over 10 min containing 0.1% TFA, 20 mL/min). 27 mg (40%) of compound **209C** as a yellow solid. HPLC: 8% at 2.88 min & 84% at 3.06 min (atropisomers, retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 402.0 $[M+H]^+$.

## Example 210

**(3aα,4β,7β,7aα)-2-(1,1-Dioxidobenzo[b]thiophen-3-yl)hexahydro-4,7-dimethyl-4,7-epoxy-1H-isoindole-1,3(2H)-dione (210)**

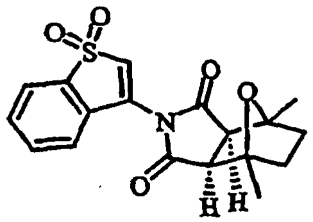

*m*CPBA (160 mg, 0.641 mmol, 70% pure) was added to a solution of compound **134** (70 mg, 0.214 mmol) in $CH_2Cl_2$ (2 mL) at rt. After the starting material was consumed, the reaction mixture was quenched with sat. $NaHCO_3$, and extracted with $CH_2Cl_2$. The organic layer was washed with 1N NaOH, dried over $Na_2SO_4$ and concentrated under reduced pressure to give 63.9 mg (83%) of compound **210** as a white solid. HPLC: 99% at 3.81 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 360.0 $[M+H]^+$.

## Example 211

**4-(1,3,3a,4,7,7a-Hexahydro-4,6,7-trimethyl-1,3-dioxo-4,7-epoxy-2H-pyrrolo[3,4-c]pyridin-2-yl)-2-(trifluoromethyl)benzonitrile (211)**

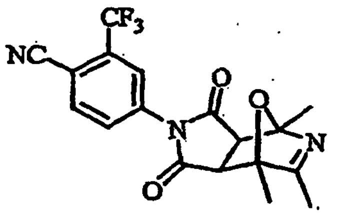

2,4,5-Trimethyl oxazole (0.48 mL, 4.14 mmol) was dissolved in toluene (2.0 mL) and 4-(2,5-dihydro-2,5-dioxo-1H-pyrrol-1-yl)-2-trifluoromethylbenzonitrile (1.0 g 3.76 mmol) was added. The reaction mixture was stirred at 75°C under nitrogen for 2.5 hrs. The solution was cooled to room temperature and the resulting precipitate was filtered and rinsed with toluene to give 0.51 g (35% yield) of compound **211** as a light grey solid. NMR analysis revealed that compound **211** was one isomer (exo/endo) however the identity of the isomer could not be determined by NMR analysis. HPLC: 100% at 2.85 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 378.42 $[M+H]^+$.

**Example 212**

**(3aα,4β,7β,7aα)-Tetrahydro-4,7-dimethyl-2-[3-(trifluoromethyl)phenyl]-4,7-epoxy-1H-isoindole-1,3,5(2H,4H)-trione & (3aα,4α,7α,7aα)-Tetrahydro-4,7-dimethyl-2-[3-(trifluoromethyl)phenyl]-4,7-epoxy-1H-isoindole-1,3,5(2H,4H)-trione (212i & 212ii, respectively)**

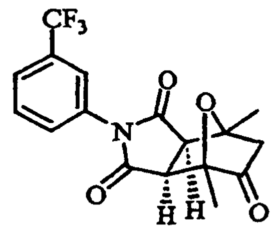

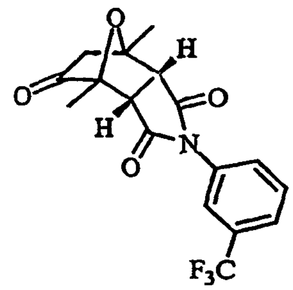

2,2-Dimethyl-3(H)-furanone (0.500 g, 4.46 mmol) and 1-[3-(trifluoromethyl)phenyl]-1H-pyrrole-2,5-dione (1.07 g, 4.46 mmol) were suspended in toluene (20 mL) in a sealed tube. The mixture was heated at 110°C for 4 h and then cooled to 25°C followed by concentration *in vacuo*. The resulting residue was purified by flash chromatography on $SiO_2$ eluting with methylene chloride to yield 0.411 g of compound **212i** as a white solid and 0.193 g of compound **212ii** as a white solid. The structural assignments were confirmed by 1-D NOE proton NMR experiments. Compound **212i:** HPLC: 100% at 2.817 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 376.0 $[M + Na]^+$. Compound **212ii:** HPLC: 100% at 3.013 min (retention time) (YMC S5 ODS column 4.6 x 50 mm eluting with 10-90% aqueous methanol over 4 minutes containing 0.2% phosphoric acid, 4 mUmin, monitoring at 220 nm), MS (ES): m/z 354.02 $[M+H]^+$.

**Example 213**

**(3aα,4β,7β,7aα)-2-(5-Chloro-1-naphthalenyl)hexahyrdro-4,7-dimethyl-4,7-epoxy-1H-isoindole-1,3(2H)-dione (213B)**

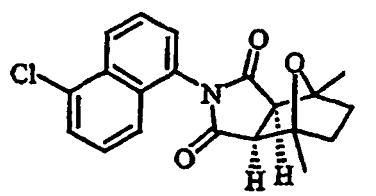

**A. 1-Amino-5-chloronaphthalene (213A)**

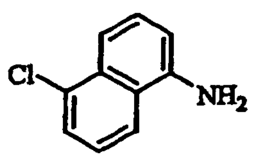

To a solution of 1.74 g (6.06 mmol) of the diazonium borofluoride (described in Example **208A**) in acetone (7 mL) were added 693 mg (7.00 mmol) of CuCl in small portions. After the evolution of nitrogen had ceased the acetone was removed under reduced pressure and the residue was taken up in $CH_2Cl_2$ (30 mL). The organic phase was washed

with $H_2O$ (30 mL), dried over $MgSO_4$, concentrated and finally purified by flash chromatography (silica gel, EtOAc in hexane, 0 to 15%) to give 754 mg (70%) of 1-chloro-5-nitronaphthalene.

The above synthesized 1-chloro-5-nitronaphthalene (540 mg, 2.6 mmol) was dissolved in 10 mL AcOH, followed by treatment with 415 mg (7.43 mmol) iron powder and subsequently acylated with $Ac_2O$ (0.26 mL, 2.73 mmol) according to the procedure described in Example **209A** to give 543 mg (95%) of 1-acetamino-5-chloronaphthalene.

A solution of the above synthesized 1-acetamino-5-chloronaphthalene (52 mg, 0.24 mmol) in 3 mL EtOH was heated to reflux and treated with 0.5 mL 40% aqueous NaOH solution. The mixture was refluxed until no more starting material could be detected, cooled and concentrated under reduced pressure. The residue was taken up in $CH_2Cl_2$ (50 mL) and was washed with $H_2O$ (25 mL). The organic layer was dried over $MgSO_4$ and concentrated to leave 41 mg (98%) of compound **213A** as a white solid.

**B. (3aα,4β,7β,7aα)-2-(5-Chloro-1-naphthalenyl)hexahydro-4,7-dimethyl-4,7-epoxy-1H-isoindole-1,3(2H)-dione (213B)**

Compound **213A** (24 mg, 0.14 mmol) was reacted in a sealed tube with compound **20A** (29 mg, 0.15 mmol), $MgSO_4$ (36 mg, 0.30 mmol) and $Et_3N$ (100 μL, 0.71 mmol) in 250 μL toluene according to the procedure described in Example **208C** to give, after purification by preparative reverse phase HPLC (YMC S5 ODS 20 x 100 mm eluting with 30-100% aqueous methanol over 10 min containing 0.1% TFA, 20 mL/min), 27 mg (40%) of compound **213B** as a white solid. HPLC: 98% at 1.82 min (retention time) (YMC S5 TurboPack Pro column 4.6 x 33 mm eluting with 10-90% aqueous methanol over 2 minutes containing 0.2% phosphoric acid, 4 mL/min, monitoring at 220 nm), MS (ES): m/z 356.4 $[M+H]^+$.

**Example 214**

**(3aα,4β,7β,7aα)-2-(5-Chloro-4-nitro-1-naphthalenyl)hexahydro-4,7-dimethyl-4,7-epoxy-1H-isoindole-1,3(2H)-dione (214B)**

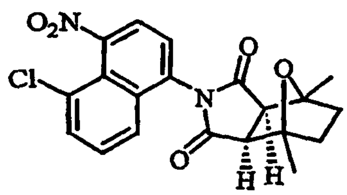

**A. 1-Amino-5-chloro-4-nitronaphthalene (214A)**

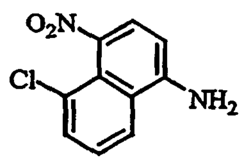

1-Acetamino-5-chloronaphthalene (150 mg, 0.68 mmol, prepared as described in Example **213A**) was dissolved in 1 mL AcOH and treated with 82 μL of red fuming $HNO_3$ and subsequently deacylated with 1 mL 40% aqueous NaOH solution in 3 mL EtOH according to the procedure described in Example **209A** to yield 49 mg (32%) of compound **214A** as a yellow solid.

**B. (3aα,4β,7β,7aα)-2-(5-Chloro-4-nitro-1-naphthalenyl)hexahydro-4,7-dimethyl-4,7-epoxy-1H-isoindole-1,3(2H)-dione (214B)**

Compound **214A** (27 mg, 0.12 mmol) was reacted in a sealed tube with compound **20A** (26 mg, 0.13 mmol), $MgSO_4$ (32 mg, 0.27 mmol) and $Et_3N$ (88 μL, 0.63 mmol) in 250 μL toluene according to the procedure described in Example **208C** to give, after purification by preparative reverse phase HPLC (YMC S5 ODS 20 x 100 mm eluting with

30-100% aqueous methanol over 10 min containing 0.1% TFA, 20 mL/min), 22 mg (45%) of compound **214B** as a yellow solid. HPLC: 24% at 3.06 min & 76% at 3.25 min (atropisomers, retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 418.0 $[M+NH_4]^+$.

**Example 215**

**(3aα,4β,7β,7aα)-4-Ethylhexahydro-7-methyl-2-(4-nitro-1-naphthalenyl)-4,7-epoxy-1H-isoindole-1,3(2H)-dione (215B)**

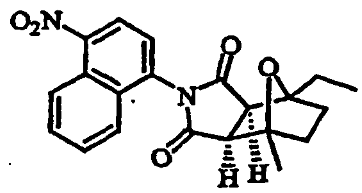

**A. (3aα,4β,7β,7aα)-4-Ethylhexahydro-7-methyl-4,7-epoxyisobenzofuran-1,3-dione (215A)**

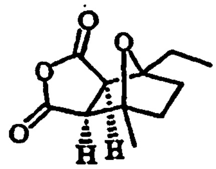

2-Ethyl-5-methylfuran (1.89 mL, 15.3 mmol) was dissolved in methylene chloride (10 mL) and maleic anhydride (1.00 g, 10.2 mmol) was added. The reaction mixture was stirred at 25°C for 18 h and then concentrated *in vacuo*. The resulting crude bicycle was dissolved in EtOAc (50 mL) and 10% Pd/C (0.40 g) was added. Hydrogen was then introduced via a balloon. After 4h, the reaction mixture was filtered through celite, rinsing with EtOAc. Concentration *in vacuo* gave the crude compound **215A** (1.93 g) as a white solid. This material was taken on directly to the next reaction without purification.

**B. (3aα,4β,7β,7aα)-4-Ethylhexahydro-7-methyl-2-(4-nitro-1-naphthalenyl)-4,7-epoxy-1H-isoindole-1,3(2H)-dione (215B)**

Compound **215A** (0.168 g, 0.798 mmol) and 4-nitro-1-naphthalamine (0.10 g, 0.53 mmol) were suspended in toluene (0.8 mL) and TEA (0.2 mL) and magnesim sulfate (0.1 g) were added. The mixture was heated at 135°C in a sealed tube for 18 h. The reaction mixture was then cooled to rt and filtered, rinsing with chloroform. Concentration gave the crude product which was purified by preparative TLC on $SiO_2$ eluting with methylene chloride. This gave compound **215B** (0.077 g) as a yellow solid. HPLC: 100% at 3.260 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 381.05 $[M+H]^+$.

**Example 216**

**(3aα,4β,7β,7aα)-2-(4-Cyano-1-naphthaienyl)-N-(4-fluorophenyl)octahydro-7-methyl-1,3-dioxo-4,7-epoxy-4H-isoindole-4-acetamide (216B)**

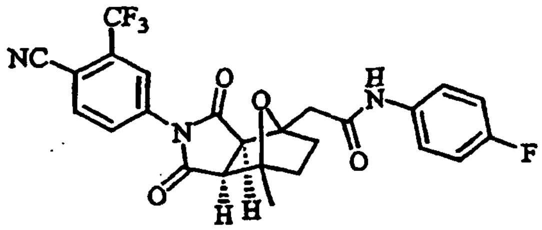

**A. N-(4-Fluorophenyl)-5-methyl-2-furanacetamide (216A)**

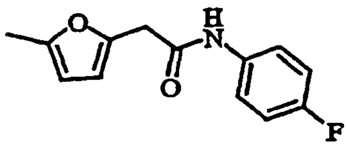

5-Methyl-2-furanacetic acid (1.00 g, 7.14 mmol, synthesized as described in WO 9507893, Example 19) was dissolved in $CH_3CN$/DMF (4:1, 25 mL), 1-[3-(dimethylamino)propyl]-3-ethylcarbodiimide (1.37 g, 7.14 mmol) and 1-hydroxy-7-azabenzotriazole (0.972 g, 7.14 mmol) were then added followed by 4-fluoroaniline (0.676 mL, 7.14 mmol). After 3 h, the reaction mixture was diluted with EtOAc (150 mL) and washed with 1 N HCl (1 x 30 mL), sat aq $NaHCO_3$ (1 x 30 mL), brine (1 x 40 mL) and dried over sodium sulfate. Compound **216A** (1.581 g) was isolated as a yellow foam after concentration *in vacuo.* No further purification was necessary. HPLC: 78% at 2.647 min (retention time) (YMC S5 ODS column 4.6 x 50 mm eluting with 10-90% aqueous methanol over 4 minutes containing 0.2% phosphoric acid, 4 mL/min, monitoring at 220 nm).

**B. (3aα,4β,7β,7aα)-2-(4-Cyano-1-naphthalenyl)-N-(4-fluorophenyl)octahydro-7-methyl-1,3-dioxo-4,7-epoxy-4H-isoindole-4-acetamide (216B)**

Compound **216A** (0.200 g, 0.858 mmol) and 4-(2,5-dihydro-2,5-dioxo-1H-pyrrol-1-yl)-2-trifluoromethylbenzonitrile (0.164 g, 0.66 mmol) were dissolved in benzene and heated at 60°C for 14 h.. The reaction mixture was then cooled and concentrated *in vacuo*. The resulting orange oil was dissolved in EtOAc (15 mL) and 10% Pd/C (0.050 g) was added. Hydrogen was then introduced via a balloon. After 3 h, the reaction mixture was filtered through celite rinsing with EtOAc and concentrated *in vacuo.* The resulting crude material was purified by preparative TLC on silica eluting with 5% acetone in methylene chloride to give 0.166 g of compound **216B** as a white solid. NMR spectroscopy showed only a single isomer which was determined to be exo by NOE experiments. HPLC: 95% at 3.200 min (retention time) (YMC S5 ODS column 4.6 x 50 mm eluting with 10-90% aqueous methanol over 4 minutes containing 0.2% phosphoric acid, 4 mUmin, monitoring at 220 nm), MS (ES): m/z 484.0 $[M+ H]^+$.

**Example 217**

**(3aα,4β,7β,7aα)-Hexahyrdro-4-methyl-2-(2-naphthalenyl)-4,7-epoxy-1H-isoindole-1,3(2H)-dione, faster eluting enantiomer & (3aα,4β,7β,7aα)-Hexahydro-4-methyl-2-(2-naphthalenyl)-4,7-epoxy-1H-isoindole-1,3(2H)-dione, slower eluting enantiomer (217i & 217ii, respectively)**

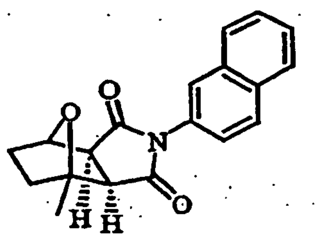

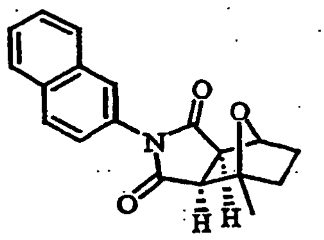

The racemic compound **137** was separated into the individual antipodes by chiral reverse phase liquid chromatography. A Chiralpak AD-R column (4.6 x 250 mm) was used eluting with 70% acetonitrile/30% water at 1 mL/min. UV detection at 220 nm was used. The faster eluting isomer, compound **217i** (retention time = 15.66 min), was found to be 99.9% ee and the slower eluting isomer, compound **217ii** (retention time = 15.66 min) was 99.6% ee by analytical chiral reverse phase chromatography.

**Example 218**

**(3aα,4β,7β,7aα)-4-[4-[2-[[(4-Fluorophenyl)methyl]methylamino]ethyl]octahydro-7-methyl-1,3-dioxo-4,7-epoxy-2H-isoindol-2-yl]-2-(trifluoromethyl)benzonitrile (218B)**

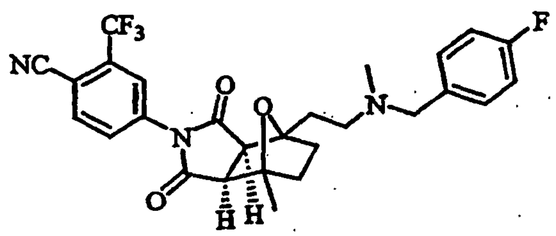

**A. (4-Fluorobenzyl)methylamine & Bis(4-fluorobenzyl)methylamine (218A & 218A')**

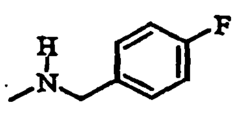

**218A**

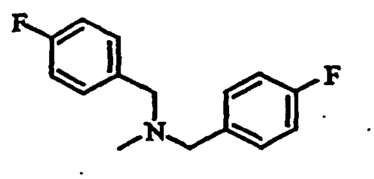

**218A'**

Compounds **218A & 218A'** were made in accordance with the procedure described in Singer *et al.*, *J. Med. Chem.* **29;** 40-44 (1986). 4-Fluorobenzyl bromide (189 mg, 1.00 mmol) was refluxed in a solution of ethanol (1.5 mL) and methylamine (5 mL, 2 M solution in MeOH) for 3 h. An additional portion of methylamine (2 mL) was added and the mixture was refluxed for an additional hour. The solution was cooled and concentrated *in vacuo*, and the residue was dissolved in a mixture of 2N HCl (3 mL) and ether (1.5 mL). The layers were separated and the aqueous layer was extracted with an additional portion of ether. The aqueous solution was chilled to 0°C, titrated to pH 11 with NaOH and extracted with $CH_2Cl_2$. The extracts were dried over $MgSO_4$ and concentrated to give 120 mg of a 2.5:1 mixture of compounds **218A** and compound **218A'** respectively. The crude mixture was taken on without further purification.

**B. (3aα,4β,7β,7aα)-4-[4-[2-[[(4-Fluorophenyl)methyl]-methylamino]ethyl]octahydro-7-methyl-1,3-dioxo-4,7-epoxy-2H-isoindol-2-yl]-2-(trifluoromethyl)benzonitrile (218B)**

A solution of compound **36** (34.3 mg, 0.075 mmol) and compounds **218A & 218A'** (21 mg, ~0.088 mmol (of **218A**)) in toluene (0.4 mL) was heated at 100°C overnight. The reaction mixture was cooled to room temperature and

then concentrated under reduced pressure. Purification by flash chromatography on silica gel eluting with 25% acetone/ 75% $CH_2Cl_2$ gave 30 mg (0.058 mmol, 77.7%) of **218B** as a yellow solid. HPLC: 99% at 2.46min (retention time) (YMC S5 ODS 4.6 x 50 mm, 10-90% aqueous methanol over 4 minutes containing 0.2% phosphoric acid, monitoring at 220 nm), MS (ES): m/z 516.26 $[M+H]^+$.

## Example 219

**(3aα,4β,5β,6β,7β,7aα)-4-(Octahydro-4,5,6,7-tetramethyl-1,3-dioxo-4,7-epoxy-2H-isoindol-2-yl)-2-(trifluoromethyl)benzonitrile (219D)**

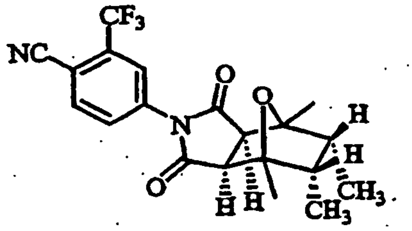

**A. 2,3,4,5-Tetramethylfuran (219A)**

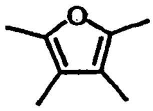

Compound **219A** was made in accordance with the procedures described in Hancock *et al., J. Org. Chem.* **42,** 1850-1856 (1977) & Amamath *et al., J. Org. Chem.* **60,** 301-307 (1995). 2-Propanone (100 mL, 1.1 mol) was refluxed over $PbO_2$ (26.7 g, 0.11 mol) for 28 h. After cooling to rt, the reaction mixture was filtered and the residue was washed with acetone. The filtrate was concentrated under reduced pressure to remove the acetone and then distilled at 20 Torr. The fraction that came over between 100-120°C was collected to give 6.75 g (42.5%) of 3,4-dimethylhexane-2,5-dione as a light yellow oil.

A solution of 3,4-dimethylhexane-2,5-dione (3.00 g, 21.1 mmol) and p-toluenesulfonic acid (401 mg, 2.11 mmol) in benzene (30 mL) was heated to reflux in a Dean-Stark trap overnight. The reaction mixture was distilled at atmospheric pressure to remove the excess benzene. The remaining mixture was transferred to a smaller flask and distilled at atmospheric pressure. The fraction that came over between 80-100°C was collected to give 509 mg (19%) of compound **219A** as a light yellow oil.

**B. (3aα,4β,7β,7aα)-4-Ethyl-3a,4,7,7a-tetrahydro-4,5,6,7-tetramethyl-4,7-epoxyisobenzofuran-1,3-dione (219B)**

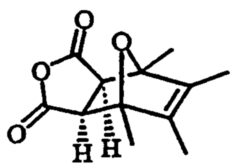

A solution of compound **219A** (400 mg, 3.22 mmol) and maleic anhydride (442 mg, 4.51 mmol) in $Et_2O$ (1.5 mL) was stirred at rt overnight. The reaction mixture was then placed in freezer for 5 days, after which time the resulting crystals were collected and dried to give 0.26 g (37%) of compound **219B** as tan crystals. The crude compound **219B**

was taken on to the next step with out further purification.

**C. (3aα,4β,5α,6α,7β,7aα)-4-Ethylhexahydro-4,5,6,7-tetramethyl-4,7-epoxyisobenzofuran-1,3-dione (219C)**

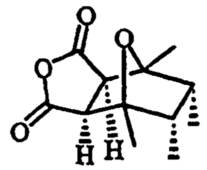

A solution of compound **219B** (120 mg, 0.545 mmol) and 10% Pd/C (24 mg, cat.) in EtOAc (2 mL) was stirred under a balloon of hydrogen at room temperature overnight. The reaction mixture was filtered through celite and concentrated under reduced pressure to give 100 mg (0.446 mmol, 81.9%) of compound **219C** as a white solid, which was carried on with no further purification.

**D. (3aα,4β,5β,6β,7β,7aα)-4-(Octahydro-4,5,6,7-tetramethyl-1,3-dioxo-4,7-epoxy-2H-isoindol-2-yl)-2-(trifluoromethyl)benzonitrile (219D)**

A solution of compound **219C** (44.4 mg, 0.2 mmol), 5-amino-2-cyanobenzotrifluoride (45 mg, 0.24 mmol), TEA (0.04 mL) and $MgSO_4$ (20 mg) in toluene (0.2 mL) was heated at 135°C overnight. The reaction mixture was cooled to room temperature, filtered and then concentrated under reduced pressure. Purification by flash chromatography on silica gel eluting with 40% EtOAc/hexanes followed by washing the resulting solid with MeOH gave 17 mg (0.043 mmol, 21.7%) of compound 219D as a white solid. HPLC: 90% at 3.11 min (retention time) (YMC S5 ODS 4.6 x 50 mm, 10-90% aqueous methanol over 4 minutes containing 0.2% phosphoric acid, monitoring at 220 nm), MS (ES): m/z 391.2 $[M-H]^-$.

**Example 220**

**(3aα,4β,7β,7aα)-4-[Octahydro-4-methyl-1,3-dioxo-7-[2-[4-(trifluoromethyl)phenoxy]ethyl]-4,7-epoxy-2H-isoindol-2-yl]-2-(trifluoromethyl)benzonitrile, faster eluting antipode & (3aα,4β,7β,7aα)-4-[Octahydro-4-methyl-1,3-dioxo-7-[2-[4-(trifluoromethyl)phenoxy]ethyl]-4,7-epoxy-2H-isoindol-2-yl]-2-(trifluoromethyl)benzonitrile, slower eluting enantiomer (220i & 220ii, respectively)**

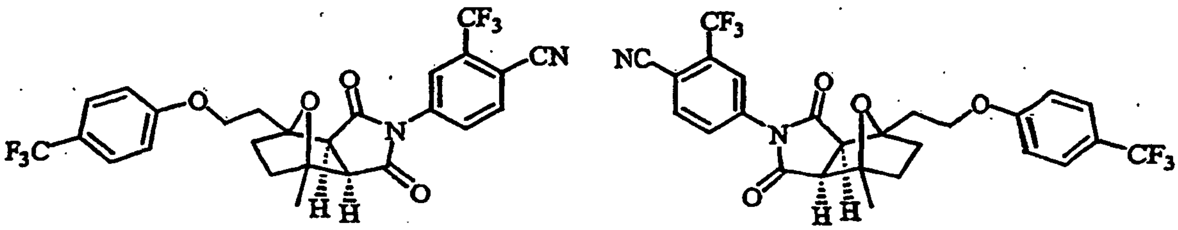

The racemic compound **35** was separated into the individual antipodes by chiral normal phase liquid chromatography. A Chiralpak AD column (50 x 500 mm) was used eluting with 85% hexanes/7.5% methanol/7.5% ethanol, @ 50mL/min. UV detection at 220 nm was used. The faster eluting isomer compound **220I** (retention time = 55.86 min) was found to have 95.8% ee ($[\alpha]_D^{25}$ = -53.02°, c = 3.134 mg/cc in $CH_2Cl_2$) and the slower eluting isomer compound **220ii** (retention time = 62.86 min) was 86% ee ($[\alpha]_D^{25}$ = +48.74°, c = 2.242 mg/cc in $CH_2Cl_2$) by analytical chiral normal phase chromatography.

**Example 221**

**(3aα,4β,5β,7β,7aα)-4-(Octahydro-5-hydroxy-4,7-dimethyl-1,3-dioxo-4,7-epoxy-2H-isoindol-2-yl)-2-(trifluoromethyl)benzonitrile (221B)**

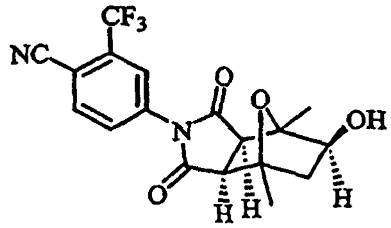

**A. (3aα,4β,7β,7aα)-4-(hexahydro-4,7-dimethyl-1,3-dioxo-4,7-epoxy-2H-isoindol-2-yl)-2-(trifluoromethyl)benzonitrile (221Ai) & (3aα,4α,7α,7aα)-4-(hexahydro-4,7-dimethyl-1,3-dioxo-4,7-epoxy-2H-isoindol-2-yl)-2-(trifluoromethyl)benzonitrile (221Aii)**

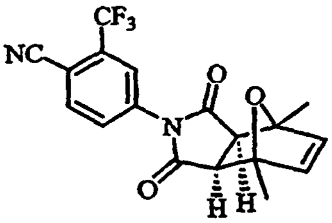

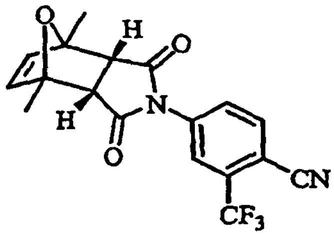

A solution of 2,5-dimethylfuran (0.8 mL, 7.51 mmol) and 4-(2,5-dihydro-2,5-dioxo-1H-pyrrol-1-yl)-2-trifluoromethylbenzonitrile (synthesized as described in Example **1B**) (1.00 g, 3.75 mmol) in benzene (4 mL) was heated at 60°C overnight. The reaction mixture was concentrated under reduced pressure and placed on a high vacuum pump until the oil solidified to give a 3:1 mixture (determined by LC and NMR) of compounds **221Ai** & **221Aii,** respectively, as a brown solid, which was used directly in the next step with out further purification.

**B. (3aα,4β,5β,7β,7aα)-4-(Octahydro-5-hydroxy-4,7-dimethyl-1,3-dioxo-4,7-epoxy-2H-isoindol-2-yl)-2-(trifluoromethyl)benzonitrile (221B)**

$BH_3$•THF (3.75 mL, 3.75 mmol, 1M in THF) was added to a solution of crude compounds **221Ai** & **221Aii** (3.75 mmol) in THF (12.5 mL) at 0°C. After the starting material was consumed the reaction mixture was concentrated under reduced pressure. The resulting residue was then dissolved in toluene (12.5 mL), $Me_3NO$ (845 mg, 11.25 mmol) was added and the mixture was heated to reflux overnight. The reaction mixture was then cooled to rt, added to $H_2O$ and extracted with EtOAc (3x). The combined organic layers were dried over $MgSO_4$ and concentrated under reduced pressure. Purification by flash chromatography on $SiO_2$ eluting with 75% EtOAc/hexanes gave 0.354 g (25%) of compound **221B** as a tan powder. HPLC: 90% at 2.45 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 381.11 $[M+H]^+$.

**Example 222**

**(3aα,4β,5α,7β,7aα)-4-(Octahydro-5-hydroxy-4,7-dimethyl-1,3-dioxo-4,7-epoxy-2H-isoindol-2-yl)-2-(trifluoromethyl)benzonitrile (222D)**

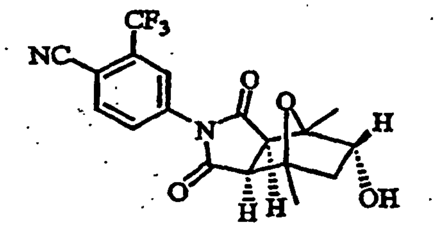

**A. 3-[[(1,1-Dimethylethyl)dimethylsilyl]oxy]-2,5-dimethylfuran (222A)**

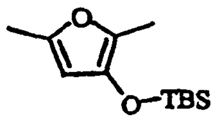

2,5-Dimethyl-3(3H)-furanone (2.00 g, 17.8 mmol) was dissolved in methylene chloride (180 mL). TEA (7.43 mL, 53.5 mmol) was added followed by TBSOTf (4.92 mL, 21.4 mmol) at 25°C. After 1 h, the reaction mixture was concentrated *in vacuo* and the resulting slurry was run through a silica gel column conditioned with 3% TEA in hexanes. The product was eluted with 3% TEA/hexanes to give 3.6 g of compound **222A** as an orange oil which was used directly in subsequent reactions.

**B. (3aα,4β,7β,7aα)-4-[5-[[(1,1-Dimethylethyl)dimethylsilyl]oxy]-1,3,3a,4,7,7a-hexahydro-4,7-dimethyl-1,3-dioxo-4,7-epoxy-2H-isoindol-2-yl]-2-(trifluoromethyl)benzonitrile (222B)**

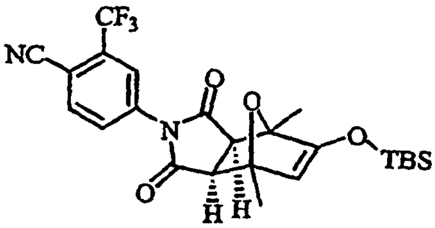

4-(2,5-Dihydro-2,5-dioxo-1H-pyrrol-1-yl)-2-trifluoromethylbenzonitrile (1.00 g, 3.85 mmol) was dissolved in benzene (5.0 mL) and the compound **222A** (1.30 g, 5.77 mmol) was added. The reaction mixture was warmed to 60°C for 2 h and then cooled to 25°C. The solution was then concentrated *in vacuo* to give compound **222B** as a yellow oil which was carried on to the next reaction without purification. HPLC: 60% at 4.013 min (retention time) (YMC S5 ODS column 4.6 x 50 mm eluting with 10-90% aqueous methanol over 4 minutes containing 0.2% phosphoric acid, 4 mL/min, monitoring at 220 nm).

**C. (3aα,4β,5α,7β,7aα)-4-[5-[[(1,1-Dimethytethyl)dimethylsityl]oxy]octahydro-4,7-dimethyl-1,3-dioxo-4,7-epoxy-2H-isoindol-2-yl]-2-(trifluoromethyl)benzonitrile (222C)**

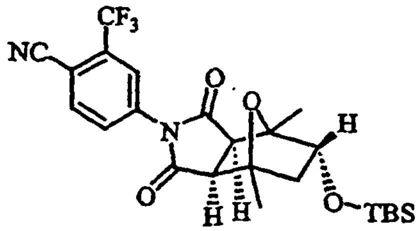

Crude compound **222B** (3.85 mmol) was dissolved in ethyl acetate (75 mL) and 10% Pd/C (1.20 g) was added. Hydrogen was then introduced via a balloon. After 24 h, the reaction mixture was filtered through celite rinsing with ethyl acetate and concentrated *in vacuo* to give a yellow oil. The crude product was purified by flash chromatography on silica gel eluting with methylene chloride/acetone (0% - 1% - 2% acetone) to give compound **222C** as a yellow solid (0.710 g). HPLC: 100% at 4.160 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 517.6 [M+Na]$^+$.

**D. (3aα,4β,5α,7β,7aα)-4-(Octahydro-5-hydroxy-4,7-dimethyl-1,3-dioxo-4,7-epoxy-2H-isoindol-2-yl)-2-(trifluoromethyl)benzonitrile (222D)**

Compound **222C** (0.040 g, 0.081 mmol) was dissolved in THF (1.0 mL) and HF• Pyridine (0.5 mL) was added. After 2 h, the reaction mixture was carefully poured into cold saturated aq $NaHCO_3$. The mixture was then extracted with methylene chloride (3 x 10 mL). The combined organics were washed with 1 N HCl (1 x 10 mL) and dried over anhydrous sodium sulfate. Concentration *in vacuo* gave compound **222D** as a yellow solid (0.031 g). NOE experiments confirmed the assigned isomer. HPLC: 98% at 2.777 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 403.06 [M+Na]$^+$.

**Example 223**

**(αR)-α-Methoxybenzeneacetic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester (223C)**

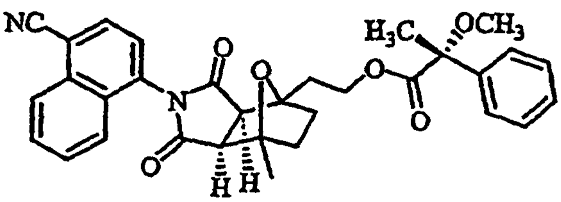

**A. (3aα,4β,7β,7aα)-4-[4-[2-[[(1,1-Dimethylethyl)dimethylsilyl]oxy]ethyl]octahydro-7-methyl-1,3-dioxo-4,7-epoxy-2H-isoindol-2-yl]-1-naphthalenecarbonitrile** (223A)

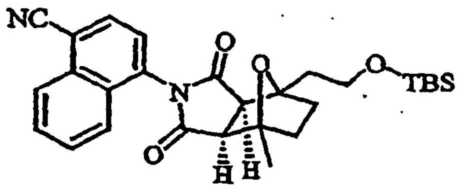

A solution of 4-amino-1-naphthalenecarbonitrile (19.2 g, 114 mmol) and maleic anhydride (14.0 g, 113 mmol)

in AcOH (230 mL) was heated at 115 °C for 12 h. After cooling to rt, the reaction mixture was concentrated under reduced pressure then diluted with $CH_2Cl_2$ (2.5 L). The organic layer was washed 3x with $H_2O$ (3 L), 1x with sat. aq $Na_2CO_3$ (1 L) and 1x with brine (1 L), dried over $MgSO_4$ and concentrated to ~200 mL under reduced pressure. Purification by flash chromatography on cation exchange resin (60 g, CUBX13M6 from United Chemical Technologies) eluting with $CH_2Cl_2$ gave 25.0 g (88%) of 4-(2,5-Dihydro-2,5-dioxo-1H-1-yl)-1-naphthalenecarbonitrile as a yellow solid. HPLC 96% at 2.48 min (Phenomenex-prime S5-C18 column 4.6 x 50 mm, 10-90% aqueous methanol over 4 minutes containing 0.2% phosphoric acid, 4 mL/min, monitoring at 220 nm), MS (ES): m/z 249.25 $[M+H]^+$.

4-(2,5-Dihydro-2,5-dioxo-1H-1-yl)-1-naphthaienecarbonitrile (1.00 g, 4.03 mmol) was suspended in benzene (6.0 mL) in a sealed tube and compound **204A** (1.11 g, 5.24 mmol) was added. The reaction mixture was heated at 60°C for 16 h and then cooled to 25°C. The benzene was removed in vacuo to give a yellow solid. The solid was dissolved in ethyl acetate (40 mL) and Pd/C (10% Pd, 0.300 g) was added. Hydrogen was then introduced via a balloon. After 4 h, the reaction mixture was filtered through celite rinsing with ethyl acetate. Concentration *in vacuo* gave a pale yellow solid which was purified by flash chromatography on silica gel eluting with acetone/chloroform (0% - 1.5% - 3% acetone) to give compound **223A** (1.53 g) as a yellow foam. HPLC: 86% at 4.173 min (retention time) (YMC S5 ODS column 4.6 x 50 mm eluting with 10-90% aqueous methanol over 4 minutes containing 0.2% phosphoric acid, 4 mL/min, monitoring at 220 nm).

**B. (3aα,4β,7β,7aα)-4-[Octahydro-4-(2-hydroxyethyl)-7-methyl-1,3-dioxo-4,7-epoxy-2H-isoindol-2-yl]-1-naphthalenecarbonitrile (223B)**

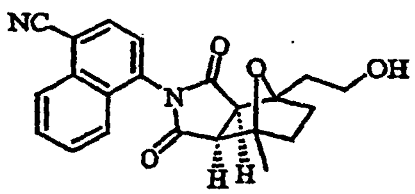

Compound **223A** (1.37 g, 2.97 mmol) was dissolved in THF (8.0 mL) and transferred to a polypropylene bottle and cooled to 0°C. HF• Pyridine (2.0 mL) was then added. After 20 min, the reaction mixture was carefully poured into cold sat. aq sodium bicarbonate and extracted with methylene chloride (3 x 30 mL). The organics were then washed with 1 N HCl and dried over anhydrous sodium sulfate. Concentration *in vacuo* gave the compound **223B** (0.99 g) as a yellow foam which was not purified further. HPLC: 96% at 2.443 and 2.597 (atropisomers) min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 399.02 $[M+Na]^+$.

**C. (αR)-α-Methoxybenzeneacetic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester (223C)**

Compound **223B** (0.200 g, 0.575 mmol) was added to a solution of WSDCC (0.138 g, 0.719 mmol) and (R) -mandelic acid (0.096 g, 0.575 mmol) in dichloromethane (6.0 mL). 4-DMAP (0.005 g) was then added and the reaction mixture was stirred at 25°C for 4 h. The mixture was then diluted with dichloromethane and washed with 1 N HCl (2 x 10 mL), once with sodium bicarbonate (10 mL) and dried over anhydrous sodium sulfate. Concentration *in vacuo* gave compound **223C** (0.220 g) as a yellow solid which was not purified further. HPLC: 100% at 3.283 min (retention time) (YMC S5 ODS column 4.6 x 50 mm eluting with 10-90% aqueous methanol over 4 minutes containing 0.2% phosphoric acid, 4 mUmin, monitoring at 220 nm), MS (ES): m/z 547.26 $[M+Na]^+$.

**Example 224**

**(3aα,4β,7β,7aα)-2-(Methylthio)-4-(octahydro-4,7-dimethyl-1,3-dioxo-4,7-epoxy-2H-isoindol-2-yl)benzonitrile (224)**

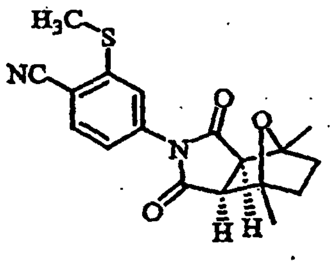

4-Amino-2-(methylthio)benzonitrile (100 mg, 0.61 mmol, synthesized as described in EP 40931 A1) was reacted in a sealed tube with compound **20A** (131 mg, 0.67 mmol), $MgSO_4$ (161 mg, 1.34 mmol) and $Et_3N$ (0.44 mL, 3.17 mmol) in 0.50 mL toluene according to the procedure described in Example **208C** to give, after purification by preparative reverse phase HPLC (YMC S5 ODS 20 x 100 mm eluting with 30-100% aqueous methanol over 10 min containing 0.1% TFA, 20 mL/min), 137 mg (0.40 mmol, 66%) of compound **224** as a white solid. HPLC: 100% at 2.73 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 401.0 $[M-H+OAc]^-$.

## Example 225

**(3aα,4β,7β,7aα)-2-(Methylsulfinyl)-4-(octahydro-4,7-dimethyl-1.3-dioxo-4,7-epoxy-2H-isoindol-2-yl)benzonitrile (225)**

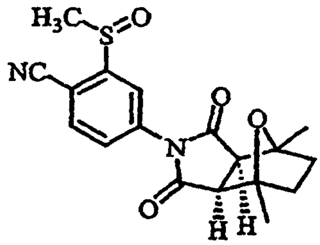

To an ice-cold suspension of compound **224** (30 mg, 0.09 mmol) in 2 mL of $H_2O$/MeOH (1:1) was added oxone (80 mg, 0.26 mmol) in one solid portion. The resulting mixture was stirred for 4 h at 0°C before it was diluted with $H_2O$ (10 mL) and extracted with $CH_2Cl_2$ (2 x 20 mL). The combined organic layers were dried and concentrated to leave a residue which was purified by filtering the material through a short pad of silica gel eluting with $CH_2Cl_2$ to yield 32 mg (0.09 mmol, 100%) of compound **225** as a colorless oil. HPLC: 99% at 2.01 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 376.0 $[M+NH_4]^+$.

## Example 226

**(3aα,4β,7β,7aα)-2-(Methylsulfonyl)-4-(octahydro-4,7-dimethyl-1,3-dioxo-4,7-epoxy-2H-isoindol-2-yl)benzonitrile (226)**

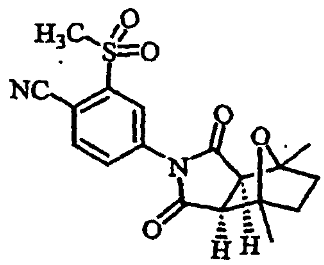

To a solution of compound **225** (48 mg, 0.14 mmol) in $CH_2Cl_2$ (2 mL) was added *m*CPBA (145 mg, 50% mixture, 0.42 mmol) in one solid portion. The resulting mixture was allowed to warm to room temperature and was stirred for 60 h at which time no more starting material could be detected by HPLC. The reaction was quenched by the addition of sat. $NaHCO_3$ solution (5 mL), the layers were separated and the aqueous layer was extracted with $CH_2Cl_2$ (20 mL). The combined organic phases were dried over $MgSO_4$ and concentrated. The remaining residue was purified by preparative reverse phase HPLC (YMC S5 ODS 20 x 100 mm eluting with 30-100% aqueous methanol over 10 min containing 0.1% TFA, 20 mL/min) to afford 48 mg (0.13 mmol, 92%) of compound **226** as a white solid. HPLC: 100% at 2.07 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 392.0 $[M+NH_4]^+$.

## Example 227

**(3aα,4β,5β,7β,7aα)-7-[2-[[(1,1-Dimethylethyl)dimethylsilyl]oxy]ethyl]hexahydro-5-hydroxy-4-methyl-2-(4-nitro-1-naphthalenyl)-4,7-epoxy-1H-isoindole-1,3(2H)-dione (227B)**

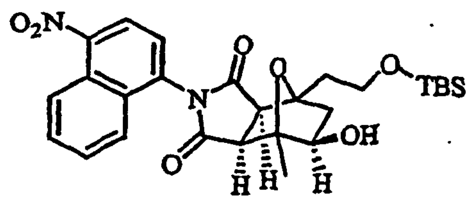

**A. (3aα,4β,7β,7aα)-4-[2-[[(1,1-Dimethylethyl)dimethylsilyl]oxy]ethyl]-3a,4,7,7a-tetrahydro-7-methyl-2-(4-nitro-1-naphthalenyl)-4,7-epoxy-1H-isoindole-1,3(2H)-dione (227A)**

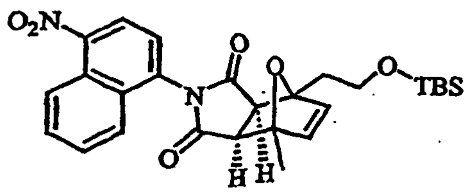

A solution of compound **204A** (455 mg, 1.894 mmol, 2 eq) and 1-[4-nitronaphthalene]-1H-pyrrole-2,5-dione (254 mg, 0.947 mmol, 1 eq) (prepared as described for 4-(2,5-dihydro-2,5-dioxo-1H-1-yl)-1-naphthalenecarbonitrile, Example 223A) in benzene (2 mL) was heated at 60°C overnight. The reaction mixture was concentrated under reduced pressure to give crude compound **227A** as a brown solid, which was used directly in the next step with out further purification.

**B. (3aα,4β,5β,7β,7aα)-7-[2-[[(1,1-Dimethylethyl)dimethylsilyl]oxy]ethyl]hexahydro-5-hydroxy-4-methyl-2-(4-nitro-1-naphthalenyl)-4,7-epoxy-1H-isoindole-1,3(2N)-dione (227B)**

$BH_3$•THF (0.95 mL, 0.95 mmol, 1M in THF, 1 eq) was added to a solution of crude compound **227A** (0.95 mmol, 1 eq) in THF (2 mL) at 0°C. After compound **227A** was consumed, as was evident by HPLC, the reaction mixture was concentrated under reduced pressure. The resulting residue was then dissolved in toluene (2 mL), $Me_3NO$ (71 mg, 2.84 mmol, 3 eq) was added and the mixture was heated to reflux over night. The reaction mixture was then cooled to rt, added to $H_2O$ and extracted with EtOAc (3x). The combined organic layers were dried over $MgSO_4$ and concentrated under reduced pressure. Purification by flash chromatography on $SiO_2$ eluting with a mixture of 75% EtOAc/Hexanes, gave 130.2 mg (26%) of compound **227B** as a brown solid. HPLC: 94% at 3.92 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 527.5 $[M+H]^+$.

**Example 228**

**(3aα,4β,5β,7β,7aα)-Hexahydro-5-hydroxy-7-(2-hydroxyethyl)-4-methyl-2-(4-nitro-1-naphthalenyl)-4,7-epoxy-1H-isoinclole-1,3(2H)-dione (228)**

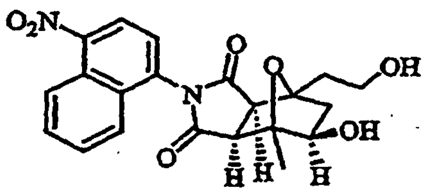

A mixture of TBAF (0.3 mL, 0.296 mmol, 1 M solution in THF) and HF (0.3 mL, 50% in $H_2O$) in $CH_3CN$ (6 mL) was added to a solution of **227B** (104 mg, 0.197 mmol) in THF (2 mL) at 0°C. The reaction mixture was stirred overnight at rt. After the starting material was consumed, as was evident by TLC, $H_2O$ and EtOAc were added and the layers were separated. The aqueous layer was extracted with EtOAc (1x) and the combined organic layers were washed with $H_2O$ (1x) and brine (1x), dried over $Na_2SO_4$ and concentrated under reduced pressure. Purification by flash chromatography on $SiO_2$ eluting with 5% $MeOH/CH_2Cl_2$ gave 61.2 mg (75%) of compound **228** as a yellow solid. HPLC: 99% at 2.47 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 411.2 $[M-H]^-$.

**Example 229**

**(3aα,4β,5β,7β,7aα)-7-[2-(4-Fluorophenoxy)ethyl]hexahydro-5-hydroxy-4-methyl-2-(4-nitro-1-naphthalenyl)-4,7-epoxy-1H-isoindole-1,3(2H)-dione (229)**

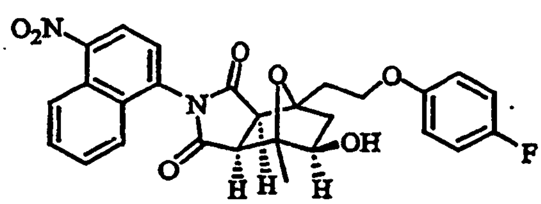

DBAD (37.7 mg, 0.164 mmol) was added to a solution of $PPh_3$ (43 mg, 0.164 mmol) in THF (1 mL). After stirring for 10 min, 4-fluorophenol (18.3 mg, 0.164 mmol) was added and the reaction mixture was stirred for a further 5 min. A solution of compound **228** (45 mg, 0.109 mmol) in THF (1 mL) was added and the mixture was stirred at rt overnight. HPLC showed the crude reaction mixture to contain mostly starting diol (compound **228),** so this mixture was added to a preformed mixture as before of $PPh_3$ (86 mg), DBAD (75.4 mg) and phenol (36.6 mg) in THF (4 mL) at rt. Stirring was

continued until all of compound **228** was consumed. The reaction mixture was then concentrated under reduced pressure. Purification by preparative chromatography [HPLC at 15.2 min (retention time) (YMC S5 ODS A column 20 x 100 mm, 10-90% aqueous methanol over 15 minutes containing 0.1% TFA, 20 mL/min, monitoring at 220 nm)] gave 25.0 mg (45%) of compound 229 as a light yellow solid. HPLC: 99% at 3.53 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 505.2 $[M-H]^-$.

**Example 230**

**(3aα,4β,5β,6β,7β,7aα)-4-(Octahydro-5,6-dihydroxy-4,7-dimethyl-1,3-dioxo-4,7-epoxy-2H-isoindol-2-yl)-2-(trifluoromethyl)benzonitrile & (3aα,4β,5α,6α,7β,7aα)-4-(Octahydro-5,6-dihydroxy-4,7-dimethyl-1,3-dioxo-4,7-epoxy-2H-isoindol-2-yl)-2-(trifluoromethyl)benzonitrile (230Bi & 230Bii, Respectively)**

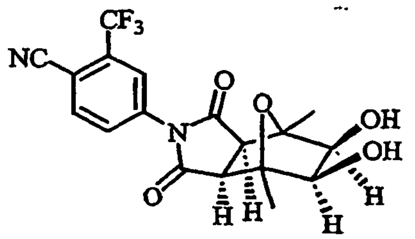

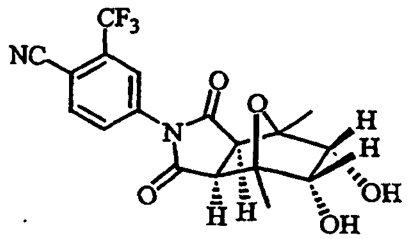

**A. (3aα,4β,7β,7aα)-4-(1,3,3a,4,7,7a-Hexahydro-4,7-dimethyl-1,3-dioxo-4,7-epoxy-2H-isoindol-2-yl)-2-(trifluoromethyl)benzonitrile (230A)**

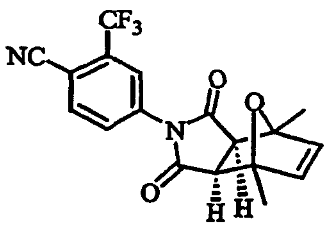

3,5-dimethyl furan (1.23 mL, 11.54 mmol) and 4-(2,5-Dihydro-2,5-dioxo-1H-pyrrol-1-yl)-2-trifluoromethylbenzonitrile (2.00 g, 7.69 mmol) were dissolved in benzene (10 mL) and heated at 60°C for 18 h. The volatile organics were then removed *in vacuo*. The resulting crude compound 230A was carried on without purification. HPLC: 71% at 3.007 min (retention time) (YMC S5 ODS column 4.6 x 50 mm eluting with 10-90% aqueous methanol over 4 minutes containing 0.2% phosphoric acid, 4 mL/min, monitoring at 220 nm).

**B. (3aα,4β,5β,6β,7β,7aα)-4-(Octahydro-5,6-dihydroxy-4,7-dimethyl-1,3-dioxo-4,7-epoxy-2H-isoindol-2-yl)-2-(trifluoromethyl)benzonitrile & (3aα,4β,5α,6α,7β,7aα)-4-(Octahydro-5,6-dihydroxy-4,7-dimethyl-1,3-dioxo-4,7-epoxy-2H-isoindol-2-yl)-2-(trifluoromethyl)benzonitrile (230Bi & 230Bii)**

Compound **230A** (0.100 g, 0.281 mmol) was dissolved in acetone and *N*-methylmorpholine-*N*-oxide (50% aq solution, 0.100 mL, 0.42 mmol) was added. $OsO_4$ (4% aq solution, 0.014 mmol) was then added. After 3 h at 25°C, the reaction was complete and sodium sulfite (0.250 g) was added with vigorous stirring. After 15 min, brine (10 mL) was added and the solution was extracted with EtOAc (3 x 15 mL). The organics were dried over anhydrous sodium sulfate and then concentrated *in vacuo*. The crude diol mixture was purified by preparative TLC eluting with 18% acetone in chloroform to give 0.038 g of compound **230Bi** (beta face) and 0.012 g of compound **230Bii** (alpha face) as pale yellow solids. Compound **230Bi:** HPLC: 100% at 2.567 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 397.08 $[M+H]^+$. Compound **230Bii**: HPLC: 100% at 2.417 min (retention time) (YMC S5 ODS column 4.6 x 50 mm

eluting with 10-90% aqueous methanol over 4 minutes containing 0.2% phosphoric acid, 4 mL/min, monitoring at 220 nm), MS (ES): m/z 397.08 $[M+H]^+$.

**Example 231**

**(3aα,4β,5β,6β,7β,7aα)-4-[Octahydro-5,6-dihydroxy-4-(hydroxyethyl)-7-methyl-1,3-dioxo-4,7-epoxy-2H-isoindol-2-yl]-1-naphthalenecarbonitrile (231C)**

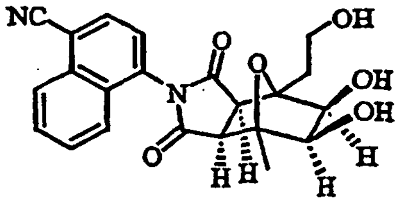

**A. (3aα,4β,7β,7aα)-4-[4-[2-[[(1,1-Dimethylethyl)dimethylsilyl]oxy]ethyl]-1,3,3a,4,7,7a-hexahydro-7-methyl-1,3-dioxo-4,7-epoxy-2H-isoindol-2-yl]-1-naphthalenecarbonitrile (231A)**

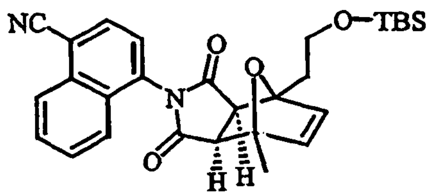

Compound **204A** (29.03 g, 120 mmol) and 4-(2,5-dihydro-2,5-dioxo-1H-1-yl)-1-naphthalenecarbonitrile (20.0 g, 80.6 mmol) were suspended in benzene (80 mL) and heated to 60°C for 14 h. The mixture was then concentrated *in vacuo* at 40°C for 40 min. The resulting slurry was cooled to 25°C and then suspended in MeOH (200 mL) and stirred at rt for 30 min. The solution was then cooled to 0°C for 30 min and then filtered rinsing with cold MeOH. The resulting solid was dried *in vacuo* to give 26.1 g of crude compound **231A** as a white solid. The methanol solution was concentrated *in vacuo* and resuspended in MeOH (50 mL) and cooled to -20°C for 4 h. The solution was then filtered rinsing with cold MeOH. The resulting solid was dried *in vacuo* to give 3.8 g of compound **231A** as a white solid. HPLC: 95% at 4.227 min (retention time) (YMC S5 ODS column 4.6 x 50 mm eluting with 10-90% aqueous methanol over 4 minutes containing 0.2% phosphoric acid, 4 mL/min, monitoring at 220 nm)

**B. (3aα,4β,5β,6β,7β,7aα)-4-[4-[2-[[(1,1-Dimethylethyl)dimethylsilyl]oxy]ethyl]octahydro-5,6-dihydroxy-7-methyl-1,3-dioxo-4,7-epoxy-2H-isoindol-2-yl]-1-naphthalenecarbonitrile (231B)**

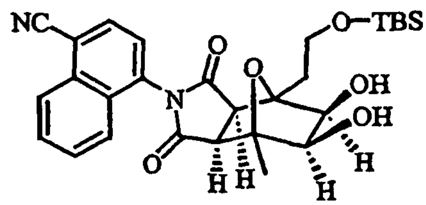

Compound **231A** (0.400 g, 0.851 mmol) was dissolved in acetone (9.0 mL) and *N*-methylmorpholine-*N*-oxide

(50% aq solution, 0.0.150 mL, 1.28 mmol) was added. $OsO_4$ (4% aq solution, 0.043 mmol) was then added. After 3 h at 25°C, the reaction was complete and sodium sulfite (1.0 g) was added with vigorous stirring. After 15 minutes, brine (30 mL) was added and the solution extracted with EtOAc (3 x 50 mL). The organics were dried over anhydrous sodium sulfate and then concentrated *in vacuo.* The crude diol was purified by flash chromatography on silica eluting with 5-25% acetone in chloroform to give 0.355 g of compound **231B** as a yellow solid. HPLC: 93% at 3.903 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 522.00 $[M+H]^+$.

**C. (3aα,4β,5β,6β,7β,7aα)-4-[Octahydro-5,6-dihydroxy-4-(hydroxyethyl)-7-methyl-1,3-dioxo-4,7-epoxy-2H-isoindol-2-yl]-1-naphthalenecarbonitrile (231C)**

Compound **231B** (0.400 g, 0.766 mmol) was dissolved in THF (5.0 mL) and transferred to a polypropylene bottle and cooled to 0°C. HF• Pyridine (1.0 mL) was then added. After 20 min, the reaction mixture was carefully poured into cold sat. aq sodium bicarbonate and extracted with methylene chloride (3 x 30 mL). The organics were then washed once with 1 N HCl and dried over anhydrous sodium sulfate. Concentration *in vacuo* gave the compound 231C (0.290 g) as a yellow foam which was not purified further. HPLC: 92% at 2.273 and 2.423 (atropisomers) min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 409.10 $[M+H]^+$.

**Example 232**

**(3aα,4β,5β,6β,7β,7aα)-4-[Octahydro-5,6-dihydroxy-4-methyl-1,3-dioxo-7-[2-[4-(trifluoromethyl)phenoxy]ethyl]-4,7-epoxy-2H-isoindol-2-yl]-2-(trifluoromethyl)benzonitrile (232C)**

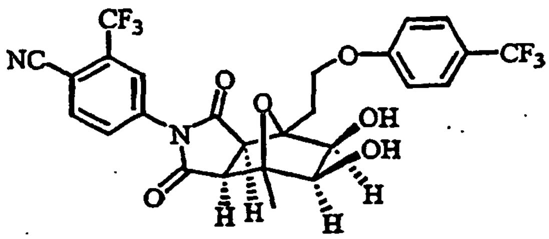

**A. 2-Methyl-5-[2-[4-(trifluoromethyl)phenoxy]ethyl]furan (232A)**

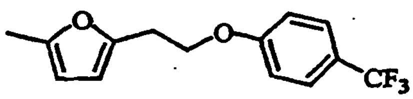

To a solution of triphenylphosphine (1.56 g, 5.95 mmol) in THF (40 mL) was added DBAD (1.37 g, 5.95 mmol). After 10 min, 4-trifluoromethylphenol (0.964 g , 5.95 mmol) was added. After 10 additional minutes, compound **21A** (0.500 g, 3.97 mmol) was added. After 14 h at 25°C, the reaction mixture was concentrated *in vacuo* and purified by flash chromatography on silica eluting with chloroform to give 0.713 g of compound **232A** as a clear oil.

**B. (3aα,4β,7β,7aα)-4-[1,3,3a,4,7,7a-hexahydro-4-methyl-1,3-dioxo-7-[2-[4-(trifluoromethyl)phenoxy]ethyl]-4,7-epoxy-2H-isoindol-2-yl]-2-(trifluoromethyl)benzonitrile (232B)**

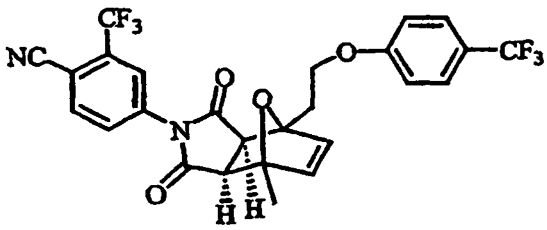

Compound **232A** (0.301 g, 1.15 mmol) and 4-(2,5-dihydro-2,5-dioxo-1H-pyrrol-1-yl)-2-trifluoromethylbenzonitrile (0.220 g, 0.846 mmol) were suspended in benzene (1.5 mL) and heated to 60°C for 14 h. The mixture was then concentrated *in vacuo* at 40°C for 40 minutes. The crude product was purified by flash chromatography on silica eluting with 10 - 0% hexanes in methylene chloride to give 0.199 g of compound 232B as a yellow solid. Compound **232B** was characterized as the exo diastereomer by NOE experiments. HPLC: 94% at 3.993 min (retention time) (YMC S5 ODS column 4.6 x 50 mm eluting with 10-90% aqueous methanol over 4 minutes containing 0.2% phosphoric acid, 4 mL/min, monitoring at 220 nm).

**C. (3aα,4β,5β,6β,7β,7aα)-4-[Octahydro-5,6-dihydroxy-4-methyl-1,3-dioxo-7-[2-[4-(trifluoromethyl)phenoxy] ethyl]-4,7-epoxy-2H-isoindol-2-yl]-2-(trifluoromethyl)benzonitrile (232C)**

Compound **232B** (0.075 g, 0.140 mmol) was dissolved in acetone (2.0 mL) and N-methytmorphoiine-N-oxide (50% aq solution, 0.025 mL, 0.21 mmol) was added. $OsO_4$ (4% aq solution, 0.007 mmol) was then added. After 3 h at 25°C, the reaction was complete and sodium sulfite (0.25 g) was added with vigorous stirring. After 15 minutes, brine (5 mL) was added and the solution extracted with EtOAc (3x10 mL). The organics were dried over anhydrous sodium sulfate and then concentrated *in vacuo.* The crude diol was purified by preparative TLC on silica gel, eluting with 10% acetone in chloroform to give 0.038 g of compound **232C** as a yellow solid. HPLC: 98% at 3.747 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 593.08 $[M+Na]^+$.

**Example 233**

(3aα,4β,5β,5aβ,8aβ,8bα)-4-(Decahydro-5-hydroxy-4-methyl-1,3-dioxo-4,8a-epoxy-2H-furo[3,2-e]isoindol-2-yl)-1-naphthalenecarbonitrile (233)

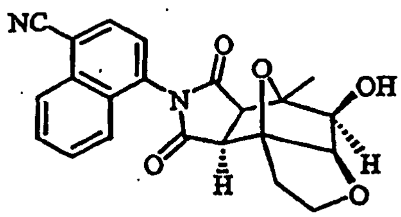

To a solution of triphenylphosphine (0.072 g, 0.276 mmol) in THF (3.0 mL) was added DBAD (0.063 g, 0.276 mmol). After 10 min, 4-cyanophenol (0.033 g , 0.276 mmol) was added. After 10 additional minutes, compound **231C** (0.075 g, 0.184 mmol) was added. After 3 h at 25°C, the reaction mixture was concentrated *in vacuo* and purified by preparative TLC on silica gel, eluting with 15% acetone in chloroform to give 0.068 g of compound **233** as a white solid. HPLC: 95% at 2.430 and 2.560 min (atropisomers) (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 391.09 $[M+H]^+$.

**Example 234**

**(3aα,4β,7β,7aα)-2-(4-Cyano-1-naphthalenyl)octahydro-7-methyl-1,3-dioxo-4,7-epoxy-4H-isoindole-4-acetic acid (234B)**

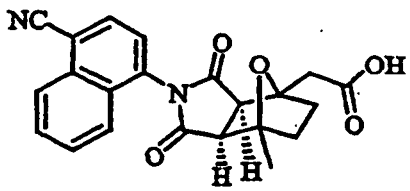

**A. (3aα,4β,7β,7aα)-2-(4-Cyano-1-naphthalenyl)-1,2,3,3a,7,7a-hexahydro-7-methyl-1,3-dioxo-4,7-epoxy-4H-isoindole-4-acetic acid (234A)**

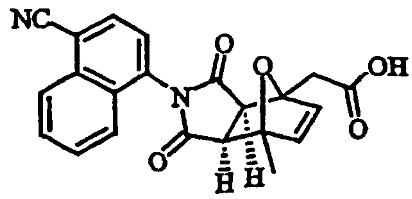

5-Methyl-2-furanacetic acid (0.500 g, 3.57 mmol) and 4-(2,5-dihydro-2,5-dioxo-1H-1-yl)-1-naphthalenecarbonitrile (0.899 g, 3.57 mmol) were dissolved in benzene (3.0 mL) and heated at 60°C for 2 h and then cooled to 25°C. After 12 h, a white solid precipitated out of solution and was filtered and rinsed with diethyl ether to yield 1.20 g of compound **234A** as a light yellow solid. NMR showed only one diastereomer. HPLC: 86% at 2.767 min (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 389.45 $[M+H]^+$.

**B (3aα,4β,7β,7aα)-2-(4-Cyano-1-naphthalenyl)octahydro-7-methyl-1,3-dioxo-4,7-epoxy-4H-isoindole-4-acetic acid (234B)**

Compound **234A** (1.1 g, 2.82 mmol) was dissolved in EtOH/EtOAc (1:1, 50 mL) and Pd/C (10% Pd, 0.4 g) was added followed by $H_2$ via a balloon. After 5 h at 25°C, the reaction mixture was filtered through celite rinsing with EtOAc and concentrated *in vacuo* to yield 1.00 g of compound **234B** as a yellow solid. HPLC: 80% at 2.84 min (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 391.1 $[M+H]^+$.

**Example 235**

**(3aα,4β,7β,7aα)-2-(4-Cyano-1-naphthalenyl)octahydro-7-methyl-1,3-dioxo-4,7-epoxy-4H-isoindole-4-acetic acid, methyl ester (235)**

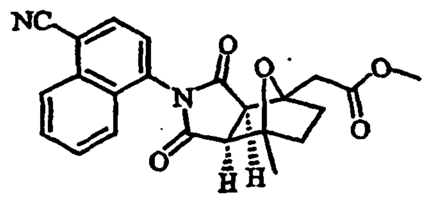

Compound **234B** (0.050 g, 0.125 mmol) was dissolved in acetonitrile (2.0 mL), then DCC (0.025 g, 0.125 mmol) was added followed by HOAc (0.018 g, 0.125 mmol). 4-Fluorobenzyl alcohol (0.014 mL, 0.125 mmol) was added and the reaction mixture was stirred for 3 h. The reaction mixture was then concentrated in vacuo and purified by preparative reverse phase HPLC (YMC S5 ODS 20 x 100 mm, 10-90% aqueous methanol over 15 min containing 0.1% TFA, 20 mL/min, monitoring at 220 nm). Purification yielded 0.040 g of compound **235** as a white solid, rather than the expected benzyl ester. None of the anticipated benzyl ester was observed by NMR or LC-MS. HPLC: 100% at 3.033 min (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 405.51 $[M+H]^+$.

**Example 236**

**(3aα,4β,7β,7aα)-2-(4-Cyano-1-naphthalenyl)-N-[(4-fluorophenyl)methyl]octahydro-7-methyl-1,3-dioxo-4,7-epoxy-4H-isoindole-4-acetamide (236)**

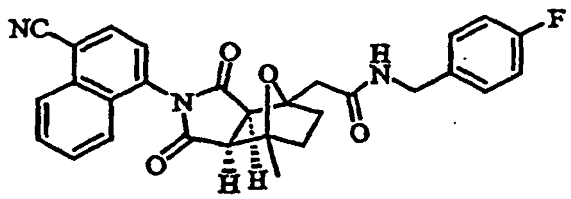

Compound **234B** (0.100 g, 0.256 mmol) was dissolved in acetonitrile (4.0 mL). HOAc (0.035 g, 0.256 mmol) and DCC (0.049 g, 0.256 mmol) were then added followed by 4-fluorobenzylamine (0.030 mL, 0.256 mmol). After 4 h at 25°C, the reaction mixture was concentrated *in vacuo* and purified by preparative HPLC (YMC S5 ODS 20 x 100 mm, 10-90% aqueous methanol over 15 minutes containing 0.1% TFA, 20 mL/min, monitoring at 220 nm) to yield 0.085 g of compound **236** as a white solid. HPLC: 100% at 3.277 min (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 498.43 $[M+H]^+$.

**Example 237**

**(3aα,4β,7β,7aα)-N-[2-[2-(4-Cyano-1-naphthalenyl)octahydro-7-methyl-1,3-dioxo-4,7-epoxy-4H-isoindol-4-yl]ethyl]-4-fluorobenzamide (237B)**

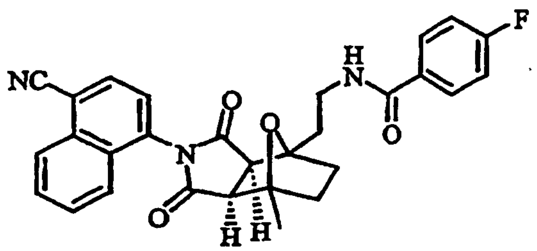

**A. 4-Fluoro-N-[2-(5-methyl-2-furanyl)ethyl]benzamide (237A)**

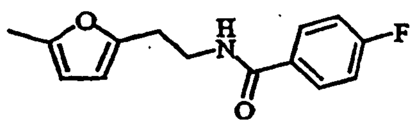

4-Fluorophenylacetyl chloride (0.29 mL, 2.44 mmol) was added dropwise to a solution of β-(5-methyl-2-furanyl) ethanamine (300 mg, 2.44 mmol, made according to the procedure of Yur'ev, Yu. K., *et al., J. Gen. Chem. USSR (Engl. Transl.)* 33, 3444-8 (1963)) in THF (2.5 mL) at rt, then $Et_3N$ (0.34 mL, 2.44 mmol) was added dropwise. Once the starting material was consumed, as was evident by HPLC, the reaction mixture was quenched with $H_2O$ and extracted with $CH_2Cl_2$. The combined organic layers were dried over $MgSO_4$ and concentrated under reduced pressure. Purification by flash chromatography eluting with 0%-50% EtOAc/hexane gradient gave 523 mg (95%) of compound **237A** as a white solid. HPLC: 99% at 2.84 min (retention time) (Phenomenex-prime S5-C18 column 4.6 x 50 mm eluting with 10-90% aqueous methanol over 4 minutes containing 0.2% phosphoric acid, 4 mL/min, monitoring at 220 nm), MS (ES): m/z 248.15 $[M+H]^+$.

**B. (3aα,4β,7β,7aα)-N-[2-[2-(4-Cyano-1-naphthalenyl)octahydro-7-methyl-1,3-dioxo-4,7-epoxy-4H-isoindol-4-yl] ethyl]-4-fluorobenzamide (237B)**

A solution of compound **237A** (221.5 mg, 0.896 mmol) and 4-(2,5-dihydro-2,5-dioxo-1H-1-yl)-1-naphthalenecarbonitrile (222.4 mg, 0.896 mmol) in benzene (4 mL) was heated at 60°C overnight. The reaction mixture was concentrated under reduced pressure and dissolved in EtOAc (30 mL). 10% Pd/C (50 mg) was added and the mixture was stirred under a hydrogen balloon overnight The reaction mixture was filtered through a pad of celite and concentrated under reduced pressure. Purification by flash chromatography eluting with 25%-75% EtOAc/hexane gradient gave 160.3 mg (36%) of compound **237B** as an off-white solid. HPLC: 97% at 3.13 & 3.23 min (retention time) (Phenomenex-prime S5-C18 column 4.6 x 50 mm eluting with 10-90% aqueous methanol over 4 minutes containing 0.2% phosphoric acid, 4 mL/min, monitoring at 220 nm), MS (ES): m/z 498.11 $[M+H]^+$.

**<u>Example 238</u>**

**[3aR-(3aα,4β,7β,7aα)]-4-[Octahydro-4-(2-hydroxyethyl)-7-methyl-1,3-dioxo-4,7-epoxy-2H-isoindol-2-yl]-1-naphthalenecarbonitrile & [3aS-(3aα,4β,7β,7aα)]-4-[Octahydro-4-(2-hydroxyethyl)-7-methyl-1,3-dioxo-4,7-epoxy-2H-isoindol-2-yl]-1-naphthalenecarbonitrile (238i & 238ii)**

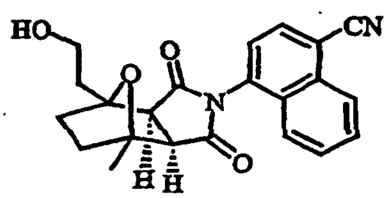

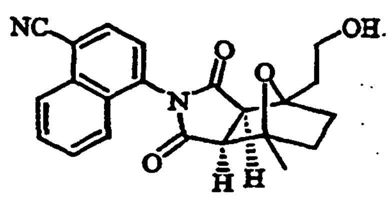

Racemic compound **223B** was separated into its enantiomers by preparative chiral HPLC (CHIRALPAK AD 5 x 50 cm column; eluting with 20% MeOH/EtOH (1:1) in heptane (isocratic) at 50 mL/min, @ 220 nm) to give the faster eluting compound **238i** (Chiral HPLC: 13.54 min; CHIRALPAK AD 4.6 x 250 mm column; eluting with 20% MeOH/EtOH (1:1) in heptane at 1 mL/min) and the slower eluting compound **238ii** (Chiral HPLC: 14.99 min; CHIRALPAK AD 4.6 x 250 mm column; eluting with 20% MeOH/EtOH (1:1) in heptane at 1 mL/min). The absolute conformation for compounds **238i** & **238ii** was not established. For simplicity in nomenclature, compound **238i** is designated herein as having an "R" configuration and compound **238ii** as having an "S" configuration. Enantiomerically pure products derived from compound **238i** are designated herein as having a "R" configuration and enantiomerically pure products derived from compound **238ii** are designated herein as having an "S" configuration.

**<u>Example 239</u>**

**[3aR-(3aα,4β,7β,7aα)]-4-[4-[2-(3-Fluorophenoxy)ethyl]octahydro-7-methyl-1,3-dioxo-4,7-epoxy-2H-isoindol-2-yl]-1-naphthalenecarbonitrile & [3aS-(-(3aα,4β,7β,7aα)]-4-[4-[2-(3-Fluorophenoxy)ethyl]octahydro-7-methyl-1,3-dioxo-4,7-epoxy-2H-isoindol-2-yl]-1-naphthalenecarbonitrile (239i & 239ii)**

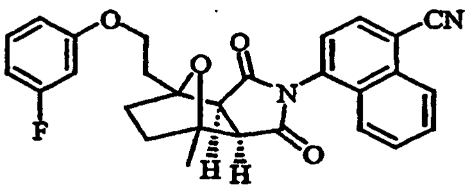

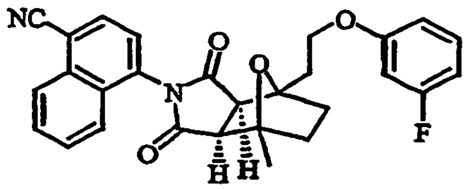

To a solution of triphenylphosphine (0.0524 g, 0.20 mmol) in THF (2.0 mL) was added DBAD (0.046 g, 0.2 mmol). After 10 min, 3-fluorophenol (0.018 mL, 0.2 mmol) was added. After 10 additional minutes, enantiomerically pure compound **238i** (0.050 g, 0.133 mmol) was added. After 3 h at 25°C, the reaction mixture was concentrated *in vacuo* and purified by preparative HPLC (YMC S5 ODS 20 x 100 mm, 10-90% aqueous methanol over 15 minutes containing 0.2% TFA, 20 mL/min, monitoring at 220 nm) to give 0.031 g of compound **239i** as a white solid. This process was repeated with enantiomerically pure compound **238ii** to yield compound **239ii.** Compound **239i:** HPLC: 100% at 3.80 min (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 471.65 $[M+H]^+$, $[\alpha]_D^{25}$ = -47.371 (c = 4.412 mg/cc, $CH_2Cl_2$). Compound **239ii:** HPLC: 100% at 3.80 min (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 471.65 $[M+H]^+$, $[\alpha]_D^{25}$ = +24.3 (c = 4.165 mg/cc, $CH_2Cl_2$).

## Example 240

**(4-Fluorophenyl)carbamic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester (240)**

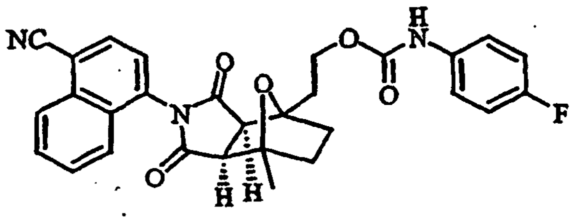

Compound **223B** (0.100 g, 0.279 mmol) was dissolved in dichloroethane (3.0 mL) and 4-fluorophenylisocyanate (0.048 mL, 0.419 mmol) was added followed by heating to 60°C. After 2 h, the reaction mixture was cooled to 25°C and diluted with methylene chloride. The mixture was washed once with sat. aq sodium bicarbonate (20 mL) and then the organics were dried over anhydrous sodium sulfate. The crude material was purified by flash chromatography on silica eluting with 15% acetone in chloroform to give 0.098 g of compound **240** as a yellow foam. HPLC: 98% at 3.320 and 3.457 min (atropisomers) (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 514.13 $[M+H]^+$.

## Example 241

**(3aα,4β,7β,7aα)-4-[Octahydro-4-(2-hydroxyethyl)-1,3-dioxo-4,7-epoxy-2H-isoindol-2-yl]-1-naphthalenecarbonitrile (241D)**

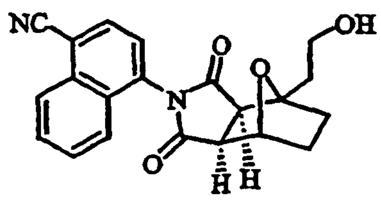

**A. 2-[2-[[(1,1-Dimethylethyl)dimethylsilyl]oxy]ethyl]furan (241A)**

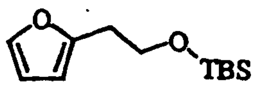

2-(2-Hydroxyethyl)furan (1.00 g, 8.93 mmol, Example **255A**) was dissolved in DMF at 25°C and imidazole (0.790 g, 11.61 mmol) was added. TBSCl (1.35 g, 8.93 mmol) was then added by portions over 5 minutes. After 2 h, the reaction mixture was poured into diethyl ether (300 mL) and washed sequentially with water (1 x 100 mL), 1 N HCl (1 x 100 mL), and brine (1 x 100 mL). The combined organics were then dried over magnesium sulfate and concentrated *in vacuo.* Compound **241A** was isolated as a clear oil (1.77 g) and was taken on without purification. HPLC: 100% at 4.233 min (retention time) (YMC S5 ODS column 4.6 x 50 mm, 10-90% aqueous methanol over 4 minutes containing 0.2% phosphoric acid, 4 mL/min, monitoring at 220 nm).

**B. (3aα,4β,7β,7aα)-4-[4-[2-[[(1,1-Dimethylethyl)dimethylsilyl]oxy]ethyl]-1,3,3a,4,7,7a-hexahydro-1,3-dioxo-4,7-epoxy-2H-isoindol-2 yl]-1-naphthalenecarbonitrile (241B)**

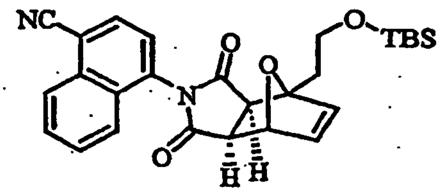

4-(2,5-Dihydro-2,5-dioxo-1H-1-yl)-1-naphthalenecarbonitrile (0.721 g, 3.40 mmol) was suspended in benzene (5.0 mL) in a sealed tube and compound **241A** (1.00 g, 4.42 mmol) was added. The reaction mixture was heated at 60°C for 16 h and then cooled to 25°C. The benzene was removed *in vacuo* to give a yellow solid. The crude material was purified by flash chromatography on silica eluting with 1-5% acetone in chloroform to give 1.37 g of compound **241B** as a yellow solid. NMR experiments confirmed exo isomer assignment. HPLC: 100% at 4.030 and 4.110 (atropsisomers) min (retention time) (YMC S5 ODS column 4.6 x 50 mm, 10-90% aqueous methanol over 4 minutes containing 0.2% phosphoric acid, 4 mL/min, monitoring at 220 nm).

**C. (3aα,4β,7β,7aα)-4-[4-[2-[[(1,1-Dimethylethyl)dimethylsilyl]oxy]ethyl]octahydro-1,3-dioxe-4,7-epoxy-2H-isoindol-2-yl]-1-naphthalenecarbonitrile (241C)**

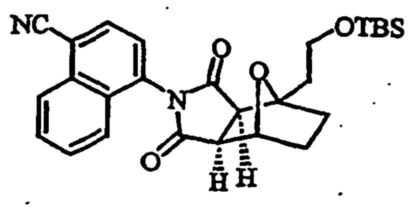

The compound **241B** (0.500 g. 1.14 mmol) was dissolved in ethyl acetate (40 mL) and Pd/C (10% Pd, 0.200 g) was added. Hydrogen was then introduced via a balloon. After 4 h, the reaction mixture was filtered through celite, rinsed with ethyl acetate and concentrated *in vacuo* to yield a pale yellow solid, which was purified by flash chromatography on silica gel eluting with acetone/chloroform (0% - 1.5% - 3% acetone) to give compound **241C** (0.450 g) as a yellow foam.

**D. (3aα,4β,7β,7aα)-4-[Octahydro-4-(2-hydroxyethyl)-1,3-dioxo-4,7-epoxy-2H-isoindol-2-yl]-1-naphthalenecarbonitrile (241 D)**

**Compound 241C** (0.283 g, 0.50 mmol) was dissolved in a solution of 2% 12N HCl in absolute ethanol (10 mL). After 1 h, the reaction mixture was quenched with sat. aq sodium bicarbonate and extracted with methylene chloride (4 x 20 mL). The combined organics were dried over sodium sulfate and concentrated *in vacuo* to give 0.211 g of compound **241D** as a white solid. HPLC: 100% at 2.14 min (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 363.45 $[M+H]^+$.

**Example 242**

**(3aα,4β,6β,7β,7aα)-4-[4-[2-(4-Cyanophenoxy)ethyl]octahydro-6-hydroxy-1,3-dioxo-4,7-epoxy-2H-isoindol-2-yl]-1-naphthatenecarbonitrile (242C)**

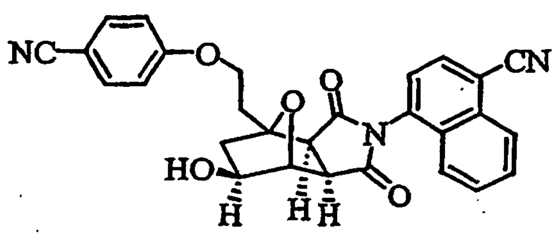

**A. (3aα,4β,6β,7β,7aα)-4-[4-[2-[[(1,1-Dimethylethyl)dimethylsilyl]oxy]ethyl]octahydro-6-hydroxy-1,3-dioxo-4,7-epoxy-2H-isoindol-2-yl]-1-naphthalenecarbonitrile (242A)**

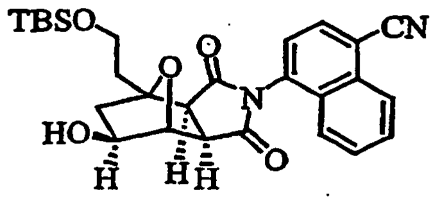

Compound **241B** (1.00 g, 2.28 mmol) and Wilkinson's catalyst (0.105 g, 0.114 mmol) were stirred rapidly under vacuum at 25°C for 1 h and then purged with $N_2$. THF (30 mL) was then added followed by catechol borane (0.487 mL, 4.57 mmol) after the olefin was completely dissolved. After 1 h, the reaction mixture was cooled to 0°C and a pH 7.2

phosphate buffer (33 mL) was added followed by EtOH (13 mL) and $H_2O_2$ (30% aq soln, 3.0 g). After 3 h at 0°C the reaction was complete by LC and the mixture was extracted with methylene chloride (3 x 50 mL). The combined organics were washed with a 1:1 mixture of 10% sodium sulfite/1N NaOH (50 mL) and once with brine (50 mL). All aqueous phases were combined and extracted with methylene chloride (50 mL) and the organic phase was combined with the previous extractions. All the organics were then dried over anhydrous sodium sulfate and then concentrated *in vacuo*. The crude material was purified by flash chromatography on silica eluting with 10-20% acetone in chloroform to give 0.634 g of compound **242A** as a white foam. HPLC: 96% at 3.797 min (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 493.13 $[M+H]^+$.

**B. (3aα,4β,6β,7β,7aα)-4-[Octahydro-6-hydroxy-4-(2-hydroxyethyl)-1,3-dioxo-4,7-epoxy-2H-isoindol-2-yl]-1-naphthalenecarbonitrile (242B)**

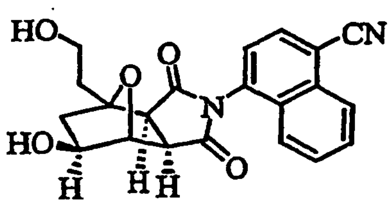

Compound **242A** (0.400 g, 0.813 mmol) was dissolved in a solution of 2% 12N HCl in absolute ethanol (10 mL). After 1 h, the reaction mixture was quenched with sat. aq sodium bicarbonate and extracted with EtOAc (4 x 20 mL). The combined organics were dried over sodium sulfate and concentrated *in vacuo* to give 0.305 g of compound 242B as a white solid. HPLC: 90% at 2.043 min (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 379.09 $[M+H]^+$.

**C. (3aα,4β,5β,7β,7aα)-4-[4-[2-(4-Cyanophenoxy)ethyl]octahydro-6-hydroxy-1,3-dioxo-4,7-epoxy-2H-isoindol-2-yl]-1-naphthalenecarbonitrile, (242C)**

To a solution of triphenylphosphine (0.054 g, 0.207 mmol) in THF (2.0 mL) was added DBAD (0.048 g, 0.207 mmol). After 10 min, 4-cyanophenol (0.025 g, 0.207 mmol) was added. After 10 additional minutes, compound **242B** (0.050 g, 0.138 mmol) was added. After 3 h at 25°C, the reaction mixture was concentrated *in vacuo* and purified by preparative TLC on silica eluting with 25% acetone/chloroform to give 0.056 g of compound **242C** as a white solid. HPLC: 90% at 2.987 min (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 480.10 $[M+H]^+$.

**<u>Example 243</u>**

**<u>[3aS-(3aα,4β,5β,7β,7aα)]-4-[Octahydro-5-hydroxy-7-(2-hydroxyethyl)-4-methyl-1,3-dioxo-4,7-epoxy-2H-isoindol-2-yl]-1-naphthalenecarbonitrile & [3aR-(3aα,4β,5β,7β,7aα)]-4-[Octahydro-5-hydroxy-7-(2-hydroxyethyl)-4-methyl-1,3-dioxo-4,7-epoxy-2H-isoindol-2-yl]-1-naphthalenecarbonitrile (243Di & 243Dii)</u>**

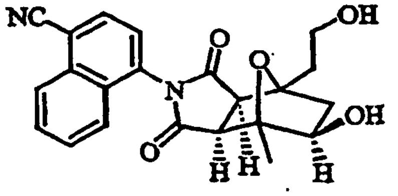

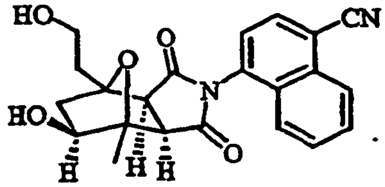

**A. (3aα,4β,7β,7aα)-4-[4-[2-[[(1,1-Dimethylethyl)dimethylsilyl]oxy]ethyl]-1,3,3a,4,7,7a-hexahydro-1,3-dioxo-4,7-epoxy-2H-isoindol-2-yl]-1-naphthalenecarbonitrile (243A)**

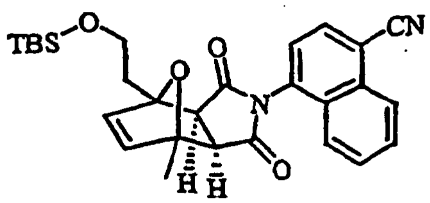

4-(2,5-Dihydro-2,5-dioxo-1H-1-yl)-1-naphthalenecarbonitrile (18.3 g, 68.7 mmol) was added to a solution of compound **204A** (26.6 g, 110.6 mmol) in benzene (75 mL) and heated to 60°C overnight. After cooling to rt, the reaction mixture was concentrated under reduced pressure. The residue was treated with MeOH (250 mL) with stirring at 0°C for 10 min. The resulting solid was filtered, washed with cold MeOH (2 x 10 mL) and dried to give 26.7 g (79.5%) of compound **243A** as a yellow solid. HPLC analysis of the above solid revealed it to be 95% pure (HPLC conditions: 95% at 2.48 min (Phenomenex-prime S5-C18 column, 4.6 x 50 mm, 10%-90% aqueous methanol over 4 minute gradient with 0.2% $H_3PO_4$, detecting at 220 nm)). The filtrate was then concentrated under reduced pressure and the resulting solid was chromatographed, eluting with 3% acetone/$CHCl_3$, to give an additional 4.36 g of compound **243A** (13%), giving a total final yield of 92.5%.

**B. (3aα,4β,5β,7β,7aα)-4-[7-[2-[[(1,1-Dimethylethyl)dimethylsilyl]oxy]ethyl]octahydro-5-hydroxy-4-methyl-1,3-dioxo-4,7-epoxy-2H-isoindol-2-yl]-1-naphthalenecarbonitrile (243B)**

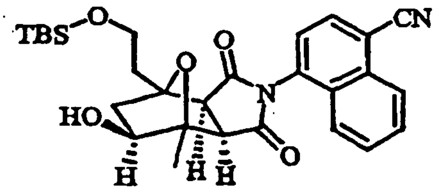

A mixture of **243A** (10 g, 20.46 mmol) and $RhCl(PPh_3)_3$ (0.947 mg, 1.02 mmol) was evacuated and filled with argon (3x). THF (200 mL) was added and once all particulates had dissolved, catechotborane (4.4 mL, 40.93 mmol) was slowly added dropwise. When the formation of product ceased, as was determined by HPLC, the reaction mixture was cooled to 0°C and quenched with phosphate buffer (330 mL, pH 7.2) then EtOH (130 mL) and $H_2O_2$ (300 mL, 30% aq sol) were added. Once boronate was consumed, the mixture was extracted with $CH_2Cl_2$ (3x) and the combined organic layers were washed with 1N NaOH, 10% aq $NaHSO_3$ (1:1, 1x) and brine (1x). The combined washes were extracted with $CH_2Cl_2$ (1x) and the combined organic layers were dried over $Na_2SO_4$. Purification by flash chromatography on silica gel eluting with 10% to 30% acetone/$CHCl_3$ gradient over 25 min gave 7.1 g (68%) of **243B** as a light yellow solid. HPLC conditions: 98% at 3.82 min (Phenomenex-prime S5-C18 column 4.6 x 50 mm, 10%-90% aqueous methanol over 4 minute gradient with 0.2% $H_3PO_4$, detecting at 220 nm).

**C. [3aR-(3aα,4β,5β,7β,7aα)]-4-[7-[2-[[(1,1-Dimethylethyl)dimethylsilyl]oxy]ethyl]octahydro-5-hydroxy-4-methyl-1,3-dioxo-4,7-epoxy-2H-isoindol-2-yl]-1-naphthalenecarbonitrile & [3aS-(3aα,4β,5β,7β,7aα)]-4-[7-[2-[[(1,1-Dimethylethyl)dimethylsilyl]oxy]ethyl]octahydro-5-hydroxy-4-methyl-1,3-dioxo-4,7-epoxy-2H-isoindol-2-yl]-1-naphthalenecarbonitrile (243Ci & 243Cii)**

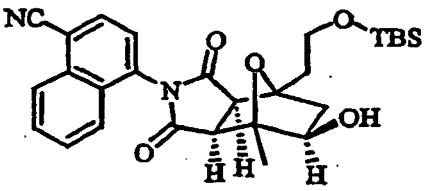
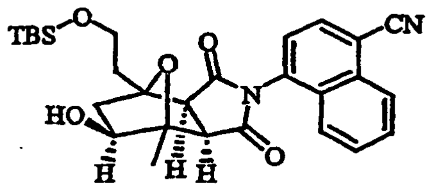

The racemic compound **243B** was separated into the individual enantiomers by chiral normal phase liquid chromatography. A Chiralpak OD column (50 x 500 mm) was used, eluting with 13% EtOH/hexanes over 99 min at 50mL/min detecting at 220 nm. The faster eluting isomer compound **243Ci** had a retention time = 45 min and the slower eluting isomer compound **243Cii** had a retention time = 66 min.

**D. [3aS-(3aα,4β,5β,7β,7aα)]-4-[Octahydro-5-hydroxy-7-(2-hydroxyethyl)-4-methyl-1,3-dioxo-4,7-epoxy-2H-isoindol-2-yl]-1-naphthalenecarbonitrile & [3aR-(3aα,4β,5β,7β,7aα)]-4-[Octahydro-5-hydroxy-7-(2-hydroxyethyl)-4-methyl-1,3-dioxo-4,7-epoxy-2H-isoindol-2-yl]-1-naphthalenecarbonitrile (243Di & 243Dii)**

Compound **243Ci** (0.84g, 2.14 mmol) was dissolved in 2% 12 N HCl/EtOH (20 mL), stirred for 5 minutes and concentrated under reduced pressure. Purification by flash chromatography on silica gel eluting with 5-10% MeOH/$CH_2Cl_2$ gave 0.57 g (88%) of **243Di**. Compound **243Di** which came from the faster eluting isomer (243Ci) was found to be 99.7% ee by analytical chiral normal phase chromatography. HPLC conditions: 99.7% at 2.17 min (Chiralcel OJ 44.6 x 250 mm, 10 micron, 40°C, isocratic 80% Heptane / 20% EtOH/MeOH (1:1), 1.0 mL/min., detection at 288 nm).

Compound **243Cii** (0.86 g, 2.19 mmol) was dissolved in 2% 12N HCl/EtOH (20 mL), stirred for 5 minutes and concentrated under reduced pressure. Purification by flash chromatography on silica gel eluting with 5-10% MeOH/$CH_2Cl_2$ gave 0.60 g (90%) of **243Dii**. Compound **243Dii** which came from the slower eluting isomer (**243Cii**) was found to have 87.1% ee by analytical chiral normal phase chromatography. HPLC conditions: 87.1% at 18.4 min (Chiralcel OJ 44.6 x 250 mm, 10 micron, 40°C, isocratic 80% heptane / 20% EtOH/MeOH (1:1), 1.0 mL/min., detection at 288 nm).

The absolute conformation for compounds **243Di** & **243Dii** was not determined. For simplicity in nomenclature, compound **243Di** is designated herein as having an "S" configuration and compound **243Dii** as having an "R" configuration. Enantiomerically pure products derived from compound **243Di** are designated herein as having an "S" configuration and enantiomerically pure products derived from compound **243Dii** are designated herein as having an "R" configuration

**Example 244**

**[3aS-(3aα,4β,5β,7β,7aα)]-4-[7-[2-(4-Cyanophenoxy)ethyl]octahydro-5-hydroxy-4-methyl-1,3-dioxo-4,7-epoxy-2H-isoindol-2-yl]-1-naphthalenecarbonitrile & [3aR-(3aα,4β,5β,7β,7aα)]-4-[7-[2-(4-Cyanophenoxy)ethyl]octahydro-5-hydroxy-4-methyl-1,3-dioxo-4,7-epoxy-2H-isoindol-2-yl]-1-naphthalenecarbonitrile (244i & 244ii)**

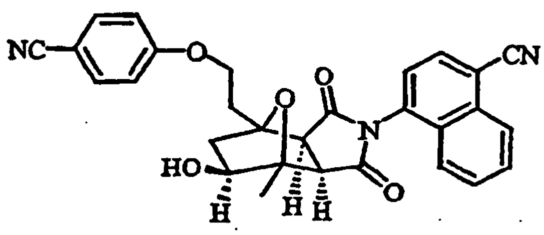
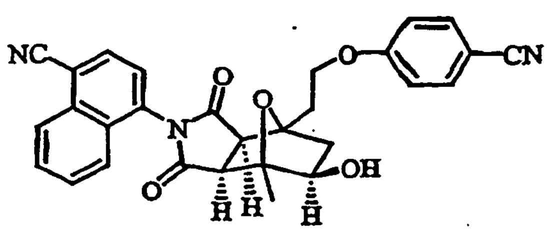

DBAD (26 mg, 0.115 mmol) was added to a solution of $PPh_3$ (30 mg, 0.115 mmol) in THF (0.65 mL). After stirring for 10 min, 4-cyanophenol (13.6 mg, 0.115 mmol) was added and the reaction mixture was stirred for a further 5 min. Compound **243Di** (30 mg, 0.076 mmol) was added and the mixture was stirred at rt for 1 h. The reaction mixture was concentrated under reduced pressure. Purification by flash chromatography on silica gel eluting with 30% acetone/ 70% $CHCl_3$ gave 23.1 mg (0.047 mmol, 61.7%) of compound **244i**. HPLC conditions: 95% at 3.06 min (YMC S5 ODS 4.6 x 50 mm, 10%-90% aqueous methanol over 4 minute gradient with 0.2% $H_3PO_4$, detecting at 220 nm). MS (ES): m/z 494.09 $[M+H]^+$. $[\alpha]_D$ = 53.30°, c = 4.5 mg/cc in THF, @ 589 nm)

DBAD (26 mg, 0.115 mmol) was added to a solution of $PPh_3$ (30mg, 0.115 mmol) in THF (0.65 mL). After

stirring for 10 min, 4-cyanophenol (13.6 mg, 0.115 mmol) was added and the reaction mixture was stirred for a further 5 min. Compound **243Dii** (30 mg, 0.076 mmol) was added and the mixture was stirred at rt for 1 h. The reaction mixture was concentrated under reduced pressure. Purification by flash chromatography on silica gel eluting with 30% acetone/ 70% $CHCl_3$ gave 20.3 mg (0.041 mmol, 54.2%) of compound **244ii.** HPLC conditions: 90% at 3.07 min (YMC S5 ODS 4.6 x 50 mm, 10%-90% aqueous methanol over 4 minute gradient with 0.2% $H_3PO_4$, detecting at 220 nm). MS (ES): m/z 494.09 $[M+H]^+$. $[\alpha]_D$ = -42.87°, c = 6.6 mg/cc in THF, @ 589 nm)

## Example 245

**(3aα,4β,7β,7aα)-4-[4-[2-(4-Cyanophenoxy)ethyl]-7-ethyloctahydro-1,3-dioxo-4,7-epoxy-2H-isoindol-2-yl]-1-naphthalenecarbonitrile. (245D)**

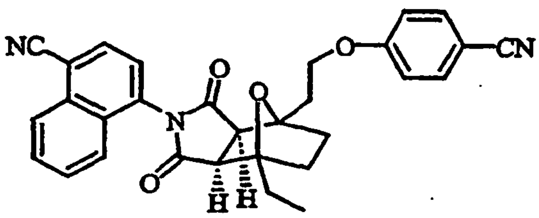

**A. 2-Ethyl-5-(2-hydroxyethyl)furan (245A)**

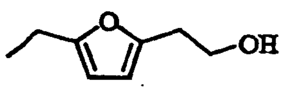

*n*-BuLi (2.5 M in hexane, 4.4 mL, 11 mmol) was added to a solution of 2-ethylfuran (1.05 mL, 10 mmol) in THF (10 mL) at -25°C. The solution was warmed to rt and stirred for 3 h. Ethylene oxide (0.75 mL) was added at -78°C. The reaction mixture was stirred for 0.5 h at -15°C and overnight at rt. Aqueous sat. $NH_4Cl$ was added and the mixture was extracted with ether (3x). The combined extracts were washed with water (1x) and brine (1x) and dried over $Na_2SO_4$. Purification by flash chromatography on silica gel eluting with 30% EtOAc/70% hexane gave 1.12 g (8.02 mmol, 80.2%) of compound 245A as a yellow oil.

**B. (3aα,4β,7β,7aα)-4-[4-Ethyl-1,3,3a,4,7,7a-hexahydro-7-(2-hydroxyethyl)-1,3-dioxo-4,7-epoxy-2H-isoindol-2-yl]-1-naphthalenecarbonitrile (245B)**

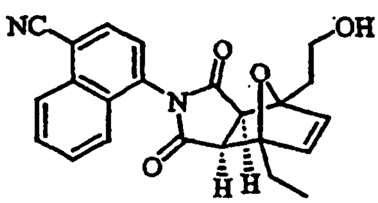

A solution of compound **245A** (280 mg, 2.00 mmol) and the 4-(2,5-dihydro-2,5-dioxo-1H-1-yl)-1-naphthalenecarbonitrile (496 mg, 2.00 mmol) in benzene (2 mL) was stirred at 60°C for 2 h. The reaction mixture was concentrated under reduced pressure. The yellow solid, compound **245B,** was used directly in the next step.

**C. (3aα,4β,7β,7aα)-4-[4-Ethyloctahydro-7-(2-hydroxyethyl)-1,3-dioxo-4,7-epoxy-2H-isoindol-2-yl]-1-naphthalenecarbonitrile (245C)**

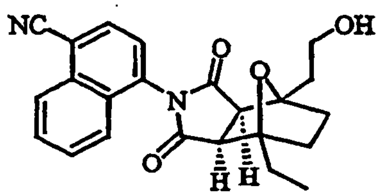

A mixture of compound **245B** (764 mg, 1.97 mmol) and 10% Pd/C (115 mg, cat.) in EtOAc (36 mL) was stirred under a hydrogen atmosphere at rt for 2 h. The reaction mixture was filtered through celite and concentrated under reduced pressure to give 779 mg of crude compound **245C**. Purification of this crude product by flash chromatography on silica gel eluting with 70% EtOAc/30% hexane gave 235 mg (0.6 mmol, 30.1%) of compound **245C**. HPLC conditions: 99% at 2.84 min (YMC S5 ODS 4.6 x 50 mm, 10%-90% aqueous methanol over 4 minute gradient with 0.2% $H_3PO_4$, detecting at 220 nm). MS (ES): m/z 391.12 $[M+H]^+$.

**D. (3aα,4β,7β,7aα)-4-[4-[2-(4-Cyanophenoxy)ethyl]-7-ethyloctahydro-1,3-dioxo-4,7-epoxy-2H-isoindol-2-yl]-1-naphthalenecarbonitrile (245D)**

DBAD (44.2 mg, 0.192 mmol) was added to a solution of $PPh_3$ (50.4 mg, 0.192 mmol) in THF (1 mL). After stirring for 10 min, 4-cyanophenol (23 mg, 0.192 mmol) was added and the reaction mixture was stirred for an additional 5 min. Compound 245C (50 mg, 0.128 mmol) was added and the mixture was stirred at rt for 2 h. The reaction mixture was concentrated under reduced pressure. Purification by flash chromatography on silica gel eluting with 40% EtOAc/ 60% hexane gave 43 mg (0.087 mmol, 68.4%) of compound **245D** as a white solid. HPLC conditions: 99% at 3.65 min (YMC S5 ODS 4.6 x 50 mm, 10%-90% aqueous methanol over 4 minute gradient With 0.2% $H_3PO_4$, detecting at 220 nm). MS (ES): m/z 492.16 $[M+H]^+$.

**Example 246**

**(3aα,4β,7β,7aα)-4-[2-(Acetyloxy)ethyl]-2-(4-cyano-1-naphthalenyl)hexahydro-7-methyl-4,7-epoxy-1H-isoindole-1,3(2H)-dione (246)**

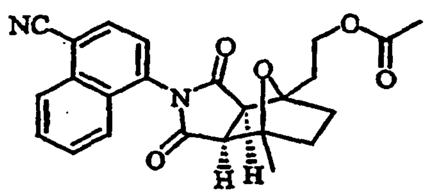

Compound **223B** (0.100 g, 0.279 mmol) was dissolved in methylene chloride (3.0 mL) at 25°C and pyridine (0.071 mL, 0.837 mmol) and 4-DMAP (1.0 mg) were added. Acetic anhydride (0.053 mL, 0.559 mmol) was then added and the reaction mixture was stirred for 20 h at 25°C. After 20 h, sat. aq sodium bicarbonate was added and the reaction mixture was stirred for 30 min. The mixture was then extracted with methylene chloride (2 x 20 mL). The organics were then washed once with 1N HCl (10 mL) and then dried over anhydrous sodium sulfate. After concentration *in vacuo,* the crude material was purified by preparative TLC on silica eluting with 12% acetone in chloroform to give 0.073 g of compound **246** as a yellow foam. HPLC: 95% at 2.837 and 3.027 min (atropisomers) (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 441.10 $[M+Na]^+$.

**Example 247**

**(3aα,4β,7β,7aα)-4-[Octahydro-4-methyl-1,3-dioxo-7-(2-oxoethyl)-4,7-epoxy-2H-isoindol-2-yl]-1-naphthalenecarbonitrile (247)**

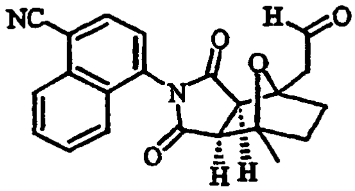

Oxalyl chloride (2.0 M soln, 1.73 mL, 3.5 mmol) was added to dry methylene chloride (10 mL) and cooled to -78°C. DMSO (0.283 mL, 3.99 mmol) was then added dropwise with the evolution of gas. After 15 min, compound **223B** (1.00 g, 2.66 mmol) was then added in methylene chloride (10 mL). After 15 min, TEA (1.10 mL, 7.98 mmol) was added and the reaction mixture was slowly warmed to 25°C. Water (30 mL) was then added and the mixture was diluted with methylene chloride (100 mL). The organics were then washed once with 1 N HCl (30 mL), once with water (30 mL) and once with brine (30 mL) and then dried over anhydrous sodium sulfate. The crude product was isolated by concentration *in vacuo* to yield compound **247** as an orange foam. Crude compound **247** was taken on directly to the next reaction. HPLC: 100% at 2.70 min (retention time) (YMC S5 ODS column 4.6 x 50 mm, 10-90% aqueous methanol over 4 minutes containing 0.2% phosphoric acid, 4 mUmin, monitoring at 220 nm), MS (ES): m/z 483.65 $[M+H]^+$.

**Example 248**

**[3aα,4β(E),7β,7aα]-4-[4-[3-(4-Cyanophenyl)-2-propenyl]octahydro-7-methyl-1,3-dioxo-4,7-epoxy-2H-isoindol-2-yl]-1-naphthalenecarbonitrile & [3aα,4β(Z),7β,7aα]-4-[4-[3-(4-Cyanophenyl)-2-propenyl]octahydro-7-methyl-1,3-dioxo-4,7-epoxy-2H-isoindol-2-yl]-1-naphthalenecarbonitrile (248i & 248ii)**

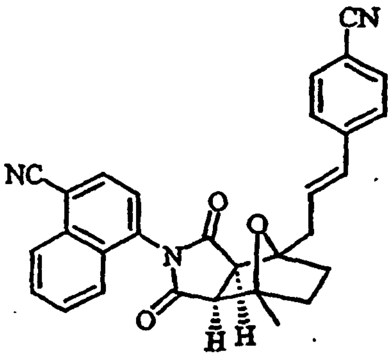

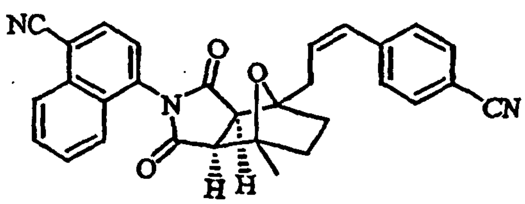

(4-cyanobenzyl)-triphenylphosphonium chloride (0.072 g, 0.174 mmol) was suspended in THF (2.0 mL) and cooled to 0°C. n-BuU (1.6 M soln, 0.092 mL, 0.147 mmol) was then added dropwise resulting in a homogenous solution. The solution was warmed to 25°C for 15 min and then cooled to 0°C. Compound 247 (0.050 g, 0.134 mmol) was then added in THF. After 1 h, the reaction mixture was quenched with sat aq ammonium chloride and then extracted with methylene chloride (3 x 20 mL). The combined organics were dried over anhydrous sodium sulfate and then concentrated *in vacuo.* The crude material was purified by preparative TLC eluting with 5% acetone in chloroform to give 0.010 g of a mixture of compounds **248i & 248ii** as a white solid. A 1:1 mixture of E.and Z olefin isomers was characterized by NMR spectroscopy. HPLC: 100% at 3.517 min (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 474.2 $[M+H]^+$.

**Example 249**

**(3aα,4β,7β,7aα)-4-[4-[3-(4-Cyanophenyl)propyl]octahydro-7-methyl-1,3-dioxo-4,7-epoxy-2H-isoindol-2-yl]-1-naphthalenecarbonitrile (249)**

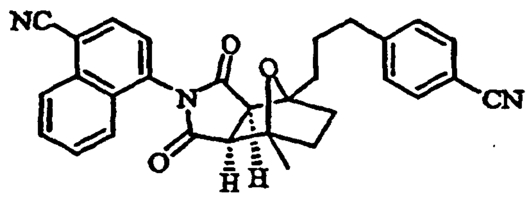

The mixture of compounds **248i** & **248ii** (0.008 g, 0.017 mmol) was dissolved in EtOH (3.0 mL) and Pd/C (10% Pd, 0.008 g) was added. $H_2$ was then introduced via a balloon. After 18 h, the reaction mixture was filtered through celite, eluting with EtOAc, followed by concentration *in vacuo*. Compound **249** was isolated as a white solid (0.007 g). HPLC: 90% at 3.520 min (retention time) (YMC S5 ODS column 4.6 x 50 mm, 10-90% aqueous methanol over 4 minutes containing 0.2% phosphoric acid, 4 mL/min, monitoring at 220 nm), MS (ES): m/z 476.13 $[M+H]^+$.

**Example 250**

**(3aα,4β,7β,7aα)-4-[4-[2-[(6-Chloro-1,2-benzisoxazol-3-yl)oxy]ethyl]octahydro-7-methyl-1,3-dioxo-4,7-epoxy-2H-isoindol-2-yl]-1-naphthalenecarbonitrile (250)**

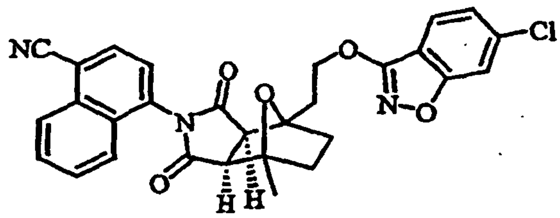

To a solution of $PPh_3$ (52 mg, 0.20 mmol) in 0.5 mL THF was added DBAD (46 mg, 0.20 mmol) as one solid portion. The resulting mixture was stirred for 10 min before 6-chloro-3-hydroxy-1,2-benzisoxazole (34 mg, 0.20 mmol) was added. Stirring was continued for 10 min before a solution of compound **223B** (50 mg, 0.13 mmol) in 0.5 mL THF was introduced via canula. The resulting mixture was stirred at ambient temperature for 24 h, concentrated and purified by preparative reverse phase HPLC (YMC S5 ODS 20 x 100 mm column; eluting with 30-100% aqueous MeOH containing 0.1% TFA over 10 min at 20 mL/min) to yield a white solid. The obtained solids were dissolved in $CH_2Cl_2$, washed with sat. $NaHCO_3$ solution, dried over $Na_2SO_4$ and concentrated to yield 50 mg (71%) of compound 250 as a colorless oil. HPLC: 26% at 3.89 min and 74% at 4.02 min (mixture of atropisomers, retention time) (YMC S5 ODS column 4.6 x 50 mm Ballistic, 10-90% aqueous methanol over 4 minutes containing 0.2% $H_3PO_4$, 4 mL/min, monitoring at 220 nm), MS (ES): m/z 528.4 $[M+ H]^+$.

**Example 251**

**(3aα,4β,7β,7aα)-4-[Octahydro-4-methyl-7-[2-[(6-nitro-1H-indazol-3-yl)oxy]ethyl]-1,3-dioxo-4,7-epoxy-2H-isoindol-2-yl]-1-naphthalenecarbonitrile (251)**

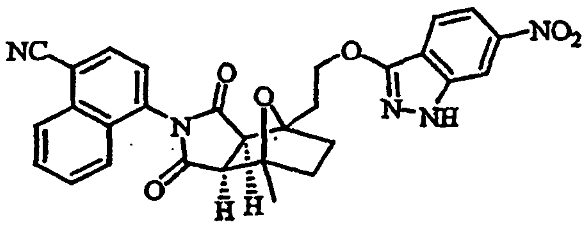

To a solution of compound **223B** (50 mg, 0.13 mmol) in toluene (1 mL) were added ADDP (50 mg, 0.20 mmol), 6-nitro-3-indazolinone (36 mg, 0.20 mmol) and n-$Bu_3P$ (50 μL, 0.2 mmol). The resulting mixture was heated to 80°C for 24 h, concentrated and purified by a combination of preparative reverse phase HPLC (YMC S5 ODS 20 x 100 mm column; eluting with 30-100% aqueous MeOH containing 0.1% TFA over 10 min at 20 mL/min) and flash chromatography (silica gel, 25% acetone in $CHCl_3$) to give 17 mg (25%) of compound **251** as a yellow solid. HPLC: 24% at 3.60 min and 76% at 3.74 min (mixture of atropisomers, retention time) (YMC S5 ODS column 4.6 x 50 mm Ballistic, 10-90% aqueous methanol over 4 minutes containing 0.2% $H_3PO_4$, 4 mL/min, monitoring at 220 nm), MS (ES): m/z 537.6 $[M+H]^+$.

**Example 252**

**[3aS-(3aα,4β,5β,7β,7aα)]-4-[7-[2-(1,2-Benzisoxazol-3-yloxy)ethyl]octahydro-5-hydroxy-4-methyl-1,3-dioxo-4,7-epoxy-2H-isoindol-2-yl]-1-naphthalenecarbonitrile (252)**

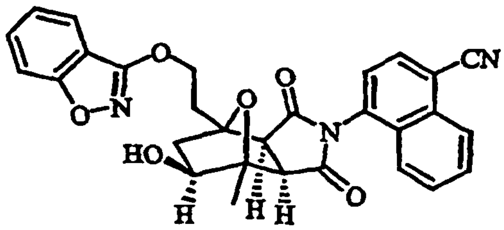

$PPh_3$ (47 mg, 0.18 mmol), DBAD (41 mg, 0.18 mmol), 3-hydroxy-1,2-benzisoxazole (24 mg, 0.18 mmol) and compound **243Di** (35 mg, 0.09 mmol) were reacted according to the procedure given for compound **250**. Purification was achieved by reverse phase HPLC (YMC S5 ODS 20 x 100 mm column; eluting with 30-100% aqueous MeOH containing 0.1 % TFA over 10 min at 20 mL/min) to yield a white solid. The obtained solids were dissolved in $CH_2Cl_2$, washed with sat. $NaHCO_3$ solution, dried over $Na_2SO_4$ and concentrated furnishing 29 mg (64%) of compound **252** as a colorless oil. HPLC: 96% at 3.29 min (mixture of abropisomers, retention time) (YMC S5 ODS column 4.6 x 50 mm Ballistic, 0-100% aqueous methanol over 4 minutes containing 0.2% $H_3PO_4$, 4 mL/min, monitoring at 220 nm), MS (ES): m/z 510.2 $[M+H]^+$.

**Example 253**

**[3aR-(3aα,4β,5β,7β,7aα)]-4-[7-[2-(1,2-Benzisoxazol-3-yloxy)ethyl]octahydro-5-hydroxy-4-methyl-1,3-dioxo-4,7-epoxy-2H isoindol-2-yl]-1-naphthalenecarbonitrile (253)**

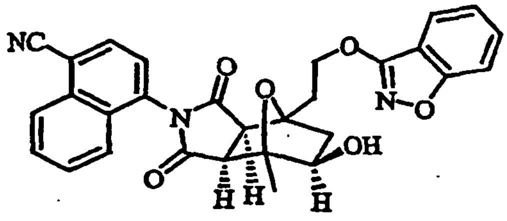

$PPh_3$ (47 mg, 0.18 mmol), DBAD (41 mg, 0.18 mmol), 3-hydroxy-1,2-benzisoxazole (24 mg, 0.18 mmol) and compound **243Dii** (35 mg, 0.09 mmol) were reacted according to the procedure given for compound 250. Purification was achieved by reverse phase HPLC (YMC S5 ODS 20 x 100 mm column; eluting with 30-100% aqueous MeOH containing 0.1% TFA over 10 min at 20 mL/min) to yield a white solid. The obtained solids were dissolved in $CH_2Cl_2$, washed with sat. $NaHCO_3$ solution, dried over $Na_2SO_4$ and concentrated furnishing 23 mg (51%) of compound **253** as a colorless oil. HPLC: 95% at 3.29 min (mixture of atropisomers, retention time) (YMC S5 ODS column 4.6 x 50 mm Ballistic, 0-100% aqueous methanol over 4 minutes containing 0.2% $H_3PO_4$, 4 mL/min, monitoring at 220 nm), MS (ES): m/z 510.4 $[M+H]^+$.

**Example 254**

**(3aα,4β,5β,7β,7aα)-4-Octahydro-5-hydroxy-4,7-dimethyl-1,3-dioxo-4,7-epoxy-2H-isoindol-2-yl)-2-(trifluoromethyl)benzonitrile & (3aα,4β,5β,7β,7aα)-4-(Octahydro-5-hydroxy-4,7-dimethyl-1,3-dioxo-4,7-epoxy-2H-isoindol-2-yl)-2-(trifluoromethyl)benzonitrile (254i & 254ii)**

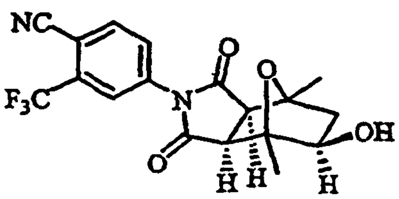 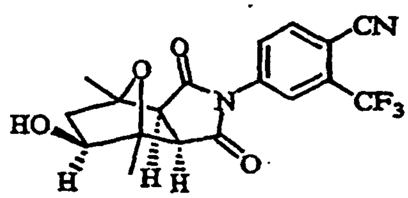

Racemic compound **221B** was separated into its enantiomers by preparative chiral HPLC (CHIRALPAK AD 5 x 50 cm column; eluting with 20% MeOH/EtOH (1:1) in heptane (isocratic) at 50 mL/min) to give the faster eluting compound **254i** (Chiral HPLC: 10.02 min; CHIRALPAK AD 4.6 x 250 mm column; eluting with 20% MeOH/EtOH (1:1) in heptane at 1 mL/min) and the slower eluting **254ii** (Chiral HPLC: 14.74 min; CHIRALPAK AD 4.6 x 250 mm column; eluting with 20% MeOH/EtOH (1:1) in heptane at 1 mL/min).

**Example 255**

**(3aα,4β,7β,7aα)-2-(4-Cyano-1-naphthalenyl)octahydro-1,3-dioxo-7-[2-(phenylmethoxy)ethyl]-4,7-epoxy-4H-isoindole-4-propanenitrile & (3aα,4α,7α,7aα)-2-(4-Cyano-1-naphthalenyl)octahydro-1,3-dioxo-7-[2-(phenylmethoxy)ethyl]-4,7-epoxy-4H-isoindole-4-propanenitrile (255Hi & 255Hii)**

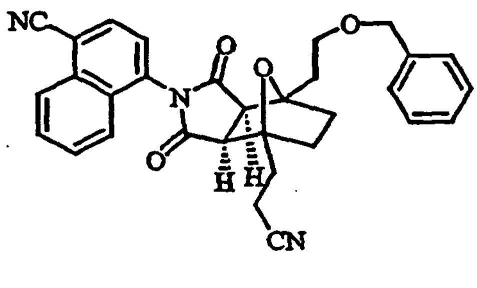 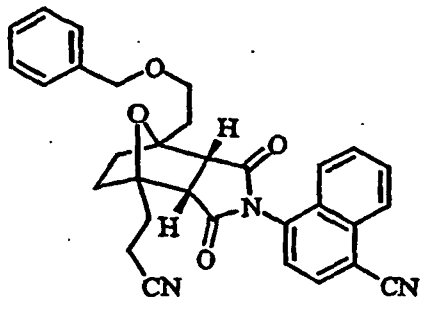

**A. 2-(2-Hydroxyethyl)furan (255A)**

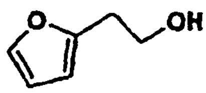

2-(2-Hydroxyethyl)furan was made in accordance with the following reference: Harmata, M., *et al., J. Org. Chem.* 60, 5077-5092 (1995). *n*-BuLi (2.5 M in hexane, 44 mL, 110 mmol) was added to a solution of furan (8 mL, 110 mmol) in 100 mL of THF at -78°C. The solution was stirred at 0°C for 4 h and then ethylene oxide (7.5 mL) was added at -78°C. The reaction mixture was stirred at -15°C for 1 h and then overnight at rt. The reaction mixture was quenched with sat. $NH_4Cl$ and extracted with ether (3x). The combined extracts were washed with water (1x) and brine (1x). The ether solution was dried over $Na_2SO_4$ and concentrated under reduced pressure. Purification by flash chromatography on silica gel eluting with 40% EtOAc/60% hexane gave 5.4 g (48.2 mmol, 43.8%) of compound 255A as a light brown oil.

**B. 2-[2-[[(1,1-Dimethylethyl)dimethylsilyl]oxy]ethyl]furan (255B)**

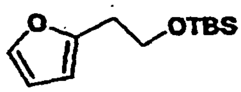

Imidazole (3.65 g, 53.6 mmol) and TBSCl (6.47 g, 42.9 mmol) were added to the solution of compound **255A** (4.00 g, 35.7 mmol) in 50 mL of DMF. The mixture was stirred at rt for 2 h and then the reaction mixture was poured into ether. The ether solution was washed with water (1x), 1 N HCl (1x), water (1x) and brine (1x). The organic layer was dried over $Na_2SO_4$ and concentrated under reduced pressure. Purification by flash chromatography on silica gel eluting with 30% $CH_2Cl_2$/70% hexane gave 7.4 g (32.7 mmol, 91.7%) of 255B as a colorless oil.

**C. 2-[2-[[(1,1-Dimethylethyl)dimethylsilyl]oxy]ethyl]-5-(2-hydroxyethyl)furan (255C)**

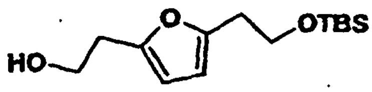

*t*-BuLi (1.2 M in pentane, 10 mL, 16.99 mmol) was added to a stirred solution of 255B (3.49 g, 15.44 mmol) in 13 mL of THF at -78°C dropwise. The mixture was stirred for an additional 4 h at 0°C. Ethylene oxide (1.05 mL) was added at -78°C to the reaction solution. The mixture was warmed to rt and stirred overnight. Aqueous sat. $NH_4Cl$ was added and most of the THF was removed under reduced pressure. The mixture was extracted with ether (3x) and the combined organic layers were washed with water (1x) and brine (1x) and dried over $Na_2SO_4$. Purification by flash chromatography on silica gel eluting with 5% EtOAc/95% $CH_2Cl_2$ gave 2.8 g (10.4 mmol, 67%) of compound 255C as a yellow oil.

**D. 2-[2-[[(1,1-Dimethlethyl)dimethylsilyl]oxy]ethyl]-5-[2-(phenylmethoxy)ethyl]furan (255D)**

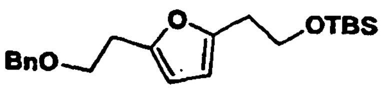

The alcohol **255C** (1.00 g, 3.7 mmol) in 12 mL of THF was treated with 60% NaH (177.8 mg, 4.44 mmol), benzyl bromide (0.53 mL, 4.44 nimol) and tetrabutylammonium iodide (50 mg, 5%) for 3 h at rt. Water was added and the mixture was extracted with EtOAc (3x). The combined extracts were washed with water (1x) and brine (1x) and dried over $Na_2SO_4$. Purification by flash chromatography on silica gel eluting with 20% hexane/80% $CH_2Cl_2$ gave 1.10 g (3.05

mmol, 82.6%) of compound 255D as a yellow oil.

**E. 2-(2-Hydroxyethyl)-5-[2-(phenylmethoxy)ethyl]furan (255E)**

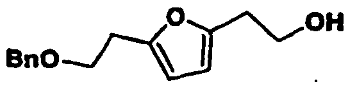

Tetrabutylammonium fluoride (1.0M in THF, 3.06 mL, 3.06 mmol) was added to the solution of compound **255D** (1.1 g, 3.06 mmol) in 10 mL of THF at 0°C. The reaction mixture was stirred at rt for 10 minutes, quenched by sat. $NH_4Cl$ and extracted with ether (3x). The combined extracts were dried over $Na_2SO_4$. Purification by flash chromatography on silica gel eluting with 10% EtOAc/90% $CH_2Cl_2$ gave 750 mg (3.05 mmol, 99.6%) of compound 255E as a light yellow oil.

**F. 5-[2-(Phenylmethoxy)ethyl]furan-2-propanenitrile (255F)**

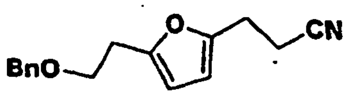

DEAD (1.285 mL, 8.17 mmol) was added to a stirred solution of $Ph_3P$ (2.14 g, 8.17 mmol) in 12 mL of dry THF at 0°C. The solution was stirred for 30 min at rt and compound **255E** (670 mg, 2.72 mmol) was added. The reaction mixture was stirred for 15 min and acetone cyanohydrin (0.745 mL, 8.17 mmol) was added at -15°C. The reaction mixture was stirred for 30 min at -15°C, then at rt overnight. The mixture was then concentrated under reduced pressure. Purification by flash chromatography on silica gel eluting with 100% $CH_2Cl_2$ gave 180 mg (0.705 mmol, 26%) of compound **255F** as a colorless oil.

**G. (3aα,4β,7β,7aα)-2-(4-Cyano-1-naphthalenyl)-1,2,3,3a,7,7a-hegahydro-1,3-dioxo-7-[2-(phenylmethoxy)ethyl]-4,7-epoxy-4H-isoindole-4-propanenitrile (255G)**

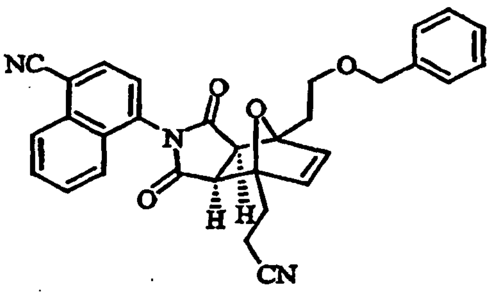

A solution of compound **255F** (180 mg, 0.706 mmol) and 4-(2,5-dihydro-2,5-dioxo-1H-1-yl)-1-naphthalenecarbonitrile (263 mg, 1.06 mmol) in $CH_2Cl_2$ (3 mL) was stirred at rt for 3 days. The reaction mixture was concentrated under reduced pressure. Purification by flash chromatography on silica gel eluting with 5% EtOAc/$CH_2Cl_2$ gave 318 mg (0.63 mmol, 89.6%) of compound 255G as a light gray solid which was used directly in the next step.

**H. (3aα,4β,7β,7aα)-2-(4-Cyano-1-naphthalenyl)octahydro-1,3-dioxo 7-[2-(phenylmethoxy)ethyl]-4,7-epoxy-4H-isoindole-4-propanenitrile & (3aα,4α,7α,7aα)-2-(4-Cyano-1-naphthalenyl)octahydro-1,3-dioxo-7-[2-(phenylmethoxy)ethyl]-4,7-epoxy-4H-isoindole-4-propanenitrite (255Hi & 255Hii)**

A mixture of compound **255G** (318 mg, 0.63 mmol) and 10% Pd/C (64 mg) in EtOH (10 mL) and EtOAc (5 mL)

was stirred under a hydrogen atmosphere at rt overnight. The reaction mixture was filtered through celite and concentrated under reduced pressure to give 320 mg of crude compounds **255Hi & 255Hii**. Purification of 25 mg of this crude product by flash chromatography on silica gel eluting with 55% EtOAc/hexane gave 6.5 mg (0.013 mmol, 26% (based on 25 mg)) of compound **255Hi** & 8.1 mg (0.016 mmol, 32.4% (based on 25 mg)) of compound **255Hii**. Compound **255Hi:** HPLC conditions: 98% at 3.57 min (YMC S5 ODS 4.6 x 50 mm, 10%-90% aqueous methanol over 4 minute gradient with 0.2% $H_3PO_4$, detecting at 220 nm, MS (ES): m/z 506.15 $[M+H]^+$. Compound **255Hii:** HPLC conditions: 98% at 3.51 min (YMC S5 ODS 4.6 x 50 mm, 10%-90% aqueous methanol over 4 minute gradient with 0.2% $H_3PO_4$, detecting at 220 nm), MS (ES): m/z 506.15 $[M+H]^+$.

**Example 256**

**(3aα,4β,7β,7aα)-2-(4-Cyano-1-naphthalenyl)octahydro-7-(2-hydroxyethyl)-1,3-dioxo-4,7-epoxy-4H-isoindole-4-propanenltrile & (3aα,4α,7α,7aα)-2-(4-Cyano-1-naphthalenyl)octahydro-7-(2-hydroxyethyl)-1,3-dioxo-4,7-epoxy-4H-isoindole-4-propanenitrile (256i & 256ii)**

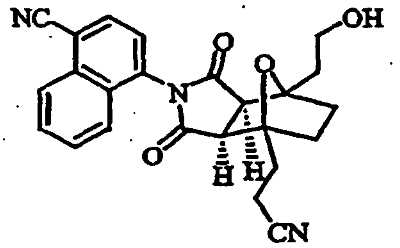

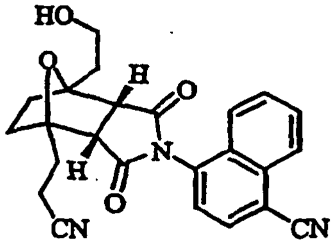

A mixture of compounds **255Hi & 255Hii** (200 mg, 0.396 mmol) and $PdCl_2$ (8.4 mg, cat.) in EtOH (1 mL) and EtOAc (3 mL) was stirred under a hydrogen atmosphere (30 psi) at rt overnight. The reaction mixture was filtered through celite and concentrated under reduced pressure. Purification by flash chromatography on silica gel eluting with 5% $MeOH/CH_2Cl_2$ followed by a second column eluting with 100% EtOAc gave 28.9 mg (0.0696 mmol, 17.6%) of compound **256ii** and 26.5 mg (0.0639 mmol, 16.1%) of compound **256i.** Compound **256ii:** HPLC conditions: 90% at 2.44 min (YMC S5 ODS 4.6 x 50 mm, 10%-90% aqueous methanol over 4 minute gradient with 0.2% $H_3PO_4$, detecting at 220 nm.), MS (ES): m/z 416.11 $[M+H]^+$. Compound **256i:** HPLC conditions: 99% at 2.47 min (YMC S5 ODS 4.6 x 50 mm, 10%-90% aqueous methanol over 4 minute gradient with 0.2% $H_3PO_4$, detecting at 220 nm), MS (ES): m/z 416.11 $[M+H]^+$.

**Example 257**

**(3aα,4β,7β,7aα)-2-(4-Cyano-1-naphthalenyl)-7-[2-(4-fluorophenoxy)ethyl]octahydro-1,3-dioxo-4,7-epoxy-4H-isoindole-4-propanenitrile (257)**

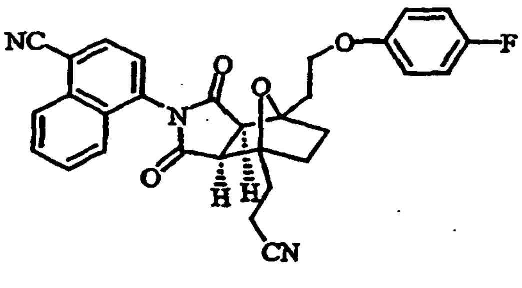

DBAD (15 mg, 0.065 mmol) was added to a solution of $PPh_3$ (17 mg, 0.065 mmol) In THF (0.3 mL). After stirring for 10 min, 4-fluorophenol (7.33 mg, 0.065 mmol) was added and the reaction mixture was stirred for a further 5 min. Compound **256i** (18.1 mg, 0.044 mmol) was added and the mixture was stirred at rt for 3 h. The reaction mixture was concentrated under reduced pressure. Purification by flash chromatography on silica gel eluting with 60% EtOAc/30% hexane gave 5.9 mg (0.0116 mmol, 26.34%) of compound **257**. HPLC conditions: 98% at 3.59 min (YMC S5 ODS 4.6

x 50 mm, 10%-90% aqueous methanol over 4 minute gradient with 0.2% $H_3PO_4$, detecting at 220 nm). MS (ES): m/z 510.14 $[M+H]^+$.

**Example 258**

**(3aα,4β,7β,7aα)-2-(7-Chloro-2,1,3-benzoxadiazol-4-yl)hexahydro-4,7-dimethyl-4,7-epoxy-1H-isoindoie-1,3(2H) -dione (258)**

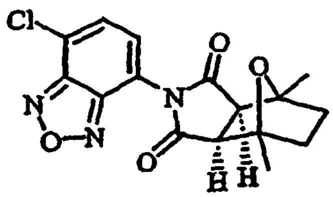

**A. 4-Amino-7-chloro-2,1,3-benzoxadiazole (258A)**

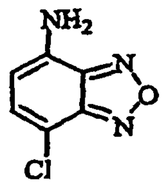

A solution of 1.0 g (5.02 mmol) of 4-chloro-7-nitrobenzofurazan in 20 mL AcOH, 10 mL EtOAc and 2 mL $H_2O$ was heated to 50°C and treated with iron powder (1.4 g, 251 mmol). The mixture was heated at 80°C for 30 min and then allowed to cool to rt. The mixture was filtered through celite eluting with EtOAc. The filtrate was washed with sat. aq $NaHCO_3$, dried over $MgSO_4$, and concentrated to give compound **258A** (0.80 g, 94%) as a red solid.

**B. (3aα,4β,7β,7aα)-2-(7-Chloro-2,1,3-benzoxadiazol-4-yl)hexahydro-4,7-dimethyl-4,7-epoxy-1H-isoindole-1,3 (2H)-dione (258B)**

Compound **258A** (42 mg, 0.25 mmol) was reacted In a sealed tube with compound **20A** (73.5 mg, 0.375 mmol), $MgSO_4$ (75 mg, 0.625 mmol) and $Et_3N$ (170 μL, 1.25 mmol) in 250 μL toluene according to the above procedure described in example **208C** to give after purification by preparative reverse phase HPLC (YMC S5 ODS 20 x 100 mm eluting with 30-100% aqueous methanol containing 0.1% TFA over 12 min, 20 mL/min) 23 mg (26%) of compound **258B** as a yellow solid. HPLC: 97.6% at 2.87 min (retention time) (YMC S5 ODS column 4.6 x 50 mm eluting with 10-90% aqueous methanol containing 0.2% phosphoric acid over 4 minutes , 4 mL/min, monitoring at 220 nm), MS (DCI): m/z 347.9 $[M]^+$.

**Example 259**

**(3aα,4β,7β,7aα)-2-(7-Chloro-2-methyl-4-benzofuranyl)hexahydro-4,7-dimethyl-4,7-epoxy-1H-isoindole-1,3(2H) -dione (259)**

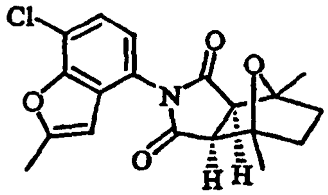

7-Chloro-2-methyl-4-benzofuranamine (38 mg, 0.25 mmol, prepared in accordance with the procedure described by Enomoto and Takemura in EP 0476697 A1) was reacted in a sealed tube with compound **20A** (73.5 mg, 0.375 mmol), $MgSO_4$ (75 mg, 0.625 mmol) and $Et_3N$ (170 µL, 1.25 mmol) in 250 µL toluene according to the procedure described in example 208C to give, after purification by preparative reverse phase HPLC (YMC S5 ODS 20 x 100 mm eluting with 30-100 % aqueous methanol containing 0.1% TFA over 12 min, 20 mL/min), 42 mg (47%) of compound 259 as a white solid. HPLC: 98% at 3.45 min (retention time) (YMC S5 ODS column 4.6 x 50 mm eluting with 10-90% aqueous methanol over 4 minutes containing 0.2% phosphoric acid, 4 mL/min, monitoring at 220 nm), MS (DCI): m/z 359.9 $[M]^{+\cdot}$.

__**Example 260**__

__**(3aα,4β,7β,7aα)-2-(7-Chloro-2-methylbenzo[b]thiophen-4-yl)hexahydro-4,7-dimethyl-4,7-epoxy-1H-isoindole-1,3(2H)-dione (260)**__

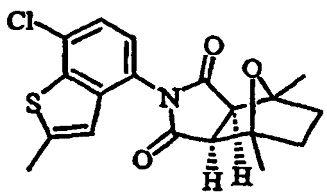

**A. 1-Chloro-2-(2-chloro-allylsulfanyl)-4-nitro-benzene (260A)**

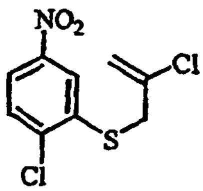

A solution of 2-chloro-5-nitro-benzenethiol (1.0 g, 5.27 mmol, prepared in accordance with the procedure described by Still *et al., Synth. Comm.* **13,** 1181 (1983)) in 15 mL DMF was treated with 2,3-dichtoropropene (693 µL, 7.52 mmol) and $K_2CO_3$ (433 mg, 3.13 mmol). The mixture was heated at 80°C for 2 h and then allowed to cool to rt. EtOAc (200 mL) and $H_2O$ (100 mL) were added. The organic phase was washed with $H_2O$ (2 x 250 mL), saturated aqueous NaCl (100 mL), dried over $MgSO_4$, and concentrated. The crude material was purified by flash column chromatography on silica gel eluting with 20% EtOAc in hexanes to give compound **260A** (1.09 g, 89%) as an orange oil.

**B. 4-Amino-7-chloro-2-methylbenzo[b]thiophene (260B)**

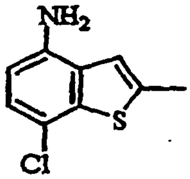

A solution of 1.09 g (4.67 mmol) of compound **260A** in 20 mL AcOH with 10 mL EtOAc and 2 mL $H_2O$ was heated to 80°C and treated with iron powder (1.3 g, 23.4 mmol). The mixture was heated at 80°C for 40 min and then allowed to cool to rt. The mixture was filtered through celite eluting with EtOAc. The filtrate was washed with sat. aq $NaHCO_3$, dried over $MgSO_4$, and concentrated *in vacuo*. N,N-diethylaniline (10 mL) was added, and the reaction mixture was heated at 215°C for 6 h. After cooling to rt, 1N aqueous HCl (20 mL) was added, and the reaction mixture was stirred at room temperature for 2h. The mixture was extracted with EtOAc (3 x 30 mL). The organic phase was washed with saturated aqueous $NaHCO_3$, dried over $MgSO_4$, and concentrated. The crude material was purified by flash column chromatography on silica gel eluting with 25% EtOAc in hexanes to give compound **260B** (320 mg, 35%) as a beige solid.

**C. (3aα,4β,7β,7aα)-2-(7-Chloro-2-methylbenzo[b]thiophen-4-yl)hexahydro-4,7-dimethyl-4,7-epoxy-1H-isoindole-1,3(2H)-dione (260C)**

Compound **260B** (49 mg, 0.25 mmol) was reacted in a sealed tube with compound **20A** (73.5 mg, 0.38 mmol), $MgSO_4$ (75 mg, 0.63 mmol) and $Et_3N$ (170 μL, 1.25 mmol) in 250 μL toluene according to the procedure described in example **208C** to give, after purification by preparative reverse phase HPLC (YMC S5 ODS 20 x 100 mm eluting with 30-100% aqueous methanol over 12 min containing 0.1% TFA, 20 mL/min). 28 mg (30%) of compound **260C** as a pate yellow solid. HPLC: 96% at 3.18 min (retention time) (YMC S5 ODS column 4.6 x 50 mm eluting with 10-90% aqueous methanol over 4 minutes containing 0.2% phosphoric acid, 4 mL/min, monitoring at 220 nm), MS (DCI): m/z 376.0 $[M]^{+\cdot}$.

## Example 261

**[3aα,4β(E),7β,7aα]-4-[2-(4-Cyano-1-naphthalenyl)octahydro-7-methyl-1,3-dioxo-4,7-epoxy-4H-isoindol-4-yl]-2-butenoic acid, phenylmethyl ester (261)**

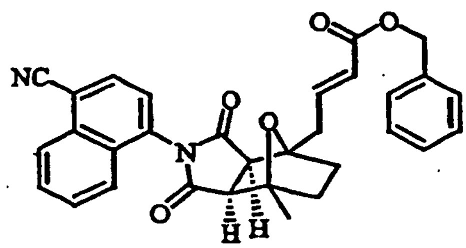

Compound **247** (0.500 g, 0.134 mmol) was dissolved in THF (20 mL) and benzyl(triphenylphosphoranylidene) (0.55 g, 0.134 mmol) was added. The reaction mixture was stirred at 67°C for 2 h and then concentrated under reduced pressure. Purification by flash chromatography on $SiO_2$ eluting with 5% acetone/95% $CHCl_3$ gave 0.65 g of compound **261** as a yellow solid. HPLC: 99% at 3.717 min (retention time) (YMC S5 ODS column 4.6 x 50 mm eluting with 10-90% aqueous methanol over 4 minutes containing 0.2% phosphoric acid, 4 mUmin, monitoring at 220 nm), MS (ES): m/z 507.1 $[M+H]^+$.

## Example 262

**(3aα,4β,7β,7aα)-2-(4-Cyano-1-naphthalenyl)octahydro-7-methyl-1,3-dioxo-4,7-epoxy-4H-isoindole-4-butanoic acid (262)**

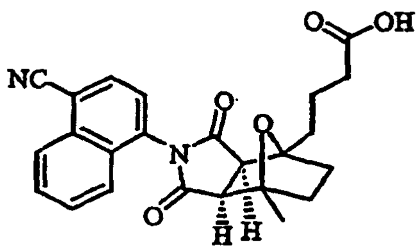

Compound **261** (0.60 g, 1.19 mmol) was dissolved in EtOH/EtOAc (5mU5mL) and 10% Pd/C (0.30 g) was added. Hydrogen was then introduced via a balloon. After 8 h the reaction mixture was filtered through celite and then concentrated under reduced pressure to give compound **262** (0.47 g) as a white solid. HPLC: 98% at 2.81 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 419.1 $[M+H]^+$.

## Example 263

**(3aα,4β,7β,7aα)-2-(4-Cyano-1-naphthalenyl)-N-(4-fluorophenyl)octahydro-7-methyl-1,3-dioxo-4,7-epoxy-4H-isoindole-4-butanamide (263)**

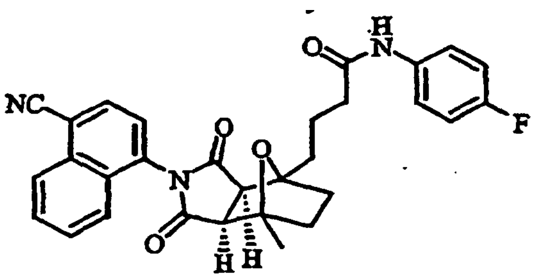

Compound **262** (0.030 g, 0.072 mmol) was dissolved in $CH_3CN$ (1 mL). DCC (0.014 g, 0.072 mmol) and HOAc (0.0098 g, 0.072 mmol) were then added, followed by 4-fluoroaniline (0.007 mL, 0.072 mmol). The reaction mixture was stirred under argon for 14 h and the crude material was dissolved in MeOH, purified by preparative HPLC (YMC VP-ODS column, 20 x 100 mm, eluting with 20% B to 100% B in 15 minutes and hold @ 100%B for 10 minutes). Compound **263** (0.020 g) was isolated as white solid. HPLC: 100% at 3.217 min (retention time) (YMC S5 ODS column 4.6 x 50 mm eluting with 10-90% aqueous methanol over 4 minutes containing 0.2% phosphoric acid, 4 mUmin, monitoring at 220 nm), MS (ES): m/z 512.1 $[M+H]^+$.

## Example 264

**[3aS-(3aα,4β,5β,7β,7aα)]-4-[7-[2-(Acetyloxy)ethyl]octahydro-5-hydroxy-4-methyl-1,3-dioxo-4,7-epoxy-2H-isoindol-2-yl]-1-naphthalenecarbonitrile & [3aR-(3aα,4β,5β,7β,7aα)]-4-[Octahydro-5-hydroxy-7-(2-hydroxyethyl)-4-methyl-1,3-dioxo-4,7-epoxy-2H-isoindol-2-yl]-1-naphthalenecarbonitrile (264 & 243Dii)**

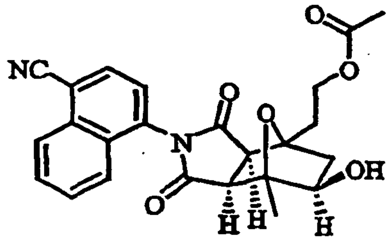 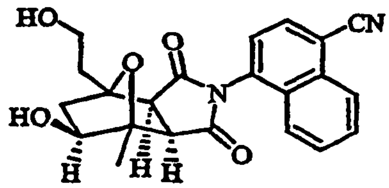

A racemic mixture of compounds **243Di & 243Dii** (1.90 gram) was dissolved in 100 mL of anhydrous THF in a 2 L flask. Anhydrous *tert*-butylmethyl ether (900 mL) and vinyl acetate (40 mL) were transferred into the flask with stirring and lipase (20 g, typell, crude, from porcine pancreas; Sigma, Cat# L3126) was added. The reaction mixture was stirred for 21 hr at rt at which point an additional 5 grams of the lipase and 20 mL of vinyl acetate were added. The reaction mixture was stirred at rt for an additional 19 h, stored at 4°C without stirring for 36 h and then stirred at rt for another 22 h (until the desired % ee was apparent by chiral HPLC). To monitor the reaction, 200 μL of the mixture were withdrawn and centrifuged. The supernatant (100 μL) was dried under nitrogen and the resulting residue was dissolved in 100 μL of EtOH and subjected to HPLC analysis:

1) Reverse phase HPLC: Column, YMC-ODS AQ 150 x 4.6; flow rate, 1.2 mL/min; sample size, 10 μL
solvent A, 1 mM HCl in water; solvent B, MeCN; monitored at 300 nm
Gradient:

| Time(min) | 0 | 8 | 8.5 | 9.5 | 10 | 12 |
|---|---|---|---|---|---|---|
| B% | 30 | 60 | 85 | 85 | 30 | 30 |

2) Chiral-HPLC: Column, CHIRALCEL OJ 4.6 x 250 mm
mobile phase, Hexane/MeOH/EtOH (8:1:1)
flow rate, 1 mL/min; sample size, 20 μL
monitored at both 220 and 300 nm
performed at 25°C & 40°C.
(for ee% determination of reaction mixture)

The enzyme was removed by filtration and the filtrate was concentrated under vacuum. The resulting mixture was dissolved in $CHCl_3$ and adsorbed onto silica gel (63-200 microns). These solids were applied to a VLC funnel (3 cm I.D., VLC is vacuum liquid chromatography using glass funnels having 24/40 joints at the bottom) containing a 5 cm bed height of silica gel (25-40 microns) and a step gradient was carried out The gradient was 100% $CHCl_3$ in the first 3 fractions, followed by $CHCl_3$-1% MeOH (3 fractions), $CHCl_3$-2% MeOH (3 fractions), $CHCl_3$-3% MeOH (3 fractions), $CHCl_3$-4% MeOH (3 fractions), and finally with $CHCl_3$-5% MeOH (3 fractions). The volume of the fractions was 100 mL until reaching $CHCl_3$-3% MeOH and from that point on it was 200 mL. Compound **264** elutes in the last two fractions of 100% $CHCl_3$ and until the first fraction of $CHCl_3$-2% MeOH. Compound **243Dii** elutes starting with the second fraction of $CHCl_3$-2% MeOH, and continues to the first fraction of $CHCl_3$-5% MeOH. The crude compound **243Dii** contained a small amount of a colored impurity which was removed by a Sephadex column [LH-20 swollen in $CHCl_3$-MeOH (2:1), column (2.5 cm I.D. & 90 cm long)] to yield 632 mg of compound **243Dii.** Compound **264:** HPLC conditions: 98% at 7.2 min (method 1), chiral HPLC conditions: 29.0 min @ 25°C (method 2). Compound **243Dii:** HPLC conditions: 98% at 4.6 min (method 1), chiral HPLC conditions: 96% ee at 25.7 min (@ 25°C) & 19.8 min (@ 40°C) (method 2).

**<u>Example 265</u>**

**<u>([3aα,4β,7β,7aα(E)]-4-[Octahydro-4-methyl-1,3-dioxo-7-(4-oxo-4-phenyl-2-butenyl)-4,7-epoxy-2H-isoindol-2-yl]-1-naphthalenecarbonitrile (265)</u>**

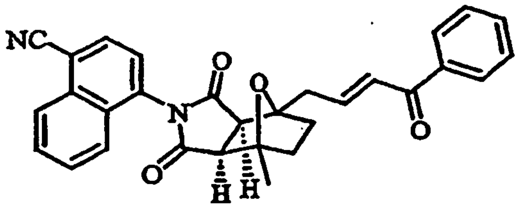

The compound **247** (0.050 g, 0.134 mmol) was dissolved in THF (1.5 mL) and (phenacylidene)triphenylphosphorane (0.051 g, 0.134 mmol) was added. The reaction mixture was stirred at 67°C for 24 h and then cooled to 23°C and concentrated *in vacuo*. The crude material was then purified by preparative HPLC. (YMC VP-ODS column, 20 x 100 mm, eluting with 20% B to 100%B in 15 minutes and hold @ 100%B for 10 minutes.) to give compound 265 (0.040 g) as white solid. HPLC: 100% at 3.503 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 477.1 $[M+H]^+$.

**Example 266**

**[3aα,4β,7β,7aα(E)]-4-[Octahydro-4-methyl-1,3-dioxo-7-(4-oxo-4-phenyl-butanyl)-4,7-epoxy-2H-isoindol-2-yl]-1-naphthalenecarbonitrile (266)**

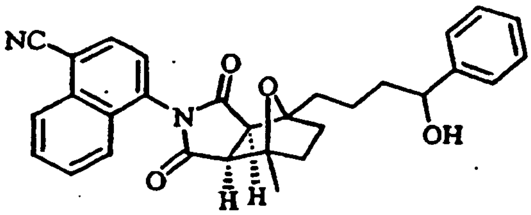

Compound **265** (0.010 g, 0.021 mmol) was dissolved in EtOH (2.0 mL) and Pd/C (10% Pd, 0.005 g) was added. Hydrogen was then introduced via a balloon and the reaction mixture was stirred at 25°C for 3 h. The reaction mixture was then filtered through celite rinsing with EtOAc and concentrated *in vacuo* to give compound **266** as a tan solid (0.009 g). No purification was necessary. HPLC: 100% at 3.38 min (retention time) (YMC S5 ODS column 4.6 x 50 mm eluting with 10-90% aqueous methanol over 4 minutes containing 0.2% phosphoric acid; 4 mL/min, monitoring at 220 nm), MS (ES): m/z 503.2 $[M+Na]^+$.

**Examples 267 to 378**

Additional compounds of the present invention were prepared by procedures analogous to those described above. The compounds of Examples 267 to 378 have the following structure (L is a bond):

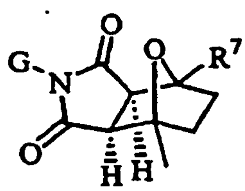

where G, $R^7$, the compound name, retention time, molecular mass, and the procedure employed, are set forth in **Table 5.** The absolute configuration for the following compounds was not determined. For simplicity in nomenclature, compound **238i** is designated herein as having an "R" configuration and compound **238ii** as having an "S" configuration. Enantiomerically pure products derived from compound **238i** are designated herein as having an "R" configuration and enantiomerically pure products derived from compound **238ii** are designated herein as having an "S" configuration.

The chromatography techniques used to determine the compound retention times of Table 5 are as follows: LCMS = YMC S5 ODS column, 4.6 x 50 mm eluting with 10-90% MeOH/$H_2O$ over 4 minutes containing 0.1 % TFA; 4 mL/min, monitoring at 220 nm. LCMS* = YMC S5 ODS column, 4.6 x 50 mm eluting with 10-90% MeOH/$H_2O$ over 2 minutes containing 0.1% TFA; 4 mL/min, monitoring at 220 nm. LC = YMC S5 ODS column 4.6 x 50 mm eluting with 10-90% MeOH/$H_2O$ over 4 minutes containing 0.2% phosphoric acid, 4 mL/min, monitoring at 220 nm. The molecular mass of the compounds listed in **Table 5** was determined by MS (ES) by the formula m/z.

## Table 5.

| Ex. No | G | $R^7$ | Compound Name | Retention Time Min./ Molecular Mass | Procedure of Ex. |
|---|---|---|---|---|---|
| 267 | 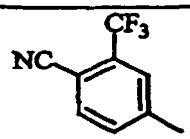 | 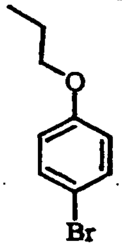 | (3aα,4β,7β,7aα)-(4-[7-[2-(4-Bromophenoxy)ethyl]octahydro-4-methyl-1,3-dioxo-4,7-epoxy-2H-isoindol-2-yl]-2-(trifluoromethyl)benzonitrile. | 3.97 LCMS 549.0 $[M+H]^+$ | 204, 35 |

| Ex. No | G | R7 | Compound Name | Retention Time Min./ Molecular Mass | Procedure of Ex. |
|---|---|---|---|---|---|
| 268 | 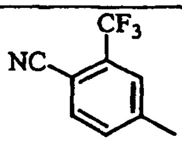 | 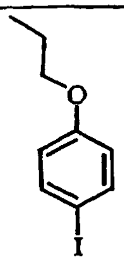 | (3aα,4β,7β,7aα)-4-[Octahydro-7-[2-(4-iodophenoxy)ethyl]-4-methyl-1,3-dioxo-4,7-epoxy-2H-isoindol-2-yl]-2-(trifluoromethyl)benzonitrile. | 4.09 LCMS 597.0 $[M+H]^+$ | 204, 35 |
| 269 | 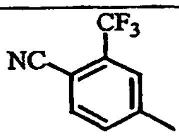 | 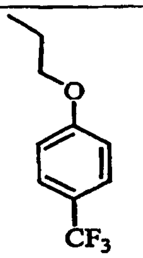 | (3aα,4β,7β,7aα)-4-[Octahydro-4-methyl-1,3-dioxo-7-[2-[4-(trifluoromethyl)phenoxy]ethyl]-4,7-epoxy-2H-isoindol-2-yl]-2-(trifluoromethyl)benzonitrile. | 3.95 LC | 204, 35 |
| 270 | 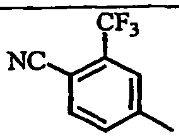 | 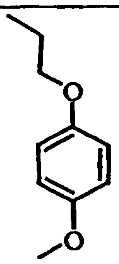 | (3aα,4β,7β,7aα)-4-[Octahydro-7-[2-(4-methoxyphenoxy)ethyl]-4-methyl-1,3-dioxo-4,7-epoxy-2H- | 3.66 LC | 204, 35 |

| Ex. No | G | $R^7$ | Compound Name | Retention Time Min./ Molecular Mass | Procedure of Ex. |
|---|---|---|---|---|---|
| | | | isoindol-2-yl]-2-(trifluoromethyl)benzonitrile. | | |
| 271 | 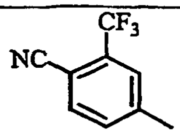 | 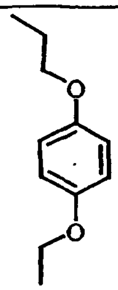 | (3aα,4β,7β,7aα)-4-[7-[2-(4-Ethoxyphenoxy)ethyl]octahydro-4-methyl-1,3-dioxo-4,7-epoxy-2H-isoindol-2-yl]-2-(trifluoromethyl)benzonitrile. | 3.81 LC | 204, 35 |
| 272 | 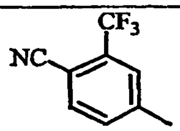 | 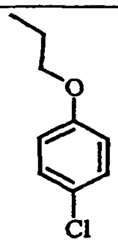 | (3aα,4β,7β,7aα)-4-[7-[2-(4-Chlorophenoxy)ethyl]octahydro-4-methyl-1,3-dioxo-4,7-epoxy-2H-isoindol-2-yl]-2-(trifluoromethyl)benzonitrile. | 3.97 LCMS 522.2 $[M+H]^+$ | 204, 35 |
| 273 | 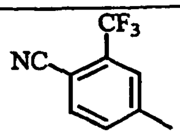 | 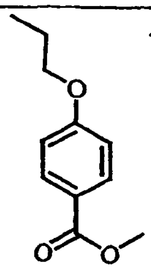 | (3aα,4β,7β,7aα)-4-[2-[2-[4-Cyano-3-(trifluoromethyl)phenyl]octahydro-7-methyl-1,3-dioxo-4,7- | 3.77 LCMS 529.12 $[M+H]^+$ | 204, 35 |

| Ex. No | G | $R^7$ | Compound Name | Retention Time Min./ Molecular Mass | Procedure of Ex. |
|---|---|---|---|---|---|
| | | | epoxy-4H-isoindol-4-yl]ethoxy]benzoic acid, methyl ester. | | |
| 274 | 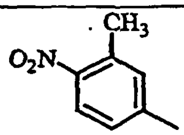 | 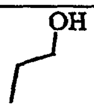 | (3aα,4β,7β,7aα)-Hexahydro-4-(2-hydroxyethyl)-7-methyl-2-(3-methyl-4-nitrophenyl)-4,7-epoxy-1H-isoindole-1,3(2H)-dione. | 2.44 LC | 204, 35 |
| 275 | 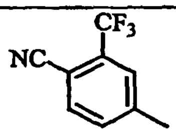 | 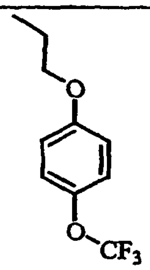 | (3aα,4β,7β,7aα)-4-[Octahydro-4-methyl-1,3-dioxo-7-[2-[4-(trifluoromethoxy)phenoxy]ethyl]-4,7-epoxy-2H-isoindol-2-yl]-2-(trifluoromethyl)benzonitrile. | 3.97 LC | 204, 35 |
| 276 | 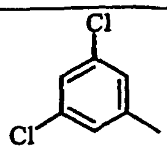 | $CH_3$ | (3aα,4β,7β,7aα)-2-(3,5-Dichlorophenyl)hexahydro-4,7-dimethyl- | 3.31 LCMS 341.2 $[M+H]^+$ | 20 |

| Ex. No | G | $R^7$ | Compound Name | Retention Time Min./ Molecular Mass | Procedure of Ex. |
|---|---|---|---|---|---|
| | | | 4,7-epoxy-1H-isoindole-1,3(2H)-dione. | | |
| 277 | 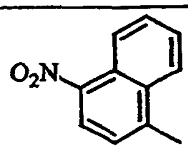 | $CH_3$ | (3aα,4β,7β,7aα)-Hexahydro-4,7-dimethyl-2-(4-nitro-1-naphthalenyl)-4,7-epoxy-1H-isoindole-1,3(2H)-dione. | 3.04 LCMS | 20 |
| 278 | 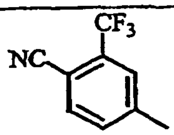 | 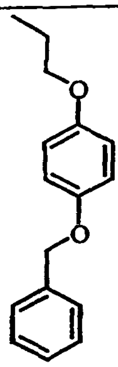 | (3aα,4β,7β,7aα)-4-[Octahydro-4-methyl-1,3-dioxo-7-[2-[4-(phenylmethoxy)phenoxy]ethyl]-4,7-epoxy-2H-isoindol-2-yl]-2-(trifluoromethyl)benzonitrile. | 4.06 LC | 204, 35 |
| 279 | 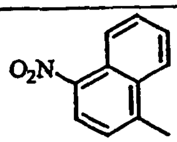 | 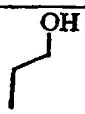 | (3aα,4β,7β,7aα)-Hexahydro-4-(2-hydroxyethyl)-7-methyl-2-(4-nitro-1-naphthalenyl)-4,7-epoxy-1H-isoindole-1,3(2H)-dione. | 2.607 & 2.743 rotational isomers LC | 204, 35 |

| Ex. No | G | R7 | Compound Name | Retention Time Min./ Molecular Mass | Procedure of Ex. |
|---|---|---|---|---|---|
| 280 | 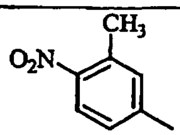 | 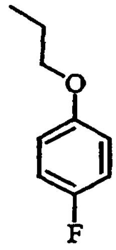 | (3aα,4β,7β,7aα)-4-[2-(4-Fluorophenoxy)ethyl]hexahydro-7-methyl-2-(3-methyl-4-nitrophenyl)-4,7-epoxy-1H-isoindole-1,3(2H)-dione. | 3.68 LC | 204, 35 |
| 281 | 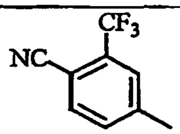 | 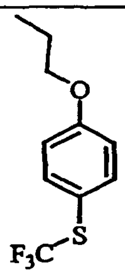 | (3aα,4β,7β,7aα)-4-[Octahydro-4-methyl-1,3-dioxo-7-[2-[4-[(trifluoromethyl)thio]phenoxy]ethyl]-4,7-epoxy-2H-isoindol-2-yl]-2-(trifluoromethyl)benzonitrile. | 4.11 LC | 204, 35 |
| 282 | 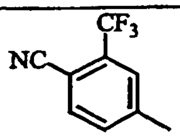 | 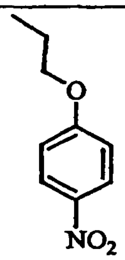 | (3aα,4β,7β,7aα)-4-[Octahydro-4-methyl-7-[2-(4-nitrophenoxy)ethyl]-1,3-dioxo-4,7-epoxy-2H-isoindol-2-yl]-2- | 3.68 LC | 204, 35 |

| Ex. No | G | $R^7$ | Compound Name | Retention Time Min./ Molecular Mass | Procedure of Ex. |
|---|---|---|---|---|---|
| | | | (trifluoromethyl)benzonitrile. | | |
| 283 | 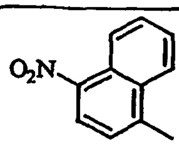 | 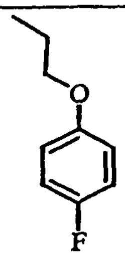 | (3aα,4β,7β,7aα)-4-[2-(4-Fluorophenoxy)ethyl]hexahydro-7-methyl-2-(4-nitro-1-naphthalenyl)-4,7-epoxy-1H-isoindole-1,3(2H)-dione. | 3.68 & 3.80 rotational isomers LC | 204, 35 |
| 284 | 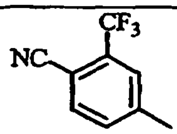 | 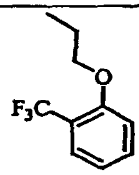 | (3aα,4β,7β,7aα)-4-[Octahydro-7-methyl-1,3-dioxo-7-[2-[2-(trifluoromethyl)phenoxy]ethyl]-4,7-epoxy-2H-isoindol-2-yl]-2-(trifluoromethyl)benzonitrile. | 3.89 LC | 204, 35 |
| 285 | 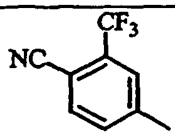 | 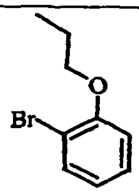 | (3aα,4β,7β,7aα)-4-[4-[2-(2-Bromophenoxy)ethyl]octahydro-7-methyl-1,3-dioxo-4,7-epoxy-2H-isoindol-2-yl]-2- | 3.91 LC | 204, 35 |

| Ex. No | G | $R^7$ | Compound Name | Retention Time Min./ Molecular Mass | Procedure of Ex. |
|---|---|---|---|---|---|
| | | | (trifluoromethyl)benzonitrile. | | |
| 286 | 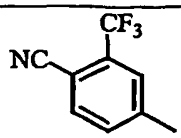 | 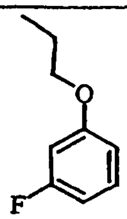 | (3aα,4β,7β,7aα)-4-[4-[2-(3-Fluorophenoxy)ethyl]octahydro-7-methyl-1,3-dioxo-4,7-epoxy-2H-isoindol-2-yl]-2-(trifluoromethyl)benzonitrile. | 3.78 LC | 204, 35 |
| 287 | 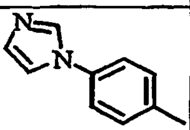 | H | (3aα,4β,7β,7aα)-Hexahydro-2-[4-(1H-imidazol-1-yl)phenyl]-4-methyl-4,7-epoxy-1H-isoindole-1,3(2H)-dione. | 1.16 LC | 3 |
| 288 | 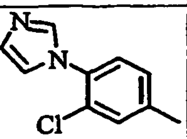 | H | (3aα,4β,7β,7aα)-2-[3-Chloro-4-(2-thiazolyl)phenyl]hexahydro-4-methyl-4,7-epoxy-1H-isoindole-1,3(2H)-dione. | 2.81 LC | 3 |

| Ex. No | G | R7 | Compound Name | Retention Time Min./ Molecular Mass | Procedure of Ex. |
|---|---|---|---|---|---|
| 289 | 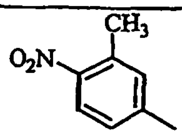 | $CH_3$ | (3aα,4β,7β,7aα)-Hexahydro-4,7-dimethyl-2-(3-methyl-4-nitrophenyl)-4,7-epoxy-1H-isoindole-1,3(2H)-dione. | 2.74 LC | 20 |
| 290 | 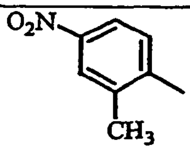 | $CH_3$ | (3aα,4β,7β,7aα)-Hexahydro-4,7-dimethyl-2-(2-methyl-4-nitrophenyl)-4,7-epoxy-1H-isoindole-1,3(2H)-dione. | 2.71 LC | 20 |
| 291 | 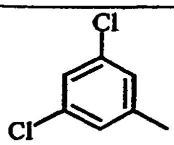 | 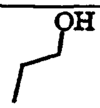 | (3aα,4β,7β,7aα)-2-(3,5-Dichlorophenyl)hexahydro-4-(2-hydroxyethyl)-7-methyl-4,7-epoxy-1H-isoindole-1,3(2H)-dione. | 2.98 LC | 204 |
| 292 | 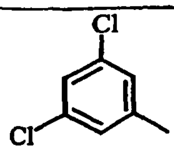 | 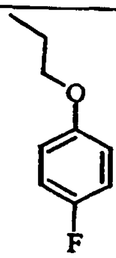 | (3aα,4β,7β,7aα)-2-(3,5-Dichlorophenyl)-4-[2-(4-fluorophenoxy)ethyl]hexahyd | 4.03 LC | 204, 35 |

| Ex. No | G | $R^7$ | Compound Name | Retention Time Min./ Molecular Mass | Procedure of Ex. |
|---|---|---|---|---|---|
| | | | ro-7-methyl-4,7-epoxy-1H-isoindole-1,3(2H)-dione. | | |
| 293 | 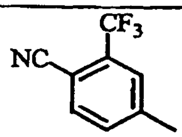 | 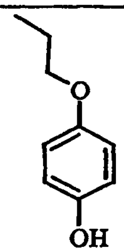 | (3aα,4β,7β,7aα)-4-[Octahydro-4-[2-(4-hydroxyphenoxy)ethyl]-7-methyl-1,3-dioxo-4,7-epoxy-2H-isoindol-2-yl]-2-(trifluoromethyl)benzonitrile. | 3.25 LC | 204, 35 |
| 294 | 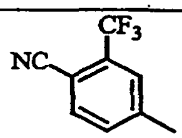 | 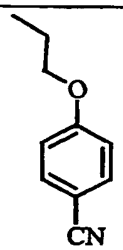 | (3aα,4β,7β,7aα)-4-[4-[2-(4-Cyanophenoxy)ethyl]octahydro-7-methyl-1,3-dioxo-4,7-epoxy-2H-isoindol-2-yl]-2-(trifluoromethyl)benzonitrile. | 3.51 LC | 204, 35 |
| 295 | 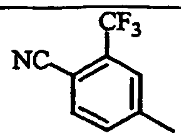 | 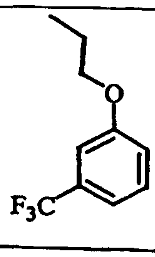 | (3aα,4β,7β,7aα)-4-[Octahydro-4-methyl-1,3-dioxo-7-[2-[3- | 3.85 LC | 204, 35 |

| Ex. No | G | $R^7$ | Compound Name | Retention Time Min./ Molecular Mass | Procedure of Ex. |
|---|---|---|---|---|---|
| | | | (trifluoromethyl)phenoxy]ethyl]-4,7-epoxy-2H-isoindol-2-yl]-2-(trifluoromethyl)benzonitrile. | | |
| 296 | 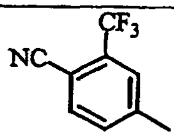 | 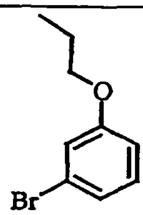 | (3aα,4β,7β,7aα)-4-[4-[2-(3-Bromophenoxy)ethyl]octahydro-7-methyl-1,3-dioxo-4,7-epoxy-2H-isoindol-2-yl]-2-(trifluoromethyl)benzonitrile. | 3.84 LC | 204, 35 |
| 297 | 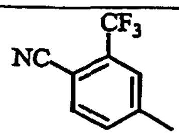 | 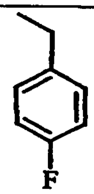 | (3aα,4β,7β,7aα)-4-[4-[(4-Fluorophenyl)methyl]octahydro-7-methyl-1,3-dioxo-4,7-epoxy-2H-isoindol-2-yl]-2-(trifluoromethyl)benzonitrile. | 3.73 LC | 205 |
| 298 | 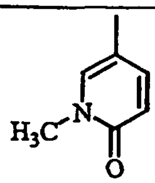 | $CH_3$ | (3aα,4β,7β,7aα)-2-(1,6-Dihydro-1-methyl-6-oxo- | 1.61 LC | 20 |

| Ex. No | G | $R^7$ | Compound Name | Retention Time Min./ Molecular Mass | Procedure of Ex. |
|---|---|---|---|---|---|
| | | | 3-pyridinyl)hexahydro-4,7-dimethyl-4,7-epoxy-1H-isoindole-1,3(2H)-dione. | | |
| 299 | 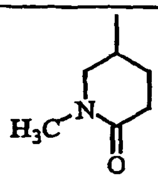 | $CH_3$ | (3aα,4β,7β,7aα)-Hexahydro-4,7-dimethyl-2-(1-methyl-6-oxo-3-piperidinyl)-4,7-epoxy-1H-isoindole-1,3(2H)-dione. | 1.73 LC | 20 |
| 300 | 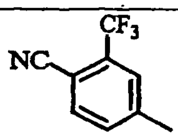 | 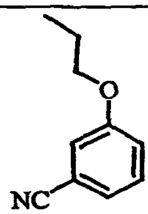 | (3aα,4β,7β,7aα)-4-[4-[2-(3-Cyanophenoxy)ethyl]octahydro-7-methyl-1,3-dioxo-4,7-epoxy-2H-isoindol-2-yl]-2-(trifluoromethyl)benzonitrile. | 3.46 LC | 20 |

| Ex. No | G | R⁷ | Compound Name | Retention Time Min./ Molecular Mass | Procedure of Ex. |
|---|---|---|---|---|---|
| 301 | 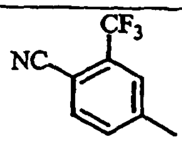 | 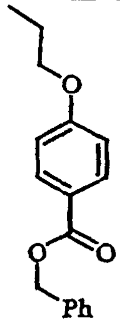 | (3aα,4β,7β,7aα)-4-[2-[4-Cyano-3-(trifluoromethyl)phenyl]octahydro-7-methyl-1,3-dioxo-4,7-epoxy-4H-isoindol-4-yl]ethoxy]benzoic acid, phenylmethyl ester. | 4.01 LC | 204, 35 |
| 302 | 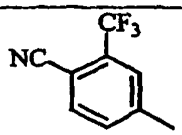 | 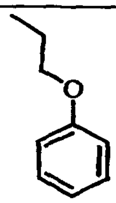 | (3aα,4β,7β,7aα)-4-[Octahydro-4-methyl-1,3-dioxo-7-(2-phenoxyethyl)-4,7-epoxy-2H-isoindol-2-yl]-2-(trifluoromethyl)benzonitrile. | 3.57 LC | 204, 35 |
| 303 | 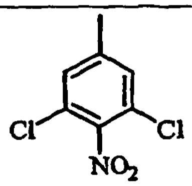 | $CH_3$ | (3aα,4β,7β,7aα)-2-(3,5-Dichloro-4-nitrophenyl)hexahydro-4,7-dimethyl-4,7-epoxy-1H-isoindole-1,3(2H)-dione. | 3.40 LC | 20 |

| Ex. No | G | $R^7$ | Compound Name | Retention Time Min./ Molecular Mass | Procedure of Ex. |
|---|---|---|---|---|---|
| 304 | 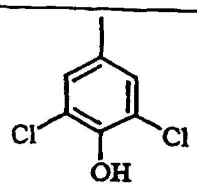 | $CH_3$ | (3aα,4β,7β,7aα)-2-(3,5-Dichloro-4-hydroxyphenyl)hexahydro-4,7-dimethyl-4,7-epoxy-1H-isoindole-1,3(2H)-dione. | 2.58 LC | 20 |
| 305 | 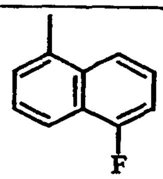 | $CH_3$ | (3aα,4β,7β,7aα)-2-(5-Fluoro-1-naphthalenyl)hexahydro-4,7-dimethyl-4,7-epoxy-1H-isoindole-1,3(2H)-dione. | 2.96 & 3.06 rotational isomers LC | 20 |
| 306 | 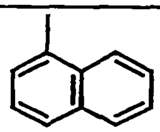 | $CH_3$ | (3aα,4β,7β,7aα)-Hexahydro-4,7-dimethyl-2-(1-naphthalenyl)-4,7-epoxy-1H-isoindole-1,3(2H)-dione. | 2.60 & 2.73 rotational isomers LC | 20 |
| 307 | 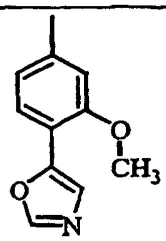 | $CH_3$ | (3aα,4β,7β,7aα)-Hexahydro-2-[3-methoxy-4-(5-oxazolyl)phenyl]-4,7- | 2.62 rotational isomers LC | 20 |

| Ex. No | G | R7 | Compound Name | Retention Time Min./ Molecular Mass | Procedure of Ex. |
|---|---|---|---|---|---|
| | | | dimethyl-4,7-epoxy-1H-isoindole-1,3(2H)-dione. | | |
| 308 | 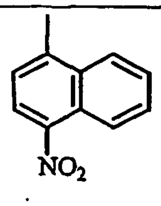 | 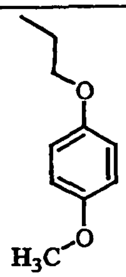 | (3aα,4β,7β,7aα)-Hexahydro-4-[2-(4-methoxyphenoxy)ethyl]-7-methyl-2-(4-nitro-1-naphthalenyl)-4,7-epoxy-1H-isoindole-1,3(2H)-dione. | 3.42 & 3.55 rotational isomers LC | 204, 35 |
| 309 | 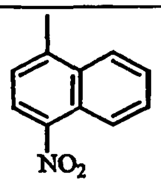 | 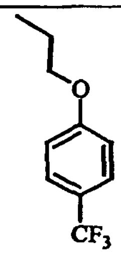 | (3aα,4β,7β,7aα)-Hexahydro-4-methyl-2-(4-nitro-1-naphthalenyl)-7-[2-[4-(trifluoromethyl)phenoxy]ethyl]-4,7-epoxy-1H-isoindole-1,3(2H)-dione. | 3.81 & 3.93 rotational isomers LC | 204, 35 |
| 310 | 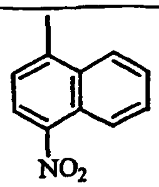 | 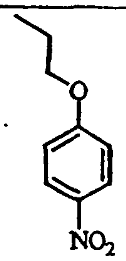 | (3aα,4β,7β,7aα)-Hexahydro-4-methyl-2-(4-nitro-1-naphthalenyl)-7-[2-(4-nitrophenoxy) | 3.48 & 3.61 rotational isomers LC | 204, 35 |

| Ex. No | G | $R^7$ | Compound Name | Retention Time Min./ Molecular Mass | Procedure of Ex. |
|---|---|---|---|---|---|
| | | | ethyl]-4,7-epoxy-1H-isoindole-1,3(2H)-dione. | | |
| 311 | 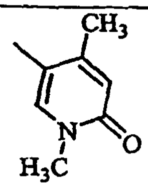 | $CH_3$ | (3aα,4β,7β,7aα)-2-(1,6-Dihydro-1,4-dimethyl-6-oxo-3-pyridinyl)hexahydro-4,7-dimethyl-4,7-epoxy-1H-isoindole-1,3(2H)-dione. | 1.89 LC | 20 |
| 312 | 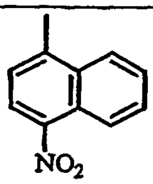 | 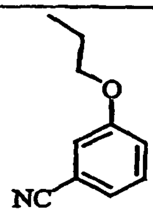 | (3aα,4β,7β,7aα)-4-[Octahydro-7-methyl-2-(4-nitro-1-naphthalenyl)-1,3-dioxo-4,7-epoxy-4H-isoindol-4-yl]ethoxy]benzonitrile. | 3.63 LC | 204, 35 |
| 313 | 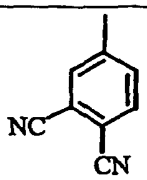 | $CH_3$ | (3aα,4β,7β,7aα)-4-(Octahydro-4,7-dimethyl-1,3-dioxo-4,7-epoxy-2H-isoindol-2-yl)- | 2.38 LC | 20 |

| Ex. No | G | R⁷ | Compound Name | Retention Time Min./ Molecular Mass | Procedure of Ex. |
|---|---|---|---|---|---|
| | | | 1,2-benzenedicarbonitrile. | | |
| 314 | 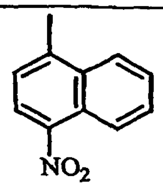 | 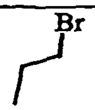 | (3aα,4β,7β,7aα)-4-(2-Bromoethyl)hexahydro-7-methyl-2-(4-nitro-1-naphthalenyl)-4,7-epoxy-1H-isoindole-1,3(2H)-dione. | 3.52 LC | 36 |
| 315 | 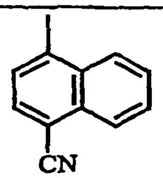 | 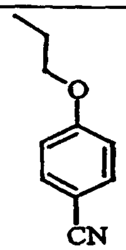 | (3aα,4β,7β,7aα)-4-[4-[2-(4-Cyanophenoxy)ethyl]octahydro-7-methyl-1,3-dioxo-4,7-epoxy-2H-isoindol-2-yl]-1-naphthalenecarbonitrile. | 3.19 & 3.35 rotational isomers LC | 223, 35 |
| 316 | 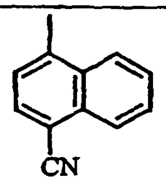 | 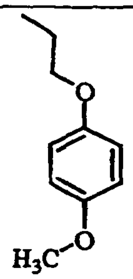 | (3aα,4β,7β,7aα)-4-[Octahydro-4-[2-(4-methoxyphenoxy)ethyl]-7-methyl-1,3-dioxo-4,7-epoxy-2H- | 3.34 & 3.50 rotational isomers LC | 223, 35 |

| Ex. No | G | R7 | Compound Name | Retention Time Min./ Molecular Mass | Procedure of Ex. |
|---|---|---|---|---|---|
| | | | isoindol-2-yl]-1-naphthalenecarbonitrile. | | |
| 317 | 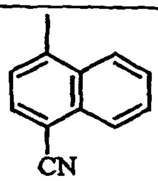 | 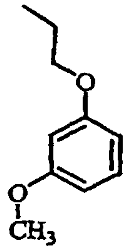 | (3aα,4β,7β,7aα)-4-[Octahydro-4-[2-(3-methoxyphenoxy)ethyl]-7-methyl-1,3-dioxo-4,7-epoxy-2H-isoindol-2-yl]-1-naphthalenecarbonitrile. | 3.34 & 3.50 rotational isomers LC | 223, 35 |
| 318 | 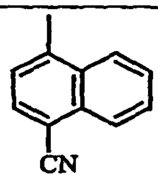 | 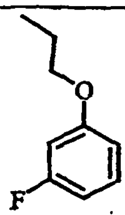 | (3aα,4β,7β,7aα)-4-[4-[2-(3-Fluorophenoxy)ethyl]octahydro-7-methyl-1,3-dioxo-4,7-epoxy-2H-isoindol-2-yl]-1-naphthalenecarbonitrile. | 3.46 & 3.61 rotational isomers LC | 223, 35 |

| Ex. No | G | $R^7$ | Compound Name | Retention Time Min./ Molecular Mass | Procedure of Ex. |
|---|---|---|---|---|---|
| 319 | 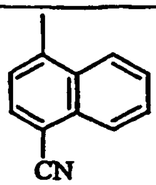 | 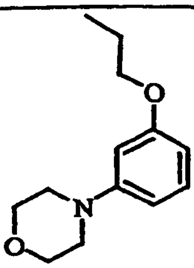 | (3aα,4β,7β,7aα)-4-[Octahydro-4-methyl-7-[2-[3-(4-morpholinyl)phenoxy]ethyl]-1,3-dioxo-4,7-epoxy-2H-isoindol-2-yl]-1-naphthalenecarbonitrile. | 3.01 & 3.18 rotational isomers LC | 223, 35 |
| 320 | 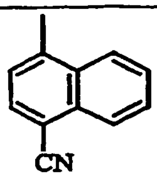 | 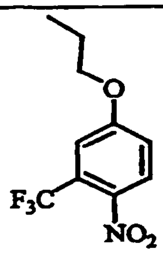 | (3aα,4β,7β,7aα)-4-[Octahydro-4-methyl-7-[2-[4-nitro-3-(trifluoromethyl)phenoxy]ethyl]-1,3-dioxo-4,7-epoxy-2H-isoindol-2-yl]-1-naphthalenecarbonitrile. | 3.70 & 3.83 rotational isomers LC | 223, 35 |
| 321 | 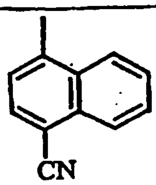 | 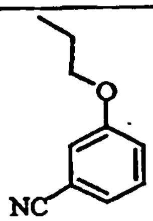 | (3aα,4β,7β,7aα)-4-[4-[2-(3-Cyanophenoxy)ethyl]octahydro-7-methyl-1,3-dioxo-4,7-epoxy-2H- | 3.39 & 3.55 rotational isomers LC | 223, 35 |

| Ex. No | G | $R^7$ | Compound Name | Retention Time Min./ Molecular Mass | Procedure of Ex. |
|---|---|---|---|---|---|
| | | | isoindol-2-yl]-1-naphthalenecarbonitrile. | | |
| 322 | 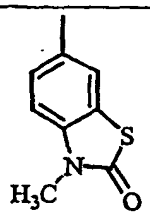 | $CH_3$ | (3aα,4β,7β,7aα)-2-(2,3-Dihydro-3-methyl-2-oxo-6-benzothiazolyl)hexahydro-4,7-dimethyl-4,7-epoxy-1H-isoindole-1,3(2H)-dione. | 2.34 rotational isomers LC | 20 |
| 323 | 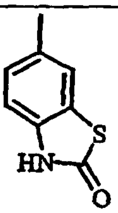 | $CH_3$ | (3aα,4β,7β,7aα)-2-(2,3-Dihydro-2-oxo-6-benzothiazolyl)hexahydro-4,7-dimethyl-4,7-epoxy-1H-isoindole-1,3(2H)-dione. | 2.16 LC | 20 |
| 324 | 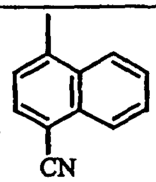 | 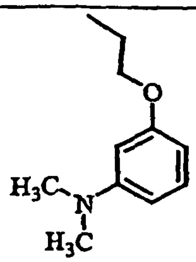 | (3aα,4β,7β,7aα)-4-[4-[2-[3-(Dimethylamino)phenoxy]ethyl]octahydro-7-methyl-1,3-dioxo-4,7-epoxy-2H- | 2.63 & 2.79 rotational isomers LC | 223, 35 |

| Ex. No | G | $R^7$ | Compound Name | Retention Time Min./ Molecular Mass | Procedure of Ex. |
|---|---|---|---|---|---|
| | | | isoindol-2-yl]-1-naphthalenecarbonitrile. | | |
| 325 | 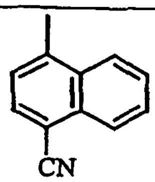 | 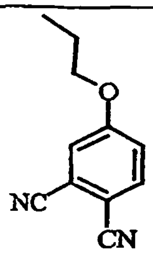 | (3aα,4β,7β,7aα)-4-[2-[4-Cyano-3-(trifluoromethyl)phenyl]octahydro-7-methyl-1,3-dioxo-4,7-epoxy-4H-isoindol-4-yl]ethoxy]-1,2-benzenedicarbonitrile. | 3.42 rotational isomers LC | 223, 35 |
| 326 | 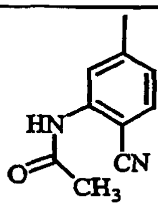 | $CH_3$ | (3aα,4β,7β,7aα)-N-[2-Cyano-5-(octahydro-4,7-dimethyl-1,3-dioxo-4,7-epoxy-2H-isoindol-2-yl)phenyl]acetamide. | 1.94 LC | 20 |
| 327 | 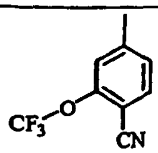 | $CH_3$ | (3aα,4β,7β,7aα)-4-(Octahydro-4,7-dimethyl-1,3-dioxo-4,7-epoxy-2H-isoindol-2-yl)- | 3.52 LC | 20 |

| Ex. No | G | R⁷ | Compound Name | Retention Time Min./ Molecular Mass | Procedure of Ex. |
|---|---|---|---|---|---|
| | | | 2-(trifluoromethoxy)benzonitrile. | | |
| 328 | 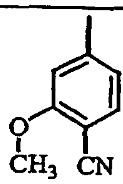 | $CH_3$ | (3aα,4β,7β,7aα)-2-Methoxy-4-(octahydro-4,7-dimethyl-1,3-dioxo-4,7-epoxy-2H-isoindol-2-yl)benzonitrile. | 2.47 LC | 20 |
| 329 | 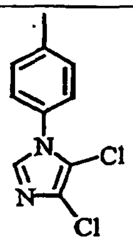 | $CH_3$ | (3aα,4β,7β,7aα)-2-[4-(4,5-Dichloro-1H-imidazol-1-yl)phenyl]hexahydro-4,7-dimethyl-4,7-epoxy-1H-isoindole-1,3(2H)-dione. | 3.09 LC | 20 |
| 330 | 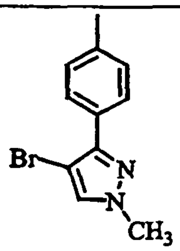 | $CH_3$ | (3aα,4β,7β,7aα)-2-[4-(4-Bromo-1-methyl-1H-pyrazol-3-yl)phenyl]hexahydro-4,7-dimethyl-4,7-epoxy-1H-isoindole-1,3(2H)-dione. | 3.04 LC | 20 |

| Ex. No | G | R⁷ | Compound Name | Retention Time Min./ Molecular Mass | Procedure of Ex. |
|---|---|---|---|---|---|
| 331 | 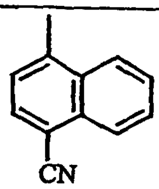 | 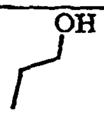 | (3aα,4β,7β,7aα)-4-[Octahydro-4-(2-hydroxyethyl)-7-methyl-1,3-dioxo-4,7-epoxy-2H-isoindol-2-yl]-1-naphthalenecarbonitrile. | 2.44 & 2.60 rotational isomers LC | 223, 35 |
| 332 | 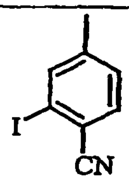 | $CH_3$ | (3aα,4β,7β,7aα)-2-Iodo-4-(Octahydro-4,7-dimethyl-1,3-dioxo-4,7-epoxy-2H-isoindol-2-yl)benzonitrile. | 2.78 rotational isomers LC | 20 |
| 333 | 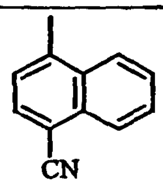 | 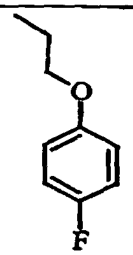 | (3aα,4β,7β,7aα)-4-[4-[2-(4-Fluorophenoxy)ethyl]octahydro-7-methyl-1,3-dioxo-4,7-epoxy-2H-isoindol-2-yl]-1-naphthalenecarbonitrile. | 3.39 & 3.53 rotational isomers LC | 223, 35 |

| Ex. No | G | R7 | Compound Name | Retention Time Min./ Molecular Mass | Procedure of Ex. |
|---|---|---|---|---|---|
| 334 | 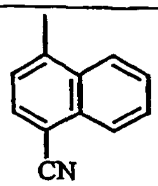 | 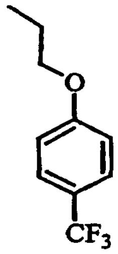 | (3aα,4β,7β,7aα)-4-[Octahydro-4-methyl-1,3-dioxo-7-[2-[4-(trifluoromethyl)phenoxy]ethyl]-4,7-epoxy-2H-isoindol-2-yl]-1-naphthalenecarbonitrile. | 3.66 & 3.78 rotational isomers LC | 223, 35 |
| 335 | 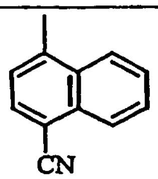 | 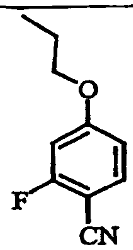 | (3aα,4β,7β,7aα)-4-[4-[2-(4-Cyano-3-fluorophenoxy)ethyl]octahydro-7-methyl-1,3-dioxo-4,7-epoxy-2H-isoindol-2-yl]-1-naphthalenecarbonitrile. | 3.26 & 3.41 rotational isomers LC | 223, 35 |
| 336 | 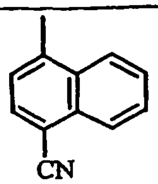 | 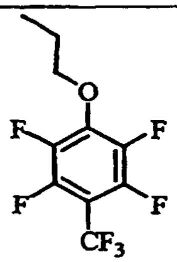 | (3aα,4β,7β,7aα)-4-[Octahydro-4-methyl-1,3-dioxo-7-[2-[2,3,5,6-tetrafluoro-4-(trifluoromethyl)phenoxy]ethy | 3.94 & 4.01 rotational isomers LC | 223, 35 |

| Ex. No | G | R7 | Compound Name | Retention Time Min./ Molecular Mass | Procedure of Ex. |
| --- | --- | --- | --- | --- | --- |
| | | | l]-4,7-epoxy-2H-isoindol-2-yl]-1-naphthalenecarbonitrile. | | |
| 337 | 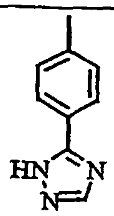 | $CH_3$ | (3aα,4β,7β,7aα)-Hexahydro-4,7-dimethyl-2-[4-(1H-1,2,4-triazol-3-yl)phenyl]-4,7-epoxy-1H-isoindole-1,3(2H)-dione. | 2.06 LC | 20 |
| 338 | 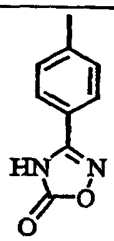 | $CH_3$ | (3aα,4β,7β,7aα)-2-[4-(4,5-Dihydro-5-oxo-1,2,4-oxadiazol-3-yl)phenyl]hexahydro-4,7-dimethyl-4,7-epoxy-1H-isoindole-1,3(2H)-dione. | 2.42 LC | 20 |
| 339 | 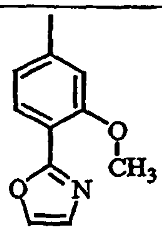 | $CH_3$ | (3aα,4β,7β,7aα)-Hexahydro-2-[3-methoxy-4-(2-oxazolyl)phenyl]-4,7-dimethyl-4,7-epoxy-1H- | 2.51 LC | 20 |

| Ex. No | G | R⁷ | Compound Name | Retention Time Min./ Molecular Mass | Procedure of Ex. |
|---|---|---|---|---|---|
| | | | isoindole-1,3(2H)-dione. | | |
| 340 | 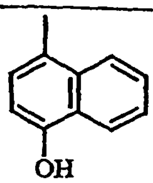 | $CH_3$ | (3aα,4β,7β,7aα)-Hexahydro-2-(4-hydroxy-1-naphthalenyl)-4,7-dimethyl-4,7-epoxy-1H-isoindole-1,3(2H)-dione. | 2.30 LC | 20 |
| 341 | 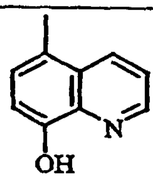 | $CH_3$ | (3aα,4β,7β,7aα)-Hexahydro-2-(8-hydroxy-5-quinolinyl)-4,7-dimethyl-4,7-epoxy-1H-isoindole-1,3(2H)-dione, trifluoroacetate (1:1). | 1.49 LC | 20 |
| 342 | 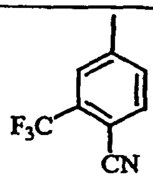 | 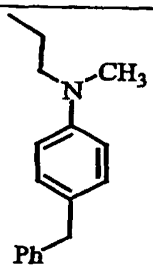 | (3aα,4β,7β,7aα)-4-[Octahydro-4-methyl-1,3-dioxo-7-[2-[methyl(phenylmethyl)amino]ethyl]-4,7-epoxy-2H-isoindol-2-yl]-2-(trifluoromethy | 2.42 LC | 223, 35 |

| Ex. No | G | $R^7$ | Compound Name | Retention Time Min./ Molecular Mass | Procedure of Ex. |
|---|---|---|---|---|---|
| | | | l)benzonitrile. | | |
| 343 | 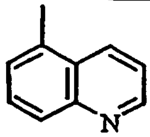 | $CH_3$ | (3aα,4β,7β,7aα)-Hexahydro-4,7-dimethyl-2-(5-quinolinyl)-4,7-epoxy-1H-isoindole-1,3(2H)-dione. | 1.69 LC | 20 |
| 344 | 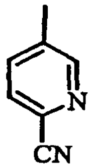 | $CH_3$ | (3aα,4β,7β,7aα)-5-(Octahydro-4,7-dimethyl-1,3-dioxo-4,7-epoxy-2H-isoindol-2-yl)-2-pyridinecarbonitrile. | 2.18 LC | 20 |
| 345 | 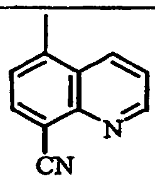 | $CH_3$ | (3aα,4β,7β,7aα)-5-(Octahydro-4,7-dimethyl-1,3-dioxo-4,7-epoxy-2H-isoindol-2-yl)-8-quinolinecarbonitrile. | 2.31 LC | 20 |
| 346 | 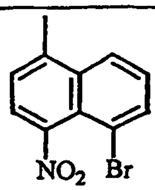 | $CH_3$ | (3aα,4β,7β,7aα)-2-(5-Bromo-4-nitro- | 3.10 & 3.29 rotational isomers LC | 20 |

| Ex. No | G | $R^7$ | Compound Name | Retention Time Min./ Molecular Mass | Procedure of Ex. |
|---|---|---|---|---|---|
| | | | 1-naphthalenyl)hexahydro-4,7-dimethyl-4,7-epoxy-1H-isoindole-1,3(2H)-dione. | | |
| 347 | 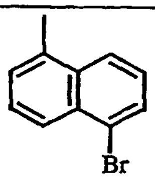 | $CH_3$ | (3aα,4β,7β,7aα)-2-(5-Bromo-1-naphthalenyl)hexahydro-4,7-dimethyl-4,7-epoxy-1H-isoindole-1,3(2H)-dione. | 3.28 & 3.40 rotational isomers LC | 20 |
| 348 | 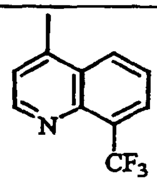 | $CH_3$ | (3aα,4β,7β,7aα)-Hexahydro-4,7-dimethyl-2-[8-(trifluoromethyl)-4-quinolinyl]-4,7-epoxy-1H-isoindole-1,3(2H)-dione. | 3.08 LC | 20 |
| 349 | 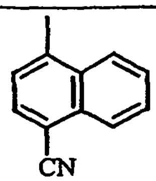 | 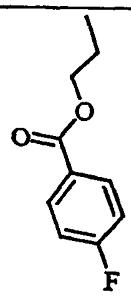 | 4-Fluorobenzoic acid, 2-[(3aα,4β,7β,7aα)-2-(4-cyano-1-naphthalenyl)octahydro-7- | 3.64 & 3.77 rotational isomers LC | 223 |

| Ex. No | G | R7 | Compound Name | Retention Time Min./ Molecular Mass | Procedure of Ex. |
|---|---|---|---|---|---|
| | | | methyl-1,3-dioxo-4,7-epoxy-4H-isoindol-4-yl]ethyl ester. | | |
| 350 | 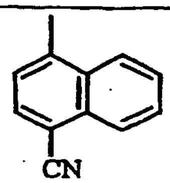 | 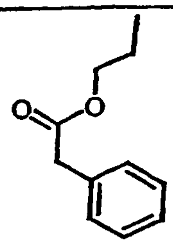 | Benzeneacetic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester. | 3.53 & 3.67 rotational isomers LC | 223 |
| 351 | 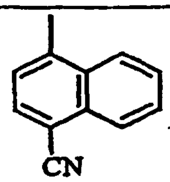 | 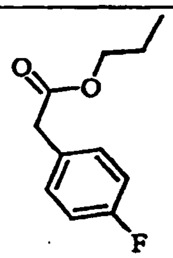 | 4-Fluorobenzeneacetic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester. | 3.53 & 3.66 rotational isomers LC | 223 |

| Ex. No | G | R⁷ | Compound Name | Retention Time Min./ Molecular Mass | Procedure of Ex. |
|---|---|---|---|---|---|
| 352 | 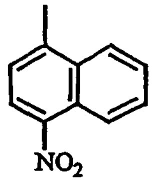 | 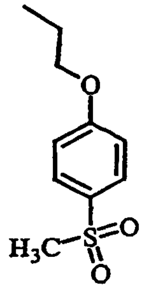 | (3aα,4β,7β,7aα)-Hexahydro-4-methyl-7-[2-[4-(methylsulfonyl)phenoxy]ethyl]-2-(4-nitro-1-naphthalenyl)-4,7-epoxy-1H-isoindole-1,3(2H)-dione. | 3.31 LC | 223, 35 |
| 353 | 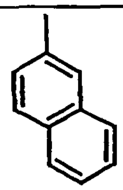 | $CH_3$ | (3aα,4β,7β,7aα)-Hexahydro-2-(2-naphthalenyl)-4,7-dimethyl-4,7-epoxy-1H-isoindole-1,3(2H)-dione. | 2.94 LC | 20 |
| 354 | 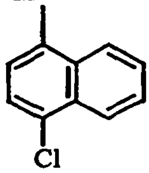 | $CH_3$ | (3aα,4β,7β,7aα)-2-(4-Chloro-1-naphthalenyl)hexahydro-4,7-dimethyl-4,7-epoxy-1H-isoindole-1,3(2H)-dione. | 3.22 & 3.34 rotational isomers LC | 20 |

| Ex. No | G | R7 | Compound Name | Retention Time Min./ Molecular Mass | Procedure of Ex. |
|---|---|---|---|---|---|
| 355 | 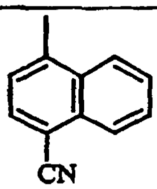 | 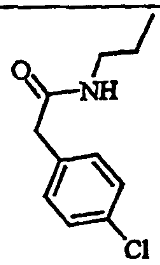 | (3aα,4β,7β,7aα)-N-[(4-Chlorophenyl)methyl]-2-(4-cyano-1-naphthalenyl)octahydro-7-methyl-1,3-dioxo-4,7-epoxy-4H-isoindole-4-acetamide. | 3.52 LC | 237 |
| 356 | 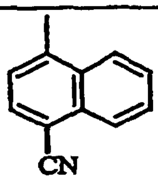 | 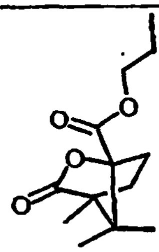 | 4,7,7-Trimethyl-3-oxo-2-oxabicyclo[2.2.1]heptane-1-carboxylic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester. | 3.45 LC | 223 |

| Ex. No | G | R7 | Compound Name | Retention Time Min./ Molecular Mass | Procedure of Ex. |
|---|---|---|---|---|---|
| 357 | 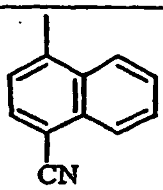 | 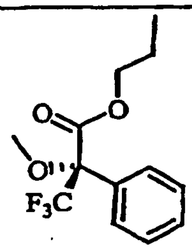 | (αS)- α-Methoxy-α-(trifluoromethyl)benzeneacetic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester. | 3.91 LC | 223 |
| 358 | 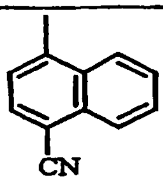 | 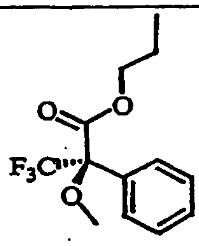 | ( αR)- α-Methoxy-α-(trifluoromethyl)benzeneacetic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester. | 2.00 LC | 223 |

| Ex. No | G | R7 | Compound Name | Retention Time Min./ Molecular Mass | Procedure of Ex. |
|---|---|---|---|---|---|
| 359 | 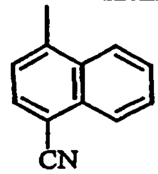 | 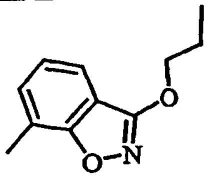 | (3aα,4β,7β,7a α)-4-[Octahydro-4-methyl-7-[2-[(7-methyl-1,2-benzisoxazol-3-yl)oxy]ethyl]-1,3-dioxo-4,7-epoxy-2H-isoindol-2-yl]-1-naphthalenecarbonitrile. | 3.79 & 3.92 LC Rotationale isomers | 250 |
| 360 | 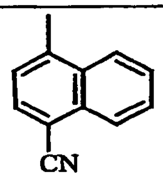 | 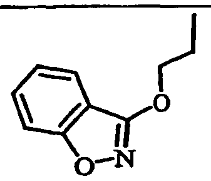 | (3aα,4β,7β,7a α)-4-[4-[2-(1,2-Benzisoxazol-3-yloxy)ethyl]octahydro-7-methyl-1,3-dioxo-4,7-epoxy-2H-isoindol-2-yl]-1-naphthalenecarbonitrile. | 3.55 & 3.70 LC Rotationale Isomers | 250 |
| 361 | 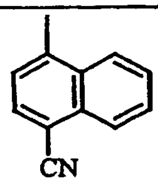 | 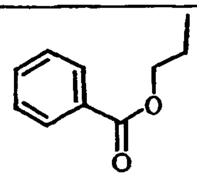 | (3aα,4β,7β,7a α)-4-[2-(Benzoyloxy)ethyl]-2-(4-cyano-1-naphthalenyl)hexahydro-7- | 3.51 & 3.66 LC Rotationale isomers | 223 |

| Ex. No | G | R7 | Compound Name | Retention Time Min./ Molecular Mass | Procedure of Ex. |
|---|---|---|---|---|---|
| | | | methyl-4,7-epoxy-1H-isoindole-1,3(2H)-dione. | | |
| 362 | 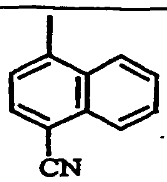 | 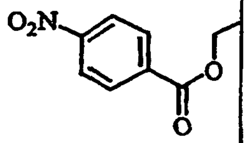 | (3aα,4β,7β,7aα)-2-(4-Cyano-1-naphthalenyl)-4-[2-[(4-nitrobenzoyl)oxy]ethyl]hexahydro-7-methyl-4,7-epoxy-1H-isoindole-1,3(2H)-dione. | 3.52 & 3.67 LC Rotationale Isomers | 223 |
| 363 | 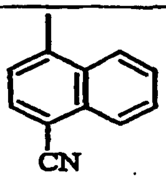 | 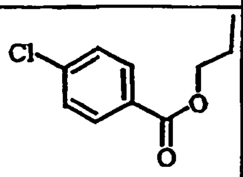 | 4-Chlorobenzoic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester. | 3.79 LC | 223 |
| 364 | 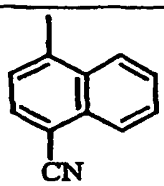 | 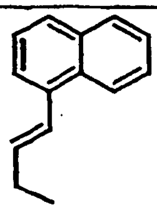 | [3aα,4β,7β,7aα(E)]-4-[Octahydro-4-methyl-7-[3-(1-naphthalenyl)- | 4.14 LC 499.13 [M+H]+ | 248 |

| Ex. No | G | R7 | Compound Name | Retention Time Min./ Molecular Mass | Procedure of Ex. |
|---|---|---|---|---|---|
| | | | 2-propenyl]-1,3-dioxo-4,7-epoxy-2H-isoindol-2-yl]-1-naphthalenecarbonitrile. | | |
| 365 | 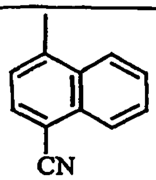 | 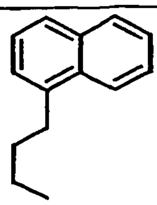 | (3aα,4β,7β,7aα)-4-[Octahydro-4-methyl-7-[3-(1-naphthalenyl)propyl]-1,3-dioxo-4,7-epoxy-2H-isoindol-2-yl]-1-naphthalenecarbonitrile. | 4.14 LC 501.14 $[M+H]^+$ | 248, 249 |
| 366 | 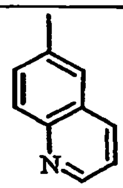 | $CH_3$ | (3aα,4β,7β,7aα)-Hexahydro-4,7-dimethyl-2-(2-methyl-6-quinolinyl)-4,7-epoxy-1H-isoindole-1,3(2H)-dione. | 1.25 LC 337.0 $[M+H]^+$ | 20 |
| 367 | 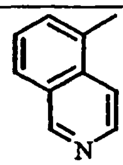 | $CH_3$ | (3aα,4β,7β,7aα)-Hexahydro-2-(5-isoquinolinyl)-4,7-dimethyl-4,7-epoxy-1H- | 1.06 & 1.29 LC Rotationale Isomers 323.0 $[M+H]^+$ | 20 |

| Ex. No | G | $R^7$ | Compound Name | Retention Time Min./ Molecular Mass | Procedure of Ex. |
|---|---|---|---|---|---|
| | | | isoindole-1,3(2H)-dione. | | |
| 368 | 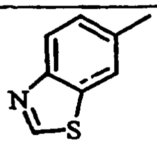 | $CH_3$ | (3aα,4β,7β,7aα)-2-(6-Benzothiazolyl)hexahydro-4,7-dimethyl-4,7-epoxy-1H-isoindole-1,3(2H)-dione. | 2.15 LC 329.0 $[M+H]^+$ | 20 |
| 369 | 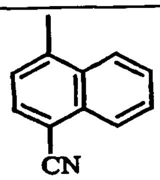 | 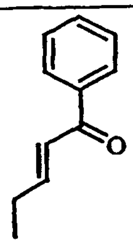 | [3aα,4β,7β,7aα(E)]-4-[Octahydro-4-methyl-1,3-dioxo-7-(4-oxo-4-phenyl-2-butenyl)-4,7-epoxy-2H-isoindol-2-yl]-1-naphthalenecarbonitrile. | 3.50 LC 482.14 $[M+H]^+$ | 248 |
| 370 | 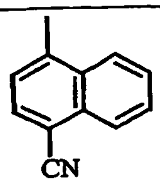 | 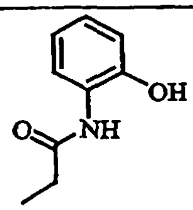 | (3aα,4β,7β,7aα)-2-(4-Cyano-1-naphthalenyl)octahydro-N-(2-hydroxyphenyl)-7-methyl-1,3-dioxo-4,7-epoxy-4H-isoindole-4-acetamide. | 3.07 LC 482.14 $[M+H]^+$ | 236 |

| Ex. No | G | $R^7$ | Compound Name | Retention Time Min./ Molecular Mass | Procedure of Ex. |
|---|---|---|---|---|---|
| 371 | 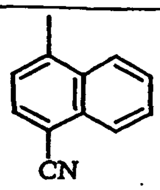 | 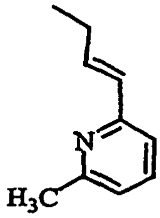 | [3aα,4β(E),7β, 7aα]-4-[Octahydro-4-methyl-7-[3-(6-methyl-2-pyridinyl)-2-propenyl]-1,3-dioxo-4,7-epoxy-2H-isoindol-2-yl]-1-naphthalenecarbonitrile. | 2.28 LC 464.19 $[M+H]^+$ | 248 |
| 372 | 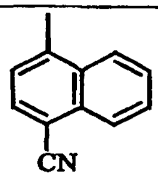 | 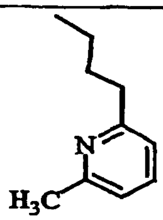 | (3aα,4β,7β,7aα)-4-[Octahydro-4-methyl-7-[3-(6-methyl-2-pyridinyl)propyl]-1,3-dioxo-4,7-epoxy-2H-isoindol-2-yl]-1-naphthalenecarbonitrile. | 2.19 LC 466.32 $[M+H]^+$ | 248, 249 |
| 373 | 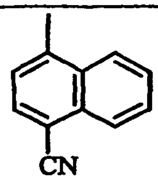 | 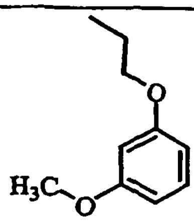 | [3aR-(3aα,4β,7β,7aα)]-4-[Octahydro-4-[2-(3-methoxyphenoxy)ethyl]-7-methyl-1,3-dioxo-4,7-epoxy-2H-isoindol-2-yl]-1- | 3.73 LC 483.65 $[M+H]^+$ | 238i, 239i |

| Ex. No | G | R7 | Compound Name | Retention Time Min./ Molecular Mass | Procedure of Ex. |
|---|---|---|---|---|---|
| | | | naphthalenecarbonitrile. | | |
| 374 | 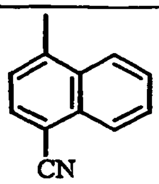 | 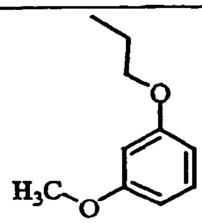 | [3aS-(3aα,4β,7β,7aα)]-4-[Octahydro-4-[2-(3-methoxyphenoxy)ethyl]-7-methyl-1,3-dioxo-4,7-epoxy-2H-isoindol-2-yl]-1-naphthalenecarbonitrile. | 3.73 LC | 238ii, 239ii |
| 375 | 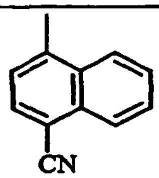 | 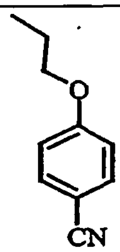 | [3aR-(3aα,4β,7β,7aα)]-4-[4-[2-(4-Cyanophenoxy)ethyl]octahydro-7-methyl-1,3-dioxo-4,7-epoxy-2H-isoindol-2-yl]-1-naphthalenecarbonitrile. | 3.33 & 3.49 LC Rotationale Isomers | 238i, 239i |
| 376 | 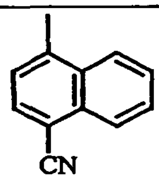 | 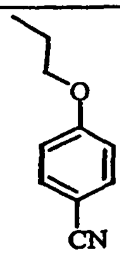 | [3aS-(3aα,4β,7β,7aα)]-4-[4-[2-(4-Cyanophenoxy)ethyl]octahydro-7-methyl-1,3-dioxo-4,7-epoxy-2H-isoindol-2-yl]-1-naphthalenecarbonitrile. | 3.73 LC 483.65 [M+H]+ | 238ii, 239ii |
| 377 | 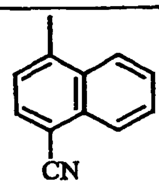 | 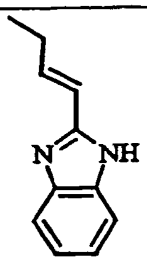 | [3aα,4β(E),7β,7aα]-4-[4-[3-(1H-Benzimidazol-2-yl)-2-propenyl]octahydro-7-methyl-1,3-dioxo-4,7-epoxy-2H- | 2.48 LC 489.26 [M+H]+ | 248 |

| Ex. No | G | $R^7$ | Compound Name | Retention Time Min./ Molecular Mass | Procedure of Ex. |
|---|---|---|---|---|---|
| | | | isoindol-2-yl]-1-naphthalenecarbonitrile | | |
| 378 | 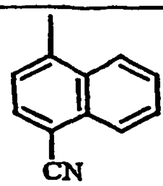 | 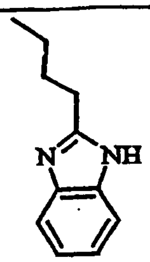 | (3aα,4β,7β,7aα)-4-[4-[3-(1H-Benzimidazol-2-yl)propyl]octahydro-7-methyl-1,3-dioxo-4,7-epoxy-2H-isoindol-2-yl]-1-naphthalenecarbonitrile | 2.37 LC 491.26 $[M+H]^+$ | 249 |

## Examples 379 to 381

Additional compounds of the present invention were prepared by procedures analogous to those described above. The compounds of Examples 379 to 381 have the following structure (L is a bond):

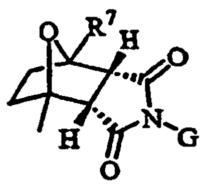

where G, $R^7$, the compound name, retention time, molecular mass, and the procedure employed, are set forth in Table 6. The chromatography techniques used to determine the compound retention times of Table 6 are as follows: LCMS = YMC S5 ODS column, 4.6 x 50 mm eluting with 10-90% $MeOH/H_2O$ over 4 minutes containing 0.1 % TFA; 4 mL/min, monitoring at 220 nm. LCMS* = YMC S5 ODS column, 4.6 x 50 mm eluting with 10-90% $MeOH/H_2O$ over 2 minutes containing 0.1 % TFA; 4 mL/min, monitoring at 220 nm. LC = YMC S5 ODS column 4.6 x 50 mm eluting with 10-90% $MeOH/H_2O$ over 4 minutes containing 0.2% phosphoric acid, 4 mL/min, monitoring at 220 nm.
The molecular mass of the compounds listed in **Table 6** was determined by MS (ES) by the formula m/z.

**Table 6**

| Ex. No | G | R⁷ | Compound Name | Retention Time Min./ Molecular Mass | Procedure of Ex. |
|---|---|---|---|---|---|
| 379 | 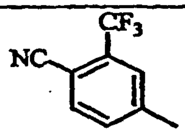 | 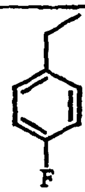 | (3aα,4α,7α,7aα)-4-[4-[(4-Fluorophenyl)methyl]octahydro-7-methyl-1,3-dioxo-4,7-epoxy-2H-isoindol-2-yl]-2-(trifluoromethyl)benzonitrile. | 3.75 LC | 205 |
| 380 | 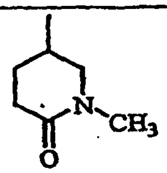 | $CH_3$ | (3aα,4α,7α,7aα)-Hexahydro-4,7-dimethyl-2-(1-methyl-6-oxo-3-piperidinyl)-4,7-epoxy-1H-isoindole-1,3(2H)-dione. | 1.88 LC | 27 |
| 381 | 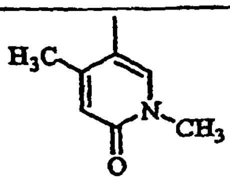 | $CH_3$ | (3aα,4α,7α,7aα)-2-(1,6-Dihydro-1,4-dimethyl-6-oxo-3- | 1.91 LC | 27 |

| Ex. No | G | R7 | Compound Name | Retention Time Min./ Molecular Mass | Procedure of Ex. |
|---|---|---|---|---|---|
| | | | pyridinyl)hexahydro-4,7-dimethyl-4,7-epoxy-1H-isoindole-1,3(2H)-dione. | | |

**Examples 382 to 383**

Additional compounds of the present invention were prepared by procedures analogous to those described above. The compounds of Examples 382 to 383 have the structure, compound name, retention time, molecular mass, and were prepared by the procedure employed, set forth in the following **Table 7**. The chromatography techniques used to determine the compound retention times of **Table 7** are as follows: LCMS = YMC S5 ODS column, 4.6 x 50 mm eluting with 10-90% MeOH/$H_2O$ over 4 minutes containing 0.1 % TFA; 4 mL/min, monitoring at 220 nm. LCMS* = YMC S5 ODS column, 4.6 x 50 mm eluting with 10-90% MeOH/$H_2O$ over 2 minutes containing 0.1% TFA; 4 mL/min, monitoring at 220 nm. LC = YMC S5 ODS column 4.6 x 50 mm eluting with 10-90% MeOH/$H_2O$ over 4 minutes containing 0.2% phosphoric acid, 4 mL/min, monitoring at 220 nm. The molecular mass of the compounds listed in **Table 7** was determined by MS (ES) by the formula m/z.

## Table 7

| Ex. No. | Structure | Compound Name | Retention Time Min. | Procedure of Example |
|---|---|---|---|---|
| 382 | 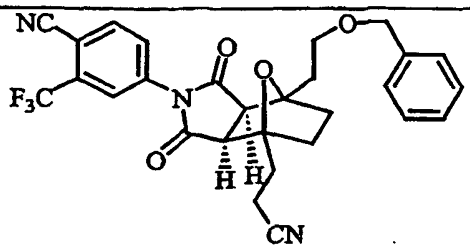 | (3aα,4β,7β,7aα)-2-[4-Cyano-3-(trifluoromethyl)phenyl]octahydro-1,3-dioxo-7-[2-(phenylmethoxy)ethyl]-4,7-epoxy-4H-isoindole-4-propanenitrile. | 3.63 LC | 255 |
| 383 | 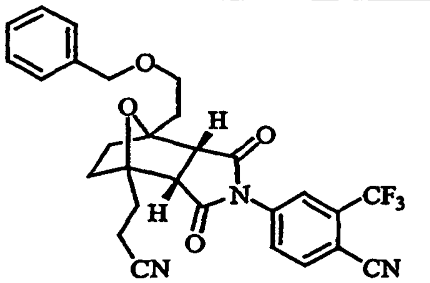 | (3aα,4β,7β,7aα)-2-[4-Cyano-3-(trifluoromethyl)phenyl]octahydro-1,3-dioxo-7-[2-(phenylmethoxy)ethyl]-4,7-epoxy-4H-isoindole-4-propanenitrile | 3.64 LC | 255 |

## Examples 384 to 418

Additional compounds of the present invention were prepared by procedures analogous to those described above. The compounds of Examples 384 to 418 have the following structure (L is a bond):

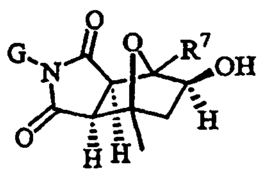

where G, $R^7$, the compound name, retention time, molecular mass, and the procedure employed, are set forth in **Table 8.** The absolute configuration for the following compounds was not determined. For simplicity in nomenclature, compound **243Di** is designated herein as having an "S" configuration and compound **243Dii** as having an "R" configuration. Enantiomerically pure products derived from compound **243Di** are designated herein as having an "S" configuration and enantiomerically pure products derived from compound **243Dii** are designated herein as having an "R" configuration.

The chromatography techniques used to determine the compound retention times of **Table 8** are as follows:

**LCMS** = YMC S5 ODS column, 4.6 x 50 mm eluting with 10-90% MeOH/$H_2O$ over 4 minutes containing 0.1 % TFA; 4 mL/min, monitoring at 220 nm. **LCMS*** = YMC S5 ODS column, 4.6 x 50 mm eluting with 10-90% MeOH/$H_2O$ over 2 minutes containing 0.1 % TFA; 4 mL/min, monitoring at 220 nm. **LC** = YMC S5 ODS column 4.6 x 50 mm eluting with 10-90% MeOH/$H_2O$ over 4 minutes containing 0.2% phosphoric acid, 4 ml/min, monitoring at 220 nm. The molecular mass of the compounds listed in **Table 8** was determined by MS (ES) by the formula m/z.

## Table 8

| Ex. No | G | $R^7$ | Compound Name | Retention Time Min./ Molecular Mass | Procedure of Ex. |
|---|---|---|---|---|---|
| 384 | 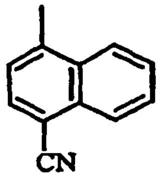 | 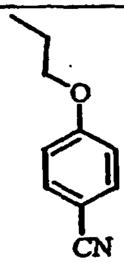 | (3aα,4β,7β,7aα) -4-[7-[2-(4-Cyanophenoxy) ethyl]octahydro-5-hydroxy-4-methyl-1,3-dioxo-4,7-epoxy-2H-isoindol-2- | 3.18 LC 494.40 $[M+H]^+$ | 227, 228, 229 |

| Ex. No | G | R⁷ | Compound Name | Retention Time Min./ Molecular Mass | Procedure of Ex. |
|---|---|---|---|---|---|
| 405 | 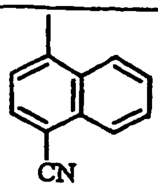 | 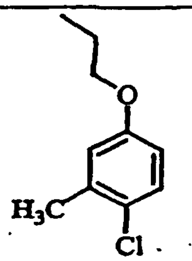 | [3aR-(3aα,4β,7β,7aα)]-4-[7-[2-(4-Chloro-3-methylphenoxy)ethyl]octahydro-5-hydroxy-4-methyl-1,3-dioxo-4,7-epoxy-2H-isoindol-2-yl]-1-naphthalenecarbonitrile. | 3.68 LC 517.33 [M+H]+ | 234Dii, 243ii |
| 406 | 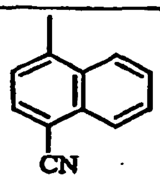 | 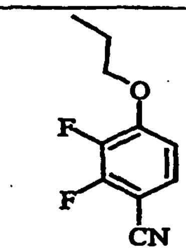 | [3aR-(3aα,4β,7β,7aα)]-4-[7-[2-(4-Cyano-2,3-difluorophenoxy)ethyl]octahydro-5-hydroxy-4-methyl-1,3-dioxo-4,7-epoxy-2H-isoindol-2-yl]-1-naphthalenecarbonitrile. | 3.23 LC 530.13 [M+H]+ | 234Dii, 243ii |
| 407 | 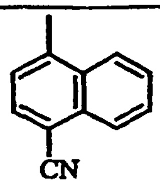 | 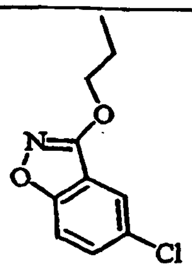 | [3aS-(3aα,4β,7β,7aα)]-4-[7-[2-[(5-Chloro-1,2-benzisoxazol-3-yl)oxy]ethyl]octahydro-5- | 3.59 LC 602.1 [M-H+OAc]- | 243Di, 252 |

| Ex. No | G | R7 | Compound Name | Retention Time Min./ Molecular Mass | Procedure of Ex. |
|---|---|---|---|---|---|
| | | | hydroxy-4-methyl-1,3-dioxo-4,7-epoxy-2H-isoindol-2-yl]-1-naphthalenecarbonitrile. | | |
| 408 | 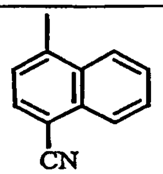 | 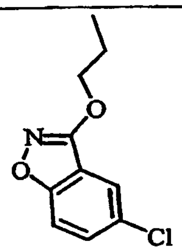 | [3aR-(3aα,4β,7β,7aα)]-4-[7-[2-[(5-Chloro-1,2-benzisoxazol-3-yl)oxy]ethyl]octahydro-5-hydroxy-4-methyl-1,3-dioxo-4,7-epoxy-2H-isoindol-2-yl]-1-naphthalenecarbonitrile. | 3.57 LC 602.0 [M-H+OAc]- | 243Dii, 253 |
| 409 | 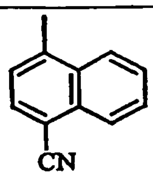 | 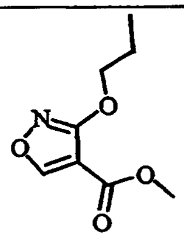 | [3aR-(3aα,4β,7β,7aα)]-3-[2-[2-(4-Cyano-1-naphthalenyl)octahydro-6-hydroxy-7-methyl-1,3-dioxo-4,7-epoxy-4H-isoindol-4-yl]ethoxy]-5-isoxazolecarbox | 2.90 LC 518.27 [M+H]+ | 243Dii, 253 |

| Ex. No | G | $R^7$ | Compound Name | Retention Time Min./ Molecular Mass | Procedure of Ex. |
|---|---|---|---|---|---|
| | | | ylic acid, methyl ester. | | |
| 410 | 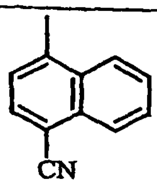 | 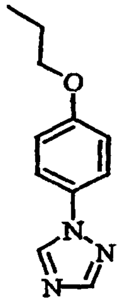 | [3aR-(3aα,4β,7β,7aα)]-4-[Octahydro-5-hydroxy-4-methyl-1,3-dioxo-7-[2-[4-(1H-1,2,4-triazol-1-yl)phenoxy]ethyl]-4,7-epoxy-2H-isoindol-2-yl]-1-naphthalenecarbonitrile. | 2.93 LC 536.30 $[M+H]^+$ | 243Dii, 244ii |
| 411 | 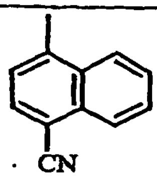 | 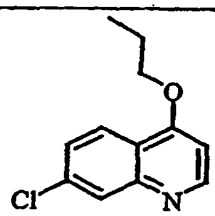 | [3aS-(3aα,4β,7β,7aα)]-4-[7-[2-[(7-Chloro-4-quinolinyl)oxy]ethyl]octahydro-5-hydroxy-4-methyl-1,3-dioxo-4,7-epoxy-2H-isoindol-2-yl]-1-naphthalenecarbonitrile, trifluoroacetate (1:1). | 2.52 LC 554.13 $[M+H]^+$ | 243Di, 244i |

| Ex. No | G | R7 | Compound Name | Retention Time Min./ Molecular Mass | Procedure of Ex. |
|---|---|---|---|---|---|
| 412 | 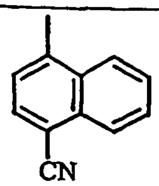 | 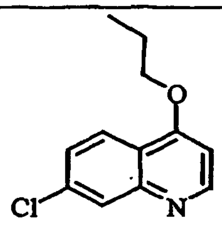 | [3aR-(3aα,4β,7β,7aα)]-4-[7-[2-[(7-Chloro-4-quinolinyl)oxy]ethyl]octahydro-5-hydroxy-4-methyl-1,3-dioxo-4,7-epoxy-2H-isoindol-2-yl]-1-naphthalenecarbonitrile, trifluoroacetate (1:1). | 2.53 LC 554.27 $[M+H]^+$ | 243Dii, 244ii |
| 413 | 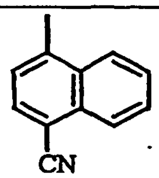 | 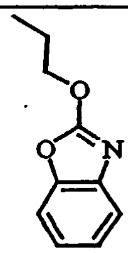 | [3aR-(3α,4β,5β,7β,7aα)]-4-[7-[2-(2-Benzoxazolyloxy)ethyl]octahydro-5-hydroxy-4-methyl-1,3-dioxo-4,7-epoxy-2H-isoindol-2-yl]-1-naphthalenecarbonitrile | 3.13 LC 568.1 $[M-H+OAc]^-$ | 243Dii, 244ii |
| 414 | 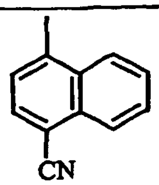 | 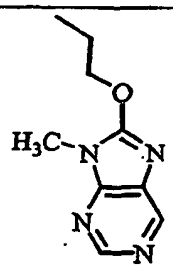 | [3aR-(3α,4β,5β,7β,7aα)]-4-[Octahydro-5-hydroxy-4-methyl-7-[2-[(9- | 2.34 LC 525.2 $[M+H]^+$ | 243Dii, 244ii |

| Ex. No | G | R⁷ | Compound Name | Retention Time Min./ Molecular Mass | Procedure of Ex. |
|---|---|---|---|---|---|
| | | | methyl-9H-purin-8-yl)oxy]ethyl}-1,3-dioxo-4,7-epoxy-2H-isoindol-2-yl]-1-naphthalenecarbonitrile | | |
| 415 | 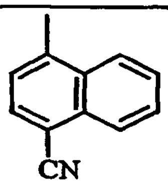 | 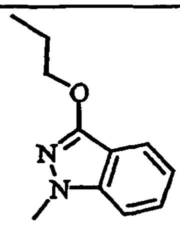 | [3aR-(3α,4β,5β,7β,7aα)]-4-[Octahydro-5-hydroxy-4-methyl-7-[2-[(1-methyl-1H-indazol-3-yl)oxy]ethyl]-1,3-dioxo-4,7-epoxy-2H-isoindol-2-yl]-1-naphthalenecarbonitrile | 3.33 LC | 251,253 |
| 416 | 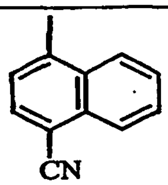 | 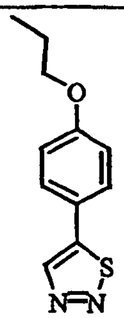 | [3aS-(3α,4β,5β,7β,7aα)]-4-[Octahydro-5-hydroxy-4-methyl-7-[2-[4-(1,2,3-thiadiazol-5-yl)phenoxy]ethyl]-1,3-dioxo-4,7-epoxy-2H-isoindol-2-yl]-1-naphthalenecarbonitrile | 3.17 LC 553.10 [M+H]⁺ | 243Dii, 244ii |

| Ex. No | G | $R^7$ | Compound Name | Retention Time Min./ Molecular Mass | Procedure of Ex. |
|---|---|---|---|---|---|
| 417 | 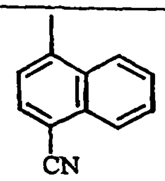 | 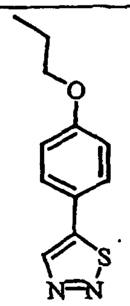 | [3aR-(3α,4β,5β,7β,7aα)]-4-[Octahydro-5-hydroxy-4-methyl-7-[2-[4-(1,2,3-thiadiazol-5-yl)phenoxy]ethyl]-1,3-dioxo-4,7-epoxy-2H-isoindol-2-yl]-1-naphthalenecarbonitrile | 3.20 LC 553.25 $[M+H]^+$ | 243Dii, 244ii |
| 418 | 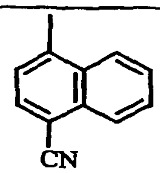 | 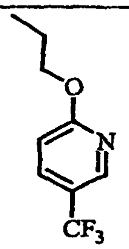 | [3aS-(3α,4β,5β,7β,7aα)]-4-[Octahydro-5-hydroxy-4-methyl-1,3-dioxo-7-[2-[[5-(trifluoromethyl)-2-pyridinyl]oxy]ethyl]-4,7-epoxy-2H-isoindol-2-yl]-1-naphthalenecarbonitrile | 3.45 LC 538.23 $[M+H]^+$ | 243Dii, 244ii |
| 419 | 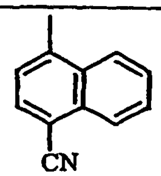 | 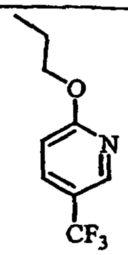 | [3aR-(3α,4β,5β,7β,7aα)]-4-[Octahydro-5-hydroxy-4-methyl-1,3-dioxo-7-[2-[[5- | 3.45 LC 538.23 $[M+H]^+$ | 243Dii, 244ii |

| Ex. No | G | R7 | Compound Name | Retention Time Min./ Molecular Mass | Procedure of Ex. |
|---|---|---|---|---|---|
| | | | (trifluoromethyl)-2-pyridinyl]oxy]ethyl]-4,7-epoxy-2H-isoindol-2-yl]-1-naphthalenecarbonitrile | | |
| 420 | 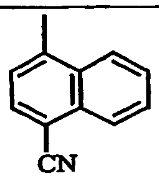 | 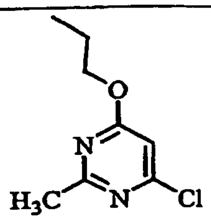 | [3aS-(3α,4β,5β,7β,7aα)]-4-[7-[2-[(6-Chloro-2-methyl-4-pyrimidinyl)oxy]ethyl]octahydro-5-hydroxy-4-methyl-1,3-dioxo-4,7-epoxy-2H-isoindol-2-yl]-1-naphthalenecarbonitrile | 3.02 LC | 243Dii, 244ii |
| 421 | 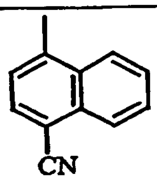 | 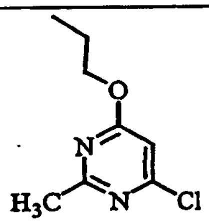 | [3aR-(3α,4β,5β,7β,7aα)]-4-[7-[2-[(6-Chloro-2-methyl-4-pyrimidinyl)oxy]ethyl]octahydro-5-hydroxy-4-methyl-1,3-dioxo-4,7-epoxy-2H-isoindol-2- | 3.02 LC | 243Dii, 244ii |

| Ex. No | G | R[7] | Compound Name | Retention Time Min./ Molecular Mass | Procedure of Ex. |
|---|---|---|---|---|---|
| | | | yl]-1-naphthalenecarbonitrile | | |

**Example 422**

**(3aα,4β,7β,7aα)-2-(7-Bromo-2,1,3-benzoxadiazol-4-yl)hexahydro-4,7-dimethyl-4,7-epoxy-1H-isoindole-1,3(2H)-dione (422C)**

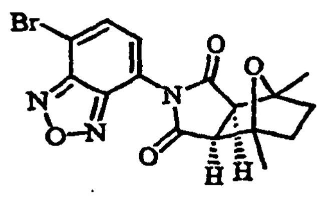

**A. 4-Bromo-7-nitrobenzofurazan (422A)**

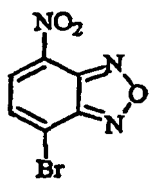

To a solution of 2,6-dibromoaniline (1.0 g, 4.0 mmol) in $CHCl_3$ (8 mL) was added a suspension of *m*CPBA (70% by HPLC, 1.4 g, 8.0 mmol) in $CHCl_3$ (8 mL) and the resulting mixture was stirred for 24 h at rt. The reaction mixture was diluted with $CHCl_3$ and washed successively with 2% $Na_2S_2O_3$ solution, 5% $Na_2CO_3$ solution and brine. The organic layer was dried over $Na_2SO_4$ and concentrated under reduced pressure to leave a solid, which was suspended into DMSO (15 mL). To this suspension was added a solution of $NaN_3$ (272 mg, 4.19 mmol) in DMSO (15 mL) at rt. The resulting mixture was stirred at rt until most of the nitrogen had evolved and was then quickly heated to 120°C for 3 min. The reaction mixture was cooled and poured onto crushed ice (100 g). After standing for 1 h the precipitates were filtered off, dried in *vacuo* and redissolved in concentrated $H_2SO_4$ (5 mL). To this solution was added a solution of $NaNO_3$ (400 mg, 4.7 mmol) in 50% $H_2SO_4$ (1.6 mL) and the temperature was maintained at 60°C. After the addition was complete, the mixture was heated to 85°C for 30 min, cooled to rt and poured onto crushed ice (40 g). EtOAc was added, the layers were separated and the aqueous layer was extracted with EtOAc. The combined organic layers were dried over $Na_2SO_4$ and concentrated to leave a solid which was purified by flash chromatography (silica gel, EtOAc (20%) in hexanes) affording compound **422A** (785 mg, 81%) as a tan solid.

**B. 4-Bromo-7-aminobenzofurazan (422B)**

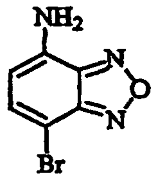

A solution of compound **422A** (563 mg, 2.31 mmol) in AcOH (5 mL) was heated to 70°C and $Fe^0$ powder (258 mg, 4.62 mmol) was added in one portion. The resulting dark reaction mixture was stirred for 15 min, cooled to rt and concentrated under reduced pressure. The residue was taken up in EtOAc and the resulting solution was washed with sat $Na_2CO_3$ solution. The organic layer was dried over $Na_2SO_4$, concentrated and purified by flash chromatography (silica gel, EtOAc in hexanes, 10 to 60%) yielding compound **422B** (470 mg, 95%) as a red solid.

**C. (3aα,4β,7β,7aα)-2-(7-Bromo-2,1,3-benzoxadiazol-4-yl)hexahydro-4,7-dimethyl-4,7-epoxy-1H-isoindole-1,3 (2H)-dione (422C)**

A mixture of compound **422B** (43 mg, 0.20 mmol), compound **20A** (45 mg, 0.23 mmol), $MgSO_4$ (60 mg, 0.50 mmol), $Et_3N$ (139 μL, 1.0 mmol) and 1,2-dimethoxyethane (300 μL) was placed in a sealed tube and heated to 135 °C for 14 h. After cooling to rt the mixture was filtered through celite eluting with MeOH to yield a dark solid which was purified by flash chromatography (silica gel, EtOAc in hexanes, 5 to 40%) furnishing compound 422C (42 mg, 54%) as a yellow solid. HPLC: 99% at 2.96 min (retention time) (YMC S5 ODS column 4.6 x 50 mm Ballistic, 10-90% aqueous methanol over 4 minutes containing 0.2% $H_3PO_4$, 4 mUmin, monitoring at 220 nm). $^1H$ NMR (acetone-$d_6$, 400 MHz): δ = 8.00 (d, $J$ = 7.5 Hz, 1H), 7.45 (d, $J$ = 7.5 Hz, 1H), 3.31 (s, 2H), 1.98-1.93 (m, 2H), 1.74-1.69 (m, 2H), 1.57 (s, 6H).

**Example 423**

**(3aα,4β,7β,7aα)-7-[Octahydro-4,7-dimethyl-1,3-dioxo-4,7-epoxy-2H-isoindol-2-yl]-2,1,3-benzoxadiazole-4-carbonitrile (423)**

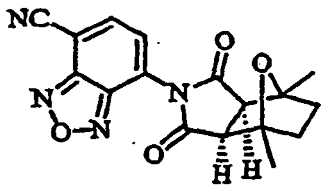

To a solution of compound **422C** (42 mg, 0.11 mmol) in DMA (1 mL) was added CuCN (20 mg, 0.22 mmol) and the resulting mixture was heated to 150°C for 5 h. The mixture was allowed to cool to rt and partitioned between EtOAc and aqueous NaCN solution (5 g/50 mL). The layers were separated and the aqueous layer was extracted once with EtOAc. The combined organic phases were dried over $Na_2SO_4$, concentrated and purified by flash chromatography (silica gel, EtOAc in hexanes, 20 to 70%) to give compound **423** (13 mg, 35%) as a yellow oil. HPLC: 99% at 2.66 min (retention time) (YMC S5 ODS column 4.6 x 50 mm Ballistic, 10-90% aqueous methanol over 4 minutes containing 0.2% $H_3PO_4$, 4 mL/min, monitoring at 220 nm), MS (ES): m/z 396.9 $[M-H+OAc]^-$.

**Example 424**

**(3aα,4β,7β,7aα)-7-[Octahydro-4,7-dimethyl-1,3-dioxo-4,7-epoxy-2H-isoindol-2-yl]-2,1,3-benzothiadiazole-4-carbonitrile (424B)**

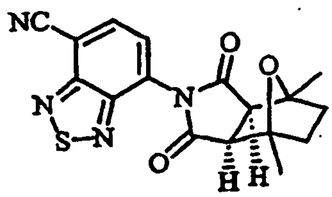

**A. 4-Cyano-7-amino-benzothiadiazole (424A)**

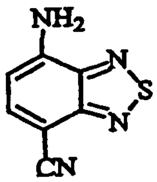

A solution of 2-cyano-5-nitrophenylenediamine (78 mg, 0.44 mmol, prepared as described in WO 0076501) in $SOCl_2$ (2 mL) was heated to reflux for 3 h. The resulting mixture was allowed to cool to rt and was then poured into ice/ water. $CH_2Cl_2$ was added, the layers were separated and the aqueous layer was extracted twice with $CH_2Cl_2$. The combined organic phases were dried over $MgSO_4$, concentrated and purified by flash chromatography (silica gel, EtOAc in hexanes, 50%) to give 4-cyano-7-nitrobenzothiadiazole. This material was dissolved in AcOH (2 mL) containing EtOAc (1 mL) and $H_2O$ (0.2 mL) and heated to 70 °C. At this temperature $Fe^0$ powder (78 mg, 1.41 mmol) was added in one solid portion and the dark mixture was stirred for 20 min and then cooled to rt. The reaction mixture was filtered through Celite eluting with EtOAc, washed with sat. $Na_2CO_3$ solution, dried over $MgSO_4$ and concentrated. Purification was achieved by flash chromatography (silica gel, EtOAc in hexanes, 20 to 70%) to yield compound 424A (47 mg, 61 %) as a brown solid.

**B. (3aα,4β,7β,7aα)-7-[Octahydro-4,7-dimethyl-1,3-dioxo-4,7-epoxy-2H-isoindol-2-yl]-2,1,3-benzothiadiazole-4-carbonitrile (424B)**

A mixture of compound **424A** (35 mg, 0.20 mmol), compound **20A** (45 mg, 0.23 mmol), $MgSO_4$ (60 mg, 0.50 mmol), $Et_3N$ (139 μL, 1.0 mmol) and DME (200 μL) was placed in a sealed tube and heated to 135 °C for 14 h. After cooling to rt the mixture was filtered through Celite eluting with MeOH to yield a dark solid which was purified by a combination of flash chromatography (silica gel, EtOAc in hexanes, 10 to 50%) and reverse phase preparative HPLC (YMC S5 ODS 20 x 100 mm eluting with 27-100% aqueous methanol over 10 min containing 0.1% TFA, 20 mL/min) furnishing compound **424B** (36 mg, 51 %) as a yellow solid. HPLC: 98% at 2.45 min (retention time) (YMC S5 ODS column 4.6 x 50 mm Ballistic, 10-90% aqueous methanol over 4 minutes containing 0.2% $H_3PO_4$, 4 mL/min, monitoring at 220 nm), MS (DCI): m/z 355.0 $[M+H]^+$.

**Example 425**

**(3aα,4β,7β,7aα)-N-[2-[2-(4-Cyano-1-naphthalenyl)octahydro-7-methyl-1,3-dioxo-4,7-epoxy-4H-isoindol-4-yl] ethyl]-4-fluoro-N-methylbenzamide (425B)**

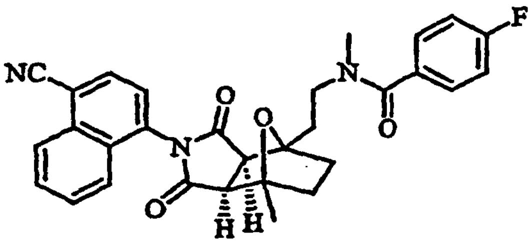

**A. 4-Fluoro-N-methyl-N-[2-(5-methyl-furan-2-yl)-ethyl]-benzamide (425A)**

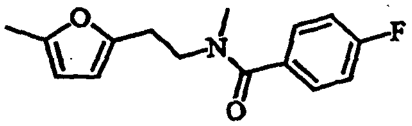

NaH (60% dispersion in oil, 65 mg, 1.63 mmol) was added portion-wise to a solution of 4-fluoro-N-[2-(5-metbyl-2-furanyl)ethyl]benzamide (269 mg, 1.09 mmol, 237A) in THF (5 mL). After gas evolution ceased, iodomethane (0.14 mL, 2.18 mmol) was added drop-wise. Once HPLC analysis showed the reaction to be 50% complete, the mixture was concentrated under reduced pressure and resubjected to the above conditions. After all the starting material was consumed, $H_2O$ was added and the resulting mixture was extracted with EtOAc (2 x 5 mL). The combined organic layers were dried over $Na_2SO_4$ and concentrated under reduced pressure. Purification by flash chromatography eluting with 20% acetone/$CHCl_3$ gave 238 mg (84%) of compound 425A. HPLC: 98% at 2.94 min (retention time) (Phenomenex-prime S5-C18 column 4.6 x 50 mm eluting with 10-90% aqueous methanol over 4 minutes containing 0.2% phosphoric acid, 4 mL/min, monitoring at 220 nm), MS (ES): m/z [M+H] = 262.38.

**B. (3aα,4β,7β,7aα)-N-[2-[2-(4-Cyano-1-naphthalenyl)octahydro-7-methyl-1,3-dioxo-4,7-epoxy-4H-isoindol-4-yl]ethyl]-4-fluoro-N-methylbenzamide (425B)**

A solution of compound 425A (183 mg, 0.75 mmol) and 4-(2,5-dihydro-2,5-dioxo-1H-1-yl)-1-naphthalenecarbonitrile (174 mg, 0.75 mmol) in benzene (1 mL) was heated at 60°C for 15 hr. The reaction mixture was concentrated under reduced pressure to give 357 mg crude intermediate. The crude intermediate (156 mg) was dissolved in EtOAc (6 mL) and 10% Pd/C (16 mg) was added and the mixture was stirred under a hydrogen balloon overnight The reaction mixture was filtered through a pad of celite and concentrated under reduced pressure. Purification by reverse phase preparative chromatography (YMC S5 ODS 20 x 100 mm, 20-100% aqueous methanol over 15 minutes containing 0.1% TFA, 20 mL/min, monitoring at 220 nm) gave 160.3 mg (72%) of compound 425B as an off-white solid. HPLC: 99% at 3.23 min (retention time) (Phenomenex-prime S5-C18 column 4.6 x 50 mm eluting with 10-90% aqueous methanol over 4 minutes containing 0.2% phosphoric acid, 4 mL/min, monitoring at 220 nm), MS (ES): m/z [M+H] = 512.19.

**<u>Example 426</u>**

**(3aα,4β,7β,7aα)-Hexahydro-4,7-dimethyl-2-[4-(2,2,2-trifluoro-1-hydroxyethyl)phenyl]-4,7-epoxy-1H-isoindole-1,3(2H)-dione (426B)**

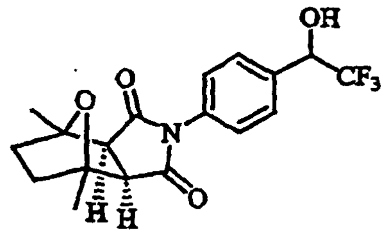

**A. 1-(4-Amino-phenyl)-2,2,2-trifluoro-ethanol (426A)**

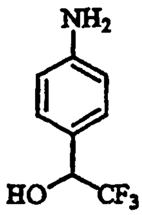

Compound 426A was made according to the procedure described in Stewart, R. *et. al., Can. J. Chem.* 58,2491-2496 (1980). $NaBH_4$ (47 mg, 1.235 mmol) was added to a solution of p-aminotrifluoroacetophenone (155.7 mg, 0.823 mmol, synthesized as described by Klabunde, K. J., *et al., J. Org. Chem.* 35, 1711-1712 (1970)) in isopropanol (3 mL) at rt. After 30 min the reaction mixture was quenched with phosphate buffer (pH 7.2), diluted with $H_2O$ and extracted with EtOAc (2 x 10 mL). The combined organic layers were dried over $Na_2SO_4$ and concentrated under reduced pressure to give 154 mg (98%) of compound 426A as a tan solid. The material was used directly in the next step without purification. HPLC: 99% at 0.42 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z [M+H] = 192.13.

**B. (3aα,4β,7β,7aα)-Hexahyaro-4,7-dimethyl-2-[4-(2,2,2-trifluoro-1-hydroxyethyl)phenyl]-4,7-epoxy-1H-isomdole-1,3(2H)-dione (426B)**

A mixture of compound 426A (75.3 mg, 0.394), compound 20A (51.5 mg, 0.262 mmol), triethylamine (0.15 mL) and $MgSO_4$ (50 mg) in toluene (1 mL) was heated in a sealed tube to 135°C for 15 hr. The mixture was filtered and concentrated under reduced pressure. Purification by reverse phase preparative chromatography (YMC S5 ODS 20 x 100 mm, 20-100% aqueous methanol over 15 minutes containing 0.1% TFA, 20 mL/min, monitoring at 220 nm) gave 63.1 mg (65%) of compound 426B as a white solid. HPLC: 98% at 2.49 min (retention time) (Phenomenex-prime S5-C18 column 4.6 x 50 mm eluting with 10-90% aqueous methanol over 4 minutes containing 0.2% phosphoric acid, 4 mL/min, monitoring at 220 nm), MS (ES): m/z [M+H] = 370.16.

**Example 427**

**(3aα,4β,7β,7aα)-4-[4-[2-[[(1,1-Dimethylethyl)dimethylsilyl]oxy]ethyl]-1,3,3a,4,7,7a-hexahydro-7-methyl-1,3-dioxo-4,7-epoxy-2H-isoindol-2-yl]-2-(trifluoromethyl)benzonitrile & (3aα,4α,7α,7aα)-4-[4-[2-[[(1,1-Dimethylethyl) dimethylsilyl]oxy]ethyl]-1,3,3a,4,7,7a-hexahydro-7-methyl-1,3-dioxo-4,7-epoxy-2H-isoindol-2-yl]-2-(trifluor-omethyl)benzonitrile (427i & 427ii)**

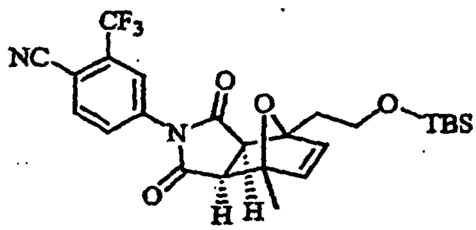 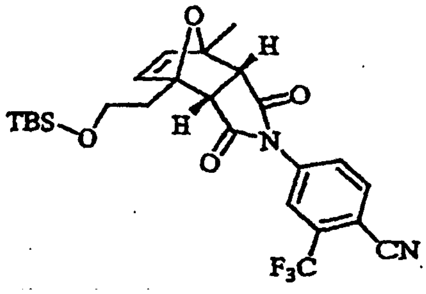

Compound **204A** (2.00 g, 8.50 mmol) and 4-(2,5-Dihydro-2,5-dioxo-1H-pyrrol-1-yl)-2-trifluoromethylbenzonitrile (1.50 g, 5.60 mmol) were mixed in benzene (5.0 mL) and heated at 60 °C for 14 h, then cooled to 25 °C. The solvent was removed at 40°C under vacuum for 1 h to give the crude material which was purified by flash chromatography on $SiO_2$ eluting with 0.5% $EtOAc/CH_2Cl_2$ to give 2.0 g of compound **427i** and 1.3 g of compound **427ii,** both as light brown solids. Compound **427i:** HPLC: 95% at 4.200 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 507.1 $[M+H]^+$. Compound **427ii:** HPLC: 95% at 4.20 min (retention time) (YMC S5 ODS column 4.6 x 50 mm eluting with 10-90% aqueous methanol over 4 minutes containing 0.2% phosphoric acid, 4 mUmin, monitoring at 220 nm), MS (ES): m/z 507.1 $[M+H]^+$.

**<u>Example 428</u>**

**<u>[3aR-(3aα,4β,5β,7β,7aα)]-4-[7-[2-[[(1,1-Dimethylethyl)dimethylsilyl]oxy]ethyl]octahydro-5-hydroxy-4-methyl-1,3-dioxo-4,7-epoxy-2H-isoindol-2-yl]-2-(trifluoromethyl)benzonitrile & [3aS-(3aα,4β,5β,7β,7aα)]-4-[7-[2-[[(1.1-Dimethylethyl)dimethylsilyl]oxy]ethyl]octahydro-5-hydroxy-4-methyl-1,3-dioxo-4,7-epoxy-2H-isoindol-2-yl]-2-(trifluoromethyl)benzonitrile (428i & 428ii)</u>**

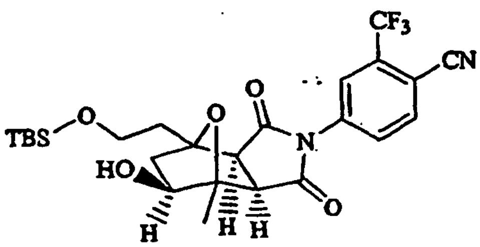 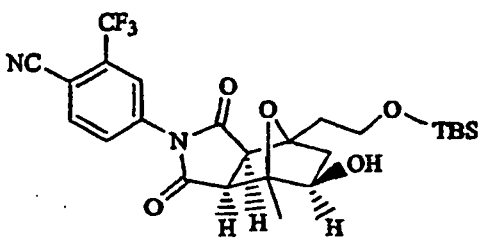

Compound **427i** (1.40 g, 2.77 mmol) and $RhCl(PPh_3)_3$ (0.128 g, 0.14 mmol) were mixed in a flask. The flask was then evacuated and filled with argon three times, followed by the syringe addition of THF (3.0 mL). Once all particulates were dissolved, catecholborane (0.59 mL, 5.54 mmol) was added dropwise. The reaction mixture was stirred at 25°C under argon for 30 min, then cooled to 0°C. Phosphate buffer (pH=7, 20 mL) was added, followed by EtOH (10 mL), 30% $H_2O/H_2O$ (2 mL). The reaction mixture was stirred at 0°C for 3 h, then extracted with dichloromethane (3 x 25 mL). The combined organic layers were washed with 1 N NaOH (25 mL), 10% $Na_2SO_3$ (25 mL) and brine (25 mL). The crude material was then concentrated and purified by flash chromatography on $SiO_2$ eluting with 2% $EtOAc/CH_2Cl_2$ to 10% $EtOAc/CH_2Cl_2$ to give 0.63 g of a racemic mixture of compounds **428i & 428ii** as a light yellow solid. HPLC: 99% at 3.867 min (retention time) (YMC S5 ODS column 4.6 x 50 mm eluting with 10-90% aqueous methanol over 4 minutes containing 0.2% phosphoric acid, 4 mL/min, monitoring at 220 nm), MS (ES): m/z 525.1 [M+H]

The racemic mixture of compounds **428i & 428ii** was separated by normal phase preparative chiral HPLC using a Chiracel OD column (5 cm x 50 cm), eluting with 13% solvent B (EtOH) in solvent A (Hexane), flow rate: 50 mL/min. Compound **428i** eluted from 34 min to 38 min and compound **428ii** eluted from 44 min to 49 min. Enantiomeric excess was determined by chiral HPLC. Compound **428i:** >99% ee (12.576 min (retention time) (Chiralcel OJ column 4.6 x 250 mm eluting with isocratic 85% heptane / 15% MeOH/ethanol (1:1), 1 mL/min, monitoring at 220 nm, 40°C). Compound **428ii:** 99% ee (18.133 min (retention time) (Chiralcel OJ column 4.6 x 250 mm eluting with isocratic 85% heptane / 15% MeOH/ethanol (1:1), 1 mL/min, monitoring at 220 nm, 40°C).

The absolute configurations for compounds **428i & 428ii** were not established. For simplicity in nomenclature, compound **428i** is designated herein as having an "R" configuration and compound **428ii** as having an "S" configuration. Enantiomerically pure products derived from compound **428i** are designated herein as having a "R" configuration and enantiomerically pure products derived from compound **428ii** are designated herein as having an "S" configuration.

## Example 429

**[3aR-(3aα,4β,5β,7β,7aα)]-4-[Octahydro-5-hydroxy-7-(2-hydroxyethyl)-4-methyl-1,3-dioxo-4,7-epoxy-2H-isoindol-2-yl]-2-(trifluoromethyl)benzonitrile & [3aS-(3aα,4β,5β,7β,7aα)]-4-[Octahydro-5-hydroxy-7-(2-hydroxyethyl)-4-methyl-1,3-dioxo-4,7-epoxy-2H-isoindol-2-yl]-2-(trifluoromethyl)benzonitrile (429i & 429ii)**

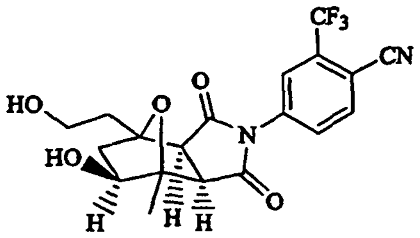
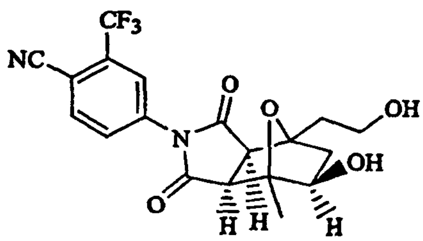

Compound **428i** (180 mg, 0.34 mmol) was dissolved in 2% HCl/EtOH (5.0 mL). After 30 min, saturated $NaHCO_3$ was added and the aqueous layer was extracted with dichloromethane (20 mL x 3), washed with brine and dried over $Na_2SO_4$ to give 135 mg of compound **429i** as a white solid. HPLC: 99% at 2.257 min (retention time) (YMC S5 ODS column 4.6 x 50 mm eluting with 10-90% aqueous methanol over 4 minutes containing 0.2% phosphoric acid, 4 mUmin, monitoring at 220 nm), MS (ES): m/z 411.1 $[M+H]^+$.

The above procedure was repeated with compound **428ii** to yield the desired diol compound **429ii** in similar yield.

## Example 430

**[3aR-(3aα,4β,5β,7β,7aα)]-4-[7-[2-[(5-Chloro-2-pyridinyl)oxy]ethyl]octahydro-5-hydroxy-4-methyl-1,3-dioxo-4,7-epoxy-2H-isoindol-2-yl]-2-(trifluoromethyl)benzonitrile (430)**

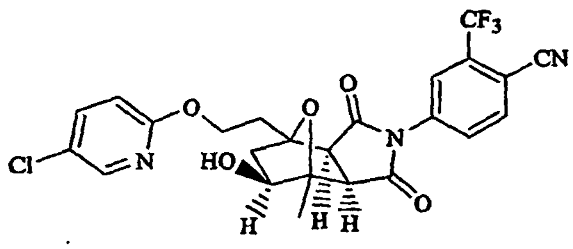

Triphenylphosphine (0.026 g, 0.098 mmol) and DBAD (0.023 g, 0.098 mmol) were mixed in THF (0.5 mL). After allowing the previous mixture to react for 15 min, 2-hydroxy-6-chloropyridine (0.016 g, 0.100 mmol) was added, the mixture was allowed to stir for 10 min and compound **429i** (0.020 g, 0.049 mmol) was added. The reaction mixture was stirred at 25°C for 2 h and then the crude material was purified by preparative TLC, eluting with 10% acetone/$CHCl_3$. to give 0.014 g of compound **430** as a light brown solid. HPLC: 100% at 3.370 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 522.08 $[M+H]^+$.

**Example 431**

**[3aS-(3aα,4β,5β,7β,7aα)]-4-[7-[2-[(5-Chloro-2-pyridinyl)oxy]ethyl]octahydro-5-hydroxy-4-methyl-1,3-dioxo-4,7-epoxy-2H-isoindol-2-yl]-2-(trifluoromethyl)benzonitrile (431)**

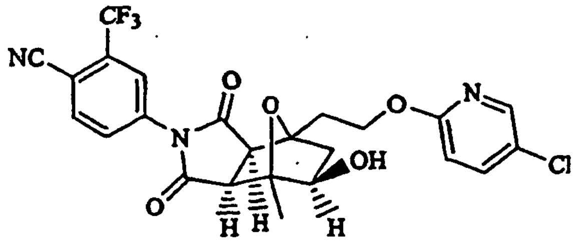

Triphenylphosphine (0.026 g, 0.098 mmol) and DBAD (0.023 g, 0.098 mmol) were mixed in THF (0.5 mL). After allowing the previous mixture to react for 15 min, 2-hydroxy-6-chloropyridine (0.016 g, 0.100 mmol) was added, the mixture was allowed to stir for 10 min and compound **429ii** (0.020 g, 0.049 mmol) was added. The reaction mixture was stirred at 25°C for 2 h and then the crude material was purified by preparative TLC, eluting with 10% acetone/$CHCl_3$, to give 0.015 g of compound **431** as a light brown solid. HPLC: 100% at 3.370 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 522.08 $[M+H]^+$.

**Example 432**

**(3aα,4β,7β,7aα)-2-(4-Cyano-1-naphthalenyl)octahydro-N-(2-hydroxyphenyl)-7-methyl-1,3-dioxo-4,7-epoxy-4H-isoindole-4-butanamide (432)**

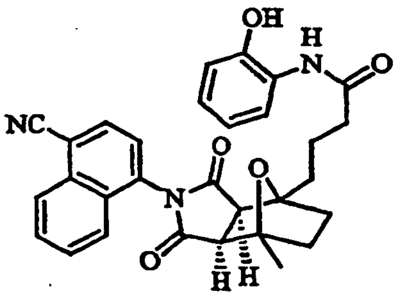

Compound **262** (0.100 g, 0.239 mmol) was dissolved in DMF (anhydrous, 1.5 mL), BOP (0.211 g, 0.478 mmol) was added followed by 2-aminophenol (0.052 g, 0.478 mmol) and N-methyl morpholine (0.052 mL, 0.478 mmol). The reaction mixture was stirred at 25°C under argon for 3 h, then the crude material was purified by reverse phase preparative-HPLC (YMC S5 ODS 20 x 100 mm, 20-100% aqueous methanol over 15 minutes containing 0.1% TFA, 20 mL/min, monitoring at 220 nm) to give 0.060 g of compound **432** as a light brown solid. HPLC: 100% at 3.037 min (retention time) (YMC S5 ODS column 4.6 x 50 mm eluting with 10-90% aqueous methanol over 4 minutes containing 0.1 % TFA, 4 mL/min, monitoring at 220 nm), MS (ES): m/z 510.34 $[M+H]^+$.

**Example 433**

**(3aα,4β,7β,7aα)-4-[4-[3-(2-Benzoxazolyl)propyl]octahydro-7-methyl-1,3-dioxo-4,7-epoxy-2H-isoindol-2-yl]-1-naphthalenecarbonitrile (433)**

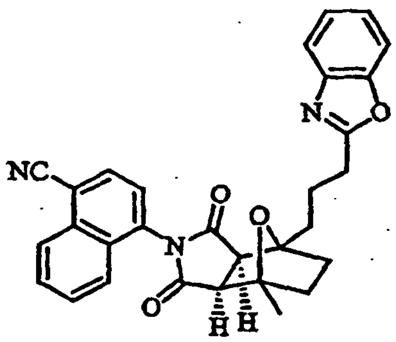

Triphenylphosphine (0.031 g, 0.118 mmol) and DBAD (0.027 g, 0.118 mmol) were mixed in THF (0.5 mL). After allowing the previous mixture to react for 15 min, compound **432** (0.030 g, 0.059 mmol) was added. The reaction mixture was stirred at 25°C for 2 h and then the crude material was purified by reverse phase preparative-HPLC (YMC S5-ODS 20 x 100 mm, 20-100% aqueous methanol over 15 minutes containing 0.1% TFA, 20 mL/min, monitoring at 220 nm) to give 0.018 g of compound **433** as a light brown solid. HPLC: 100% at 3.357 min (retention time) (YMC S5 ODS column 4.6 x 50 mm eluting with 10-90% aqueous methanol over 4 minutes containing 0.1% TFA, 4 mUmin, monitoring at 220 nm), MS (ES): m/z 492.37 $[M+H]^+$.

**Example 434**

**(3aα,4β,5β,7β,7aα)-4-[4-Ethyloctahydro-5-hydroxy-7-(2-hydroxyethyl)-1,3-dioxo-4,7-epoxy-2H-isoindol-2-yl]-1-naphthalenecarbonitrile (434C)**

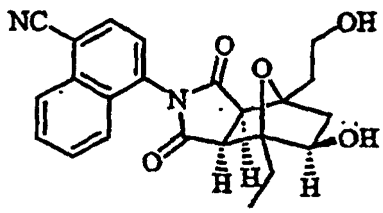

**A. tert-Butyl-[2-(5-ethyl-furan-2-yl)-ethoxy]-dimethyl-silane (434A)**

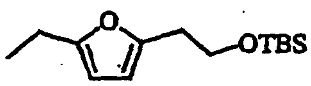

Imidazole (255 mg, 3.75 mmol) and TBSCl (414 mg, 2.75 mmol) were added to the solution of 245A (350 mg, 2.5 mmol) in DMF (4 mL). The mixture was stirred at rt for 15 hr and then 100 mg (0.66 mmol) of additional TBSCl were added to drive the reaction to completion. After stirring for an additional hour, the reaction mixture was diluted with diethylether (100 mL) and washed with water (20 mL), 1 N HCl (20 mL), water (20 mL) and brine (20 mL). The organic layer was dried over $Na_2SO_4$ and concentrated under reduced pressure to give 509 mg of compound 434A (80.3%) as a yellow oil.

**B. (3aα,4β,7β,7aα)-4-[4-[2-[[(1,1-Dimethylethyl)-dimethylsilyl]oxy]ethyl]-4-ethyl-1,3,3a,4,7,7a-hexahydro-1,3-dioxo-4,7-epoxy-2H-isoindol-2-yl]-1-naphthalenecarbonitrile (434B)**

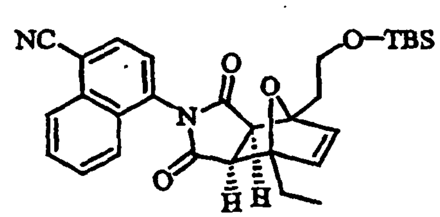

A solution of compound **434A** (509 mg, 2.00 mmol) and 4-(2,5-dihydro-2,5-dioxo-1H-1-yl)-1-naphthalenecarbonitrile (498 mg, 2.00 mmol) in benzene (2 mL) was heated at 60°C for 18 h. The reaction mixture was concentrated under reduced pressure to give 992 mg (99%) of crude compound **434B,** which was used directly in the next step without further purification.

**C. (3aα,4β,5β,7β,7aα)-4-[4-Ethyloctahydro-5-hydroxy-7-(2-hydroxyethyl)-1,3-dioxo-4,7-epoxy-2H-isoindol-2-yl]-1-naphthalenecarbonitrile (434C)**

A mixture of compound **434B** (992 mg, 1.98 mmol) and $RhCl_2(PPh_3)_3$ (183 mg, 0.198 mmol) was evacuated and filled with argon (3x). THF (20 mL) was added and once all particulates had dissolved, catecholborane (0.42 mL, 3.96 mmol) was slowly added dropwise. When the formation of product ceased, as was determined by HPLC, the reaction mixture was cooled to 0°C and quenched with phosphate buffer (34 mL, pH 7.2) followed by the addition of EtOH (19 mL) and $H_2O_2$ (2.9 mL, 30% aq sol). After 2 h, additional phosphate buffer (6.8 mL, pH 7.2), EtOH (3.8 mL) and $H_2O_2$ (0.6 mL) were added. The reaction mixture was stirred at rt for 3 h. Once the boronate intermediate was consumed, the mixture was extracted with $CH_2Cl_2$ (300 mL) and the combined organic layers were washed with 1N NaOH, 10% aq $NaHSO_3$ and brine. The combined organic layers were dried over $Na_2SO_4$. Purification by flash chromatography on silica gel eluting with 10% MeOH/$CH_2Cl_2$ gave 75 mg (9.3%) of compound **434C** as a gray solid. HPLC conditions: 97% at 2.43 min (Phenomenex-prime S5-C18 column 4.6 x 50 mm, 10%-90% aqueous methanol over 4 minute gradient with 0.2% $H_3PO_4$, detecting at 220 nm). MS (ES): m/z 407.18 $[M+H]^+$.

**Example 435**

**(3aα,4β,5β,7β,7aα)-4-[7-[2-(4-Cyanophenoxy)ethyl]-4-ethyloctahydro-5-hydroxy-1,3-dioxo-4,7-epoxy-2H-isoindol-2-yl]-1-naphthalenecarbonitrile (435)**

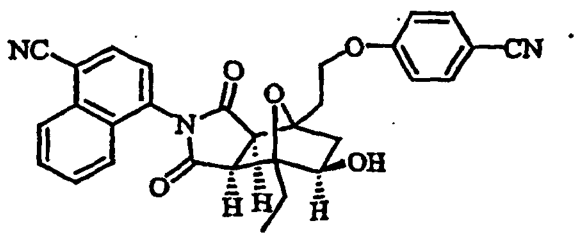

DBAD (39.6 mg, 0.172 mmol) was added to a solution of $PPh_3$ (45.1 mg, 0.172 mmol) in THF (0.8 mL). After stirring for 10 min, 4-cyanophenol (20.5 mg, 0.172 mmol) was added and the reaction mixture was stirred for an additional 5 min. Compound **434C** (25.0 mg, 0.062 mmol) was added and the mixture was stirred at rt for 2 h. The reaction mixture was concentrated under reduced pressure. Purification by Prep TLC eluting with 10% acetone/$CHCl_3$ gave 18.1 mg (0.036 mmol, 57.6%) of compound **435.** HPLC condittons: 96% at 3.15 min (YMC S5 ODS 4.6 x 50 mm, 10%-90% aqueous methanol over 4 minute gradient with 0.2% $H_3PO_4$, detecting at 220 nm). MS (ES): m/z 508.14 $[M+H]^+$.

**Example 436**

**(3aα,4β,7β,7aα)-2-(4-Cyano-1-naphthylenyl)octahydro-N-(2-hydroxyphenyl)-7-methyl-1,3-dioxo-4,7-expoxy-4H-isoindole-4-ethanamide (436)**

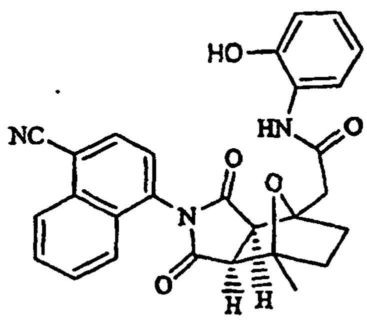

Compound **234B** (0.100 g, 0.256 mmol) was dissolved in DMF (anhydrous, 1.5 mL), BOP (0.225 g, 0.51 mmol) was added followed by 2-aminophenol (0.056 g, 0.51 mmol) and N-methyl morpholine (0.056 mL, 0.51 mmol). The reaction mixture was stirred at 25°C under argon for 3 h, then the crude material was purified by reverse phase preparative-HPLC (YMC S5 ODS 20 x 100 mm, 20-100% aqueous methanol over 15 minutes containing 0.1% TFA, 20 mUmin, monitoring at 220 nm) to give 0.078 g of compound **436** as a light brown solid. HPLC: 100% at 3.037 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 482.34 $[M+H]^+$.

**Example 437**

**(3aα,4β,7β,7aα)-4-[4-(2-Benzoxazolylmethyl)octahydro-7-methyl-1,3-dioxo-4,7-epoxy-2H-isoindol-2-yl]-1-naphthalenecarbonitrile (437)**

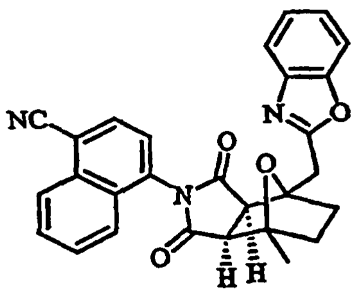

Triphenylphosphine (0.082 g, 0.312 mmol) and DBAD (0.072 g, 0.312 mmol) were mixed in THF (0.5 mL). After allowing the previous mixture to react for 15 min, compound **436** (0.075 g, 0.156 mmol) was added. The reaction mixture was stirred at 25°C for 2 h and then the crude material was purified by reverse phase preparative-HPLC (YMC S5 ODS 20 x 100 mm, 20-100% aqueous methanol over 15 minutes containing 0.1% TFA, 20 mL/min, monitoring at 220 nm) to give 0.052 g of compound **437** as a light brown solid. HPLC: 100% at 3.443 min (retention time) (YMC S5 ODS column 4.6 x 50 mm eluting with 10-90% aqueous methanol over 4 minutes containing 0.1% TFA, 4 mL/min, monitoring at 220 nm), MS (ES): m/z 464.18 $[M+H]^+$.

**Example 438**

**(3aα,4β,7β,7aα)-Hexahydro-4,7-dimethyl-2-[4-[2,2,2-trifluoro-1-hydroxy-1-(trifluoromethyl)ethyl]phenyl]-4,7-epoxy-1H-isoindole-1,3(2H)-dione (438)**

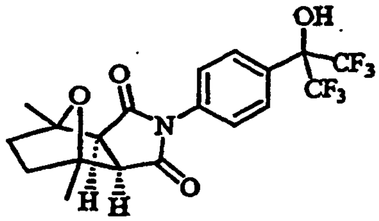

A mixture of 2-(4'-aminophenyl)-1,1,1,3,3,3-hexafluoro-2-propanol (95.7 mg, 0.369 mmol), compound 20A (48.3 mg, 0.246 mmol), triethylamine (0.15 mL) and $MgSO_4$ (50 mg) in toluene (1 mL) was heated in a sealed tube to 135°C overnight. The mixture was filtered and concentrated under reduced pressure. Purification by reverse phase preparative chromatography (YMC S5 ODS 20 x 100 mm, 20-100% aqueous methanol over 15 minutes containing 0.1% TFA, 20 mL/min, monitoring at 220 nm) gave 44.0 mg (41 %) of compound **438** as a white solid. HPLC: 99% at 3.10 min (retention time) (Phenomenex-prime S5-C18 column 4.6 x 50 mm eluting with 10-90% aqueous methanol over 4 minutes containing 0.2% phosphoric acid, 4 mL/min, monitoring at 220 nm), MS (ES): m/z [M+H] = 438.14.

**Examples 439 to 454**

Additional compounds of the present invention were prepared by procedures analogous to those described above. The compounds of Examples 439 to 454 have the following structure (L is a bond):

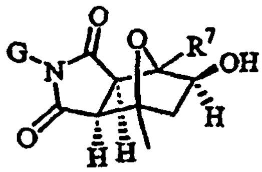

where G, $R^7$, the compound name, retention time, molecular mass, and the procedure employed, are set forth in Table 9. The absolute configuration for the following compounds was not determined. For simplicity in nomenclature, compound **243Di** is designated herein as having an "S" configuration and compound **243Dii** as having an "R" configuration. Enantiomerically pure products derived from compound **243Di** are designated herein as having an "S" configuration and enantiomerically pure products derived from compound 243Dii are designated herein as having an "R" configuration. Similarly, compound 428i is designated herein as having an "S" configuration and compound 428il as having an "R" configuration. Enantiomerically pure products derived from compound 428i are designated herein as having an "S" configuration and enantiomerically pure products derived from compound 428ii are designated herein as having an "R" configuration.

The chromatography techniques used to determine the compound retention times of Table 9 are as follows: LCMS = YMC S5 ODS column, 4.6 x 50 mm eluting with 10-90% MeOH/$H_2O$ over 4 minutes containing 0.1% TFA; 4 mL/min, monitoring at 220 nm. LCMS* = YMC S5 ODS column, 4.6 x 50 mm eluting with 10-90% MeOH/$H_2O$ over 2 minutes containing 0.1 % TFA; 4 mL/min, monitoring at 220 mn. LC = YMC S5 ODS column 4.6 x 50 mm eluting with 10-90% MeOH/$H_2O$ over 4 minutes containing 0.2% phosphoric acid, 4 mL/min, monitoring at 220 nm. The molecular mass of the compounds listed in Table 9 was determined by MS (ES) by the formula m/z.

## Table 9

| Ex. No | G | R7 | Compound Name | Retention Time Min./ Molecular Mass | Procedure of Ex. |
|---|---|---|---|---|---|
| 439 | 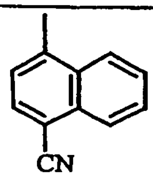 | 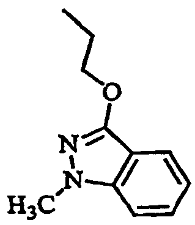 | [3aR-(3aα,4β,5β,7β,7aα)]-4-[Octahydro-5-hydroxy-4-methyl-7-[2-[(1-methyl-1H-indazol-3-yl)oxy]ethyl]-1,3-dioxo-4,7-epoxy-2H-isoindol-2-yl]-1-naphthalenecarbonitrile | 3.33 LC 523.3 $[M+H]^+$ | 251, 253 |
| 440 | 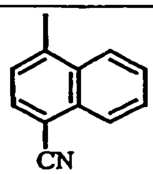 | 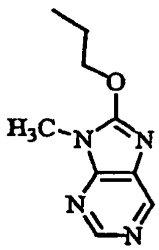 | [3aR-(3aα,4β,5β,7β,7aα)]-4-[Octahydro-5-hydroxy-4-methyl-7-[2-[(9-methyl-9H-purin-8-yl)oxy]ethyl]-1,3-dioxo-4,7-epoxy-2H- | 2.34 LC 525.2 $[M+H]^+$ | 251, 253 |

| Ex. No | G | R⁷ | Compound Name | Retention Time Min./ Molecular Mass | Procedure of Ex. |
|---|---|---|---|---|---|
| | | | isoindol-2-yl]-1-naphthalenecarbonitrile | | |
| 441 | 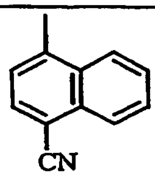 | 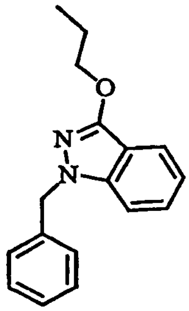 | [3aR-(3aα,4β,5β,7β,7aα)]-4-[Octahydro-5-hydroxy-4-methyl-1,3-dioxo-7-[2-[[1-(phenylmethyl)-1H-indazol-3-yl]oxy]ethyl]-4,7-epoxy-2H-isoindol-2-yl]-1-naphthalenecarbonitrile | 3.73 LC | 243Dii, 244Dii |
| 442 | 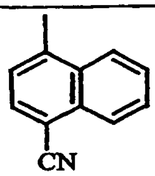 | 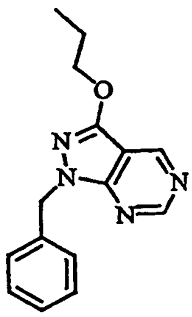 | [3aR-(3aα,4β,5β,7β,7aα)]-4-[Octahydro-5-hydroxy-4-methyl-1,3-dioxo-7-[2-[[1-(phenylmethyl) | 3.37 LC | 251, 253 |

| Ex. No | G | R7 | Compound Name | Retention Time Min./ Molecular Mass | Procedure of Ex. |
|---|---|---|---|---|---|
| | | | -1H-pyrazolo[3,4-d]pyrimidin-3-yl]oxy]ethyl]-4,7-epoxy-2H-isoindol-2-yl]-1-naphthalenecarbonitrile | | |
| 443 | 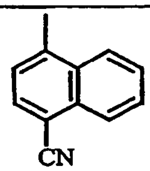 | 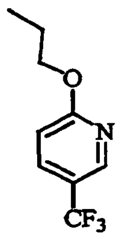 | [3aS-(3aα,4β,5β,7β,7aα)]-4-[Octahydro-5-hydroxy-4-methyl-1,3-dioxo-7-[2-[[5-(trifluoromethyl)-2-pyridinyl]oxy]ethyl]-4,7-epoxy-2H-isoindol-2-yl]-1-naphthalenecarbonitrile | 3.45 LC 538.23 $[M+H]^+$ | 243Di, 244Di |

| Ex. No | G | R⁷ | Compound Name | Retention Time Min./ Molecular Mass | Procedure of Ex. |
|---|---|---|---|---|---|
| 444 | 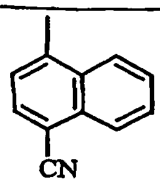 | 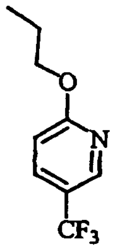 | [3aR-(3aα,4β,5β,7β,7aα)]-4-[Octahydro-5-hydroxy-4-methyl-1,3-dioxo-7-[2-[[5-(trifluoromethyl)-2-pyridinyl]oxy]ethyl]-4,7-epoxy-2H-isoindol-2-yl]-1-naphthalenecarbonitrile | 3.46 LC 538.24 $[M+H]^+$ | 243Dii, 244Dii |
| 445 | 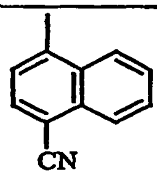 | 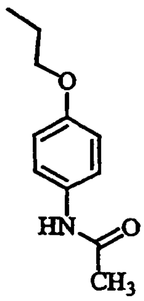 | [3aR-(3aα,4β,5β,7β,7aα)]-N-[4-[2-[2-(4-Cyano-1-naphthalenyl)octahydro-5-hydroxy-4-methyl-1,3-dioxo-4,7-epoxy-7H-isoindol-7-yl]ethoxy]phen | 2.747 LC 526.28 $[M+H]^+$ | 243Dii, 244Dii |

| Ex. No | G | $R^7$ | Compound Name | Retention Time Min./ Molecular Mass | Procedure of Ex. |
|---|---|---|---|---|---|
| | | | yl]acetamide | | |
| 446 | 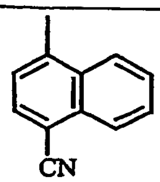 | 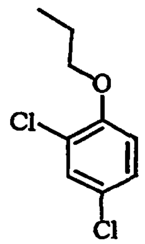 | [3aR-(3aα,4β,5β,7β,7aα)]-4-[7-[2-(2,4-Dichlorophenoxy)ethyl]octahydro-5-hydroxy-4-methyl-1,3-dioxo-4,7-epoxy-2H-isoindol-2-yl]-1-naphthalenecarbonitrile | 3.71 LC 537.17 $[M+H]^+$ | 243Dii, 244Dii |
| 447 | 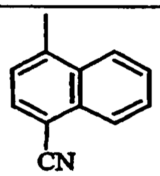 | 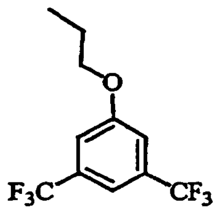 | [3aR-(3aα,4β,5β,7β,7aα)]-4-[7-[2-[3,5-Bis(trifluoromethyl)phenoxy]ethyl]octahydro-5-hydroxy-4-methyl-1,3-dioxo-4,7-epoxy-2H-isoindol-2-yl]- | 3.89 LC 605.25 $[M+H]^+$ | 243Dii, 244Dii |

| Ex. No | G | R⁷ | Compound Name | Retention Time Min./ Molecular Mass | Procedure of Ex. |
|---|---|---|---|---|---|
| | | | 1-naphthalenecarbonitrile | | |
| 448 | 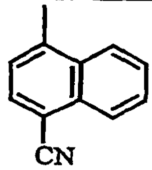 | 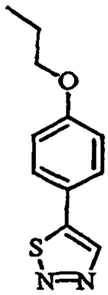 | [3aS-(3aα,4β,5β,7β,7aα)]-4-[Octahydro-5-hydroxy-4-methyl-1,3-dioxo-7-[2-[4-(1,2,3-thiadiazol-5-yl)phenoxy]ethyl]-4,7-epoxy-2H-isoindol-2-yl]-1-naphthalenecarbonitrile | 3.14 LC 553.1 [M+H]+ | 243Di, 244Di |
| 449 | 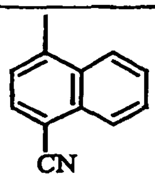 | 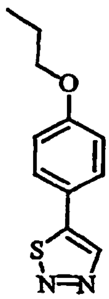 | [3aR-(3aα,4β,5β,7β,7aα)]-4-[Octahydro-5-hydroxy-4-methyl-1,3-dioxo-7-[2-[4-(1,2,3-thiadiazol-5- | 3.15 LC 553.23 [M+H]+ | 243Dii, 244Dii |

| Ex. No | G | R⁷ | Compound Name | Retention Time Min./ Molecular Mass | Procedure of Ex. |
|---|---|---|---|---|---|
| | | | yl)phenoxy]ethyl]-4,7-epoxy-2H-isoindol-2-yl]-1-naphthalenecarbonitrile | | |
| 450 | 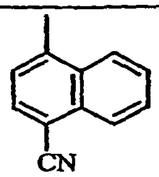 | 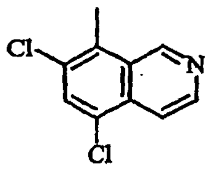 | [3aR-(3aα,4β,5β,7β,7aα)]-4-[7-[2-[(5,7-Dichloro-8-quinolinyl)oxy]ethyl]octahydro-5-hydroxy-4-methyl-1,3-dioxo-4,7-epoxy-2H-isoindol-2-yl]-1-naphthalenecarbonitrile, trifluoroacetate (1:1) | 3.70 LC 588.26 [M+H]⁺ | 243Dii, 244Dii |
| 451 | 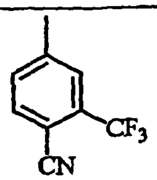 | 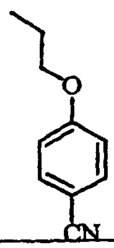 | [3aS-(3aα,4β,5β,7β,7aα)]-4-[7-[2-(4- | 3.087 LC 512.13 [M+H]⁺ | 431 |

| Ex. No | G | R7 | Compound Name | Retention Time Min./ Molecular Mass | Procedure of Ex. |
|---|---|---|---|---|---|
| | | | Cyanophenoxy)ethyl]octahydro-5-hydroxy-4-methyl-1,3-dioxo-4,7-epoxy-2H-isoindol-2-yl]-2-(trifluoromethyl)benzonitrile | | |
| 452 | 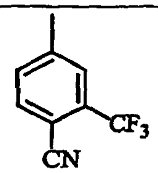 | 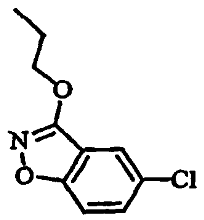 | [3aS-(3aα,4β,5β,7β,7aα)]-4-[7-[2-[(5-Chloro-1,2-benzisoxazol-3-yl)oxy]ethyl]octahydro-5-hydroxy-4-methyl-1,3-dioxo-4,7-epoxy-2H-isoindol-2-yl]-2-(trifluoromethyl)benzonitrile | 3.563 LC 562.08 [M+H]+ | 431 |

| Ex. No | G | R⁷ | Compound Name | Retention Time Min./ Molecular Mass | Procedure of Ex. |
|---|---|---|---|---|---|
| 453 | 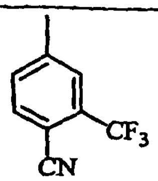 | 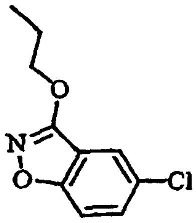 | [3aR-(3aα,4β,5β,7β,7aα)]-4-[7-[2-[(5-Chloro-1,2-benzisoxazol-3-yl)oxy]ethyl]octahydro-5-hydroxy-4-methyl-1,3-dioxo-4,7-epoxy-2H-isoindol-2-yl]-2-(trifluoromethyl)benzonitrile | 3.57 LC 562.08 $[M+H]^+$ | 430 |
| 454 | 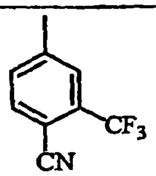 | 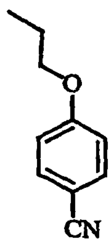 | [3aR-(3aα,4β,5β,7β,7aα)]-4-[7-[2-(4-Cyanophenoxy)ethyl]octahydro-5-hydroxy-4-methyl-1,3-dioxo-4,7-epoxy-2H-isoindol-2-yl]- | 3.087 LC 512.08 $[M+H]^+$ | 430 |

| Ex. No | G | R7 | Compound Name | Retention Time Min./ Molecular Mass | Procedure of Ex. |
|---|---|---|---|---|---|
| | | | 2-(trifluoromethyl)benzonitrile | | |
| | | | | | |

## Examples 455 to 457

Additional compounds of the present invention were prepared by procedures analogous to those described above. The compounds of Examples 455 to 457 have the following structure (L is a bond):

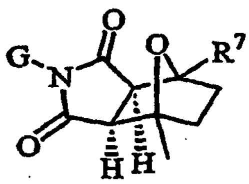

where G, $R^7$, the compound name, retention time, molecular mass, and the procedure employed, are set forth in **Table 10.** The absolute configuration for the following compounds was not determined. For simplicity in nomenclature, compound **238i** is designated herein as having an "R" configuration and compound **238ii** as having an "S" configuration. Enantiomerically pure products derived from compound **238i** are designated herein as having an "R" configuration and enantiomerically pure products derived from compound **238ii** are designated herein as having an "S" configuration.

The chromatography techniques used to determine the compound retention times of **Table 10** are as follows: **LCMS** = YMC S5 ODS column, 4.6 x 50 mm eluting with 10-90% MeOH/$H_2O$ over 4 minutes containing 0.1% TFA; 4 mL/min, monitoring at 220 nm. **LCMS*** = YMC S5 ODS column, 4.6 x 50 mm eluting with 10-90% MeOH/$H_2O$ over 2 minutes containing 0.1 % TFA; 4 mL/min, monitoring at 220 nm. LC = YMC S5 ODS column 4.6 x 50 mm eluting with 10-90% MeOH/$H_2O$ over 4 minutes containing 0.2% phosphoric acid, 4 mL/min, monitoring at 220 nm. The molecular mass of the compounds listed in **Table 10** was determined by MS (ES) by the formula m/z.

## Table 10

| Ex. No | G | R⁷ | Compound Name | Retention Time Min./ Molecular Mass | Procedure of Ex. |
|---|---|---|---|---|---|
| 455 | 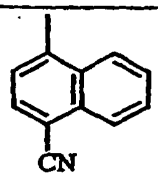 | 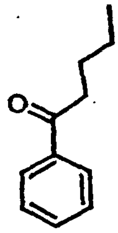 | (3aα,4β,5β,7β,7aα)-4-[Octahydro-4-methyl-1,3-dioxo-7-(4-oxo-4-phenylbutyl)-4,7-epoxy-2H-isoindol-2-yl]-1-naphthalenecarbonitrile | 3.53 LC 479.35 [M+H]⁺ | 265, 249 |
| 456 | 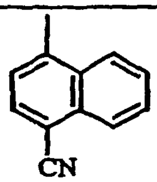 | 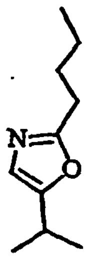 | (3aα,4β,5β,7β,7aα)-4-[Octahydro-4-methyl-7-[3-[5-(1-methylethyl)- | 3.547 LC 484.28 [M+H]⁺ | 248, 249 |

| Ex. No | G | R[7] | Compound Name | Retention Time Min./ Molecular Mass | Procedure of Ex. |
|---|---|---|---|---|---|
| | | | 2-oxazolyl]propyl]-1,3-dioxo-4,7-epoxy-2H-isoindol-2-yl]-1-naphthalenecarbonitrile | | |
| 457 | 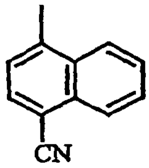 | 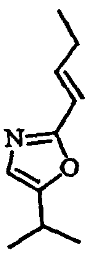 | [3aα,4β,5β,7β,7aα(E)]-4-[Octahydro-4-methyl-7-[3-[5-(1-methylethyl)-2-oxazolyl]-2-propenyl]-1,3-dioxo-4,7-epoxy-2H-isoindol-2-yl]-1-naphthalenecarbonitrile | 3.66 LC 482.28 [M+H]+ | 248, 249 |
| | | | | | |

## Example 458

(3aα,4β,5β,7β,7aα)-4-(Octahydro-5-hydroxy-4,7-dimethyl-1,3-dioxo-4,7-epoxy-2H-isoindol-2-yl)-2-(trifluoromethyl)benzonitrile & (3aα,4β,5α,7β,7aα)-4-(Octahydro-5-hydroxy-4,7-dimethyl-1,3-dioxo-4,7-epoxy-2H-isoindol-2-yl)-2-(trifluoromethyl)benzonitrile (221B & 222D)

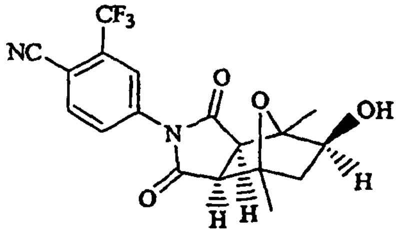 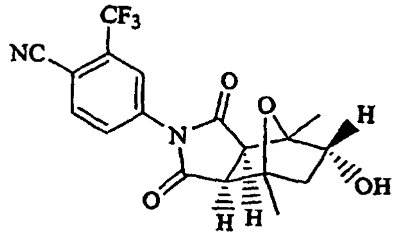

Compound **20B** was converted to compounds **221B** and **222D** (also synthesized as compounds **221B** and **222D**) by biotransformation.

Compound **20B** was hydroxylated by *Amycolatopsis orientalis* (ATCC 43491). A 1 ml culture from a frozen vial was used to inoculate 100 ml medium in a 500 mL portion Erlenmeyer flask and the flask was incubated at 28°C, at 200 rpm for 3 days. A 10 mL portion of this culture was used to inoculate 100 mL medium in a 500 ml Erlenmeyer flask and the flask was incubated at 28°C, at 200 rpm for 1 day. 10 mL portions of the 1-day culture were distributed to each of three 50 ml flasks. Compound **20B** (3 mg in 0.1 mL methanol) was added to each culture and the incubations were continued for 3 days. The culture broth in each flask was extracted with 20 mL ethyl acetate, and the pooled ethyl acetate extracts were evaporated to dryness at 40 °C under a nitrogen stream. The residue was dissolved in 1.2 mL methanol and analyzed by HPLC, LC/MS and LC/NMR. The solution contained 2.5 mg of remaining Compound **20B**, 1.6 mg of compound **221B**, and 1.3 mg of compound **222D**. MS and NMR analyses were in agreement with the structures shown above.

Medium: 0.5% toasted nutrisoy, 2% glucose, 0.5% yeast extract, 0.5% $K_2HPO_4$, 0.5% NaCl, adjusted to pH 7 with HCl (R. V. Smith and J. P. Rosazza, Arch. Biochem. Biophys., **161,** 551-558 (1974)

HPLC Analysis

Column: Phenomenex Luna C18, 150 x 2 mm, 5μm
mobile phase: solvent A: 95% 20 mM ammonium acetate pH 5.1, 5% acetonitrile
solvent B: 95% acetonitrile, 5% 20 mM ammonium acetate pH 5.1 linear gradient going from 100% solvent A to 5% solvent A in 25 minutes followed by equilibration at 100% solvent A for 8 minutes.
temperature: 40°C
detection: 250 nm
injection volume: 1 μL
retention times: compound **20B,** 20.8 min; compound **221B,** 16.5 min; compound **222D,** 17.8 min

HPLC Conditions

Chiral HPLC conditions were employed for the separation of enantiomers and achirel HPLC conditions were employed for the separation of diastereomers of the hydroxylated analogs of compound **20B** (i.e., compounds **221B** and **222D** and compounds **254i** and **254ii**)

Two methods were used under chiral HPLC conditions, reverse phase (RP) for chiral analysis of biotransformation products in biological samples and normal phase (NP) for non-biological samples.

| **Chiral RP-HPLC Condition** | |
|---|---|
| Column: | CHIRALPAK AD-R 4.6 x 250 mm, 10 μm |
| Temperature: | 40°C |
| Injection Volume: | 5 or 20 μL |
| Mobile Phase: | A: MeCN |
| | B: $H_2O$ |
| | Isocratic, 30% of A, 18 min. |
| Flow Rate: | 1 mL/min. |
| UV Detection: | 242 nm |

**Chiral NP-HPLC Condition**

| | |
|---|---|
| Column: | CHIRALPAK AD 4.6 x 250 mm, 10 μm |
| Temperature: | 25°C |
| Injection Volume: | 5 or 20 μL |
| Mobile Phase: | A: Heptane |
| | B: MeOH/Ethanol (1:1) |
| | Isocratic, 80% of A, 20 min. |
| Flow Rate: | 1 mL/min. |
| UV Detection: | 242 nm |

Under these conditions compounds **254i** and **254ii** had retention times of 8.5 minutes and 9.85 minutes, respectively.

Reverse phase HPLC was employed for the separation of the diastereomeric compounds **221B** and **222D:**

**Mobile Phase:**

| | |
|---|---|
| Solvent A: | 95% 20 mM ammonium acetate pH 5.1,5% acetonitrile |
| Solvent B: | 95 % acetonitrile, 5% 20 mM ammonium acetate pH 5.1 |

**Gradient:**

Linear gradient going from 100% solvent A to 5% solvent A in 25 minutes followed by equilibration at 100% solvent A for 8 minutes. Total run time of 36 minutes.

**Flow Rate:**

0.2 ml/min

**Column:**

Phenomenex Luna 5 micron $C_{18}$ 150 x 2.0 mm id

**Detection:**

UV detection at 242 nm

Under these conditions, compounds **221B** and **222D** had retention times of 18.983 min and 20.362 min, respectively.

**<u>Example 459</u>**

**(3aα,4β,5β,7β,7aα)-4-[Octahydro-5-hydroxy-7-(2-hydroxyethyl)-4-methyl-1,3-dioxo-4,7-epoxy-2H-isoindol-2-yl]-1-naphthalenecarbonitrile (459)**

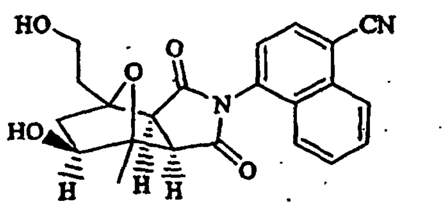

Compounds **223A** and **331** were converted to compound **459** by biotransformation.

**Microbial hydroxylation of compound 223A**

**1. Reaction**

To a 500 mL flask containing 100 ml of the transformation medium was added one frozen vial (approximately 2 ml) of *Streptomyces griseus* ATCC 10137. The transformation medium was prepared as follows: to a 2 L plastic beaker were added 20 g of dextrose, 5.0 g of yeast extract, 5.0 g of soybean meal, 5.0 g of sodium chloride, 5.0 g of potassium phosphate (diabasic) and 1 L of deionized water, and the mixture was stirred at room temperature for 3 to 30 min. The pH of the mixture was then adjusted to 7.0 with 1 N HCl or 1 N NaOH. The resulting mixture was dispensed into 500 ml flasks (100 ml per flask). The flasks were covered with Bio/Wrap and autoclaved at 121°C for 15 min. and cooled down to room temperature before use.
The culture was incubated at 28°C and at 250 rpm for 24 hours. Ten mL of the resulting culture was transferred to a 50 mL flask, to which 1 mg of compound 223A in 0.2 ml ethanol was added. The flask was incubated at 28°C and 250 rpm for 24 hours, and the reaction culture was extracted with EtOAc (10 ml). The EtOAc extract was dried under $N_2$ and the residue was dissolved in 1 ml of MeOH (reaction extract).

**2. Product analysis**

**HPLC:**

10 μL of the reaction extract was injected into HPLC column (YMC ODS-AQ C-18 column, 150 x 6.0 mm i.d.). The column was eluted with 1 mM HCl in water/$CH_3CN$ at 1.2 mL/min flow rate: 30 to 60% $CH_3CN$ over 8 min, 60 to 85% $CH_3CN$ over 0.5 min, 85% $CH_3CN$ for 1 min, 85 to 30% $CH_3CN$ over 0.5 min. The eluents were monitored at 300 nm. Two major peaks with about a 1 to 1 area ratio were observed, which had the same UV spectra as those of compounds **459** and **331**, and had retention times of 4.55 min and 7.23 min, respectively, matching the retention times of authentic samples of compound 459 (4.53 min) and compound 331 (7.2 min).

**LC/MS**

The reaction extract: two major UV peaks.

Peak 1, Tr 4.68 min: 391 $[M+H]^+$, 343, 319, 303, 289
Peak 2, Tr 5.35 min: 375 $[M+H]^+$, 345

**Authentic samples**

Compound **459,** Tr 4.82 min: 391 $[M+H]^+$, 343, 319, 289
Compound **331,** Tr 5.48 min: 375 $[M+H]^+$, 345

**Microbial hydroxylation of Compound 331**

To a 500 mL flask containing 100 ml of the transformation medium was added one frozen vial (approximately 2 ml) of *Streptomyces griseus* ATCC 10137. The transformation medium was prepared as follows: to a 2 L plastic beaker was added 20 g of dextrose, 5.0 g of yeast extract, 5.0 g of soybean meal, 5.0 g of sodium chloride, 5.0 g of potassium phosphate (dibasic) and one L of deionized water, and the mixture was stirred at room temperature for 3 to 30 min. The pH of the mixture was then adjusted to 7.0 with 1 N HCl or 1 N NaOH. The resulting mixture was dispensed into 500 mL flasks (100 ml per flask). The flasks were covered with Bio/Wrap and autoclaved at 121°C for 15 min. and cooled down to room temperature before use.
The culture was incubated at 28°C and 250 rpm for 3 days. One mL of the resulting culture was added to a 500 mL flask containing 100 mL of the transformation medium and the flask was incubated at 28°C and 250 rpm for 24 hours. Ten mL of the resulting culture was transferred to a 50 mL flask, to which 1 mg of compound 331 in 0.2 mL ethanol was added. The flask was incubated at 28°C and 250 rpm for 23 hours. HPLC analysis showed that the peak area ratio of compound 459 to compound **331** in the reaction culture was about 1.1/1.

**Example 460**

**(1aα,2β,2aα,5aα,6βb,6aα)-4-[2-[2-[[(1,1-Dimethylethyl)dimethylsilyl]oxy]ethyl]octahydro-6-methyl-3,5-dioxo-2,6-epoxy-4H-oxireno[f]isoindol-4-yl]-1-naphthalenecarbonitrile (460)**

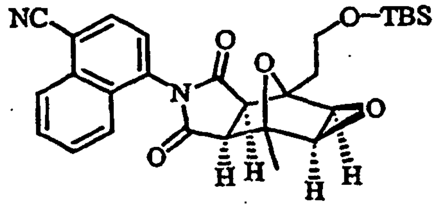

Compound **231A** (2.00 g, 4.10 mmol) was dissolved in dichloromethane (40 ml) and cooled to 0°C. mCPBA (2.36 g, 8.20 mmol) was added. The reaction mixture was then warmed up to room temperature and stirred under argon for 18 hours, followed by the addition of 10% $Na_2SO_3$ (25 ml) and saturated $NaHCO_3$ (25 ml). After stirring for 20 minutes, the organic layer was separated and the aqueous layer was extracted with dichloromethane (3 x 50 ml). The combined organic layers were washed with brine, dried over $Na_2SO_4$ and concentrated to give 2.0 g compound **460** as light yellow solid. HPLC: 99% at 4.00 min (retention time) (Phenomenex-prime SS-C18 column 4.6 x 50 mm eluting with 10-90% aqueous methanol over 4 minutes containing 0.2% phosphoric acid, 4 mL/min, monitoring at 220 nm), MS (ES): m/z [M+H]= 505.19

**Example 461**

**[3aR-(3aα,4β,7β,7aα)]-4-[4-Ethyloctahydro-7-(2-hydroxyethyl)-1,3-dioxo-4,7-epoxy-2H-isoindol-2-yl]-1-naphthalenecarbonitrile & [3aS-(3aα,4β,7β,7aα)]-4-[4-Ethyloctahydro-7-(2-hydroxyethyl)-1,3-dioxo-4,7-epoxy-2H-isoindol-2-yl]-1-naphthalenecarbonitril (461i & 461ii)**

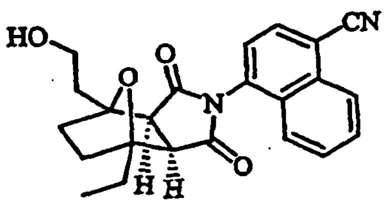 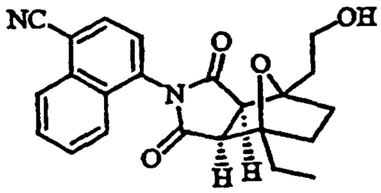

The racemic mixture of compounds **245C** was separated by normal phase preparative chiral HPLC using a Chiracel AD column (5 cm x 50 cm), eluting with 20% solvent B (50% MeOH/EtOH) in solvent A (Heptane), flow rate: 50 mL/min. Compound **461i** eluted from 80 min to 100 min and compound **461ii** eluted from 125 min to 150 min.

The absolute conformation for compounds **461i** and **461ii** was not determined. For simplicity in nomenclature, compound **461i** is designated herein as having an "R" configuration and compound **461ii** as having an "S" configuration. Enantiomerically pure products derived from compound **461i** are designated herein as having an "R" configuration and enantiomerically pure products derived from compound **461ii** are designated herein as having an "S" configuration.

**Example 462**

**[3aR-(3aα,4β,7β,7aα)]-4-[4-[2-(4-Cyanophenoxy)ethyl]-7-ethyloctahydro-1,3-dioxo-4,7-epoxy-2H-isoindol-2-yl]-1-naphthalenecarbonitrile (462)**

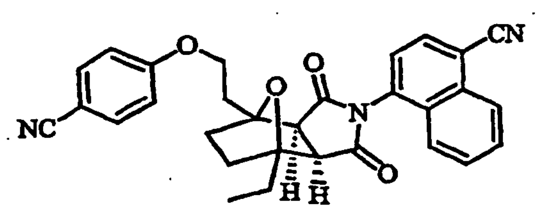

DBAD (29.5 mg, 0.128 mmol) was added to a solution of $PPh_3$ (33.6 mg, 0.128 mmol) in THF (0.5 mL). After stirring for 10 min, 4-cyanophenol (15.2 mg, 0.128 mmol) was added and the reaction mixture was stirred for an additional 5 min. Compound **461i** (18.3 mg, 0.047 mmol) was added and the mixture was stirred at rt for 2 h. The reaction mixture was concentrated under reduced pressure. Purification by flash chromatography on silica gel eluting with 40% EtOAc/hexane gave 16.9 mg (0.034 mmol, 73.2%) of compound **462.** HPLC conditions: 98% at 3.64 min (YMC S5 ODS 4.6 x 50 mm, 10%-90% aqueous methanol over 4 minute gradient with 0.2% $H_3PO_4$, detecting at 220 nm). MS (ES): m/z 492.23 $[M+H]^+$.

**Example 463**

**[3aS-(3aα,4β,7β,7aα)]-4-[4-[2-(4-Cyanophenoxy)ethyl]-7-ethyloctahydro-1,3-dioxo-4,7-epoxy-2H-isoindol-2-yl]-1-naphthalenecarbonitrile (463)**

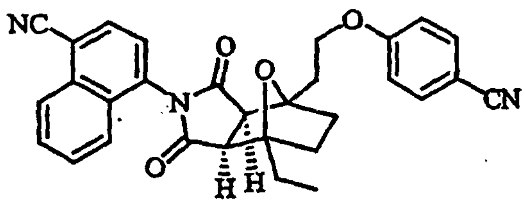

DBAD (29.5 mg, 0.128 mmol) was added to a solution of $PPh_3$ (33.6 mg, 0.128 mmol) in THF (0.5 mL). After stirring for 10 min, 4-cyanophenol (15.2 mg, 0.128 mmol) was added and the reaction mixture was stirred for an additional 5 min. Compound **461ii** (18.3 mg, 0.047 mmol) was added and the mixture was stirred at rt for 2 h. The reaction mixture was concentrated under reduced pressure. Purification by flash chromatography on silica gel eluting with 40% EtOAc/hexane gave 18.1 mg (0.037 mmol, 78.4%) of compound 463. HPLC conditions: 97% at 3.63 min (YMC S5 ODS 4.6 x 50 mm, 10%-90% aqueous methanol over 4 minute gradient with 0.2% $H_3PO_4$, detecting at 220 nm). MS (ES): m/z 492.17 $[M+H]^+$.

## Claims

**1.** A compound of the following formula (I) or a salt or hydrate thereof:

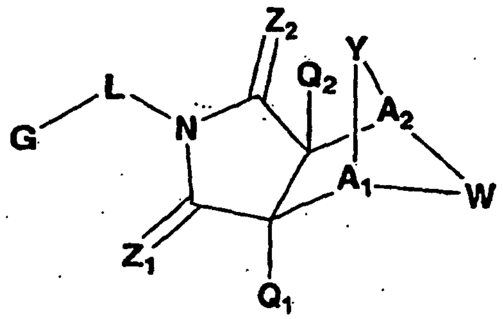

(I)

wherein the symbols have the following meanings and are, for each occurrence, independently selected:

G is an aryl or heterocyclo group, where said group is mono- or polycyclic, and is optionally substituted at one or more positions;
L is a bond, $(CR^7R^{7'})_n$ (where n is 1 and $R^7$ and $R^{7'}$ are each independently H, alkyl or substituted alkyl), or $-CH_2-NH-$;
$A_1$ and $A_2$ are each independently $CR^7$ where $R^7$ (i) is hydrogen, alkyl or substituted alkyl, arylalkyl or substituted arylalkyl, alkenyl or substituted alkenyl, aryl or substituted aryl, heterocyclo or substituted heterocyclo, heterocycloalkyl or substituted heterocycloalkyl, where, for each substituents are one or more groups selected from $V^1$, or (ii) forms, together with $R^7$ of a group W, a heterocyclic ring;
$V^1$ is OH, CN, halo, -O-aryl, -O-substituted aryl, -O-heterocyclo, -O-substituted heterocyclo, -O-CO-alkyl, -O-CO-substituted alkyl, -O-(alkylsilyl), -O-arylalkyl, -O-substituted arylalkyl, -O-CO-arylalkyl, -O-CO-substituted arylalkyl, -O-CO-aryl. -O-CO-substituted aryl, -O-CO-heterocyclo, -O-CO-substituted heterocyclo, -S-(optionally substituted aryl)-NH-CO-(optionally substituted alkyl), -SO-(optionally substituted aryl)-NH-CO-(optionally substituted alkyl), $-SO_2$-(optionally substituted aryl)-NH-CO-(optionally substituted alkyl), $-NH-SO_2$-aryl, $-NH-SO_2$-substituted aryl, -NH-CO-O-(optionally substituted arylalkyl), -NH-COO-alkyl, -NH-CO-O-substituted alkyl, -NH-CO-alkyl, -NH-CO-substituted alkyl, -NH-CO-aryl, -NH-CO-substituted aryl, -NH-CO-(optionally substituted arylalkyl), -NH-CO-(optionally substituted alkyl)-O-(optionally substituted aryl), -N(optionally substituted alkyl)(optionally substituted aryl), -N(optionally substituted alkyl)(optionally substituted arylalkyl), -COH, -COOH, -CO-O-alkyl, -CO-O-substituted alkyl, -CO-O-optionally substituted arylalkyl, -CO-aryl, -CO-substituted aryl, -O-CO-NH-aryl, -O-CO-NH-substituted aryl, -CO-NH-aryl, -CO-NH-substituted aryl, -CO-NH-arylalkyl, -CO-NH-substituted arylalkyl, or -O-(optionally substituted aryl)-NH-CO-(optionally substituted alkyl);
Y is -O-, -SO-, $-N(V^2)-$, $-CH_2-N(V^2)-$, -CO-N(alkyl)-, $-CH_2-S-$, or $-CH_2-SO_2-$;
$V^2$ is hydrogen, alkyl, arylalkyl, -CO-alkyl, -CO-O-aryl, or -CO-O-arylalkyl;
$Z_1$ and $Z_2$ are O;
$Q_1$ and $Q_2$ are H;
W is $CR^7R^{7'}-CR^7R^{7'}$, $CR^7R^{7'}-C{=}O$, $NR^9-CR^7R^{7'}$, $N{=}CR^8$, N=N, $NR^9-NR^{9'}$, cycloalkyl or substituted cycloalkyl, cycloalkenyl or substituted cycloalkenyl, heterocyclo or substituted heterocyclo, or aryl or substituted aryl, wherein, when W is not $NR^9-CR^7R^{7'}$, $N{=}CR^8$, N=N, $NR^9-NR^{9'}$, or heterocyclo or substituted heterocyclo, then Y must be -O-, $-CH_2-S-$, -SO-, $-CH_2-SO_2-$, $N(V^2)-$ or $-CH_2-N(V^2)$,
$R^1$ and $R^{1'}$ are each independently H, alkyl or substituted alkyl, cycloalkyl or substituted cycloalkyl, cycloalkenyl or substituted cycloalkenyl, heterocyclo or substituted beterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl;
$R^2$ is alkyl or substituted alkyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl;
$R^4$ is H, alkyl or substituted alkyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, $R^1C{=}O$, $R^1NHC{=}O$, $SO_2OR^1$, or $SO_2NR^1R^{1'}$;
$R^5$ is alkyl or substituted alkyl, cycloalkyl or substituted cycloalkyl, cycloalkenyl or substituted cycloalkenyl,

heterocyclo or substituted heterocyclo, cycloalkylalkyl or substituted cycloalkylalkyl, cycloalkenylalkyl or substituted cycloalkenylalkyl, heterocycloalkyl or substituted hetemcycloalkyl, aryl or substituted aryl, arylalkyl or substituted arylalkyl, $R^1C=O$, $R^1NHC=O$, $SO_2R^1$, $SO_2OR^1$, or $SO_2NR^1R^{1'}$;

$R^7$ and $R^{7'}$ are each independently H, alkyl or substituted alkyl, alkenyl or substituted alkenyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, halo, CN, $OR^1$, nitro, hydroxylamine, hydroxylamide, amino, $NHR^4$, $NR^2R^5$, $NOR^1$, thiol, alkylthio or substituted alkylthio, $R^1C=O$, $R^1OC=O$, $R^1NHC=O$, $SO_2R^1$, $SOR^1$, $PO_3R^1R^{1'}$, $R^1R^{1'}NC=O$, $C=OSR^1$, $SO_2R^1$, $SO_2OR^1$, or $SO_2NR^1R^{1'}$, or, wherein $A_1$ or $A_2$ contains a group $R^7$ and W contains a group $R^7$, said $R^7$ groups of $A_1$ or $A_2$ and W together form a heterocyclic ring;

$R^8$ is H, alkyl or substituted alkyl, alkenyl or substituted alkenyl, cycloalkyl or substituted cycloalkyl, cycloalkenyl or substituted cycloalkenyl, heterocyclo or substituted heterocyclo, cycloalkylalkyl or substituted cycloalkyalkyl, cycloalkenylalkyl or substituted cycloalkenylalkyl, heterocycloalkyl or substituted heterocycloalkyl, aryl or substituted aryl, arylalkyl or substituted arylalkyl, nitro, halo, CN, $OR^1$, amino, $NHR^4$, $NR^2R^5$, $NOR^1$, alkylthio or substituted alkylthio, $C=OSR^1$, $R^1OC=O$,$R^1C=O$, $R^1NHC=O$, $R^1R^{1'}NC=O$, $SO_2OR^1$, $S=OR^1$, $SO_2R^1$, $PO_3R^1R^{1'}$, or $SO_2NR^1R^{1'}$; and

$R^9$ and $R^{9'}$ are each independently H, alkyl or substituted alkyl, alkenyl or substituted alkenyl, cycloalkyl or substituted cycloalkyl, cycloalkenyl or substituted cycloalkenyl; heterotyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, CN, OH, $OR^1$, $R^1C=O$, $R^1OC=O$, $R^1NHC=O$, $SO_2R^1$, $SO_2OR^1$, or $SO_2NR^1R^{1'}$;

with the provisos that:

(1) when Y is -O-, $Q_1$ and $Q_2$ are hydrogen, $Z_1$ and $Z_2$ are O, W is $-CH_2-CH_2-$, and one of $A_1$ and $A_2$ is CH and the other is $CR^7$, then G-L is not unsubstituted phenyl;

(2) when Y is -O-, $Q_1$ and $Q_2$ are hydrogen, $Z_1$ and $Z_2$ are O, W is $-CH_2-CH_2-$, and one of $A_1$ and $A_2$ is CH and the other is $C-CH_3$, then G-L is not phenyl substituted with chloro and/or methyl;

(3) when Y is -O-, $Q_1$ and $Q_2$ are hydrogen, $Z_1$ and $Z_2$ are O, W is $-CH_2-CH_2-$, and one of $A_1$ and $A_2$ is CH and the other is C-alkyl, then G-L is not N-substituted piperazine-alkyl- or N-substituted imidazolidine-alkyl-;

(4) when Y is -O-; $Q_1$ and $Q_2$ are hydrogen, $Z_1$ and $Z_2$ are O, W is $-CH_2-CH_2-$, and $A_1$ and $A_2$ are $C-CH_3$, then G-L is not thiazole or substituted thiazole;

(5) when Y is $-CH_2-S-$, -SO-, $-CH_2-SO_2-$, $-N(V^2)-$ or $-CH_2-N(V^2)-$, W is $CR^7R^T- Ck^7R^T$, and $Z_1$ and $Z_2$ are O, then G-L is not unsubstituted phenyl;

(6) when Y is $NR^7$, W is unsubstituted or substituted phenyl, and $Q_1$ and $Q_2$ are hydrogen, then these compounds of formula I are not included;

(7) when Y is -O-, $Q_1$ and $Q_2$ are hydrogen, $Z_1$ and $Z_2$ are O, W is ethylene oxide, and $A_1$ and $A_2$ are CH, then G-L is not methylphenyl or chlorophenyl;

(8) the compound of formula I is not 6,10-epithio-4H-thieno-[3',4':5,6]cyclooct[1,2-*f*]isoindole-7,9(5H,8H)dione, 8-(3,5-dichlorophenyl)-6,6a,9a,10,11,12,-hexahydro-1,3,6,10-tetramethyl-2,2,13-trioxide, (6R,6aR,9aS,10S);

(9) when Y is -O-, $Q_1$ and $Q_2$ are hydrogen, $Z_1$ and $Z_2$ are O, W is cyclopentyl, cyclohexyl, 3-phenyl-2-isoxazoline or $CR^7R^{7'}-CR^7R^{7'}$ where $R^7$ and $R^{7'}$ are each independently defined as H and 4-butyrolactone and $R^7$ and $R^{7'}$ are not all simultaneously H, and $A_1$ and $A_2$ are CH, then G-L is not an unsubstituted naphthyl ring or a monosubstituted phenyl ring, where said substituent is methoxy, Br, Cl, $NO_2$, methyl, ethyl, $CH_2$-phenyl, S-phenyl, or O-phenyl;

(10) when Y is -O- and W is $-CH_2-CH_2-$, then at least one of $A_1$ or $A_2$ is not CH;

(11) wherein the compound of formula (I) is not

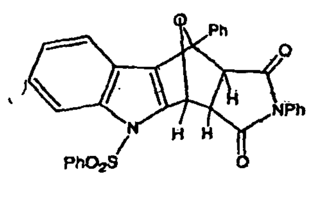 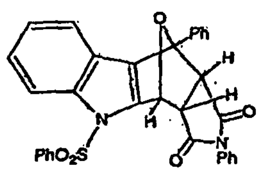

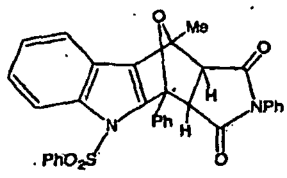

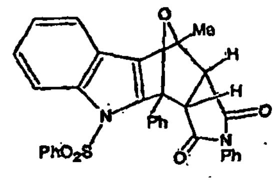

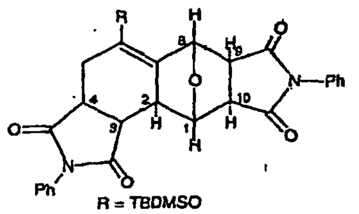

R = TBDMSO

R = Me

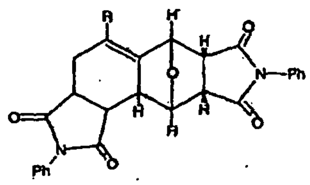

R = TBDMSO

R = Me

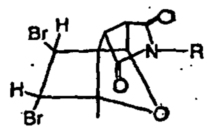

R = $C_6H_4$-$CH_3$ (2')

R = $C_6H_4$-$OCH_3$ (2')

R = 1-Naphthyl

wherein the terms "alkyl" and "alk" refer to a straight or branched chain alkane radical containing from 1 to 12 carbon atoms; "substituted alkyl" refers to an alkyl group substituted with 1 to 4 substituents, at any available point of attachment, wherein the substituents are one or more of the following groups: halo, alkoxy, alkylthio, hydroxy, carboxy, alkoxycarbonyl, alkylcarbonyloxy, amino, carbamoyl or substituted carbamoyl, carbamate or substituted carbamate, urea or substituted urea, amidinyl or substituted amidinyl, thiol, aryl, heterocycle, cycloalkyl, heterocycloalkyl, -S-aryl, -S-heterocycle, -S=O-aryl, -S=O-heterocycle, arylalkyl-O-, -$S(O)_2$-aryl, -$S(O)_2$-heterocycle, -NHS$(O)_2$-aryl, -NHS$(O)_2$-heterocycle,- NHS$(O)_2$NH-aryl, -NHS$(O)_2$NH-heterocycle, -P$(O)_2$-aryl, -P$(O)_2$-heterocycle, -NHP$(O)_2$-aryl, -NHP$(O)_2$-heterocycle, -NHP$(O)_2$NH-aryl, -NHP$(O)_2$NH-heterocycle, -O-aryl, -O-heterocycle, -NH-aryl, -NH-heterocycle, -NHC=O-aryl, -NHC=O-alkyl, -NHC=O-heterocycle, -OC=O-aryl, -OC=O-heterocycle, -NHC=ONH-aryl, -NHC=ONH-laeterocycle, -OC=OO-aryl, -OC=OO-heterocycle, -OC=ONH-aryl, -OC=ONH-heterocycle, -NHC=OO-aryl, -NHC=OO-heteracycle, -NHC=OO-alkyl, -C=ONH-aryl, -C=ONH-heterocycle, -C=OO-aryl, -C=OO-heterocycle, -N(alkyl)S$(O)_2$-aryl, -N(alkyl)S$(O)_2$-heterocycle, -N(alkyl)S$(O)_2$NH-aryl, -N(alkyl)S$(O)_2$NH-heterocycle, -N(alkyl)P$(O)_2$-aryl, -N(alkyl)P$(O)_2$-heterocycle, -N(alkyl)P$(O)_2$NH-aryl, -N(alkyl)P$(O)_2$NH-heterocycle, -N (alkyl)-aryl, -N(alkyl)-heterocycle, -N(alkyl)C=O-aryl, -N(allcyl)C=O-heterocycle, -N(alkyl)C=ONH-aryl, -N(alkyl) C=ONH-heterocycle, -OC=ON(alkyl)-aryl, -OC=ON(alkyl)-heterocycle, -N(alkyl)C=OO-aryl, -N(alkyl)C=OO-heterocycle, -C=ON(alkyl)-aryl, -C=ON(alkyl)-heterocycle, -NHS$(O)_2$N(alkyl)-aryl, -NHS$(O)_2$N(alkyl)-heterocycle, -NHP $(O)_2$N(alkyl)-aryl, NHP$(O)_2$N(alkyl)-heterocycle, -NHC=ON(alkyl)-aryl, -NHC=ON(alkyl)-heterocycle, -N(alkyl)S $(O)_2$N(alkyl)-aryl, -N(alkyl)S$(O)_2$N(alkyl)-heterocycle, -N(alkyl)P$(O)_2$N(alkyl)-aryl, -N(alkyl)P$(O)_2$N(alkyl)-heterocycle, -N(alkyl)C=ON(alkyl)-aryl, and -N(alkyl)C=ON(alkyl)-heterocycle, wherein in each instance "alkyl", "aryl" and "heterocycle" groups can themselves be optionally substituted; alkyl substituents also include the groups "T" and "T-$R^{\cdot 2}$" wherein T is defined as a nitrogen, oxygen or sulfur-containing group, and $R^{\cdot 2}$ is H, alkyl or substituted alkyl, alkenyl or substituted alkenyl, cycloalkyl or substituted cycloalkyl, cycloalkenyl or substituted cycloalkenyl, heterocyclo or substituted heterocyclo, cycloalkylalkyl or substituted cycloalkylalkyl, cycloalkenylalkyl or substituted cycloalkenylalkyl, heterocycloalkyl or substituted heterocycloalkyl, aryl or substituted aryl, arylalkyl or substituted arylalkyl, halo, CN, $OR^1$, nitro, hydroxylamine, hydroxylamide, amino, $NHR^4$, $NR^2R^5$, $NOR^1$, thiol, alkylthio or substituted alkylthio, $R^1C$=O, $R^1OC$=O, $R^1NHC$=O, $SO_2R^1$, $SOR^1$, $PO_3R^1R^{1'}$, $R^1R^{1'}NC$=O, C=$OSR^1$, $SO_2R^1$, $SO_2OR^1$, or $SO_2NR^1R^{1'}$;

the term "alkenyl" refers to a straight or branched chain hydrocarbon radical containing from 2 to 12 carbon atoms and at least one carbon-carbon double bond; "substituted alkenyl" refers to an alkenyl group substituted with 1 to 4 substituents, at any available point of attachment, wherein the substituents are selected from alkyl or substituted alkyl, as well as those groups recited above as alkyl substituents;

the term "cycloalkyl" refers to a fully saturated cyclic hydrocarbon group containing from 1 to 4 rings and 3 to 8 carbons per ring; "substituted cycloalkyl" refers to a cycloalkyl group substituted with 1 to 4 substituents, at any available point of attachment, wherein the substituents are selected from nitro, cyano, alkyl or substituted alkyl, as well as those groups recited above as alkyl substituents, and hydrogen, alkyl or substituted alkyl, alkenyl or substituted alkenyl, alkynyl or substituted alkynyl, halo, cycloalkyl or substituted cycloalkyl, cycloalkenyl or substituted cycloalkenyl, aryl or substituted aryl, heterocyclo or substituted heterocyclo, arylalkyl or substituted arylalkyl, heterocycloalkyl or substituted heterocycloalkyl, CN, $R^1OC$=O, $R^1C$=O, $R^1C$=S, $R^1HNC$=O, $R^1R^2NC$=O, $HOCR^3R^{3'}$, nitro, $R^1OCH_2$, $R^1O$, $NH_2$, $NR^4R^5$, $SR^1$, S=$OR^1$, $SO_2R^1$, $SO_2NR^1R^{1'}$, $(R^1O)(R^{1'}O)$P=O, oxo, $(R^1)(R^{1'})$P=O, $(R^1)(NHR^1)$P=O and spiro-attached or fused cycloalkenyl or substituted cycloalkenyl;

the term "cycloalkenyl" refers to a partially unsaturated cyclic hydrocarbon group containing 1 to 4 rings and 3 to 8 carbons per ring; "substituted cycloalkenyl" refers to a cycloalkenyl group substituted with 1 to 4 substituents, at any available point of attachment; wherein the substituents are selected from nitro, cyano, alkyl or substituted alkyl, as well as those groups recited above as alkyl substituents, and hydrogen, alkyl or substituted alkyl, alkenyl or substituted alkenyl, alkynyl or substituted alkynyl, halo, cycloalkyl or substituted cycloalkyl, cycloalkenyl or substituted cycloalkenyl, aryl or substituted aryl, heterocyclo or substituted heterocyclo, arylalkyl or substituted arylalkyl, heterocycloalkyl or substituted heterocycloalkyl, CN, $R^1OC{=}O$, $R^1C{=}O$, $R^1C{=}S$, $R^1HNC{=}O$, $R^1R^2NC{=}O$, $HOCR^3R^{3'}$, nitro, $R^1OCH_2$, $R^1O$, $NH_2$, $NR^4R^5$, $SR^1$, $S{=}OR^1$, $SO_2R^1$, $SO_2OR^1$, $SO_2NR^1R^{1'}$, $(R^1O)(R^{1'}O)P{=}O$, oxo, $(R^1)(R^{1'})P{=}O$, $(R^{1'})(NHR^1)P{=}O$, and spiro-attached or fused cycloalkyl or substituted cycloalkyl;

the terms "alkoxy" or "alkylthio" refer to an alkyl group as described above bonded through an oxygen linkage (-O-) or a sulfur linkage (-S-), respectively; the terms "substituted alkoxy" or "substituted alkylthio" refer to a substituted alkyl group as described above bonded through an oxygen or sulfur linkage, respectively;

the term "alkoxycarbonyl" refers to an alkoxy group bonded through a carbonyl group;

the term "alkylcarbonyl" refers to an alkyl group bonded through a carbonyl group; the term "alkylcarbonyloxy" refers to an alkylcarbonyl group bonded through an oxygen linkage;

the terms "arylalkyl", "substituted arylalkyl", "cycloalkylalkyl", "substituted cycloalkylalkyl", "cycloalkenylalkyl", "substituted cycloalkenylalkyl", "heterocycloalkyl" and "substituted heterocycloalkyl" refer to aryl, cycloalkyl, cycloalkenyl and heterocyclo groups bonded through an alkyl group, substituted on the aryl, cycloalkyl, cycloalkenyl or heterocyclo and/or the alkyl group where indicated as "substituted";

the term "aryl" refers to cyclic, aromatic hydrocarbon groups which have 1 to 5 aromatic rings; where containing two or more aromatic rings, the aromatic rings of the aryl group may be joined at a single point or fused, "substituted aryl" refers to an aryl group substituted by 1,2,3,4 or 5 substituents, at any point of attachment, wherein the substituents are selected from nitro, cycloalkyl or substituted cycloalkyl, cycloalkenyl or substituted cycloalkenyl, cyano, alkyl-$S(O)_m$- (m=0, 1 or 2), alkyl or substituted alkyl, as well as those groups recited above as alkyl substituents and hydrogen, alkyl or substituted alkyl, alkenyl or substituted alkenyl, alkynyl or substituted alkynyl, halo, cycloalkyl or substituted cycloalkyl, cycloalkenyl or substituted cycloalkenyl, aryl or substituted aryl, heterocyclo or substituted heterocyclo, arylalkyl or substituted arylalkyl, heterocycloallcyl or substituted heterocycloalkyl, CN, $R^1OC{=}O$, $R^1C{=}O$, $R^1C{=}S$, $R^1HNC{=}O$, $R^1R^2NC{=}O$, $HOCR^3R^{3'}$, nitro, $R^1OCH_2$, $R^1O$, $NH_2$, $NR^4R^5$, $SR^1$, $S{=}OR^1$, $SO_2R^1$, $SO_2OR^1$, $SO_2NR^1R^{1'}$, $(R^1O)(R^{1'}O)P{=}O$, oxo, $(R^1)(R^{1'})P{=}O$, $(R^{1'})(NHR^1)P{=}O$, and fused heterocyclo or cycloalkenyl or substituted heterocyclo or cycloalkenyl groups; "substituted carbamoyl", "substituted carbamate", "substituted urea" and "substituted amidinyl" refer to carbamoyl, carbamate, urea or amidinyl groups in which one or more of the hydrogen groups are replaced by an organic moiety listed above;

the terms "heterocycle", heterocyclic" and "heterocyclo" refer to fully saturated, or partially or fully unsaturated, including aromatic 3 to 7 membered monocyclic, 7 to 11 membered bicyclic, or 10 to 16 membered tricyclic ring systems which have at least one heteroatom in at least one carbon atom-containing ring, wherein each ring of the heterocyclic group containing a heteroatom may have 1, 2, 3. or 4 heteroatoms selected from nitrogen atoms, oxygen atoms and/or sulfur atoms, where the nitrogen and sulfur heteroatoms may optionally be oxidized and the nitrogen heteroatoms may optionally be quaternized;

"substituted heterocycle", "substituted heterocyclic", and "substituted heterocyclo" refer to heterocycle, heterocyclic or heterocyclo groups substituted with 1 to 4 substituents; at any available point of attachment, wherein the substituents are selected from cycloalkyl or substituted cycloalkyl, cycloalkenyl or substituted cycloalkenyl, nitro, oxo, cyano, alkyl-$S(O)_m$- (m = 0, 1 or 2), alkyl or substituted alkyl, as well as those groups recited above as alkyl substituents, and hydrogen, alkyl or substituted alkyl, alkenyl or substituted alkenyl, alkynyl or substituted alkynyl, halo, cycloalkyl or substituted cycloalkyl, cycloalkenyl or substituted cycloalkenyl, aryl or substituted aryl, heterocyclo or substituted heterocyclo, arylalkyl or substituted arylalkyl, heterocycloalkyl or substituted heterocycloalkyl, CN, $R^1OC{=}O$, $R^1C{=}O$, $R^1C{=}S$, $R^1HNC{=}O$, $R^1R^2NC{=}O$, $HOCR^3R^{3'}$, nitro, $R^1OCH_2$, $R^1O$, $NH_2$, $NR^4R^5$, $SR^1$, $S{=}OR^1$, $SO_2R^1$, $SO_2OR^1$, $SO_2NR^1R^{1'}$, $(R^1O)(R^{1'}O)P{=}O$, oxo, $(R^1)(R^{1'})P{=}O$, $(R^{1'})(NHR^1)P{=}O$.

**2.** A compound according to claim 1 wherein L is a bond.

**3.** A compound selected from the group consisting of:

(3aα,4α,7α,7aα)-2-(4-Bromo-3-methylphenyl)tetrahydro-4,7-ethanothiopyrano[3,4-c]pyrrole-1,3,8(2H,4H)-trione (1C);

(3aα,4α,7α,7aα)-2-(4-Bromo-3-methylphenyl)tetrahydro-4,7-ethanothiopyrano[3,4-c]pyrrole-1,3,8(2H,4H)-trione 5,5-dioxide (2);

(3aα,4β,7β,7aα)-2-(3-Chlorophenyl)hexahydro-4-methyl-4, 7-epoxy-1H-isoindole-1,3(2H)-dione (3);

(3aα,4α,7α,7aα)- and (3aα,4β,7β,7aα)-4-[(Acetyloxy)methyl]-3a,4,7,7a-tetrahydro-2-[3-(trifluoromethyl)phenyl]-4,7-epoxy-1H-isoindole-1,3(2H)-dione (4i & 4ii, respectively);
(3aα,4α,7α,7aα)- and (3aα,4β,7β,7aα)-4-[(Acetyloxy)methyl]-Hexahydro-2-[3-(trifluoromethyl)phenyl]-4,7-epoxy-1H-isoindole-1,3(2H)-dione (5i & 5ii, respectively);
(3aα,4α,7α,7aα)- and (3aα,4β,7β,7aα)-3a,4,7,7a-Tetrahydro-5-(hydroxymethyl)-2-[3-(trifluoromethyl)phenyl]-4,7-epoxy-1H-isoindole-1,3(2H)-dione (6i & 6ii, respectively);
(3aα,4α,7α,7aα)-3a,4,7,7a-Tetrahydro-5-(hydroxymethyl)-4-methyl-2-[3-(trifluoromethyl)phenyl]-4,7-epoxy-1H-isoindole-1,3(2H)-dione (7);
(3aα,4β,7β,7aα)-2-[3,5-Bis(trifluoromethyl)phenyl]hexahydro-4,7-epoxy-1H-isoindole-1,3(2H)-dione (8);
(3aα,4α,7α,7aα)-2-(4-Bromophenyl)octahydro-1,3-dioxo-4,7-etheno-5H-pyrrolo[3,4-c]pyridine-5-carboxylic acid phenyl ester (9);
(3aα,4α,7α,7aα)-2-(4-Bromophenyl)octahydro-1,3-dioxo-4,7-etheno-5H-pyrrolo[3,4-c]pyridine-5-carboxylic acid phenylmethyl ester (10);
(3aα,4α,7α,7aα)-Hexahydro-2-[3-(trifluoromethyl)phenyl]-4,7-ethano-1H-pyrrolo[3,4-c]pyridine-1,3(2H)-dione trifluoroacetate (11);
(3aα,4α,7α,7aα)-5-Acetylhexahydro-2-[3-(trifluoromethyl)phenyl]-4,7-ethano-1 H-pyrrolo[3,4-c]pyridine-1,3(2H)-dione (12);
(3aα,4α,7α,7aα)-5-Benzoylhexahydro-2-[3-(trifluoromethyl)phenyl]-4,7-ethano-1H-pyrrolo[3,4-c]pyridine-1,3(2H)-dione (13);
(3aα,4α,7α,7aα)-Hexahydro-5-methyl-2-[3-(trifluoromethyl)phenyl]-4,7-ethano-1H-pyrrolo[3,4-c]pyridine-1,3(2H)-dione (14);
(3aα,4α,7α,7aα)-Hexahydro-5-(phenylmethyl)-2-[3-(trifluoromethyl)phenyl]-4,7-ethano-1H-pyrrolo[3,4-c]pyridine-1,3(2H)-dione trifluoroacetate (15);
(3aα,4α,7α,7aα)-Hexahydro-5-propyl-2-[3-(trifluoromethyl)phenyl]-4,7-ethano-1H-pyrrolo[3,4-c]pyridine-1,3(2H)-dione trifluoroacetate (16);
(3aα,4α,4aβ,5aβ,6α,6aα)-2-[4-Cyano-3-(trifluoromethyl)phenyl]decahydro-1,3-dioxo-4,6-(iminomethano)cycloprop[f]isoindole-7-carboxylic acid phenylmethyl ester (17);
(3aα,4α,4aβ,5aβ,6α,6aα)-4-[Decahydro-1,3-dioxo-4,6-(iminomethano)cycloprop[f]isoindol-2-yl]-2-(trifluoromethyl)benzonitrile (18);
(3aα,4α,4aβ,5aβ,6α,6aα)-4-[Decahydro-7-methyl-1,3-dioxo-4,6-(iminomeihano)cycloprop[f]isoindol-2-yl]-2-(trifluoromethyl)benzonitrile (19);
(3aα,4β,7β,7aα)-4-(Octahydro-4,7-dimethyl-1.3-dioxo-4,7-epoxy-2H-isoindol-2-yl)-2-(trifluoromethyl)benzonitrile (20B);
(3aα, 4β, 7β, 7aα) -N-[4-[[2-[2-[4-Cyano- 3-(trifluoromethyl) phenyl] octahydro- 7-methyl- 1,3-dioxo- 4,7-epoxy-4H-isoindol-4-yl]ethyl]thio]phenyl]acetamide (21E);
(3aα, 4β, 7β, 7aα) -N-[4-[[2-[2-[4-Cyano- 3-(trifluoromethyl) phenyl] octahydro- 7-methyl- 1,3-dioxo- 4,7-epoxy-4H-isoindol-4-yl]ethyl]sulfinyl]phenyl]acetamide (22);
(3aα, 4β, 7β, 7aα) -N-[4-[[2-[2-[4-Cyano- 3-(trifluoromethyl) phenyl] octahydro- 7-methyl- 1,3-dioxo- 4,7-epoxy-4H-isoindol-4-yl]ethyl]sulfonyl]phenyl]acetamide (23);
(3aα,4β,7β,7aα)- and (3aα,4α,7α,7aα)-N-[2-[2-[4-Cyano-3-(trifluoromethyl)phenyl]octahydro-7-methyl-1,3-dioxo-4,7-epoxy-4H-isoindol-4-yl]ethyl]benzenesulfonamide (24Ci & 24Cii, respectively);
(3aα,4β,7β,7aα)-4-[Octahydro-4-(2-hydroxyethyl)-7-methyl-1,3-dioxo-4,7-epoxy-2H-isoindol-2-yl]-2-(trifluoromethyl)benzonitrile (25B);
(3aα,4α,7α,7aα)- and (3aα,4β,7β,7aα)-N-[4-[2-[2-[4-Cyano-3-(trifluoromethyl)phenyl]octahydro-7-methyl-1,3-dioxo-4,7-epoxy-4H-isoindol-4-yl]ethoxy]phenyl]acetamide (26Ci & 26Cii, respectively);
(3aα,4α,7α,7aα)-Hexahydro-2-(2-naphthalenyl)-4,7-epoxy-1H-isoindole-1,3(2H)-dione (27D);
(1aα, 2β, 2aα, 5aα, 6β, 6aα) -Hexahydro- 4-(2-naphthalenyl)- 2,6-epoxy- 3H-oxireno [f] isoindole- 3,5 (4H) -dione (28B);
(3aα,4α,7α,7aα)-2-[4-Bromo-3-(trifluoromethyl)phenyl]-3a,4,7,7a-tetrahydro-4,7-dimethyl-4,7-epithio-1H-isoindole-1,3(2H)-dione 8-oxide (29);
(3aα,4α,7α,7aα)-2-[4-Bromo-3-(trifluoromethyl)phenyl]-3a,4,7,7a-tetrahydro-4,7-epithio-1H-isoindole-1,3(2H)-dione 8-oxide (30);
(3aα,4α,7α,7aα)-Hexahydro-2-[3-(trifluoromethyl)phenyl]-4,7-imino-1H-isoindole-1,3(2H)-dione (31D);
(3aα,4β,7β,7aα)- and (3aα,4α,7α,7aα)-3a,4,7,7a-Tetrahydro-4,7-dimethyl-2-[3-(trifluoromethyl)phenyl]-4,7-epoxy-1H-isoindole-1,3(2H)-dione (32i & 32ii, respectively);
(3aα,4α,7α,7aα)-Hexahydro-4,7-dimethyl-2-[3-(trifluoromethyl)phenyl]-4,7-epoxy-1H-isoindote-1,3,(2H)-dione (33);
(3aα,4α,7α,7aα)-Tetrahydro-5-methyl-2-(4-nitro-1-naphthalenyl)-4,7-etheno-1 H-pyrrolo[3,4-c]pyridine-1,3,6(2H,5H)-trione (34B);

(3aα,4β,7β,7aα)-4-[4-[2-(4-Fluorophenoxy)ethyl]octahydro-7-methyl-1,3-dioxo-4,7-epoxy-2H-isoindol-2-yl]-2-(trifluoromethyl)benzonitrile (35);
(3aα,4β,7β,7aα)-4-[4-(2-Bromoethyl)octahydro-7-methyl-1,3-dioxo-4,7-epoxy-2H-isoindol-2-yl]-2-(trifluoromethyl)bentonitrile (36);
(3aα,4β,7β,7aα)-Hexahydro-4,7-dimethyl-2-(3-methyl-4-nitrophenyl)-4,7-epoxy-1H-isoindole-1,3(2H)-dione (37);
(3aα,4β,7β,7aα)-2-(2-Fluorenyl)hexahydro-4,7-epoxy-1H-isoindole-1,3(2H)-dione;
(3aα,4β,7β,7aα)-2-[3-Chloro-4-(4-morpholinyl)phenyl]hexahydro-4,7-epoxy-1 H-isoindole-1,3(2H)-dione;
(3aα,4β,7β,7aα)-2-(2,3-Dihydro-1H-inden-5-yl)hexahydro-4,7-epoxy-1H-isoindole-1,3(2H)-dione;
(3aα,4β,7β,7aα)-2-(4-Bromo-1-naphthalenyl)hexahydro-4,7-epoxy-1H-isoindole-1,3(2H)-dione;
(3aα,4β,7β,7aα)-2-(4-Chloro-1-naphthalenyl)hexahydro-4,7-epoxy-1H-isoindole-1,3(2H)-dione;
(3aα,4β,7β,7aα)-2-(5-Amino-1-naphthalenyl)hexahydro-4,7-epoxy-1H-isoindole-1,3(2H)-dione;
(3aα,4β,7β,7aα)-Hexahydro-2-(7-hydroxy-1-naphthalenyl)-4,7-epoxy-1H-isoindole-1,3(2H)-dione;
(3aα,4β,7β,7aα)-Hexahydro-2-(4-nitro-1-naphthalenyl)-4,7-epoxy-1H-isoindole-1,3(2H)-dione;
(3aα,4β,7β,7aα)-Hexahydro-2-(1H-indol-5-yl)-4,7-epoxy-1H-isoindole-1,3(2H)-dione;
(3aα,4β,7β,7aα)-Hexahydro-2-(1H-indazol-6-yl)-4,7-epoxy-1H-isoindole-1,3(2H)-dione;
(3aα,4β,7β,7aα)-2-(1,3-Benzodioxol-5-yl)hexahydro-4,7-epoxy-1H-isoindole-1,3(2H)-dione;
(3aα,4β,7β,7aα)-2-[4-Amino-3-(trifluoromethyl)phenyl]hexahydro-4,7-epoxy-1 H-isoindole-1,3(2H)-dione;
(3aα,4β,7β,7aα)-2-(3-Chloro-4-iodophenyl)hexahydro-4,7-epoxy-1H-isoindole-1,3(2H)-dione;
(3aα,4β,7β,7aα)-Hexahydro-2-(8-quinolinyl)-4,7-epoxy-1H-isoindole-1,3(2H)-dione;
(3aα,4β,7β,7aα)-2-(2,3-Dihydro-1,4-benzodioxin-6-yl)hexahydro-4,7-epoxy-1H-isoindole-1,3(2H)-dione;
(3aα,4β,7β,7aα)-Hexahydro-2-[2-oxo-4-(trifluoromethyl)-2H-1-benzopyran-7-yl]-4,7-epoxy-1H-isoindole-1,3(2H)-dione;
(3aα,4β,7β,7aα)-Hexahydro-2-(4-methyl-2-oxo-2H-1-benzopyran-7-yl)-4,7-epoxy-1H-isoindole-1,3(2H)-dione;
(3aα,4β,7β,7aα)-2-(2,5-Dimethoxy-4-nitrophenyl)hexahydro-4,7-epoxy-1H-isoindole-1,3(2H)-dione;
(3aα,4β,7β,7aα)-2,3,5,6-Tetrafluoro-4-(octahydro-1,3-dioxo-4,7-epoxy-2H-isoindol-2-yl)benzonitrile;
(3aα,4β,7β,7aα)-Hexahydro-2-(2,4,5-trifluorophenyl)-4,7-epoxy-1H-isoindole-1,3(2H)-dione;
(3aα,4β,7β,7aα)-Hexahydro-2-(2,4,5-trichlorophenyl)-4,7-epoxy-1H-isoindole-1,3(2H)-dione;
(3aα,4β,7β,7aα)-2-(2-Amino-4,5-dichlorophenyl)hexahydro-4,7-epoxy-1H-isoindole-1,3(2H)-dione;
(3aα,4β,7β,7aα)-2-(3,4-Difluorophenyl)hexahydro-4,7-epoxy-1H-isoindole-1,3(2H)-dione;
(3aα,4β,7β,7aα)-1-Acetyl-2,3-dihydro-6-(octahydro-1,3-dioxo-4,7-epoxy-2H-isoindol-2-yl)-1H-indole;
(3aα,4β,7β,7aα)-2-(3-Chloro-4-fluorophenyl)hexahydro-4,7-epoxy-1H-isoindole-1,3(2H)-dione;
(3aα,4β,7β,7aα)-2-(3,4-Dichlorophenyl)hexahydro-4,7-epoxy-1H-isoindole-1,3(2H)-dione;
(3aα,4β,7β,7aα)-Hexahydro-2-(3,4,5-trichlorophenyl)-4,7-epoxy-1H-isoindole-1,3(2H)-dione;
(3aα,4β,7β,7aα)-2-(3-Chloro-4-methoxyphenyl)hexahydro-4,7-opoxy-1H-isoindole-1,3(2H)-dione;
(3aα,4β,7β,7aα)-2-(3-Chloro-4-methylphenyl)hexahydro-4,7-epoxy-1H-isoindole-1,3(2H)-dione;
(3aα,4β,7β,7aα)-Hexahydro-2-(2-methyl-1-naphthalenyl)-4,7-epoxy-1H-isoindole-1,3(2H)-dione;
(3aα,4β,7β,7aα)-2-(4-Chloro-3-methylphenyl)hexahydro-4,7-epoxy-1H-isoindole-1,3(2H)-dione;
(3aα,4β,7β,7aα)-2-(3,4-Dimethylphenyl)hexahydro-4,7-epoxy-1H-isoindole-1,3(2H)-dione;
(3aα,4β,7β,7aα)-2-[4-Bromo-3-(trifluoromethyl)phenyl]hexahydro-4,7-epoxy-1H-isoindole-1,3(2H)-dione;
(3aα,4β,7β,7aα)-2-(4-Bromo-3-methylphenyl)hexahydro-4,7-epoxy-1H-isoindole-1,3(2H)-dione;
(3aα,4β,7β,7aα)-2-(4-Fluoro-3-nitrophenyl)hexahydro-4,7-epoxy-1H-isoindole-1,3(2H)-dione;
(3aα,4β,7β,7aα)-2-[4-Fluoro-3-(trifluoromethyl)phenyl]hexahydro-4,7-epoxy-1H-isoindole-1,3(2H)-dione;
(3aα,4β,7β,7aα)-2-(4-Chloro-3-nitrophenyl)hexahydro-4,7-epoxy-1H-isoindole-1,3(2H)-dione;
(3aα,4β,7β,7aα)-2-[4-Chloro-3-(trifluoromethyl)phenyl]hexahydro-4,7-epoxy-1H-isoindole-1,3(2H)-dione;
(3aα,4β,7β,7aα)-2-(4-Chloro-2-methoxy-5-methylphenyl)hexahydro-4,7-epoxy-1H-isoindole-1,3(2H)-dione;
(3aα,4β,7β,7aα)-2-(4-Amino-3-nitrophenyl)hexahydro-4,7-epoxy-1H-isoindole-1,3(2H)-dione;
(3aα,4β,7β,7aα)-Hexahydro-2-(4-methyl-3-nitrophenyl)-4,7-epoxy-1H-isoindole-1,3(2H)-dione;
(3aα,4β,7β,7aα)-2-(3,4-Dimethoxyphenyl)hexahydro-4,7-epoxy-1H-isoindole-1,3(2H)-dione;
(3aα,4β,7β,7aα)-Hexahydro-2-(3-hydroxy-4-methoxyphenyl)-4,7-epoxy-1H-isoindole-1,3(2H)-dione;
(3aα,4β,7β,7aα)-Hexahydro-2-(4-methyl-5-nitro-2-pyridinyl)-4,7-epoxy-1H-isoindole-1,3(2H)-dione;
(3aα,4β,7β,7aα)-2-Chloro-4-(octahydro-1,3-dioxo-4,7-epoxy-2H-isoindol-2-yl)-α-phenylbenzeneacetonitrile;
(3aα,4β,7β,7aα)-Hexahydro-2-(2-methoxy-3-dibenzofuranyl)-4,7-epoxy-1H-isoindole-1,3(2H)-dione;
(3aα,4β,7β,7aα)-Hexahydro-2-(2,3,4-trifluorophenyl)-4,7-epoxy-1H-isoindole-1,3(2H)-dione;
(3aα,4β,7β,7aα)-2-(2,3-Dihydro-2-methyl-1,3-dioxo-1H-isoindol-5-yl)hexahydro-4,7-epoxy-1H-isoindole-1,3(2H)-dione;
(3aα,4β,7β,7aα)-2-(4-Bromo-2,3,5,6-tetrafluorophenyl)hexahydro-4,7-epoxy-1H-isoindole-1,3(2H)-dione;

(3aα,4β,7β,7aα)-Hexahydro-2-(2-hydroxy-1-naphthalenyl)-4,7-epoxy-1H-isoindole-1,3(2H)-dione;
(3aα,4β,7β,7aα)-2-[2,5-Dichloro-4-(1H-pyrrol-1-yl)phenyl]hexahydro-4,7-epoxy-1H-isoindole-1,3(2H)-dione;
(3aα,4β,7β,7aα)-Hexahydro-2-[4-(methoxymethyl)-2-oxo-2H-1-benzopyran-7-yl]-4,7-epoxy-1H-isoindole-1,3(2H)-dione;
(3aα,4β,7β,7aα)-2-(6-Benzothiazolyl)hexahydro-4,7-epoxy-1H-isoindole-1,3(2H)-dione;
(3aα,4β,7β,7aα)-2-Methoxy-4-(octahydro-1,3-dioxo-4,7-epoxy-2H-isoindol-2-yl)benzoic acid methyl ester;
(3aα,4β,7β,7aα)-2-Methyl-5-(octahydro-1,3-dioxo-4,7-epoxy-2H-isoindol-2-yl)benzonitrile;
(3aα,4β,7β,7aα)-Hexahydro-2-(2-oxo-2H-1-benzopyran-6-yl)-4,7-epoxy-1H-isoindote-1,3(2H)-dione;
(3aα,4β,7β,7aα)-Hexahydro-2-(2,3,5,6-tetramethyl-4-nitrophenyl)-4,7-epoxy-1H-isoindole-1,3(2H)-dione;
(3aα,4β,7β,7aα)-Hexahydro-2-(2,4,5-trimethylphenyl)-4,7-epoxy-1H-isoindole-1,3(2H)-dione;
(3aα,4β,7β,7aα)-2-(4-Fluoro-3-methylphenyl)hexahydro-4,7-epoxy-1H-isoindole-1,3(2H)-dione;
(3aα,4β,7β,7aα)-Hexahydro-2-(3-methoxy-4-methylphenyl)-4,7-epoxy-1H-isoindole-1,3(2H)-dione;
(3aα,4β,7β,7aα)-N-Ethyl-2-methyl-5-(octahydro-1,3-dioxo-4,7-epoxy-2H-isoindol-2-yl)-N-phenylbenzenesulfonamide;
(3aα,4β,7β,7aα)-2,6-Dibromo-4-(octahydro-1,3-dioxo-4,7-epoxy-2H-isoindol-2-yl)benzenesulfonamide;
(3aα,4β,7β,7aα)-2,4-Dimethyl-6-(octahydro-1,3-dioxo-4,7-epoxy-2H-isoindol-2-yl)-3-pyridinecarbonitrile;
(3aα,4β,7β,7aα)-2-(2,3-Dimethyl-1H-indol-5-yl)hexahydro-4,7-epoxy-1H-isoindole-1,3(2H)-dione;
(3aα,4β,7β,7aα)-2-(3-Dibenzofuranyl)hexahydro-4,7-epoxy-1H-isoindole-1,3(2H)-dione;
(3aα,4β,7β,7aα)-Hexahydro-2-(2'-hydroxy[1,1':3',1"-terphenyl]-5'-yl)-4,7-epoxy-1H-isoindole-1,3(2H)-dione;
(3aα,4β,7β,7aα)-Hexahydro-2-(5,6,7,8-tetrahydro-3-hydroxy-2-naphthatenyl)-4,7-epoxy-1H-isoindole-1,3(2H)-dione;
(3aα,4β,7β,7aα)-2-(2,3-Dihydro-1H-indol-6-yl)hexahydro-4,7-epoxy-1H-isoindole-1,3(2H)-dione;
(3aα,4β,7β,7aα)-2-(1,3-Dihydro-2,2-dioxidobenzo[c]thiophen-5-yl)hexahydro-4,7-epoxy-1H-isoindole-1,3(2H)-dione;
(3aα,4β,7β,7aα)-Hexahydro-2-(2-hydroxy-4,5-dimethylphenyl)-4,7-epoxy-1H-isoindole-1,3(2H)-dione;
(3aα,4β,7β,7aα)-2-(2,3-Dihydro-2,2,3,3-tetrafluoro-1,4-benzodioxin-6-yl)hexahydro-4,7-epoxy-1H-isoindole-1,3(2H)-dione;
(3aα,4β,7β,7aα)-Hexahydro-2-(1H-indazol-5-yl)-4,7-epoxy-1H-isoindole-1,3(2H)-dione;
(3aα,4β,7β,7aα)-2-(4-Amino-2,3,5,6-tetrafluorophenyl)hexahydro-4,7-epoxy-1H-isoindole-1,3(2H)-dione;
(3aα,4β,7β,7aα)-2-(4-Bromo-3-chlorophenyl)hexahydro-4,7-epoxy-1H-isoindole-1,3(2H)-dione;
(3aα,4β,7β,7aα)-Hexahydro-2-(5-hydroxy-1-naphthalenyl)-4,7-epoxy-1H-isoindole-1,3(2H)-dione;
(3aα,4β,7β,7aα)-4-(Octahydro-1,3-dioxo-4,7-epoxy-2H-isoindol-2-yl)-2-(trifluoromethyl)benzonitrile;
(3aα,4β,7β,7aα)-2-(4-Morpholinyl)-5-(octahydro-1,3-dioxo-4,7-epoxy-2H-isoindol-2-yl)benzoic acid methyl ester;
(3aα,4β,7β,7aα)-2-Fluoro-5-(octahydro-1,3-dioxo-4,7-epoxy-2H-isoindol-2-yl)benzonitrile;
(3aα,4β,7β,7aα)-Hexahydro-2-(2-naphthalenyl)-4,7-epoxy-1H-isoindole-1,3(2H)-dione;
(3aα,4β,7β,7aα)-2-(9-Ethyl-9H-carbazol-3-yl)hexahydro-4,7-epoxy-1H-isoindole-1,3(2H)-dione;
(3aα,4β,7β,7aα)-2-[1,2-Dihydro-8-methyl-2-oxo-4-(trifluoromethyl)-7-quinolinyl]hexahydro-4,7-epoxy-1H-isoindole-1,3(2H)-dione;
(3aα,4α,7α,7aα)-Hexahydro-2-[3-(trifluoromethyl)phenyl]-4,7-epoxy-1H-isoindole-1,3(2H)-dione;
(3aα,4α,7α,7aα)-Hexahydro-2-(4-nitro-1-naphthalenyl)-4,7-epoxy-1 H-isoindole-1,3(2H)-dione;
(3aα,4β,7β,7aα)-2-(4-Bromo-3-methylphenyl)-3a,4,7,7a-tetrahydro-4,7-epoxy-1H-isoindole-1,3(2H)-dione;
(3aα,4β,7β,7aα)-3a,4,7,7a-Tetrahydro-2-(2-naphthalenyl)-4,7-epoxy-1H-isoindole-1,3(2H)-dione;
(3aα,4β,7β,7aα)-2-(9-Ethyl-9H-carbazol-3-yl)-3a,4,7,7a-tetrahydro-4,7-epoxy-1H-isoindole-1,3(2H)-dione;
(3aα,4β,7β,7aα)-2-[4-Fluoro-3-(trifluoromethyl)phenyl]-3a,4,7,7a-tetrahydro-4,7-epoxy-1H-isoindole-1,3(2H)-dione;
(3aα, 4β, 7β, 7aα)- 2-[1,2-Dihydro- 8-methyl- 2-oxo- 4-(trifluoromethyl)- 7-quinolinyl]- 3a, 4,7,7a-tetrahydro-4,7-epoxy-1H-isoindole-1,3(2H)-dione;
(3aα,4α,7α,7aα)-4-[(Acetyloxy)methyl]-2-(4-bromo-3-methylphenyl)-3a,4,7,7a-tetrahydro-4,7-epoxy-1H-isoindole-1,3(2H)-dione;
(3aα,4β,7β,7aα)-4-[(Acetyloxy)methyl]-2-(4-bromo-3-methylphenyl)-3a,4,7,7a-tetrahydro-4,7-epoxy-1H-isoindole-1,3(2H)-dione.;
(3aα,4β,7β,7aα)-Hexahydro-4,7-dimethyl-2-[3-(trifluoromethyl)phenyl]-4,7-epoxy-1H-isoindole-1,3(2H)-dione;
(3aα,4β,7β,7aα)-4-(Octahydro-4,7-dimethyl-1,3-dioxo-4,7-epoxy-2H-isoindol-2-yl)-1-naphthalenecarbonitrile;
(3aα,4β,7β,7aα)-(Benzo[b]thiophen-3-yl)hexahydro-4,7-dimethyl-4,7-epoxy1H-isoindole-1,3(2H)-dione;
(3aα,4β,7β,7aα)-Hexahydro-4,7-dimethyl-2-[4-nitro-3-(trifluoromethyl)phenyl]-4,7-epoxy-1H-isoindole-1,3(2H)-dione;
(3aα,4β,7β,7aα)-4-(1,3,3a,4,7,7a-Hexahydro-4,7-dimethyl-1,3-dioxo-4,7-epoxy-2H-isoindol-2-yl)-1-naphthalenecarbonitrile;
(3aα,4α,7α,7aα)-Hexahydro-4-methyl-2-(2-naphthalenyl)-4,7-epoxy-1H-isoindole-1,3(2H)-dione;
(3aα,4β,7β,7aα)-2-(4-Bromo-3-methylphenyl)hexahydro-4-methyl-4,7-epoxy-1 H-isoindote-1,3(2H)-dione;
(3aα,4β,7β,7aα)-Hexahydro-4-methyl-2-[3-(trifluoromethyl)phenyl]-4,7-epoxy-1H-isoindole-1,3(2H)-dione;
(3aα,4β,7β,7aα)-2-(3,5-Dichlorophenyl)hexahydro-4-methyl-4,7-epoxy-1H-isoindole-1,3(2H)-dione;
(3aα,4β,7β,7aα)-2-(3-Chloro-4-fluorophenyl)hexahydro-4-methyl-4,7-epoxy-1 H-isoindole-1,3(2H)-dione;
(3aα,4β,7β,7aα)-2-Methoxy-4-(octahydro-1,3-dioxo-4-methyl-4,7-epoxy-2H-isoindol-2-yl)-1-naphthalenecarbonitrile;
(3aα,4β,7β,7aα)-Hexahydro-4-methyl-2-[4-nitro-3-(trifluoromethyl)phenyl]-4,7-epoxy-1H-isoindole-1,3(2H)-dione;
(3aα,4β,7β,7aα)-Hexahydro-2-[4-(1H-imidazol-1-yl)phenyl]-4-methyl-4,7-epoxy-1H-isoindole-1,3(2H)-dione;
(3aα, 4β, 7β, 7aα)-2-[3-Chloro-4-(2-thiazolyl) phenyl] hexahydro-4-methyl-4,7-epoxy-1H-isoindole-1,3 (2H) -dione;
(3aα,4α,7α,7aα)-2-(3,5-Dichlorophenyl)hexahydro-4,7-imino-1H-isoindole-1,3(2H)-dione;
(3aα,4α,7α,7aα)-2-(4-Bromo-1-naphthalenyl)hexahydro-4,7-imino-1H-isoindole-1,3(2H)-dione;
(3aα,4α,7α,7aα)-2-(4-Bromo-3-methylphenyl)hexahydro-4,7-imino-1H-isoindole-1,3(2H)-dione;
(3aα,4α,7α,7aα)-Hexahydro-2-(4-nitro-1-naphthalenyl)-4,7-imino-1H-isoindole-1,3(2H)-dione;
(3aα,4α,7α,7aα)-8-Acetyl-2-(3,5-dichlorophenyl)hexahydro-4,7-imino-1H-isoindole-1,3(2H)-dione;
(3aα, 4α, 7α, 7aα) -Octahydro-1,3-dioxo-2-[3-(trifluoromethyl) phenyl]-4,7-ethano-5H-pyrrolo [3,4-c] pyridine-5-carboxylic acid phenyl ester;
(3aα,4α,7α,7aα)-4-(Octahydro-1,3-dioxo-4,7-ethano-2H-pyrrolo[3,4-c]pyridin-2-yl)-1-naphthalenecarbonitrile;
(3aα,4α,7α,7aα)-4-(Octahydro-5-methyl-1,3-dioxo-4,7-ethano-2H-pyrrolo[3,4-c]pyridin-2-yl)-1-naphthalenecarbonitrile;
(3aα,4α,7α,7aα)-2-(4-Cyano-1-naphthalenyl)octahydro-1,3-dioxo-4,7-etheno-5H-pyrrolo[3,4-c]pyridine-5-carboxylic acid phenylmethyl ester;
(3aα,4α,7α,7aα)-4-(Octahydro-1,3-dioxo-4,7-ethano-2H-pyrrolo[3,4-c]pyridin-2-yl)-2-(trifluoromethyl)benzonitrile;
(3aα, 4α, 7α, 7aα)-4-(Octahydro-5-methyl-1,3-dioxo-4,7-ethano-2H-pyrrolo [3,4-c] pyridin-2-yl)-2-(trifluoromethyl)benzonitrile;
(3aα,4α,7α,7aα)-2-[4-Cyano-3-(trifluoromethyl)phenyl]octahydro-1,3-dioxo-4,7-etheno-5H-pyrrolo[3,4-c]pyridine-5-carboxylic acid phenylmethyl ester;
(3aα,4α,7α,7aα)-2-[4-Bromo-3-(trifluoromethyl)phenyl]tetrahydro-5-methyl-4,7-etheno-1H-pyrrolo[3,4-c]pyridine-1,3,6(2H,5H)-trione;
(3aα, 4α, 7α, 7aα) -Tetrahydro-5-methyl-2-[3-(trifluoromethyl) phenyl]-4,7-etheno-1H-pyrrolo [3,4-c] pyridine-1,3,6(2H,5H)-trione;
(3aα,4α,7α,7aα)-Tetrahydro-5-methyl-2-(2-naphthalenyl)-4,7-etheno-1H-pyrrolo[3,4-c]pyridine-1,3,6(2H,5H) -trione;
(1aα,2β,2aα,5aα,6β,6aα)-Hexahydro-4-[3-(trifluoromethyl)phenyl]-2,6-epoxy-3H-oxireno[f]isoindole-3,5(4H) -dione;
(1aα,2β,2aα,5aα,6β,6aα)-4-(3,5-Dichlorophenyl)hexahydro-2,6-epoxy-3H-oxireno[f]isoindole-3,5(4H)-dione;
(1aα,2β,2aα,5aα,6β,6aα)-Hexahydro-4-(4-nitro-1-naphthalenyl)-2,6-epoxy-3H-oxireno[f]isoindole-3,5(4H)-dione;
(1aα,2β,2aα,5aα,6β,6aα)-4-(3,4-Dichlorophenyl)hexahydro-2,6-epoxy-3H-oxireno[f]isoindole-3,5(4H)-dione;
2-[4-(4-Bromophenoxy)phenyl]-3a,4,7,7a-tetrahydro-4,7-dimethyl-4,7-epoxy-1 H-isoindole-1,3(2H)-dione;
3a,4,7,7a-Tetrahydro-2-(2-methoxyphenyl)-4,7-dimethyl-4,7-epoxy-1H-isoindole-1,3(2H) diane;
[(1,2,3,3a,7,7a-Hexahydro-2-phenyl-4,7-epoxy-4H-isoindol-4-yl)methyl]carbamic acid (3,5-dimethoxyphenyl) methyl ester;
2-(2,4-Dimethylphenyl)-3a,4,7,7a-tetrahydro-4-(hydroxymethyl)-4,7-epoxy-1H-isoindole-1,3(2H)-dione;
2-(1,3-Benzodioxol-5-yl)-3a,4,7,7a-tetrahydro-4-methyl-4,7-epoxy-1H-isoindole-1,3(2H)-dione;
4-[Bis(acetyloxy)methyl]-2-(3-bromophenyl)-3a,4,7,7a-tetrahydro-4,7-epoxy-1 H-isoindole-1,3(2H)-dione;
N-[[1,2,3,3a, 7,7a-Hexahydro-2-(2,4,6-trimethylphenyl)-4,7-epoxy-4H-isoindol-4-yl] methyl]-2,2-dimethylpropanamide;
3a,4,7,7a-Tetrahydro-4-(hydroxymethyl)-2-[2-(trifluoromethyl)phenyl]-4,7-epoxy-1H-isoindole-1,3(2H)-dione;
3a,4,7,7a-Tetrahydro-4-(hydroxymethyl)-2-(1-naphthalenyl)-4,7-epoxy-1H-isoindole-1,3(2H)-dione;
2-Chloro-5-(1,3,3a,4,7,7a-hexahydro-4,7-dimethyl-4,7-epoxy-2H-isoindol-2-yl)benzoic acid methyl ester;
4-[Bis(acetyloxy)methyl]-2-(4-bromo-2-nitrophenyl)-3a,4,7,7a-tetrahydro-4,7-epoxy-1H-isoindole-1,3(2H)-dione;
3a,4,7,7a-Tetrahydro-4-methyl-2-(4-methyl-3-nitrophenyl)-4,7-epoxy-1H-isoindole-1,3(2H)-dione;

2-[2-Chloro-5-(trifluoromethyl)phenyl]-3a,4,7,7a-tetrahydro-4-methyl-4,7-epoxy-1H-isoindole-1,3(2H)-dione;
2-[4-Chloro-3-(trifluoromethyl)phenyl]-3a,4,7,7a-tetrahydro-4,7-dimethyl-4,7-epoxy-1H-isoindole-1,3(2H)-dione;
2-(1,3,3a,4,7,7a-Hexahydro-4-methyl-4,7-epoxy-2H-isoindol-2-yl)benzonitrile; 2-(4-Fluorophenyl)-3a,4,7,7a-tetrahydro-4-methyl-4,7-epoxy-1H-isoindole-1,3(2H)-dione;
2,2,2-Trifluoro-N-[(1,2,3,3a,7,7a-hexahydro-2-phenyl-4,7-epoxy-4H-isoindol-4-yl)methyl]acetamide;
3a,4,7,7a-Tetrahydro-4,7-dimethyl-2-(4-methyl-3-nitrophenyl)-4,7-epoxy-1H-isoindole-1,3(2H)-dione;
2-Chloro-5-[1,3,3a,4,7,7a-hexahydro-4-(hydroxymethyl)-4,7-epoxy-2H-isoindol-2-yl]benzoic acid;
3a,4,7,7a-Tetrahydro-4,7-dimethyl-2-(4-nitrophenyl)-4,7-epoxy-1H-isoindole-1,3(2H)-dione;
3a,4,7,7a-Tetrahydro-2-(2-mercaplophenyl)-4,7-epoxy-1H-isoindole-1,3(2H)-dione;
3a,4,7,7a-Tetrahydro-2-[2-[(phenylmethyl)thio]phenyl]-4,7-epoxy-1H-isoindole-1,3(2H)-dione;
[[2-(4-Chlorophenyl)-1,2,3,3a,7,7a-hexahydro-4,7-epoxy-4H-isoindol-4-yl]methyl]carbamic acid 2-methylpropyl ester;
4-(1,1-Dimethylethyl)-N-[[1,2,3,3a,7,7a-hexahydro-2-(4-methylphenyl)-4,7-epoxy-4H-isoindol-4 yl]methyl]beniamide;
2,4-Dichloro-N-[[1,2,3,3a,7,7a-hexahydro-2-(4-nitrophenyl)-4,7-epoxy-4H-isoindol-4-yl]methyl]benzamide;
N-[[2-(4-Chlorophenyl)- 1,2,3,3a, 7,7a-hexahydro- 4,7-epoxy- 4H-isoindol- 4-yl] methyl]- 2,4,6-trimethylbenzenesulfonamide;
[(1,2,3,3a,7,7a-Hexahydro-2-phenyl-4,7-epoxy-4H-isoindol-4-yl)methyl]carbamic acid 1,1-dimethylethyl ester;
N-[(1,2,3,3a,7,7a-Hexahydro-2-phenyl-4,7-epoxy-4H-isoindol-4-yl)methyl]-2-phenoxyacetamide;
N-[[1,2,3,3a,7,7a-Hexahydro-2-(4-nitrophenyl)-4,7-epoxy-4H-isoindol-4-yl]methyl]-2,2-dimethylpropanamide;
2-(2,4-Dichlorophenoxy) -N-[[1,2,3,3a, 7,7a-hexahydro- 2-(4-nitrophenyl)- 4,7-epoxy- 4H-isoindol- 4-yl] methyl] acetamide;
N-[[1,2,3,3a, 7,7a-Hexahydro- 2-(4-methylphenyl)- 4,7-epoxy- 4H-isoindol- 4-yl] methyl]- 3,5-dimethoxybenzamide;
N-[[2-(4-Chlorophenyl)- 1,2,3,3a, 7,7a-hexahydro- 4,7-epoxy- 4H-isoindol- 4-yl] methyl]- 2-nitrobenzenesulfonamide;
(3aα,4β,7β,7aα)-Hexahydro-2-[(1S)-1-phenylethyl]-4,7-epoxy-1H-isoindole-1,3(2H)-dione;
(3aα,4β,7β,7aα)-Hexahydro-2-[(1S)-2-hydroxy-1-phenylethyl]-4,7-epoxy-1H-isoindole-1,3(2H)-dione;
(3aα, 4β, 7β, 7aα)- 2-[(1S)- 2-(Acetyloxy)- 1-phenylethyl]- 3a, 4,7,7a-tetrahydro- 4,7-epoxy- 1H-isoindole- 1,3 (2H) -dione;
(3aα,4α,7α,7aα)-3a,4,7,7a-Tetrahydro-2-[(1S)-1-phenylethyl]-4,7-epoxy-1H-isoindole-1,3(2H)-dione;
(3aα,4β,7β,7aα)-Hexahydro-2-[(1R)-1-phenylethyl]-4,7-epoxy-1H-isoindole-1,3(2H)-dione;
(3aα,4β,7β,7aα)-4-[[(Octahydro-1,3-dioxo-4,7-epoxy-2H-isoindol-2-yl)methyl]amino]benzoic acid;
(3aα,4β,7β,7aα)-Hexahydro-2-(4-morpholinylmethyl)-4,7-epoxy-1H-isoindole-1,3(2H)-dione
(3aα,4β,7β,7aα)-4-[Octahydro-4-(2-hydroxyethyl)-7-methyl-1,3-dioxo-4,7-epoxy-2H-isoindol-2-yl]-2-(trifluoromethyl)benzonitrile;
(3aα,4β,7β,7aα)- and (3aα,4α,7α,7aα)-4-[Octahydro-4-methyl-1,3-dioxo-7-(phenylmethyl)-4,7-epoxy-2H-isoindol-2-yl]-2-(trifluoromethyl)benzonitrile;
(3aα, 4β, 7β, 7aα)-4-[7-[2-(4-Bromophenoxy) ethyl] octahydro- 4-methyl- 1,3-dioxo- 4,7-epoxy- 2H-isoindol- 2-yl]-2-(trifluoromethyl)benzonitrile;
(3aα, 4β, 7β, 7aα)- 4-[Octahydro- 7-[2-(4-iodophenoxy) ethyl]- 4-methyl- 1,3-dioxo- 4,7-epoxy- 2H-isoindol- 2-yl]-2-(trifluoromethyl)benzonitrile;
(3aα,4β,7β,7aα)-4-[Octahydro-4-methyl-1,3-dioxo-7-[2-[4-(trifluoromethyl)phenoxy]ethyl]-4,7-epoxy-2H-isoindol-2-yl]-2-(trifluoromethyl)benzonitrile;
(3aα, 4β, 7β, 7aα)- 4-[Octahydro- 7-[2-(4-methoxyphenoxy) ethyl]- 4-methyl- 1,3-dioxo- 4,7-epoxy- 2H-isoindol-2-yl]-2-(trifluoromethyl)benzonitrile;
(3aα, 4β, 7β, 7aα)-4-[7-[2-(4-Ethoxyphenoxy) ethyl] octahydro- 4-methyl- 1,3-dioxo- 4,7-epoxy- 2H-isoindol- 2-yl]-2-(trifluoromethyl)benzonitrile;
(3aα, 4β, 7β, 7aα)-4-[7-[2-(4-Chlorophenoxy) ethyl] octahydro- 4-methyl- 1,3-dioxo- 4,7-epoxy- 2H-isoindol- 2-yl]-2-(trifluoromethyl)benzonitrile;
(3aα,4β,7β,7aα)-4-[2-[2-[4-Cyano-3-(trifluoromethyl)phenyl]octahydro-7-methyl-1,3-dioxo-4,7-epoxy-4H-isoindol-4-yl]ethoxy]benzoic acid, methyl ester;
(3aα,4β,7β,7aα)-Hexahydro-4-(2-hydroxyethyl)-7-methyl-2-(3-methyl-4-nitrophenyl)-4,7-epoxy-1H-isoindole-1,3(2H)-dione;
(3aα,4β,7β,7aα)-4-[Octahydro-4-methyl-1,3-dioxo-7-[2-[4-(trifluoromethoxy)phenoxy]ethyl]-4,7-epoxy-2H-isoindol-2-yl]-2-(trifluoromethyl)benzonitrile;
(3aα,4β,7β,7aα)-2-(3,5-Dichlorophenyl)hexahydro-4,7-dimethyl-4,7-epoxy-1H-isoindole-1,3(2H)-dione;

(3aα,4β,7β,7aα)-Hexahydro-4,7-dimethyl-2-(4-nitro-1-naphthalenyl)-4,7-epoxy-1H-isoindole-1,3(2H)-dione;
(3aα,4β,7β,7aα)-2-[4-Cyano-3-(trifluoromethyl)phenyl]octahydro-7-methyl-1,3-dioxo-4,7-epoxy-4H-isoindole-4-propanenitrile;
(3aα, 4β, 7β, 7aα)- 4-[Octahydro- 4-methyl- 7-[2-(4-morpholinyl) ethyl]- 1,3-dioxo- 4,7-epoxy- 2H-isoindol- 2-yl]-2-(trifluoromethyl)benzonitrile, trifluoroacetate;
(3aα,4β,7β,7aα)-2-(5-Fluoro-1-naphthalenyl)hexahydro-4,7-dimethyl-4,7-epoxy-1H-isoindole-1,3(2H)-dione;
(3aα,4β,7β,7aα)-2-(5-Fluoro-4-nitro-1-naphthalenyl)hexahydro-4,7-dimethyl-4,7-epoxy-1H-isoindole-1,3(2H)-dione;
(3aα, 4β, 7β, 7aα)- 2-(1,1-Dioxidobenzo [b] thiophen- 3-yl) hexahydro- 4,7-dimethyl- 4,7-epoxy- 1H-isoindole- 1,3 (2H)-dione;
4-(1,3,3a,4,7,7a-Hexahydro-4,6,7-trimethyl-1,3-dioxo-4,7-epoxy-2H-pyrrolo[3,4-c]pyridin-2-yl)-2-(trifluoromethyl)benzonitrile;
(3aα,4β,7β,7aα)-Tetrahydro-4,7-dimethyl-2-[3-(trifluoromethyl)phenyl]-4,7-epoxy-1H-isoindole-1,3,5(2H,4H)-trione;
(3aα,4α,7α,7aα)-Tetrahydro-4,7-dimethyl-2-[3-(trifluoromethyl)phenyl]-4,7-epoxy-1H-isoindole-1,3,5(2H,4H)-trione;
(3aα,4β,7β,7aα)-2-(5-Chloro-1-naphthalenyl)hexahydro-4,7-dimethyl-4,7-epoxy-1H-isoindole-1,3(2H)-dione;
(3aα,4β,7β,7aα)-2-(5-Chloro-4-nitro-1-naphthalenyl)hexahydro-4,7-dimethyl-4,7-epoxy-1H-isoindole-1,3(2H)-dione;
(3aα, 4β, 7β, 7aα)- 4-Ethylhexahydro- 7-methyl- 2-(4-nitro- 1-naphthalenyl)- 4,7-epoxy- 1H-isoindole- 1,3 (2H) -dione;
(3aα, 4β, 7β, 7aα)- 2-(4-Cyano- 1-naphthalenyl) -N-(4-fluorophenyl) octahydro- 7-methyl- 1,3-dioxo- 4,7-epoxy-4H-isoindole-4-acetamide;
(3aα,4β,7β,7aα)-Hexahydro-4-methyl-2-(2-naphthalenyl)-4,7-epoxy-1H-isoindole-1,3(2H)-dione, faster eluting enantiomer;
(3aα,4β,7β,7aα)-Hexahydro-4-methyl-2-(2-naphthalenyl)-4,7-epoxy-1H-isoindole-1,3(2H)-dione, slower eluting enantiomer;
(3aα, 4β, 7β, 7aα)- 4-[4-[2-[[(4-Fluorophenyl) methyl] methylamino] ethyl] octahydro- 7-methyl- 1,3-dioxo-4,7-epoxy-2H-isoindol-2-yl]-2-(trifluoromethyl)benzonitrile;
(3aα, 4β, 5β, 6β, 7β, 7aα)- 4-(Octahydro- 4,5,6,7-tetramethyl- 1,3-dioxo- 4,7-epoxy- 2H-isoindol- 2-yl)- 2-(trifluoromethyl)benzonitrile;
(3aα,4β,7β,7aα)-4-[Octahydro-4-methyl-1,3-dioxo-7-[2-[4-(trifluoromethyl)phenoxy]ethyl]-4,7-epoxy-2H-isoindol-2-yl]-2-(trifluoromethyl)benzonitrile, faster eluting antipode;
(3aα,4β,7β,7aα)-4-[Octahydro-4-methyl-1,3-dioxo-7-[2-[4-(trifluoromethyl)phenoxy]ethyl]-4,7-epoxy-2H-isoindol-2-yl]-2-(trifluoromethyl)benzonitrile, slower eluting enantiomer;
(3aα, 4β, 5β, 7β, 7aα)- 4-(Octahydro- 5-hydroxy- 4,7-dimethyl- 1,3-dioxo- 4,7-epoxy- 2H-isoindol- 2-yl)- 2-(trifluoromethyl)benzonitrile;
(3aα, 4β, 5α, 7β, 7aα)- 4-(Octahydro- 5-hydroxy- 4,7-dimethyl- 1,3-dioxo- 4,7-epoxy- 2H-isoindol- 2-yl)- 2-(trifluoromethyl)benzonitrile;
(αR)-α-Methoxybenzeneacetic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester;
(3aα,4β,7β,7aα)-2-(Methylthio)-4-(octahydro-4,7-dimethyl-1,3-dioxo-4,7-epoxy-2H-isoindol-2-yl)benzonitrile;
(3aα,4β,7β,7aα)-2-(Methylsulfinyl)-4-(octahydro-4,7-dimethyl-1,3-dioxo-4,7-epoxy-2H-isoindol-2-yl)benzonitrile;
(3aα,4β,7β,7aα)-2-(Methylsulfonyl)-4-(octahydro-4,7-dimethyl-1,3-dioxo-4,7-epoxy-2H-isoindol-2-yl)benzonitrile;
(3aα,4β,5β,7β,7aα)-7-[2-[[(1,1-Dimethylethyl)dimethylsilyl]oxy]ethyl]hexahydro-5-hydroxy-4-methyl-2-(4-nitro-1-naphthalenyl)-4,7-epoxy-1H-isoindole-1,3(2H)-dione;
(3aα,4β,5β,7β,7aα)-Hexahydro-5-hydroxy-7-(2-hydroxyethyl)-4-methyl-2-(4-nitro-1-naphthalenyl)-4,7-epoxy-1H-isoindole-1,3(2H)-dione;
(3aα,4β,5β,7β,7aα)-7-[2-(4-Fluorophenoxy)ethyl] hexahydro- 5-hydroxy-4-methyl- 2-(4-nitro- 1-naphthalenyl)-4,7-epoxy-1H-isoindole-1,3(2H)-dione;
(3aα,4β,5β,6β,7β,7aα)-4-(Octahydro-5,6-dihydroxy-4,7-dimethyl-1,3-dioxo-4,7-epoxy-2H-isoindol-2-yl)-2-(trifluoromethyl)benzonitrile;
(3aα,4β,5α,6α,7β,7aα)-4-(Octahydro-5,6-dihydroxy-4,7-dimethyl-1,3-dioxo-4,7-epoxy-2H-isoindol-2-yl)-2-(trifluoromethyl)benzonitrile;
3aα,4β,5β,6β,7β,7aα)-4-[Octahydro-5,6-dihydroxy-4-(hydroxyethyl)-7-methyl-1,3-dioxo-4,7-epoxy-2H-isoindol-2-yl]-1-naphthalenecarbonitrile;

(3aα, 4β, 5β, 6β, 7β, 7aα)- 4-[Octahydro- 5,6-dihydroxy- 4-methyl- 1,3-dioxo- 7-[2-[4-(trifluoromethyl) phenoxy] ethyl]-4,7-epoxy-2H-isoindol-2-yl]-2-(trifluoromethyl)benzonitrile;
(3aα, 4β, 5β, 5aβ, 8aβ, 8bα)- 4-(Decahydro- 5-hydroxy- 4-methyl- 1,3-dioxo- 4,8a-epoxy- 2H-furo [3,2-e] isoindol-2-yl)-1-naphthalenecarbonitrile;
(3aα,4β,7β,7aα)-2-(4-Cyano-1-naphthalenyl)octahydro-7-methyl-1,3-dioxo-4,7-epoxy-4H-isoindole-4-acetic acid;
(3aα,4β,7β,7aα)-2-(4-Cyano-1-naphthalenyl)octahydro-7-methyl-1,3-dioxo-4,7-epoxy-4H-isoindole-4-acetic acid, methyl ester;
(3aα, 4β, 7β, 7aα)- 2-(4-Cyano- 1-naphthalenyl) -N-[(4-fluorophenyl) methyl] octahydro- 7-methyl- 1,3-dioxo-4,7-epoxy-4H-isoindole-4-acetamide;
(3aα,4β,7β,7aα)-N-[2-[2-(4-Cyano-1-naphthalenyl)octahydro-7-methyl-1,3-dioxo-4,7-epoxy-4H-isoindol-4-yl] ethyl]-4-fluorobenzamide;
[3aR-(3aα, 4β, 7β, 7aα)]- 4-[Octahydro- 4-(2-hydroxyethyl)- 7-methyl- 1,3-dioxo- 4,7-epoxy- 2H-isoindol- 2-yl]-1-naphthalenecarbonitrile;
[3aS-(3aα, 4β, 7β, 7aα)]- 4-[Octahydro- 4-(2-hydroxyethyl)- 7-methyl- 1,3-dioxo- 4,7-epoxy- 2H-isoindol- 2-yl]-1-naphthalenecarbonitrile;
[3aR-(3aα,4β,7β,7aα)]-4-[4-[2-(3-Fluorophenoxy)ethyl]octahydro-7-methyl-1,3-dioxo-4,7-epoxy-2H-isoindol-2-yl]-1-naphthalenecarbonitrile;
[3aS-(3aα,4β,7β,7aα)]-4-[4-[2-(3-Fluorophenoxy)ethyl]octahydro-7-methyl-1,3-dioxo-4,7-epoxy-2H-isoindol-2-yl]-1-naphthalenecarbonitrile;
(4-Fluorophenyl)carbamic acid, 2-(3aα,4β,7β,7aα)-2-(4-cyano-1-naphthalenyl)octahydro-7-methyl-1,3-dioxo-4,7-epoxy-4H-isoindol-4-yl]ethyl ester;
(3aα, 4β, 7β, 7aα)- 4-[Octahydro- 4-(2-hydroxyethyl)- 1,3-dioxo- 4,7-epoxy- 2H-isoindol- 2-yl]- 1-naphthalenecarbonitrile;
(3aα, 4β, 6β, 7β, 7aα)- 4-[4-[2-(4-Cyanophenoxy) ethyl] octahydro- 6-hydroxy- 1,3-dioxo- 4,7-epozy- 2H-isoindol-2-yl]-1-naphthalenecarbonitrile;
[3aS-(3aα,4β,5β,7β,7aα)]-4-[Octahydro-5-hydroxy-7-(2-hydroxyethyl)-4-methyl-1,3-diozo-4,7-epoxy-2H-isoindol-2-yl]-1-naphthalenecarbonitrile;
[3aR-(3aα,4β,5β,7β,7aα)]-4-[Octahydro-5-hydroxy-7-(2-hydroxyethyl)-4-methyl-1,3-dioxo-4,7-epoxy-2H-isoindol-2-yl]-1-naphthalenecarbonitrile;
(3aα, 4β, 7β, 7aα)- 4-[4-[2-(4-Cyanophenoxy) ethyl]- 7-ethyloctahydro- 1,3-dioxo- 4,7-epoxy- 2H-isoindol- 2-yl]-1-naphthalenecarbonitrile;
(3aα, 4β, 7β, 7aα)- 4-[2-(Acetyloxy) ethyl]- 2-(4-cyano- 1-naphthalenyl) hexahydro- 7-methyl- 4,7-epoxy- 1H-isoindole-1,3(2H)-dione;
(3aα,4β,7β,7aα)-4-[Octahydro-4-methyl-1,3-dioxo-7-(2-oxoethyl)-4,7-epoxy-2H-isoindol-2-yl]-1-naphthalenecarbonitrile;
[3aα,4β(E),7β,7aα]-4-[4-[3-(4-Cyanophenyl)-2-propenyl]octahydro-7-methyl-1,3-dioxo-4,7-epoxy-2H-isoindol-2-yl]-1-naphthalenecarbonitrile;
[3aα,4β(Z),7β,7aα]-4-[4-[3-(4-Cyanophenyl)-2-propenyl]octahydro-7-methyl-1,3-dioxo-4,7-epoxy-2H-isoindol-2-yl]-1-naphthalenecarbonitrile;
(3aα, 4β, 7β, 7aα)- 4-[4-[3-(4-Cyanophenyl) propyl] octahydro- 7-methyl- 1,3-dioxo- 4,7-epoxy- 2H-isoindol- 2-yl]-1-naphthalenecarbonitrile;
(3aα,4β,7β,7aα)-4-[4-[2-[(6-Chloro-1,2-benzisoxazol-3-yl)oxy]ethyl]octahydro-7-methyl-1,3-dioxo-4,7-epoxy-2H-isoindol-2-yl]-1-naphthalenecarbonitrile;
(3aα,4β,7β,7aα)-4-[Octahydro-4-methyl-7-[2-[(6-nitro-1H-indazol-3-yl)oxy]ethyl]-1,3-dioxo-4,7-epoxy-2H-isoindol-2-yl]-1-naphthalenecarbonitrile;
[3aS-(3aα, 4β, 5β, 7β, 7aα)]- 4-[7-[2-(1,2-Benzisoxazol- 3-yloxy) ethyl] octahydro- 5-hydroxy- 4-methyl- 1,3-dioxo-4,7-epoxy-2H-isoindol-2-yl]-1-naphthalenecarbonitrile;
[3aR-(3aα, 4β, 5β, 7β, 7aα)]- 4-[7-[2-(1,2-Benzisoxazol- 3-yloxy) ethyl] octahydro- 5-hydroxy- 4-methyl- 1,3-dioxo-4,7-epoxy-2H-isoindol-2-yl]-1-naphthalenecarbonitrile;
(3aα, 4β, 5β, 7β, 7aα)- 4-(Octahydro- 5-hydroxy- 4,7-dimethyl- 1,3-dioxo- 4,7-epoxy- 2H-isoindol- 2-yl)- 2-(trifluoromethyl)benzonitrile;
(3aα, 4β, 5β, 7β, 7aα)- 4-(Octahydro- 5-hydroxy- 4,7-dimethyl- 1,3-dioxo- 4,7-epoxy- 2H-isoindol- 2-yl)- 2-(trifluoromethyl)benzonitrile;
(3aα, 4β, 7β, 7aα)- 2-(4-Cyano- 1-naphthalenyl) octahydro- 1,3-dioxo- 7-[2-(phenylmethoxy) ethyl]- 4,7-epoxy-4H-isoindole-4-propanenitrile;
(3aα, 4α, 7α, 7aα)- 2-(4-Cyano- 1-naphthalenyl) octahydro- 1,3-dioxo- 7-[2-(phenylmethoxy) ethyl]- 4,7-epoxy-4H-isoindole-4-propanenitrile;

(3aα,4β,7β,7aα)-2-(4-Cyano-1-naphthalenyl)octahydro-7-(2-hydroxyethyl)-1,3-dioxo-4,7-epoxy-4H-isoindole-4-propanenitrile;
(3aα,4α,7α,7aα)-2-(4-Cyano-1-naphthalenyl)octahydro-7-(2-hydroxyethyl)-1,3-dioxo-4,7-epoxy-4H-isoindole-4-propanenitrile;
(3aα, 4β, 7β, 7aα)- 2-(4-Cyano- 1-naphthalenyl)- 7-[2-(4-fluorophenoxy) ethyl] octahydro- 1,3-dioxo- 4,7-epoxy-4H-isoindole-4-propanenitrile;
(3aα, 4β, 7β, 7aα)- 2-(7-Chloro- 2,1,3-benzoxadiazol- 4-yl) hexahydro- 4,7-dimethyl- 4,7-epoxy- 1H-isoindole- 1,3 (2H)-dione;
(3aα, 4β, 7β, 7aα)- 2-(7-Chloro- 2-methyl- 4-benzofuranyl) hexahydro- 4,7-dimethyl- 4,7-epoxy- 1H-isoindole- 1,3 (2H)-dione;
(3aα,4β,7β,7aα)-2-(7-Chloro-2-methylbenzo[b]thiophen-4-yl)hexahydro-4,7-dimethyl-4,7-epoxy-1H-isoindole-1,3(2H)-dione;
[3aα,4β(E),7β,7aα]-4-[2-(4-Cyano-1-naphthalenyl)octahydro-7-methyl-1,3-dioxo-4,7-epoxy-4H-isoindol-4-yl]-2-butenoic acid, phenylmethyl ester;
(3aα,4β,7β,7aα)-2-(4-Cyano-1-naphthalenyl)octahydro-7-methyl-1,3-dioxo-4,7-epoxy-4H-isoindole-4-butanoic acid;
(3aα, 4β, 7β, 7aα)- 2-(4-Cyano- 1-naphthalenyl) -N-(4-nuorophenyl) octahydro- 7-methyl- 1,3-dioxo- 4,7-epoxy-4H-isoindole-4-butanamide;
[3aS-(3aα,4β,5β,7β,7aα)]-4-[7-[2-(Acetyloxy)ethyl]octahydro-5-hydroxy-4-methyl-1,3-dioxo-4,7-epoxy-2H-isoindol-2-yl]-1-naphthalenecarbonitrile;
[3aR-(3aα,4β,5β,7β,7aα)]-4-[Octahydro-5-hydroxy-7-(2-hydroxyethyl)-4-methyl-1,3-dioxo-4,7-epoxy-2H-isoindol-2-yl]-1-naphthalenecarbonitrile;
[(3aα, 4β, 7β, 7aα (E)]-4-[Octahydro-4-methyl-1,3-dioxo-7-(4-oxo-4-phenyl-2-butenyl)-4,7-epoxy-2H-isoindol-2-yl]-1-naphthalenecarbonitrile;
[ (3aα, 4β, 7β, 7aα (E)]- 4-[Octahydro- 4-methyl- 1,3-dioxo- 7-(4-oxo- 4-phenyl-butanyl)- 4,7-epoxy- 2H-isoindol-2-yl]-1-naphthalenecarbonitrile;
(3aα, 4β, 7β, 7aα)-4-[7-[2-(4-Bromophenoxy) ethyl] octahydro-4-methyl-1,3-dioxo-4,7-epoxy-2H-isoindol-2-yl]-2-(trifluoromethyl)benzonitrile;
(3aα, 4β, 7β, 7aα)- 4-[Octahydro- 7-[2-(4-iodophenoxy) ethyl]- 4-methyl- 1,3-dioxo- 4,7-epoxy- 2H-isoindol- 2-yl]-2-(trifluoromethyl)benzonitrile;
(3aα,4β,7β,7aα)-4-[Octahydro-4-methyl-1,3-dioxo-7-[2-[4-(trifluoromethyl)phenoxy]ethyl]-4,7-epoxy-2H-isoindol-2-yl]-2-(trifluoromethyl)benzonitrile;
(3aα, 4β, 7β, 7aα)- 4-[Octahydro- 7-[2-(4-methoxyphenoxy) ethyl]- 4-methyl- 1,3-dioxo- 4,7-epoxy- 2H-isoindol-2-yl]-2-(trifluoromethyl)benzonitrile;
(3aα, 4β, 7β, 7aα)-4-[7-[2-(4-Ethoxyphenoxy) ethyl] octahydro-4-methyl-1,3-dioxo-4,7-epoxy-2H-isoindol-2-yl]-2-(trifluoromethyl)benzonitrile;
(3aα, 4β, 7β, 7aα)-4-[7-[2-(4-Chlorophenoxy) ethyl] octahydro-4-methyl-1,3-dioxo-4,7-epoxy-2H-isoindol-2-yl]-2-(trifluoromethyl)benzonitrile;
(3aα,4β,7β,7aα)-4-[2-[2-[4-Cyano-3-(trifluoromethyl) phenyl] octahydro-7-methyl-1,3-dioxo-4,7-epoxy-4H-isoindol-4-yl]ethoxy]benzoic acid, methyl ester;
(3aα,4β,7β,7aα)-Hexahydro-4-(2-hydroxyethyl)-7-methyl-2-(3-methyl-4-nitrophenyl)-4,7-epoxy-1H-isoindole-1,3(2H)-dione;
(3aα,4β,7β,7aα)-4-[Octahydro-4-methyl-1,3-dioxo-7-[2-[4-(trifluoromethoxy)phenoxy]ethyl]-4,7-epoxy-2H-isoindol-2-yl]-2-(trifluoromethyl)benzonitrile;
(3aα,4β,7β,7aα)-2-(3,5-Dichlorophenyl)hexahydro-4,7-dimethyl-4,7-epoxy-1H-isoindole-1,3(2H)-dione;
(3aα,4β,7β,7aα)-Hexahydro-4,7-dimethyl-2-(4-nitro-1-naphthalenyl)-4,7-epoxy-1H-isoindole-1,3(2H)-dione;
(3aα,4β,7β,7aα)-4-[Octahydro-4-methyl-1,3-dioxo-7-[2-[4-(phenylmethoxy)phenoxy] ethyl]-4,7-epoxy-2H-isoindol-2-yl]-2-(trifluoromethyl)benzonitrile;
(3aα,4β,7β,7aα)-Hexahydro-4-(2-hydroxyethyl)-7-methyl-2-(4-nitro-1-naphthalenyl)-4,7-epoxy-1H-isoindole-1,3(2H)-dione;
(3aα, 4β, 7β, 7aα)- 4-[2-(4-Fluorophenoxy) ethyl] hexahydro- 7-methyl- 2-(3-methyl- 4-nitrophenyl)- 4,7-epoxy-1H-isoindole-1,3(2H)-dione;
(3aα, 4β, 7β, 7aα)- 4-[Octahydro- 4-methyl- 1,3-dioxo- 7-[2-[4-[(trifluoromethyl) thio] phenoxy] ethyl]- 4,7-epoxy-2H-isoindol-2-yl]-2-(trifluoromethyl)benzonitrile;
(3aα, 4β, 7β, 7aα)- 4-[Octahydro- 4-methyl- 7-[2-(4-nitrophenoxy) ethyl]- 1,3-dioxo- 4,7-epoxy- 2H-isoindol- 2-yl]-2-(trifluoromethyl)benzonitrile;
(3aα, 4β, 7β, 7aα)- 4-[2-(4-Fluorophenoxy) ethyl] hexahydro- 7-methyl- 2-(4-nitro- 1-naphthalenyl)- 4,7-epoxy-1H-isoindole-1,3(2H)-dione;

(3aα,4β,7β,7aα)-4-[Octahydro-7-methyl-1,3-dioxo-7-[2-[2-(trifluoromethyl)phenoxy]ethyl]-4,7-epoxy-2H-isoindol-2-yl]-2-(trifluoromethyl)benzonitrile;
(3aα,4β,7β,7aα)-4-[4-[2-(2-Bromophenoxy)ethyl]octahydro-7-methyl-1,3-dioxo-4,7-epoxy-2H-isoindol-2-yl]-2-(trifluoromethyl)benzonitrile;
(3aα,4β,7β,7aα)-4-[4-[2-(3-Fluorophenoxy)ethyl]octahydro-7-methyl-1,3-dioxo-4,7-epoxy-2H-isoindol-2-yl]-2-(trifluoromethyl)benzonitrile;
(3aα,4β,7β,7aα)-Hexahydro-2-[4-(1H-imidazol-1-yl)phenyl]-4-methyl-4,7-epoxy-1H-isoindole-1,3(2H)-dione;
(3aα,4β,7β,7aα)-2-[3-Chioro-4-(2-thiazolyl)phenyl]hexahydro-4-methyl-4,7-epoxy-1H-isoindole-1,3(2H)-dione;
(3aα,4β,7β,7aα)-Hexahydro-4,7-dimethyl-2-(3-methyl-4-nitrophenyl)-4,7-epoxy-1H-isoindole-1,3(2H)-dione;
(3aα,4β,7β,7aα)-Hexahydro-4,7-dimethyl-2-(2-methyl-4-nitrophenyl)-4,7-epoxy-1H-isoindole-1,3(2H)-dione;
(3aα,4β,7β,7aα)-2-(3,5-Dichlorophenyl)hexahydro-4-(2-hydroxyethyl)-7-methyl-4,7-epoxy-1H-isoindole-1,3(2H)-dione;
(3aα,4β,7β,7aα)-2-(3,5-Dichlorophenyl)-4-[2-(4-fluorophenoxy)ethyl]hexahydro-7-methyl-4,7-epoxy-1H-ispindole-1,3(2H)-dione;
(3aα,4β,7β,7aα)-4-[Octahydro-4-[2-(4-hydroxyphenoxy)ethyl]-7-methyl-1,3-dioxo-4,7-epoxy-2H-isoindol-2-yl]-2-(trifluoromethyl)benzonitrile;
(3aα,4β,7β,7aα)-4-[4-[2-(4-Cyanophenoxy)ethyl]octahydro-7-methyl-1,3-dioxo-4,7-epoxy-2H-isoindol-2-yl]-2-(trifluoromethyl)benzonitrile;
(3aα,4β,7β,7aα)-4-[Octahydro-4-methyl-1,3-dioxo-7-[2-[3-(trifluoromethyl)phenoxy]ethyl]-4,7-epoxy-2H-isoindol-2-yl]-2-(trifluoromethyl)benzonitrile;
(3aα,4β,7β,7aα)-4-[4-[2-(3-Bromophenoxy)ethyl]octahydro-7-methyl-1,3-dioxo-4,7-epoxy-2H-isoindol-2-yl]-2-(trifluoromethyl)benzonitrile;
(3aα, 4β, 7β, 7aα)-4-[4-[(4-Fluorophenyl) methyl] octahydro-7-methyl-1,3-dioxo-4,7-epoxy-2H-isoindol-2-yl]-2-(trifluoromethyl)benzonitrile;
(3aα,4β,7β,7aα)-2-(1,6-Dihydro-1-methyl-6-oxo-3-pyridinyl)hexahydro-4,7-dimethyl-4,7-epoxy-1H-isoindole-1,3(2H)-dione;
(3aα,4β,7β,7aα)-Hexahydro-4,7-dimethyl-2-(1-methyl-6-oxo-3-piperidinyl)-4,7-epoxy-1H-isoindole-1,3(2H)-dione;
(3aα,4β,7β,7aα)-4-[4-[2-(3-Cyanophenoxy)ethyl]octahydro-7-methyl-1,3-dioxo-4,7-epoxy-2H-isoindol-2-yl]-2-(trifluoromethyl)benzonitrile;
(3aα,4β,7β,7aα)-4-[2-[4-Cyano-3-(trifluoromethyl)phenyl]octahydro-7-methyl-1,3-dioxo-4,7-epoxy-4H-isoindol-4-yl]ethoxy]benzoic acid, phenylmethyl ester;
(3aα,4β,7β,7aα)-4-[Octahydro-4-methyl-1,3-dioxo-7-(2-phenoxyethyl)-4,7-epoxy-2H-isoindol-2-yl]-2-(trifluoromethyl)benzonitrile;
(3aα,4β,7β,7aα)-2-(3,5-Dichloro-4-nitrophenyl)hexahydro-4,7-dimethyl-4,7-epoxy-1H-isoindole-1,3(2H)-dione;
(3aα,4β,7β,7aα)-2-(3,5-Dichloro-4-hydroxyphenyl)hexahydro-4,7-dimethyl-4,7-epoxy-1H-isoindole-1,3(2H)-dione;
(3aα,4β,7β,7aα)-2-(5-Fluoro-1-naphthalenyl)hexahydro-4,7-dimethyl-4,7-epoxy-1H-isoindole-1,3(2H)-dione;
(3aα,4β,7β,7aα)-Hexahydro-4,7-dimethyl-2-(1-naphthalenyl)-4,7-epoxy-1H-isoindole-1,3(2H)-dione;
(3aα,4β,7β,7aα)-Hexahydro-2-[3-methoxy-4-(5-oxazolyl)phenyl]-4,7-dimethyl-4,7-epoxy-1H-isoindole-1,3(2H)-dione;
(3aα,4β,7β,7aα)-Hexahydro-4-[2-(4-methoxyphenoxy)ethyl]-7-methyl-2-(4-nitro-1-naphthalenyl)-4,7-epoxy-1H-isoindole-1,3(2H)-dione;
(3aα, 4β, 7β, 7aα) -Hexahydro-4-methyl-2-(4-nitro-1-naphthalenyl)-7-[2-[4-(trifluoromethyl) phenoxy] ethyl]-4,7-epoxy-1H-isoindole-1,3(2H)-dione;
(3aα, 4β, 7β, 7aα) -Hexahydro-4-methyl-2-(4-nitro-1-naphthalenyl)-7-[2-(4-nitrophenoxy) ethyl]-4,7-epoxy-1H-isoindole-1,3(2H)-dione;
(3aα,4β,7β,7aα)-2-(1,6-Dihydro-1,4-dimethyl-6-oxo-3-pyridinyl)hexahydro-4,7-dimethyl-4,7-epoxy-1H-isoindole-1,3(2H)-dione;
(3aα, 4β, 7β, 7aα)-4-[Octahydro-7-methyl-2-(4-nitro-1-naphthalenyl)-1,3-dioxo-4,7-epoxy-4H-isoindol-4-yl]ethoxy]benzonitrile;
(3aα,4β,7β,7aα)-4-(Octahydro-4,7-dimethyl-1,3-dioxo-4,7-epoxy-2H-isoindol-2-yl)-1,2-benzenedicarbonitrile;
(3aα,4β,7β,7aα)-4-(2-Bromoethyl)hexahydro-7-methyl-2-(4-nitro-1-naphthalenyl)-4,7-epoxy-1H-isoindole-1,3(2H)-dione;
(3aα,4β,7β,7aα)-4-[4-[2-(4-Cyanophenoxy)ethyl]octahydro-7-methyl-1,3-dioxo-4,7-epoxy-2H-isoindol-2-yl]-1-naphthalenecarbonitrile;

(3aα, 4β, 7β, 7aα)- 4-[Octahydro- 4-[2-(4-methoxyphenoxy) ethyl]- 7-methyl- 1,3-dioxo- 4,7-epoxy- 2H-isoindol-2-yl]-1-naphthalenecarbouitrile;
(3aα, 4β, 7β, 7aα)- 4-[Octahydro- 4-[2-(3-methoxyphenoxy) ethyl]- 7-methyl- 1,3-diozo- 4,7-epoxy- 2H-isoindol-2-yl]-1-naphthalenecarbonitrile;
(3aα, 4β, 7β, 7aα)- 4-[4-[2-(3-Fluorophenoxy) ethyl] octahydro- 7-methyl- 1,3-dioxo- 4,7-epoxy- 2H-isoindol- 2-yl]-1-naphthalenecarbonitrile;
(3aα,4β,7β,7aα)-4-[Octahydro-4-methyl-7-[2-[3-(4-morpholinyl)phenoxy]ethyl]-1,3-dioxo-4,7-epoxy-2H-isoindol-2-yl]-1-naphthalenecarbonitrile;
(3aα, 4β, 7β, 7aα)- 4-[Octahydro- 4-methyl- 7-[2-[4-nitro- 3-(trifluoromethyl) phenoxy] ethyl]- 1,3-dioxo- 4,7-epoxy-2H-isoindol-2-yl]-1-naphthalenecarbonitrile;
(3aα, 4β, 7β, 7aα)- 4-[4-[2-(3-Cyanophenoxy) ethyl] octahydro- 7-methyl- 1,3-dioxo- 4,7-epoxy- 2H-isoindol- 2-yl]-1-naphthalenecarbonitrile;
(3aα,4β,7β,7aα)-2,-(2,3-Dihydro-3-methyl-2-oxo-6-benzothiazolyl)hexahydro-4,7-dimethyl-4,7-epoxy-1H-isoindole-1,3(2H)-dione;
(3aα,4β,7β,7aα)-2-(2,3-Dihydro-2-oxo-6-benzothiazolyl) hexahydro-4,7-dimethyl-4,7-epoxy-1H-isoindole-1,3(2H)-dione;
(3aα,4β,7β,7aα)-4-[4-[2-[3-(Dimethylamino)phenoxy]ethyl]octahydro-7-methyl-1,3-dioxo-4,7-epoxy-2H-isoindol-2-yl]-1-naphthalenecarbonitrile;
(3aα, 4β, 7β, 7aα)- 4-[2-[4-Cyano- 3-(trifluoromethyl) phenyl] octahydro- 7-methyl- 1,3-dioxo- 4,7-epoxy- 4H-isoindol-4-yl]ethoxy]-1,2-benzenedicarbonitrile;
(3aα,4β,7β,7aα)-N-[2-Cyano-5-(octahydro-4,7-dimethyl-1,3-dioxo-4,7-epoxy-2H-isoindol-2-yl)phenyl] acetamide;
(3aα, 4β, 7β, 7aα)- 4-(Octahydro- 4,7-dimethyl- 1,3-dioxo- 4,7-epoxy- 2H-isoindol- 2-yl)- 2-(trifluoromethoxy) benzonitrile;
(3aα,4β,7β,7aα)-2-Methoxy-4-(octahydro-4,7-dimethyl-1,3-dioxo-4,7-epoxy-2H-isoindol-2-yl)benzonitrile;
(3aα,4β,7β,7aα)-2-[4-(4,5-Dichloro-1H-imidazol-1-yl)phenyl]hexahydro-4,7-dimethyl-4,7-epoxy-1H-isoindole-1,3(2H)-dione;
(3aα,4β,7β,7aα)-2-[4-(4-Bromo-1-methyl-1H-pyrazol-3-yl)phenyl]hexahydro-4,7-dimethyl-4,7-epoxy-1H-isoindole-1,3(2H)-dione;
(3aα,4β,7β,7aα)-4-[Octahydro-4-(2-hydroxyethyl)-7-methyl-1,3-dioxo-4,7-epoxy-2H-isoindol-2-yl]-1-naphthalenecarbonitrile;
(3aα,4β,7β,7aα)-2-Iodo-4-(Octahydro-4,7-dimethyl-1,3-dioxo-4,7-epoxy-2H-isoindol-2-yl)benzonitrile;
(3aα, 4β, 7β, 7aα)- 4-[4-[2-(4-Fluorophenoxy) ethyl] octahydro- 7-methyl- 1,3-dioxo- 4,7-epoxy- 2H-isoindol- 2-yl]-1-naphthalenecarbonitrile;
(3aα,4β,7β,7aα)-4-[Octahydro-4-methyl-1,3-dioxo-7-[2-[4-(trifluoromethyl)phenoxy]ethyl]-4,7-epoxy-2H-isoindol-2-yl]-1-naphthalenecarbonitrile;
(3aα, 4β, 7β, 7aα)- 4-[4-[2-(4-Cyano- 3-fluorophenoxy) ethyl] octahydro- 7-methyl- 1,3-dioxo- 4,7-epoxy- 2H-isoindol-2-yl]-1-naphthalenecarbonitrile;
(3aα,4β,7β,7aα)-4-[Octahydro-4-methyl-1,3-dioxo-7-[2-[2,3,5,6-tetrafluoro-4-(trifluoromethyl)phenoxy]ethyl]-4,7-epoxy-2H-isoindol-2-yl]-1-naphthalenecarbonitrile;
(3aα,4β,7β,7aα)-Hexahydro-4,7-dimethyl-2-[4-(1H-1,2,4-triazol-3-yl)phenyl]-4,7-epoxy-1H-isoindole-1,3(2H)-dione;
(3aα, 4β, 7β, 7aα)- 2-[4-(4,5-Dihydro- 5-oxo- 1,2,4-oxadiazol- 3-yl) phenyl] hexahydro- 4,7-dimethyl- 4,7-epoxy-1H-isoindole-1,3(2H)-dione;
(3aα, 4β, 7β, 7aα) -Hexahydro- 2-[3-methoxy- 4-(2-oxazolyl) phenyl]- 4,7-dimethyl- 4,7-epoxy- 1H-isoindole- 1,3(2H)-dione;
(3aα,4β,7β,7aα)-Hexahydro-2-(4-hydroxy-1-naphthalenyl)-4,7-dimethyl-4,7-epoxy-1H-isoindole-1,3(2H)-dione;
(3aα,4β,7β,7aα)-Hexahydro-2-(8-hydroxy-5-quinolinyl)-4,7-dimethyl-4,7-epoxy-1H-isoindole-1,3(2H)-dione, trifluoroacetate;
(3aα,4β,7β,7aα)-4-[Octahydro-4-methyl-1,3-dioxo-7-[2-[methyl(phenylmethyl)amino]ethyl]-4,7-epoxy-2H-isoindol-2-yl]-2-(trifluoromethyl)benzonitrile;
(3aα,4β,7β,7aα)-Hexahydro-4,7-dimethyl-2-(5-quinolinyl)-4,7-epoxy-1H-isoindole-1,3(2H)-dione;
(3aα,4β,7β,7aα)-5-(Octahydro-4,7-dimethyl-1,3-dioxo-4,7-epoxy-2H-isoindol-2-yl)-2-pyridinecarbonitrile;
(3aα,4β,7β,7aα)-5-(Octahydro-4,7-dimethyl-1,3-dioxo-4,7-epoxy-2H-isoindol-2-yl)-8-quinolinecarbonitrile;
(3aα,4β,7β,7aα)-2-(5-Bromo-4-nitro-1-naphthalenyl)hexahydro-4,7-dimethyl-4,7-epoxy-1H-isoindole-1,3(2H)-dione;
(3aα,4β,7β,7aα)-2-(5-Bromo-1-naphthalenyl)hexahydro-4,7-dimethyl-4,7-epoxy-1H-isoindole-1,3(2H)-dione;

(3aα,4β,7β,7aα)-Hexahydro-4,7-dimethyl-2-[8-(trifluoromethyl)-4-quinolinyl]-4,7-epoxy-1H-isoindole-1,3(2H)-dione;
4-Fluorobenzoic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester;
Benzeneacetic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester;
4-Fluorobenzeneacetic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester;
(3aα, 4β, 7β, 7aα) -Hexahydro- 4-methyl- 7-[2-[4-(methylsulfonyl) phenoxy] ethyl]- 2-(4-nitro- 1-naphthalenyl)-4,7-epoxy-1H-isoindole-1,3(2H)-dione;
(3aα,4β,7β,7aα)-Hexahydro-2-(2-naphthalenyl)-4,7-dimethyl-4,7-epoxy-1H-isoindole-1,3(2H)-dione;
(3aα,4β,7β,7aα)-2-(4-Chloro-1-naphthalenyl)hexahydro-4,7-dimethyl-4,7-epoxy-1H-isoindole-1,3(2H)-dione;
(3aα, 4β, 7β, 7aα) -N-[(4-Chlorophenyl) methyl]- 2-(4-cyano- 1-naphthalenyl) octahydro- 7-methyl- 1,3-dioxo-4,7-epoxy-4H-isoindole-4-acetamide;
4,7,7-Trimethyl-3-oxo-2-oxabicyclo[2.2.1]heptane-1-carboxylic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester;
(αS)- α-Methoxy-α-(trifluoromethyl)benzeneacetic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester;
(αR)- α-Methoxy-α-(trifluoromethyl)benzeneacetic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester;
(3aα,4β,7β,7aα)-4-[Octahydro-4-methyl-7-[2-[(7-methyl-1,2-benzisoxazol-3-yl)oxy]ethyl]-1,3-dioxo-4,7-epoxy-2H-isoindol-2-yl]-1-naphthalenecarbonitrile;
(3aα,4β,7β,7aα)-4-[4-[2-(1,2-Benzisoxazol-3-yloxy)ethyl]octahydro-7-methyl-1,3-dioxo-4,7-epoxy-2H-isoindol-2-yl]-1-naphthalenecarbonitrile;
(3aα,4β,7β,7aα)-4-[2-(Benzoyloxy)ethyl]-2-(4-cyano-1-naphthalenyl)hexahydro-7-methyl-4,7-epoxy-1H-isoindole-1,3(2H)-dione;
(3aα,4β,7β,7aα)-2-(4-Cyano-1-naphthalenyl)-4-[2-[(4-nitrobenzoyl)oxy]ethyl]hexahydro-7-methyl-4,7-epoxy-1H-isoindole-1,3(2H)-dione;
4-Chlorobenzoic acid, 2-[(3aα,4β,7β,7aα)-2-(4-cyano-1-naphthalenyl)octahydro-7-methyl-1,3-dioxo-4,7-epoxy-4H-isoindol-4-yl]ethyl ester;
[3aα,4β,7β,7aα(E)]-4-[Octahydro-4-methyl-7-[3-(1-naphthalenyl)-2-propenyl]-1,3-dioxo-4,7-epoxy-2H-isoindol-2-yl]-1-naphthalenecarbonitrile;
(3aα,4β,7β,7aα)-4-[Octahydro-4-methyl-7-[3-(1-naphthalenyl)propyl]-1,3-dioxo-4,7-epoxy-2H-isoindol-2-yl]-1-naphthalenecarbonitrile;
(3aα,4β,7β,7aα)-Hexahydro-4,7-dimethyl-2-(2-methyl-6-quinolinyl)-4,7-epoxy-1H-isoindole-1,3(2H)-dione;
(3aα,4β,7β,7aα)-Hexahydro-2-(5-isoquinolinyl)-4,7-dimethyl-4,7-epoxy-1H-isoindole-1,3(2H)-dione;
(3aα,4β,7β,7aα)-2-(6-Benzothiazolyl)hexahydro-4,7-dimethyl-4,7-epoxy-1H-isoindole-1,3(2H)-dione;
[3aα,4β,7β,7aα(E)]-4-[Octahydro-4-methyl-1,3-dioxo-7-(4-oxo-4-phenyl-2-butenyl)-4,7-epoxy-2H-isoindol-2-yl]-1-naphthalenecarbonitrile;
(3aα,4β,7β,7aα)-2-(4-Cyano-1-naphthalenyl)octahydro-N-(2-hydroxyphenyl)-7-methyl-1,3-dioxo-4,7-epoxy-4H-isoindole-4-acetamide;
[3aα, 4β (E), 7β, 7aα]- 4-[Octahydro- 4-methyl- 7-[3-(6-methyl- 2-pyridinyl)- 2-propenyl]- 1,3-dioxo- 4,7-epoxy-2H-isoindol-2-yl]-1-naphthalenecarbonitrile;
(3aα,4β,7β,7aα)-4-[Octahydro-4-methyl-7-[3-(6-methyl-2-pyridinyl)propyl]-1,3-dioxo-4,7-epoxy-2H-isoindol-2-yl]-1-naphthalenecarbonitrile;
[3aR-(3aα,4β,7β,7aα)]-4-[Octahydro-4-[2-(3-methoxyphenoxy)ethyl]-7-methyl-1,3-dioxo-4,7-epoxy-2H-isoindol-2-yl]-1-naphthalenecarbonitrile;
[3aS-(3aα,4β,7β,7aα)]-4-[Octahydro-4-[2-(3-methoxyphenoxy)ethyl]-7-methyl-1,3-dioxo-4,7-epoxy-2H-isoindol-2-yl]-1-naphthalenecarbonitrile;
[3aR-(3aα,4β,7β,7aα)]-4-[4-[2-(4-Cyanophenoxy)ethyl]octahydro-7-methyl-1,3-dioxo-4,7-epoxy-2H-isoindol-2-yl]-1-naphthalenecarbonitrile;
[3aS-(3aα,4β,7β,7aα)]-4-[4-[2-(4-Cyanophenoxy)ethyl]octahydro-7-methyl-1,3-dioxo-4,7-epoxy-2H-isoindol-2-yl]-1-naphthalenecarbonitrile;
(3aα, 4α, 7α, 7aα)- 4-[4-[(4-Fluorophenyl) methyl] octahydro- 7-methyl- 1,3-dioxo- 4,7-epoxy- 2H-isoindol- 2-yl]-2-(trifluoromethyl)benzonitrile;
(3aα,4α,7α,7aα)-Hexahydro-4,7-dimethyl-2-(1-methyl-6-oxo-3-piperidinyl)-4,7-epoxy-1H-isoindole-1,3(2H)-dione;
(3aα,4α,7α,7aα)-2-(1,6-Dihydro-1,4-dimethyl-6-oxo-3-pyridinyl)hexahydro-4,7-dimethyl-4,7-epoxy-1H-isoindole-1,3(2H)-dione;
(3aα, 4β, 7β, 7aα)- 2-[4-Cyano- 3-(trifluoromethyl) phenyl] octahydro- 1,3-dioxo- 7-[2-(phenylmethoxy) ethyl]-4,7-epoxy-4H-isoindole-4-propanemtrile;
(3aα, 4β, 7β, 7aα)- 2-[4-Cyano- 3-(trifluoromethyl) phenyl] octahydro- 1,3-dioxo- 7-[2-(phenylmethoxy) ethyl]-4,7-epoxy-4H-isoindole-4-propanenitrile;
(3aα,4β,7β,7aα)-4-[7-[2-(4-Cyanophenoxy)ethyl]octahydro-5-hydroxy-4-methyl-1,3-dioxo-4,7-epoxy-2H-isoindol-2-yl]-1-naphthalenecarbonitrile;
[3aS-(3aα, 4β, 7β, 7aα)]- 4-[7-[2-(1,3-Benzodioxol- 5-yloxy) ethyl] octahydro- 5-hydroxy- 4-methyl- 1,3-dioxo-4,7-epoxy-2H-isoindol-2-yl]-1-naphthalenecarbonitrile;
[3aR-(3aα, 4β, 7β, 7aα)]- 4-[7-[2-(1,3-Benzodioxol- 5-yloxy) ethyl] octahydro- 5-hydroxy- 4-methyl- 1,3-dioxo-4,7-epoxy-2H-isoindol-2-yl]-1-naphthalenecarbonitrile;
[3aS-(3aα, 4β, 7β, 7aα)]- 4-[7-[2-[(5-Chloro- 2-pyridinyl) oxy] ethyl] octahydro- 5-hydroxy- 4-methyl- 1,3-dioxo-4,7-epoxy-2H-isoindol-2-yl]-1-naphthalenecarbonitrile;
[3aR-(3aα, 4β, 7β, 7aα)]- 4-[7-[2-[(5-Chloro- 2-pyridinyl) oxy] ethyl] octahydro- 5-hydroxy- 4-methyl- 1,3-dioxo-4,7-epoxy-2H-isoindol-2-yl]-1-naphthalenecarbonitrile;
[3aS-(3aα, 4β, 7β, 7aα)]- 4-[7-[2-(4-Chlorophenoxy) ethyl] octahydro- 5-hydroxy- 4-methyl- 1,3-dioxo- 4,7-epoxy-2H-isoindol-2-yl]-1-naphthalenecarbonitrile;
[3aR-(3aα, 4β, 7β, 7aα)]- 4-[7-[2-(4-Chlorophenoxy) ethyl] octahydro- 5-hydroxy- 4-methyl- 1,3-dioxo- 4,7-epoxy-2H-isoindol-2-yl]-1-naphthalenecarbonitrile;
[3aS-(3aα, 4β, 7β, 7aα)]- 4-[7-[2-(4-Acetylphenoxy) ethyl] octahydro- 5-hydroxy- 4-methyl- 1,3-dioxo- 4,7-epoxy-2H-isoindol-2-yl]-1-naphthalenecarbonitrile;
[3aR-(3aα, 4β, 7β, 7aα)]- 4-[7-[2-(4-Acetylphenoxy) ethyl] octahydro- 5-hydroxy- 4-methyl- 1,3-dioxo- 4,7-epoxy-2H-isoindol-2-yl]-1-naphthalenecarbonitrile;
[3aS-(3aα, 4β, 7β, 7aα)]- 4-[7-[2-(3-Cyanophenoxy) ethyl] octahydro- 5-hydroxy- 4-methyl- 1,3-dioxo- 4,7-epoxy-2H-isoindol-2-yl]-1-naphthalenecarbonitrile;
[3aR-(3aα, 4β, 7β, 7aα)]- 4-[7-[2-(3-Cyanophenoxy) ethyl] octahydro- 5-hydroxy- 4-methyl- 1,3-dioxo- 4,7-epoxy-2H-isoindol-2-yl]-1-naphthalenecarbonitrile;
[3aS-(3aα, 4β, 7β, 7aα)]- 4-[Octahydro- 5-hydroxy- 4-methyl- 1,3-dioxo- 7-[2-[(5,6,7,8-tetrahydro- 1-naphthalenyl) oxy]ethyl]-4,7-epoxy-2H-isoindol-2-yl]-1-naphthalenecarbonitrile;
[3aR-(3aα, 4β, 7β, 7aα)]- 4-[Octahydro- 5-hydroxy- 4-methyl- 1,3-dioxo- 7-[2-[(5,6,7,8-tetrahydro- 1-naphthalenyl) oxy]ethyl]-4,7-epoxy-2H-isoindol-2-yl]-1-naphthalenecarbonitrile;
[3aS-(3aα, 4β, 7β, 7aα)]- 4-[Octahydro- 5-hydroxy- 4-methyl- 1,3-dioxo- 7-[2-[(5,6,7,8-tetrahydro- 5-oxo- 1-naphthalenyl)oxy]ethyl]-4,7-epoxy-2H-isoindol-2-yl]-1-naphthalenecarbonitrile;
[3aR-(3aα, 4β, 7β, 7aα)]- 4-[Octahydro- 5-hydroxy- 4-methyl- 1,3-dioxo- 7-[2-[(5,6,7,8-tetrahydro- 5-oxo- 1-naphthalenyl)oxy]ethyl]-4,7-epoxy-2H-isoindol-2-yl]-1-naphthalenecarbonitrile;
[3aS-(3aα, 4β, 7β, 7aα)]- 4-[7-[2-(4-Fluorophenoxy) ethyl] octahydro- 5-hydroxy- 4-methyl- 1,3-dioxo- 4,7-epoxy-2H-isoindol-2-yl]-1-naphthalenecarbonitrile;
[3aR-(3aα, 4β, 7β, 7aα)]- 4-[7-[2-(4-Fluorophenoxy) ethyl] octahydro- 5-hydroxy- 4-methyl- 1,3-dioxo- 4,7-epoxy-2H-isoindol-2-yl]-1-naphthalenecarbonitrile;
[3aS-(3aα, 4β, 7β, 7aα)]- 4-[Octahydro- 5-hydroxy- 4-methyl- 7-[2-[(4-methyl- 2-oxo- 2H- 1-benzopyran- 7-yl) oxy] ethyl]-1,3-dioxo-4,7-epoxy-2H-isoindol-2-yl]-1-naphthalenecarbonitrile;
[3aR-(3aα, 4β, 7β, 7aα)]- 4-[Octahydro- 5-hydroxy- 4-methyl- 7-[2-[(4-methyl- 2-oxo- 2H- 1-benzopyran- 7-yl) oxy] ethyl]-1,3-dioxo-4,7-epoxy-2H-isoindol-2-yl]-1-naphthalenecarbonitrile;
[3aS-(3aα, 4β, 7β, 7aα)]- 4-[7-[2-(3,5-Dimethoxyphenogy) ethyl] octahydro- 5-hydroxy- 4-methyl- 1,3-dioxo-4,7-epoxy-2H-isoindol-2-yl]-1-naphthalenecarbonitrile;
[3aR-(3aα, 4β, 7β, 7aα)]- 4-[7-[2-(3,5-Dimethogyphenoxy) ethyl] octahydro- 5-hydroxy- 4-methyl- 1,3-dioxo-4,7-epoxy-2H-isoindol-2-yl]-1-naphthalenecarbonitrile;
[3aR-(3aα, 4β, 7β, 7aα)]- 4-[7-[2-(4-Chloro- 3-methylphenoxy) ethyl] octahydro- 5-hydroxy- 4-methyl- 1,3-dioxo-4,7-epoxy-2H-isoindol-2-yl]-1-naphthalenecarbonitrile;
[3aR-(3aα,4β,7β,7aα)]-4-[7-[2-(4-Cyano-2,3-difluorophenoxy)ethyl]octahydro-5-hydroxy-4-methyl-1,3-dioxo-4,7-epoxy-2H-isoindol-2-yl]-1-naphthalenecarbonitrile;
[3aS-(3aα, 4β, 7β, 7aα)]- 4-[7-[2-[(5-Chloro- 1,2-benzisoxazol- 3-yl) oxy] ethyl] octahydro- 5-hydroxy- 4-methyl-1,3-dioxo-4,7-epoxy-2H-isoindol-2-yl]-1-naphthalenecarbonitrile;
[3aR-(3aα, 4β, 7β, 7aα)]- 4-[7-[2-[(5-Chloro- 1,2-benzisoxazol- 3-yl) oxy] ethyl] octahydro- 5-hydroxy- 4-methyl-1,3-dioxo-4,7-epoxy-2H-isoindol-2-yl]-1-naphthalenecarbonitrile;
[3aR-(3aα, 4β, 7β, 7aα)]-3-[2-[2-(4-Cyano- 1-naphthalenyl) octahydro-6-hydroxy-7-methyl- 1,3-dioxo-4,7-epoxy-4H-isoindol-4-yl]ethoxy]-5-isoxazolecarboxylic acid, methyl ester;
[3aR-(3aα, 4β, 7β, 7aα)]- 4-[Octahydro- 5-hydroxy- 4-methyl- 1,3-dioxo- 7-[2-[4-(1H- 1,2,4-triazol- 1-yl) phenoxy]ethyl]-4,7-epoxy-2H-isoindol-2-yl]-1-naphthalenecarbonitrile;
[3aS-(3aα, 4β, 7β, 7aα)]- 4-[7-[2-[(7-Chloro- 4-quinolinyl) oxy] ethyl] octahydro- 5-hydroxy- 4-methyl- 1,3-dioxo-4,7-epoxy-2H-isoindol-2-yl]-1-naphthalenecarbonitrile, trifluoroacetate;
[3aR-(3aα,4β,7β,7aα)]-4-[7-[2-[(7-Chloro-4-quinolinyl)oxy]ethyl] octahydro-5-hydroxy-4-methyl-1,3-dioxo-4,7-epoxy-2H-isoindol-2-yl]-1-naphthalenecarbonitrile, trifluoroacetate;
(1aα,2β,2aα,5aα,6βb,6aα)-4-[2-[2-[[(1,1-Dimethylethyl)-dimethylsilyl]oxy]ethyl]octahydro- 6-methyl-3,5-dioxo-2,6-epoxy-4H-oxireno[f]isoindol-4-yl]-1-naphthalenecarbonitrile;
[3aR-(3aα,4β,7β,7aα)]-4-[4-Ethyloctahydro-7-(2-hydroxyethyl)-1,3-dioxo-4,7-epoxy-2H-isoindol-2-yl]-1-naphthalenecarbonitrile;
[3aS-(3aα,4β,7β,7aα)]-4-[4-Ethyloctahydro-7-(2-hydroxyethyl)-1,3-dioxo-4,7-epoxy-2H-isoindol-2-yl]-1-naphthalenecarbonitrile;
[3aR-(3aα, 4β, 7β, 7aα)]- 4-[4-[2-(4-Cyanophenoxy) ethyl]- 7-ethyloctahydro- 1,3-dioxo- 4,7-epoxy- 2H-isoindol-2-yl]-1-naphthalenecarbonitrile; and
[3aS-(3aα, 4β, 7β, 7aα)]- 4-[4-[2-(4-Cyanophenoxy) ethyl]- 7-ethyloctahydro- 1,3-dioxo- 4,7-epoxy- 2H-isoindol-2-yl]-1-naphthalenecarbonitrile.

**4.** A compound according to claim 3 being:

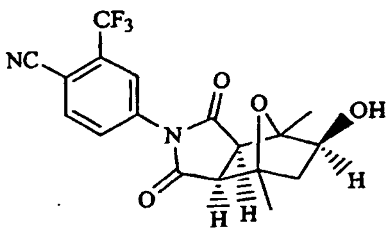

or

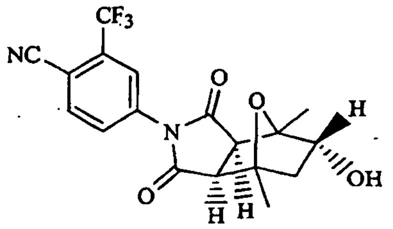

,

or a salt or hydrate thereof.

**5.** A pharmaceutical composition capable of treating a nuclear hormone receptor-associated condition, comprising a compound of the formula I as defined in claim 1 with proviso (10) of claim 1 or a pharmaceutically acceptable salt or hydrate thereof, and a pharmaceutically acceptable carrier.

**6.** A pharmaceutical composition comprising a compound according to claim 1, 2, 3 or 4.

**7.** A pharmaceutical composition containing a compound according to claim 4.

**8.** A pharmaceutical composition of claim 5, 6 or 7 further comprising another anti-cancer agent.

**9.** Use of a compound as defined in claim 1, 2, 3 or 4 for the preparation of a medicament for modulating the function of a nuclear hormone receptor.

**10.** The use according to claim 9 wherein said nuclear hormone receptor is selected from the group consisting of a steroid binding nuclear hormone receptor as defined in claim 1, 2, 3 or 4, the androgen receptor, the estrogen receptor, the progesterone receptor, the flucocorticoid receptor, the mineralocorticoid receptor, and the aldosterone receptor.

**11.** Use of a compound as defined in claim 1, 2, 3 or 4 for the preparation of a medicament for treating a condition or disorder
wherein said condition or disorder is selected from the group consisting of proliferate diseases, cancers, benign prostate hypertrophia, adenomas and neoplasies of the prostate, benign or malignant tumor cells containing the androgen receptor, heart disease, angiogenic conditions or disorders, hirsutism, acne, hyperpilosity, inflammation, immune modulation, seborrhea, endometriosis, polycystic ovary syndrome, androgenic alopecia, hypogonadism, osteoporosis, suppressing spermatogenisis, libido, cachexia, anorexia, inhibition of muscular atrophy in ambulatory patients, androgen supplementation for age related decreased testosterone levels in men, cancers expressing the estrogen receptor, prostate cancer, breast cancer, endometrial cancer, hot flushes, vaginal dryness, menopause, amenorrhea, dysmenorrhea, contraception, pregnancy termination, cancers containing the progesterone receptor,

endometriosis, cyclesynchrony, meningioma, fibroids, labor induction, ajutoimmune diseases, Alzheimer's disease, psychotic disorders, drug dependence, non-insulin dependent Diabetes Mellitus, dopamine receptor mediated disorders, congestive heart failure, disregulation of cholesterol homeostasis, and attenuating the metabolism of a pharmaceutical agent.

**12.** A method for preparation of a compoun of the following formula XVI, or salt thereof:

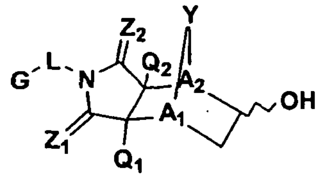

XVI

where G, L, $Z_1$, $Z_2$, $Q_1$, $Q_2$, $A_1$, $A_2$ and Y are as defined in claim 1,
comprising the steps of contacting a compound of the following formula XV, or salt thereof:

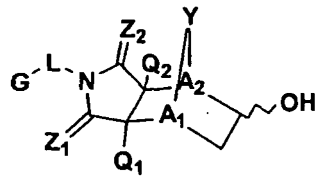

XVI

where the symbols are as defined above;
with an enzyme or microorganism capable of catalyzing the hydroxylation of said compound XV to said compound XVI, and effecting said hydroxylation.

**13.** A method for preparation of a compound of the following formula XVIII, or salt thereof:

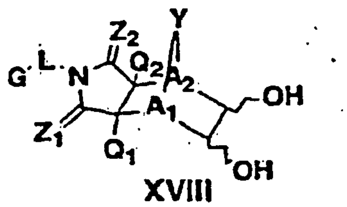

XVIII

where G, L, $Z_1$, $Z_2$, $Q_1$, $Q_2$, $A_1$, $A_2$ and Y are as defined in claim 3;
comprising the steps of contacting a compound of the following formula XVII, or salt thereof:

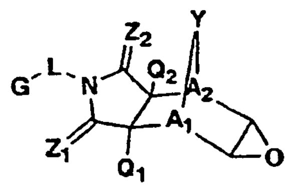

XVII

where the symbols are as defined above;
with an enzyme or microorganism capable of catalyzing the opening of the epoxide ring of compound XVII to form the diol of said compound XVIII, and effecting said ring opening and fiol formation.

**Patentansprüche**

1. Verbindung der folgenden Formel (I) oder ein Salz oder Hydrat davon:

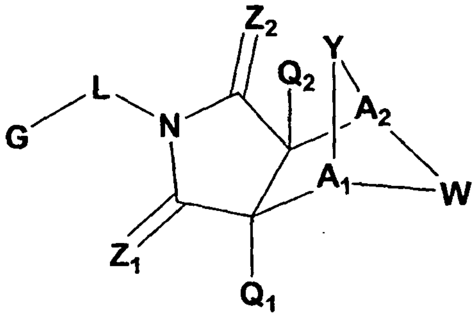

(Ia)

wobei die Symbole die folgenden Bedeutungen haben und bei jedem Vorkommen unabhängig voneinander ausgewählt sind:

G ist ein Aryl- oder Heterocyclorest, wobei der Rest mono- oder polycyclisch ist und gegebenenfalls an einer oder mehreren Stellen substituiert ist;
L ist eine Bindung, $(CR^7R^{7'})_n$ (wobei n 1 ist und $R^7$ und $R^{7'}$ jeweils unabhängig voneinander H, Alkyl oder substituiertes Alkyl sind) oder $-CH_2$-NH-;
$A_1$ und $A_2$ sind jeweils unabhängig voneinander $CR^7$, wobei $R^7$ (i) Wasserstoff, Alkyl oder substituiertes Alkyl, Arylalkyl oder substituiertes Arylalkyl, Alkenyl oder substituiertes Alkenyl, Aryl oder substituiertes Aryl, Heterocyclo oder substituiertes Heterocyclo, Heterocycloalkyl oder substituiertes Heterocycloalkyl ist, wobei Substituenten jeweils ein oder mehrere aus $V^1$ ausgewählte Reste sind, oder (ii) zusammen mit $R^7$ eines Restes W einen heterocyclischen Ring bildet;
$V^1$ ist OH, CN, Halogen, -O-Aryl, -O-substituiertes Aryl, -O-Heterocyclo, -O-substituiertes Heterocyclo, -O-CO-Alkyl, -O-CO-substituiertes Alkyl, -O-(Alkylsilyl), -O-Arylalkyl, -O-substituiertes Arylalkyl, -O-CO-Arylalkyl, -O-CO-substituiertes Arylalkyl, -O-CO-Aryl, -O-CO-substituiertes Aryl, -O-CO-Heterocyclo, -O-CO-substituiertes Heterocyclo, -S-(gegebenenfalls substituiertes Aryl)-NH-CO-(gegebenenfalls substituiertes Alkyl), -SO-(gegebenenfalls substituiertes Aryl)-NH-CO-(gegebenenfalls substituiertes Alkyl), $-SO_2$-(gegebenenfalls substituiertes Aryl)-NH-CO-(gegebenenfalls substituiertes Alkyl), $-NH-SO_2$-Aryl, $-NH-SO_2$-substituiertes Aryl, -NH-CO-O-(gegebenenfalls substituiertes Arylalkyl), -NH-CO-O-Alkyl, -NH-CO-O-substituiertes Alkyl, -NH-CO-Alkyl, -NH-CO-substituiertes Alkyl, -NH-CO-Aryl, -NH-CO-substituiertes Aryl, -NH-CO-(gegebenenfalls substituiertes Arylalkyl), -NH-CO-(gegebenenfalls substituiertes Alkyl)-O-(gegebenenfalls substituiertes Aryl), -N(gegebenenfalls substituiertes Alkyl)(gegebenenfalls substituiertes Aryl), -N(gegebenenfalls substituiertes Alkyl)(gegebenenfalls substituiertes Arylalkyl), -COH, -COOH, -CO-O-Alkyl, -CO-O-substituiertes Alkyl, -CO-O-gegebenenfalls substituiertes Arylalkyl, -CO-Aryl, -CO-substituiertes Aryl, -O-CO-NH-Aryl, -O-CO-NH-substituiertes Aryl, -CO-NH-Aryl, -CO-NH-substituiertes Aryl, -CO-NH-Arylalkyl, -CO-NH-substituiertes Arylalkyl oder -O-(gegebenenfalls substituiertes Aryl)-NH-CO-(gegebenenfalls substituiertes Alkyl);
Y ist -O-, -SO-, $-N(V^2)-$, $-CH_2-N(V^2)-$, -CO-N(Alkyl)-, $-CH_2$-S- oder $-CH_2-SO_2-$;
$V^2$ ist Wasserstoff, Alkyl, Arylalkyl, -CO-Alkyl, -CO-O-Aryl oder -CO-O-Arylalkyl;
$Z_1$ und $Z_2$ sind O;
$Q_1$ und $Q_2$ sind H;
W ist $CR^7R^{7'}-CR^7R^{7'}$, $CR^7R^{7'}$-C=O, $NR^9-CR^7R^{7'}$, $N=CR^8$, N=N, $NR^9-NR^{9'}$, Cycloalkyl oder substituiertes Cycloalkyl, Cycloalkenyl oder substituiertes Cycloalkenyl, Heterocyclo oder substituiertes Heterocyclo oder Aryl oder substituiertes Aryl, wobei, wenn W nicht $NR^9-CR^7R^{7'}$, $N=CR^8$, N=N, $NR^9-NR^{9'}$ oder Heterocyclo oder substituiertes Heterocyclo ist, dann muss Y gleich -O-, $-CH_2$-S-, -SO-, $-CH_2-SO_2-$, $N(V^2)$- oder $-CH_2-N(V^2)$ sein;
$R^1$ und $R^{1'}$ sind jeweils unabhängig voneinander H, Alkyl oder substituiertes Alkyl, Cycloalkyl oder substituiertes

Cycloalkyl, Cycloalkenyl oder substituiertes Cycloalkenyl, Heterocyclo oder substituiertes Heterocyclo, Cycloalkylalkyl oder substituiertes Cycloalkyalkyl, Cycloalkenylalkyl oder substituiertes Cycloalkenylalkyl, Heterocycloalkyl oder substituiertes Heterocycloalkyl, Aryl oder substituiertes Aryl, Arylalkyl oder substituiertes Arylalkyl;

$R^2$ ist Alkyl oder substituiertes Alkyl, Cycloalkyl oder substituiertes Cycloalkyl, Cycloalkenyl oder substituiertes Cycloalkenyl, Heterocyclo oder substituiertes Heterocyclo, Cycloalkylalkyl oder substituiertes Cycloalkylalkyl, Cycloalkenylalkyl oder substituiertes Cycloalkenylalkyl, Heterocycloalkyl oder substituiertes Heterocycloalkyl, Aryl oder substituiertes Aryl, Arylalkyl oder substituiertes Arylalkyl;

$R^4$ ist H, Alkyl oder substituiertes Alkyl, Cycloalkyl oder substituiertes Cycloalkyl, Cycloalkenyl oder substituiertes Cycloalkenyl, Heterocyclo oder substituiertes Heterocyclo, Cycloalkylalkyl oder substituiertes Cycloalkylalkyl, Cycloalkenylalkyl oder substituiertes Cycloalkenylalkyl, Heterocycloalkyl oder substituiertes Heterocycloalkyl, Aryl oder substituiertes Aryl, Arylalkyl oder substituiertes Arylalkyl, $R^1C{=}O$, $R^1NHC{=}O$, $SO_2OR^1$ oder $SO_2NR^1R^{1'}$;

$R^5$ ist Alkyl oder substituiertes Alkyl, Cycloalkyl oder substituiertes Cycloalkyl, Cycloalkenyl oder substituiertes Cycloalkenyl, Heterocyclo oder substituiertes Heterocyclo, Cycloalkylalkyl oder substituiertes Cycloalkylalkyl, Cycloalkenylalkyl oder substituiertes Cycloalkenylalkyl, Heterocycloalkyl oder substituiertes Heterocycloalkyl, Aryl oder substituiertes Aryl, Arylalkyl oder substituiertes Arylalkyl, $R^1C{=}O$, $R^1NHC{=}O$, $SO_2R^1$, $SO_2OR^1$ oder $SO_2NR^1R^{1'}$;

$R^7$ und $R^{7'}$ sind jeweils unabhängig voneinander H, Alkyl oder substituiertes Alkyl, Alkenyl oder substituiertes Alkenyl, Cycloalkyl oder substituiertes Cycloalkyl, Cycloalkenyl oder substituiertes Cycloalkenyl, Heterocyclo oder substituiertes Heterocyclo, Cycloalkylalkyl oder substituiertes Cycloalkylalkyl, Cycloalkenylalkyl oder substituiertes Cycloalkenylalkyl, Heterocycloalkyl oder substituiertes Heterocycloalkyl, Aryl oder substituiertes Aryl, Arylalkyl oder substituiertes Arylalkyl, Halogen, CN, $OR^1$, Nitro, Hydroxylamin, Hydroxylamid, Amino, $NHR^4$, $NR^2R^5$, $NOR^1$, Thiol, Alkylthio oder substituiertes Alkylthio, $R^1C{=}O$, $R^1OC{=}O$, $R^1NHC{=}O$, $SO_2R^1$, $SOR^1$, $PO_3R^1R^{1'}$, $R^1R^{1'}NC{=}O$, $C{=}OSR^1$, $SO_2R^1$, $SO_2OR^1$ oder $SO_2NR^1R^{1'}$, oder, wenn $A_1$ oder $A_2$ einen Rest $R^7$ enthält und W einen Rest $R^7$ enthält, die Reste $R^7$ von $A_1$ oder $A_2$ und W zusammen einen heterocyclischen Ring bilden;

$R^8$ ist H, Alkyl oder substituiertes Alkyl, Alkenyl oder substituiertes Alkenyl, Cycloalkyl oder substituiertes Cycloalkyl, Cycloalkenyl oder substituiertes Cycloalkenyl, Heterocyclo oder substituiertes Heterocyclo, Cycloalkylalkyl oder substituiertes Cycloalkylalkyl, Cycloalkenylalkyl oder substituiertes Cycloalkenylalkyl, Heterocycloalkyl oder substituiertes Heterocycloalkyl, Aryl oder substituiertes Aryl, Arylalkyl oder substituiertes Arylalkyl, Nitro, Halogen, CN, $OR^1$, Amino, $NHR^4$, $NR^2R^5$, $NOR^1$, Alkylthio oder substituiertes Alkylthio, $C{=}OSR^1$, $R^1OC{=}O$, $R^1C{=}O$, $R^1NHC{=}O$, $R^1R^{1'}NC{=}O$, $SO_2OR^1$, $S{=}OR^1$, $SO_2R^1$, $PO_3R^1R^{1'}$ oder $SO_2NR^1R^{1'}$; und

$R^9$ und $R^{9'}$ sind jeweils unabhängig voneinander H, Alkyl oder substituiertes Alkyl, Alkenyl oder substituiertes Alkenyl, Cycloalkyl oder substituiertes Cycloalkyl, Cycloalkenyl oder substituiertes Cycloalkenyl, Heterocyclo oder substituiertes Heterocyclo, Cycloalkylalkyl oder substituiertes Cycloalkylalkyl, Cycloalkenylalkyl oder substituiertes Cycloalkenylalkyl, Heterocycloalkyl oder substituiertes Heterocycloalkyl, Aryl oder substituiertes Aryl, Arylalkyl oder substituiertes Arylalkyl, CN, OH, $OR^1$, $R^1C{=}O$, $R^1OC{=}O$, $R^1NHC{=}O$, $SO_2R^1$, $SO_2OR^1$ oder $SO_2NR^1R^{1'}$;

mit den Maßgaben, dass:

(1) wenn Y gleich -O- ist , $Q_1$ und $Q_2$ Wasserstoff sind, $Z_1$ und $Z_2$ gleich O sind, W gleich $-CH_2-CH_2-$ ist und einer von $A_1$ und $A_2$ gleich CH ist und der andere $CR^7$ ist, dann G-L nicht unsubstituiertes Phenyl ist;

(2) wenn Y gleich -O- ist, $Q_1$ und $Q_2$ Wasserstoff sind, $Z_1$ und $Z_2$ gleich O sind, W gleich $-CH_2-CH_2-$ ist und einer von $A_1$ und $A_2$ gleich CH ist und der andere $C-CH_3$ ist, dann G-L nicht mit Chlor und/oder Methyl substituiertes Phenyl ist;

(3) wenn Y gleich -O- ist, $Q_1$ und $Q_2$ Wasserstoff sind, $Z_1$ und $Z_2$ gleich O sind, W gleich $-CH_2-CH_2-$ ist und einer von $A_1$ und $A_2$ gleich CH ist und der andere C-Akyl ist, dann G-L nicht N-substituiertes Piperazinalkyl oder N-substituiertes Imidazolidinalkyl ist;

(4) wenn Y gleich -O- ist; $Q_1$ und $Q_2$ Wasserstoff sind, $Z_1$ und $Z_2$ gleich O sind, W gleich $-CH_2-CH_2-$ ist und $A_1$ und $A_2$ gleich $C-CH_3$ sind, dann G-L nicht Thiazol oder substituiertes Thiazol ist;

(5) wenn Y gleich $-CH_2-S-$, -SO-, $-CH_2-SO_2-$, $-N(V^2)-$ oder $-CH_2-N(V^2)-$ ist, W gleich $CR^7R^{7'}-CR^7R^{7'}$ ist und $Z_1$ und $Z_2$ gleich O sind, dann G-L nicht unsubstituiertes Phenyl ist;

(6) wenn Y gleich $NR^7$ ist, W unsubstituiertes oder substituiertes Phenyl ist und $Q_1$ und $Q_2$ Wasserstoff sind, dann diese Verbindungen der Formel I nicht beinhaltet sind;

(7) wenn Y gleich -O- ist, $Q_1$ und $Q_2$ Wasserstoff sind, $Z_1$ und $Z_2$ gleich O sind, W Ethylenoxid ist und $A_1$ und $A_2$ gleich CH sind, dann G-L nicht Methylphenyl oder Chlorphenyl ist;

(8) die Verbindung der Formel I nicht 6,10-Epithio-4H-thieno-[3',4':5,6]-cyclooct[1,2-f]isoindol-7,9(5H,8H)dion, 8-(3,5-Dichlorphenyl)-6,6a,9a,10,11,12-hexahydro-1,3,6,10-tetramethyl-2,2,13-trioxid, (6R,6aR,9aS,10S) ist;

(9) wenn Y gleich -O- ist, $Q_1$ und $Q_2$ Wasserstoff sind, $Z_1$ und $Z_2$ gleich O sind, W Cyclopentyl, Cyclohexyl, 3-Phenyl-2-isoxazolin oder $CR^7R^{7'}$-$CR^7R^{7'}$ ist, wobei $R^7$ und $R^{7'}$ jeweils unabhängig voneinander als H und 4-Butyrolacton definiert sind und $R^7$ und $R^{7'}$ nicht alle gleichzeitig H sind, und $A_1$ und $A_2$ gleich CH sind, dann G-L nicht ein unsubstituierter Naphthylring oder ein monosubstituierter Phenylring ist, wobei der Substituent Methoxy, Br, Cl, $NO_2$, Methyl, Ethyl, $CH_2$-Phenyl, S-Phenyl oder O-Phenyl ist;

(10) wenn Y gleich -O- ist und W gleich -$CH_2$-$CH_2$- ist, dann mindestens einer von $A_1$ oder $A_2$ nicht CH ist;

(11) wobei die Verbindung der Formel I nicht folgendes ist

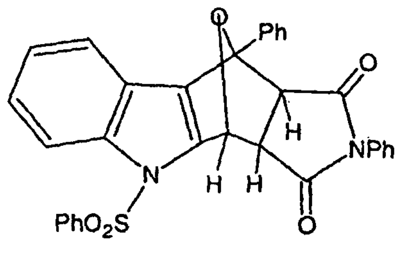

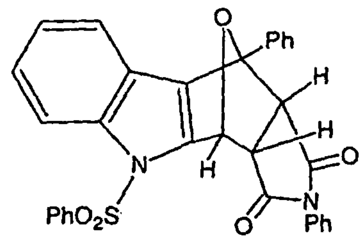

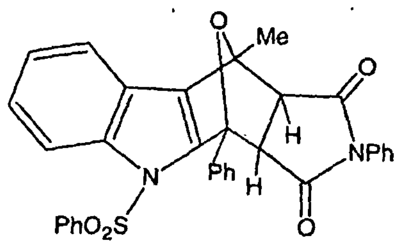

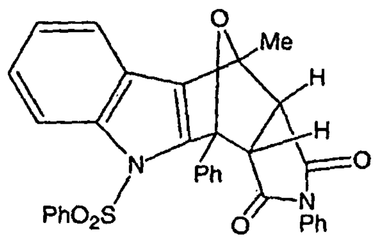

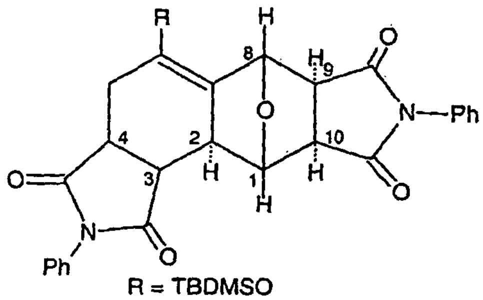

R = TBDMSO

R = Me

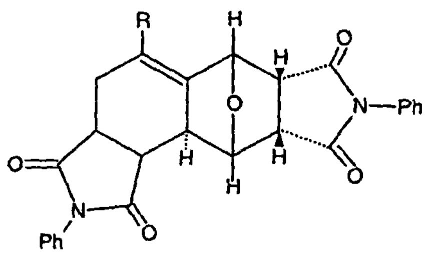

R = TBDMSO

R = Me

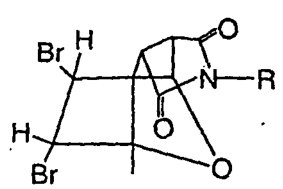

R = $C_6H_4$-$CH_3$ (2')

R = $C_6H_4$-$OCH_3$ (2')

R = 1-Naphthyl

wobei die Ausdrücke "Alkyl" und "Alk" einen geradkettigen oder verzweigten Alkanrest, der 1 bis 12 Kohlenstoffatome enthält, bezeichnen, "substituiertes Alkyl" einen Alkylrest, der an beliebigen zur Verfügung stehenden Bindungspunkten mit 1 bis 4 Substituenten substituiert ist, bedeutet, wobei es sich bei den Substituenten um einen oder mehrere aus den folgenden Gruppen handelt: Halogen, Alkoxy, Alkylthio, Hydroxy, Carboxy, Alkoxycarbonyl, Alkylcarbonyloxy, Amino, Carbamoyl oder substituiertes Carbamoyl, Carbamat oder substituiertes Carbamat, Harnstoff oder substituierter Harnstoff, Amidinyl oder substituiertes Amidinyl, Thiol, Aryl, Heterocyclus, Cycloalkyl, Heterocycloalkyl, -S-Aryl, -S-Heterocyclus, -S=O-Aryl, -S=O-Heterocyclus, Arylalkyl-O-, $-S(O)_2$-Aryl, $-S(O)_2$-Heterocyclus, $-NHS(O)_2$-Aryl, $-NHS(O)_2$-Heterocyclus, $-NHS(O)_2$NH-Aryl, $-NHS(O)_2$NH-Heterocyclus, $-P(O)_2$-Aryl, $-P(O)_2$-Heterocyclus, $-NHP(O)_2$-Aryl, $-NHP(O)_2$-Heterocyclus, $-NHP(O)_2$NH-Aryl, $-NHP(O)_2$NH-Heterocyclus, -O-Aryl, -O-Heterocyclus, -NH-Aryl, -NH-Heterocyclus, -NHC=O-Aryl, -NHC=O-Alkyl, -NHC=O-Heterocyclus, -OC=O-Aryl, -OC=O-Heterocyclus, -NHC=ONH-Aryl, -NHC=ONH-Heterocyclus, -OC=OO-Aryl, -OC=OO-Heterocyclus, -OC=ONH-Aryl, -OC=ONH-Heterocyclus, -NHC=OO-Aryl, -NHC=OO-Heterocyclus, -NHC=OO-Alkyl, -C=ONH-Aryl, -C=ONH-Heterocyclus, -C=00-Aryl, -C=OO-Heterocyclus, -N(Alkyl)$S(O)_2$-aryl, -N(Alkyl)$S(O)_2$-heterocyclus, -N(Alkyl)$S(O)_2$NH-aryl, -N(Alkyl)$S(O)_2$NH-heterocyclus, -N(Alkyl)$P(O)_2$-aryl, -N(Alkyl)$P(O)_2$-heterocyclus, -N(Alkyl)$P(O)_2$NH-aryl, -N(Alkyl)$P(O)_2$NH-heterocyclus, -N(Alkyl)-aryl, -N(Alkyl)-heterocyclus, -N(Alkyl)C=O-aryl, -N(Alkyl)C=O-heterocyclus, -N(Alkyl)C=ONH-aryl, -N(Alkyl)C=ONH-heterocyclus, -OC=ON(Alkyl)-aryl, -OC=ON(Alkyl)-heterocyclus, -N(Alkyl)C=OO-aryl, -N(Alkyl)C=OO-heterocyclus, -C=ON(Alkyl)-aryl, -C=ON(Alkyl)-heterocyclus, $-NHS(O)_2$N(Alkyl)-aryl, $-NHS(O)_2$N(Alkyl)-heterocyclus, $-NHP(O)_2$N(Alkyl)-aryl, $NHP(O)_2$N(Alkyl)-heterocyclus, -NHC=ON(Alkyl)-aryl, -NHC=ON(Alkyl)-heterocyclus, -N(Alkyl)$S(O)_2$N(alkyl)-aryl, -N(Alkyl)$S(O)_2$N(alkyl)-heterocyclus, -N(Alkyl)$P(O)_2$N(alkyl)-aryl, -N(Alkyl)$P(O)_2$N(alkyl)-heterocyclus, -N(Alkyl)C=ON(alkyl)-aryl und -N(Alkyl)C=ON(alkyl)-heterocyclus, wobei "Alkyl"-, "Aryl"- und "Heterocyclus"-Reste bei jedem Vorkommen gegebenenfalls selbst substituiert sein können; Alkyl-Substituenten auch die Reste "T" und "T-$R'^2$" beinhalten, wobei T als Stickstoff-, Sauerstoff- oder Schwefel-haltiger Rest definiert ist, und $R'^2$ H, Alkyl oder substituiertes Alkyl, Alkenyl oder substituiertes Alkenyl, Cycloalkyl oder substituiertes Cycloalkyl, Cycloalkenyl oder substituiertes Cycloalkenyl, Heterocyclo oder substituiertes Heterocyclo, Cycloalkylalkyl oder substituiertes Cycloallcylalkyl, Cycloalkenylalkyl oder substituiertes Cycloalkenylalkyl, Heterocycloalkyl oder substituiertes Heterocycloalkyl, Aryl oder substituiertes Aryl, Arylalkyl oder substituiertes Arylalkyl, Halogen, CN, $OR^1$, Nitro, Hydroxylamin, Hydroxylamid, Amino, $NHR^4$, $NR^2R^5$, $NOR^1$, Thiol, Alkylthio oder substituiertes Alkylthio, $R^1C$=O, $R^1OC$=O, $R^1NHC$=O, $SO_2R^1$, $SOR^1$, $PO_3R^1R^{1'}$, $R^1R^{1'}NC$=O, $C=OSR^1$, $SO_2R^1$, $SO_2OR^1$ oder $SO_2NR^1R^{1'}$ ist;

der Ausdruck "Alkenyl" einen geradkettigen oder verzweigten Kohlenwasserstoffrest, der 2 bis 12 Kohlenstoffatome und mindestens eine Kohlenstoff-Kohlenstoff-Doppelbindung enthält, bezeichnet; "substituiertes Alkenyl" einen Alkenylrest, der mit 1 bis 4 Substituenten an beliebigen zur Verfügung stehenden Bindungspunkten substituiert ist, bezeichnet; wobei die Substituenten aus Alkyl oder substituiertem Alkyl sowie den oben als Alkylsubstituenten genannten Resten ausgewählt sind;

der Ausdruck "Cycloalkyl" einen vollständig gesättigten cyclischen Kohlenwasserstoffrest mit 1 bis 4 Ringen und 3 bis 8 Kohlenstoffatomen pro Ring bezeichnet; "substituiertes Cycloalkyl" einen Cycloalkylrest, der mit 1 bis 4 Substituenten an beliebigen zur Verfügung stehenden Bindungspunkten substituiert ist, bezeichnet, wobei die Substituenten aus Nitro, Cyano, Alkyl oder substituiertem Alkyl sowie den oben als Alkylsubstituenten genannten Resten und Wasserstoff, Alkyl oder substituiertem Alkyl, Alkenyl oder substituiertem Alkenyl, Alkinyl oder substituiertem Alkinyl, Halogen, Cycloalkyl oder substituiertem Cycloalkyl, Cycloalkenyl oder substituiertem Cycloalkenyl, Aryl oder substituiertem Aryl, Heterocyclo oder substituiertem Heterocyclo, Arylalkyl oder substituiertem Arylalkyl, Heterocycloalkyl oder substituiertem Heterocycloalkyl, CN, $R^1OC$=O, $R^1C$=O, $R^1C$=S, $R^1HNC$=O, $R^1R^2NC$=O, $HOCR^3R^{3'}$, Nitro, $R^1OCH_2$, $R^1O$, $NH_2$, $NR^4R^5$, $SR^1$, $S=OR^1$, $SO_2R^1$, $SO_2NR^1R^{1'}$, $(R^1O)(R^{1'}O)P$=O, Oxo, $(R^1)(R^{1'})P$=O, $(R^1)(NHR^1)P$=O und spirogebundenen oder kondensiertem Cycloalkenyl oder substituiertem Cycloalkenyl ausgewählt sind;

der Ausdruck "Cycloalkenyl" einen teilweise ungesättigten cyclischen Kohlenwasserstoffrest mit 1 bis 4 Ringen und 3 bis 8 Kohlenstoffatomen pro Ring bezeichnet; "substituiertes Cycloalkenyl" einen Cycloalkenylrest, der mit 1 bis 4 Substituenten an beliebigen zur Verfügung stehenden Bindungspunkten substituiert ist, bezeichnet, wobei die Substituenten aus Nitro, Cyano, Alkyl oder substituiertem Alkyl sowie den oben als Alkylsubstituenten genannten Resten und Wasserstoff, Alkyl oder substituiertem Alkyl, Alkenyl oder substituiertem Alkenyl, Alkinyl oder substituiertem Alkinyl, Halogen, Cycloalkyl oder substituiertem Cycloalkyl, Cycloalkenyl oder substituiertem Cycloalkenyl, Aryl oder substituiertem Aryl, Heterocyclo oder substituiertem Heterocyclo, Arylalkyl oder substituiertem Arylalkyl, Heterocycloalkyl oder substituiertem Heterocycloalkyl, CN, $R^1OC$=O, $R^1C$=O, $R^1C$=S, $R^1HNC$=O, $R^1R^2NC$=O, $HOCR^3R^{3'}$, Nitro, $R^1OCH_2$, $R^1O$, $NH_2$, $NR^4R^5$, $SR^1$, $S=OR^1$, $SO_2R^1$, $SO_2OR^1$, $SO_2NR^1R^{1'}$, $(R^1O)(R^{1'}O)P$=O, Oxo, $(R^1)(R^{1'})P$=O, $(R^{1'})(NHR^1)P$=O und spirogebundenem oder kondensiertem Cycloalkyl oder substituiertem Cycloalkyl ausgewählt sind;

die Ausdrücke "Alkoxy" oder "Alkylthio" einen wie oben beschriebenen Alkylrest bezeichnen, der über eine Sauerstoffbindung (-O-) bzw. eine Schwefelbindung (-S-) gebunden ist; die Ausdrücke "substituiertes Alkoxy" oder "substituiertes Alkylthio" einen wie oben beschriebenen substituierten Alkylrest bezeichnen, der über eine Sauerstoff- bzw. Schwefelbindung gebunden ist;
der Ausdruck "Alkoxycarbonyl" einen Alkoxyrest bezeichnet, der über eine Carbonylgruppe gebunden ist;
der Ausdruck "Alkylcarbonyl" einen Alkylrest bezeichnet, der über eine Carbonylgruppe gebunden ist; der Ausdruck "Alkylcarbonyloxy" einen Alkylcarbonylrest bezeichnet, der über eine Sauerstoffbindung gebunden ist;
die Ausdrücke "Arylalkyl", "substituiertes Arylalkyl", "Cycloalkylalkyl", "substituiertes Cycloalkylalkyl", "Cycloalkenylalkyl", "substituiertes Cycloalkenylalkyl", "Heterocycloalkyl" und "substituiertes Heterocycloalkyl" Aryl-, Cycloalkyl-, Cycloalkenyl- und Heterocycloreste bezeichnen, die über einen Alkylrest gebunden sind und die an dem Aryl-, Cycloalkyl-, Cycloalkenyl- oder Heterocyclo- und/oder dem Alkylrest substituiert sind, wenn sie als "substituiert" bezeichnet sind;
der Ausdruck "Aryl" cyclische, aromatische Kohlenwasserstoffreste mit 1 bis 5 aromatischen Ringen bezeichnet; wenn sie zwei oder mehr aromatische Ringe enthalten, die aromatischen Ringe des Arylrests an einem einzigen Punkt verbunden oder kondensiert sein können, "substituiertes Aryl" einen mit 1, 2, 3, 4 oder 5 Substituenten an beliebigen zur Verfügung stehenden Bindungspunkten substituierten Arylrest bezeichnet, wobei die Substituenten aus Nitro, Cycloalkyl oder substituiertem Cycloalkyl, Cycloalkenyl oder substituiertem Cycloalkenyl, Cyano, Alkyl-$S(O)_m$- (m = 0, 1 oder 2), Alkyl oder substituiertem Alkyl sowie den oben als Alkylsubstituenten angegebenen Resten und Wasserstoff, Alkyl oder substituiertem Alkyl, Alkenyl oder substituiertem Alkenyl, Alkinyl oder substituiertem Alkinyl, Halogen, Cycloalkyl oder substituiertem Cycloalkyl, Cycloalkenyl oder substituiertem Cycloalkenyl, Aryl oder substituiertem Aryl, Heterocyclo oder substituiertem Heterocyclo, Arylalkyl oder substituiertem Arylalkyl, Heterocycloalkyl oder substituiertem Heterocycloalkyl, CN, $R^1OC{=}O$, $R^1C{=}O$, $R^1C{=}S$, $R^1HNC{=}O$, $R^1R^2NC{=}O$, $HOCR^3R^{3'}$, Nitro, $R^1OCH_2$, $R^1O$, $NH_2$, $NR^4R^5$, $SR^1$, $S{=}OR^1$, $SO_2R^1$, $SO_2OR^1$, $SO_2NR^1R^{1'}$, $(R^1O)(R^{1'}O)P{=}O$, Oxo, $(R^1)(R^{1'})P{=}O$, $(R^{1'})(NHR^1)P{=}O$, und kondensierten Heterocyclo- oder Cycloalkenyl-oder substituierten Heterocyclo- oder Cycloalkenylresten ausgewählt sind; "substituiertes Carbamoyl", "substituiertes Carbamat", "substituierter Harnstoff" und "substituiertes Amidinyl" Carbamoyl-, Carbamat-, Harnstoff- oder Amidinylreste bezeichnen, in denen einer oder mehrere der Wasserstoffreste durch einen der vorstehend aufgeführten organischen Reste ersetzt sind;
die Ausdrücke "Heterocyclus", "heterocyclisch" und "Heterocyclo" vollständig gesättigte oder teilweise oder vollständig ungesättigte, einschließlich aromatischer 3- bis 7-gliedriger monocyclischer, 7- bis 11-gliedriger bicyclischer oder 10- bis 16-gliedriger tricyclischer Ringsysteme bezeichnen, die mindestens ein Heteroatom in mindestens einem Kohlenstoffatom-enthaltenden Ring aufweisen, wobei jeder Ring des heterocyclischen Rests, der ein Heteroatom enthält, 1, 2, 3 oder 4 Heteroatome, ausgewählt aus Stickstoffatomen, Sauerstoffatomen und/oder Schwefelatomen, aufweisen kann, wobei die Stickstoff- und Schwefel-Heteroatome gegebenenfalls oxidiert sein können und die Stickstoff-Heteroatome gegebenenfalls quatemisiert sein können;
"substituierter Heterocyclus", "substituierter/s heterocyclischer/s" und "substituiertes Heterocyclo" einen Heterocyclus, heterocyclischen Rest oder Heterocyclorest bezeichnen, der an beliebigen zur Verfügung stehenden Bindungspunkten mit 1 bis 4 Substituenten substituiert ist, wobei die Substituenten aus Cycloalkyl oder substituiertem Cycloalkyl, Cycloalkenyl oder substituiertem Cycloalkenyl, Nitro, Oxo, Cyano, $AlkylS(O)_m$- (m = 0, 1 oder 2), Alkyl oder substituiertem Alkyl sowie den oben als Alkylsubstituenten angegebenen Resten und Wasserstoff, Alkyl oder substituiertem Alkyl, Alkenyl oder substituiertem Alkenyl, Alkinyl oder substituiertem Alkinyl, Halogen, Cycloalkyl oder substituiertem Cycloalkyl, Cycloalkenyl oder substituiertem Cycloalkenyl, Aryl oder substituiertem Aryl, Heterocyclo oder substituiertem Heterocyclo, Arylalkyl oder substituiertem Arylalkyl, Heterocycloalkyl oder substituiertem Heterocycloalkyl, CN, $R^1OC{=}O$, $R^1C{=}O$, $R^1C{=}S$, $R^1HNC{=}O$, $R^1R^2NC{=}O$, $HOCR^3R^{3'}$, Nitro, $R^1OCH_2$, $R^1O$, $NH_2$, $NR^4R^5$, $SR^1$, $S{=}OR^1$, $SO_2R^1$, $SO_2OR^1$, $SO_2NR^1R^{1'}$, $(R^1O)(R^{1'}O)P{=}O$, Oxo, $(R^1)(R^{1'})P{=}O$, $(R^{1'})(NHR^1)P{=}O$ ausgewählt sind.

**2.** Verbindung nach Anspruch 1, wobei L eine Bindung ist.

**3.** Verbindung, ausgewählt aus:

(3aα,4α,7α,7aα)-2-(4-Brom-3-methylphenyl)tetrahydro-4,7-ethanothiopyrano[3,4-c]pyrrol-1,3,8(2H,4H)-trion (1C);
(3aα,4α,7α,7aα)-2-(4-Brom-3-methylphenyl)tetrahydro-4,7-ethanothiopyrano[3,4-c]pyrrol-1,3,8(2H,4H)-trion-5,5-dioxid (2);
(3aα,4β,7β,7aα)-2-(3-Chlorphenyl)hexahydro-4-methyl-4,7-epoxy-1H-isoindol-1,3(2H)-dion (3);
(3aα,4α,7α,7aα)- und (3aα,4β,7β,7aα)-4-[(Acetyloxy)methyl]-3a,4,7,7a-tetrahydro-2-[3-(trifluormethyl)phenyl]-4,7-epoxy-1H-isoindol-1,3(2H)-dion (4i bzw. 4ii);
(3aα,4α,7α,7aα)- und (3aα,4β,7β,7aα)-4-[(Acetyloxy)methyl]-hexahydro-2-[3-(trifluormethyl)phenyl]-4,7-epoxy-1H-isoindol-1,3(2H)-dion (5i bzw. 5ii);
(3aα,4α,7α,7aα)- und (3aα,4β,7β,7aα)-3a,4,7,7a-Tetrahydro-5-(hydroxymethyl)-2-[3-(trifluorrnethyl)phenyl]-4,7-epoxy-1H-isoindol-1,3(2H)-dion (6i bzw. 6ii);
(3aα, 4α, 7α, 7aα)- 3a, 4,7,7a-Tetrahydro- 5-(hydroxymethyl)- 4-methyl- 2-[3-(trifluormethyl) phenyl]- 4,7-epoxy-1H-isoindol-1,3(2H)-dion (7);
(3aα,4β,7β,7aα)-2-[3,5-Bis(trifluormethyl)phenyl]hexahydro-4,7-epoxy-1H-isoindol-1,3(2H)-dion (8);
(3aα,4α,7α,7aα)-2-(4-Bromphenyl)octahydro-1,3-dioxo-4,7-etheno-5H-pyrrolo[3,4-c]pyridin-5-carbonsäurephenylester (9);
(3aα,4α,7α,7aα)-2-(4-Bromphenyl)octahydro-1,3-dioxo-4,7-etheno-5H-pyrrolo[3,4-c]pyridin-5-carbonsäurephenylmethylester (10);
(3aα, 4α, 7α, 7aα) -Hexahydro- 2-[3-(trifluormethyl) phenyl]- 4,7-ethano- 1H-pyrrolo [3,4-c] pyridin- 1,3 (2H) -diontrifluoracetat (11);
(3aα,4α,7α,7aα)-5-Acetylhexahydro-2-[3-(trifluormethyl)phenyl]-4,7-ethano-1H-pyrrolo[3,4-c]pyridin-1,3(2H)-dion (12);
(3aα, 4α, 7α, 7aα)- 5-Benzoylhexahydro- 2-[3-(trifluormethyl) phenyl]- 4,7-ethano- 1H-pyrrolo [3,4-c] pyridin- 1,3 (2H)-dion (13);
(3aα, 4α, 7α, 7aα) -Hexahydro- 5-methyl- 2-[3-(trifluormethyl) phenyl]- 4,7-ethano- 1H-pyrrolo [3,4-c] pyridin- 1,3 (2H)-dion (14);
(3aα,4α,7α,7aα)-Hexahydro-5-(phenylmethyl)-2-[3-(trifluormethyl)phenyl]-4,7-ethano-1H-pyrrolo[3,4-c]pyridin-1,3(2H)-diontrifluoracetat (15);
(3aα,4α,7α,7aα)-Hexahydro-5-propyl-2-[3-(trifluormethyl)phenyl]-4,7-ethano-1H-pyrrolo[3,4-c]pyridin-1,3(2H)-diontrifluoracetat (16);
(3aα,4α,4aβ,5aβ,6α,6aα)-2-[4-Cyano-3-(trifluormethyl)phenyl]decahydro-1,3-dioxo-4,6-(iminomethano)cycloprop[f]isoindol-7-carbonsäurephenylmethylester (17);
(3aα,4α,4aβ,5aβ,6α,6aα)-4-[Decahydro-1,3-dioxo-4,6-(iminomethano)cycloprop[f]isoindol-2-yl]-2-(trifluormethyl)benzonitril (18);
(3aα, 4α, 4aβ, 5aβ, 6α, 6aα)- 4-[Decahydro- 7-methyl- 1,3-dioxo- 4,6-(iminomethano) cycloprop [f] isoindol- 2-yl]-2-(trifluormethyl)benzonitril (19);
(3aα,4β,7β,7aα)-4-(Octahydro-4,7-dimethyl-1,3-dioxo-4,7-epoxy-2H-isoindol-2-yl)-2-(trifluormethyl)benzonitril (20B);
(3aα, 4β, 7β, 7aα) -N-[4-[[2-[2-[4-Cyano- 3-(trifluormethyl) phenyl] octahydro- 7-methyl- 1,3-dioxo- 4,7-epoxy-4H-isoindol-4-yl]ethyl]thio]phenyl]acetamid (21E);
(3aα, 4β, 7β, 7aα) -N-[4-[[2-[2-[4-Cyano- 3-(trifluormethyl) phenyl] octahydro- 7-methyl- 1,3-dioxo- 4,7-epoxy-4H-isoindol-4-yl]ethyl]sulfinyl]phenyl]acetamid (22);
(3aα, 4β, 7β, 7aα) -N-[4-[[2-[2-[4-Cyano- 3-(trifluormethyl) phenyl] octahydro- 7-methyl- 1,3-dioxo- 4,7-epoxy-4H-isoindol-4-yl]ethyl]sulfonyl]phenyl]acetamid (23);
(3aα,4β,7β,7aα)- und (3aα,4α,7α,7aα)-N-[2-[2-[4-Cyano-3-(trifluormethyl)phenyl]octahydro-7-methyl-1,3-dioxo-4,7-epoxy-4H-isoindol-4-yl]ethyl]benzolsulfonamid (24Ci bzw. 24Cii);
(3aα,4β,7β,7aα)-4-[Octahydro-4-(2-hydroxyethyl)-7-methyl-1,3-dioxo-4,7-epoxy-2H-isoindol-2-yl]-2-(trifluormethyl)benzonitril (25B);
(3aα,4α,7α,7aα)- und (3aα,4β,7β,7aα)-N-[4-[2-[2-[4-Cyano-3-(trifluormethyl)phenyl]-octahydro-7-methyl-1,3-dioxo-4,7-epoxy-4H-isoindol-4-yl]ethoxy]phenyl]acetamid (26Ci bzw. 26Cii);
(3aα,4α,7α,7aα)-Hexahydro-2-(2-naphthalenyl)-4,7-epoxy-1H-isoindol-1,3(2H)-dion (27D);
(1aα,2β,2aα,5aα,6β,6aα)-Hexahydro-4-(2-naphthalenyl)-2,6-epoxy-3H-oxireno[f]isoindol-3,5(4H)-dion (28B);
(3aα,4α,7α,7aα)-2-[4-Brom-3-(trifluormethyl)phenyl]-3a,4,7,7a-tetrahydro-4,7-dimethyl-4,7-epithio-1H-isoindol-1,3(2H)-dion-8-oxid (29);
(3aα,4α,7α,7aα)-2-[4-Brom-3-(trifluormethyl)phenyl]-3a,4,7,7a-tetrahydro-4,7-epithio-1H-isoindol-1,3(2H)-dion-8-oxid (30);
(3aα,4α,7α,7aα)-Hexahydro-2-[3-(trifluormethyl)phenyl]-4,7-imino-1H-isoindol-1,3(2H)-dion (3 1 D);
(3aα,4β,7β,7aα)- und (3aα,4α,7α,7aα)-3a,4,7,7a-Tetrahydro-4,7-dimethyl-2-[3-(trifluormethyl)phenyl]-4,7-epoxy-1H-isoindol-1,3(2H)-dion (32i bzw. 32ii);
(3aα, 4α, 7α, 7aα) -Hexahydro- 4,7-dimethyl- 2-[3-(trifluormethyl) phenyl]- 4,7-epoxy- 1H-isoindol- 1,3 (2H) -dion (33);
(3aα, 4α, 7α, 7aα) -Tetrahydro- 5-methyl- 2-(4-nitro- 1-naphthalenyl)- 4,7-etheno- 1H-pyrrolo [3,4-c] pyridin- 1,3,6 (2H,5H)-trion (34B);
(3aα, 4β, 7β, 7aα)- 4-[4-[2-(4-Fluorphenoxy) ethyl] octahydro- 7-methyl- 1,3-dioxo- 4,7-epoxy- 2H-isoindol- 2-yl]-2-(trifluormethyl)benzonitril (35);
(3aα,4β,7β,7aα)-4-[4-(2-Bromethyl)octahydro-7-methyl-1,3-dioxo-4,7-epoxy-2H-isoindol-2-yl]-2-(trifluormethyl)benzonitril (36);
(3aα,4β,7β,7aα)-Hexahydro-4,7-dimethyl-2-(3-methyl-4-nitrophenyl)-4,7-epoxy-1H-isoindol-1,3(2H)-dion (37);

(3aα,4β,7β,7aα)-2-(2-Fluorenyl)hexahydro-4,7-epoxy-1H-isoindol-1,3(2H)-dion;
(3aα,4β,7β,7aα)-2-[3-Chlor-4-(4-morpholinyl)phenyl]hexahydro-4,7-epoxy-1H-isoindol-1,3(2H)-dion;
(3aα,4β,7β,7aα)-2-(2,3-Dihydro-1H-inden-5-yl)hexahydro-4,7-epoxy-1H-isoindol-1,3(2H)-dion;
(3aα,4β,7β,7aα)-2-(4-Brom-1-naphthalenyl)hexahydro-4,7-epoxy-1H-isoindol-1,3(2H)-dion;
(3aα,4β,7β,7aα)-2-(4-Chlor-1-naphthalenyl)hexahydro-4,7-epoxy-1H-isoindol-1,3(2H)-dion;
(3aα,4β,7β,7aα)-2-(5-Amino-1-naphthalenyl)hexahydro-4,7-epoxy-1H-isoindol-1,3(2H)-dion;
(3aα,4β,7β,7aα)-Hexahydro-2-(7-hydroxy-1-naphthalenyl)-4,7-epoxy-1H-isoindol-1,3(2H)-dion;
(3aα,4β,7β,7aα)-Hexahydro-2-(4-nitro-1-naphthalenyl)-4,7-epoxy-1H-isoindol-1,3(2H)-dion;
(3aα,4β,7β,7aα)-Hexahydro-2-(1H-indol-5-yl)-4,7-epoxy-1H-isoindol-1,3(2H)-dion;
(3aα,4β,7β,7aα)-Hexahydro-2-(1H-indazol-6-yl)-4,7-epoxy-1H-isoindol-1,3(2H)-dion;
(3aα,4β,7β,7aα)-2-(1,3-Benzodioxol-5-yl)hexahydro-4,7-epoxy-1H-isoindol-1,3(2H)-dion;
(3aα,4β,7β,7aα)-2-[4-Amino-3-(trifluormethyl)phenyl]hexahydro-4,7-epoxy-1H-isoindol-1,3(2H)-dion;
(3aα,4β,7β,7aα)-2-(3-Chlor-4-iodphenyl)hexahydro-4,7-epoxy-1H-isoindol-1,3(2H)-dion;
(3aα,4β,7β,7aα)-Hexahydro-2-(8-chinolinyl)-4,7-epoxy-1H-isoindol-1,3(2H)-dion;
(3aα,4β,7β,7aα)-2-(2,3-Dihydro-1,4-benzodioxin-6-yl)hexahydro-4,7-epoxy-1H-isoindol-1,3(2H)-dion;
(3aα,4β,7β,7aα)-Hexahydro-2-[2-oxo-4-(trifluormethyl)-2H-1-benzopyran-7-yl]-4,7-epoxy-1 H-isoindol-1,3(2H)-dion;
(3aα,4β,7β,7aα)-Hexahydro-2-(4-methyl-2-oxo-2H-1-benzopyran-7-yl)-4,7-epoxy-1H-isoindol-1,3(2H)-dion;
(3aα,4β,7β,7aα)-2-(2,5-Dimethoxy-4-nitrophenyl)hexahydro-4,7-epoxy-1H-isoindol-1,3(2H)-dion;
(3aα,4β,7β,7aα)-2,3,5,6-Tetrafluor-4-(octahydro-1,3-dioxo-4,7-epoxy-2H-isoindol-2-yl)benzonitril;
(3aα,4β,7β,7aα)-Hexahydro-2-(2,4,5-trifluorphenyl)-4,7-epoxy-1H-isoindol-1,3(2H)-dion;
(3aα,4β,7β,7aα)-Hexahydro-2-(2,4,5-trichlorphenyl)-4,7-epoxy-1H-isoindol-1,3(2H)-dion;
(3aα,4β,7β,7aα)-2-(2-Amino-4,5-dichlorphenyl)hexahydro-4,7-epoxy-1H-isoindol-1,3(2H)-dion;
(3aα,4β,7β,7aα)-2-(3,4-Difluorphenyl)hexahydro-4,7-epoxy-1H-isoindol-1,3(2H)-dion;
(3aα,4β,7β,7aα)-1-Acetyl-2,3-dihydro-6-(octahydro-1,3-dioxo-4,7-epoxy-2H-isoindol-2-yl)-1H-indol;
(3aα,4β,7β,7aα)-2-(3-Chlor-4-fluorphenyl)hexahydro-4,7-epoxy-1H-isoindol-1,3(2H)-dion;
(3aα,4β,7β,7aα)-2-(3,4-Dichlorphenyl)hexahydro-4,7-epoxy-1H-isoindol-1,3(2H)-dion;
(3aα,4β,7β,7aα)-Hexahydro-2-(3,4,5-trichlorphenyl)-4,7-epoxy-1H-isoindol-1,3(2H)-dion;
(3aα,4β,7β,7aα)-2-(3-Chlor-4-methoxyphenyl)hexahydro-4,7-epoxy-1H-isoindol-1,3(2H)-dion;
(3aα,4β,7β,7aα)-2-(3-Chlor-4-methylphenyl)hexahydro-4,7-epoxy-1H-isoindol-1,3(2H)-dion;
(3aα,4β,7β,7aα)-Hexahydro-2-(2-methyl-1-naphthalenyl)-4,7-epoxy-1H-isoindol-1,3(2H)-dion;
(3aα,4β,7β,7aα)-2-(4-Chlor-3-methylphenyl)hexahydro-4,7-epoxy-1H-isoindol-1,3(2H)-dion;
(3aα,4β,7β,7aα)-2-(3,4-Dimethylphenyl)hexahydro-4,7-epoxy-1H-isoindol-1,3(2H)-dion;
(3aα,4β,7β,7aα)-2-[4-Brom-3-(trifluormethyl)phenyl]hexahydro-4,7-epoxy-1H-isoindol-1,3(2H)-dion;
(3aα,4β,7β,7aα)-2-(4-Brom-3-methylphenyl)hexahydro-4,7-epoxy-1H-isoindol-1,3(2H)-dion;
(3aα,4β,7β,7aα)-2-(4-Fluor-3-nitrophenyl)hexahydro-4,7-epoxy-1H-isoindol-1,3(2H)-dion;
(3aα,4β,7β,7aα)-2-[4-Fluor-3-(trifluormethyl)phenyl]hexahydro-4,7-epoxy-1H-isoindol-1,3(2H)-dion;
(3aα,4β,7β,7aα)-2-(4-Chlor-3-nitrophenyl)hexahydro-4,7-epoxy-1H-isoindol-1,3(2H)-dion;
(3aα,4β,7β,7aα)-2-[4-Chlor-3-(trifluormethyl)phenyl]hexahydro-4,7-epoxy-1H-isoindol-1,3(2H)-dion;
(3aα,4β,7β,7aα)-2-(4-Chlor-2-methoxy-5-methylphenyl)hexahydro-4,7-epoxy-1H-isoindol-1,3(2H)-dion;
(3aα,4β,7β,7aα)-2-(4-Amino-3-nitrophenyl)hexahydro-4,7-epoxy-1H-isoindol-1,3(2H)-dion;
(3aα,4β,7β,7aα)-Hexahydro-2-(4-methyl-3-nitrophenyl)-4,7-epoxy-1H-isoindol-1,3(2H)-dion;
(3aα,4β,7β,7aα)-2-(3,4-Dimethoxyphenyl)hexahydro-4,7-epoxy-1H-isoindol-1,3(2H)-dion;
(3aα,4β,7β,7aα)-Hexahydro-2-(3-hydroxy-4-methoxyphenyl)-4,7-epoxy-1H-isoindol-1,3(2H)-dion;
(3aα,4β,7β,7aα)-Hexahydro-2-(4-methyl-5-nitro-2-pyridinyl)-4,7-epoxy-1H-isoindol-1,3(2H)-dion;
(3aα,4β,7β,7aα)-2-Chlor-4-(octahydro-1,3-dioxo-4,7-epoxy-2H-isoindol-2-yl)- α-phenylbenzolacetonitril;
(3aα,4β,7β,7aα)-Hexahydro-2-(2-methoxy-3-dibenzofuranyl)-4,7-epoxy-1H-isoindol-1,3(2H)-dion;
(3aα,4β,7β,7aα)-Hexahydro-2-(2,3,4-trifluorphenyl)-4,7-epoxy-1H-isoindol-1,3(2H)-dion;
(3aα, 4β, 7β, 7aα)- 2-(2,3-Dihydro- 2-methyl- 1,3-dioxo- 1H-isoindol- 5-yl) hexahydro- 4,7-epoxy- 1H-isoindol- 1,3 (2H)-dion;
(3aα,4β,7β,7aα)-2-(4-Brom-2,3,5,6-tetrafluorphenyl)hexahydro-4,7-epoxy-1H-isoindol-1,3(2H)-dion;
(3aα,4β,7β,7aα)-Hexahydro-2-(2-hydroxy-1-naphthalenyl)-4,7-epoxy-1H-isoindol-1,3(2H)-dion;
(3aα,4β,7β,7aα)-2-[2,5-Dichlor-4-(1H-pyrrol-1-yl)phenyl]hexahydro-4,7-epoxy-1H-isoindol-1,3(2H)-dion;
(3aα, 4β, 7β, 7aα) -Hexahydro- 2-[4-(methoxymethyl)- 2-oxo- 2H- 1-benzopyran- 7-yl]- 4,7-epoxy- 1H-isoindol- 1,3 (2H)-dion;
(3aα,4β,7β,7aα)-2-(6-Benzothiazolyl)hexahydro-4,7-epoxy-1H-isoindol-1,3(2H)-dion;
(3aα,4β,7β,7aα)-2-Methoxy-4-(octahydro-1,3-dioxo-4,7-epoxy-2H-isoindol-2-yl)benzoesäuremethylester;
(3aα,4β,7β,7aα)-2-Methyl-5-(octahydro-1,3-dioxo-4,7-epoxy-2H-isoindol-2-yl)benzonitril;

(3aα,4β,7β,7aα)-Hexahydro-2-(2-oxo-2H-1-benzopyran-6-yl)-4,7-epoxy-1H-isoindol-1,3(2H)-dion;
(3aα,4β,7β,7aα)-Hexahydro-2-(2,3,5,6-tetramethyl-4-nitrophenyl)-4,7-epoxy-1H-isoindol-1,3(2H)-dion;
(3aα,4β,7β,7aα)-Hexahydro-2-(2,4,5-trimethylphenyl)-4,7-epoxy-1H-isoindol-1,3(2H)-dion;
(3aα,4β,7β,7aα)-2-(4-Fluor-3-methylphenyl)hexahydro-4,7-epoxy-1H-isoindol-1,3(2H)-dion;
(3aα,4β,7β,7aα)-Hexabydro-2-(3-methoxy-4-methylphenyl)-4,7-epoxy-1H-isoindol-1,3(2H)-dion;
(3aα,4β,7β,7aα)-N-Ethyl-2-methyl-5-(octahydro-1,3-dioxo-4,7-epoxy-2H-isoindol-2-yl)-N-phenylbenzolsulfonamid;
(3aα,4β,7β,7aα)-2,6-Dibrom-4-(octahydro-1,3-dioxo-4,7-epoxy-2H-isoindol-2-yl)benzolsulfonamid;
(3aα,4β,7β,7aα)-2,4-Dimethyl-6-(octahydro-1,3-dioxo-4,7-epoxy-2H-isoindol-2-yl)-3-pyridincarbonitril;
(3aα,4β,7β,7aα)-2-(2,3-Dimethyl-1H-indol-5-yl)hexahydro-4,7-epoxy-1H-isoindol-1,3(2H)-dion;
(3aα,4β,7β,7aα)-2-(3-Dibenzofuranyl)hexahydro-4,7-epoxy-1H-isoindol-1,3(2H)-dion;
(3aα,4β,7β,7aα)-Hexahydro-2-(2'-hydroxy[1,1':3',1"-terphenyl]-5'-yl)-4,7-epoxy-1H-isoindol-1,3(2H)-dion;
(3aα,4β,7β,7aα)-Hexahydro-2-(5,6,7,8-tetrahydro-3-hydroxy-2-naphthalenyl)-4,7-epoxy-1H-isoindol-1,3(2H)-dion;
(3aα,4β,7β,7aα)-2-(2,3-Dihydro-1H-indol-6-yl)hexahydro-4,7-epoxy-1H-isoindol-1,3(2H)-dion;
(3aα,4β,7β,7aα)-2-(1,3-Dihydro-2,2-dioxidobenzo[c]thiophen-5-yl)hexahydro-4,7-epoxy-1H-isoindol-1,3(2H)-dion;
(3aα,4β,7β,7aα)-Hexahydro-2-(2-hydroxy-4,5-dimethylphenyl)-4,7-epoxy-1H-isoindol-1,3(2H)-dion;
(3aα,4β,7β,7aα)-2-(2,3-Dihydro-2,2,3,3-tetrafluor-1,4-benzodioxin-6-yl)hexahydro-4,7-epoxy-1H-isoindol-1,3(2H)-dion;
(3aα,4β,7β,7aα)-Hexahydro-2-(1H-indazol-5-yl)-4,7-epoxy-1H-isoindol-1,3(2H)-dion;
(3aα,4β,7β,7aα)-2-(4-Amino-2,3,5,6-tetrafluorphenyl)hexahydro-4,7-epoxy-1H-isoindol-1,3(2H)-dion;
(3aα,4β,7β,7aα)-2-(4-Brom-3-chlorphenyl)hexahydro-4,7-epoxy-1H-isoindol-1,3(2H)-dion;
(3aα,4β,7β,7aα)-Hexahydro-2-(5-hydroxy-1-naphthalenyl)-4,7-epoxy-1H-isoindol-1,3(2H)-dion;
(3aα,4β,7β,7aα)-4-(Octahydro-1,3-dioxo-4,7-epoxy-2H-isoindol-2-yl)-2-(trifluormethyl)benzonitril;
(3aα,4β,7β,7aα)-2-(4-Morpholinyl)-5-(octahydro-1,3-dioxo-4,7-epoxy-2H-isoindol-2-yl)benzoesäuremethylester;
(3aα,4β,7β,7aα)-2-Fluor-5-(octahydro-1,3-dioxo-4,7-epoxy-2H-isoindol-2-yl)benzonitril;
(3aα,4β,7β,7aα)-Hexahydro-2-(2-naphthalenyl)-4,7-epoxy-1H-isoindol-1,3(2H)-dion;
(3aα,4β,7β,7aα)-2-(9-Ethyl-9H-carbazol-3-yl)hexahydro-4,7-epoxy-1H-isoindol-1,3(2H)-dion;
(3aα,4β,7β,7aα)-2-[1,2-Dihydro-8-methyl-2-oxo-4-(trifluormethyl)-7-chinolinyl]hexahydro-4,7-epoxy-1H-isoindol-1,3(2H)-dion;
(3aα,4α,7α,7aα)-Hexahydro-2-[3-(trifluormethyl)phenyl]-4,7-epoxy-1H-isoindol-1,3(2H)-dion;
(3aα,4α,7α,7aα)-Hexahydro-2-(4-nitro-1-naphthalenyl)-4,7-epoxy-1H-isoindol-1,3(2H)-dion;
(3aα,4β,7β,7aα)-2-(4-Brom-3-methylphenyl)-3a,4,7,7a-tetrahydro-4,7-epoxy-1H-isoindol-1,3(2H)-dion;
(3aα,4β,7β,7aα)-3a,4,7,7a-Tetrahydro-2-(2-naphthalenyl)-4,7-epoxy-1H-isoindol-1,3(2H)-dion;
(3aα,4β,7β,7aα)-2-(9-Ethyl-9H-carbazol-3-yl)-3a,4,7,7a-tetrahydro-4,7-epoxy-1H-isoindol-1,3(2H)-dion;
(3aα,4β,7β,7aα)-2-[4-Fluor-3-(trifluormethyl)phenyl]-3a,4,7,7a-tetrahydro-4,7-epoxy-1H-isoindol-1,3(2H)-dion;
(3aα,4β,7β,7aα)-2-[1,2-Dihydro-8-methyl-2-oxo-4-(trifluormethyl)-7-chinolinyl]-3a,4,7,7a-tetrahydro-4,7-epoxy-1H-isoindol-1,3(2H)-dion;
(3aα,4α,7α,7aα)-4-[(Acetyloxy)methyl]-2-(4-brom-3-methylphenyl)-3a,4,7,7a-tetrahydro-4,7-epoxy-1H-isoindol-1,3(2H)-dion;
(3aα,4β,7β,7aα)-4-[(Acetyloxy)methyl]-2-(4-brom-3-methylphenyl)-3a,4,7,7a-tetrahydro-4,7-epoxy-1H-isoindol-1,3(2H)-dion;
(3aα,4β,7β,7aα)-Hexahydro-4,7-dimethyl-2-[3-(trifluormethyl)phenyl]-4,7-epoxy-1H-isoindol-1,3(2H)-dion;
(3aα,4β,7β,7aα)-4-(Octahydro-4,7-dimethyl-1,3-dioxo-4,7-epoxy-2H-isoindol-2-yl)-1-naphthalincarbonitril;
(3aα,4β,7β,7aα)-(Benzo[b]thiophen-3-yl)hexahydro-4,7-dimethyl-4,7-epoxy-1H-isoindol-1,3(2H)-dion;
(3aα,4β,7β,7aα)-Hexahydro-4,7-dimethyl-2-[4-nitro-3-(trifluormethyl)phenyl]-4,7-epoxy-1H-isoindol-1,3(2H)-dion;
(3aα, 4β, 7β, 7aα)-4-(1,3,3a, 4,7,7a-Hexahydro-4,7-dimethyl-1,3-dioxo-4,7-epoxy-2H-isoindol-2-yl)-1-naphthalincarbonitril;
(3aα,4α,7α,7aα)-Hexahydro-4-methyl-2-(2-naphthalenyl)-4,7-epoxy-1H-isoindol-1,3(2H)-dion;
(3aα,4β,7β,7aα)-2-(4-Brom-3-methylphenyl)hexahydro-4-methyl-4,7-epoxy-1H-isoindol-1,3(2H)-dion;
(3aα,4β,7β,7aα)-Hexahydro-4-methyl-2-[3-(trifluormethyl)phenyl]-4,7-epoxy-1H-isoindol-1,3(2H)-dion;
(3aα,4β,7β,7aα)-2-(3,5-Dichlorphenyl)hexahydro-4-methyl-4,7-epoxy-1H-isoindol-1,3(2H)-dion;
(3aα,4β,7β,7aα)-2-(3-Chlor-4-fluorphenyl)hexahydro-4-methyl-4,7-epoxy-1H-isoindol-1,3(2H)-dion;
(3aα,4β,7β,7aα)-2-Methoxy-4-(octahydro-1,3-dioxo-4-methyl-4,7-epoxy-2H-isoindol-2-yl)-1-naphthalincarbonitril;
(3aα,4β,7β,7aα)-Hexahydro-4-methyl-2-[4-nitro-3-(trifluormethyl)phenyl]-4,7-epoxy-1H-isoindol-1,3(2H)-dion;
(3aα,4β,7β,7aα)-Hexahydro-2-[4-(1H-imidazol-1-yl)phenyl]-4-methyl-4,7-epoxy-1H-isoindol-1,3(2H)-dion;
(3aα,4α,7α,7aα)-2-[3-Chlor-4-(2-thiazolyl)phenyl]hexahydro-4-methyl-4,7-epoxy-1H-isoindol-1,3(2H)-dion;
(3aα,4α,7α,7aα)-2-(3,5-Dichlorphenyl)hexahydro-4,7-imino-1H-isoindol-1,3(2H)-dion;
(3aα,4α,7α,7aα)-2-(4-Brom-1-naphthalenyl)hexahydro-4,7-imino-1H-isoindol-1,3(2H)-dion;
(3aα,4α,7α,7aα)-2-(4-Brom-3-methylphenyl)hexahydro-4,7-imino-1H-isoindol-1,3(2H)-dion;
(3aα,4α,7α,7aα)-Hexahydro-2-(4-nitro-1-naphthalenyl)-4,7-imino-1H-isoindol-1,3(2H)-dion;
(3aα,4α,7α,7aα)-8-Acetyl-2-(3,5-dichlorphenyl)hexahydro-4,7-imino-1H-isoindol-1,3(2H)-dion;
(3aα,4α,7α,7aα)-Octahydro-1,3-dioxo-2-[3-(trifluormethyl)phenyl]-4,7-ethano-5H-pyrrolo[3,4-c]pyridin-5-carbonsäurephenylester;
(3aα,4α,7α,7aα)-4-(Octahydro-1,3-dioxo-4,7-ethano-2H-pyrrolo[3,4-c]pyridin-2-yl)-1-naphthalincarbonitril;
(3aα,4α,7α,7aα)-4-(Octahydro-5-methyl-1,3-dioxo-4,7-ethano-2H-pyrrolo[3,4-c]pyridin-2-yl)-1-naphthalincarbonitril;
(3aα,4α,7α,7aα)-2-(4-Cyano-1-naphthalenyl)octahydro-1,3-dioxo-4,7-etheno-5H-pyrrolo[3,4-c]pyridin-5-carbonsäurephenylmethylester;
(3aα,4α,7α,7aα)-4-(Octahydro-1,3-dioxo-4,7-ethano-2H-pyrrolo[3,4-c]pyridin-2-yl)-2-(trifluormethyl)benzonitril;
(3aα,4α,7α,7aα)-4-(Octahydro-5-methyl-1,3-dioxo-4,7-ethano-2H-pyrrolo[3,4-c]pyridin-2-yl)-2-(trifluormethyl)benzonitril;
(3aα,4α,7α,7aα)-2-[4-Cyano-3-(trifluormethyl)phenyl]octahydro-1,3-dioxo-4,7-etheno-5H-pyrrolo[3,4-c]pyridin-5-carbonsäurephenylmethylester;
(3aα,4α,7α,7aα)-2-[4-Brom-3-(trifluormethyl)phenyl]tetrahydro-5-methyl-4,7-etheno-1H-pyrrolo[3,4-c]pyridin-1,3,6(2H,5H)-trion;
(3aα,4α,7α,7aα)-Tetrahydro-5-methyl-2-[3-(trifluormethyl)phenyl]-4,7-etheno-1H-pyrrolo[3,4-c]pyridin-1,3,6(2H,5H)-trion;
(3aα,4α,7α,7aα)-Tetrahydro-5-methyl-2-(2-naphthalenyl)-4,7-etheno-1H-pyrrolo[3,4-c]pyridin-1,3,6(2H,5H)-trion;
(1aα,2β,2aα,5aα,6β,6aα)-Hexahydro-4-[3-(trifluormethyl)phenyl]-2,6-epoxy-3H-oxireno[f]isoindol-3,5(4H)-dion;
(1aα,2β,2aα,5aα,6β,6aα)-4-(3,5-Dichlorphenyl)hexahydro-2,6-epoxy-3H-oxireno[f]isoindol-3,5(4H)-dion;
(1aα,2β,2aα,5aα,6β,6aα)-Hexahydro-4-(4-nitro-1-naphthalenyl)-2,6-epoxy-3H-oxireno[f]isoindol-3,5(4H)-dion;
(1aα,2β,2aα,5aα,6β,6aα)-4-(3,4-Dichlorphenyl)hexahydro-2,6-epoxy-3H-oxireno[f]isoindol-3,5(4H)-dion;
2-[4-(4-Bromphenoxy)phenyl]-3a,4,7,7a-tetrahydro-4,7-dimethyl-4,7-epoxy-1H-isoindol-1,3(2H)-dion;
3a,4,7,7a-Tetrahydro-2-(2-methoxyphenyl)-4,7-dimethyl-4,7-epoxy-1H-isoindol-1,3(2H)-dion;
[(1,2,3,3a,7,7a-Hexahydro-2-phenyl-4,7-epoxy-4H-isoindol-4-yl)methyl]carbaminsäure-(3,5-dimethoxyphenyl)methylester;
2-(2,4-Dimethylphenyl)-3a,4,7,7a-tetrahydro-4-(hydroxymethyl)-4,7-epoxy-1H-isoindol-1,3(2H)-dion;
2-(1,3-Benzodioxol-5-yl)-3a,4,7,7a-tetrahydro-4-methyl-4,7-epoxy-1H-isoindol-1,3(2H)-dion;
4-[Bis(acetyloxy)methyl]-2-(3-bromphenyl)-3a,4,7,7a-tetrahydro-4,7-epoxy-1H-isoindol-1,3(2H)-dion;
N-[[1,2,3,3a, 7,7a-Hexahydro- 2-(2,4,6-trimethylphenyl)- 4,7-epoxy- 4H-isoindol- 4-yl] methyl]- 2,2-dimethylpropanamid;
3a,4,7,7a-Tetrahydro-4-(hydroxymethyl)-2-[2-(trifluormethyl)phenyl]-4,7-epoxy-1H-isoindol-1,3(2H)-dion;
3a,4,7,7a-Tetrahydro-4-(hydroxymethyl)-2-(1-naphthalenyl)-4,7-epoxy-1H-isoindol-1,3(2H)-dion;
2-Chlor-5-(1,3,3a,4,7,7a-hexahydro-4,7-dimethyl-4,7-epoxy-2H-isoindol-2-yl)benzoesäuremethylester;
4-[Bis(acetyloxy)methyl]-2-(4-brom-2-nitrophenyl)-3a,4,7,7a-tetrahydro-4,7-epoxy-1H-isoindol-1,3(2H)-dion;
3a,4,7,7a-Tetrahydro-4-methyl-2-(4-methyl-3-nitrophenyl)-4,7-epoxy-1H-isoindol-1,3(2H)-dion;
2-[2-Chlor-5-(trifluormethyl)phenyl]-3a,4,7,7a-tetrahydro-4-methyl-4,7-epoxy-1H-isoindol-1,3(2H)-dion;
2-[4-Chlor-3-(trifluormethyl)phenyl]-3a,4,7,7a-tetrahydro-4,7-dimethyl-4,7-epoxy-1H-isoindol-1,3(2H)-dion;
2-(1,3,3a,4,7,7a-Hexahydro-4-methyl-4,7-epoxy-2H-isoindol-2-yl)benzonitril;
2-(4-Fluorphenyl)-3a,4,7,7a-tetrahydro-4-methyl-4,7-epoxy-1H-isoindol-1,3(2H)-dion;
2,2,2-Trifluor-N-[(1,2,3,3a,7,7a-hexahydro-2-phenyl-4,7-epoxy-4H-isoindol-4-yl)methyl]acetamid;
3a,4,7,7a-Tetrahydro-4,7-dimethyl-2-(4-methyl-3-nitrophenyl)-4,7-epoxy-1H-isoindol-1,3(2H)-dion;
2-Chlor-5-[1,3,3a,4,7,7a-hexahydro-4-(hydroxymethyl)-4,7-epoxy-2H-isoindol-2-yl]benzoesäure;
3a,4,7,7a-Tetrahydro-4,7-dimethyl-2-(4-nitrophenyl)-4,7-epoxy-1H-isoindol-1,3(2H)-dion;
3a,4,7,7a-Tetrahydro-2-(2-mercaptophenyl)-4,7-epoxy-1H-isoindol-1,3(2H)-dion;
3a,4,7,7a-Tetrahydro-2-[2-[(phenylmethyl)thio]phenyl]-4,7-epoxy-1H-isoindol-1,3(2H)-dion;

[[2-(4-Chlorphenyl)-1,2,3,3a,7,7a-hexahydro-4,7-epoxy-4H-isoindol-4-yl]methyl]carbaminsäure-2-methylpropylester;
4-(1,1-Dimethylethyl)-N-[[1,2,3,3a,7,7a-hexahydro-2-(4-methylphenyl)-4,7-epoxy-4H-isoindol-4 yl]methyl]benzamid;
2,4-Dichlor-N-[[1,2,3,3a,7,7a-hexahydro-2-(4-nitrophenyl)-4,7-epoxy-4H-isoindol-4-yl]methyl]benzamid;
N-[[2-(4-Chlorphenyl)-1,2,3,3a,7,7a-hexahydro-4,7-epoxy-4H-isoindol-4-yl]methyl]-2,4,6-trimethylbenzolsulfonamid;
[(1,2,3,3a,7,7a-Hexahydro-2-phenyl-4,7-epoxy-4H-isoindol-4-yl)methyl]carbaminsäure-1,1-dimethylethylester;
N-[(1,2,3,3a,7,7a-Hexahydro-2-phenyl-4,7-epoxy-4H-isoindol-4-yl)methyl]-2-phenoxyacetamid;
N-[[1,2,3,3a,7,7a-Hexahydro-2-(4-nitrophenyl)-4,7-epoxy-4H-isoindol-4-yl]methyl]-2,2-dimethylpropanamid;
2-(2,4-Dichlorphenoxy)-N-[[1,2,3,3a,7,7a-hexahydro-2-(4-nitrophenyl)-4,7-epoxy-4H-isoindol-4-yl]methyl]acetamid;
N-[[1,2,3,3a,7,7a-Hexahydro-2-(4-methylphenyl)-4,7-epoxy-4H-isoindol-4-yl]methyl]-3,5-dimethoxybenzamid;
N-[[2-(4-Chlorphenyl)-1,2,3,3a,7,7a-hexahydro-4,7-epoxy-4H-isoindol-4-yl]methyl]-2-nitrobenzolsulfonamid;
(3aα,4β,7β,7aα)-Hexahydro-2-[(1S)-1-phenylethyl]-4,7-epoxy-1H-isoindol-1,3(2H)-dion;
(3aα,4β,7β,7aα)-Hexahydro-2-[(1S)-2-hydroxy-1-phenylethyl]-4,7-epoxy-1H-isoindol-1,3(2H)-dion;
(3aα,4β,7β,7aα)-2-[(1S)-2-(Acetyloxy)-1-phenylethyl]-3a,4,7,7a-tetrahydro-4,7-epoxy-1H-isoindol-1,3(2H)-dion;
(3aα,4α,7α,7aα)-3a,4,7,7a-Tetrahydro-2-[(1S)-1-phenylethyl]-4,7-epoxy-1H-isoindol-1,3(2H)-dion;
(3aα,4β,7β,7aα)-Hexahydro-2-[(1R)-1-phenylethyl]-4,7-epoxy-1H-isoindol-1,3(2H)-dion;
(3aα,4β,7β,7aα)-4-[[(Octahydro-1,3-dioxo-4,7-epoxy-2H-isoindol-2-yl)methyl]amino]benzoesäure;
(3aα,4β,7β,7aα)-Hexahydro-2-(4-morpholinylmethyl)-4,7-epoxy-1H-isoindol-1,3(2H)-dion;
(3aα,4β,7β,7aα)-4-[Octahydro-4-(2-hydroxyethyl)-7-methyl-1,3-dioxo-4,7-epoxy-2H-isoindol-2-yl]-2-(trifluormethyl)benzonitril;
(3aα,4β,7β,7aα)- und (3aα,4α,7α,7aα)-4-[Octahydro-4-methyl-1,3-dioxo-7-(phenylmethyl)-4,7-epoxy-2H-isoindol-2-yl]-2-(trifluormethyl)benzonitril;
(3aα,4β,7β,7aα)-4-[7-[2-(4-Bromphenoxy)ethyl]octahydro-4-methyl-1,3-dioxo-4,7-epoxy-2H-isoindol-2-yl]-2-(trifluormethyl)benzonitril;
(3aα,4β,7β,7aα)-4-[Octahydro-7-[2-(4-iodphenoxy)ethyl]-4-methyl-1,3-dioxo-4,7-epoxy-2H-isoindol-2-yl]-2-(trifluormethyl)benzonitril;
(3aα,4β,7β,7aα)-4-[Octahydro-4-methyl-1,3-dioxo-7-[2-[4-(trifluormethyl)phenoxy]ethyl]-4,7-epoxy-2H-isoindol-2-yl]-2-(trifluormethyl)benzonitril;
(3aα,4β,7β,7aα)-4-[Octahydro-7-[2-(4-methoxyphenoxy)ethyl]-4-methyl-1,3-dioxo-4,7-epoxy-2H-isoindol-2-yl]-2-(trifluormethyl)benzonitril;
(3aα,4β,7β,7aα)-4-[7-[2-(4-Ethoxyphenoxy)ethyl]octahydro-4-methyl-1,3-dioxo-4,7-epoxy-2H-isoindol-2-yl]-2-(trifluormethyl)benzonitril;
(3aα,4β,7β,7aα)-4-[7-[2-(4-Chlorphenoxy)ethyl]octahydro-4-methyl-1,3-dioxo-4,7-epoxy-2H-isoindol-2-yl]-2-(trifluormethyl)benzonitril;
(3aα,4β,7β,7aα)-4-[2-[2-[4-Cyano-3-(trifluormethyl)phenyl]octahydro-7-methyl-1,3-dioxo-4,7-epoxy-4H-isoindol-4-yl]ethoxy]benzoesäure-methylester;
(3aα,4β,7β,7aα)-Hexahydro-4-(2-hydroxyethyl)-7-methyl-2-(3-methyl-4-nitrophenyl)-4,7-epoxy-1H-isoindol-1,3(2H)-dion;
(3aα, 4β, 7β, 7aα)- 4-[Octahydro- 4-methyl- 1,3-dioxo- 7-[2-[4-(trifluormethoxy) phenoxy] ethyl]- 4,7-epoxy-2H-isoindol-2-yl]-2-(trifluormethyl)benzonitril;
(3aα,4β,7β,7aα)-2-(3,5-Dichlorphenyl)hexahydro-4,7-dimethyl-4,7-epoxy-1H-isoindol-1,3(2H)-dion;
(3aα,4β,7β,7aα)-Hexahydro-4,7-dimethyl-2-(4-nitro-1-naphthalenyl)-4,7-epoxy-1H-isoindol-1,3(2H)-dion;
(3aα,4β,7β,7aα)-2-[4-Cyano-3-(trifluormethyl)phenyl]octahydro-7-methyl-1,3-dioxo-4,7-epoxy-4H-isoindol-4-propannitril;
(3aα,4β,7β,7aα)-4-[Octahydro-4-methyl-7-[2-(4-morpholinyl)ethyl]-1,3-dioxo-4,7-epoxy-2H-isoindol-2-yl]-2-(trifluormethyl)benzonitril-trifluoracetat;
(3aα,4β,7β,7aα)-2-(5-Fluor-1-naphthalenyl)hexahydro-4,7-dimethyl-4,7-epoxy-1H-isoindol-1,3(2H)-dion;
(3aα,4β,7β,7aα)-2-(5-Fluor-4-nitro-1-naphthalenyl)hexahydro-4,7-dimethyl-4,7-epoxy-1H-isoindol-1,3(2H)-dion;
(3aα,4β,7β,7aα)-2-(1,1-Dioxidobenzo[b]thiophen-3-yl)hexahydro-4,7-dimethyl-4,7-epoxy-1H-isoindol-1,3(2H)-dion;
4-(1,3,3a,4,7,7a-Hexahydro-4,6,7-trimethyl-1,3-dioxo-4,7-epoxy-2H-pyrrolo[3,4-c]pyridin-2-yl)-2-(trifluormethyl)benzonitril;

(3aα,4β,7β,7aα)-Tetrahydro-4,7-dimethyl-2-[3-(trifluormethyl)phenyl]-4,7-epoxy-1H-isoindol-1,3,5(2H,4H)-trion;

(3aα,4α,7α,7aα)-Tetrahydro-4,7-dimethyl-2-[3-(trifluormethyl)phenyl]-4,7-epoxy-1H-isoindol-1,3,5(2H,4H)-trion;

(3aα,4β,7β,7aα)-2-(5-Chlor-1-naphthalenyl)hexahydro-4,7-dimethyl-4,7-epoxy-1H-isoindol-1,3(2H)-dion;

(3aα,4β,7β,7aα)-2-(5-Chlor-4-nitro-1-naphthalenyl)hexahydro-4,7-dimethyl-4,7-epoxy-1H-isoindol-1,3(2H)-dion;

(3aα,4β,7β,7aα)-4-Ethylhexahydro-7-methyl-2-(4-nitro-1-naphthalenyl)-4,7-epoxy-1H-isoindol-1,3(2H)-dion;

(3aα, 4β, 7β, 7aα)- 2-(4-Cyano- 1-naphthalenyl) -N-(4-fluorphenyl) octahydro- 7-methyl- 1,3-dioxo- 4,7-epoxy-4H-isoindol-4-acetamid;

(3aα,4β,7β,7aα)-Hexahydro-4-methyl-2-(2-naphthalenyl)-4,7-epoxy-1H-isoindol-1,3(2H)-dion, schneller eluierendes Enantiomer;

(3aα,4β,7β,7aα)-Hexahydro-4-methyl-2-(2-naphthalenyl)-4,7-epoxy-1H-isoindol-1,3(2H)-dion, langsamer eluierendes Enantiomer;

(3aα,4β,7β,7aα)-4-[4-[2-[[(4-Fluorphenyl)methyl]methylamino]ethyl]octahydro-7-methyl-1,3-dioxo-4,7-epoxy-2H-isoindol-2-yl]-2-(trifluormethyl)benzonitril;

(3aα,4β,5β,6β,7β,7aα)-4-(Octahydro-4,5,6,7-tetramethyl-1,3-dioxo-4,7-epoxy-2H-isoindol-2-yl)-2-(trifluormethyl)benzonitril;

(3aα,4β,7β,7aα)-4-[Octahydro-4-methyl-1,3-dioxo-7-[2-[4-(trifluormethyl)phenoxy]ethyl]-4,7-epoxy-2H-isoindol-2-yl]-2-(trifluormethyl)benzonitril, schneller eluierendes Antipod;

(3aα, 4β, 5β, 7β, 7aα)- 4-[Octahydro- 4-methyl- 1,3-dioxo- 7-[2-[4-(trifluormethyl) phenoxy] ethyl]- 4,7-epoxy-2H-isoindol-2-yl]-2-(trifluormethyl)benzonitril, langsamer eluierendes Enantiomer;

(3aα,4β,5α,7β,7aα)-4-(Octahydro-5-hydroxy-4,7-dimethyl-1,3-dioxo-4,7-epoxy-2H-isoindol-2-yl)-2-(trifluormethyl)benzonitril;

(3aα, 4β, 7β, 7aα)- 4-(Octahydro- 5-hydroxy- 4,7-dimethyl- 1,3-dioxo- 4,7-epoxy- 2H-isoindol- 2-yl)- 2-(trifluormethyl)benzonitril;

(αR)- α-Methoxybenzolessigsäure, 2-[(3aα,4β,7β,7aα)-2-(4-cyano-1-naphthalenyl)octahydro-7-methyl-1,3-dioxo-4,7-epoxy-4H-isoindol-4-y]ethylester;

(3aα,4β,7β,7aα)-2-(Methylthio)-4-(octahydro-4,7-dimethyl-1,3-dioxo-4,7-epoxy-2H-isoindol-2-yl)benzonitril;

(3aα,4β,7β,7aα)-2-(Methylsulfinyl)-4-(octahydro-4,7-dimethyl-1,3-dioxo-4,7-epoxy-2H-isoindol-2-yl)benzonitril;

(3aα,4β,7β,7aα)-2-(Methylsulfonyl)-4-(octahydro-4,7-dimethyl-1,3-dioxo-4,7-epoxy-2H-isoindol-2-yl)benzonitril;

(3aα,4β,5β,7β,7aα)-7-[2-[[(1,1-Dimethylethyl)dimethylsilyl]oxy]ethyl]hexahydro-5-hydroxy-4-methyl-2-(4-nitro-1-naphthalenyl)-4,7-epoxy-1H-isoindol-1,3(2H)-dion;

(3aα,4β,5β,7β,7aα)-Hexahydro-5-hydroxy-7-(2-hydroxyethyl)-4-methyl-2-(4-nitro-1-naphthalenyl)-4,7-epoxy-1H-isoindol-1,3(2H)-dion;

(3aα, 4β, 5β, 7β, 7aα)- 7-[2-(4-Fluorphenoxy) ethyl] hexahydro- 5-hydroxy- 4-methyl- 2-(4-nitro- 1-naphthalenyl)-4,7-epoxy-1H-isoindol-1,3(2H)-dion;

(3aα, 4β, 5β, 6β, 7β, 7aα)- 4-(Octahydro- 5,6-dihydroxy- 4,7-dimethyl- 1,3-dioxo- 4,7-epoxy- 2H-isoindol- 2-yl)-2-(trifluormethyl)benzonitril;

(3aα, 4β, 5α, 6α, 7β, 7aα)- 4-(Octahydro- 5,6-dihydroxy- 4,7-dimethyl- 1,3-dioxo- 4,7-epoxy- 2H-isoindol- 2-yl)-2-(trifluormethyl)benzonitril;

(3aα,4β,5β,6β,7β,7aα)-4-[Octahydro-5,6-dihydroxy-4-(hydroxyethyl)-7-methyl-1,3-dioxo-4,7-epoxy-2H-isoindol-2-yl]-1-naphthalincarbonitril;

(3aα,4β,5β,6β,7β,7aα)-4-[Octahydro-5,6-dihydroxy-4-methyl-1,3-dioxo-7-[2-[4-(trifluormethyl)phenoxy] ethyl]-4,7-epoxy-2H-isoindol-2-yl]-2-(trifluormethyl)benzonitril;

(3aα,4β,5β,5aβ,8aβ, 8bα)-4-(Decahydro-5-hydroxy-4-methyl-1,3-dioxo-4,8a-epoxy-2H-furo[3,2-e]isoindol-2-yl)-1-naphthalincarbonitril;

(3aα, 4β, 7β, 7aα)- 2-(4-Cyano- 1-naphthalenyl) octahydro- 7-methyl- 1,3-dioxo- 4,7-epoxy- 4H-isoindol- 4-essigsäure;

(3aα, 4β, 7β, 7aα)- 2-(4-Cyano- 1-naphthalenyl) octahydro- 7-methyl- 1,3-dioxo- 4,7-epoxy- 4H-isoindol- 4-essigsäure-methylester;

(3aα,4β,7β,7aα)-2-(4-Cyano-1-naphthalenyl)-N-[(4-fluorphenyl)methyl]octahydro-7-methyl-1,3-dioxo-4,7-epoxy-4H-isoindol-4-acetamid;

(3aα,4β,7β,7aα)-N-[2-[2-(4-Cyano-1-naphthalenyl)octahydro-7-methyl-1,3-dioxo-4,7-epoxy-4H-isoindol-4-yl] ethyl]-4-fluorbenzamid;

[3aR-(3aα, 4β, 7β, 7aα)]- 4-[Octahydro- 4-(2-hydroxyethyl)- 7-methyl- 1,3-dioxo- 4,7-epoxy- 2H-isoindol- 2-yl]-1-naphthalincarbonitril;
[3aS-(3aα, 4β, 7β, 7aα)]- 4-[Octahydro- 4-(2-hydroxyethyl)- 7-methyl- 1,3-dioxo- 4,7-epoxy- 2H-isoindol- 2-yl]-1-naphthalincarbonitril;
[3aR-(3aα, 4β, 7β, 7aα)]- 4-[4-[2-(3-Fluorphenoxy) ethyl] octahydro- 7-methyl- 1,3-dioxo- 4,7-epoxy- 2H-isoindol-2-yl]-1-naphthalincarbonitril;
[3aS-(3aα, 4β, 7β, 7aα)]- 4-[4-[2-(3-Fluorphenoxy) ethyl] octahydro- 7-methyl- 1,3-dioxo- 4,7-epoxy- 2H-isoindol-2-yl]-1-naphthalincarbonitril;
(4-Fluorphenyl)carbaminsäure, 2-[(3aα,4β,7β,7aα)-2-(4-cyano-1-naphthalenyl)octahydro-7-methyl-1,3-dioxo-4,7-epoxy-4H-isoindol-4-yl]ethylester;
(3aα,4β,7β,7aα)-4-[Octahydro-4-(2-hydroxyethyl)-1,3-dioxo-4,7-epoxy-2H-isoindol-2-yl]-1-naphthalincarbonitril;
(3aα, 4β, 6β, 7β, 7aα)- 4-[4-[2-(4-Cyanophenoxy) ethyl] octahydro- 6-hydroxy- 1,3-dioxo- 4,7-epoxy- 2H-isoindol-2-yl]-1-naphthalincarbonitril;
[3aS-(3aα,4β,5β,7β,7aα)]-4-[Octahydro-5-hydroxy-7-(2-hydroxyethyl)-4-methyl-1,3-dioxo-4,7-epoxy-2H-isoindol-2-yl]-1-naphthalincarbonitril;
[3aR-(3aα, 4β, 5β, 7β, 7aα)]- 4-[Octahydro- 5-hydroxy- 7-(2-hydroxyethyl)- 4-methyl- 1,3-dioxo- 4,7-epoxy-2H-isoindol-2-yl]-1-naphthalincarbonitril;
(3aα, 4β, 7β, 7aα)- 4-[4-[2-(4-Cyanophenoxy) ethyl]- 7-ethyloctahydro- 1,3-dioxo- 4,7-epoxy- 2H-isoindol- 2-yl]-1-naphthalincarbonitril;
(3aα, 4β, 7β, 7aα)- 4-[2-(Acetyloxy) ethyl]- 2-(4-cyano- 1-naphthalenyl) hexahydro- 7-methyl- 4,7-epoxy- 1H-isoindol-1,3(2H)-dion;
(3aα, 4β, 7β, 7aα)- 4-[Octahydro- 4-methyl- 1,3-dioxo- 7-(2-oxoethyl)- 4,7-epoxy- 2H-isoindol- 2-yl]- 1-naphthalincarbonitril;
[3aα,4β(E),7β,7aα]-4-[4-[3-(4-Cyanophenyl)-2-propenyl]octahydro-7-methyl-1,3-dioxo-4,7-epoxy-2H-isoindol-2-yl]-1-naphthalincarbonitril;
[3aα,4β(Z),7β,7aα]-4-[4-[3-(4-Cyanophenyl)-2-propenyl]octahydro-7-methyl-1,3-dioxo-4,7-epoxy-2H-isoindol-2-yl]-1-naphthalincarbonitril;
(3aα, 4β, 7β, 7aα)- 4-[4-[3-(4-Cyanophenyl) propyl] octahydro- 7-methyl- 1,3-dioxo- 4,7-epoxy- 2H-isoindol- 2-yl]-1-naphthalincarbonitril;
(3aα, 4β, 7β, 7aα)- 4-[4-[2-[(6-Chlor- 1,2-benzisoxazol- 3-yl) oxy] ethyl] octahydro- 7-methyl- 1,3-dioxo- 4,7-epoxy-2H-isoindol-2-yl]-1-naphthalincarbonitril;
(3aα, 4β, 7β, 7aα)- 4-[Octahydro- 4-methyl- 7-[2-[(6-nitro- 1H-indazol- 3-yl) oxy] ethyl]- 1,3-dioxo- 4,7-epoxy-2H-isoindol-2-yl]-1-naphthalincarbonitril;
[3aS-(3aα, 4β, 5β, 7β, 7aα)]- 4-[7-[2-(1,2-Benzisoxazol- 3-yloxy) ethyl] octahydro- 5-hydroxy- 4-methyl- 1,3-dioxo-4,7-epoxy-2H-isoindol-2-yl]-1-naphthalincarbonitril;
[3aR-(3aα, 4β, 5β, 7β, 7aα)]- 4-[7-[2-(1,2-Benzisoxazol- 3-yloxy) ethyl] octahydro- 5-hydroxy- 4-methyl- 1,3-dioxo-4,7-epoxy-2H-isoindol-2-yl]-1-naphthalincarbonitril;
(3aα,4β,5β,7β,7aα)-4-(Octahydro-5-hydroxy-4,7-dimethyl-1,3-dioxo-4,7-epoxy-2H-isoindol-2-yl)-2-(trifluormethyl)benzonitril;
(3aα,4β,5β,7β,7aα)-4-(Octahydro-5-hydroxy-4,7-dimethyl-1,3-dioxo-4,7-epoxy-2H-isoindol-2-yl)-2-(trifluormethyl)benzonitril;
(3aα, 4β, 7β, 7aα)- 2-(4-Cyano- 1-naphthalenyl) octahydro- 1,3-dioxo- 7-[2-(phenylmethoxy) ethyl]- 4,7-epoxy-4H-isoindol-4-propannitril;
(3aα, 4α, 7α, 7aα)- 2-(4-Cyano- 1-naphthalenyl) octahydro- 1,3-dioxo- 7-[2-(phenylmethoxy) ethyl]- 4,7-epoxy-4H-isoindol-4-propannitril;
(3aα, 4β, 7β, 7aα)- 2-(4-Cyano- 1-naphthalenyl) octahydro- 7-(2-hydroxyethyl)- 1,3-dioxo- 4,7-epoxy- 4H-isoindol-4-propannitril;
(3aα, 4α, 7α, 7aα)- 2-(4-Cyano- 1-naphthalenyl) octahydro- 7-(2-hydroxyethyl)- 1,3-dioxo- 4,7-epoxy- 4H-isoindol-4-propannitril;
(3aα, 4β, 7β, 7aα)- 2-(4-Cyano- 1-naphthalenyl)- 7-[2-(4-fluorphenoxy) ethyl] octahydro- 1,3-dioxo- 4,7-epoxy-4H-isoindol-4-propannitril;
(3aα,4β,7β,7aα)-2-(7-Chlor-2,1,3-benzoxadiazol-4-yl)hexahydro-4,7-dimethyl-4,7-epoxy-1H-isoindol-1,3(2H)-dion;
(3aα,4β,7β,7aα)-2-(7-Chlor-2-methyl-4-benzofuranyl)hexahydro-4,7-dimethyl-4,7-epoxy-1H-isoindol-1,3(2H)-dion;
(3aα, 4β, 7β, 7aα)- 2-(7-Chlor- 2-methylbenzo [b] thiophen- 4-yl) hexahydro- 4,7-dimethyl- 4,7-epoxy- 1H-isoindol-1,3(2H)-dion;
[3aα,4β(E),7β,7aα]-4-[2-(4-Cyano-1-naphthalenyl)octahydro-7-methyl-1,3-dioxo-4,7-epoxy-4H-isoindol-4-yl]-2-butensäure-phenylmethylester;
(3aα, 4β, 7β, 7aα)-2-(4-Cyano-1-naphthalenyl) octahydro-7-methyl-1,3-dioxo-4,7-epoxy-4H-isoindol-4-butansäure;
(3aα, 4β, 7β, 7aα)-2-(4-Cyano-1-naphthalenyl) -N-(4-fluorphenyl) octahydro-7-methyl-1,3-dioxo-4,7-epoxy-4H-isoindol-4-butanamid;
[3aS-(3aα, 4β, 5β, 7β, 7aα)]-4-[7-[2-(Acetyloxy) ethyl] octahydro-5-hydroxy-4-methyl-1,3-dioxo-4,7-epoxy-2H-isoindol-2-yl]-1-naphthalincarbonitril;
[3aR-(3aα, 4β, 5β, 7β, 7aα)]-4-[Octahydro-5-hydroxy-7-(2-hydroxyethyl)-4-methyl-1,3-dioxo-4,7-epoxy-2H-isoindol-2-yl]-1-naphthalincarbonitril;
[3aα, 4β, 7β, 7aα (E)]-4-[Octahydro-4-methyl-1,3-dioxo-7-(4-oxo-4-phenyl-2-butenyl)-4,7-epoxy-2H-isoindol-2-yl]-1-naphthalincarbonitril;
[3aα,4β,7β,7aα(E)]-4-[Octahydro-4-methyl-1,3-dioxo-7-(4-oxo-4-phenyl-butanyl)-4,7-epoxy-2H-isoindol-2-yl]-1-naphthalincarbonitril;
(3aα, 4β, 7β, 7aα)-4-[7-[2-(4-Bromphenoxy) ethyl] octahydro-4-methyl-1,3-dioxo-4,7-epoxy-2H-isoindol-2-yl]-2-(trifluormethyl)benzonitril;
(3aα, 4β, 7β, 7aα)-4-[Octahydro-7-[2-(4-iodphenoxy) ethyl]-4-methyl-1,3-dioxo-4,7-epoxy-2H-isoindol-2-yl]-2-(trifluormethyl)benzonitril;
(3aα, 4β, 7β, 7aα)-4-[Octahydro-4-methyl-1,3-dioxo-7-[2-[4-(trifluormethyl) phenoxy] ethyl]-4,7-epoxy-2H-isoindol-2-yl]-2-(trifluormethyl)benzonitril;
(3aα, 4β, 7β, 7aα)-4-[Octahydro-7-[2-(4-methoxyphenoxy) ethyl]-4-methyl-1,3-dioxo-4,7-epoxy-2H-isoindol-2-yl]-2-(trifluormethyl)benzonitril;
(3aα, 4β, 7β, 7aα)-4-[7-[2-(4-Ethoxyphenoxy) ethyl] octahydro-4-methyl-1,3-dioxo-4,7-epoxy-2H-isoindol-2-yl]-2-(trifluormethyl)benzonitril;
(3aα, 4β, 7β, 7aα)-4-[7-[2-(4-Chlorphenoxy) ethyl] octahydro-4-methyl-1,3-dioxo-4,7-epoxy-2H-isoindol-2-yl]-2-(trifluormethyl)benzonitril;
(3aα,4β,7β,7aα)-4-[2-[2-[4-Cyano-3-(trifluormethyl)phenyl]octahydro-7-methyl-1,3-dioxo-4,7-epoxy-4H-isoindol-4-yl]ethoxy]benzoesäure-methylester;
(3aα, 4β, 7β, 7aα) -Hexahydro-4-(2-hydroxyethyl)-7-methyl-2-(3-methyl-4-nitrophenyl)-4,7-epoxy-1H-isoindol-1,3(2H)-dion;
(3aα, 4β, 7β, 7aα)-4-[Octahydro-4-methyl-1,3-dioxo-7-[2-[4-(trifluormethoxy) phenoxy] ethyl]-4,7-epoxy-2H-isoindol-2-yl]-2-(trifluormethyl)benzonitril;
(3aα,4β,7β,7aα)-2-(3,5-Dichlorphenyl)hexahydro-4,7-dimethyl-4,7-epoxy-1H-isoindol-1,3(2H)-dion;
(3aα,4β,7β,7aα)-Hexahydro-4,7-dimethyl-2-(4-nitro-1-naphthalenyl)-4,7-epoxy-1H-isoindol-1,3(2H)-dion;
(3aα, 4β, 7β, 7aα)-4-[Octahydro-4-methyl-1,3-dioxo-7-[2-[4-(phenylmethoxy) phenoxy] ethyl]-4,7-epoxy-2H-isoindol-2-yl]-2-(trifluormethyl)benzonitril;
(3aα, 4β, 7β, 7aα) -Hexahydro-4-(2-hydroxyethyl)-7-methyl-2-(4-nitro-1-naphthalenyl)-4,7-epoxy-1H-isoindol-1,3(2H)-dion;
(3aα, 4β, 7β, 7aα)-4-[2-(4-Fluorphenoxy) ethyl] hexahydro-7-methyl-2-(3-methyl-4-nitrophenyl)-4,7-epoxy-1H-isoindol-1,3(2H)-dion;
(3aα, 4β, 7β, 7aα)-4-[Octahydro-4-methyl-1,3-dioxo-7-[2-[4-[(trifluormethyl) thio] phenoxy] ethyl]-4,7-epoxy-2H-isoindol-2-yl]-2-(trifluormethyl)benzonitril;
(3aα, 4β, 7β, 7aα)-4-[Octahydro-4-methyl-7-[2-(4-nitrophenoxy) ethyl]-1,3-dioxo-4,7-epoxy-2H-isoindol-2-yl]-2-(trifluormethyl)benzonitril;
(3aα, 4β, 7β, 7aα)-4-[2-(4-Fluorphenoxy) ethyl] hexahydro-7-methyl-2-(4-nitro-1-naphthalenyl)-4,7-epoxy-1H-isoindol-1,3(2H)-dion;
(3aα, 4β, 7β, 7aα)-4-[Octahydro-7-methyl-1,3-dioxo-7-[2-[2-(trifluormethyl) phenoxy] ethyl]-4,7-epoxy-2H-isoindol-2-yl]-2-(trifluormethyl)benzonitril;
(3aα, 4β, 7β, 7aα)-4-[4-[2-(2-Bromphenoxy) ethyl] octahydro-7-methyl-1,3-dioxo-4,7-epoxy-2H-isoindol-2-yl]-2-(trifluormethyl)benzonitril;
(3aα, 4β, 7β, 7aα)-4-[4-[2-(3-Fluorphenoxy) ethyl] octahydro-7-methyl-1,3-dioxo-4,7-epoxy-2H-isoindol-2-yl]-2-(trifluormethyl)benzonitril;
(3aα,4β,7β,7aα)-Hexahydro-2-[4-(1H-imidazol-1-yl)phenyl]-4-methyl-4,7-epoxy-1H-isoindol-1,3(2H)-dion;
(3aα,4β,7β,7aα)-2-[3-Chlor-4-(2-thiazolyl)phenyl]hexahydro-4-methyl-4,7-epoxy-1H-isoindol-1,3(2H)-dion;
(3aα,4β,7β,7aα)-Hexahydro-4,7-dimethyl-2-(3-methyl-4-nitrophenyl)-4,7-epoxy-1H-isoindol-1,3(2H)-dion;
(3aα,4β,7β,7aα)-Hexahydro-4,7-dimethyl-2-(2-methyl-4-nitrophenyl)-4,7-epoxy-1H-isoindol-1,3(2H)-dion;
(3aα,4β,7β,7aα)-2-(3,5-Dichlorphenyl)hexahydro-4-(2-hydroxyethyl)-7-methyl-4,7-epoxy-1H-isoindol-1,3(2H)-dion;
(3aα,4β,7β,7aα)-2-(3,5-Dichlorphenyl)-4-[2-(4-fluorphenoxy)ethyl]hexahydro-7-methyl-4,7-epoxy-1H-isoindol-1,3(2H)-dion;
(3aα,4β,7β,7aα)-4-[Octahydro-4-[2-(4-hydroxyphenoxy)ethyl]-7-methyl-1,3-dioxo-4,7-epoxy-2H-isoindol-2-yl]-2-(trifluormethyl)benzonitril;
(3aα,4β,7β,7aα)-4-[4-[2-(4-Cyanophenoxy) ethyl] octahydro-7-methyl-1,3-dioxo-4,7-epoxy-2H-isoindol-2-yl]-2-(trifluormethyl)benzonitril;
(3aα,4β,7β,7aα)-4-[Octahydro-4-methyl-1,3-dioxo-7-[2-[3-(trifluormethyl) phenoxy] ethyl]-4,7-epoxy-2H-isoindol-2-yl]-2-(trifluormethyl)benzonitril;
(3aα, 4β, 7β, 7aα)-4-[4-[2-(3-Bromphenoxy) ethyl] octahydro-7-methyl-1,3-dioxo-4,7-epoxy-2H-isoindol-2-yl]-2-(trifluormethyl)benzonitril;
(3aα, 4β, 7β, 7aα)-4-[4-[(4-Fluorphenyl) methyl] octahydro-7-methyl-1,3-dioxo-4,7-epoxy-2H-isoindol-2-yl]-2-(trifluormethyl)benzonitril;
(3aα,4β,7β,7aα)-2-(1,6-Dihydro-1-methyl-6-oxo-3-pyridinyl) hexahydro-4,7-dimethyl-4,7-epoxy-1H-isoindol-1,3(2H)-dion;
(3aα,4β,7β,7aα)-Hexahydro-4,7-dimethyl-2-(1-methyl-6-oxo-3-piperidinyl)-4,7-epoxy-1H-isoindol-1,3(2H)-dion;
(3aα,4β,7β,7aα)-4-[4-[2-(3-Cyanophenoxy) ethyl] octahydro-7-methyl-1,3-dioxo-4,7-epoxy-2H-isoindol-2-yl]-2-(trifluormethyl)benzonitril;
(3aα,4β,7β,7aα)-4-[2-[4-Cyano-3-(trifluormethyl)phenyl]octahydro-7-methyl-1,3-dioxo-4,7-epoxy-4H-isoindol-4-yl]ethoxy]benzoesäure-phenylmethylester;
(3aα,4β,7β,7aα)-4-[Octahydro-4-methyl-1,3-dioxo-7-(2-phenoxyethyl)-4,7-epoxy-2H-isoindol-2-yl]-2-(trifluormethyl)benzonitril;
(3aα,4β,7β,7aα)-2-(3,5-Dichlor-4-nitrophenyl)hexahydro-4,7-dimethyl-4,7-epoxy-1H-isoindol-1,3(2H)-dion;
(3aα,4β,7β,7aα)-2-(3,5-Dichlor-4-hydroxyphenyl) hexahydro-4,7-dimethyl-4,7-epoxy-1H-isoindol-1,3(2H)-dion;
(3aα,4β,7β,7aα)-2-(5-Fluor-1-naphthalenyl)hexahydro-4,7-dimethyl-4,7-epoxy-1H-isoindol-1,3(2H)-dion;
(3aα,4β,7β,7aα)-Hexahydro-4,7-dimethyl-2-(1-naphthalenyl)-4,7-epoxy-1H-isoindol-1,3(2H)-dion;
(3aα,4β,7β,7aα)-Hexahydro-2-[3-methoxy-4-(5-oxazolyl) phenyl]-4,7-dimethyl-4,7-epoxy-1H-isoindol-1,3(2H)-dion;
(3aα,4β,7β,7aα)-Hexahydro-4-[2-(4-methoxyphenoxy) ethyl]-7-methyl-2-(4-nitro-1-naphthalenyl)-4,7-epoxy-1H-isoindol-1,3(2H)-dion;
(3aα, 4β, 7β, 7aα) -Hexahydro-4-methyl-2-(4-nitro-1-naphthalenyl)-7-[2-[4-(trifluormethyl) phenoxy] ethyl]-4,7-epoxy-1H-isoindol-1,3(2H)-dion;
(3aα, 4β, 7β, 7aα) -Hexahydro-4-methyl-2-(4-nitro-1-naphthalenyl)-7-[2-(4-nitrophenoxy) ethyl]-4,7-epoxy-1H-isoindol-1,3(2H)-dion;
(3aα,4β,7β,7aα)-2-(1,6-Dihydro-1,4-dimethyl-6-oxo-3-pyridinyl) hexahydro-4,7-dimethyl-4,7-epoxy-1H-isoindol-1,3(2H)-dion;
(3aα, 4β, 7β, 7aα)-4-[Octahydro-7-methyl-2-(4-nitro-1-naphthalenyl)-1,3-dioxo-4,7-epoxy-4H-isoindol-4-yl] ethoxy]benzonitril;
(3aα,4β,7β,7aα)-4-(Octahydro-4,7-dimethyl-1,3-dioxo-4,7-epoxy-2H-isoindol-2-yl)-1,2-benzoldicarbonitril;
(3aα,4β,7β,7aα)-4-(2-Bromethyl) hexahydro-7-methyl-2-(4-nitro-1-naphthalenyl)-4,7-epoxy-1H-isoindol-1,3(2H)-dion;
(3aα,4β,7β,7aα)-4-[4-[2-(4-Cyanophenoxy) ethyl] octahydro-7-methyl-1,3-dioxo-4,7-epoxy-2H-isoindol-2-yl]-1-naphthalincarbonitril;
(3aα, 4β, 7β, 7aα)-4-[Octahydro-4-[2-(4-methoxyphenoxy) ethyl]-7-methyl-1,3-dioxo-4,7-epoxy-2H-isoindol-2-yl]-1-naphthalincarbonitril;
(3aα, 4β, 7β, 7aα)-4-[Octahydro-4-[2-(3-methoxyphenoxy) ethyl]-7-methyl-1,3-dioxo-4,7-epoxy-2H-isoindol-2-yl]-1-naphthalincarbonitril;
(3aα, 4β, 7β, 7aα)-4-[4-[2-(3-Fluorphenoxy) ethyl] octahydro-7-methyl-1,3-dioxo-4,7-epoxy-2H-isoindol-2-yl]-1-naphthalincarbonitril;
(3aα,4β,7β,7aα)-4-[Octahydro-4-methyl-7-[2-[3-(4-morpholinyl)phenoxy]ethyl]-1,3-dioxo-4,7-epoxy-2H-isoindol-2-yl]-1-naphthalincarbonitril;
(3aα,4β,7β,7aα)-4-[Octahydro-4-methyl-7-[2-[4-nitro-3-(trifluormethyl) phenoxy] ethyl]-1,3-dioxo-4,7-epoxy-2H-isoindol-2-yl]-1-naphthalincarbonitril;
(3aα,4β,7β,7aα)-4-[4-[2-(3-Cyanophenoxy) ethyl] octahydro-7-methyl-1,3-dioxo-4,7-epoxy-2H-isoindol-2-yl]-1-naphthalincarbonitril;
(3aα, 4β, 7β, 7aα)-2-(2,3-Dihydro-3-methyl-2-oxo-6-benzothiazolyl) hexahydro-4,7-dimethyl-4,7-epoxy-1H-isoindol-1,3(2H)-dion;
(3aα,4β,7β,7aα)-2-(2,3-Dihydro-2-oxo-6-benzothiazolyl) hexahydro-4,7-dimethyl-4,7-epoxy-1H-isoindol-1,3(2H)-dion;
(3aα,4β,7β,7aα)-4-[4-[2-[3-(Dimethylamino)phenoxy]ethyl]octahydro-7-methyl-1,3-dioxo-4,7-epoxy-2H-isoindol-2-yl]-1-naphthalincarbonitril;
(3aα,4β,7β,7aα)-4-[2-[4-Cyano-3-(trifluormethyl)phenyl]octahydro-7-methyl-1,3-dioxo-4,7-epoxy-4H-isoindol-4-yl]ethoxy]-1,2-benzoldicarbonitril;
(3aα, 4β, 7β, 7aα) -N-[2-Cyano- 5-(octahydro- 4,7-dimethyl- 1,3-dioxo- 4,7-epoxy- 2H-isoindol- 2-yl) phenyl] acetamid;
(3aα,4β,7β,7aα)-4-(Octahydro-4,7-dimethyl-1,3-dioxo-4,7-epoxy-2H-isoindol-2-yl)-2-(trifluormethoxy)benzonitril;
(3aα,4β,7β,7aα)-2-Methoxy-4-(octahydro-4,7-dimethyl-1,3-dioxo-4,7-epoxy-2H-isoindol-2-yl)benzonitril;
(3aα,4β,7β,7aα)-2-[4-(4,5-Dichlor-1H-imidazol-1-yl)phenyl]hexahydro-4,7-dimethyl-4,7-epoxy-1H-isoindol-1,3(2H)-dion;
(3aα,4β,7β,7aα)-2-[4-(4-Brom-1-methyl-1H-pyrazol-3-yl)phenyl]hexahydro-4,7-dimethyl-4,7-epoxy-1H-isoindol-1,3(2H)-dion;
(3aα, 4β, 7β, 7aα)- 4-[Octahydro- 4-(2-hydroxyethyl)- 7-methyl- 1,3-dioxo- 4,7-epoxy- 2H-isoindol- 2-yl]- 1-naphthalincarbonitril;
(3aα,4β,7β,7aα)-2-Iod-4-(Octahydro-4,7-dimethyl-1,3-dioxo-4,7-epoxy-2H-isoindol-2-yl)benzonitril;
(3aα, 4β, 7β, 7aα)- 4-[4-[2-(4-Fluorphenoxy) ethyl] octahydro- 7-methyl- 1,3-dioxo- 4,7-epoxy- 2H-isoindol- 2-yl]-1-naphthalincarbonitril;
(3aα,4β,7β,7aα)-4-[Octahydro-4-methyl-1,3-dioxo-7-[2-[4-(trifluormethyl)phenoxy]ethyl]-4,7-epoxy-2H-isoindol-2-yl]-1-naphthalincarbonitril;
(3aα,4β,7β,7aα)-4-[4-[2-(4-Cyano-3-fluorphenoxy)ethyl]octahydro-7-methyl-1,3-dioxo-4,7-epoxy-2H-isoindol-2-yl]-1-naphthalincarbonitril;
(3aα, 4β, 7β, 7aα)- 4-[Octahydro- 4-methyl- 1,3-dioxo- 7-[2-[2,3,5,6-tetrafluor- 4-(trifluormethyl) phenoxy] ethyl]-4,7-epoxy-2H-isoindol-2-yl]-1-naphthalincarbonitril;
(3aα,4β,7β,7aα)-Hexahydro-4,7-dimethyl-2-[4-(1H-1,2,4-triazol-3-yl)phenyl]-4,7-epoxy-1H-isoindol-1,3(2H)-dion;
(3aα, 4β, 7β, 7aα)- 2-[4-(4,5-Dihydro- 5-oxo- 1,2,4-oxadiazol- 3-yl) phenyl] hexahydro- 4,7-dimethyl- 4,7-epoxy-1H-isoindol-1,3(2H)-dion;
(3aα,4β,7β,7aα)-Hexahydro-2-[3-methoxy-4-(2-oxazolyl)phenyl]-4,7-dimethyl-4,7-epoxy-1H-isoindol-1,3(2H)-dion;
(3aα,4β,7β,7aα)-Hexahydro-2-(4-hydroxy-1-naphthalenyl)-4,7-dimethyl-4,7-epoxy-1H-isoindol-1,3(2H)-dion;
(3aα, 4β, 7β, 7aα) -Hexahydro- 2-(8-hydroxy- 5-chinolinyl)- 4,7-dimethyl- 4,7-epoxy- 1H-isoindol- 1,3 (2H) -dion-trifluoracetat;
(3aα, 4β, 7β, 7aα)- 4-[Octahydro- 4-methyl- 1,3-dioxo- 7-[2-[methyl (phenylmethyl) amino] ethyl]- 4,7-epoxy-2H-isoindol-2-yl]-2-(trifluormethyl)benzonitril;
(3aα,4β,7β,7aα)-Hexahydro-4,7-dimethyl-2-(5-chinolinyl)-4,7-epoxy-1H-isoindol-1,3(2H)-dion;
(3aα,4β,7β,7aα)-5-(Octahydro-4,7-dimethyl-1,3-dioxo-4,7-epoxy-2H-isoindol-2-yl)-2-pyridincarbonitril;
(3aα,4β,7β,7aα)-5-(Octahydro-4,7-dimethyl-1,3-dioxo-4,7-epoxy-2H-isoindol-2-yl)-8-chinolincarbonitril;
(3aα,4β,7β,7aα)-2-(5-Brom-4-nitro-1-naphthalenyl)hexahydro-4,7-dimethyl-4,7-epoxy-1H-isoindol-1,3(2H)-dion;
(3aα,4β,7β,7aα)-2-(5-Brom-1-naphthalenyl)hexahydro-4,7-dimethyl-4,7-epoxy-1H-isoindol-1,3(2H)-dion;
(3aα,4β,7β,7aα)-Hexahydro-4,7-dimethyl-2-[8-(trifluormethyl)-4-chinolinyl]-4,7-epoxy-1H-isoindol-1,3(2H)-dion;
4-Fluorbenzoesäure, 2-[(3aα,4β,7β,7aα)-2-(4-cyano-1-naphthalenyl)octahydro-7-methyl-1,3-dioxo-4,7-epoxy-4H-isoindol-4-yl]ethylester;
Benzolessigsäure, 2-[(3aα,4β,7β,7aα)-2-(4-cyano-1-naphthalenyl)octahydro-7-methyl-1,3-dioxo-4,7-epoxy-4H-isoindol-4-yl]ethylester;
4-Fluorbenzolessigsäure, 2-[(3aα,4β,7β,7aα)-2-(4-cyano-1-naphthalenyl)octahydro-7-methyl-1,3-dioxo-4,7-epoxy-4H-isoindol-4-yl]ethylester;
(3aα, 4β, 7β, 7aα) -Hexahydro- 4-methyl- 7-[2-[4-(methylsulfonyl) phenoxy] ethyl]- 2-(4-nitro- 1-naphthalenyl)-4,7-epoxy-1H-isoindol-1,3(2H)-dion;
(3aα,4β,7β,7aα)-Hexahydro-2-(2-naphthalenyl)-4,7-dimethyl-4,7-epoxy-1H-isoindol-1,3(2H)-dion;
(3aα,4β,7β,7aα)-2-(4-Chlor-1-naphthalenyl)hexahydro-4,7-dimethyl-4,7-epoxy-1H-isoindol-1,3(2H)-dion;
(3aα, 4β, 7β, 7aα) -N-[(4-Chlorphenyl) methyl]- 2-(4-cyano- 1-naphthalenyl) octahydro- 7-methyl- 1,3-dioxo-4,7-epoxy-4H-isoindol-4-acetamid;
4,7,7-Trimethyl-3-oxo-2-oxabicyclo[2.2.1]heptan-1-carbonsäure-2-[(3aα,4β,7β,7aα)-2-(4-cyano-1-naphthalenyl)octahydro-7-methyl-1,3-dioxo-4,7-epoxy-4H-isoindol-4-yl]ethylester;

(αS)- α-Methoxy-α-(trifluormethyl)benzolessigsäure-2-[(3aα,4β,7β,7aα)-2-(4-cyano-1-naphthalenyl)octahydro-7-methyl-1,3-dioxo-4,7-epoxy-4H-isoindol-4-yl]ethylester;
(αR)- α-Methoxy-α-(trifluormethyl)benzolessigsäure-2-[(3aα,4β,7β,7aα)-2-(4-cyano-1-naphthalenyl)octahydro-7-methyl-1,3-dioxo-4,7-epoxy-4H-isoindol-4-yl]ethylester;
(3aα,4β,7β,7aα)-4-[Octahydro-4-methyl-7-[2-[(7-methyl-1,2-benzisoxazol-3-yl)oxy]ethyl]-1,3-dioxo-4,7-epoxy-2H-isoindol-2-yl]-1-naphthalincarbonitril;
(3aα,4β,7β,7aα)-4-[4-[2-(1,2-Benzisoxazol-3-yloxy)ethyl]octahydro-7-methyl-1,3-dioxo-4,7-epoxy-2H-isoindol-2-yl]-1-naphthalincarbonitril;
(3aα,4β,7β,7aα)-4-[2-(Benzoyloxy)ethyl]-2-(4-cyano-1-naphthalenyl)hexahydro-7-methyl-4,7-epoxy-1H-isoindol-1,3(2H)-dion;
(3aα,4β,7β,7aα)-2-(4-Cyano-1-naphthalenyl)-4-[2-[(4-nitrobenzoyl)oxy]ethyl]hexahydro-7-methyl-4,7-epoxy-1H-isoindol-1,3(2H)-dion;
4-Chlorbenzoesäure, 2-[(3aα,4β,7β,7aα)-2-(4-cyano-1-naphthalenyl)octahydro-7-methyl-1,3-dioxo-4,7-epoxy-4H-isoindol-4-yl]ethylester;
[3aα,4β,7β,7aα(E)]-4-[Octahydro-4-methyl-7-[3-(1-naphthalenyl)-2-propenyl]-1,3-dioxo-4,7-epoxy-2H-isoindol-2-yl]-1-naphthalincarbonitril;
(3aα,4β,7β,7aα)-4-[Octahydro-4-methyl-7-[3-(1-naphthalenyl)propyl]-1,3-dioxo-4,7-epoxy-2H-isoindol-2-yl]-1-naphthalincarbonitril;
(3aα,4β,7β,7aα)-Hexahydro-4,7-dimethyl-2-(2-methyl-6-chinolinyl)-4,7-epoxy-1H-isoindol-1,3(2H)-dion;
(3aα,4β,7β,7aα)-Hexahydro-2-(5-isochinolinyl)-4,7-dimethyl-4,7-epoxy-1H-isoindol-1,3(2H)-dion;
(3aα,4β,7β,7aα)-2-(6-Benzothiazolyl)hexahydro-4,7-dimethyl-4,7-epoxy-1H-isoindol-1,3(2H)-dion;
[3aα,4β,7β,7aα(E)]-4-[Octahydro-4-methyl-1,3-dioxo-7-(4-oxo-4-phenyl-2-butenyl)-4,7-epoxy-2H-isoindol-2-yl]-1-naphthalincarbonitril;
(3aα,4β,7β,7aα)-2-(4-Cyano-1-naphthalenyl)octahydro-N-(2-hydroxyphenyl)-7-methyl-1,3-dioxo-4,7-epoxy-4H-isoindol-4-acetamid;
[3aα, 4β (E), 7β, 7aα]-4-[Octahydro-4-methyl-7-[3-(6-methyl-2-pyridinyl)-2-propenyl]-1,3-dioxo-4,7-epoxy-2H-isoindol-2-yl]-1-naphthalincarbonitril;
(3aα,4β,7β,7aα)-4-[Octahydro-4-methyl-7-[3-(6-methyl-2-pyridinyl)propyl]-1,3-dioxo-4,7-epoxy-2H-isoindol-2-yl]-1-naphthalincarbonitril;
[3aR-(3aα,4β,7β,7aα)]-4-[Octahydro-4-[2-(3-methoxyphenoxy)ethyl]-7-methyl-1,3-dioxo-4,7-epoxy-2H-isoindol-2-yl]-1-naphthalincarbonitril;
[3aS-(3aα,4β,7β,7aα)]-4-[Octahydro-4-[2-(3-methoxyphenoxy)ethyl]-7-methyl-1,3-dioxo-4,7-epoxy-2H-isoindol-2-yl]-1-naphthalincarbonitril;
[3aR-(3aα,4β,7β,7aα)]-4-[4-[2-(4-Cyanophenoxy)ethyl]octahydro-7-methyl-1,3-dioxo-4,7-epoxy-2H-isoindol-2-yl]-1-naphthalincarbonitril;
[3aS-(3aα,4β,7β,7aα)]-4-[4-[2-(4-Cyanophenoxy)ethyl]octahydro-7-methyl-1,3-dioxo-4,7-epoxy-2H-isoindol-2-yl]-1-naphthalincarbonitril;
(3aα, 4α, 7α, 7aα)-4-[4-[(4-Fluorphenyl) methyl] octahydro-7-methyl-1,3-dioxo-4,7-epoxy-2H-isoindol-2-yl]-2-(trifluormethyl)benzonitril;
(3aα,4α,7α,7aα)-Hexahydro-4,7-dimethyl-2-(1-methyl-6-oxo-3-piperidinyl)-4,7-epoxy-1H-isoindol-1,3(2H)-dion;
(3aα,4α,7α,7aα)-2-(1,6-Dihydro-1,4-dimethyl-6-oxo-3-pyridinyl)hexahydro-4,7-dimethyl-4,7-epoxy-1H-isoindol-1,3(2H)-dion;
(3aα, 4β, 7β, 7aα)-2-[4-Cyano-3-(trifluormethyl) phenyl] octahydro-1,3-dioxo-7-[2-(phenylmethoxy) ethyl]-4,7-epoxy-4H-isoindol-4-propannitril;
(3aα, 4β, 7β, 7aα)-2-[4-Cyano-3-(trifluormethyl) phenyl] octahydro-1,3-dioxo-7-[2-(phenylmethoxy) ethyl]-4,7-epoxy-4H-isoindol-4-propannitril;
(3aα,4β,7β,7aα)-4-[7-[2-(4-Cyanophenoxy)ethyl]octahydro-5-hydroxy-4-methyl-1,3-dioxo-4,7-epoxy-2H-isoindol-2-yl]-1-naphthalincarbonitril;
[3aS-(3aα, 4β, 7β, 7aα)]-4-[7-[2-(1,3-Benzodioxol-5-yloxy) ethyl] octahydro-5-hydroxy-4-methyl-1,3-dioxo-4,7-epoxy-2H-isoindol-2-yl]-1-naphthalincarbonitril;
[3aR-(3aα, 4β, 7β, 7aα)]-4-[7-[2-(1,3-Benzodioxol-5-yloxy) ethyl] octahydro-5-hydroxy-4-methyl-1,3-dioxo-4,7-epoxy-2H-isoindol-2-yl]-1-naphthalincarbonitril;
[3aS-(3aα, 4β, 7β, 7aα)]-4-[7-[2-[(5-Chlor-2-pyridinyl) oxy] ethyl] octahydro-5-hydroxy-4-methyl-1,3-dioxo-4,7-epoxy-2H-isoindol-2-yl]-1-naphthalincarbonitril;
[3aR-(3aα, 4β, 7β, 7aα)]-4-[7-[2-[(5-Chlor-2-pyridinyl) oxy] ethyl] octahydro-5-hydroxy-4-methyl-1,3-dioxo-4,7-epoxy-2H-isoindol-2-yl]-1-naphthalincarbonitril;
[3aS-(3aα, 4β, 7β, 7aα)]-4-[7-[2-(4-Chlorphenoxy) ethyl] octahydro-5-hydroxy-4-methyl-1,3-dioxo-4,7-epoxy-2H-isoindol-2-yl]-1-naphthalincarbonitril;
[3aR-(3aα, 4β, 7β, 7aα)]- 4-[7-[2-(4-Chlorphenoxy) ethyl] octahydro- 5-hydroxy- 4-methyl- 1,3-dioxo- 4,7-epoxy-2H-isoindol-2-yl]-1-naphthalincarbonitril;
[3aS-(3aα, 4β, 7β, 7aα)]- 4-[7-[2-(4-Acetylphenoxy) ethyl] octahydro- 5-hydroxy- 4-methyl- 1,3-dioxo- 4,7-epoxy-2H-isoindol-2-yl]-1-naphthalincarbonitril;
[3aR-(3aα, 4β, 7β, 7aα)]- 4-[7-[2-(4-Acetylphenoxy) ethyl] octahydro- 5-hydroxy- 4-methyl- 1,3-dioxo- 4,7-epoxy-2H-isoindol-2-yl]-1-naphthalincarbonitril;
[3aS-(3aα, 4β, 7β, 7aα)]- 4-[7-[2-(3-Cyanophenoxy) ethyl] octahydro- 5-hydroxy- 4-methyl- 1,3-dioxo- 4,7-epoxy-2H-isoindol-2-yl]-1-naphthalincarbonitril;
[3aR-(3aα, 4β, 7β, 7aα)]- 4-[7-[2-(3-Cyanophenoxy) ethyl] octahydro- 5-hydroxy- 4-methyl- 1,3-dioxo- 4,7-epoxy-2H-isoindol-2-yl]-1-naphthalincarbonitril;
[3aS-(3aα,4β,7β,7aα)]-4-[Octahydro-5-hydroxy-4-methyl-1,3-dioxo-7-[2-[(5,6,7,8-tetrahydro-1-naphthalenyl) oxy]ethyl]-4,7-epoxy-2H-isoindol-2-yl]-1-naphthalincarbonitril;
[3aR-(3aα,4β,7β,7aα)]-4-[Octahydro-5-hydroxy-4-methyl-1,3-dioxo-7-[2-[(5,6,7,8-tetrahydro-1-naphthalenyl) oxy]ethyl]-4,7-epoxy-2H-isoindol-2-yl]-1-naphthalincarbonitril;
[3aS-(3aα, 4β, 7β, 7aα)]- 4-[Octahydro- 5-hydroxy- 4-methyl- 1,3-dioxo- 7-[2-[(5,6,7,8-tetrahydro- 5-oxo- 1-naphthalenyl)oxy]ethyl]-4,7-epoxy-2H-isoindol-2-yl]-1-naphthalincarbonitril;
[3aR-(3aα, 4β, 7β, 7aα)]- 4-[Octahydro- 5-hydroxy- 4-methyl- 1,3-dioxo- 7-[2-[(5,6,7,8-tetrahydro- 5-oxo- 1-naphthalenyl)oxy]ethyl]-4,7-epoxy-2H-isoindol-2-yl]-1-naphthalincarbonitril;
[3aS-(3aα, 4β, 7β, 7aα)]- 4-[7-[2-(4-Fluorphenoxy) ethyl] octahydro- 5-hydroxy- 4-methyl- 1,3-dioxo- 4,7-epoxy-2H-isoindol-2-yl]-1-naphthalincarbonitril;
[3aR-(3aα, 4β, 7β, 7aα)]- 4-[7-[2-(4-Fluorphenoxy) ethyl] octahydro- 5-hydroxy- 4-methyl- 1,3-dioxo- 4,7-epoxy-2H-isoindol-2-yl]-1-naphthalincarbonitril;
[3aS-(3aα, 4β, 7β, 7aα)]- 4-[Octahydro- 5-hydroxy- 4-methyl- 7-[2-[(4-methyl- 2-oxo- 2H- 1-benzopyran- 7-yl) oxy] ethyl]-1,3-dioxo-4,7-epoxy-2H-isoindol-2-yl]-1-naphthalincarbonitril;
[3aR-(3aα, 4β, 7β, 7aα)]- 4-[Octahydro- 5-hydroxy- 4-methyl- 7-[2-[(4-methyl- 2-oxo- 2H- 1-benzopyran- 7-yl) oxy] ethyl]-1,3-dioxo-4,7-epoxy-2H-isoindol-2-yl]-1-naphthalincarbonitril;
[3aS-(3aα, 4β, 7β, 7aα)]- 4-[7-[2-(3,5-Dimethoxyphenoxy) ethyl] octahydro- 5-hydroxy- 4-methyl- 1,3-dioxo-4,7-epoxy-2H-isoindol-2-yl]-1-naphthalincarbonitril;
[3aR-(3aα, 4β, 7β, 7aα)]- 4-[7-[2-(3,5-Dimethoxyphenoxy) ethyl] octahydro- 5-hydroxy- 4-methyl- 1,3-dioxo-4,7-epoxy-2H-isoindol-2-yl]-1-naphthalincarbonitril;
[3aR-(3aα, 4β, 7β, 7aα)]- 4-[7-[2-(4-Chlor- 3-methylphenoxy) ethyl] octahydro- 5-hydroxy- 4-methyl- 1,3-dioxo-4,7-epoxy-2H-isoindol-2-yl]-1-naphthalincarbonitril;
[3aR-(3aα, 4β, 7β, 7aα)]- 4-[7-[2-(4-Cyano- 2,3-difluorphenoxy) ethyl] octahydro- 5-hydroxy- 4-methyl- 1,3-dioxo-4,7-epoxy-2H-isoindol-2-yl]-1-naphthalincarbonitril;
[3aS-(3aα, 4β, 7β, 7aα)]- 4-[7-[2-[(5-Chlor- 1,2-benzisoxazol- 3-yl) oxy] ethyl] octahydro- 5-hydroxy- 4-methyl-1,3-dioxo-4,7-epoxy-2H-isoindol-2-yl]-1-naphthalincarbonitril;
[3aR-(3aα, 4β, 7β, 7aα)]- 4-[7-[2-[(5-Chlor- 1,2-benzisoxazol- 3-yl) oxy] ethyl] octahydro- 5-hydroxy- 4-methyl-1,3-dioxo-4,7-epoxy-2H-isoindol-2-yl]-1-naphthalincarbonitril;
[3aR-(3aα,4β,7β,7aα)]-3-[2-[2-(4-Cyano-1-naphthalenyl)octahydro-6-hydroxy-7-methyl-1,3-dioxo-4,7-epoxy-4H-isoindol-4-yl]ethoxy]-5-isoxazolecarbonsäure-methylester;
[3aR-(3aα, 4β, 7β, 7aα)]- 4-[Octahydro- 5-hydroxy- 4-methyl- 1,3-dioxo- 7-[2-[4-(1H- 1,2,4-triazol- 1-yl) phenoxy] ethyl]-4,7-epoxy-2H-isoindol-2-yl]-1-naphthalincarbonitril;
[3aS-(3aα, 4β, 7β, 7aα)]- 4-[7-[2-[(7-Chlor- 4-chinolinyl) oxy] ethyl] octahydro- 5-hydroxy- 4-methyl- 1,3-dioxo-4,7-epoxy-2H-isoindol-2-yl]-1-naphthalincarbonitril, Trifluoracetat;
[3aR-(3aα, 4β, 7β, 7aα)]- 4-[7-[2-[(7-Chlor- 4-chinolinyl) oxy] ethyl) octahydro- 5-hydroxy- 4-methyl- 1,3-dioxo-4,7-epoxy-2H-isoindol-2-yl]-1-naphthalincarbonitril, Trifluoracetat;
(1aα,2β,2aα,5aα,6βb,6aα)-4-[2-[2-[[(1,1-Dimethylethyl)dimethylsilyl]oxy]ethyl]octahydro-6-methyl-3,5-dioxo-2,6-epoxy-4H-oxireno[f]isoindol-4-yl]-1-naphthalincarbonitril;
[3aR-(3aα,4β,7β,7aα)]-4-[4-Ethyloctahydro-7-(2-hydroxyethyl)-1,3-dioxo-4,7-epoxy-2H-isoindol-2-yl]-1-naphthalincarbonitril;
[3aS-(3aα,4β,7β,7aα)]-4-[4-Ethyloctahydro-7-(2-hydroxyethyl)-1,3-dioxo-4,7-epoxy-2H-isoindol-2-yl]-1-naphthalincarbonitril;
[3aR-(3aα, 4β, 7β, 7aα)]- 4-[4-[2-(4-Cyanophenoxy) ethyl]- 7-ethyloctahydro- 1,3-dioxo- 4,7-epoxy- 2H-isoindol-2-yl]-1-naphthalincarbonitril; und
[3aS-(3aα, 4β, 7β, 7aα)]- 4-[4-[2-(4-Cyanophenoxy) ethyl]- 7-ethyloctahydro- 1,3-dioxo- 4,7-epoxy- 2H-isoindol-2-yl]-1-naphthalincarbonitril.

4. Verbindung nach Anspruch 3, bei der es sich um:

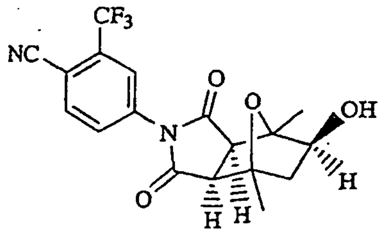

oder

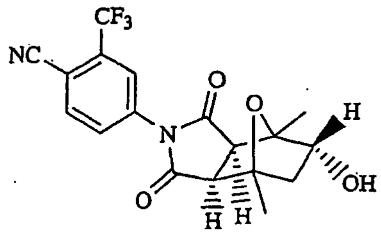

handelt, oder ein Salz oder Hydrat davon.

5. Arzneimittel, das zur Behandlung eines mit einem nuklearen Hormonrezeptor verbundenen Zustandes in der Lage ist, umfassend eine Verbindung der Formel I wie in Anspruch 1 definiert, mit der Maßgabe (10) nach Anspruch 1 oder ein pharmazeutisch verträgliches Salz oder Hydrat davon und einen pharmazeutisch verträglichen Träger.

6. Arzneimittel umfassend eine Verbindung nach Anspruch 1, 2, 3 oder 4.

7. Arzneimittel enthaltend eine Verbindung nach Anspruch 4.

8. Arzneimittel nach Anspruch 5, 6 oder 7, welches ferner ein anderes Mittel gegen Krebs umfasst.

9. Verwendung einer Verbindung wie in Anspruch 1, 2, 3 oder 4 definiert zur Herstellung eines Medikaments zur Modulation der Funktion eines nuklearen Hormonrezeptors.

10. Verwendung nach Anspruch 9, wobei der nukleare Hormonrezeptor ausgewählt ist aus einem Steroid-bindenden nuklearen Hormonrezeptor wie in Anspruch 1, 2, 3 oder 4 definiert, dem Androgen-Rezeptor, dem Östrogen-Rezeptor, dem Progesteron-Rezeptor, dem Glucocorticoid-Rezeptor, dem Mineralcorticoid-Rezeptor und dem Aldosteron-Rezeptor.

11. Verwendung einer Verbindung wie in Anspruch 1, 2, 3 oder 4 definiert zur Herstellung eines Medikaments zur Behandlung eines Zustandes oder einer Störung,
wobei der Zustand oder die Störung ausgewählt ist aus proliferierenden Erkrankungen, Krebs, gutartiger Prostatahypertrophie, Adenomen und Neoplasien der Prostata, gutartigen oder bösartigen Tumorzellen, die den Androgen-Rezeptor enthalten, Herzerkrankung, angiogenen Zuständen oder Störungen, Hirsutismus, Akne, Hyperpilosität, Entzündung, Immunmodulation, Seborrhoe, Endometriose, polyzystischem Ovarsyndrom, androgener Alopecia, Hypogonadismus, Osteoporose, unterdrückter Spermatogenese, Libido, Kachexie, Anorexia, Hemmung der muskulären Atrophie bei gehfähigen Patienten, Androgen-Supplementierung bei altersabhängigem vermindertem Testosteronspiegel bei Männern, den Östrogen-Rezeptor exprimierende Krebsarten, Prostatakrebs, Brustkrebs, Endometriumkrebs, Hitzewallungen, Scheidentrockenheit, Menopause, Amenorrhoe, Dysmenorrhoe, Empfängnisverhütung, Schwangerschaftsabbruch, Krebsarten, die den Progesteron-Rezeptor enthalten, Endometriose, gleichmäßigem Zyklus, Meningioma, Fibrose, Weheneinleitung, Autoimmunerkrankungen, Alzheimer-Krankheit, psychotischen Störungen, Drogen-/Medikamentenabhängigkeit, nicht-insulinabhängigem Diabetes Mellitus, Dopaminrezeptor-vermittelten Störungen, dekompensierter Herzinsuffizienz, Dysregulierung der Cholesterin-Homöostase und Herabsetzen des Stoffwechsels eines Arzneimittels.

12. Verfahren zur Herstellung einer Verbindung der folgenden Formel XVI oder eines Salzes davon:

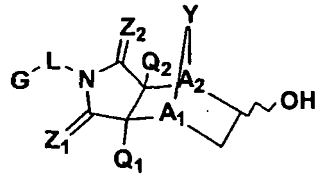

XVI

wobei G, L, $Z_1$, $Z_2$, $Q_1$, $Q_2$, $A_1$, $A_2$ und Y wie in Anspruch 1 definiert sind, umfassend die Schritte des In-Kontakt-Bringens einer Verbindung der folgenden Formel XV oder eines Salzes davon:

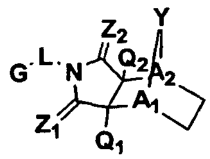

XV

wobei die Symbole wie vorstehend definiert sind, mit einem Enzym oder Mikroorganismus, das/der in der Lage ist, die Hydroxylierung der Verbindung XV zu der Verbindung XVI zu katalysieren, und des Durchführens der Hydroxylierung.

**13.** Verfahren zur Herstellung einer Verbindung der folgenden Formel XVIII oder eines Salzes davon:

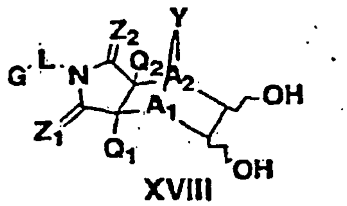

XVIII

wobei G, L, $Z_1$, $Z_2$, $Q_1$, $Q_2$, $A_1$, $A_2$ und Y wie in Anspruch 3 definiert sind, umfassend die Schritte des In-Kontakt-Bringens einer Verbindung der folgenden Formel XVII oder eines Salzes davon:

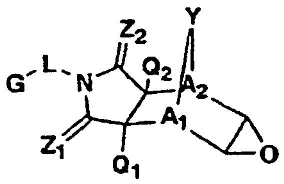

XVII

wobei die Symbole wie vorstehend definiert sind, mit einem Enzym oder Mikroorganismus, das/der in der Lage ist, die Öffnung des Epoxidrings der Verbindung XVII zu katalysieren, um das Diol der Verbindung XVIII zu bilden, und des Durchführens der Ringöffnung und der Diolbildung.

## Revendications

**1.** Composé répondant à la formule (I) suivante ou sel ou hydrate de celui-ci :

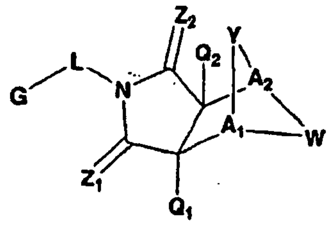

(I)

dans lequel les symboles ont les significations suivantes et sont, pour chaque occurrence, indépendamment choisis :

G représente un groupe aryle ou hétérocyclo, où ledit groupe est mono- ou polycyclique, et qui est éventuellement substitué au niveau d'une ou plusieurs positions ;
L représente une liaison, un groupe $(CR^7R^{7'})_n$ (où n est égal à 1 et $R^7$ et $R^{7'}$ représentent chacun indépendamment un atome de H, un groupe alkyle ou alkyle substitué), ou un groupe $-CH_2$-NH- ;
$A_1$ et $A_2$ représentent chacun indépendamment un groupe $CR^7$ où $R^7$ (i) représente un atome d'hydrogène, un groupe alkyle ou alkyle substitué, arylalkyle ou arylalkyle substitué, alcényle ou alcényle substitué, aryle ou aryle substitué, hétérocyclo ou hétérocyclo substitué, hétérocycloalkyle ou hétérocycloalkyle substitué, où chaque substituant représente un ou plusieurs groupes choisis parmi $V^1$, ou (ii) forme, avec le groupe $R^7$ d'un groupe W, un noyau hétérocyclique ;
$V^1$ représente un groupe OH, CN, halogéno, -O-aryle, -O-aryle substitué, -O-hétérocyclo, -O-hétérocyclo substitué, -O-CO-alkyle, -O-CO-alkyle substitué, -O-(alkylsilyle), -O-arylalkyle, -O-arylalkyle substitué, -O-CO-arylalkyle, -O-CO-arylalkyle substitué, -O-CO-aryle, -O-CO-aryle substitué, -O-CO-hétérocyclo, -O-CO-hétérocyclo substitué, -S-(aryle éventuellement substitué)-NH-CO-(alkyle éventuellement substitué), -SO-(aryle éventuellement substitué)-NH-CO-(alkyle éventuellement substitué), $-SO_2$-(aryle éventuellement substitué)-NH-CO-(alkyle éventuellement substitué), $-NH-SO_2$-aryle, $-NH-SO_2$-aryle substitué, -NH-CO-O-(arylalkyle éventuellement substitué), -NH-CO-O-alkyle, -NH-CO-O-alkyle substitué, -NH-CO-alkyle, -NH-CO-alkyle substitué, -NH-CO-aryle, -NH-CO-aryle substitué, -NH-CO-(arylalkyle éventuellement substitué), -NH-CO-(alkyle éventuellement substitué)-O-(aryle éventuellement substitué), -N(alkyle éventuellement substitué)(aryle éventuellement substitué), -N(alkyle éventuellement substitué)(arylalkyle éventuellement substitué), -COH, -COOH, -CO-O-alkyle, -CO-O-alkyle substitué, -CO-O-arylalkyle éventuellement substitué, -CO-aryle, -CO-aryle substitué, -O-CO-NH-aryle, -O-CO-NH-aryle substitué, -CO-NH-aryle, -CO-NH-aryle substitué, -CO-NH-arylalkyle, -CO-NH-arylalkyle substitué, ou -O-(aryle éventuellement substitué)-NH-CO-(alkyle éventuellement substitué) ;
Y représente un groupe -O-, -SO-, $-N(V^2)$-, $-CH_2-N(V^2)$-, -CO-N(alkyle)-, $-CH_2$-S-, ou $-CH_2-SO_2$- ;
$V^2$ représente un atome d'hydrogène, un groupe alkyle, arylaikyle, -CO-alkyle, -CO-O-aryle, ou -CO-O-arylalkyle ;
$Z_1$ et $Z_2$ représentent un atome de O ;
$Q_1$ et $Q_2$ représentent un atome de H ;
W représente un groupe $CR^7R^{7'}-CR^7R^{7'}$, $CR^7R^{7'}$-C=O, $NR^9$- $CR^7R^{7'}$, $N=CR^8$, N=N, $NR^{9}$-$NR^{9'}$, cycloalkyle ou cycloalkyle substitué, cycloalcényle ou cycloalcényle substitué, hétérocyclo ou hétérocyclo substitué, ou aryle ou aryle substitué, dans lequel, quand W ne représente pas un groupe $NR^9$- $CR^7R^{7'}$, $N=CR^8$, N=N, $NR^9-NR^{9'}$, ou un groupe hétérocyclo ou hétérocyclo substitué, alors Y doit représenter un groupe -O-, $-CH_2$-S-, -SO-, $-CH_2-SO_2$-, $N(V^2)$- ou $-CH_2-N(V^2)$,
$R^1$ et $R^{1'}$ représentent chacun indépendamment un atome de H, un groupe alkyle ou alkyle substitué, cycloalkyle ou cycloalkyle substitué, cycloalcényle ou cycloalcényle substitué, hétérocyclo ou hétérocyclo substitué, cycloalkylalkyle ou cycloalkylalkyle substitué, cycloalcénylalkyle ou cycloalcénylalkyle substitué, hétérocyctoalkyle ou hétérocycloalkyle substitué, aryle ou aryle substitué, arylalkyle ou arylalkyle substitué ;
$R^2$ représente un groupe alkyle ou alkyle substitué, cycloalkyle ou cycloalkyle substitué, cycloalcényle ou cycloalcényle substitué, hétérocyclo ou hétérocyclo substitué, cycloalkylalkyle ou cycloalkylalkyle substitué, cycloalcénylalkyle ou cycloalcénylalkyle substitué, hétérocycloalkyle ou hétérocycloalkyle substitué, aryle ou aryle substitué, arylalkyle ou arylalkyle substitué ;

$R^4$ représente un atome de H, un groupe alkyle ou alkyle substitué, cycloalkyle ou cycloalkyle substitué, cycloalcényle ou cycloalcényle substitué, hétérocyclo ou hétérocyclo substitué, cycloalkylalkyle ou cycloalkylalkyle substitué, cycloalcénylalkyle ou cycloalcénylalkyle substitué, hétérocycloalkyle ou hétérocycloalkyle substitué, aryle ou aryle substitué, arylalkyle ou arylalkyle substitué, $R^1C=O$, $R^1NHC=O$, $SO_2OR^1$, ou $SO_2NR^1R^{1'}$ ;
$R^5$ représente un groupe alkyle ou alkyle substitué, cycloalkyle ou cycloalkyle substitué, cycloalcényle ou cycloalcényle substitué, hétérocyclo ou hétérocyclo substitué, cycloalkylalkyle ou cycloalkylalkyle substitué, cycloalcénylalkyle ou cycloalcénylalkyle substitué, hétérocycloalkyle ou hétérocycloalkyle substitué, aryle ou aryle substitué, arylalkyle ou arylalkyle substitué, $R^1C=O$, $R^1NHC=O$, $SO_2R^1$, $SO_2OR^1$, ou $SO_2NR^1R^{1'}$ ;
$R^7$ et $R^{7'}$ représentent chacun indépendamment un atome de H, un groupe alkyle ou alkyle substitué, alcényle ou alcényle substitué, cycloalkyle ou cycloalkyle substitué, cycloalcényle ou cycloalcényle substitué, hétérocyclo ou hétérocyclo substitué, cycloalkylalkyle ou cycloalkylalkyle substitué, cycloalcénylalkyle ou cycloalcénylalkyle substitué, hétérocycloalkyle ou hétérocycloalkyle substitué, aryle ou aryle substitué, arylalkyle ou arylalkyle substitué, halogéno, CN, $OR^1$, nitro, hydroxylamine, hydroxylamide, amino, $NHR^4$, $NR^2R^5$, $NOR^1$, thiol, alkylthio ou alkylthio substitué, $R^1C=O$, $R^1OC=O$, $R^1NHC=O$, $SO_2R^1$, $SOR^1$, $PO_3R^1R^{1'}$, $R^1R^{1'}NC=O$, $C=OSR^1$, $SO_2R^1$, $SO_2OR^1$, ou $SO_2NR^1R^{1'}$, ou dans lequel $A_1$ ou $A_2$ contient un groupe $R^7$ et W contient un groupe $R^7$, lesdits groupes $R^7$ de $A_1$ ou $A_2$ et W forment ensemble un noyau hétérocyclique ;
$R^8$ représente un atome de H, un groupe alkyle ou alkyle substitué, alcényle ou alcényle substitué, cycloalkyle ou cycloalkyle substitué, cycloalcényle ou cycloalcényle substitué, hétérocyclo ou hétérocyclo substitué, cycloalkylalkyle ou cycloalkylalkyle substitué, cycloalcénylalkyle ou cycloalcénylalkyle substitué, hétérocycloalkyle ou hétérocycloalkyle substitué, aryle ou aryle substitué, arylalkyle ou arytalkyle substitué, nitro, halogéno, CN, $OR^1$, amino, $NHR^4$, $NR^2R^5$, $NOR^1$, alkylthio ou alkylthio substitué, $C=OSR^1$, $R^1OC=O$, $R^1C=O$, $R^1NHC=O$, $R^1R^{1'}NC=O$, $SO_2OR^1$, $S=OR^1$, $SO_2R^1$, $PO_3R^1R^{1'}$, ou $SO_2NR^1R^{1'}$ ; et
$R^9$ et $R^{9'}$ représentent chacun indépendamment un atome de H, un groupe alkyle ou alkyle substitué, alcényle ou alcényle substitué, cycloalkyle ou cycloalkyle substitué, cycloalcényle ou cycloalcényle substitué, hétérocyclo ou hétérocyclo substitué, cycloalkylalkyle ou cycloalkylalkyle substitué, cycloalcénylalkyle ou cycloalcénylalkyle substitué, hétérocycloalkyle ou hétérocycloalkyle substitué, aryle ou aryle substitué, arylalkyle ou arylalkyle substitué, CN, OH, $OR^1$, $R^1C=O$, $R^1OC=O$, $R^1NHC=O$, $SO_2R^1$, $SO_2OR^1$, ou $SO_2NR^1R^{1'}$ ;

à condition que :

(1) quand Y représente un groupe -O-, que $Q_1$ et $Q_2$ représentent un atome d'hydrogène, que $Z_1$ et $Z_2$ représentent un atome de O, que W représente un groupe $-CH_2-CH_2-$, et que l'un des groupes $A_1$ et $A_2$ représente un groupe CH et l'autre représente un groupe $CR^7$, alors G-L ne représente pas un groupe phényle non substitué ;
(2) quand Y représente un groupe -O-, que $Q_1$ et $Q_2$ représentent un atome d'hydrogène, que $Z_1$ et $Z_2$ représentent un atome de O, que W représente un groupe $-CH_2-CH_2-$, et que l'un des groupes $A_1$ et $A_2$ représente un groupe CH et l'autre représente un groupe $C-CH_3$, alors G-L ne représente pas un groupe phényle substitué avec un groupe chloro et/ou méthyle ;
(3) quand Y représente un groupe -O-, que $Q_1$ et $Q_2$ représentent un atome d'hydrogène, que $Z_1$ et $Z_2$ représentent un atome de O, que W représente un groupe $-CH_2-CH_2-$, et que l'un des groupes $A_1$ et $A_2$ représente un groupe CH et l'autre représente un groupe C-alkyle, alors G-L ne représente pas un groupe pipérazine-alkyle-N-substitué - ou un groupe imidazolidine-alkyle- N-substitué ;
(4) quand Y représente un groupe -O-; que $Q_1$ et $Q_2$ représentent un atome d'hydrogène, que $Z_1$ et $Z_2$ représentent un atome de O, que W représente un groupe $-CH_2-CH_2-$, et que $A_1$ et $A_2$ représentent un groupe $C-CH_3$, alors G-L ne représente pas un groupe thiazole ou thiazole substitué ;
(5) quand Y représente un groupe $-CH_2-S-$, -SO-, $-CH_2-SO_2-$, $-N(V^2)-$ ou $-CH_2-N(V^2)-$, que W représente un groupe $CR^7R^{7'}- CR^7R^{7'}$, et que $Z^1$ et $Z^2$ représentent un atome de O, alors G-L ne représente pas un groupe phényle non substitué ;
(6) quand Y représente un groupe $NR^7$, que W représente un groupe phényle non substitué ou substitué, et que $Q_1$ et $Q_2$ représentent un atome d'hydrogène, alors les composés répondant à la formule I ne sont pas inclus ;
(7) quand Y représente un groupe -O-, que $Q_1$ et $Q_2$ représentent un atome d'hydrogène, que $Z_1$ et $Z_2$ représentent un atome de O, que W représente de l'oxyde d'éthylène, et que $A_1$ et $A_2$ représentent un groupe CH, alors G-L ne représente pas un groupe méthylphényle ou chlorophényle ;
(8) le composé répondant à la formule I ne représente pas un groupe 6,10-épithio-4H-thiéno-[3',4':5,6]cyclooct [1,2-*f*]isoindole-7,9(5H,8H)dione, 8-(3,5-dichlorophényl)-6,6a,9a,10,11,12-hexahydro-1,3,6,10-tétraméthyl-2,2,13-trioxyde, (6R,6aR,9aS,10S) ;
(9) quand Y représente un groupe -O-, que $Q_1$ et $Q_2$ représentent un atome d'hydrogène, que $Z_1$ et $Z_2$ représentent un atome de O, que W représente un groupe cyclopentyle, cyclohexyle, 3-phényl-2-isoxazoline ou $CR^7R^{7'}-CR^7R^{7'}$ où $R^7$ et $R^{7'}$ sont chacun indépendamment définis comme représentant un atome de H et un

groupe 4-butyrolactone et que $R^7$ et $R^{7'}$ ne représentent pas tous simultanément un atome de H, et que $A_1$ et $A_2$ représentent un groupe CH, alors G-L ne représente pas un noyau naphtyle non substitué ou un noyau phényle monosubstitué, où ledit substituant représente un groupe méthoxy, Br, Cl, $NO_2$, méthyle, éthyle, $CH_2$-phényle, S-phényle, ou O-phényle ;

(10) quand Y représente un groupe -O- et que W représente un groupe $-CH_2-CH_2-$, alors au moins l'un des groupes $A_1$ ou $A_2$ ne représente pas un groupe CH ;

(11) dans lequel le composé répondant à la formule (I) ne représente pas un groupe

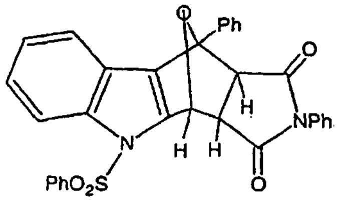

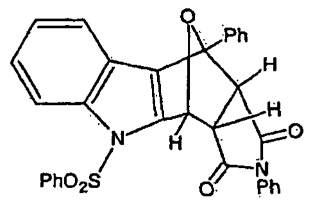

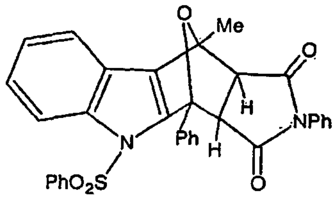

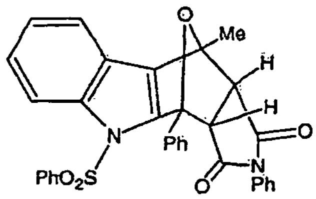

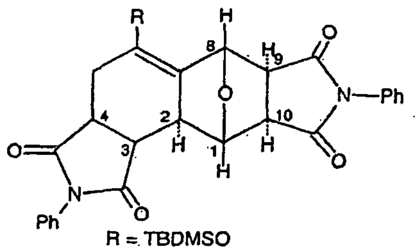

R = TBDMSO

R = Me

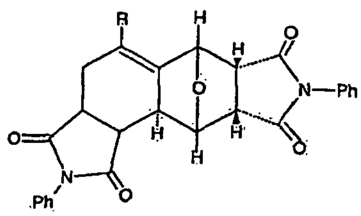

R = TBDMSO

R = Me

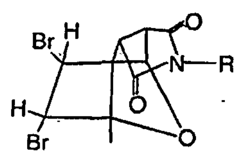

R = $C_6H_4$-$CH_3$ (2')
R = $C_6H_4$-$OCH_3$ (2')
R = 1-Naphthyle

dans lequel les termes « alkyle » et « alk » font référence à un radical alcane à chaîne linéaire ou ramifiée contenant de 1 à 12 atomes de carbone ; le terme « alkyle substitué » fait référence à un groupe alkyle substitué avec de 1 à 4 substituants, au niveau de n'importe quel point de fixation disponible, dans lequel les substituants représentent un ou plusieurs des groupes suivants : halogéno, alkoxy, alkylthio, hydroxy, carboxy, alkoxycarbonyle, alkylcarbonyloxy, amino, carbamoyle ou carbamoyle substitué, carbamate ou carbamate substitué, urée ou urée substituée, amidinyle ou amidinyle substitué, thiol, aryle, hétérocycle, cycloalkyle, hétérocycloalkyle, -S-aryle, -S-hétérocycle, -S=O-aryle, -S=O-hétérocycle, arylalkyl-O-, $-S(O)_2$-aryle, $-S(O)_2$-hétérocycle, $-NHS(O)_2$-aryle, $-NHS(O)_2$-hétérocy-

cle, -NHS(O)$_2$NH-aryle, -NHS(O)$_2$NH-hétérocycle, -P(O)$_2$-aryle, -P(O)$_2$-hétérocycle, -NHP(O)$_2$-aryle,- NHP(O)$_2$-hétérocycle, -NHP(O)$_2$NH-aryle, -NHP(O)$_2$NH-hétérocycle, -O-aryle, -O-hétérocycle, -NH-aryle, -NH-hétérocycle, -NHC=O-aryle, -NHC=O-alkyle, -NHC=O-hétérocycle, -OC=O-aryle, -OC=O-hétérocycle, -NHC=ONH-aryle, -NHC=ONH-hétérocycle, -OC=OO-aryle, -OC=OO-hétérocycle, -OC=ONH-aryle, -OC=ONH-hétérocycle, -NHC=OO-aryle, -NHC=OO-hétérocycle, -NHC=OO-alkyle, -C=ONH-aryle, -C=ONH-hétérocycle, -C=OO-aryle, -C=OO-hétérocycle, -N(alkyl)S(O)$_2$-aryle, -N(alkyl)S(O)$_2$-hétérocycle, -N(alkyl)S(O)$_2$NH-aryle, -N(alkyl)S(O)$_2$NH-hétérocycle, -N(alkyl)P(O)$_2$-aryle, -N(alkyl)P(O)$_2$-hétérocycle, -N(alkyl)P(O)$_2$NH-aryle, -N(alkyl)P(O)$_2$NH-hétérocycle, -N (alkyl)-aryle, -N(alkyl)-hétérocycle, -N(alkyl)C=O-aryle, -N(alkyl)C=O-hétérocycle, -N(alkyl)C=ONH-aryle, -N(alkyl) C=ONH-hétérocycle, -OC=ON(alkyl)-aryle, -OC=ON(alkyl)-hétérocycle, -N(alkyl)C=OO-aryle, -N(alkyl)C=OO-hétérocycle, -C=ON(alkyl)-aryle, -C=ON(alkyl)-hétérocycle, -NHS(O)$_2$N(alkyl)-aryle, -NHS(O)$_2$N(alkyl)-hétérocycle, -NHP(O)$_2$N(alkyl)-aryle, NHP(O)$_2$N(alkyl)-hétérocycle, -NHC=ON(alkyl)-aryle, -NHC=ON(alkyl)-hétérocycle, -N (alkyl)S(O)$_2$N(alkyl)-aryle, N(alkyl)S(O)$_2$N(alkyl)-hétérocycle, -N(alkyl)P(O)$_2$N(alkyl)-aryle, -N(alkyl)P(O)$_2$N(alkyl) -hétérocycle, -N(alkyl)C=ON(alkyl)-aryle, et -N(alkyl)C=ON(alkyl)-hétérocycle, dans lequel dans chaque cas les groupes « alkyle », « aryle » et « hétérocycle » peuvent eux-mêmes être éventuellement substitués ; les substituants alkyles comprennent également les groupes « T » et « T-R$^{'2}$ » dans lesquels T est défini comme étant un groupe contenant de l'azote, de l'oxygène ou du soufre, et R$^{'2}$ représente un atome de H, un groupe alkyle ou alkyle substitué, alcényle ou alcényle substitué, cycloalkyle ou cycloalkyle substitué, cycloalcényle ou cycloalcényle substitué, hétérocyclo ou hétérocyclo substitué, cycloalkylalkyle ou cycloalkylalkyle substitué, cycloalcénylalkyle ou cycloalcénylalkyle substitué, hétérocycloalkyle ou hétérocycloalkyle substitué, aryle ou aryle substitué, arylalkyle ou arylalkyle substitué, halogéno, CN, OR$^1$, nitro, hydroxylamine, hydroxylamide, amino, NHR$^4$, NR$^2$R$^5$, NOR$^1$, thiol, alkylthio ou alkylthio substitué, R$^1$C=O, R$^1$OC=O, R$^1$NHC=O, SO$_2$R$^1$, SOR$^1$, PO$_3$R$^1$R$^{1'}$, R$^1$R$^{1'}$NC=O, C=OSR$^1$, SO$_2$R$^1$, SO$_2$OR$^1$, ou SO$_2$NR$^1$R$^{1'}$ ;

le terme « alcényle » fait référence à un radical hydrocarbure à chaîne linéaire ou ramifiée contenant de 2 à 12 atomes de carbone et au moins une double liaison carbone-carbone ; le terme « alcényle substitué » fait référence à un groupe alcényle substitué avec de 1 à 4 substituants, au niveau de n'importe quel point de fixation disponible, dans lequel les substituants sont choisis parmi un groupe alkyle ou alkyle substitué, ainsi que les groupes cités ci-dessus comme représentant des substituants alkyles ;

le terme « cycloalkyle » fait référence à un groupe hydrocarbure cyclique complètement saturé contenant de 1 à 4 noyaux et de 3 à 8 atomes de carbone par noyau ; le terme « cycloalkyle substitué » fait référence à un groupe cycloalkyle substitué avec de 1 à 4 substituants, au niveau de n'importe quel point de fixation disponible, dans lequel les substituants sont choisis parmi les groupes nitro, cyano, alkyle ou alkyle substitué, ainsi que les groupes cités ci-dessus comme représentant des substituants alkyles, et un atome d'hydrogène, un groupe alkyle ou alkyle substitué, alcényle ou alcényle substitué, alcynyle ou alcynyle substitué, halogéno, cycloalkyle ou cycloalkyle substitué, cycloalcényle ou cycloalcényle substitué, aryle ou aryle substitué, hétérocyclo ou hétérocyclo substitué, arylalkyle ou arylalkyle substitué, hétérocycloalkyle ou hétérocycloalkyle substitué, CN, R$^1$OC=O, R$^1$C=O, R$^1$C=S, R$^1$HNC=O, R$^1$R$^2$NC=O, HOCR$^3$R$^{3'}$, nitro, R$^1$OCH$_2$, R$^1$O, NH$_2$, NR$^4$R$^5$, SR$^1$, S=OR$^1$, SO$_2$R$^1$, SO$_2$NR$^1$R$^{1'}$, (R$^1$O)(R$^{1'}$O)P=O, oxo, (R$^1$)(R$^{1'}$)P=O, (R$^1$)(NHR$^1$)P=O et un groupe cycloalcényle spiro-attaché ou fusionné ou cycloalcényle substitué;

le terme « cycloalcényle » fait référence à un groupe hydrocarbure cyclique partiellement insaturé contenant de 1 à 4 noyaux et de 3 à 8 atomes de carbone par noyau ; le terme « cycloalcényle substitué » fait référence à un groupe cycloalcényle substitué avec de 1 à 4 substituants, au niveau de n'importe quel point de fixation disponible ; dans lequel les substituants sont choisis parmi les groupes nitro, cyano, alkyle ou alkyle substitué, ainsi que les groupes cités ci-dessus comme représentant des substituants alkyles, et un atome d'hydrogène, un groupe alkyle ou alkyle substitué, alcényle ou alcényle substitué, alcynyle ou alcynyle substitué, halogéno, cycloalkyle ou cycloalkyle substitué, cycloalcényle ou cycloalcényle substitué, aryle ou aryle substitué, hétérocyclo ou hétérocyclo substitué, arylalkyle ou arylalkyle substitué, hétérocycloalkyle ou hétérocycloalkyle substitué, CN, R$^1$OC=O, R$^1$C=O, R$^1$C=S, R$^1$HNC=O, R$^1$R$^2$NC=O, HOCR$^3$R$^{3'}$, nitro, R$^1$OCH$_2$, R$^1$O, NH$_2$, NR$^4$R$^5$, SR$^1$, S=OR$^1$, SO$_2$R$^1$, SO$_2$OR$^1$, SO$_2$NR$^1$R$^{1'}$, (R$^1$O)(R$^{1'}$O)P=O, oxo, (R$^1$)(R$^{1'}$)P=O, (R$^{1'}$)(NHR$^1$)P=O, et un groupe cycloalkyle spiro-attaché ou fusionné ou cycloalkyle substitué;

les termes « alkoxy » ou « alkylthio » font référence à un groupe alkyle tel que décrit ci-dessus lié par l'intermédiaire d'une liaison oxygène (-O-) ou une liaison soufre (-S-), respectivement ; les termes « alkoxy substitué » ou « alkylthio substitué » font référence à un groupe alkyle substitué tel que décrit ci-dessus lié par l'intermédiaire d'une liaison oxygène ou soufre, respectivement ;

le terme « alkoxycarbonyle » fait référence à un groupe alkoxy lié par l'intermédiaire d'un groupe carbonyle ;

le terme « alkylcarbonyle » fait référence à un groupe alkyle lié par l'intermédiaire d'un groupe carbonyle ; le terme « alkylcarbonyloxy » fait référence à un groupe alkylcarbonyle lié par l'intermédiaire d'une liaison oxygène ;

les termes « arylalkyle », « arylalkyle substitué », « cycloalkylalkyle », « cycloalkylalkyle substitué », « cycloalcénylalkyle », « cycloalcénylalkyle substitué », « hétérocycloalkyle » et « hétérocycloalkyle substitué » font référence aux groupes aryle, cycloalkyle, cycloalcényle et hétérocyclo liés par l'intermédiaire d'un groupe alkyle, substitués sur le groupe aryle, cycloalkyle, cycloalcényle ou hétérocyclo et/ou alkyle quand ils sont indiqués comme étant « substitués » ;
le terme « aryle » fait référence à des groupes hydrocarbures cycliques, aromatiques qui ont de 1 à 5 noyaux aromatiques ; quand ils contiennent deux noyaux aromatiques ou plus, les noyaux aromatiques du groupe aryle peuvent être liés au niveau d'un point unique ou fusionnés, le terme « aryle substitué » fait référence à un groupe aryle substitué par 1,2,3,4 ou 5 substituants, au niveau de n'importe quel point de fixation, dans lequel les substituants sont choisis parmi les groupes nitro, cycloalkyle ou cycloalkyle substitué, cycloalcényle ou cycloalcényle substitué, cyano, alkyl-$S(O)_m$- (m = 0, 1 ou 2), alkyle ou alkyle substitué, ainsi que les groupes cités ci-dessus comme représentant des substituants alkyles et un atome d'hydrogène, un groupe alkyle ou alkyle substitué, alcényle ou alcényle substitué, alcynyle ou alcynyle substitué, halogéno, cycloalkyle ou cycloalkyle substitué, cycloalcényle ou cycloalcényle substitué, aryle ou aryle substitué, hétérocyclo ou hétérocyclo substitué, arylalkyle ou arylalkyle substitué, hétérocycloalkyle ou hétérocycloalkyle substitué, CN, $R^1OC=O$, $R^1C=O$, $R^1C=S$, $R^1HNC=O$, $R^1R^2NC=O$, $HOCR^3R^{3'}$, nitro, $R^1OCH_2$, $R^1O$, $NH_2$, $NR^4R^5$, $SR^1$, $S=OR^1$, $SO_2R^1$, $SO_2OR^1$, $SO_2NR^1R^{1'}$, $(R^1O)(R^{1'}O)P=O$, oxo, $(R^1)(R^{1'})P=O$, $(R^{1'})(NHR^1)P=O$, et des groupes hétérocyclo ou cycloalcényles fusionnés ou des groupes hétérocyclo ou cycloalcényles substitués ; les termes « carbamoyle substitué », « carbamate substitué », « urée substituée » et « amidinyle substitué » font référence aux groupes carbamoyle, carbamate, urée ou amidinyle dans lesquels un ou plusieurs des groupes hydrogène sont remplacés par un groupe organique listé ci-dessus ;
les termes « hétérocycle », « hétérocyclique » et « hétérocyclo » font référence à des systèmes de noyaux complètement saturés, ou partiellement ou complètement insaturés, comprenant les systèmes de noyaux aromatiques monocycliques de 3 à 7 chaînons, bicycliques de 7 à 11 chaînons, ou tricycliques de 10 à 16 chaînons, qui ont au moins un hétéroatome dans au moins un noyau contenant des atomes de carbone, dans lesquels chaque noyau du groupe hétérocyclique contenant un hétéroatome peut avoir 1, 2, 3 ou 4 hétéroatomes choisis parmi les atomes d'azote, les atomes d'oxygène et/ou les atomes de soufre, où les hétéroatomes d'azote et de soufre peuvent éventuellement être oxydés et les hétéroatomes d'azote peuvent éventuellement être quaternisés ;
les termes « hétérocycle substitué », « hétérocyclique substitué », et « hétérocyclo substitué » font référence aux groupes hétérocycles, hétérocycliques ou hétérocyclo substitués avec de 1 à 4 substituants, au niveau de n'importe quel point de fixation disponible, dans lesquels les substituants sont choisis parmi les groupes cycloalkyle ou cycloalkyle substitué, cycloalcényle ou cycloalcényle substitué, nitro, oxo, cyano, alkyl-$S(O)_m$- (m = 0, 1 ou 2), alkyle ou alkyle substitué, ainsi que les groupes cités ci-dessus comme représentant des substituants alkyles, et un atome d'hydrogène, un groupe alkyle ou alkyle substitué, alcényle ou alcényle substitué, alcynyle ou alcynyle substitué, halogéno, cycloalkyle ou cycloalkyle substitué, cycloalcényle ou cycloalcényle substitué, aryle ou aryle substitué, hétérocyclo ou hétérocyclo substitué, arylalkyle ou arylalkyle substitué, hétérocycloalkyle ou hétérocycloalkyle substitué, CN, $R^1OC=O$, $R^1C=O$, $R^1C=S$, $R^1HNC=O$, $R^1R^2NC=O$, $HOCR^3R^{3'}$, nitro, $R^1OCH_2$, $R^1O$, $NH_2$, $NR^4R^5$, $SR^1$, $S=OR^1$, $SO_2R^1$, $SO_2OR^1$, $SO_2NR^1R^{1'}$, $(R^1O)(R^{1'}O)P=O$, oxo, $(R^1)(R^{1'})P=O$, $(R^{1'})(NHR^1)P=O$.

**2.** Composé selon la revendication 1 dans lequel L représente une liaison.

**3.** Composé choisi dans le groupe constitué par :

la (3aα,4α,7α,7aα)-2-(4-bromo-3-méthylphényl)tétrahydro-4,7-éthanothiopyrano[3,4-c]pyrrole-1,3,8(2H,4H)-trione (1C) ;
le 5,5-dioxyde de (3aα,4α,7α,7aα)-2-(4-bromo-3-méthylphényl)tétrahydro-4,7-éthanothiopyrano[3,4-c]pyrrole-1,3,8(2H,4H)-trione (2) ;
la (3aα,4β,7β,7aα)-2-(3-chlorophényl)hexahydro-4-méthyl-4,7-époxy-1H-isoindole-1,3(2H)-dione (3) ;
la (3aα,4α,7α,7aα)- et la (3aα,4β,7β,7aα)-4-[(acétyloxy)méthyl]-3a,4,7,7a-tétrahydro-2-[3-(trifluorométhyl)phényl]-4,7-époxy-1H-isoindole-1,3(2H)-dione (4i & 4ii, respectivement) ;
la (3aα,4α,7α,7aα)- et la (3aα,4β,7β,7aα)-4-[(acétyloxy)méthyl]-hexahydro-2-[3-(trifluorométhyl)phényl]-4,7-époxy-1H-isoindole-1,3(2H)-dione (5i & 5ii, respectivement) ;
la (3aα,4α,7α,7aα)- et la (3aα,4β,7β,7aα)-3a,4,7,7a-tétrahydro-5-(hydroxyméthyl)-2-[3-(trifluorométhyl)phényl]-4,7-époxy-1H-isoindole-1,3(2H)-dione (6i & 6ii, respectivement) ;
la (3aα,4α,7α,7aα)-3a,4,7,7a-tétrahydro-5-(hydroxyméthyl)-4-méthyl-2-[3-(trifluorométhyl)phényl]-4,7-époxy-1H-isoindole-1,3(2H)-dione (7) ;
la (3aα,4β,7β,7aα)-2-[3,5-bis(trifluorométhyl)phényl]hexahydro-4,7-époxy-1H-isoindole-1,3(2H)-dione (8) ;
l'ester phénylique d'acide (3aα,4α,7α,7aα)-2-(4-bromophényl)octahydro-1,3-dioxo-4,7-ethéno-5H-pyrrolo[3,4-c]pyridine-5-carboxylique (9) ;
l'ester phénylméthylique d'acide (3aα,4α,7α,7aα)-2-(4-bromophényl)octahydro-1,3-dioxo-4,7-éthéno-5H-pyrrolo[3,4-c]pyridine-5-carboxylique (10) ;

le trifluoroacétate de (3aα,4α,7α,7aα)-hexahydro-2-[3-(trifluorométhyl)phényl]-4,7-éthano-1H-pyrrolo[3,4-c]pyridine-1,3(2H)-dione (11) ;
la (3aα,4α,7α,7aα)-5-acétylhexahydro-2-[3-(trifluorométhyl)phényl]-4,7-éthano-1H-pyrrolo[3,4-c]pyridine-1,3 (2H)-dione (12) ;
la (3aα,4α,7α,7aα)-5-benzoylhexahydro-2-[3-(trifluorométhyl)phényl]-4,7-éthano-1H-pyrrolo[3,4-c]pyridine-1,3(2H)-dione (13) ;
la (3aα,4α,7α,7aα)-hexahydro-5-méthyl-2-[3-(trifluorométhyl)phényl]-4,7-éthano-1H-pyrrolo[3,4-c]pyridine-1,3 (2H)-dione (14) ;
le trifluoroacétate de (3aα,4α,7α,7aα)-hexahydro-5-(phénylméthyl)-2-[3-(trifluorométhyl)phényl]-4,7-éthano-1H-pyrrolo[3,4-c]pyridine-1,3(2H)-dione (15) ;
le trifluoroacétate de (3aα,4α,7α,7aα)-hexahydro-5-propyl-2-[3-(trifluorométhyl)phényl]-4,7-éthano-1H-pyrrolo [3,4-c]pyridine-1,3(2H)-dione (16) ;
l'ester phénylméthylique d'acide (3aα,4α,4aβ,5aβ,6α,6aα)-2-[4-cyano-3-(trifluorométhyl)phényl]décahydro-1,3-dioxo-4,6-(iminométhano)cycloprop[f]isoindole-7-carboxylique (17) ;
le (3aα,4α,4aβ,5aβ,6α,6aα)-4-[décahydro-1,3-dioxo-4,6-(iminométhano)cycloprop[f]isoindol-2-yl]-2-(trifluorométhyl)benzonitrile (18) ;
le (3aα,4α,4aβ,5aβ,6α,6aα)-4-[décahydro-7-méthyl-1,3-dioxo-4,6-(iminométhano)cycloprop[f]isoindol-2-yl]-2-(trifluorométhyl)benzonitrile (19) ;
le (3aα,4β,7β,7aα)-4-(octahydro-4,7-diméthyl-1,3-dioxo-4,7-époxy-2H-isoindol-2-yl)-2-(trifluorométhyl)benzonitrile (20B) ;
le (3aα,4β,7β,7aα)-N-[4-[[2-[2-[4-cyano-3-(trifluorométhyl)phényl]octahydro-7-méthyl-1,3-dioxo-4,7-époxy-4H-isoindol-4-yl]éthyl]thio]phényl]acétamide (21 E) ;
le (3aα,4β,7β,7aα)-N-[4-[[2-[2-[4-cyano-3-(trifluorométhyl)phényl]octahydro-7-méthyl-1,3-dioxo-4,7-époxy-4H-isoindol-4-yl]éthyl]sulfinyl]phényl]acétamide (22) ;
le (3aα,4β,7β,7aα)-N-[4-[[2-[2-[4-cyano-3-(trifluorométhyl)phényl]octahydro-7-méthyl-1,3-dioxo-4,7-époxy-4H-isoindol-4-yl]éthyl]sulfonyl]phényl]acétamide (23) ;
le (3aα,4β,7β,7aα)- et le (3aα,4α,7α,7aα)-N-[2-[2-[4-cyano-3-(trifluorométhyl)phényl]octahydro-7-méthyl-1,3-dioxo-4,7-époxy-4H-isoindol-4-yl]éthyl]benzènesulfonamide (24Ci & 24Cii, respectivement) ;
le (3aα,4β,7β,7aα)-4-[octahydro-4-(2-hydroxyéthyl)-7-méthyl-1,3-dioxo-4,7-époxy-2H-isoindol-2-yl]-2-(trifluorométhyl)benzonitrile (25B) ;
le (3aα,4α,7α,7aα)- et le (3aα,4β,7β,7aα)-N-[4-[2-[2-[4-cyano-3-(trifluorométhyl)phényl]octahydro-7-méthyl-1,3-dioxo-4,7-époxy-4H-isoindol-4-yl]éthoxy]phényl]acétamide (26Ci & 26Cii, respectivement) ;
la (3aα,4α,7α,7aα)-hexahydro-2-(2-naphtalényl)-4,7-époxy-1H-isoindole-1,3(2H)-dione (27D) ;
la (1aα,2β,2aα,5aα,6β,6aα)-hexahydro-4-(2-naphtalényl)-2,6-époxy-3H-oxiréno[f]isoindole-3,5(4H)-dione (28B) ;
le 8-oxyde de (3aα,4α,7α,7aα)-2-[4-bromo-3-(trifluorométhyl)phényl]-3a,4,7,7a-tétrahydro-4,7-diméthyl-4,7-épithio-1H-isoindole-1,3(2H)-dione (29);
le 8-oxyde de (3aα,4α,7α,7aα)-2-[4-bromo-3-(trifluorométhyl)phényl]-3a,4,7,7a-tétrahydro-4,7-épithio-1H-isoindole-1,3(2H)-dione (30) ;
la (3aα,4α,7α,7aα)-hexahydro-2-[3-(trifluorométhyl)phényl]-4,7-imino-1H-isoindole-1,3(2H)-dione (31 D) ;
la (3aα,4β,7β,7aα)- et la (3aα,4α,7α,7aα)-3a,4,7,7a-tétrahydro-4,7-diméthyl-2-[3-(trifluorométhyl)phényl]-4,7-époxy-1H-isoindole-1,3(2H)-dione (32i & 32ii, respectivement) ;
la (3aα,4α,7α,7aα)-hexahydro-4,7-diméthyl-2-[3-(trifluorométhyl)phényl]-4,7-époxy-1H-isoindole-1,3(2H)-dione (33) ;
la (3aα,4α,7α,7aα)-tétrahydro-5-méthyl-2-(4-nitro-1-naphtalényl)-4,7-éthéno-1H-pyrrolo[3,4-c]pyridine-1,3,6 (2H,5H)-trione (34B) ;
le (3aα,4β,7β,7aα)-4-[4-[2-(4-fluorophénoxy)éthyl]octahydro-7-méthyl-1,3-dioxo-4,7-époxy-2H-isoindol-2-yl]-2-(trifluorométhyl)benzonitrile (35) ;
le (3aα,4β,7β,7aα)-4-[4-(2-bromoéthyl)octahydro-7-méthyl-1,3-dioxo-4,7-époxy-2H-isoindol-2-yl]-2-(trifluorométhyl)benzonitrile (36) ;
la (3aα,4β,7β,7aα)-hexahydro-4,7-diméthyl-2-(3-méthyl-4-nitrophényl)-4,7-époxy-1H-isoindole-1,3(2H)-dione (37) ;
la (3aα,4β,7β,7aα)-2-(2-fluorényl)hexahydro-4,7-époxy-1H-isoindole-1,3(2H)-dione ;
la (3aα,4β,7β,7aα)-2-[3-chloro-4-(4-morpholinyl)phényl]hexahydro-4,7-époxy-1H-isoindole-1,3(2H)-dione ;
la (3aα,4β,7β,7aα)-2-(2,3-dihydro-1H-indén-5-yl)hexahydro-4,7-époxy-1H-isoindole-1,3(2H)-dione ;
la (3aα,4β,7β,7aα)-2-(4-bromo-1-naphtalényl)hexahydro-4,7-époxy-1H-isoindole-1,3(2H)-dione ;
la (3aα,4β,7β,7aα)-2-(4-chloro-1-naphtalényl)hexahydro-4,7-époxy-1H-isoindole-1,3(2H)-dione ;
la (3aα,4β,7β,7aα)-2-(5-amino-1-naphtalényl)hexahydro-4,7-époxy-1H-isoindole-1,3(2H)-dione ;

la (3aα,4β,7β,7aα)-hexahydro-2-(7-hydroxy-1-naphtalényl)-4,7-époxy-1H-isoindole-1,3(2H)-dione ;
la (3aα,4β,7β,7aα)-hexahydro-2-(4-nitro-1-naphtalényl)-4,7-époxy-1H-isoindole-1,3(2H)-dione ;
la (3aα,4β,7β,7aα)-hexahydro-2-(1H-indol-5-yl)-4,7-époxy-1H-isoindole-1,3(2H)-dione ;
la (3aα,4β,7β,7aα)-hexahydro-2-(1H-indazol-6-yl)-4,7-époxy-1H-isoindole-1,3(2H)-dione ;
la (3aα,4β,7β,7aα)-2-(1,3-benzodioxol-5-yl)hexahydro-4,7-époxy-1H-isoindole-1,3(2H)-dione ;
la (3aα,4β,7β,7aα)-2-[4-amino-3-(trifluorométhyl)phényl]hexahydro-4,7-époxy-1H-isoindole-1,3(2H)-dione ;
la (3aα,4β,7β,7aα)-2-(3-chloro-4-iodophényl)hexahydro-4,7-époxy-1H-isoindole-1,3(2H)-dione ;
la (3aα,4β,7β,7aα)-hexahydro-2-(8-quinolinyl)-4,7-époxy-1H-isoindole-1,3(2H)-dione ;
la (3aα,4β,7β,7aα)-2-(2,3-dihydro-1,4-benzodioxin-6-yl)hexahydro-4,7-époxy-1H-isoindole-1,3(2H)-dione ;
la (3aα,4β,7β,7aα)-hexahydro-2-[2-oxo-4-(trifluorométhyl)-2H-1-benzopyran-7-yl]-4,7-époxy-1H-isoindole-1,3(2H)-dione ;
la (3aα,4β,7β,7aα)-hexahydro-2-(4-méthyl-2-oxo-2H-1-benzopyran-7-yl)-4,7-époxy-1H-isoindole-1,3(2H)-dione ;
la (3aα,4β,7β,7aα)-2-(2,5-diméthoxy-4-nitrophényl)hexahydro-4,7-époxy-1H-isoindole-1,3(2H)-dione ;
le (3aα,4β,7β,7aα)-2,3,5,6-tétrafluoro-4-(octahydro-1,3-dioxo-4,7-époxy-2H-isoindol-2-yl)benzonitrile ;
la (3aα,4β,7β,7aα)-hexahydro-2-(2,4,5-trifluorophényl)-4,7-époxy-1H-isoindole-1,3(2H)-dione ;
la (3aα,4β,7β,7aα)-hexahydro-2-(2,4,5-trichlorophényl)-4,7-époxy-1H-isoindole-1,3(2H)-dione ;
la (3aα,4β,7β,7aα)-2-(2-amino-4,5-dichlorophényl)hexahydro-4,7-époxy-1H-isoindole-1,3(2H)-dione ;
la (3aα,4β,7β,7aα)-2-(3,4-difluorophényl)hexahydro-4,7-époxy-1H-isoindole-1,3(2H)-dione ;
le (3aα,4β,7β,7aα)-1-acétyl-2,3-dihydro-6-(octahydro-1,3-dioxo-4,7-époxy-2H-isoindol-2-yl)-1H-indole ;
la (3aα,4β,7β,7aα)-2-(3-chloro-4-fluorophényl)hexahydro-4,7-époxy-1H-isoindole-1,3(2H)-dione ;
la (3aα,4β,7β,7aα)-2-(3,4-dichlorophényl)hexahydro-4,7-époxy-1H-isoindole-1,3(2H)-dione ;
la (3aα,4β,7β,7aα)-hexahydro-2-(3,4,5-trichlorophényl)-4,7-époxy-1H-isoindole-1,3(2H)-dione ;
la (3aα,4β,7β,7aα)-2-(3-chloro-4-méthoxyphényl)hexahydro-4,7-époxy-1H-isoindole-1,3(2H)-dione ;
la (3aα,4β,7β,7aα)-2-(3-chloro-4-méthylphényl)hexahydro-4,7-époxy-1H-isoindole-1,3(2H)-dione ;
la (3aα,4β,7β,7aα)-hexahydro-2-(2-méthyl-1-naphtalényl)-4,7-époxy-1H-isoindole-1,3(2H)-dione ;
la (3aα,4β,7β,7aα)-2-(4-chloro-3-méthylphényl)hexahydro-4,7-époxy-1 H-isoindole-1,3(2H)-dione ;
la (3aα,4β,7β,7aα)-2-(3,4-diméthylphényl)hexahydro-4,7-époxy-1H-isoindole-1,3(2H)-dione ;
la (3aα,4β,7β,7aα)-2-[4-bromo-3-(trifluorométhyl)phényl]hexahydro-4,7-époxy-1 H-isoindole-1,3(2H)-dione ;
la (3aα,4β,7β,7aα)-2-(4-bromo-3-méthylphényl)hexahydro-4,7-époxy-1H-isoindole-1,3(2H)-dione ;
la (3aα,4β,7β,7aα)-2-(4-fluoro-3-nitrophényl)hexahydro-4,7-époxy-1H-isoindole-1,3(2H)-dione ;
la (3aα,4β,7β,7aα)-2-[4-fluoro-3-(trifluorométhyl)phényl]hexahydro-4,7-époxy-1H-isoindole-1,3(2H)-dione ;
la (3aα,4β,7β,7aα)-2-(4-chloro-3-nitrophényl)hexahydro-4,7-époxy-1H-isoindole-1,3(2H)-dione ;
la (3aα,4β,7β,7aα)-2-[4-chlolo-3-(trifluorométhyl)phényl]hexahydro-4,7-époxy-1H-isoindole-1,3(2H)-dione ;
la (3aα,4β,7β,7aα)-2-(4-chloro-2-méthoxy-5-méthylphényl)hexahydro-4,7-époxy-1H-isoindole-1,3(2H)-dione ;
la (3aα,4β,7β,7aα)-2-(4-amino-3-nitrophényl)hexahydro-4,7-époxy-1H-isoindole-1,3(2H)-dione ;
la (3aα,4β,7β,7aα)-hexahydro-2-(4-méthyl-3-nitrophényl)-4,7-époxy-1H-isoindole-1,3(2H)-dione ;
la (3aα,4β,7β,7aα)-2-(3,4-diméthoxyphényl)hexahydro-4,7-époxy-1H-isoindole-1,3(2H)-dione ;
la (3aα,4β,7β,7aα)-hexahydro-2-(3-hydroxy-4-méthoxyphényl)-4,7-époxy-1H-isoindole-1,3(2H)-dione ;
la (3aα,4β,7β,7aα)-hexahydro-2-(4-méthyl-5-nitro-2-pyridinyl)-4,7-époxy-1H-isoindole-1,3(2H)-dione ;
le (3aα,4β,7β,7aα)-2-chloro-4-(octahydro-1,3-dioxo-4,7-époxy-2H-isoindol-2-yl)-α-phénylbenzèneacétonitrile ;
la (3aα,4β,7β,7aα)-hexahydro-2-(2-méthoxy-3-dibenzofuranyl)-4,7-époxy-1H-isoindole-1,3(2H)-dione ;
la (3aα,4β,7β,7aα)-hexahydro-2-(2,3,4-trifluorophényl)-4,7-époxy-1H-isoindole-1,3(2H)-dione ;
la (3aα,4β,7β,7aα)-2-(2,3-dihydro-2-méthyl-1,3-dioxo-1H-isoindol-5-yl)hexahydro-4,7-époxy-1H-isoindofe-1,3(2H)-dione ;
la (3aα,4β,7β,7aα)-2-(4-bromo-2,3,5,6-tétrafluorophényl)hexahydro-4,7-époxy-1H-isoindole-1,3(2H)-dione ;
la (3aα,4β,7β,7aα)-hexahydro-2-(2-hydroxy-1-naphtalényl)-4,7-époxy-1H-isoindole-1,3(2H)-dione ;
la (3aα,4β,7β,7aα)-2-[2,5-dichloro-4-(1H-pyrrol-1-yl)phényl]hexahydro-4,7-époxy-1H-isoindole-1,3(2H)-dione ;
la (3aα,4β,7β,7aα)-hexahydro-2-[4-(méthoxyméthyl)-2-oxo-2H-1-benzopyran-7-yl]-4,7-époxy-1H-isoindole-1,3(2H)-dione ;
la (3aα,4β,7β,7aα)-2-(6-benzothiazolyl)hexahydro-4,7-époxy-1H-isoindole-1,3(2H)-dione ;
l'ester méthylique d'acide (3aα,4β,7β,7aα)-2-méthoxy-4-(octahydro-1,3-dioxo-4,7-époxy-2H-isoindol-2-yl)benzoïque ;
le (3aα,4β,7β,7aα)-2-méthyl-5-(octahydro-1,3-dioxo-4,7-époxy-2H-isoindol-2-yl)benzonitrile ;
la (3aα,4β,7β,7aα)-hexahydro-2-(2-oxo-2H-1-benzopyran-6-yl)-4,7-époxy-1H-isoindole-1,3(2H)-dione ;
la (3aα,4β,7β,7aα)-hexahydro-2-(2,3,5,6-tétraméthyl-4-nitrophényl)-4,7-époxy-1H-isoindole-1,3(2H)-dione ;

la (3aα,4β,7β,7aα)-hexahydro-2-(2,4,5-triméthylphényl)-4,7-époxy-1H-isoindole-1,3(2H)-dione ;
la (3aα,4β,7β,7aα)-2-(4-fluoro-3-méthylphényl)hexahydro-4,7-époxy-1H-isoindole-1,3(2H)-dione ;
la (3aα,4β,7β,7aα)-hexahydro-2-(3-méthoxy-4-méthylphényl)-4,7-époxy-1H-isoindole-1,3(2H)-dione ;
le (3aα,4β,7β,7aα)-N-éthyl-2-méthyl-5-(octahydro-1,3-dioxo-4,7-époxy-2H-isoindol-2-yl)-N-phénylbenzènesulfonamide ;
le (3aα,4β,7β,7aα)-2,6-dibromo-4-(octahydro-1,3-dioxo-4,7-époxy-2H-isoindol-2-yl)benzènesulfonamide ;
le (3aα,4β,7β,7aα)-2,4-diméthyl-6-(octahydro-1,3-dioxo-4,7-époxy-2H-isoindol-2-yl)-3-pyridinecarbonitrile ;
la (3aα,4β,7β,7aα)-2-(2,3-diméthyl-1H-indol-5-yl)hexahydro-4,7-époxy-1H-isoindole-1,3(2H)-dione ;
la (3aα,4β,7β,7aα)-2-(3-dibenzofuranyl)hexahydro-4,7-époxy-1H-isoindole-1,3(2H)-dione ;
la (3aα,4β,7β,7aα)-hexahydro-2-(2'-hydroxy[1,1':3',1''-terphényl]-5'-yl)-4,7-époxy-1H-isoindole-1,3(2H)-dione ;
la (3aα,4β,7β,7aα)-hexahydro-2-(5,6,7,8-tétrahydro-3-hydroxy-2-naphtalényl)-4,7-époxy-1H-isoindole-1,3(2H)-dione ;
la (3aα,4β,7β,7aα)-2-(2,3-dihydro-1H-indol-6-yl)hexahydro-4,7-époxy-1H-isoindole-1,3(2H)-dione ;
la (3aα,4β,7β,7aα)-2-(1,3-dihydro-2,2-dioxydobenzo[c]thiophén-5-yl)hexahydro-4,7-époxy-1H-isoindole-1,3(2H)-dione ;
la (3aα,4β,7β,7aα)-hexahydro-2-(2-hydroxy-4,5-diméthylphényl)-4,7-époxy-1H-isoindole-1,3(2H)-dione ;
la (3aα,4β,7β,7aα)-2-(2,3-dihydro-2,2,3,3-tétrafluoro-1,4-benzodioxin-6-yl)hexahydro-4,7-époxy-1H-isoindole-1,3(2H)-dione ;
la (3aα,4β,7β,7aα)-hexahydro-2-(1H-indazol-5-yl)-4,7-époxy-1H-isoindole-1,3(2H)-dione ;
la (3aα,4β,7β,7aα)-2-(4-amino-2,3,5,6-tétrafluorophényl)hexahydro-4,7-époxy-1H-isoindole-1,3(2H)-dione ;
la (3aα,4β,7β,7aα)-2-(4-bromo-3-chlorophényl)hexahydro-4,7-époxy-1H-isoindole-1,3(2H)-dione ;
la (3aα,4β,7β,7aα)-hexahydro-2-(5-hydroxy-1-naphtalényl)-4,7-époxy-1H-isoindole-1,3(2H)-dione ;
le (3aα,4β,7β,7aα)-4-(octahydro-1,3-dioxo-4,7-époxy-2H-isoindo)-2-yl)-2-(trifluorométhyl)benzonitrile ;
l'ester méthylique d'acide (3aα,4β,7β,7aα)-2-(4-morpholinyl)-5-(octahydro-1,3-dioxo-4,7-époxy-2H-isoindol-2-yl)benzoïque ;
le (3aα,4β,7β,7aα)-2-fluoro-5-(octahydro-1,3-dioxo-4,7-époxy-2H-isoindol-2-yl)benzonitrile ;
la (3aα,4β,7β,7aα)-hexahydro-2-(2-naphtalényl)-4,7-époxy-1H-isoindole-1,3(2H)-dione ;
la (3aα,4β,7β,7aα)-2-(9-éthyl-9H-carbazol-3-yl)hexahydro-4,7-époxy-1H-isoindole-1,3(2H)-dione ;
la (3aα,4β,7β,7aα)-2-[1,2-dihydro-8-méthyl-2-oxo-4-(trifluorométhyl)-7-quinolinyl]hexahydro-4,7-époxy-1H-isoindole-1,3(2H)-dione ;
la (3aα,4α,7α,7aα)-hexahydro-2-[3-(trifluorométhyl)phényl]-4,7-époxy-1H-isoindole-1 ,3(2H)-dione ;
la (3aα,4α,7α,7aα)-hexahydro-2-(4-nitro-1-naphtalényl)-4,7-époxy-1H-isoindole-1,3(2H)-dione ;
la (3aα,4β,7β,7aα)-2-(4-bromo-3-méthylphényl)-3a,4,7,7a-tétrahydro-4,7-époxy-1H-isoindole-1,3(2H)-dione ;
la (3aα,4β,7β,7aα)-3a,4,7,7a-tétrahydro-2-(2-naphtalényl)-4,7-époxy-1H-isoindole-1,3(2H)-dione ;
la (3aα,4β,7β,7aα)-2-(9-éthyl-9H-carbazol-3-yl)-3a,4,7,7a-tétrahydro-4,7-époxy-1H-isoindole-1,3(2H)-dione ;
la (3aα,4β,7β,7aα)-2-[4-fluoro-3-(trifluorométhyl)phényl]-3a,4,7,7a-tétrahydro-4,7-époxy-1H-isoindole-1,3(2H)-dione ;
la (3aα,4β,7β,7aα)-2-[1,2-dihydro-8-méthyl-2-oxo-4-(trifluorométhyl)-7-quinolinyl]-3a,4,7,7a-tétrahydro-4,7-époxy-1H-isoindole-1,3(2H)-dione ;
la (3aα,4α,7α,7aα)-4-[(acétyloxy)méthyl]-2-(4-bromo-3-méthylphényl)-3a,4,7,7a-tétrahydro-4,7-époxy-1H-isoindole-1,3(2H)-dione ;
la (3aα,4β,7β,7aα)-4-[(acétyloxy)méthyl]-2-(4-bromo-3-méthylphényl)-3a,4,7,7a-tétrahydro-4,7-époxy-1H-isoindole-1,3(2H)-dione ;
la (3aα,4β,7β,7aα)-hexahydro-4,7-diméthyl-2-[3-(trifluorométhyl)phényl]-4,7-époxy-1H-isoindole-1,3(2H)-dione ;
le (3aα,4β,7β,7aα)-4-(octahydro-4,7-diméthyl-1,3-dioxo-4,7-époxy-2H-isoindol-2-yl)-1-naphtalènecarbonitrile ;
la (3aα,4β,7β,7aα)-(benzo[b]thiophén-3-yl)hexahydro-4,7-diméthyl-4,7-époxy-1H-isoindole-1,3(2H)-dione;
la (3aα,4β,7β,7aα)-hexahydro-4,7-diméthyl-2-[4-nitro-3-(trifluorométhyl)phényl]-4,7-époxy-1H-isoindole-1,3(2H)-dione ;
le (3aα,4β,7β,7aα)-4-(1,3,3a,4,7,7a-hexahydro-4,7-diméthyl-1,3-dioxo-4,7-époxy-2H-isoindol-2-yl)-1-naphtalènecarbonitrile ;
la (3aα,4α,7α,7aα)-hexahydro-4-méthyl-2-(2-naphtalényl)-4,7-époxy-1H-isoindole-1,3(2H)-dione ;
la (3aα,4β,7β,7aα)-2-(4-bromo-3-méthylphényl)hexahydro-4-méthyl-4,7-époxy-1H-isoindole-1,3(2H)-dione ;
la (3aα,4β,7β,7aα)-hexahydro-4-méthyl-2-[3-(trifluorométhyl)phényl]-4,7-époxy-1H-isoindole-1,3(2H)-dione ;
la (3aα,4β,7β,7aα)-2-(3,5-dichlorophényl)hexahydro-4-méthyl-4,7-époxy-1H-isoindole-1,3(2H)-dione ;
la (3aα,4β,7β,7aα)-2-(3-chloro-4-fluorophényl)hexahydro-4-méthyl-4,7-époxy-1H-isoindole-1,3(2H)-dione ;

le (3aα,4β,7β,7aα)-2-méthoxy-4-(octahydro-1,3-dioxo-4-méthyl-4,7-époxy-2H-isoindol-2-yl)-1-naphtalènecarbonitrile ;
la (3aα,4β,7β,7aα)-hexahydro-4-méthyl-2-[4-nitro-3-(trifluorométhyl)phényl]-4,7-époxy-1H-isoindole-1,3(2H) -dione ;
la (3aα,4β,7β,7aα)-hexahydro-2-[4-(1H-imidazol-1-yl)phényl]-4-méthyl-4,7-époxy-1H-isoindole-1,3(2H)-dione ;
la (3aα,4β,7β,7aα)-2-[3-chloro-4-(2-thiazolyl)phényl]hexahydro-4-méthyl-4,7-époxy-1H-isoindole-1,3(2H)-dione ;
la (3aα,4α,7α,7aα)-2-(3,5-dichlorophényl)hexahydro-4,7-imino-1H-isoindole-1,3(2H)-dione ;
la (3aα,4α,7α,7aα)-2-(4-bromo-1-naphtalényl)hexahydro-4,7-imino-1H-isoindole-1,3(2H)-dione ;
la (3aα,4α,7α,7aα)-2-(4-bromo-3-méthylphényl)hexahydro-4,7-imino-1H-isoindole-1,3(2H)-dione ;
la (3aα,4α,7α,7aα)-hexahydro-2-(4-nitro-1-naphtalényl)-4,7-imino-1H-isoindole-1,3(2H)-dione ;
la (3aα,4α,7α,7aα)-8-acétyl-2-(3,5-dichlorophényl)hexahydro-4,7-imino-1H-isoindole-1,3(2H)-dione ;
l'ester phénylique d'acide (3aα,4α,7α,7aα)-octahydro-1,3-dioxo-2-[3-(trifluorométhyl)phényl]-4,7-éthano-5H-pyrrolo[3,4-c]pyridine-5-carboxylique ;
le (3aα,4α,7α,7aα)-4-(octahydro-1,3-dioxo-4,7-éthano-2H-pyrrolo[3,4-c]pyridin-2-yl)-1-naphtalènecarbonitrile ;
le (3aα,4α,7α,7aα)-4-(octahydro-5-méthyl-1,3-dioxo-4,7-éthano-2H-pyrrolo[3,4-c]pyridin-2-yl)-1-naphtalène-carboni- trile ;
l'ester phénylméthylique d'acide (3aα,4α,7α,7aα)-2-(4-cyano-1-naphtalényl)octahydro-1,3-dioxo-4,7-éthéno-5H-pyrrolo[3,4-c]pyridine-5-carboxylique ;
le (3aα,4α,7α,7aα)-4-(octahydro-1,3-dioxo-4,7-éthano-2H-pyrrolo[3,4-c]pyridin-2-yl)-2-(trifluorométhyl)benzonitrile ;
le (3aα,4α,7α,7aα)-4-(octahydro-5-méthyl-1,3-dioxo-4,7-éthano-2H-pyrrolo[3,4-c]pyridin-2-yl)-2-(trifluorométhyl)benzonitrile ;
l'ester phénylméthylique d'acide (3aα,4α,7α,7aα)-2-[4-cyano-3-(trifluorométhyl)phényl]octahydro-1,3-dioxo-4,7-éthéno-5H-pyrrolo[3,4-c]pyridine-5-carboxylique ;
la (3aα,4α,7α,7aα)-2-[4-bromo-3-(trifluorométhyl)phényl]tétrahydro-5-méthyl-4,7-éthéno-1H-pyrrolo[3,4-c]pyridine-1,3,6(2H,5H)-trione ;
la (3aα,4α,7α,7aα)-tétrahydro-5-méthyl-2-[3-(trifluorométhyl)phényl]-4,7-éthéno-1H-pyrrolo[3,4-c]pyridine-1, 3,6(2H,5H)-trione ;
la (3aα,4α,7α,7aα)-tétrahydro-5-méthyl-2-(2-naphtalényl)-4,7-éthéno-1H-pyrrolo[3,4-c]pyridine-1,3,6(2H,5H) -trione ;
la (1aα,2β,2aα,5aα,6β,6aα)-hexahydro-4-[3-(trifluorométhyl)phényl]-2,6-époxy-3H-oxiréno[f]isoindole-3,5(4H) -dione ;
la (1aα,2β,2aα,5aα,6β,6aα)-4-(3,5-dichlorophényl)hexahydro-2,6-époxy-3H-oxiréno[f]isoindole-3,5(4H)-dione ;
la (1aα,2β,2aα,5aα,6β,6aα)-hexahydro-4-(4-nitro-1-naphtalényi)-2,6-époxy-3H-oxiréno[f]isoindole-3,5(4H) -dione ;
la (1aα,2β,2aα,5aα,6β,6aα)-4-(3,4-dichlorophényl)hexahydro-2,6-époxy-3H-oxiréno[f]isoindole-3,5(4H)-dione ;
la 2-[4-(4-bromophénoxy)phényl]-3a,4,7,7a-tétrahydro-4,7-diméthyl-4,7-époxy-1H-isoindole-1,3(2H)-dione ;
la 3a,4,7,7a-tétrahydro-2-(2-méthoxyphényl)-4,7-diméthyl-4,7-époxy-1H-isoindole-1,3(2H)-dione ;
l'ester (3,5-diméthoxyphényl)méthylique d'acide [(1,2,3,3a,7,7a-hexahydro-2-phényl-4,7-époxy-4H-isoindol-4-yl)méthyl]carbamique ;
la 2-(2,4-diméthylphényl)-3a,4,7,7a-tétrahydro-4-(hydroxyméthyl)-4,7-époxy-1H-isoindole-1,3(2H)-dione ;
la 2-(1,3-benzodioxol-5-yl)-3a,4,7,7a-tétrahydro-4-méthyl-4,7-époxy-1H-isoindole-1,3(2H)-dione ;
la 4-[bis(acétyloxy)méthyl]-2-(3-bromophényl)-3a,4,7,7a-tétrahydro-4,7-époxy-1H-isoindole-1,3(2H)-dione ;
le N-[[1,2,3,3a,7,7a-hexahydro-2-(2,4,6-triméthylphényl)-4,7-époxy-4H-isoindol-4-yl]méthyl]-2,2-diméthylpropanamide ;
la 3a,4,7,7a-tétrahydro-4-(hydroxyméthyl)-2-[2-(trifluorométhyl)phényl]-4,7-époxy-1H-isoindole-1,3(2H)-dione ;
la 3a,4,7,7a-tétrahydro-4-(hydroxyméthyl)-2-(1-naphtalényl)-4,7-époxy-1H-isoindole-1,3(2H)-dione ;
l'ester méthylique d'acide 2-chloro-5-(1,3,3a,4,7,7a-hexahydro-4,7-diméthyl-4,7-époxy-2H-isoindol-2-yl)benzoïque ;
la 4-[bis(acétyloxy)méthyl]-2-(4-bromo-2-nitrophényl)-3a,4,7,7a-tétrahydro-4,7-époxy-1H-isoindole-1,3(2H) -dione ;
la 3a,4,7,7a-tétrahydro-4-méthyl-2-(4-méthyl-3-nitrophényl)-4,7-époxy-1H-isoindole-1,3(2H)-dione ;

la 2-[2-chloro-5-(trifluorométhyl)phényl]-3a,4,7,7a-tétrahydro-4-méthyl-4,7-époxy-1H-isoindole-1,3(2H)-dione ;
la 2-[4-chloro-3-(trifluorométhyl)phényl]-3a,4,7,7a-tétrahydro-4,7-diméthyl-4,7-époxy-1H-isoindole-1,3(2H)-dione ;
le 2-(1,3,3a,4,7,7a-hexahydro-4-méthyl-4,7-époxy-2H-isoindol-2-yl)benzonitrile ;
la 2-(4-fluorophényl)-3a,4,7,7a-tétrahydro-4-méthyl-4,7-époxy-1H-isoindole-1,3(2H)-dione ;
le 2,2,2-trifluoro-N-[(1,2,3,3a,7,7a-hexahydro-2-phényl-4,7-époxy-4H-isoindol-4-yl)méthyl]acétamide ;
la 3a,4,7,7a-tétrahydro-4,7-diméthyl-2-(4-méthyl-3-nitrophényl)-4,7-époxy-1H-isoindole-1,3(2H)-dione ;
l'acide 2-chloro-5-[1,3,3a,4,7,7a-hexahydro-4-(hydroxyméthyl)-4,7-époxy-2H-isoindol-2-yl]benzoïque ;
la 3a,4,7,7a-tétrahydro-4,7-diméthyl-2-(4-nitrophényl)-4,7-époxy-1H-isoindole-1,3(2H)-dione ;
la 3a,4,7,7a-tétrahydro-2-(2-mercaptophényl)-4,7-époxy-1H-isoindole-1,3(2H)-dione ;
la 3a,4,7,7a-tétrahydro-2-[2-[(phénylméthyl)thio]phényl]-4,7-époxy-1H-isoindole-1,3(2H)-dione ;
l'ester 2-méthylpropylique d'acide [[2-(4-chlorophényl)-1,2,3,3a,7,7a-hexahydro-4,7-époxy-4H-isoindol-4-yl]méthyl]carbamique ;
le 4-(1,1-diméthyléthyl)-N-[[1,2,3,3a,7,7a-hexahydro-2-(4-méthylphényl)-4,7-époxy-4H-isoindol-4-yl]méthyl]benzamide ;
le 2,4-dichloro-N-[[1,2,3,3a,7,7a-hexahydro-2-(4-nitrophényl)-4,7-époxy-4H-isoindol-4-yl]méthyl]benzamide ;
le N-[[2-(4-chlorophényl)-1,2,3,3a,7,7a-hexahydro-4,7-époxy-4H-isoindol-4-yl]méthyl]-2,4,6-triméthylbenzènesulfonamide ;
l'ester 1,1-diméthyléthylique d'acide [(1,2,3,3a,7,7a-hexahydro-2-phényl-4,7-époxy-4H-isoindol-4-yl)méthyl]carbamique ;
le N-[(1,2,3,3a,7,7a-hexahydro-2-phényl-4,7-époxy-4H-isoindol-4-yl)méthyl]-2-phénoxyacétamide ;
le N-[[1,2,3,3a,7,7a-hexahydro-2-(4-nitrophényl)-4,7-époxy-4H-isoindol-4-yl]méthyl]-2,2-diméthylpropanamide ;
le 2-(2,4-dichlorophénoxy)-N-[[1,2,3,3a,7,7a-hexahydro-2-(4-nitrophényl)-4,7-époxy-4H-isoindol-4-yl]méthyl]acétamide ;
le N-[[1,2,3,3a,7,7a-hexahydro-2-(4-méthylphény4)-4,7-époxy-4H-isoindol-4-yl]méthyl]-3,5-diméthoxybenzamide ;
le N-[[2-(4-chlorophényl)-1,2,3,3a,7,7a-hexahydro-4,7-époxy-4H-isoindol-4-yl]méthyl]-2-nitrobenzènesulfonamide ;
la (3aα,4β,7β,7aα)-hexahydro-2-[(1S)-1-phényléthyl]-4,7-époxy-1H-isoindole-1,3(2H)-dione ;
la (3aα,4β,7β,7aα)-hexahydro-2-[(1S)-2-hydroxy-1-phényléthyl]-4,7-époxy-1H-isoindole-1,3(2H)-dione ;
la (3aα,4β,7β,7aα)-2-[(1S)-2-(acétyloxy)-1-phényléthyl]-3a,4,7,7a-tétrahydro-4,7-époxy-1H-isoindole-1,3(2H)-dione ;
la (3aα,4α,7α,7aα)-3a,4,7,7a-tétrahydro-2-[(1S)-1-phényléthyl]-4,7-époxy-1H-isoindole-1,3(2H)-dione ;
la (3aα,4β,7β,7aα)-hexahydro-2-[(1R)-1-phényléthyl]-4,7-époxy-1H-isoindole-1,3(2H)-dione ;
l'acide (3aα,4β,7β,7aα)-4-[[(octahydro-1,3-dioxo-4,7-époxy-2H-isoindol-2-yl)méthyl]amino]benzoïque ;
la (3aα,4β,7β,7aα)-hexahydro-2-(4-morpholinylméthyl)-4,7-époxy-1H-isoindole-1,3(2H)-dione ;
le (3aα,4β,7β,7aα)-4-[octahydro-4-(2-hydroxyéthyl)-7-méthyl-1,3-dioxo-4,7-époxy-2H-isoindol-2-yl]-2-(trifluorométhyl)benzonitrile ;
le (3aα,4β,7β,7aα)- et le (3aα,4α,7α,7aα)-4-[octahydro-4-méthyl-1,3-dioxo-7-(phénylméthyl)-4,7-époxy-2H-isoindol-2-yl]-2-(trifluorométhyl)benzonitrile ;
le (3aα,4β,7β,7aα)-4-[7-[2-(4-bromophénoxy)éthyl]octahydro-4-méthyl-1,3-dioxo-4,7-époxy-2H-isoindol-2-yl]-2-(trifluorométhyl)benzonitrile ;
le (3aα,4β,7β,7aα)-4-[octahydro-7-[2-(4-iodophénoxy)éthyl]-4-méthyl-1,3-dioxo-4,7-époxy-2H-isoindol-2-yl]-2-(trifluorométhyl)benzonitrile ;
le (3aα,4β,7β,7aα)-4-[octahydro-4-méthyl-1,3-dioxo-7-[2-[4-(trifluorométhyl)phénoxy]éthyl]-4,7-époxy-2H-isoindol-2-yl]-2-(trifluorométhyl)benzonitrile ;
le (3aα,4β,7β,7aα)-4-[octahydro-7-[2-(4-méthoxyphénoxy)éthyl]-4-méthyl-1,3-dioxo-4,7-époxy-2H-isoindol-2-yl]-2-(trifluorométhyl)benzonitrile ;
le (3aα,4β,7β,7aα)-4-[7-[2-(4-éthoxyphénoxy)éthyl]octahydro-4-méthyl-1,3-dioxo-4,7-époxy-2H-isoindol-2-yl]-2-(trifluorométhyl)benzonitrile ;
le (3aα,4β,7β,7aα)-4-[7-[2-(4-chlorophénoxy)éthyl]octahydro-4-méthyl-1,3-dioxo-4,7-époxy-2H-isoindol-2-yl]-2-(trifluorométhyl)benzonitrile ;
l'ester méthylique d'acide (3aα,4β,7β,7aα)-4-[2-[2-[4-cyano-3-(trifluorométhyl)phényl]octahydro-7-méthyl-1,3-dioxo-4,7-époxy-4H-isoindol-4-yl]éthoxy]benzoïque ;
la (3aα,4β,7β,7aα)-hexahydro-4-(2-hydroxyéthyl)-7-méthyl-2-(3-méthyl-4-nitrophényl)-4,7-époxy-1 H-isoindole-1,3(2H)-dione ;
le (3aα,4β,7β,7aα)-4-[octahydro-4-méthyl-1,3-dioxo-7-[2-[4-(trifluorométhoxy)phénoxy]éthyl]-4,7-époxy-2H-isoindol-2-yl]-2-(trifluorométhyl)benzonitrile ;
la (3aα,4β,7β,7aα)-2-(3,5-dichlorophényl)hexahydro-4,7-diméthyl-4,7-époxy-1H-isoindole-1,3(2H)-dione ;
la (3aα,4β,7β,7aα)-hexahydro-4,7-diméthyl-2-(4-nitro-1-naphtalényl)-4,7-époxy-1H-isoindole-1,3(2H)-dione ;
le (3aα,4β,7β,7aα)-2-[4-cyano-3-(trifluorométhyl)phényl]octahydro-7-méthyl-1,3-dioxo-4,7-époxy-4H-isoindole-4-propanenitrile ;
le trifluoroacétate de (3aα,4β,7β,7aα)-4-[octahydro-4-méthyl-7-[2-(4-morpholinyl)éthyl]-1,3-dioxo-4,7-époxy-2H-isoindol-2-yl]-2-(trifluorométhyl)benzonitrile ;
la (3aα,4β,7β,7aα)-2-(5-fluoro-1-naphtalényl)hexahydro-4,7-diméthyl-4,7-époxy-1H-isoindole-1,3(2H)-dione ;
la (3aα,4β,7β,7aα)-2-(5-fluoro-4-nitro-1-naphtalényl)hexahydro-4,7-diméthyl-4,7-époxy-1H-isoindole-1,3(2H)-dione ;
la (3aα,4β,7β,7aα)-2-(1,1-dioxydobenzo[b]thiophén-3-yl)hexahydro-4,7-diméthyl-4,7-époxy-1H-isoindole-1,3(2H)-dione ;
le 4-(1,3,3a,4,7,7a-hexahydro-4,6,7-triméthyl-1,3-dioxo-4,7-époxy-2H-pyrrolo[3,4-c]pyridin-2-yl)-2-(trifluorométhyl)benzonitrile ;
la (3aα,4β,7β,7aα)-tétrahydro-4,7-diméthyl-2-[3-(trifluorométhyl)phényl]-4,7-époxy-1H-isoindole-1,3,5(2H,4H)-trione ;
la (3aα,4α,7α,7aα)-tétrahydro-4,7-diméthyl-2-[3-(trifluorométhyl)phényl]-4,7-époxy-1H-isoindole-1,3,5(2H,4H)-trione ;
la (3aα,4β,7β,7aα)-2-(5-chloro-1-naphtalényl)hexahydro-4,7-diméthyl-4,7-époxy-1H-isoindole-1,3(2H)-dione ;
la (3aα,4β,7β,7aα)-2-(5-chloro-4-nitro-1-naphtalényl)hexahydro-4,7-diméthyl-4,7-époxy-1H-isoindole-1,3(2H)-dione ;
la (3aα,4β,7β,7aα)-4-éthylhexahydro-7-méthyl-2-(4-nitro-1-naphtalényl)-4,7-époxy-1H-isoindole-1,3(2H)-dione ;
le (3aα,4β,7β,7aα)-2-(4-cyano-1-naphtalényl)-N-(4-fluorophényl)octahydro-7-méthyl-1,3-dioxo-4,7-époxy-4H-isoindole-4-acétamide ;
la (3aα,4β,7β,7aα)-hexahydro-4-méthyl-2-(2-naphtalényl)-4,7-époxy-1H-isoindole-1,3(2H)-dione, énantiomère à élution rapide ;
la (3aα,4β,7β,7aα)-hexahydro-4-méthyl-2-(2-naphtalényl)-4,7-époxy-1H-isoindole-1,3(2H)-dione, énantiomère à élution lente ;
le (3aα,4β,7β,7aα)-4-[4-[2-[[(4-fluorophényl)méthyl]méthylamino]éthyl]octahydro-7-méthyl-1,3-dioxo-4,7-époxy-2H-isoindol-2-yl]-2-(trifluorométhyl)benzonitrile ;
le (3aα,4β,5β,6β,7β,7aα)-4-(octahydro-4,5,6,7-tétraméthyl-1,3-dioxo-4,7-époxy-2H-isoindol-2-yl)-2-(trifluorométhyl)benzonitrile ;
le (3aα,4β,7β,7aα)-4-[octahydro-4-méthyl-1,3-dioxo-7-[2-[4-(trifluorométhyl)phénoxy]éthyl]-4,7-époxy-2H-isoindol-2-yl]-2-(trifluorométhyl)benzonitrile, antipode à élution rapide ;
le (3aα,4β,7β,7aα)-4-[octahydro-4-méthyl-1,3-dioxo-7-[2-[4-(trifluprométhyl)phénoxy]éthyl]-4,7-époxy-2H-isoindol-2-yl]-2-(trifluorométhyl)benzonitrile, énantiomère à élution lente ;
le (3aα,4β,5β,7β,7aα)-4-(octahydro-5-hydroxy-4,7-diméthyl-1,3-dioxo-4,7-époxy-2H-isoindol-2-yl)-2-(trifluorométhyl)benzonitrile ;
le (3aα,4β,5α,7β,7aα)-4-(octahydro-5-hydroxy-4,7-diméthyl-1,3-dioxo-4,7-époxy-2H-isoindol-2-yl)-2-(trifluorométhyl)benzonitrile ;
l'ester 2-[(3aα,4β,7β,7aα)-2-(4-cyano-1-naphtalényl)octahydro-7-méthyl-1,3-dioxo-4,7-époxy-4H-isoindol-4-yl]éthylique d'acide (αR)-α-méthoxybenzèneacétique ;
le (3aα,4β,7β,7aα)-2-(méthylthio)-4-(octahydro-4,7-diméthyl-1,3-dioxo-4,7-époxy-2H-isoindol-2-yl)benzonitrile ;
le (3aα,4β,7β,7aα)-2-(méthylsulfinyl)-4-(octahydro-4,7-diméthyl-1,3-dioxo-4,7-époxy-2H-isoindol-2-yl)benzonitrile ;
le (3aα,4β,7β,7aα)-2-(méthylsulfonyl)-4-(octahydro-4,7-diméthyl-1,3-dioxo-4,7-époxy-2H-isoindol-2-yl)benzonitrile ;
la (3aα,4β,5β,7β,7aα)-7-[2-[[(1,1-diméthyléthyl)diméthylsilyl]oxy]éthyl]hexahydro-5-hydroxy-4-méthyl-2-(4-nitro-1-naphtalényl)-4,7-époxy-1H-isoindole-1,3(2H)-dione ;
la (3aα,4β,5β,7β,7aα)-hexahydro-5-hydroxy-7-(2-hydroxyéthyl)-4-méthyl-2-(4-nitro-1-naphtalényl)-4,7-époxy-1H-isoindole-1,3(2H)-dione ;
la (3aα,4β,5β,7β,7aα)-7-[2-(4-fluorophénoxy)éthyl]hexahydro-5-hydroxy-4-méthyl-2-(4-nitro-1-naphtalényl)-4,7-époxy-1H-isoindole-1,3(2H)-dione ;
le (3aα,4β,5β,6β,7β,7aα)-4-(octahydro-5,6-dihydroxy-4,7-diméthyl-1,3-dioxo-4,7-époxy-2H-isoindol-2-yl)-2-(trifluorométhyl)benzonitrile ;
le (3aα,4β,5α,6α,7β,7aα)-4-(octahydro-5,6-dihydroxy-4,7-diméthyl-1,3-dioxo-4,7-époxy-2H-isoindol-2-yl)-2-(trifluorométhyl)benzonitrile ;
le (3aα,4β,5β,6β,7β,7aα)-4-[octahydro-5,6-dihydroxy-4-(hydroxyéthyl)-7-méthyi-1,3-dioxo-4,7-époxy-2H-isoindol-2-yl]-1-naphtalènecarbonitrile ;
le (3aα,4β,5β,6β,7β,7aα)-4-[octahydro-5,6-dihydroxy-4-méthyl-1,3-dioxo-7-[2-[4-(trifluorométhyl)phénoxy]éthyl]-4,7-époxy-2H-isoindol-2-yl]-2-(trifluorométhyl)benzonitrile ;
le (3aα,4β,5β,5aβ,8aβ,8bα)-4-(décahydro-5-hydroxy-4-méthyl-1,3-dioxo-4,8a-époxy-2H-furo[3,2-e]isoindol-2-yl)-1-naphtalènecarbonitrile ;
l'acide (3aα,4β,7β,7aα)-2-(4-cyano-1-naphtalényl)octahydro-7-méthyl-1,3-dioxo-4,7-époxy-4H-isoindole-4-acétique ;
l'ester méthylique d'acide (3aα,4β,7β,7aα)-2-(4-cyano-1-naphtalényl)octahydro-7-méthyl-1,3-dioxo-4,7-époxy-4H-isoindole-4-acétique ;
le (3aα,4β,7β,7aα)-2-(4-cyano-1-naphtalényl)-N-[(4-fluorophényl)méthyl]octahydro-7-méthyl-1,3-dioxo-4,7-époxy-4H-isoindole-4-acétamide ;
le (3aα,4β,7β,7aα)-N-[2-[2-(4-cyano-1-naphtalényl)octahydro-7-méthyl-1,3-dioxo-4,7-époxy-4H-isoindol-4-yl]éthyl]-4-fluorobenzamide ;
le [3aR-(3aα,4β,7β,7aα)]-4-[octahydro-4-(2-hydroxyéthyl)-7-méthyl-1,3-dioxo-4,7-époxy-2H-isoindol-2-yl]-1-naphtalènecarbonitrile ;
le [3aS-(3aα,4β,7β,7aα)]-4-[octahydro-4-(2-hydroxyéthyl)-7-méthyl-1,3-dioxo-4,7-époxy-2H-isoindol-2-yl]-1-naphtalènecarbonitrile ;
le [3aR-(3aα,4β,7β,7aα)]-4-[4-[2-(3-fluorophénoxy)éthyl]octahydro-7-méthyl-1,3-dioxo-4,7-époxy-2H-isoindol-2-yl]-1-naphtalènecarbonitrile ;
le [3aS-(3aα,4β,7β,7aα)]-4-[4-[2-(3-fluorophénoxy)éthyl]octahydro-7-méthyl-1,3-dioxo-4,7-époxy-2H-isoindol-2-yl]-1-naphtalènecarbonitrile ;
l'ester 2-[(3aα,4β,7β,7aα)-2-(4-cyano-1-naphtalényl)octahydro-7-méthyl-1,3-dioxo-4,7-époxy-4H-isoindol-4-yl]éthylique d'acide (4-fluorophényl)carbamique ;
le (3aα,4β,7β,7aα)-4-[octahydro-4-(2-hydroxyéthyl)-1,3-dioxo-4,7-époxy-2H-isoindol-2-yl]-1-naphtalènecarbonitrile ;
le (3aα,4β,6β,7β,7aα)-4-[4-[2-(4-cyanophénoxy)éthyl]octahydro-6-hydroxy-1,3-dioxo-4,7-époxy-2H-isoindol-2-yl]-1-naphtalènecarbonitrile ;
le [3aS-(3aα,4β,5β,7β,7aα)]-4-[octahydro-5-hydroxy-7-(2-hydroxyéthyl)-4-méthyl-1,3-dioxo-4,7-époxy-2H-isoindol-2-yl]-1-naphtalènecarbonitrile ;
le [3aR-(3aα,4β,5β,7β,7aα)]-4-[octahydro-5-hydroxy-7-(2-hydroxyéthyl)-4-méthyl-1,3-dioxo-4,7-époxy-2H-isoindol-2-yl]-1-naphtalènecarbonitrile ;
le (3aα,4β,7β,7aα)-4-[4-[2-(4-cyanophénoxy)éthyl]-7-éthyloctahydro-1,3-dioxo-4,7-époxy-2H-isoindol-2-yl]-1-naphtalènecarbonitrile ;
la (3aα,4β,7β,7aα)-4-[2-(acétyloxy)éthyl]-2-(4-cyano-1-naphtalényl)hexahydro-7-méthyl-4,7-époxy-1H-isoindole-1,3(2H)-dione ;
le (3aα,4β,7β,7aα)-4-[octahydro-4-méthyl-1,3-dioxo-7-(2-oxoéthyl)-4,7-époxy-2H-isoindol-2-yl]-1-naphtalènecarbonitrile ;
le [3aα,4β(E),7β,7aα]-4-[4-[3-(4-cyanophényl)-2-propényl]octahydro-7-méthyl-1,3-dioxo-4,7-époxy-2H-isoindol-2-yl]-1-naphtalènecarbonitrile ;
le [3aα,4β(Z),7β,7aα]-4-[4-[3-(4-cyanophényl)-2-propényl]octahydro-7-méthyl-1,3-dioxo-4,7-époxy-2H-isoindol-2-yl]-1-naphtalènecarbonitrile ;
le (3aα,4β,7β,7aα)-4-[4-[3-(4-cyanophényl)propyl]octahydro-7-méthyl-1,3-dioxo-4,7-époxy-2H-isoindol-2-yl]-1-naphtalènecarbonitrile ;
le (3aα,4β,7β,7aα)-4-[4-[2-[(6-chloro-1,2-benzisoxazol-3-yl)oxy]éthyl]octahydro-7-méthyl-1,3-dioxo-4,7-époxy-2H-isoindol-2-yl]-1-naphtalènecarbonitrile ;
le (3aα,4β,7β,7aα)-4-[octahydro-4-méthyl-7-[2-[(6-nitro-1H-indazal-3-yl)oxy]éthyl]-1,3-dioxo-4,7-époxy-2H-isoindol-2-yl]-1-naphtalènecarbonitrile ;
le [3aS-(3aα,4β,5β,7β,7aα)]-4-[7-[2-(1,2-benzisoxazol-3-yloxy)éthyl]octahydro-5-hydroxy-4-méthyl-1,3-dioxo-4,7-époxy-2H-isoindol-2-yl]-1-naphtalènecarbonitrile ;
le [3aR-(3aα,4β,5β,7β,7aα)]-4-[7-[2-(1,2-benzisoxazol-3-yloxy)éthyl]octahydro-5-hydroxy-4-méthyl-1,3-dioxo-4,7-époxy-2H-isoindol-2-yl]-1-naphtalènecarbonitrile ;
le (3aα,4β,5β,7β,7aα)-4-(octahydro-5-hydroxy-4,7-diméthyl-1,3-dioxo-4,7-époxy-2H-isoindol-2-yl)-2-(trifluorométhyl)benzonitrile ;
le (3aα,4β,5β,7β,7aα)-4-(octahydro-5-hydroxy-4,7-diméthyl-1,3-dioxo-4,7-époxy-2H-isoindol-2-yl)-2-(trifluorométhyl)benzonitrile ;
le (3aα,4β,7β,7aα)-2-(4-cyano-1-naphtalényl)octahydro-1,3-dioxo-7-[2-(phénylméthoxy)éthyl]-4,7-époxy-4H-isoindole-4-propanenitrile ;
le (3aα,4α,7α,7aα)-2-(4-cyano-1-naphtalényl)octahydro-1,3-dioxo-7-[2-(phénylméthoxy)éthyl]-4,7-époxy-4H-isoindole-4-propanenitrile ;
le (3aα,4β,7β,7aα)-2-(4-cyano-1-naphtalényl)octahydro-7-(2-hydroxyéthyl)-1,3-dioxo-4,7-époxy-4H-isoindole-4-propanenitrile ;
le (3aα,4α,7α,7aα)-2-(4-cyano-1-naphtalényl)octahydro-7-(2-hydroxyéthyl)-1,3-dioxo-4,7-époxy-4H-isoindole-4-propanenitrile ;
le (3aα,4β,7β,7aα)-2-(4-cyano-1-naphtalényl)-7-[2-(4-fluorophénoxy)éthyl]octahydro-1,3-dioxo-4,7-époxy-4H-isoindole-4-propanenitrile ;
la (3aα,4β,7β,7aα)-2-(7-chloro-2,1,3-benzoxadiazol-4-yl)hexahydro-4,7-diméthyl-4,7-époxy-1H-isoindole-1,3(2H)-dione ;
la (3aα,4β,7β,7aα)-2-(7-chloro-2-méthyl-4-benzofuranyl)hexahydro-4,7-diméthyl-4,7-époxy-1H-isoindole-1,3(2H)-dione ;
la (3aα,4β,7β,7aα)-2-(7-chloro-2-méthylbenzo[b]thiophén-4-yl)hexahydro-4,7-diméthyl-4,7-époxy-1H-isoindole-1,3(2H)-dione ;
l'ester phénylméthylique d'acide [3aα,4β(E),7β,7aα]-4-[2-(4-cyano-1-naphtalényl)octahydro-7-méthyl-1,3-dioxo-4,7-époxy-4H-isoindol-4-yl]-2-buténoïque ;
l'acide (3aα,4β,7β,7aα)-2-(4-cyano-1-naphtalényl)octahydro-7-méthyl-1,3-dioxo-4,7-époxy-4H-isoindole-4-butanoïque ;
le (3aα,4β,7β,7aα)-2-(4-cyano-1-naphtalényl)-N-(4-fluorophényl)octahydro-7-méthyl-1,3-dioxo-4,7-époxy-4H-isoindole-4-butanamide ;
le [3aS-(3aα,4β,5β,7β,7aα)]-4-[7-[2-(acétyloxy)éthyl]octahydro-5-hydroxy-4-méthyl-1,3-dioxo-4,7-époxy-2H-isoindol-2-yl]-1-naphtalènecarbonitrile ;
le [3aR-(3aα,4β,5β,7β,7aα)]-4-[octahydro-5-hydroxy-7-(2-hydroxyéthyl)-4-méthyl-1,3-dioxo-4,7-époxy-2H-isoindol-2-yl]-1-naphtalènecarbonitrile ;
le [3aα,4β,7β,7aα(E)]-4-[octahydro-4-méthyl-1,3-dioxo-7-(4-oxo-4-phényl-2-butényl)-4,7-époxy-2H-isoindol-2-yl]-1-naphtalènecarbonitrile ;
le [3aα,4β,7β,7aα(E)]-4-[octahydro-4-méthyl-1,3-dioxo-7-(4-oxo-4-phényl-butanyl)-4,7-époxy-2H-isoindol-2-yl]-1-naphtalènecarbonitrile ;
le (3aα,4β,7β,7aα)-4-[7-[2-(4-bromophénoxy)éthyl]octahydro-4-méthyl-1,3-dioxo-4,7-époxy-2H-isoindol-2-yl]-2-(trifluorométhyl)benzonitrile ;
le (3aα,4β,7β,7aα)-4-[octahydro-7-[2-(4-iodophénoxy)éthyl]-4-méthyl-1,3-dioxo-4,7-époxy-2H-isoindol-2-yl]-2-(trifluorométhyl)benzonitrile ;
le (3aα,4β,7β,7aα)-4-[octahydro-4-méthyl-1,3-dioxo-7-[2-[4-(trifluorométhyl)phénoxy]éthyl]-4,7-époxy-2H-isoindol-2-yl]-2-(trifluorométhyl)benzonitrile ;
le (3aα,4β,7β,7aα)-4-[octahydro-7-[2-(4-méthoxyphénoxy)éthyl]-4-méthyl-1,3-dioxo-4,7-époxy-2H-isoindol-2-yl]-2-(trifluorométhyl)benzonitrile ;
le (3aα,4β,7β,7aα)-4-[7-[2-(4-éthoxyphénoxy)éthyl]octahydro-4-méthyl-1,3-dioxo-4,7-époxy-2H-isoindol-2-yl]-2-(trifluorométhyl)benzonitrile ;
le (3aα,4β,7β,7aα)-4-[7-[2-(4-chlorophénoxy)éthyl]octahydro-4-méthyl-1,3-dioxo-4,7-époxy-2H-isoindol-2-yl]-2-(trifluorométhyl)benzonitrile ;
l'ester méthylique d'acide (3aα,4β,7β,7aα)-4-[2-[2-[4-cyano-3-(trifluorométhyl)phényl]octahydro-7-méthyl-1,3-dioxo-4,7-époxy-4H-isoindol-4-yl]éthoxy]benzoïque ;
la (3aα,4β,7β,7aα)-hexahydro-4-(2-hydroxyéthyl)-7-méthyl-2-(3-méthyl-4-nitrophényl)-4,7-époxy-1H-isoindole-1,3(2H)-dione ;
le (3aα,4β,7β,7aα)-4-[octahydro-4-méthyl-1,3-dioxo-7-[2-[4-(trifluorométhoxy)phénoxy]éthyl]-4,7-époxy-2H-isoindol-2-yl]-2-(trifluorométhyl)benzonitrile ;
la (3aα,4β,7β,7aα)-2-(3,5-dichlorophényl)hexahydro-4,7-diméthyl-4,7-époxy-1H-isoindole-1,3(2H)-dione ;
la (3aα,4β,7β,7aα)-hexahydro-4,7-diméthyl-2-(4-nitro-1-naphtalényl)-4,7-époxy-1H-isoindole-1,3(2H)-dione ;
le (3aα,4β,7β,7aα)-4-[octahydro-4-méthyl-1,3-dioxo-7-[2-[4-(phénylméthoxy)phénoxy]éthyl]-4,7-époxy-2H-isoindol-2-yl]-2-(trifluorométhyl)benzonitrile ;
la (3aα,4β,7β,7aα)-hexahydro-4-(2-hydroxyéthyl)-7-méthyl-2-(4-nitro-1-naphtalényl)-4,7-époxy-1H-isoindole-1,3(2H)-dione ;
la (3aα,4β,7β,7aα)-4-[2-(4-fluorophénoxy)éthyl]hexahydro-7-méthyl-2-(3-méthyl-4-nitrophényl)-4,7-époxy-1H-isoindole-1,3(2H)-dione ;
le (3aα,4β,7β,7aα)-4-[octahydro-4-méthyl-1,3-dioxo-7-[2-[4-[(trifluorométhyl)thio]phénoxy]éthyl]-4,7-époxy-2H-isoindol-2-yl]-2-(trifluorométhyl)benzonitrile ;
le (3aα,4β,7β,7aα)-4-[octahydro-4-méthyl-7-[2-(4-nitrophénoxy)éthyl]-1,3-dioxo-4,7-époxy-2H-isoindol-2-yl]-2-(trifluorométhyl)benzonitrile ;
la (3aα,4β,7β,7aα)-4-[2-(4-fluorophénoxy)éthyl]hexahydro-7-méthyl-2-(4-nitro-1-naphtalényl)-4,7-époxy-1H-isoindole-1,3(2H)-dione ;
le (3aα,4β,7β,7aα)-4-[octahydro-7-méthyl-1,3-dioxo-7-[2-[2-(trifluorométhyl)phénoxy]éthyl]-4,7-époxy-2H-isoindol-2-yl]-2-(trifluorométhyl)benzonitrile ;
le (3aα,4β,7β,7aα)-4-[4-[2-(2-bromophénoxy)éthyl]octahydro-7-méthyl-1,3-dioxo-4,7-époxy-2H-isoindol-2-yl]-2-(trifluorométhyl)benzonitrile ;
le (3aα,4β,7β,7aα)-4-[4-[2-(3-fluorophénoxy)éthyl]octahydro-7-méthyl-1,3-dioxo-4,7-époxy-2H-isoindol-2-yl]-2-(trifluorométhyl)benzonitrile ;
la (3aα,4β,7β,7aα)-hexahydro-2-[4-(1H-imidazol-1-yl)phényl]-4-méthyl-4,7-époxy-1H-isoindole-1,3(2H)-dione ;
la (3aα,4β,7β,7aα)-2-[3-chloro-4-(2-thiazolyl)phényl]hexahydro-4-méthyl-4,7-époxy-1H-isoindole-1,3(2H)-dione ;
la (3aα,4β,7β,7aα)-hexahydro-4,7-diméthyl-2-(3-méthyl-4-nitrophényl)-4,7-époxy-1H-isoindole-1,3(2H)-dione ;
la (3aα,4β,7β,7aα)-hexahydro-4,7-diméthyl-2-(2-méthyl-4-nitrophényl)-4,7-époxy-1H-isoindole-1,3(2H)-dione ;
la (3aα,4β,7β,7aα)-2-(3,5-dichlorophényl)hexahydro-4-(2-hydroxyéthyl)-7-méthyl-4,7-époxy-1H-isoindole-1,3(2H)-dione ;
la (3aα,4β,7β,7aα)-2-(3,5-dichlorophényl)-4-[2-(4-fluorophénoxy)éthyl]hexahydro-7-méthyl-4,7-époxy-1H-isoindolo-1,3(2H)-dione ;
le (3aα,4β,7β,7aα)-4-[octahydro-4-[2-(4-hydroxyphénoxy)éthyl]-7-méthyl-1,3-dioxo-4,7-époxy-2H-isoindol-2-yl]-2-(trifluorométhyl)benzonitrile ;
le (3aα,4β,7β,7aα)-4-[4-[2-(4-cyanophénoxy)éthyl]octahydro-7-méthyl-1,3-dioxo-4,7-époxy-2H-isoindol-2-yl]-2-(trifluorométhyl)benzonitrile ;
le (3aα,4β,7β,7aα)-4-[octahydro-4-méthyl-1,3-dioxo-7-[2-[3-(trifluorométhyl)phénoxy]éthyl]-4,7-époxy-2H-isoindol-2-yl]-2-(trifluorométhyl)benzonitrile ;
le (3aα,4β,7β,7aα)-4-[4-[2-(3-bromophénoxy)éthyl]octahydro-7-méthyl-1,3-dioxo-4,7-époxy-2H-isoindol-2-yl]-2-(trifluorométhyl)benzonitrile ;
le (3aα,4β,7β,7aα)-4-[4-[(4-fluorophényl)méthyl]octahydro-7-méthyl-1,3-dioxo-4,7-époxy-2H-isoindol-2-yl]-2-(trifluorométhyl)benzonitrile ;
la (3aα,4β,7β,7aα)-2-(1,6-dihydro-1-méthyl-6-oxo-3-pyridinyl)hexahydro-4,7-diméthyl-4,7-époxy-1H-isoindole-1,3(2H)-dione ;
la (3aα,4β,7β,7aα)-hexahydro-4,7-diméthyl-2-(1-méthyl-6-oxo-3-pipéridinyl)-4,7-époxy-1H-isoindole-1,3(2H)-dione ;
le (3aα,4β,7β,7aα)-4-[4-[2-(3-cyanophénoxy)éthyl]octahydro-7-méthyl-1,3-dioxo-4,7-époxy-2H-isoindol-2-yl]-2-(trifluorométhyl)benzonitrile ;
l'ester phénylméthylique d'acide (3aα,4β,7β,7aα)-4-[2-[4-Cyano-3-(trifluorométhyl)phényl]octahydro-7-méthyl-1,3-dioxo-4,7-époxy-4H-isoindol-4-yl]éthoxy]benzoïque ;
le (3aα,4β,7β,7aα)-4-[octahydro-4-méthyl-1,3-dioxo-7-(2-phénoxyéthyl)-4,7-époxy-2H-isoindol-2-yl]-2-(trifluorométhyl)benzonitrile ;
la (3aα,4β,7β,7aα)-2-(3,5-dichloro-4-nitrophényl)hexahydro-4,7-diméthyl-4,7-époxy-1H-isoindole-1,3(2H)-dione ;
la (3aα,4β,7β,7aα)-2-(3,5-dichloro-4-hydroxyphényl)hexahydro-4,7-diméthyl-4,7-époxy-1H-isoindole-1,3(2H)-dione ;
la (3aα,4β,7β,7aα)-2-(5-fluoro-1-naphtalényl)hexahydro-4,7-diméthyl-4,7-époxy-1H-isoindole-1,3(2H)-dione ;
la (3aα,4β,7β,7aα)-hexahydro-4,7-diméthyl-2-(1-naphtalényl)-4,7-époxy-1H-isoindole-1,3(2H)-dione ;
la (3aα,4β,7β,7aα)-hexahydro-2-[3-méthoxy-4-(5-oxazolyl)phényl]-4,7-diméthyl-4,7-époxy-1H-isoindole-1,3(2H)-dione ;
la (3aα,4β,7β,7aα)-hexahydro-4-[2-(4-méthoxyphénoxy)éthyl]-7-méthyl-2-(4-nitro-1-naphtalényl)-4,7-époxy-1H-isoindole-1,3(2H)-dione ;
la (3aα,4β,7β,7aα)-hexahydro-4-méthyl-2-(4-nitro-1-naphtalényl)-7-[2-[4-(trifluorométhyl)phénoxy]éthyl]-4,7-époxy-1H-isoindole-1,3(2H)-dione ;
la (3aα,4β,7β,7aα)-hexahydro-4-méthyl-2-(4-nitro-1-naphtalényl)-7-[2-(4-nitrophénoxy)éthyl]-4,7-époxy-1H-isoindole-1,3(2H)-dione ;
la (3aα,4β,7β,7aα)-2-(1,6-dihydro-1,4-diméthyl-6-oxo-3-pyridinyl)hexahydro-4,7-diméthyl-4,7-époxy-1H-isoindole-1,3(2H)-dione ;
le (3aα,4β,7β,7aα)-4-[octahydro-7-méthyl-2-(4-nitro-1-naphtalényl)-1,3-dioxo-4,7-époxy-4H-isoindol-4-yl]éthoxy]benzonitrile ;
le (3aα,4β,7β,7aα)-4-(octahydro-4,7-diméthyl-1,3-dioxo-4,7-époxy-2H-isoindol-2-yl)-1,2-benzènedicarbonitrile ;
la (3aα,4β,7β,7aα)-4-(2-bromoéthyl)hexahydro-7-méthyl-2-(4-nitro-1-naphtalényl)-4,7-époxy-1H-isoindole-1,3(2H)-dione ;
le (3aα,4β,7β,7aα)-4-[4-[2-(4-cyanophénoxy)éthyl]octahydro-7-méthyl-1,3-dioxo-4,7-époxy-2H-isoindol-2-yl]-1-naphtalènecarbonitrile ;
le (3aα,4β,7β,7aα)-4-[octahydro-4-[2-(4-méthoxyphénoxy)éthyl]-7-méthyl-1,3-dioxo-4,7-époxy-2H-isoindol-2-yl]-1-naphtalènecarbonitrile ;
le (3aα,4β,7β,7aα)-4-[octahydro-4-[2-(3-méthoxyphénoxy)éthyl]-7-méthyl-1,3-dioxo-4,7-époxy-2H-isoindol-2-yl]-1-naphtalènecarbonitrile ;
le (3aα,4β,7β,7aα)-4-[4-[2-(3-fluorophénoxy)éthyl]octahydro-7-méthyl-1,3-dioxo-4,7-époxy-2H-isoindol-2-yl]-1-naphtalènecarbonitrile ;
le (3aα,4β,7β,7aα)-4-[octahydro-4-méthyl-7-[2-[3-(4-morpholinyl)phénoxy]éthyl]-1,3-dioxo-4,7-époxy-2H-isoindol-2-yl]-1-naphtalènecarbonitrile ;
le (3aα,4β,7β,7aα)-4-[octahydro-4-méthyl-7-[2-[4-nitro-3-(trifluorométhyl)phénoxy]éthyl]-1,3-dioxo-4,7-époxy-2H-isoindol-2-yl]-1-naphtalènecarbonitrile ;
le (3aα,4β,7β,7aα)-4-[4-[2-(3-cyanophénoxy)éthyl]octahydro-7-méthyl-1,3-dioxo-4,7-époxy-2H-isoindol-2-yl]-1-naphtalènecarbonitrile ;
la (3aα,4β,7β,7aα)-2-(2,3-dihydro-3-méthyl-2-oxo-6-benzothiazolyl)hexahydro-4,7-diméthyl-4,7-époxy-1H-isoindole-1,3(2H)-dione ;
la (3aα,4β,7β,7aα)-2-(2,3-dihydro-2-oxo-6-benzothiazolyl)hexahydro-4,7-diméthyl-4,7-époxy-1H-isoindole-1,3(2H)-dione ;
le (3aα,4β,7β,7aα)-4-[4-[2-[3-(diméthylamino)phénoxy]éthyl]octahydro-7-méthyl-1,3-dioxo-4,7-époxy-2H-isoindol-2-yl]-1-naphtalènecarbonitrile ;
le (3aα,4β,7β,7aα)-4-[2-[4-cyano-3-(trifluorométhyl)phényl]octahydro-7-méthyl-1,3-dioxo-4,7-époxy-4H-isoindol-4-yl]éthoxy]-1,2-benzènedicarbonitrile ;
le (3aα,4β,7β,7aα)-N-[2-cyano-5-(octahydro-4,7-diméthyl-1,3-dioxo-4,7-époxy-2H-isoindol-2-yl)phényl] acétamide ;
le (3aα,4β,7β,7aα)-4-(octahydro-4,7-diméthyl-1,3-dioxo-4,7-époxy-2H-isoindol-2-yl)-2-(trifluorométhoxy) benzonitrile ;
le (3aα,4β,7β,7aα)-2-méthoxy-4-(octahydro-4,7-diméthyl-1,3-dioxo-4,7-époxy-2H-isoindol-2-yl)benzonitrile ;
la (3aα,4β,7β,7aα)-2-[4-(4,5-dichloro-1H-imidazol-1-yl)phényl]hexahydro-4,7-diméthyl-4,7-époxy-1H-isoindole-1,3(2H)-dione ;
la (3aα,4β,7β,7aα)-2-[4-(4-bromo-1-méthyl-1H-pyrazol-3-yl)phényl]hexahydro-4,7-diméthyl-4,7-époxy-1H-isoindole-1,3(2H)-dione ;
le (3aα,4β,7β,7aα)-4-[octahydro-4-(2-hydroxyéthyl)-7-méthyl-1,3-dioxo-4,7-époxy-2H-isoindol-2-yl]-1-naphtalènecarbonitrile ;
le (3aα,4β,7β,7aα)-2-iodo-4-(octahydro-4,7-diméthyl-1,3-dioxo-4,7-époxy-2H-isoindol-2-yl)benzonitrile ;
le (3aα,4β,7β,7aα)-4-[4-[2-(4-fluorophénoxy)éthyl]octahydro-7-méthyl-1,3-dioxo-4,7-époxy-2H-isoindol-2-yl]-1-naphtalènecarbonitrile ;
le (3aα,4β,7β,7aα)-4-[octahydro-4-méthyl-1,3-dioxo-7-[2-[4-(trifluorométhyl)phénoxy]éthyl]-4,7-époxy-2H-isoindol-2-yl]-1-naphtalènecarbonitrile ;
le (3aα,4β,7β,7aα)-4-[4-[2-(4-cyano-3-fluorophénoxy)éthyl]octahydro-7-méthyl-1,3-dioxo-4,7-époxy-2H-isoindol-2-yl]-1-naphtalènecarbonitrile ;
le (3aα,4β,7β,7aα)-4-[octahydro-4-méthyl-1,3-dioxo-7-[2-[2,3,5,6-tétrafluoro-4-(trifluorométhyl)phénoxy]éthyl]-4,7-époxy-2H-isoindol-2-yl]-1-naphtalènecarbonitrile ;
la (3aα,4β,7β,7aα)-hexahydro-4,7-diméthyl-2-[4-(1H-1,2,4-triazol-3-yl)phényl]-4,7-époxy-1H-isoindole-1,3(2H)-dione ;
la (3aα,4β,7β,7aα)-2-[4-(4,5-dihydro-5-oxo-1,2,4-oxadiazol-3-yl)phényl]hexahydro-4,7-diméthyl-4,7-époxy-1H-isoindole-1,3(2H)-dione ;
la (3aα,4β,7β,7aα)-hexahydro-2-[3-méthoxy-4-(2-oxazolyl)phényl]-4,7-diméthyl-4,7-époxy-1H-isoindole-1,3(2H)-dione ;
la (3aα,4β,7β,7aα)-hexahydro-2-(4-hydroxy-1-naphtalényl)-4,7-diméthyl-4,7-époxy-1H-isoindole-1,3(2H)-dione ;
le trifluoroacétate de (3aα,4β,7β,7aα)-hexahydro-2-(8-hydroxy-5-quinolinyl)-4,7-diméthyl-4,7-époxy-1 H-isoindole-1,3(2H)-dione ;
le (3aα,4β,7β,7aα)-4-[octahydro-4-méthyl-1,3-dioxo-7-[2-[méthyl(phénylméthyl)amino]éthyl]-4,7-époxy-2H-isoindol-2-yl]-2-(trifluorométhyl)benzonitrile ;
la (3aα,4β,7β,7aα)-hexahydro-4,7-diméthyl-2-(5-quinolinyl)-4,7-époxy-1H-isoindole-1 ,3(2H)-dione ;
le (3aα,4β,7β,7aα)-5-(octahydro-4,7-diméthyl-1,3-dioxo-4,7-époxy-2H-isoindol-2-yl)-2-pyridinecarbonitrile ;
le (3aα,4β,7β,7aα)-5-(octahydro-4,7-diméthyl-1,3-dioxo-4,7-époxy-2H-isoindol-2-yl)-8-quinolinecarbonitrile ;
la (3aα,4β,7β,7aα)-2-(5-bromo-4-nitro-1-naphtalényl)hexahydro-4,7-diméthyl-4,7-époxy-1H-isoindole-1,3(2H)-dione ;
la (3aα,4β,7β,7aα)-2-(5-bromo-1-naphtalényl)hexahydro-4,7-diméthyl-4,7-époxy-1H-isoindole-1,3(2H)-dione ;
la (3aα,4β,7β,7aα)-hexahydro-4,7-diméthyl-2-[8-(trifluorométhyl)-4-quinolinyl]-4,7-époxy-1H-isoindole-1,3(2H)-dione ;
l'ester 2-[(3aα,4β,7β,7aα)-2-(4-cyano-1-naphtalényl)octahydro-7-méthyl-1,3-dioxo-4,7-époxy-4H-isoindol-4-yl]éthylique d'acide 4-fluorobenzoïque ;
l'ester 2-[(3aα,4β,7β,7aα)-2-(4-cyano-1-naphtalényl)octahydro-7-méthyl-1,3-dioxo-4,7-époxy-4H-isoindol-4-yl]éthylique d'acide benzèneacétique ;
l'ester 2-[(3aα,4β,7β,7aα)-2-(4-cyano-1-naphtalényl)octahydro-7-méthyl-1,3-dioxo-4,7-époxy-4H-isoindol-4-yl]éthylique d'acide 4-fluorobenzèneacétique ;
la (3aα,4β,7β,7aα)-hexahydro-4-méthyl-7-[2-[4-(méthylsulfonyl)phénoxy]éthyl]-2-(4-nitro-1-naphtalényl)-4,7-époxy-1H-isoindole-1,3(2H)-dione ;
la (3aα,4β,7β,7aα)-hexahydro-2-(2-naphtalényl)-4,7-d'méthyl-4,7-époxy-1H-isoindole-1,3(2H)-dione ;
la (3aα,4β,7β,7aα)-2-(4-chloro-1-naphtalényl)hexahydro-4,7-diméthyl-4,7-époxy-1H-isoindole-1,3(2H)-dione ;
le (3aα,4β,7β,7aα)-N-[(4-chlorophényl)méthyl]-2-(4-cyano-1-naphtalényl)octahydro-7-méthyl-1,3-dioxo-4,7-époxy-4H-isoindole-4-acétamide ;
l'ester 2-[(3aα,4β,7β,7aα)-2-(4-cyano-1-naphtalényl)octahydro-7-méthyl-1,3-dioxo-4,7-époxy-4H-isoindol-4-yl]éthylique d'acide 4,7,7-triméthyl-3-oxo-2-oxabicyclo[2.2.1]heptane-1-carboxylique ;
l'ester 2-[(3aα,4β,7β,7aα)-2-(4-cyano-1-naphtalényl)octahydro-7-méthyl-1,3-dioxo-4,7-époxy-4H-isoindol-4-yl]éthylique d'acide (αS)-α-méthoxy-α-(trifluorométhyl)benzèneacétique ;
l'ester 2-[(3aα,4β,7β,7aα)-2-(4-cyano-1-naphtalényl)octahydro-7-méthyl-1,3-dioxo-4,7-époxy-4H-isoindol-4-yl]éthylique d'acide (αR)-α-méthoxy-α-(trifluorométhyl)benzèneacétique ;
le (3aα,4β,7β,7aα)-4-[octahydro-4-méthyl-7-[2-[(7-méthyl-1,2-benzisoxazol-3-yl)oxy]éthyl]-1,3-dioxo-4,7-époxy-2H-isoindol-2-yl]-1-naphtalènecarbonitrile ;
le (3aα,4β,7β,7aα)-4-[4-[2-(1,2-benzisoxazol-3-yloxy)éthyl]octahydro-7-méthyl-1,3-dioxo-4,7-époxy-2H-isoindol-2-yl]-1-naphtalènecarbonitrile ;
la (3aα,4β,7β,7aα)-4-[2-(benzoyloxy)éthyl]-2-(4-cyano-1-naphtalényl)hexahydro-7-méthyl-4,7-époxy-1H- isoindole-1,3(2H)-dione ;
la (3aα,4β,7β,7aα)-2-(4-cyano-1-naphtalényl)-4-[2-[(4-nitrobenzoyl)oxy]éthyl]hexahydro-7-méthyl-4,7-époxy-1H-isoindole-1,3(2H)-dione ;
l'ester 2-[(3aα,4β,7β,7aα)-2-(4-cyano-1-naphtalényl)octahydro-7-méthyl-1,3-dioxo-4,7-époxy-4H-isoindol-4-yl]éthylique d'acide 4-chlorobenzoïque ;
le [3aα,4β,7β,7aα(E)]-4-[octahydro-4-méthyl-7-[3-(1-naphtalényl)-2-propényl]-1,3-dioxo-4,7-époxy-2H-isoindol-2-yl]-1-naphtalènecarbonitrile ;
le (3aα,4β,7β,7aα)-4-[octahydro-4-méthyl-7-[3-(1-naphtalényl)propyl]-1,3-dioxo-4,7-époxy-2H-isoindol-2-yl]-1-naphtalènecarbonitrile ;
la (3aα,4β,7β,7aα)-hexahydro-4,7-diméthyl-2-(2-méthyl-6-quinolinyl)-4,7-époxy-1H-isoindole-1,3(2H)-dione ;
la (3aα,4β,7β,7aα)-hexahydro-2-(5-isoquinolinyl)-4,7-diméthyl-4.7-époxy-1H-isoindole-1,3(2H)-dione ;
la (3aα,4β,7β,7aα)-2-(6-benzothiazolyl)hexahydro-4,7-diméthyl-4,7-époxy-1H-isoindole-1,3(2H)-dione ;
le [3aα,4β,7β,7aα(E)]-4-[octahydro-4-méthyl-1,3-dioxo-7-(4-oxo-4-phényl-2-butényl)-4,7-époxy-2H-isoindol-2-yl]-1-naphtalènecarbonitrile ;
le (3aα,4β,7β,7aα)-2-(4-cyano-1-naphtalényl)octahydro-N-(2-hydroxyphényl)-7-méthyl-1,3-dioxo-4,7-époxy-4H-isoindole-4-acétamide ;
le [3aα,4β(E),7β,7aα]-4-[octahydro-4-méthyl-7-[3-(6-méthyl-2-pyridinyl)-2-propényl]-1,3-dioxo-4,7-époxy-2H-isoindol-2-yl]-1-naphtalènecarbonitrile ;
le (3aα,4β,7β,7aα)-4-[octahydro-4-méthyl-7-[3-(6-méthyl-2-pyridinyl)propyl]-1,3-dioxo-4,7-époxy-2H-isoindol-2-yl]-1-naphtalènecarbonitrile ;
le [3aR-(3aα,4β,7β,7aα)]-4-[octahydro-4-[2-(3-méthoxyphénoxy)éthyl]-7-méthyl-1,3-dioxo-4,7-époxy-2H-isoindol-2-yl]-1-naphtalènecarbonitrile ;
le [3aS-(3aα,4β,7β,7aα)]-4-[octahydro-4-[2-(3-méthoxyphénoxy)éthyl]-7-méthyl-1,3-dioxo-4,7-époxy-2H-isoindol-2-yl]-1-naphtalènecarbonitrile ;
le [3aR-(3aα,4β,7β,7aα)]-4-[4-[2-(4-cyanophénoxy)éthyl]octahydro-7-méthyl-1,3-dioxo-4,7-époxy-2H-isoindol-2-yl]-1-naphtalènecarbonitrile ;

le [3aS-(3aα,4β,7β,7aα)]-4-[4-[2-(4-cyanophénoxy)éthyl]octahydro-7-méthyl-1,3-dioxo-4,7-époxy-2H-isoindol-2-yl]-1-naphtalènecarbonitrile ;
le (3aα,4α,7α,7aα)-4-[4-[(4-fluorophényl)méthyl]octahydro-7-méthyl-1,3-dioxo-4,7-époxy-2H-isoindol-2-yl]-2-(trifluorométhyl)benzonitrile ;
la (3aα,4α,7α,7aα)-hexahydro-4,7-diméthyl-2-(1-méthyl-6-oxo-3-pipéridinyl)-4,7-époxy-1H-isoindole-1,3(2H)-dione ;
la (3aα,4α,7α,7aα)-2-(1,6-dihydro-1,4-diméthyl-6-oxo-3-pyridinyl)hexahydro-4,7-diméthyl-4,7-époxy-1H-isoindole-1,3(2H)-dione ;
le (3aα,4β,7β,7aα)-2-[4-cyano-3-(trifluorométhyl)phényl]octahydro-1,3-dioxo-7-[2-(phénylméthoxy)éthyl]-4,7-époxy-4H-isoindole-4-propanenitrile ;
le (3aα,4β,7β,7aα)-2-[4-cyano-3-(trifluorométhyl)phényl]octahydro-1,3-dioxo-7-[2-(phénylméthoxy)éthyl]-4,7-époxy-4H-isoindole-4-propanenitrile ;
le (3aα,4β,7β,7aα)-4-[7-[2-(4-cyanophénoxy)éthyl]octahydro-5-hydroxy-4-méthyl-1,3-dioxo-4,7-époxy-2H-isoindol-2-yl]-1-naphtalènecarbonitrile ;
le [3aS-(3aα,4β,7β,7aα)]-4-[7-[2-(1,3-benzodioxol-5-yloxy)éthyl]octahydro-5-hydroxy-4-méthyl-1,3-dioxo-4,7-époxy-2H-isoindol-2-yl]-1-naphtalènecarbonitrile ;
le [3aR-(3aα,4β,7β,7aα)]-4-[7-[2-(1,3-benzodioxol-5-yloxy)éthyl]octahydro-5-hydroxy-4-méthyl-1,3-dioxo-4,7-époxy-2H-isoindol-2-yl]-1-naphtalènecarbonitrile ;
le [3aS-(3aα,4β,7β,7aα)]-4-[7-[2-[(5-chloro-2-pyridinyl)oxy]éthyl]octahydro-5-hydroxy-4-méthyl-1,3-dioxo-4,7-époxy-2H-isoindol-2-yl]-1-naphtalènecarbonitrile ;
le [3aR-(3aα,4β,7β,7aα)]-4-[7-[2-[(5-chloro-2-pyridinyl)oxy]éthyl]octahydro-5-hydroxy-4-méthyl-1,3-dioxo-4,7-époxy-2H-isoindol-2-yl]-1-naphtalènecarbonitrile ;
le [3aS-(3aα,4β,7β,7aα)]-4-[7-[2-(4-chlorophénoxy)éthyl]octahydro-5-hydroxy-4-méthyl-1,3-dioxo-4,7-époxy-2H-isoindol-2-yl]-1-naphtalènecarbonitrile ;
le [3aR-(3aα,4β,7β,7aα)]-4-[7-[2-(4-chlorophénoxy)éthyl]octahydro-5-hydroxy-4-méthyl-1,3-dioxo-4,7-époxy-2H-isoindol-2-yl]-1-naphtalènecarbonitrile ;
le [3aS-(3aα,4β,7β,7aα)]-4-[7-[2-(4-acétylphénoxy)éthyl]octahydro-5-hydroxy-4-méthyl-1,3-dioxo-4,7-époxy-2H-isoindol-2-yl]-1-naphtalènecarbonitrile ;
le [3aR-(3aα,4β,7β,7aα)]-4-[7-[2-(4-acétylphénoxy)éthyl]octahydro-5-hydroxy-4-méthyl-1,3-dioxo-4,7-époxy-2H-isoindol-2-yl]-1-naphtalènecarbonitrile ;
le [3aS-(3aα,4β,7β,7aα)]-4-[7-[2-(3-cyanophénoxy)éthyl]octahydro-5-hydroxy-4-méthyl-1,3-dioxo-4,7-époxy-2H-isoindol-2-yl]-1-naphtalènecarbonitrile ;
le [3aR-(3aα,4β,7β,7aα)]-4-[7-[2-(3-cyanophénoxy)éthyl]octahydro-5-hydroxy-4-méthyl-1,3-dioxo-4,7-époxy-2H-isoindol-2-yl]-1-naphtalènecarbonitrile ;
le [3aS-(3aα,4β,7β,7aα)]-4-[octahydro-5-hydroxy-4-méthyl-1,3-dioxo-7-[2-[(5,6,7,8-tétrahydro-1-naphtalényl)oxy]éthyl]-4,7-époxy-2H-isoindol-2-yl]-1-naphtalènecarbonitrile ;
le [3aR-(3aα,4β,7β,7aα)]-4-[octahydro-5-hydroxy-4-méthyl-1,3-dioxo-7-[2-[(5,6,7,8-tétrahydro-1-naphtalényl)oxy]éthyl]-4,7-époxy-2H-isoindol-2-yl]-1-naphtalènecarbonitrile ;
le [3aS-(3aα,4β,7β,7aα)]-4-[octahydro-5-hydroxy-4-méthyl-1,3-dioxo-7-[2-[(5,6,7,8-tétrahydro-5-oxo-1-naphtalényl)oxy]éthyl]-4,7-époxy-2H-isoindol-2-yl]-1-naphtalènecarbonitrile ;
le [3aR-(3aα,4β,7β,7aα)]-4-[octahydro-5-hydroxy-4-méthyl-1,3-dioxo-7-[2-[(5,6,7,8-tétrahydro-5-oxo-1-naphtalényl)oxy]éthyl]-4,7-époxy-2H-isoindol-2-yl]-1-naphtalènecarbonitrile ;
le [3aS-(3aα,4β,7β,7aα)]-4-[7-[2-(4-fluorophénoxy)éthyl]octahydro-5-hydroxy-4-méthyl-1,3-dioxo-4,7-époxy-2H-isoindol-2-yl]-1-naphtalènecarbonitrile ;
le [3aR-(3aα,4β,7β,7aα)]-4-[7-[2-(4-fluorophénoxy)éthyl]octahydro-5-hydroxy-4-méthyl-1,3-dioxo-4,7-époxy-2H-isoindol-2-yl]-1-naphtalènecarbonitrile ;
le [3aS-(3aα,4β,7β,7aα)]-4-[octahydro-5-hydroxy-4-méthyl-7-[2-[(4-méthyl-2-oxo-2H-1-benzopyran-7-yl)oxy]éthyl]-1,3-dioxo-4,7-époxy-2H-isoindol-2-yl]-1-naphtalènecarbonitrile ;
le [3aR-(3aα,4β,7β,7aα)]-4-[octahydro-5-hydroxy-4-méthyl-7-[2-[(4-méthyl-2-oxo-2H-1-benzopyran-7-yl)oxy]éthyl]-1,3-dioxo-4,7-époxy-2H-isoindol-2-yl]-1-naphtalènecarbonitrile ;
le [3aS-(3aα,4β,7β,7aα)]-4-[7-[2-(3,5-diméthoxyphénoxy)éthyl]octahydro-5-hydroxy-4-méthyl-1,3-dioxo-4,7-époxy-2H-isoindol-2-yl]-1-naphtalènecarbonitrile ;
le [3aR-(3aα,4β,7β,7aα)]-4-[7-[2-(3,5-diméthoxyphénoxy)éthyl]octahydro-5-hydroxy-4-méthyl-1,3-dioxo-4,7-époxy-2H-isoindol-2-yl]-1-naphtalènecarbonitrile ;
le [3aR-(3aα,4β,7β,7aα)]-4-[7-[2-(4-chloro-3-méthylphénoxy)éthyl]octahydro-5-hydroxy-4-méthyl-1,3-dioxo-4,7-époxy-2H-isoindol-2-yl]-1-naphtalènecarbonitrile ;
le [3aR-(3aα,4β,7β,7aα)]-4-[7-[2-(4-cyano-2,3-difluorophénoxy)éthyl]octahydro-5-hydroxy-4-méthyl-1,3-dioxo-4,7-époxy-2H-isoindol-2-yl]-1-naphtalènecarbonitrile ;

le [3aS-(3aα,4β,7β,7aα)]-4-[7-[2-[(5-chloro-1,2-benzisoxazol-3-yl)oxy]éthyl]octahydro-5-hydroxy-4-méthyl-1,3-dioxo-4,7-époxy-2H-isoindol-2-yl]-1-naphtaiènecarbonitrile ;
le [3aR-(3aα,4β,7β,7aα)]-4-[7-[2-[(5-chloro-1,2-benzisoxazol-3-yl)oxy]éthyl]octahydro-5-hydroxy-4-méthyl-1,3-dioxo-4,7-époxy-2H-isoindol-2-yl]-1-naphtalènecarbonitrile ;
l'ester méthylique d'acide [3aR-(3aα,4β,7β,7aα)]-3-[2-[2-(4-cyano-1-naphtalényl)octahydro-6-hydroxy-7-méthyl-1,3-dioxo-4,7-époxy-4H-isoindol-4-yl]éthoxy]-5-isoxazolecarboxylique ;
le [3aR-(3aα,4β,7β,7aα)]-4-[octahydro-5-hydroxy-4-méthyl-1,3-dioxo-7-[2-[4-(1H-1,2,4-triazol-1-yl)phénoxy]éthyl]-4,7-époxy-2H-isoindol-2-yl]-1-naphtalènecarbonitrile ;
le trifluoroacétate de [3aS-(3aα,4β,7β,7aα)]-4-[7-[2-[(7-chloro-4-quinolinyl)oxy]éthyl]octahydro-5-hydroxy-4-méthyl-1,3-dioxo-4,7-époxy-2H-isoindol-2-yl]-1-naphtalènecarbonitrile ;
le trifluoroacétate de [3aR-(3aα,4β,7β,7aα)]-4-[7-[2-[(7-chloro-4-quinolinyl)oxy]éthyl]octahydro-5-hydroxy-4-méthyl-1,3-dioxo-4,7-époxy-2H-isoindol-2-yl]-1-naphtalènecarbonitrile ;
le (1aα,2β,2aα,5aα,6βb,6aα)-4-[2-[2-[[(1,1-diméthyléthyl)-diméthylsilyl]oxy]éthyl]octahydro-6-méthyl-3,5-dioxo-2,6-époxy-4H-oxiréno[f]isoindol-4-yl]-1-naphtatènecarbonitrile ;
le [3aR-(3aα,4β,7β,7aα)]-4-[4-éthyloctahydro-7-(2-hydroxyéthyl)-1,3-dioxo-4,7-époxy-2H-isoindol-2-yl]-1-naphtalènecarbonitrile ;
le [3aS-(3aα,4β,7β,7aα)]-4-[4-éthyloctahydro-7-(2-hydroxyéthyl)-1,3-dioxo-4,7-époxy-2H-isoindol-2-yl]-1-naphtalènecarbonitrile ;
le [3aR-(3aα,4β,7β,7aα)]-4-[4-[2-(4-cyanophénoxy)éthyl]-7-éthyloctahydro-1,3-dioxo-4,7-époxy-2H-isoindol-2-yl]-1-naphtalènecarbonitrile ; et
le [3aS-(3aα,4β,7β,7aα)]-4-[4-[2-(4-cyanophénoxy)éthyl]-7-éthyloctahydro-1,3-dioxo-4,7-époxy-2H-isoindol-2-yl]-1-naphtalènecarbonitrile.

4. Composé selon la revendication 3, ledit composé étant:

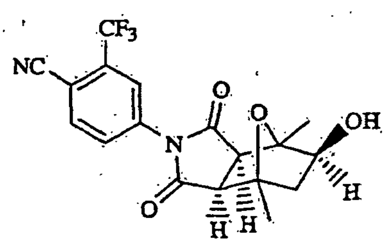

ou

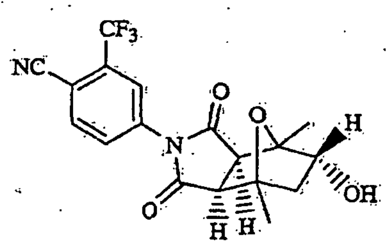

ou un sel ou hydrate de celui-ci.

5. Composition pharmaceutique capable de traiter une pathologie associée à un récepteur hormonal nucléaire, comprenant un composé répondant à la formule I selon la revendication 1 selon la condition (10) de la revendication 1 ou sel ou hydrate pharmaceutiquement acceptable de celui-ci, et support pharmaceutiquement acceptable.

6. Composition pharmaceutique comprenant un composé selon la revendication 1, 2, 3 ou 4.

7. Composition pharmaceutique contenant un composé selon la revendication 4.

8. Composition pharmaceutique selon la revendication 5, 6 ou 7 comprenant en outre un autre agent anti-cancer.

9. Utilisation d'un composé selon la revendication 1, 2, 3 ou 4 pour la préparation d'un médicament destiné à moduler la fonction d'un récepteur hormonal nucléaire.

10. Utilisation selon la revendication 9 dans laquelle ledit récepteur hormonal nucléaire est choisi dans le groupe constitué par un récepteur hormonal nucléaire de liaison à un stéroïde selon la revendication 1, 2, 3 ou 4, le récepteur de l'androgène, le récepteur de l'estrogène, le récepteur de la progestérone, le récepteur du glucocorticoïde, le récepteur du minéralocorticoïde, et le récepteur de l'aldostérone.

11. Utilisation d'un composé selon la revendication 1, 2, 3 ou 4 pour la préparation d'un médicament destiné à traiter une pathologie ou un trouble

dans laquelle ladite pathologie ou trouble est choisi dans le groupe constitué par les maladies prolifératives, les cancers, l'hypertrophie bénigne de la prostate, les adénomes et les néoplasies de la prostate, les cellules de tumeur bénigne ou maligne contenant le récepteur de l'androgène, les cardiopathies, les pathologies ou les troubles angiogéniques, l'hirsutisme, l'acné, l'hyperpilosité, l'inflammation, la modulation immunitaire, la séborrhée, l'endométriose, le syndrome des ovaires polycystiques, l'alopécie androgénique, l'hypogonadisme, l'ostéoporose, la suppression de la spermatogenèse, de la libido, la cachexie, l'anorexie, l'inhibition de l'atrophie musculaire chez les malades ambulatoires, la supplémentation en androgènes pour les baisses du niveau de testostérone dues à l'âge chez les hommes, les cancers exprimant le récepteur de l'estrogène, le cancer de la prostate, le cancer du sein, le cancer de l'endomètre, les bouffées de chaleur, la sécheresse vaginale, la ménopause, l'aménorrhée, la dysménorrhée, la contraception, l'interruption de grossesse, les cancers contenant le récepteur de la progestérone, l'endométriose, la synchronie des cycles, le méningiome, les fibromyomes, l'induction du travail, les maladies auto-immunes, la maladie d'Alzheimer, les troubles psychotiques, la pharmacodépendance, le diabète pas dépendant d'insuline, les troubles véhiculés par les récepteurs dopaminergiques, l'insuffisance cardiaque congestive, le dérèglement de l'homéostasie du cholestérol, et l'atténuation du métabolisme d'un agent pharmaceutique.

**12.** Méthode de préparation d'un composé répondant à la formule XVI suivante, ou d'un sel de celui-ci :

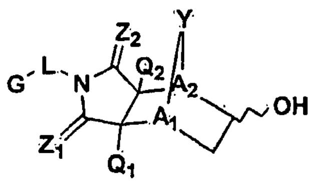

XVI

où G, L, $Z_1$, $Z_2$, $Q_1$, $Q_2$, $A_1$, $A_2$ et Y sont tels que définis dans la revendication 1,
comprenant les étapes consistant à mettre en contact un composé répondant à la formule XV suivante, ou un sel de celui-ci :

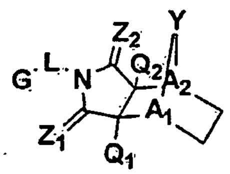

XV

où les symboles sont tels que définis ci-dessus ;
avec une enzyme ou un microorganisme capable de catalyser l'hydroxylation dudit composé XV en ledit composé XVI, et à réaliser ladite hydroxylation.

**13.** Méthode de préparation d'un composé répondant à la formula XVIII suivante, ou d'un sel de celui-ci :

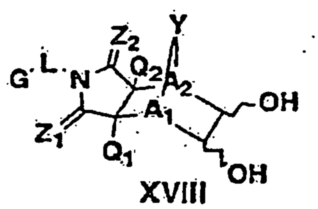

XVIII

où G, L, $Z_1$, $Z_2$, $Q_1$, $O_2$, $A_1$, $A_2$ et Y sont tels que définis dans la revendication 3 ;
comprenant les étapes consistant à mettre en contact un composé répondant à la formule XVII suivante, ou un sel

de celui-ci :

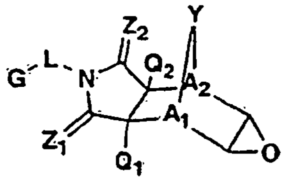

XVII

où les symboles sont tels que définis ci-dessus ;
avec une enzyme ou un microorganisme capable de catalyser l'ouverture du noyau époxyde du composé XVII pour former le diol dudit composé XVIII, et à réaliser l'ouverture dudit noyau et la formation du diol.